# EUROPEAN PATENT APPLICATION

(11) **EP 0 985 670 A1**
(43) Date of publication of application: **15.03.2000**
(21) Application number: 99306382.5
(22) Date of filing: 12.08.1999
(51) Int. Cl.: C07D 417/12, C07D 417/14, C07D 413/12, A01N 43/76, A01N 43/78

(54) **1-(3-Heterocyclylphenyl)isothiourea, -isourea, -guanidine and -amidine compounds as herbicides**

(30) Priority: 13.08.1998 US 133872
(71) Applicant: American Cyanamid Company, Madison, New Jersey 07940-0874 (US)
(72) Inventor: Karp, Gary Mitchell, Princeton Junction, New Jersey 08550 (US)
(74) Representative: Mannion, Sally Kim, Dr.

(57) **Abstract**

There are provided 1-(3-heterocyclyphenyl)isothiourea, -isourea, -guanidine and -amidine compounds of formula I

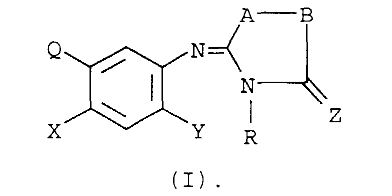

Further provided are compositions and methods comprising those compounds for the control of undesirable plant species.

## Description

### BACKGROUND OF THE INVENTION

Weeds cause tremendous global economic losses by reducing crop yields and lowering crop quality. In the United States alone, agronomic crops must compete with hundreds of weed species.

In spite of the commercial herbicides available today, damage to crops caused by weeds still occurs. Accordingly, there is ongoing research to create new and more effective herbicides.

JP 07304759-A and DE 3505432-A describe certain iminothiazolone derivatives which are useful as herbicidal agents. However, none of the compounds described in those applications are substituted with the 3-heterocyclylphenyl group of the present invention.

U.S. 3,287,466 describes certain 3-alkyl-4-imino-5-(arylimino)-2-thiazolidinone compounds which are useful as herbicidal agents. However, the arylimino groups of those compounds are not substituted with the heterocyclyl groups of this invention.

It is therefore an object of the present invention to provide compounds which are highly effective for controlling undesirable plant species.

It is also an object of the present invention to provide methods for controlling undesirable plant species.

These and other objects of the present invention will become more apparent from the detailed description thereof set forth below.

### SUMMARY OF THE INVENTION

The present invention describes 1-(3-heterocyclylphenyl)isothiourea, -isourea, -guanidine and -amidine compounds which are useful as herbicidal agents.

The 1-(3-heterocyclylphenyl)isothiourea, -isourea, -guanidine and -amidine compounds of the present invention have the structural formula I 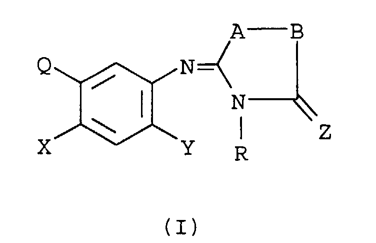 wherein
- X and Y: are each independently hydrogen, halogen, nitro, cyano, C₁-C₄alkyl, C₁-C₄haloalkyl or S(O)ₘR₁;
- R is: hydrogen,
a C₁-C₈alkyl, C₃-C₇cycloalkyl, C₃-C₈alkenyl, C₅-C₇cycloalkenyl or C₃-C₈alkynyl group, wherein each group is optionally substituted with any combination of one to six halogen atoms, one or two cyano groups, one or two nitro groups, one or two C(W)R₂ groups, one or two C(W)OR₃ groups, one or two C(W)NR₄R₅ groups, one or two P(V) (OR₆)₂ groups, one or two OR₇ groups, one or two SR₈ groups, one or two NR₉R₁₀ groups, one or two OC(W)R₁₁ groups, one or two NR₁₂S(O)ₙR₁₃ groups, one or two C(W)NR₁₂S(O)ₙR₁₃ groups,
one 4- to 10-membered heterocyclic ring optionally substituted with any combination of one or two oxo groups, one or two thioxo groups, one to three halogen atoms, one to three nitro groups, one to three cyano groups, one to three C₁-C₄alkyl groups, one to three C₁-C₄haloalkyl groups, one to three C₁-C₄alkoxy groups, one to three C₁-C₄haloalkoxy groups, one or two C₂-C₆alkoxyalkyl groups, one or two C₂-C₆haloalkoxyalkyl groups, one or two C(O)OR₁₄ groups, one or two C(O)R₁₄ groups, one to three C₃-C₆alkoxycarbonylalkyl groups, one to three C₃-C₆haloalkoxycarbonylalkyl groups, one to three C₃-C₆alkylcarbonyalkyl groups, one to three C₃-C₆haloalkylcarbonylalkyl groups, one or two NR₁₅S(O)ₚR₁₆ groups or one or two C(W)NR₁₅S(O)ₚR₁₆ groups, or
one phenyl group optionally substituted with any combination of one to three halogen atoms, one to three nitro groups, one to three cyano groups, one to three C₁-C₄alkyl groups, one to three C₁-C₄haloalkyl groups, one to three C₁-C₄alkoxy groups, one to three C₁-C₄haloalkoxy groups, one or two C₂-C₆alkoxyalkyl groups, one or two C₂-C₆₋haloalkoxyalkyl groups, one or two C(O)OR₁₄ groups, one or two C(O)R₁₄ groups, one to three C₃-C₆alkoxycarbonylalkyl groups, one to three C₃-C₆haloalkoxycarbonylalkyl groups, one to three C₃-C₆alkylcarbonylalkyl groups, one to three C₃-C₆haloalkylcarbonylalkyl groups one or two NR₁₅S(O)ₚR₁₆ groups or one or two C(W)NR₁₅S(O)ₚR₁₆ groups,
a 4- to 10-membered heterocyclic ring optionally substituted with any combination of one or two oxo groups, one or two thioxo groups, one to three halogen atoms, one to three nitro groups, one to three cyano groups, one to three C₁-C₄alkyl groups, one to three C₁-C₄haloalkyl groups, one to three C₁-C₄alkoxy groups, one to three C₁-C₄haloalkoxy groups, one or two C₂-C₆alkoxyalkyl groups, one or two C₂-C₆haloalkoxyalkyl groups, one or two C(O)OR₁₄ groups, one or two C(O)R₁₄ groups, one to three C₃-C₆alkoxycarbonylalkyl groups, one to three C₃-C₆haloalkoxycarbonylalkyl groups, one to three C₃-C₆alkylcarbonylalkyl groups, one to three C₃-C₆haloalkylcarbonylalkyl groups, one or two NR₁₅S(O)ₚR₁₆ groups or one or two C(W)NR₁₅S(O)ₚR₁₆ groups,
phenyl optionally substituted with any combination of one to three halogen atoms, one to three nitro groups, one to three cyano groups, one to three C₁-C₄alkyl groups, one to three C₁-C₄haloalkyl groups, one to three C₁-C₄alkoxy groups, one to three C₁-C₄haloalkoxy groups, one or two C₂-C₆alkoxyalkyl groups, one or two C₂-C₆haloalkoxyalkyl groups, one or two C(O)OR₁₄ groups, one or two C(O)R₁₄ groups, one to three C₃-C₆alkoxycarbonylalkyl groups, one to three C₃-C₆haloalkoxycarbonylalkyl groups, one to three C₃-C₆alkylcarbonylalkyl groups, one to three C₃-C₆haloalkylcarbonylalkyl groups, one or two NR₁₅S(O)ₚR₁₆ groups or one or two C(W)NR₁₅S(O)ₚR₁₆ groups,
OR₁₇,
NR₁₈R₁₉,
NR₂₀S(O)_{q}R₂₁,
NR₂₂C(V)R₂₃,
NR₂₄P(V) (OR₂₅)₂,
C(V)NR₂₀S(O)_{q}R₂₁,
cyano,
S(O)_{q}R₂₆,
P(V)(OR₂₇)₂,
C(V)R₂₈ or
C(V)OR₂₉;
- W is: O, S, NR₃₀, NOR₃₁ or NNR₃₂R₃₃;
- V and Z: are each independently O or S;
- R₁ and R₁₄: are each independently C₁-C₄alkyl or C₁-C₄haloalkyl;
- R₂ is: hydrogen, C₁-C₆alkyl, C₃-C₇cycloalkyl, C₁-C₆haloalkyl, C₃-C₆alkenyl, C₃-C₆alkynyl, C₂-C₆alkoxyalkyl, C₂-C₆haloalkoxyalkyl, C₃-C₆alkoxycarbonylalkyl,
phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one or two C₂-C₆₋alkoxyalkyl groups, one or two C₂-C₆haloalkoxyalkyl groups, one or two nitro groups, one or two cyano groups, one NR₁₅S(O)ₚR₁₆ group, one C₂-C₆alkoxycarbonyl group, one C₂-C₆haloalkoxycarbonyl group, one C₂-C₆alkylcarbonyl group, one C₂-C₆haloalkylcarbonyl group, one C₃-C₆₋alkoxycarbonylalkyl group, one C₃-C₆haloalkoxycarbonylalkyl group, one C₃-C₆alkylcarbonylalkyl group or one C₃-C₆haloalkylcarbonylalkyl group,
a 4- to 10-membered heterocyclic ring optionally substituted with any combination of one or two oxo groups, one or two thioxo groups, one to three halogen atoms, one to three C₁-C₄alkyl groups, one to three C₁-C₄haloalkyl groups, one to three C₁-C₄alkoxy groups, one to three C₁-C₄haloalkoxy groups, one or two C₂-C₆alkoxyalkyl groups, one or two C₂-C₆haloalkoxyalkyl groups, one or two nitro groups, one or two cyano groups, one NR₁₅S(O)ₚR₁₆ group, one C₂-C₆alkoxycarbonyl group, one C₂-C₆haloalkoxycarbonyl group, one C₂-C₆alkylcarbonyl group, one C₂-C₆haloalkylcarbonyl group, one C₃-C₆alkoxycarbonylalkyl group, one C₃-C₆haloalkoxycarbonylalkyl group, one C₃-C₆alkylcarbonylalkyl group or one C₃-C₆haloalkylcarbonylalkyl group,
C₁-C₄alkyl substituted with one phenyl group wherein the phenyl group is optionally substituted with any combination of one to three halogen atoms, one or two nitro groups, one or two cyano groups, one to three C₁-C₄alkyl groups, one to three C₁-C₄haloalkyl groups, one to three C₁-C₄alkoxy groups or one to three C₁-C₄haloalkoxy groups, or
C₁-C₄alkyl substituted with one 4- to 10-membered heterocyclic ring wherein the heterocyclic ring is optionally substituted with any combination of one to three halogen atoms, one or two nitro groups, one or two cyano groups, one to three C₁-C₄alkyl groups, one to three C₁-C₄haloalkyl groups, one to three C₁-C₄alkoxy groups or one to three C₁-C₄haloalkoxy groups;
- R₃ is: hydrogen, C₁-C₆alkyl, C₁-C₆haloalkyl, C₃-C₇cycloalkyl, C₃-C₆alkenyl C₃-C₆alkynyl, C₂-C₆alkoxyalkyl, C₂-C₆haloalkoxyalkyl, C₃-C₆alkoxycarbonylalkyl,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups,
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups,
furfural optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, or
an alkali metal, alkaline earth metal, manganese, copper, zinc, cobalt, silver, nickel, ammonium or organic ammonium cation;
- R₄ and R₅: are each independently hydrogen, C₁-C₆alkyl, C₁-C₆haloalkyl, C₃-C₇cycloalkyl, C₁-C₆alkoxy, hydroxy, C₃-C₆alkenyl, C₃-C₆haloalkenyl, C₃-C₆alkynyl, C₃-C₆₋ haloalkynyl, C₃-C₆alkylcarbonylalkyl, C₃-C₆haloalkylcarbonylalkyl, C₃-C₆alkoxycarbonylalkyl, C₃-C₆₋haloalkoxycarbonylalkyl, C₂-C₆hydroxycarbonylalkyl, C₂-C₆alkylsulfonylalkyl, C₂-C₆haloalkylsulfonylalkyl, S(O)ₚR₁₆,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups,
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, or
furfural optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups,
provided that only one of R₄ and R₅ can be hydroxy or C₁-C₆alkoxy, and
when R₄ and R₅ are taken together with the atom to which they are attached, they represent a three- to seven-membered saturated or unsaturated ring optionally interrupted by oxygen, sulfur or nitrogen, and optionally substituted with one to three methyl groups or one or more halogen atoms;
- R₆, R₂₅ and R₂₇: are each independently hydrogen,
C₁-C₆alkyl, C₁-C₆haloalkyl,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, or
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups;
- R₇, R₈ and R₁₇: are each independently hydrogen, C₁-C₆alkyl, C₁-C₆haloalkyl, C₃-C₇cycloalkyl, C₃-C₆alkenyl, C₃-C₆haloalkenyl, C₃-C₆alkynyl, C₃-C₆haloalkynyl, C₂-C₆alkoxyalkyl, C₂-C₆haloalkoxyalkyl, C₂-C₆alkylcarbonylalkyl, C₂-C₆haloalkylcarbonylalkyl, C₂-C₆₋alkoxycarbonylalkyl, C₂-C₆haloalkoxycarbonylalkyl, C₂-C₆alkylsulfonylalkyl, C₂-C₆haloalkylsulfonylalkyl, C₂-C₆hydroxycarbonylalkyl, C(V)NR₁₂S(O)ₙR₁₃,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups,
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, or
furfural optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups;
- R₉, R₁₀, R₁₈, R₁₉, R₃₂ and R₃₃: are each independently hydrogen, C₁-C₆alkyl, C₁-C₆haloalkyl, C₃-C₇cycloalkyl, C₂-C₆alkenyl, C₂-C₆haloalkenyl, C₂-C₆alkynyl, C₃-C₆haloalkynyl, C₃-C₆alkylcarbonylalkyl, C₃-C₆haloalkylcarbonylalkyl, C₃-C₆alkoxycarbonylalkyl, C₃-C₆haloalkoxycarbonylalkyl, C₂-C₆hydroxycarbonylalkyl, C₂-C₆alkylsulfonylalkyl, C₂-C₆haloalkylsulfonylalkyl,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups,
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄₋haloalkoxy groups, or
furfural optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, and
when R₉ and R₁₀ or R₁₈ and R₁₉ or R₃₂ and R₃₃ are taken together with the atom to which they are attached, they represent a three- to seven-membered saturated or unsaturated ring optionally interrupted by oxygen, sulfur or nitrogen, and optionally substituted with one to three methyl groups or one or more halogen atoms;
- R₁₁ is: hydrogen, C₁-C₆alkyl, C₁-C₆haloalkyl, C₃-C₇cycloalkyl, C₃-C₆alkenyl, C₃-C₆haloalkenyl, C₃-C₆alkynyl, C₃-C₆haloalkynyl, C₂-C₆alkoxyalkyl, C₂-C₆haloalkoxyalkyl, C₃-C₆alkylcarbonylalkyl, C₃-C₆haloalkylcarbonylalkyl, C₃-C₆alkoxycarbonylalkyl, C₃-C₆haloalkoxycarbonylalkyl, C₂-C₆hydroxycarbonylalkyl,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups,
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, or
furfural optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups;
- R₁₂, R₁₅, R₂₀ and R₂₄: are each independently hydrogen, C₁-C₆alkyl, C₁-C₆haloalkyl, C₃-C₆alkenyl or C₃-C₆alkynyl;
- R₁₃, R₂₁, R₂₂, R₂₃, R₂₆, R₂₈ and R₂₉: are each independently hydrogen, C₁-C₆alkyl, C₁-C₆haloalkyl, C₃-C₇cycloalkyl, C₃-C₆alkenyl, C₃-C₆haloalkenyl, C₃-C₆alkynyl, C₃-C₆haloalkynyl, C₃-C₆alkylcarbonylalkyl, C₃-C₆haloalkylcarbonylalkyl, C₃-C₆alkoxycarbonylalkyl, C₃-C₆haloalkoxycarbonylalkyl,
phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one or two C₂-C₆alkoxyalkyl groups, one or two C₂-C₆haloalkoxyalkyl groups, one or two nitro groups, one or two cyano groups, one C₃-C₆alkoxycarbonylalkyl group, one C₃-C₆haloalkoxycarbonylalkyl group, one C₃-C₆alkylcarbonylalkyl group, one C₃-C₆haloalkylcarbonylalkyl group or one C₂-C₆hydroxycarbonylalkyl group,
a 4- to 10-membered heterocyclic ring optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one or two C₂-C₆₋alkoxyalkyl groups, one or two C₂-C₆haloalkoxyalkyl groups, one or two nitro groups, one or two cyano groups, one C₃-C₆alkoxycarbonylalkyl group, one C₃-C₆haloalkoxycarbonylalkyl group, one C₃-C₆alkylcarbonylalkyl group, one C₃-C₆haloalkylcarbonylalkyl group or one C₂-C₆hydroxycarbonylalkyl group,
benzyl optionally substituted on the phenyl ring with any combination of one to three halogen atoms, one to three C₁-C₄alkyl groups, one to three C₁-C₄haloalkyl groups, one to three C₁-C₄alkoxy groups or one to three C₁-C₄haloalkoxy groups, or
furfural optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups;
- R₁₆ is: hydrogen, C₁-C₆alkyl, C₁-C₆haloalkyl, C₃-C₇cycloalkyl, C₃-C₆alkenyl, C₃-C₆haloalkenyl, C₃-C₆alkynyl, C₃-C₆haloalkynyl, C₃-C₆alkylcarbonylalkyl, C₃-C₆haloalkylcarbonylalkyl, C₃-C₆alkoxycarbonylalkyl, C₃-C₆haloalkoxycarbonylalkyl,
phenyl optionally substituted on the phenyl ring with any combination of one to three halogen atoms, one to three C₁-C₄alkyl groups, one to three C₁-C₄haloalkyl groups, one to three C₁-C₄alkoxy groups or one to three C₁-C₄haloalkoxy groups,
benzyl optionally substituted on the phenyl ring with any combination of one to three halogen atoms, one to three C₁-C₄alkyl groups, one to three C₁-C₄haloalkyl groups, one to three C₁-C₄alkoxy groups or one to three C₁-C₄haloalkoxy groups, or
furfural optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups;
- R₃₀ is: hydrogen, C₁-C₆alkyl, C₁-C₆haloalkyl, C₃-C₇cycloalkyl, C₃-C₆alkenyl, C₃-C₆alkynyl, C₂-C₆alkoxyalkyl, C₃-C₆alkoxycarbonylalkyl,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups, one to three C₁-C₄haloalkoxy groups, one C₂-C₆alkoxyalkyl group, one C₂-C₆haloalkoxyalkyl group, one NR₁₅S(O)ₙR₁₆ group, one C₂-C₆alkoxycarbonyl group, one C₂-C₆alkylcarbonyl group, one C₃-C₆alkoxycarbonylalkyl group, one C₃-C₆alkylcarbonylalkyl group, one C₃-C₆haloalkoxycarbonylalkyl group or one C₃-C₆haloalkylcarbonylalkyl group,
a 4- to 10-membered heterocyclic ring optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups, one to three C₁-C₄haloalkoxy groups, one C₂-C₆alkoxyalkyl group, one C₂-C₆haloalkoxyalkyl group, one NR₁₅S(O)ₙR₁₆ group, one C₂-C₆alkoxycarbonyl group, one C₂-C₆alkylcarbonyl group, one C₃-C₆alkoxycarbonylalkyl group, one C₃-C₆alkylcarbonylalkyl group, one C₃-C₆haloalkoxycarbonylalkyl group or one C₃-C₆haloalkylcarbonylalkyl group,
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄₋haloalkoxy groups, or
furfural optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups;
- R₃₁ is: hydrogen, C₁-C₆alkyl, C₁-C₆haloalkyl, C₃-C₇cycloalkyl, C₃-C₆alkenyl, C₃-C₆haloalkenyl, C₃-C₆alkynyl, C₃-C₆haloalkynyl, C₂-C₆alkoxyalkyl, C₂-C₆haloalkoxyalkyl, C₃-C₆alkylcarbonylalkyl, C₃-C₆haloalkylcarbonylalkyl, C₃-C₆alkoxycarbonylalkyl, C₃-C₆haloalkoxycarbonylalkyl, C₂-C₆alkylsulfonylalkyl, C₂-C₆haloalkylsulfonylalkyl, C₂-C₆hydroxycarbonylalkyl, C(V)NR₁₂S(O)ₙR₁₃,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups,
a 4- to 10-membered heterocyclic ring optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups,
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, or
furfural optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups;
- A is: -O-, -S(O)ᵣ-, -NR₃₄- or -CR₃₅R₃₆-;
- B is: -CR₃₇R₃₈(CR₃₉R₄₀)s-, -C(=T)- or -C(=CR₄₁R₄₂)-;
- R₃₄, R₃₅, R₃₆, R₃₇, R₃₈, R₃₉ and R₄₀: are each independently hydrogen, halogen,
C₁-C₆alkyl optionally substituted with one to three halogen atoms, one or two cyano groups, one or two nitro groups, one or two C(W)R₂ groups, one or two C(W)OR₃ groups, one or two C(W)NR₄R₅ groups, one or two P(V) (OR₆)₂ groups, one or two OR₇ groups, one or two SR₈ groups, one or two NR₉R₁₀ groups or one or two OC(W)R₁₁ groups,
C₃-C₇cycloalkyl,
C₃-C₆alkenyl,
C₃-C₆haloalkenyl
C₃-C₆alkynyl,
C₃-C₆haloalkynyl,
C(V)NR₁₂S(O)ₙR₁₃,
phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one or two C₂-C₆alkoxyalkyl groups, one or two C₂-C₆haloalkoxyalkyl groups, one or two nitro groups, one or two cyano groups, one NR₁₅S(O)ₚR₁₆ group, one C₂-C₆alkoxycarbonyl group, one C₂-C₆haloalkoxycarbonyl group, one C₂-C₆alkylcarbonyl group, one C₂-C₆haloalkylcarbonyl group, one C₃-C₆alkoxycarbonylalkyl group, one C₃-C₆haloalkoxycarbonylalkyl group, one C₃-C₆alkylcarbonylalkyl group or one C₃-C₆haloalkylcarbonylalkyl group,
a 4- to 10-membered heterocyclic ring optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one or two C₂-C₆alkoxyalkyl groups, one or two C₂-C₆haloalkoxyalkyl groups, one or two nitro groups, one or two cyano groups, one NR₁₅S(O)ₚR₁₆ group, one C₂-C₆alkoxycarbonyl group, one C₂-C₆haloalkoxycarbonyl group, one C₂-C₆alkylcarbonyl group, one C₂-C₆haloalkylcarbonyl group, one C₃-C₆alkoxycarbonylalkyl group, one C₃-C₆haloalkoxycarbonylalkyl group, one C₃-C₆alkylcarbonylalkyl group or one C₃-C₆haloalkylcarbonylalkyl group,
C₁-C₄alkyl substituted with one phenyl group wherein the phenyl group is optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one or two C₂-C₆alkoxyalkyl groups, one or two C₂-C₆haloalkoxyalkyl groups, one C₂-C₆alkoxycarbonyl group, one C₂-C₆haloalkoxycarbonyl group, one C₂-C₆alkylcarbonyl group, one C₂-C₆haloalkylcarbonyl group, one C₃-C₆alkoxycarbonylalkyl group, one C₃-C₆haloalkoxycarbonylalkyl group, one C₃-C₆alkylcarbonylalkyl group, one C₃-C₆haloalkylcarbonylalkyl group, one C₃-C₈alkoxycarbonylalkoxy group or one C₂-C₈hydroxycarbonylalkoxy group, or
C₁-C₄alkyl substituted with one 4- to 10-membered heterocyclic ring wherein the heterocyclic ring is optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one or two C₂-C₆alkoxyalkyl groups, one or two C₂-C₆haloalkoxyalkyl groups, one C₂-C₆alkoxycarbonyl group, one C₂-C₆haloalkoxycarbonyl group, one C₂-C₆alkylcarbonyl group, one C₂-C₆haloalkylcarbonyl group, one C₃-C₆alkoxycarbonylalkyl group, one C₃-C₆haloalkoxycarbonylalkyl group, one C₃-C₆alkylcarbonylalkyl group or one C₃-C₆haloalkylcarbonylalkyl group, and
when R₃₅ and R₃₆ or R₃₇ and R₃₈ or R₃₉ and R₄₀ are taken together with the atom to which they are attached, they represent a ring in which R₃₅R₃₆ or R₃₇R₃₈ or R₃₉R₄₀ is a C₂-C₆alkylene group;
- T is: O, S, NR₃₀, NOR₃₁ or NNR₃₂R₃₃;
- R₄₁ and R₄₂: are each independently hydrogen, C₁-C₆alkyl, C₁-C₆haloalkyl, C₃-C₇cycloalkyl, C₃-C₆alkenyl, C₃-C₆haloalkenyl, C₃-C₆alkynyl, C₃-C₆haloalkynyl, C₂-C₆alkylcarbonyl, C₂-C₆haloalkylcarbonyl, C₂-C₆alkoxycarbonyl, C₂-C₆haloalkoxycarbonyl, hydroxycarbonyl, C₃-C₆alkylcarbonylalkyl, C₃-C₆haloalkylcarbonylalkyl, C₃-C₆alkoxycarbonylalkyl, C₃-C₆haloalkoxycarbonylalkyl, C₂-C₆hydroxycarbonylalkyl, C₂-C₆alkylsulfonylalkyl, C₂-C₆haloalkylsulfonylalkyl,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups, one to three C₁-C₄haloalkoxy groups or one C₃-C₈alkoxycarbonylalkoxy group,
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, or
furfural optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, and
when R₄₁ and R₄₂ are taken together with the atom to which they are attached, they represent a three- to seven-membered saturated or unsaturated ring optionally interrupted with one group selected from O, S(O)ᵣ or NR₃₁, and optionally substituted with one to three methyl groups or one or more halogen atoms;
m and r are each independently an integer of O, 1 or 2; n, p and q are each independently an integer of 1 or 2; s is an integer of 0 or 1;
Q is selected from 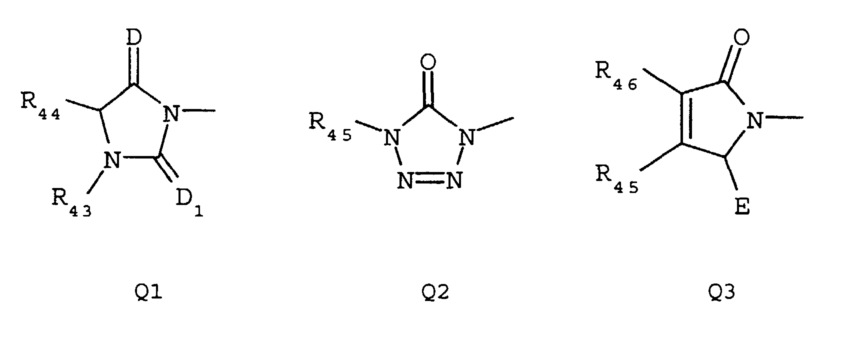 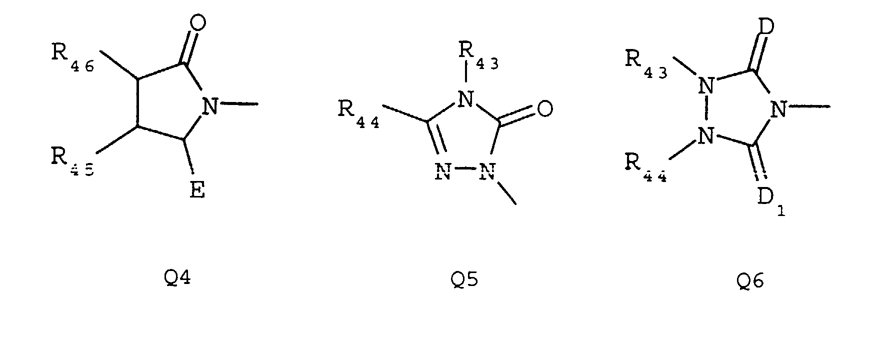 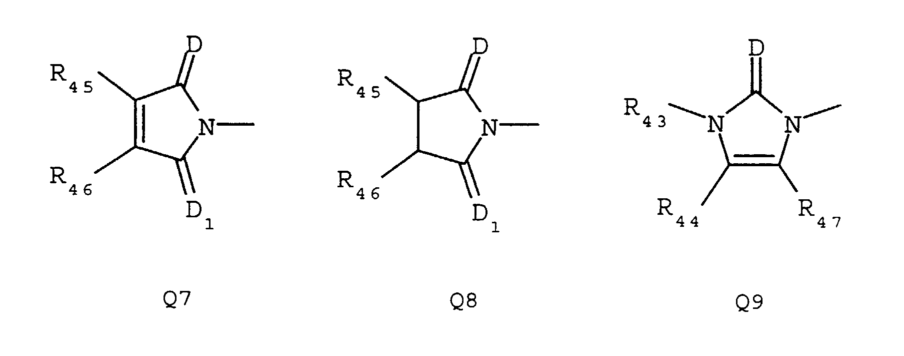 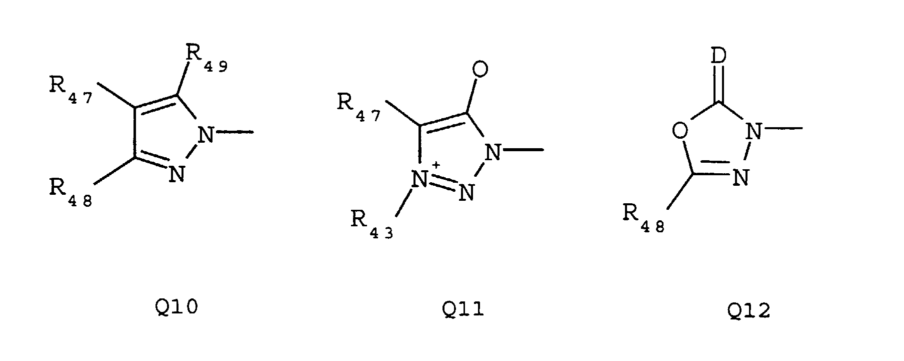 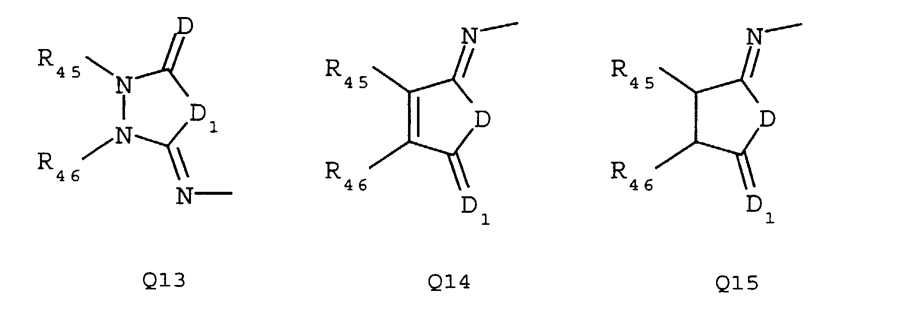 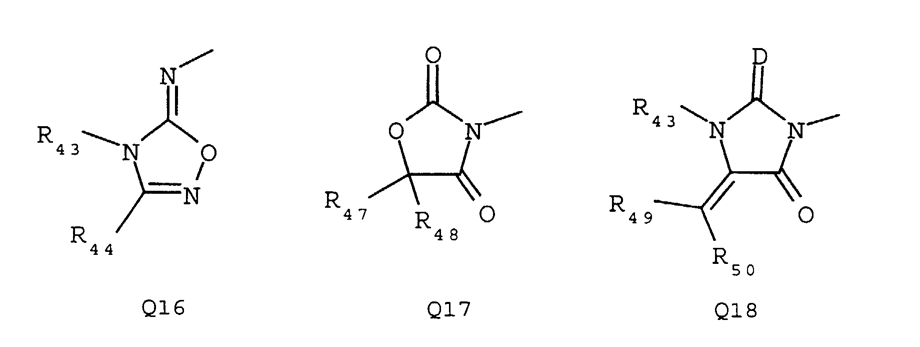 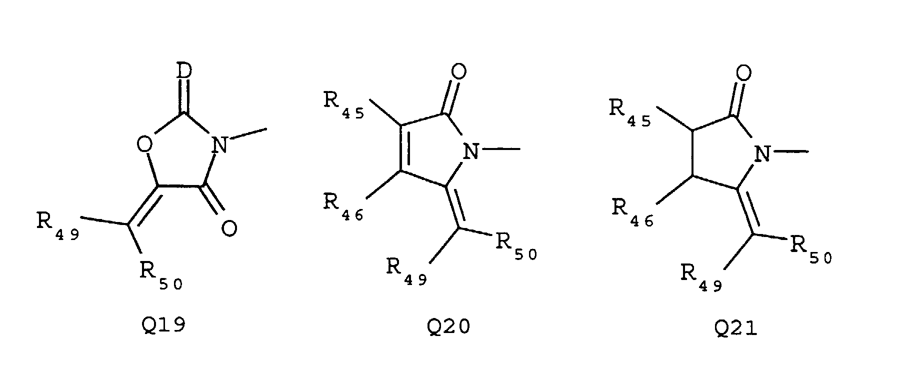 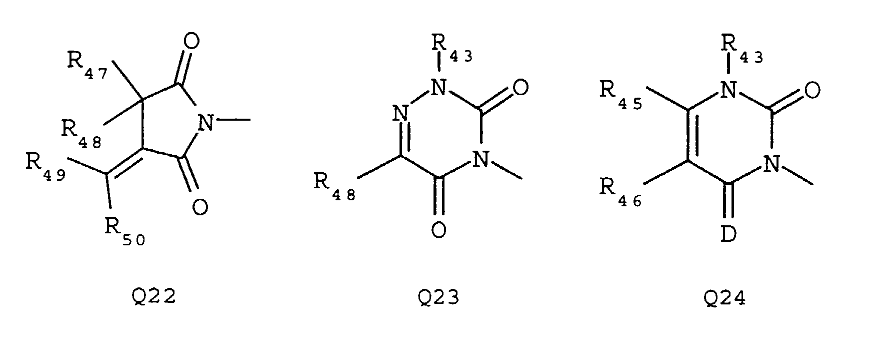 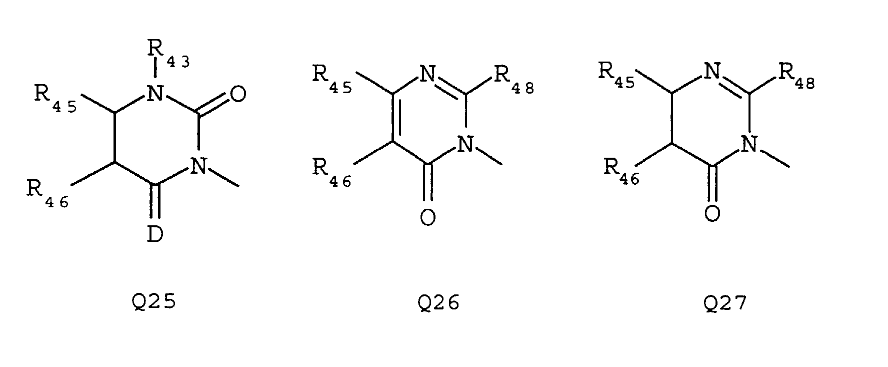 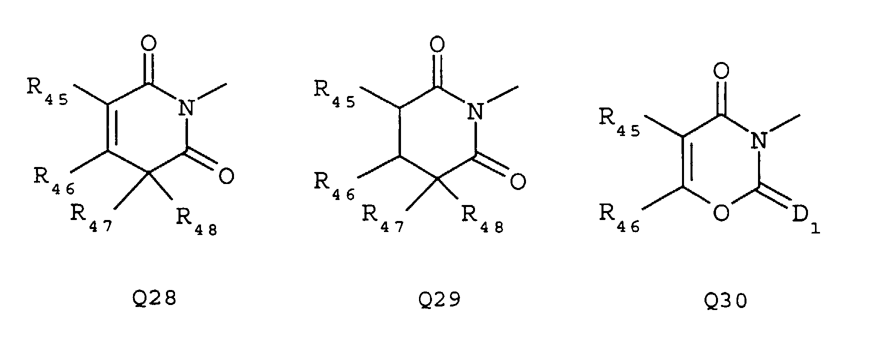 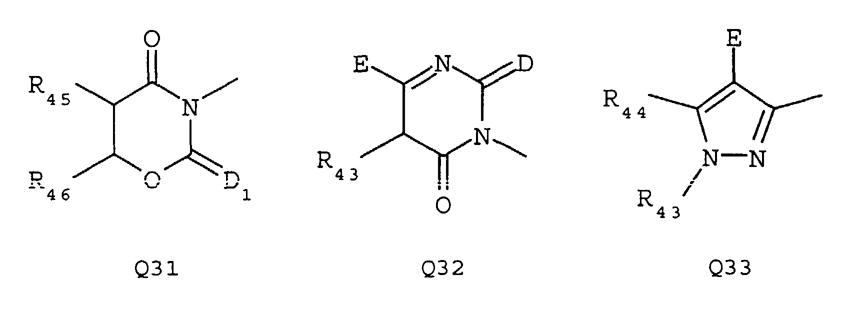 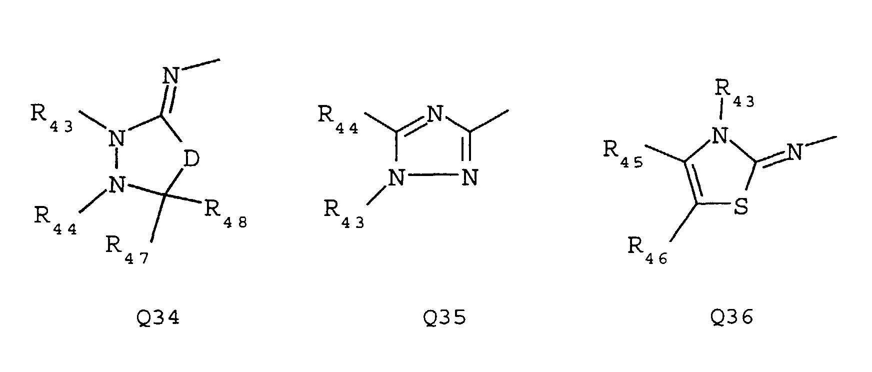 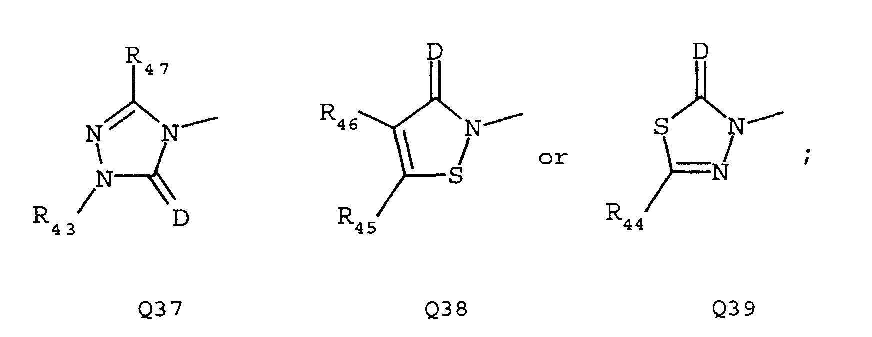
- D and D₁: are each independently O or S;
- E is: hydroxy, halogen, C₁-C₄alkoxy or C₁-C₄alkylthio;
- R₄₃ and R₄₄: are each independently hydrogen, halogen, C₁-C₆alkyl, C₁-C₆haloalkyl, C₃-C₇cycloalkyl, C₃-C₆alkenyl, C₃-C₆haloalkenyl, C₃-C₆alkynyl, cyano, amino, hydroxy or benzyl, and when R₄₃ and R₄₄ are taken together with the atom to which they are attached, they represent a three- to seven-membered saturated or unsaturated rina optionally interrupted by oxygen, sulfur or nitrogen, and optionally substituted with one to three methyl groups or one or more halogen atoms;
- R₄₅ and R₄₆: are each independently hydrogen, halogen, C₁-C₆alkyl, C₁-C₆haloalkyl, C₃-C₇cycloalkyl, C₃-C₆alkenyl, C₃-C₆haloalkenyl, C₃-C₆alkynyl, OR₅₁, S(O)ₜR₅₂ or NR₅₃R₅₄, and when R₄₅ and R₄₆ are taken together with the atom to which they are attached, they represent a three- to seven-membered saturated or unsaturated ring optionally interrupted by oxygen, sulfur or nitrogen, and optionally substituted with one to three methyl groups or one or more halogen atoms;
- R₄₇, R₄₉ and R₅₀: are each independently hydrogen, halogen or C₁-C₆alkyl;
- R₄₈ is: hydrogen, halogen, C₁-C₆alkyl, C₁-C₆haloalkyl, C₃-C₇cycloalkyl, C₃-C₆alkenyl, C₃-C₆haloalkenyl, C₃-C₆alkynyl, OR₅₁ or SR₅₂;
- R₅₁ and R₅₂: are each independently hydrogen, C₁-C₄alkyl, C₁-C₄haloalkyl, C₃-C₇cycloalkyl, C₁-C4cyanoalkyl, C₃-C₆alkenyl, C₃-C₆haloalkenyl, C₃-C₆alkynyl, benzyl or
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups;
- R₅₃ is: hydrogen, C₁-C₄alkyl, benzyl or
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups;
- R₅₄: in hydrogen, C₁-C₄alkyl, C₃-C₆alkenyl, C₃-C₇cycloalkyl, benzyl, S(O)ₜR₅₂ or
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups; and
t is an integer of O, 1 or 2; and the agriculturally acceptable salts thereof.

This invention also relates to compositions containing those compounds and methods for using those compounds and compositions. Advantageously, it has been found that the compounds of the present invention, and compositions containing them, are useful for the control of undesirable plant species

### DETAILED DESCRIPTION OF THE INVENTION

The present invention provides a method for the control of undesirable plant species which comprises applying to the foliage of said plants or to the soil or water containing seeds or other propagating organs thereof, a herbicidally effective amount of a formula I, 1-(3-heterocyclylphenyl)isothiourea, -isourea, -guanidine or -amidine compound.

The 1-(3-heterocyclylphenyl)isothiourea, -isourea, -guanidine and -amidine compounds of the present invention have the structural formula I 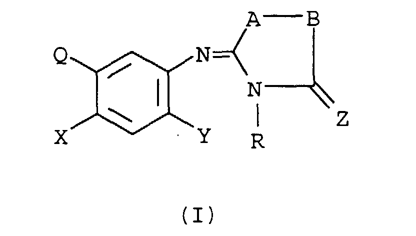 wherein A, B, Q, R, X, Y and Z are as defined above for formula I.

Preferred formula I compounds of this invention are those wherein
- X is: hydrogen or halogen;
- Y is: hydrogen, halogen, nitro or cyano;
- R is: hydrogen,
a C₁-C₈alkyl, C₃-C₇cycloalkyl, C₃-C₈alkenyl,
   C₅-C₇cycloalkenyl or C₃-C₈alkynyl group, wherein each group is optionally substituted with any combination of one to six halogen atoms, one or two cyano groups, one or two nitro groups, one or two C(O)R₂ groups, one or two C(O)OR₃ groups, one or two C(O)NR₄R₅ groups, one or two P(O) (OR₆)₂ groups, one or two OR₇ groups, one or two SR₈ groups, one or two NR₉R₁₀ groups, one or two OC(O)R₁₁ groups, one or two NR₁₂S(O)ₙR₁₃ groups, one or two C(O)NR₁₂S(O)ₙR₁₃ groups,
   one 4- to 10-membered heterocyclic ring optionally substituted with any combination of one or two oxo groups, one or two thioxo groups, one to three halogen atoms, one to three nitro groups, one to three cyano groups, one to three C₁-C₄alkyl groups, one to three C₁-C₄haloalkyl groups, one to three C₁-C₄alkoxy groups, one to three C₁-C₄haloalkoxy groups, one or two C₂-C₆alkoxyalkyl groups, one or two C₂-C₆-haloalkoxyalkyl groups, one or two C(O)OR₁₄ groups, one or two C(O)R₁₄ groups, one to three C₃-C₆alkoxycarbonylalkyl groups, one to three C₃-C₆haloalkoxycarbonylalkyl groups, one to three C₃-C₆alkylcarbonyalkyl groups, one to three C₃-C₆haloalkylcarbonylalkyl groups, one or two NR₁₅S(O)ₚR₁₆ groups or one or two C(O)NR₁₅S(O)ₚR₁₆ groups, or
   one phenyl group optionally substituted with any combination of one to three halogen atoms, one to three nitro groups, one to three cyano groups, one to three C₁-C₄alkyl groups, one to three C₁-C₄haloalkyl groups, one to three C₁-C₄alkoxy groups, one to three C₁-C₄haloalkoxy groups, one or two C₂-C₆alkoxyalkyl groups, one or two C₂-C₆haloalkoxyalkyl groups, one or two C(O)OR₁₄ groups, one or two C(O)R₁₄ groups, one to three C₃-C₆alkoxycarbonylalkyl groups, one to three C₃-C₆haloalkoxycarbonylalkyl groups, one to three C₃-C₆₋alkylcarbonylalkyl groups, one to three C₃-C₆haloalkylcarbonylalkyl groups, one or two NR₁₅S(O)ₚR₁₆ groups or one or two C(O)NR₁₅S(O)ₚR₁₆ groups,
phenyl optionally substituted with any combination of one to three halogen atoms, one to three nitro groups, one to three cyano groups, one to three C₁-C₄alkyl groups, one to three C₁-C₄-haloalkyl groups, one to three C₁-C₄alkoxy groups, one to three C₁-C₄haloalkoxy groups, one or two C₂-C₆alkoxyalkyl groups, one or two C₂-C₆haloalkoxyalkyl groups, one or two C(O)OR₁₄ groups, one or two C(O)R₁₄ groups, one to three C₃-C₆alkoxycarbonylalkyl groups, one to three C₃-C₆haloalkoxycarbonylalkyl groups, one to three C₃-C₆alkylcarbonylalkyl groups, one to three C₃-C₆haloalkylcarbonylalkyl groups or one or two NR₁₅S(O)ₚR₁₆ groups,
OR₁₇,
NR₁₈R₁₉,
NR₂₀S(O)_{q}R₂₁,
NR₂₂C(O)R₂₃,
C(O)NR₂₀S(O)_{q}R₂₁,
S(O)_{q}R₂₆,
C(O)R₂₈ or
C(O)OR₂₉;
- Z is: O;
- R₁₄ is: C₁-C₄alkyl or C₁-C₄haloalkyl;
- R₂ is: hydrogen, C₁-C₆alkyl, C₃-C₇cycloalkyl, C₁-C₆-haloalkyl, C₂-C₆alkoxyalkyl, C₃-C₆alkoxycarbonylalkyl, or
phenyl optionally substituted with any combination of one to three halogen atoms, one to three C₁-C₄alkyl groups, one to three C₁-C₄alkoxy groups, one or two C₂-C₆alkoxyalkyl groups, one C₂-C₆alkoxycarbonyl group, one C₃-C₆alkoxycarbonylalkyl group or one C₃-C₆haloalkoxycarbonylalkyl group;
- R₃ is: hydrogen, C₁-C₆alkyl, C₁-C₆haloalkyl, C₃-C₇cycloalkyl, C₃-C₆alkenyl, C₃-C₆alkynyl, C₂-C₆alkoxyalkyl, C₂-C₆haloalkoxyalkyl, C₃-C₆alkoxycarbonylalkyl,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups,
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups,
furfural optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, or
an alkali metal, alkaline earth metal, manganese, copper, zinc, cobalt, silver, nickel, ammonium or organic ammonium cation;
- R₄ and R₅: are each independently hydrogen, C₁-C₆alkyl, C₃-C₇cycloalkyl, C₁-C₆alkoxy, hydroxy, C₃-C₆alkenyl, C₃-C₆alkylcarbonylalkyl, C₃-C₆alkoxycarbonylalkyl, C₂-C₆alkylsulfonylalkyl, C₂-C₆haloalkylsulfonylalkyl, S(O)ₚR₁₆,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, or
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups,
provided that only one of R₄ and R₅ can be hydroxy or C₁-C₆alkoxy, and
when R₄ and R₅ are taken together with the atom to which they are attached, they represent a three- to seven-membered saturated or unsaturated ring optionally interrupted by oxygen, sulfur or nitrogen, and optionally substituted with one to three methyl groups or one or more halogen atoms;
- R₆ is: hydrogen, C₁-C₆alkyl, C₁-C₆haloalkyl,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, or
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups;
- R₇ and R₈: are each independently hydrogen, C₁-C₆-alkyl, C₂-C₆alkoxycarbonylalkyl or C₂-C₆hydroxycarbonylalkyl;
- R₁₇ is: hydrogen, C₁-C₆alkyl, C₃-C₇cycloalkyl, C₃-C₆alkenyl, C₃-C₆alkynyl, C₂-C₆alkoxyalkyl, C₂-C₆haloalkoxyalkyl, C₃-C₆alkylcarbonylalkyl, C₂-C₆haloalkylcarbonylalkyl, C₃-C₆alkoxycarbonylalkyl, C₂-C₆hydroxycarbonylalkyl,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, or
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups;
- R₉ and R₁₀: are each independently hydrogen, C₁-C₆alkyl, C₃-C₆alkylcarbonylalkyl, C₃-C₆alkoxycarbonylalkyl, C₂-C₆hydroxycarbonylalkyl, or
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, and
when R₉ and R₁₀ are taken together with the atom to which they are attached, they represent a three- to seven-membered saturated or unsaturated ring optionally interrupted by oxygen, sulfur or nitrogen, and optionally substituted with one to three methyl groups or one or more halogen atoms;
- R₁₈ and R₁₉: are each independently hydrogen, C₁-C₆alkyl, C₃-C₆alkoxycarbonylalkyl, C₃-C₆haloalkoxycarbonylalkyl, C₂-C₆hydroxycarbonylalkyl, C₂-C₆alkylsulfonylalkyl, C₂-C₆haloalkylsulfonylalkyl,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups,
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, or
furfural optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, and
when R₁₈ and R₁₉ are taken together with the atom to which they are attached, they represent a three- to seven-membered saturated or unsaturated ring optionally interrupted by oxygen, sulfur or nitrogen, and optionally substituted with one to three methyl groups or one or more halogen atoms;
- R₁₁ is: hydrogen, C₁-C₆alkyl or C₁-C₆haloalkyl;
- R₁₂, R₁₅ and R₂₀: are each independently hydrogen or C₁-C₆alkyl;
- R₁₃, R_{16,} R₂₁ and R₂₆: are each independently C₁-C₆alkyl, C₁-C₆haloalkyl, C₃-C₇cycloalkyl, C₃-C₆alkenyl, C₃-C₆haloalkenyl, C₃-C₆alkynyl, C₃-C₆haloalkynyl, or
benzyl optionally substituted on the phenyl ring with any combination of one to three halogen atoms, one to three C₁-C₄alkyl groups, one to three C₁-C₄haloalkyl groups, one to three C₁-C₄alkoxy groups or one to three C₁-C₄haloalkoxy groups;
- R₂₂ is: hydrogen or C₁-C₆alkyl;
- R₂₃ is: hydrogen, C₁-C₆alkyl, C₁-C₆haloalkyl, C₃-C₇cycloalkyl, C₃-C₆alkylcarbonylalkyl, C₃-C₆haloalkylcarbonylalkyl, C₃-C₆alkoxycarbonylalkyl, C₃-C₆haloalkoxycarbonylalkyl,
phenyl optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups, or
benzyl optionally substituted on the phenyl ring with any combination of one to three halogen atoms, one to three C₁-C₄alkyl groups, one to three C₁-C₄haloalkyl groups, one to three C₁-C₄alkoxy groups or one to three C₁-C₄₋haloalkoxy groups;
- R₂₈ is: hydrogen, C₁-C₆alkyl, C₁-C₆haloalkyl, C₃-C₇-cycloalkyl, C₃-C₆alkenyl, C₃-C₆alkynyl, C₃-C₆alkoxycarbonylalkyl, or
phenyl optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups;
- R₂₉ is: hydrogen, C₁-C₆alkyl or benzyl;
- A is: -O-, -S(O)ᵣ-, or -NR₃₄-;
- B is: -CR₃₇R₃₈-, -C(O)- or -C(=CR₄₁R₄₂)-;
- R₃₄ is: hydrogen,
C₁-C₆alkyl optionally substituted with one to three halogen atoms, one or two cyano groups, one or two nitro groups, one or two C(O)R₂ groups, one or two C(O)OR₃ groups, one or two C(O)NR₄R₅ groups, one or two P(O) (OR₆)₂ groups, one or two OR₇ groups, one or two SR₈ groups, one or two NR₉R₁₀ groups or one or two OC(O)R₁₁ groups,
C₃-C₇cycloalkyl,
C₃-C₆alkenyl,
C₃-C₆alkynyl,
C(O)NR₁₂S(O)ₙR_{13,}
phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one or two C₂-C₆₋ alkoxyalkyl groups, one or two nitro groups, one or two cyano groups, one C₂-C₆alkoxycarbonyl group, one C₂-C₆alkylcarbonyl group, one C₃-C₆₋ alkoxycarbonylalkyl group or one C₃-C₆alkylcarbonylalkyl group, or
C₁-C₄alkyl substituted with one phenyl group wherein the phenyl group is optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one or two C₂-C₆₋alkoxyalkyl groups, groups, one C₂-C₆alkoxycarbonyl group, one C₂-C₆alkylcarbonyl group, one C₃-C₆alkoxycarbonylalkyl group or one C₃-C₆-alkylcarbonylalkyl group;
- R₃₇ and R₃₈: are each independently hydrogen, halogen C₁-C₆alkyl optionally substituted with one to three halogen atoms, one or two cyano groups, one or two nitro groups, one or two C(O)R₂ groups, one or two C(O)OR₃ groups, one or two C(O)NR₄R₅ groups, one or two P(O) (OR₆)₂ groups, one or two OR₇ groups, one or two SR₈ groups, one or two NR₉R₁₀ groups or one or two OC(O)R₁₁ groups,
C₃-C₇cycloalkyl,
C₃-C₆alkenyl,
C₃-C₆haloalkenyl
C₃-C₆alkynyl,
C₃-C₆haloalkynyl,
C(O)NR₁₂S(O)ₙR₁₃,
phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one or two C₂-C₆alkoxyalkyl groups, one or two nitro groups, one or two cyano groups, one C₂-C₆₋alkoxycarbonyl group, one C₂-C₆alkylcarbonyl group, one C₃-C₆alkoxycarbonylalkyl group or one C₃-C₆alkylcarbonylalkyl group, or
C₁-C₄alkyl substituted with one phenyl group wherein the phenyl group is optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one or two C₂-C₆alkoxyalkyl groups, one C₂-C₆alkoxycarbonyl group, one C₂-C₆alkylcarbonyl group, one C₃-C₆alkoxycarbonylalkyl group or one C₃-C₆-alkylcarbonylalkyl group, and
when R₃₇ and R₃₈ are taken together with the atom to which they are attached, they represent a ring in which R₃₇R₃₈ is a C₂-C₆alkylene group;
- R₄₁ and R₄₂: are each independently hydrogen, C₁-C₆alkyl, C₂-C₆alkylcarbonyl, C₂-C₆alkoxycarbonyl, hydroxycarbonyl, C₃-C₆alkylcarbonylalkyl, C₃-C₆alkoxycarbonylalkyl, C₂-C₆hydroxycarbonylalkyl,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups,
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, or
furfural optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, and
when R₄₁ and R₄₂ are taken together with the atom to which they are attached, they represent a three- to seven-membered saturated or unsaturated ring optionally interrupted with one group selected from O, S(O)ᵣ or NR₃₁, and optionally substituted with one to three methyl groups or one or more halogen atoms;
- r is: an integer of O, 1 or 2;
- n, p and q: are each independently an integer of 1 or 2;
- Q is: selected from 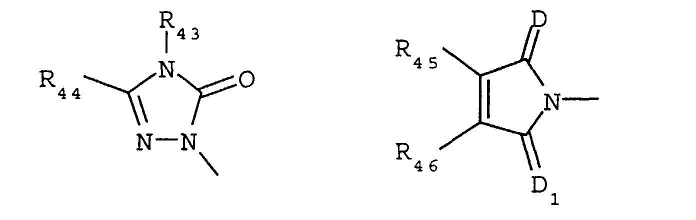 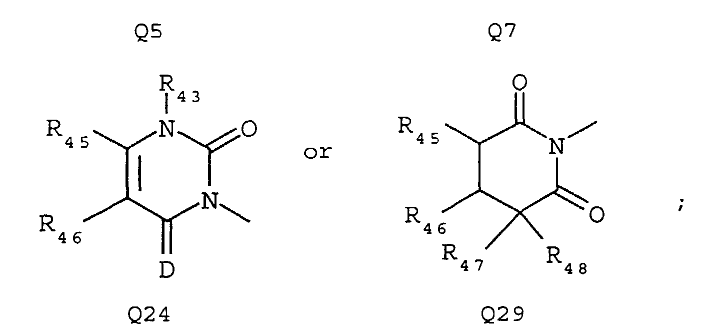
- D and D₁: are each independently O or S;
- R₄₃ and R₄₄: are each independently hydrogen, C₁-C₆alkyl, C₁-C₆haloalkyl, C₃-C₇cycloalkyl, C₃-C₆alkenyl, C₃-C₆haloalkenyl, C₃-C₆alkynyl, amino, or benzyl;
- R₄₅ and R₄₆: are each independently hydrogen, halogen, C₁-C₆alkyl, C₁-C₆haloalkyl, C₃-C₇cycloalkyl, C₃-C₆alkenyl, C₃-C₆haloalkenyl or C₃-C₆alkynyl, and when R₄₅ and R₄₆ are taken together with the atom to which they are attached, they represent a three- to seven-membered saturated or unsaturated ring optionally interrupted by oxygen, sulfur or nitrogen, and optionally substituted with one to three methyl groups or one or more halogen atoms; and
- R₄₇ and R₄₈: are each independently hydrogen, halogen or C₁-C₆alkyl.

More preferred formula I herbicidal agents of the present invention are those wherein
- X is: hydrogen, fluorine or chlorine;
- Y is: fluorine, chlorine, nitro or cyano;
- R is: hydrogen,
C₁-C₈alkyl optionally substituted with any combination of one to six halogen atoms, one or two cyano groups, one or two nitro groups, one or two C(O)R₂ groups, one or two C(O)OR₃ groups, one or two C(O)NR₄R₅ groups, one or two P(O) (OR₆)₂ groups, one or two OR₇ groups, one or two SR₈ groups, one or two NR₉R₁₀ groups, one or two C(O)NR₁₂S(O)ₙR₁₃ groups,
   one furyl group optionally substituted with any combination of one to three C₁-C₄alkyl groups, one to three C₁-C₄alkoxy groups, one or two C(O)OR₁₄ groups or one or two C(O)R₁₄ groups,
   one pyridyl group optionally substituted with any combination of one to three C₁-C₄alkyl groups, one to three C₁-C₄alkoxy groups, one or two C(O)OR₁₄ groups or one or two C(O)R₁₄ groups, or
   one phenyl group optionally substituted with any combination of one to three C₁-C₄alkyl groups, one to three C₁-C₄alkoxy groups, one or two C(O)OR₁₄ groups or one or two C(O)R₁₄ groups,
phenyl optionally substituted with any combination of one to three C₁-C₄alkyl groups, one to three C₁-C₄alkoxy groups, one or two C₂-C₆alkoxyalkyl groups, one or two C(O)OR₁₄ groups, one or two C(O)R₁₄ groups, one to three C₃-C₆alkoxycarbonylalkyl groups or one or two NR₁₅S(O)ₚR₁₆ groups,
C₃-C₈alkenyl,
C₃-C₈alkynyl,
OR₁₇,
NR₁₈R₁₉,
C(O)R₂₈ or
C(O)OR₂₉;
- Z is: O;
- R₁₄ is: C₁-C₄alkyl;
- R₂ is: hydrogen, C₁-C₆alkyl, C₂-C₆alkoxyalkyl or C₃-C₆-alkoxycarbonylalkyl;
- R₃ is: hydrogen, C₁-C₆alkyl, C₁-C₆haloalkyl, C₃-C₆alkenyl, C₃-C₆alkynyl, C₂-C₆alkoxyalkyl, C₂-C₆haloalkoxyalkyl, C₃-C₆alkoxycarbonylalkyl,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, or
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄-haloalkoxy groups;
- R₄ and R₅: are each independently hydrogen, C₁-C₆alkyl, C₃-C₆alkoxycarbonylalkyl,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, or
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, and
when R₄ and R₅ are taken together with the atom to which they are attached, they represent a ring in which R₄R₅ is a C₂-C₆alkylene group;
- R₆ is: hydrogen, C₁-C₆alkyl or benzyl;
- R₇ and R₈: are each independently hydrogen, C₁-C₆-alkyl, C₂-C₆alkoxycarbonylalkyl or C₂-C₆hydroxycarbonylalkyl;
- R₁₇ is: hydrogen, C₁-C₆alkyl, C₃-C₆alkenyl, C₃-C₆alkynyl, C₂-C₆alkoxyalkyl, C₃-C₆alkoxycarbonylalkyl, C₂-C₆-hydroxycarbonylalkyl,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, or
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups;
- R₉ and R₁₀: are each independently hydrogen, C₁-C₆alkyl or C₃-C₆alkoxycarbonylalkyl, and
when R₉ and R₁₀ are taken together with the atom to which they are attached, they represent a ring in which R₉R₁₀ is a C₂-C₆alkylene group;
- R₁₈ and R₁₉: are each independently hydrogen, C₁-C₆alkyl, C₃-C₆alkoxycarbonylalkyl, C₂-C₆hydroxycarbonylalkyl, C₂-C₆alkylsulfonylalkyl, C₂-C₆haloalkylsulfonylalkyl, or
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, and
when R₁₈ and R₁₉ are taken together with the atom to which they are attached, they represent a ring in which R₁₈R₁₉ is a C₂-C₆alkylene group;
- R₁₂ and R₁₅: are each independently hydrogen or C₁-C₆alkyl;
- R₁₃ and R₁₆: are each independently C₁-C₆alkyl, C₁-C₆haloalkyl, C₃-C₇cycloalkyl, C₃-C₆alkenyl, C₃-C₆haloalkenyl, C₃-C₆alkynyl, C₃-C₆haloalkynyl, or
benzyl optionally substituted on the phenyl ring with any combination of one to three halogen atoms, one to three C₁-C₄alkyl groups, one to three C₁-C₄haloalkyl groups, one to three C₁-C₄-alkoxy groups or one to three C₁-C₄haloalkoxy groups;
- R₂₈ is: hydrogen, C₁-C₆alkyl, C₁-C₆haloalkyl, C₃-C₆alkenyl C₃-C₆alkynyl, C₃-C₆alkoxycarbonylalkyl, or
phenyl optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups;
- R₂₉ is: hydrogen, C₁-C₆alkyl or benzyl;
- A is: -O-, -S(O)ᵣ-, or -NR₃₄-;
- B is: -CR₃₇R₃₈- or -C(=CR₄₁R₄₂)-;
- R₃₄ is: hydrogen,
C₁-C₆alkyl optionally substituted with one or two C(O)OR₃ groups or one or two C(O)NR₄R₅ groups,
C₃-C₆alkenyl,
C₃-C₆alkynyl,
phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one or two C₂-C₆-alkoxyalkyl groups, one or two nitro groups, one or two cyano groups, one C₂-C₆alkoxycarbonyl group, one C₂-C₆alkylcarbonyl group, one C₃-C₆-alkoxycarbonylalkyl group or one C₃-C₆alkylcarbonylalkyl group, or
benzyl optionally substituted on the phenyl ring with any combination of one to three halogen atoms, one to three C₁-C₄alkyl groups, one to three C₁-C₄haloalkyl groups, one to three C₁-C₄alkoxy groups or one to three C₁-C₄haloalkoxy groups;
- R₃₇ and R₃₈: are each independently hydrogen, halogen,
C₁-C₆alkyl optionally substituted with one or two cyano groups, one or two C(O)R₂ groups, one or two C(O)OR₃ groups, one or two C(O)NR₄R₅ groups, one or two P(O) (OR₆)₂ groups, one or two OR₇ groups or one or two NR₉R₁₀ groups,
C₃-C₇cycloalkyl,
C₃-C₆alkenyl,
C₃-C₆alkynyl,
C(O)NR₁₂S(O)ₙR₁₃, or
benzyl optionally substituted on the phenyl ring with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one or two C₂-C₆alkoxyalkyl groups, one C₂-C₆alkoxycarbonyl group, one C₂-C₆alkylcarbonyl group, one C₃-C₆alkoxycarbonylalkyl group or one C₃-C₆alkylcarbonylalkyl group, and
when R₃₇ and R₃₈ are taken together with the atom to which they are attached, they represent a ring in which R₃₇R₃₈ is a C₂-C₆alkylene group;
- R₄₁ and R₄₂: are each independently hydrogen, C₁-C₆alkyl, C₂-C₆alkylcarbonyl, C₂-C₆alkoxycarbonyl, hydroxycarbonyl, or
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups;
- r is: an integer of O, 1 or 2;
- n and p: are each independently an integer of 2; and
- Q is: selected from

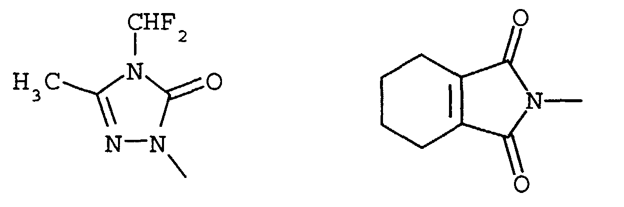

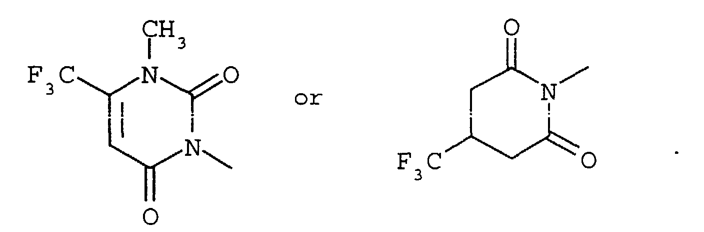

Most preferred formula I herbicidal agents of this invention are those wherein
- X is: fluorine;
- Y is: chlorine;
- R is: C₁-C₈alkyl optionally substituted with any combination of one or two C(O)OR₃ groups, one or two C(O)NR₄R₅ groups, one or two P(O) (OR₆)₂ groups,
one or two OR₇ groups, one or two SR₈ groups,
one or two NR₉R₁₀ groups, one or two
C(O)NR₁₂S(O)ₙR₁₃ groups or
one phenyl group optionally substituted with any combination of one to three C₁-C₄alkyl groups or one to three C₁-C₄alkoxy groups,

phenyl optionally substituted with any combination of one to three C₁-C₄alkyl groups, one to three C₁-C₄alkoxy groups, one or two C₂-C₆alkoxyalkyl groups, one or two C₃-C₆alkoxycarbonylalkyl groups or one or two NR₁₅S(O)ₚR₁₆ groups,
C₃-C₈alkenyl or
C₃-C₈alkynyl;
- Z is: O;
- R₃ is: hydrogen, C₁-C₆alkyl, C₁-C₆haloalkyl, C₃-C₆alkenyl, C₃-C₆alkynyl, C₃-C₆alkoxycarbonylalkyl,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, or
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄-haloalkoxy groups;
- R₄ and R₅: are each independently hydrogen or C₁-C₆alkyl, and when R₄ and R₅ are taken together with the atom to which they are attached, they represent a ring in which R₄R₅ is a C₂-C₆alkylene group;
- R₆ is: hydrogen or C₁-C₆alkyl;
- R₇ and R₈: are each independently hydrogen, C₁-C₆alkyl C₂-C₆alkoxycarbonylalkyl or C₂-C₆hydroxycarbonylalkyl;
- R₉ and R₁₀: are each independently hydrogen, C₁-C₆alkyl or C₃-C₆alkoxycarbonylalkyl, and when R₉ and R₁₀ are taken together with the atom to which they are attached, they represent a ring in which R₉R₁₀ is a C₂-C₆alkylene group;
- R₁₂ and R₁₅: are hydrogen;
- R₁₃ and R₁₆: are each independently C₁-C₆alkyl, C₁-C₆-haloalkyl, C₃-C₆alkenyl, C₃-C₆alkynyl, or
benzyl optionally substituted on the phenyl ring with any combination of one to three halogen atoms, one to three C₁-C₄alkyl groups, one to three C₁-C₄haloalkyl groups, one to three C₁-C₄-alkoxy groups or one to three C₁-C₄haloalkoxy groups;
- A is: -O- or -S(O)ᵣ-;
- B is: -CR₃₇R₃₈- or -C(=CR₄₁R₄₂)-;
- R₃₇ and R₃₈: are each independently hydrogen, halogen, C₁-C₆alkyl optionally substituted with one or two C(O)OR₃ groups, one or two C(O)NR₄R₅ groups, one or two OR₇ groups or one or two NR₉R₁₀ groups, or
benzyl optionally substituted on the phenyl ring with any combination of one to three C₁-C₄alkyl groups, one to three C₁-C₄alkoxy groups or one C₃-C₆alkoxycarbonylalkyl group, and
when R₃₇ and R₃₈ are taken together with the atom to which they are attached, they represent a ring in which R₃₇R₃₈ is a C₂-C₆alkylene group;
- R₄₁ and R₄₂: are each independently hydrogen, C₁-C₆alkyl, C₂-C₆alkylcarbonyl, C₂-C₆alkoxycarbonyl or hydroxycarbonyl;
- r is: an integer of O, 1 or 2;
- n and p: are each independently an integer of 2; and
- Q is: selected from 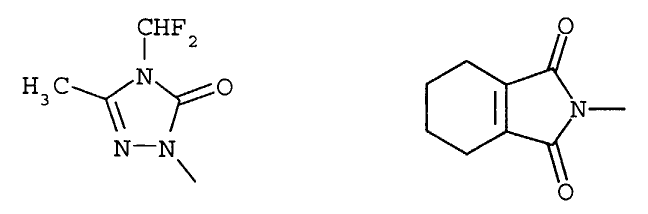 or

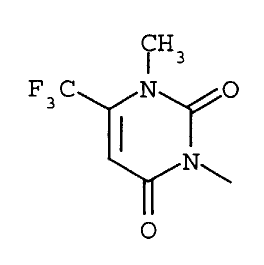

Formula I compounds of the present invention which are particularly effective herbicidal agents include
N-{4-chloro-2-fluoro-5-[(3-methyl-4-oxo-2-thiazolidinylidene)amino]phenyl}-1-cyclohexene-1,2-dicarboximide;
3-{4-chloro-2-fluoro-5-[3-methyl-4-oxo-2-thiazolidinylidene)amino]phenyl}-1-methyl-6-(trifluoromethyl)-2,4(1H,3H)-pyrimidinedione;
ethyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
3-(carboxymethyl)-2-{{2-chloro-5-[3,6-dihydro-3-methyl-2,6-dioxo-4-(trifluoromethyl)-1(2H)-pyrimidinyl]-4-fluorophenyl}imino}-4-oxo-delta5,alpha-thiazolidineacetic acid, diethyl ester;
ethyl 5-{{2-chloro-5-[3,6-dihydro-3-methyl-2,6-dioxo-4-(trifluoromethyl)-1(2H)-pyrimidinyl]-4-fluorophenyl}imino}-7-oxo-4-thia-6-azaspiro[2.4]heptane-6-acetate;
2-{{2-chloro-5-[3,6-dihydro-3-methyl-2,6-dioxo-4-(trifluoromethyl)-1(2H)-pyrimidinyl]-4-fluorophenyl}-4-oxo-3,5-thiazolidinediacetic acid, 3-ethyl ester;
ethyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-oxazolidineacetate;
α-benzyl-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetic acid, L-;
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid, 3-ethyl methyl ester;
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid, 3-ethyl isopropyl ester;
ethyl 5-(carbamoylmethyl)-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
ethyl 2-{{2-chloro-5-[3,6-dihydro-3-methyl-2,6-dioxo-4-(trifluoromethyl)-1(2H)-pyrimidinyl]-4-fluorophenyl}imino)-5-[(methylcarbamoyl)methyl]-4-oxo-3-thiazolidineacetate;
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid, 3-ethyl (2-propynyl) ester;
methyl 3-allyl-2-{{2-chloro-5-[3,6-dihydro-3-methyl-2,6-dioxo-4-(trifluoromethyl)-1(2H)-pyrimidinyl]-4-fluorophenyl}imino}-4-oxo-5-thiazolidineacetate;
isopropyl 3-allyl-2-{{2-chloro-5-[3,6-dihydro-3-methyl-2,6-dioxo-4-(trifluoromethyl)-1(2H)-pyrimidinyl]-4-fluorophenyl}imino}-4-oxo-5-thiazolidineacetate;
methyl α-benzyl-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate, L-;
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid, 3-ethyl 2-fluoroethyl ester;
2-fluoroethyl 3-allyl-2-{{2-chloro-5-[3,6-dihydro-3-methyl-2,6-dioxo-4-(trifluoromethyl)-1(2H)-pyrimidinyl]-4-fluorophenyl}imino}-4-oxo-5-thiazolidineacetate;
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-oxazolidinediacetic acid, 3-ethyl ester;
ethyl 2-{{2-chloro-5-[3,6-dihydro-3-methyl-2,6-dioxo-4-(trifluoromethyl)-1(2H)-pyrimidinyl]-4-fluorophenyl}imino}-5-(hydroxyethyl)-4-oxo-3-thiazolidineacetate;
allyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
isopropyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
3-{4-chloro-2-fluoro-5-[(3-furfuryl-4-oxo-2-thiazolidinylidene)amino]phenyl}-1-methyl-6-(trifluoromethyl)-2,4(1H,3H)-pyrimidinedione;
cyclopropylmethyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
ethyl 5-chloro-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
3-{4-chloro-5-[(3-cyclopropyl-4-oxo-2-thiazolidinylidene)amino]-2-fluorophenyl}-1-methyl-6-(trifluoromethyl)-2,4(1H,3H)-pyrimidinedione;
3-{4-chloro-2-fluoro-5-{[3-(2-methoxyethyl)-4-oxo-2-thiazolidinylidene]amino}phenyl}-1-methyl-6-(trifluoromethyl)-2,4(1H,3H)-pyrimidinedione;
3-{4-chloro-2-fluoro-5-{[3-(1-methyl-2-propynyl)-4-oxo-2-thiazolidinylidene]amino}phenyl}-1-methyl-6-(trifluoromethyl)-2,4(1H,3H)-pyrimidinedione;
ethyl 5-benzyl-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
3-{5-[(3-benzyl-4-oxo-2-thiazolidinylidene)amino]-4-chloro-2-fluorophenyl}-1-methyl-6-(trifluoromethyl)-2,4(1H,3H)-pyrimidinedione;
methyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidinepropionate;
methyl α-benzyl-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate, D-;
methyl 2-{{2-chloro-5-[3,6-dihydro-3-methyl-2,6-dioxo-4-(trifluoromethyl)-1(2H)-pyrimidinyl]-4-fluorophenyl}imino}-4-oxo-α-(*p*-nitrobenzyl)-3-thiazolidineacetate, L-;
ethyl α-benzyl-2-{{2-chloro-5-[3,6-dihydro-3-methyl-2,6-dioxo-4-(trifluoromethyl)-1(2H)-pyrimidinyl]-4-(fluorophenyl}imino}-4-oxo-3-thiazolidineacetate, L-; and
methyl α-(*p*-chlorobenzyl)-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate, DL-, among others.

Exemplary of halogen hereinabove are fluorine, chlorine, bromine and iodine. The terms haloalkyl, halocycloalkyl, haloalkoxy, haloalkenyl, halocycloalkenyl and haloalkynyl as used in the specification and claims designate an alkyl group, a cycloalkyl group, an alkoxy group, an alkenyl group, a cycloalkenyl group and an alkynyl group substituted with one or more halogen atoms, respectively. In formula I above, alkali metals include: sodium, potassium and lithium. Alkaline earth metals of formula I include magnesium and calcium. Further, the term organic ammonium is defined as a group consisting of a positively charged nitrogen atom joined to from one to four aliphatic groups, each containing from one to sixteen carbon atoms.

In formula I above, 4- to 10-membered heterocyclic rings include, but are not limited to, 1,2,4-triazole, 1,3,4-thiadiazole, 1,3,5-triazine, 2-thiazoline, benzimidazole, benzofuran, benzothiophene, coumarin, furan, imidazole, imidazoline-2-thione, indole, isatoic anhydride, isoquinoline, isoxazole, morpholine, oxazole, piperazine, piperidine, purine, pyrazine, pyrazole, pyridazine, pyridine, pyrimidine, pyrrole, pyrrolidine, quinoline, tetrahydrofuran, tetrahydrothiophene, thiazole and thiophene rings, wherein each ring is optionally substituted as described hereinabove for formula I.

The formula I compounds of the present invention are effective herbicidal agents useful for the control of a wide variety of undesirable plant species. Those compounds are effective for controlling weeds native to both dry land and wet land areas. The compounds are effective in controlling the above-said plants when applied to the foliage thereof or to the soil or water containing seeds or other propagating organs thereof such as stolons, tubers or rhizomes, at rates of from about 0.01 kg/ha to 4 kg/ha and preferably from about 0.01 kg/ha to 1 kg/ha.

The compounds of this invention are best suited for use as broad spectrum herbicides. However, certain compounds of this invention are selective in soybeans and/or cereal crops such as corn, wheat and rice when applied as preemergence and/or postemergence treatments.

In addition, it has been found that the formula I compounds of this invention may be used for the selective control of undesirable plant species in transplanted rice culture by applying a herbicidally effective amount of a formula I compound to the soil or water containing seeds or other propagating organs of said undesirable plant species after the rice has been transplanted.

Formula I compounds of this invention which are especially useful for the selective control of undesirable plant species in the presence of corn include
N-{4-chloro-2-fluoro-5-[(3-methyl-4-oxo-2-thiazolidinylidene)amino]phenyl}-1-cyclohexene-1,2-dicarboximide;
ethyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
2-{{2-chloro-5-[3,6-dihydro-3-methyl-2,6-dioxo-4-(trifluoromethyl)-1(2H)-pyrimidinyl]-4-fluorophenyl}-4-oxo-3,5-thiazolidinediacetic acid, 3-ethyl ester;
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid, 3-ethyl methyl ester;
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid, 3-ethyl isopropyl ester;
ethyl 5-(carbamoylmethyl)-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
2-{{2-chloro-5-[3,6-dihydro-3-methyl-2,6-dioxo-4-(trifluoromethyl)-1(2H)-pyrimidinyl]-4-fluorophenyl}imino)-4-oxo-3,5-thiazolidinediacetic acid, 3-ethyl (2-propynyl) ester;
isopropyl 3-allyl-2-{{2-chloro-5-[3,6-dihydro-3-methyl-2,6-dioxo-4-(trifluoromethyl)-1(2H)-pyrimidinyl]-4-fluorophenyl}imino}-4-oxo-5-thiazolidineacetate;
methyl α-benzyl-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate, L-;
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid, 3-ethyl 2-fluoroethyl ester;
2-fluoroethyl 3-allyl-2-{{2-chloro-5-[3,6-dihydro-3-methyl-2,6-dioxo-4-(trifluoromethyl)-1(2H)-pyrimidinyl]-4-fluorophenyl}imino}-4-oxo-5-thiazolidineacetate; and
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-oxazolidinediacetic acid, 3-ethyl ester, among others.

Formula I compounds of the present invention which are particularly useful for the selective control of undesirable plant species in the presence of wheat include
3-(carboxymethyl)-2-{{2-chloro-5-[3,6-dihydro-3-methyl-2,6-dioxo-4-(trifluoromethyl)-1(2H)-pyrimidinyl]-4-fluorophenyl}imino}-4-oxo-delta5,alpha-thiazolidineacetic acid, diethyl ester;
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid, 3-ethyl methyl ester;
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid, 3-ethyl isopropyl ester;
ethyl 5-(carbamoylmethyl)-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
ethyl 2-{{2-chloro-5-[3,6-dihydro-3-methyl-2,6-dioxo-4-(trifluoromethyl)-1(2H)-pyrimidinyl]-4-fluorophenyl}imino}-5-[(methylcarbamoyl)methyl]-4-oxo-3-thiazolidineacetate;
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid, 3-ethyl (2-propynyl) ester;
isopropyl 3-allyl-2-{{2-chloro-5-[3,6-dihydro-3-methyl-2,6-dioxo-4-(trifluoromethyl)-1(2H)-pyrimidinyl]-4-fluorophenyl}imino}-4-oxo-5-thiazolidineacetate;
methyl α-benzyl-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate, L-;
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid, 3-ethyl 2-fluoroethyl ester;
2-fluoroethyl 3-allyl-2-{{2-chloro-5-[3,6-dihydro-3-methyl-2,6-dioxo-4-(trifluoromethyl)-1(2H)-pyrimidinyl]-4-fluorophenyl}imino}-4-oxo-5-thiazolidineacetate;
2-{{2-chloro-5-[3,6-dihydro-3-methyl-2,6-dioxo-4-(trifluoromethyl)-1(2H)-pyrimidinyl]-4-fluorophenyl}imino)-4-oxo-3,5-oxazolidinediacetic acid, 3-ethyl ester;
ethyl 2-{{2-chloro-5-[3,6-dihydro-3-methyl-2,6-dioxo-4-(trifluoromethyl)-1(2H)-pyrimidinyl]-4-fluorophenyl}imino}-5-(hydroxyethyl)-4-oxo-3-thiazolidineacetate; and
cyclopropylmethyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate, among others.

Formula I compounds of this invention which are particularly useful for the selective control of undesirable plant species in the presence of transplanted rice include
ethyl 5-benzyl-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
3-{5-[(3-benzyl-4-oxo-2-thiazolidinylidene)amino]-4-chloro-2-fluorophenyl}-1-methyl-6-(trifluoromethyl)-2,4(1H,3H)-pyrimidinedione;
methyl 2-{{2-chloro-5-[3,6-dihydro-3-methyl-2,6-dioxo-4-(trifluoromethyl)-1(2H)-pyrimidinyl]-4-fluorophenyl)imino)-4-oxo-3-thiazolidinepropionate;
methyl α-benzyl-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate, L-;
methyl α-benzyl-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate, D-;
methyl 2-{{2-chloro-5-[3,6-dihydro-3-methyl-2,6-dioxo-4-(trifluoromethyl)-1(2H)-pyrimidinyl]-4-fluorophenyl}imino}-4-oxo-α-(*p*-nitrobenzyl)-3-thiazolidineacetate, L-;
ethyl α-benzyl-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate, L-; and
methyl α-(*p*-chlorobenzyl)-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate, DL-, among others.

Formula I compounds of this invention which are particularly useful for the selective control of undesirable plant species in the presence of soybeans include
ethyl 5-(carbamoylmethyl)-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid, 3-ethyl 2-fluoroethyl ester;
2-{{2-chloro-5-[3,6-dihydro-3-methyl-2,6-dioxo-4-(trifluoromethyl)-1(2H)-pyrimidinyl]-4-fluorophenyl}imino)-4-oxo-3,5-oxazolidinediacetic acid, 3-ethyl ester;
allyl 2-{{2-chloro-5-[3,6-dihydro-3-methyl-2,6-dioxo-4-(trifluoromethyl)-1(2H)-pyrimidinyl]-4-fluorophenyl)imino)-4-oxo-3-thiazolidineacetate; and
3-{4-chloro-2-fluoro-5-[(3-furfuryl-4-oxo-2-thiazolidinylidene)amino]phenyl}-1-methyl-6-(trifluoromethyl)-2,4(1H,3H)-pyrimidinedione, among others.

While the compounds of this invention are effective for controlling undesirable plant species when employed alone, they may also be used in combination with or in conjunction with one or more other biological chemicals, including herbicides.

The compounds of this invention may be applied to the foliage of undesirable plant species or to the soil or water containing seeds or other propagating organs thereof in the form of a solid or liquid herbicidal composition, comprising a herbicidally effective amount of the desired compound dispersed or dissolved in an agronomically acceptable, inert solid or liquid carrier. The compositions may be applied as preemergence or postemergence treatments.

The formula I compounds of the present invention may be formulated as emulsifiable concentrates, wettable powders, granular formulations, suspension concentrates, flowable concentrates and the like.

Formula I compounds wherein Q is Q1, Q2, Q3, Q5, Q6, Q7, Q9, Q10, Q11, Q12, Q13, Q14, Q16, Q17, Q18, Q19, Q20, Q22, Q24 and Q25 may be prepared from 1-(3-aminophenyl)-isothiourea, -isourea, -guanidine and -amidine compounds of formula II 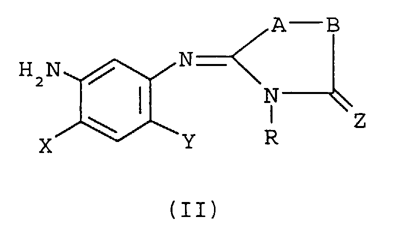 wherein A, B, R, X, Y and Z are as described hereinabove, using essentially the same procedures as described in U.S. 5,523,278.

Formula I compounds wherein Q is Q8 may be prepared by reacting an amine of formula II with a substituted tetrahydrofuran of formula III as shown below in Flow Diagram I.

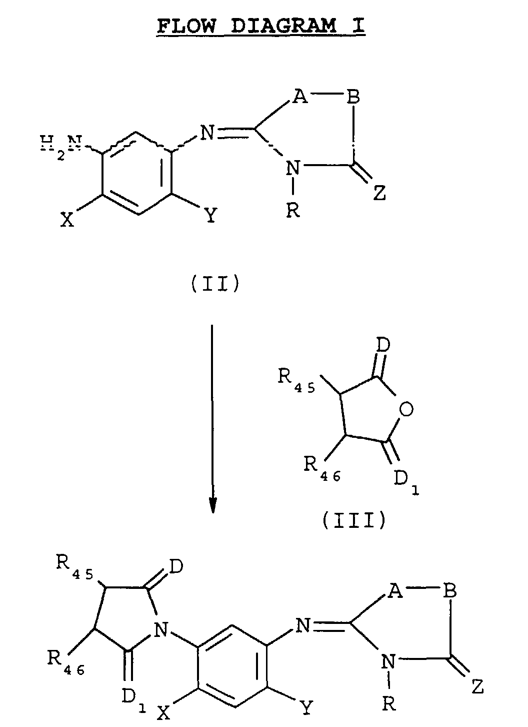

Formula I compounds wherein Q is Q4 may be prepared from formula I compounds wherein Q is Q8 using essentially the same procedures used to prepare formula I compounds wherein Q is Q3 from formula I compounds wherein Q is Q7.

Compounds of formula I wherein Q is Q15 may be prepared by reacting an amine of formula II with a substituted tetrahydrofuran of formula III to form an acid-amide of formula IV, and dehydrating the acid-amide with a dehydrating agent such as 1,3-dicyclohexylcarbodiimide. The reaction scheme is shown below in Flow Diagram II.

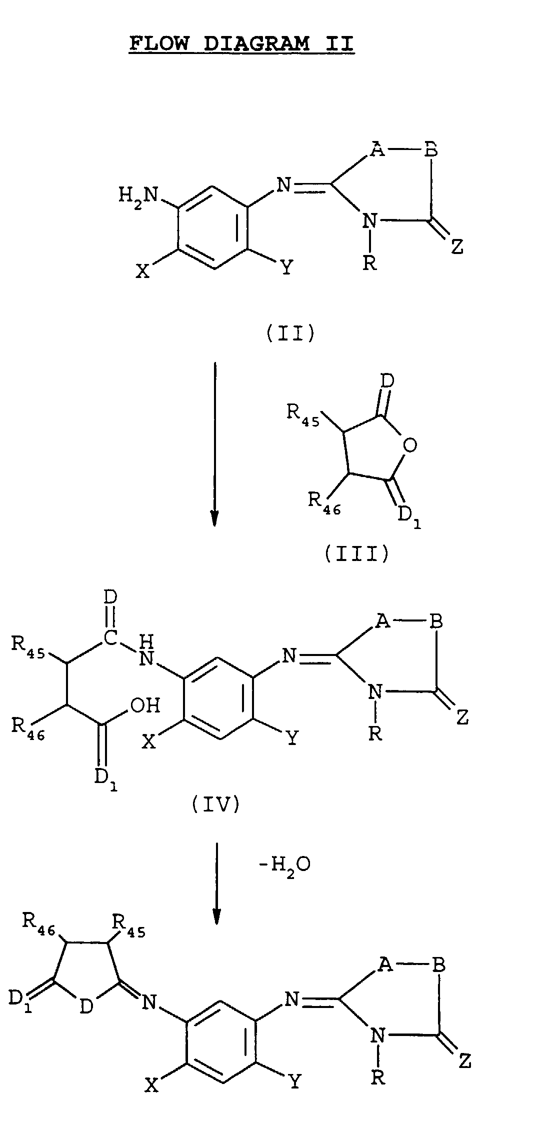

Formula I compounds wherein Q is Q21 may be prepared from formula I compounds wherein Q is Q8 using essentially the same procedure used to prepare formula I compounds wherein Q is Q20 from formula I compounds wherein Q is Q7.

Compounds of formula I wherein Q is Q23 may be prepared by converting an amine of formula II to its corresponding isocyanate of formula V using standard methods such as phosgene in an inert solvent or palladium chloride and carbon monoxide, reacting the formula V compound with a substituted hydrazine of formula VI to form an intermediate compound of formula VII, and reacting the formula VII compound with an ester of formula VIII. The reaction scheme is shown in Flow Diagram III.

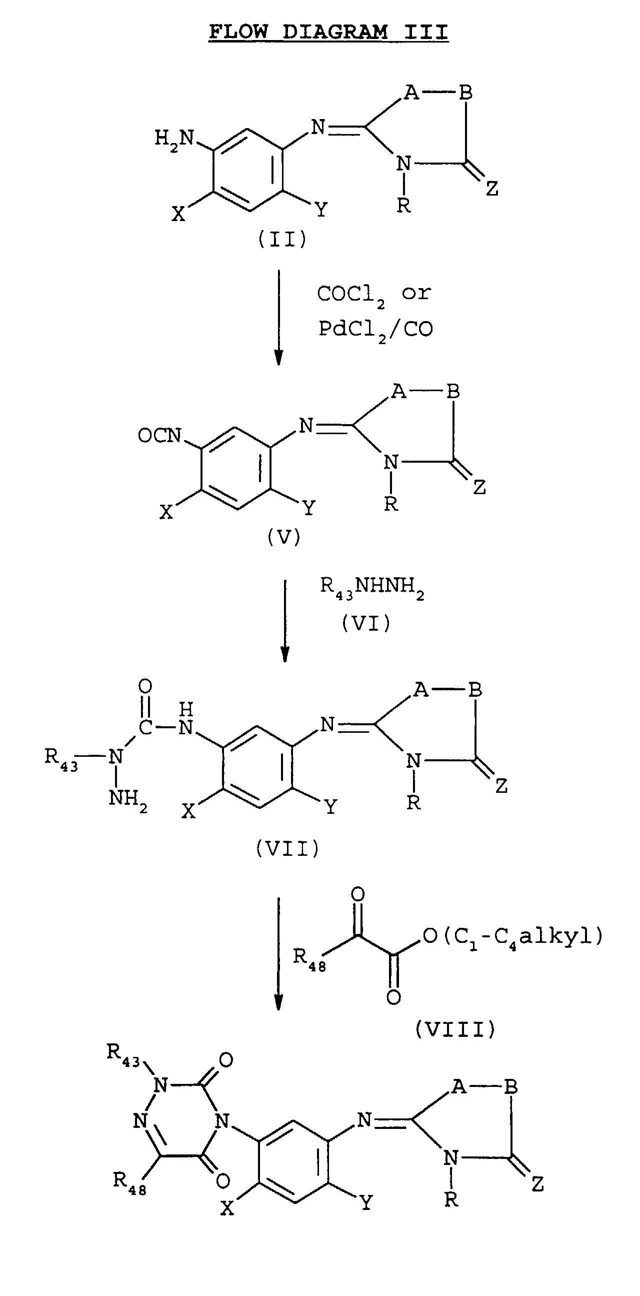

Formula I compounds wherein Q is Q26 may be prepared by reacting an amine of formula II with a β-aminoacrylic acid chloride of formula IX to form an intermediate compound of formula X, and reacting the intermediate compound with an acid chloride of formula XI. The reaction scheme is shown in Flow Diagram IV.

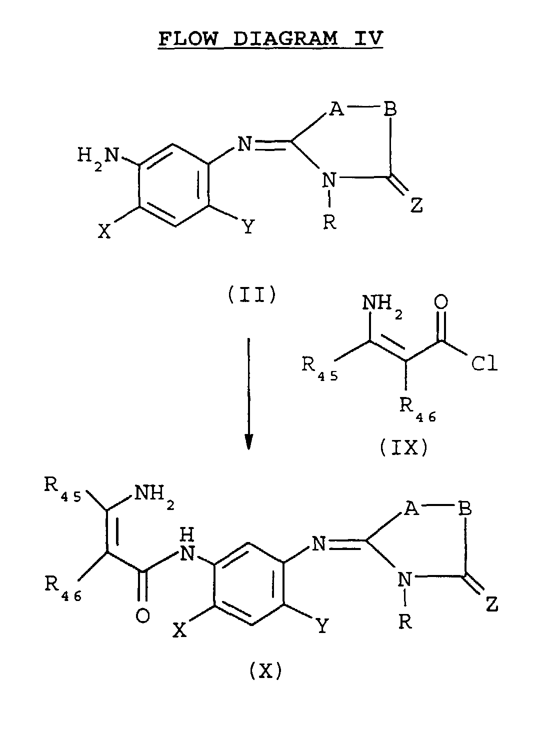

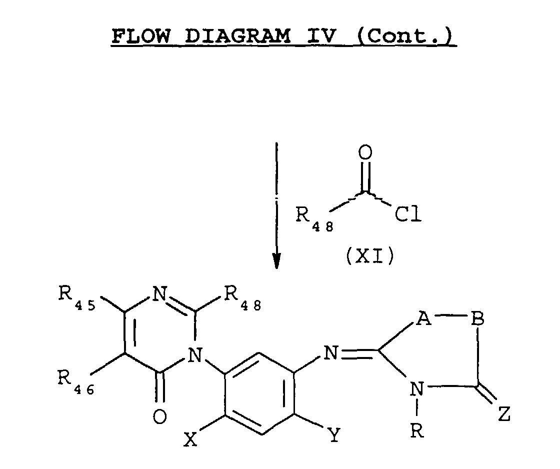

Compounds of formula I wherein Q is Q27 may be prepared by reacting an amine of formula II with an acid chloride of formula XII to form an intermediate compound of formula XIII, and reacting the intermediate compound (after deprotection) with an acid chloride of formula XI. The reaction sequence is shown below in Flow Diagram V.

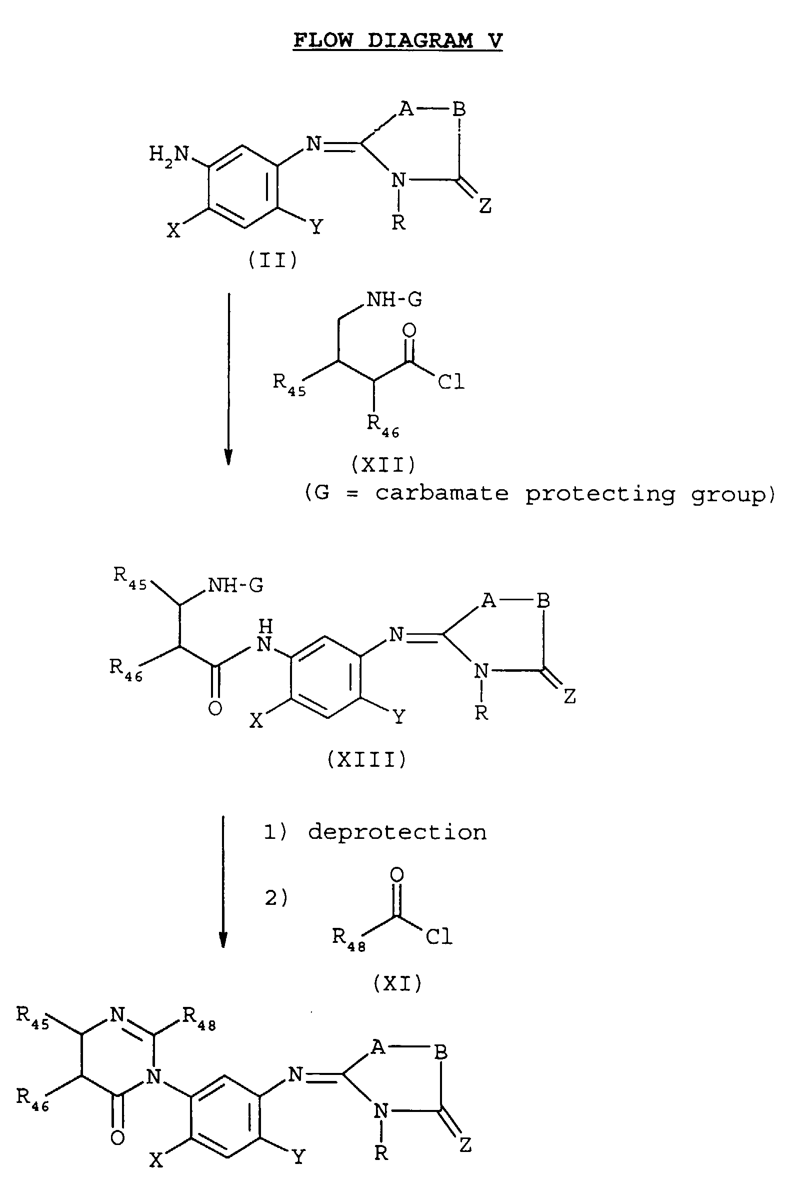

Formula I compounds wherein Q is Q28 may be prepared by reacting an amine of formula II with an unsaturated lactone of formula XIV as shown below in Flow Diagram VI.

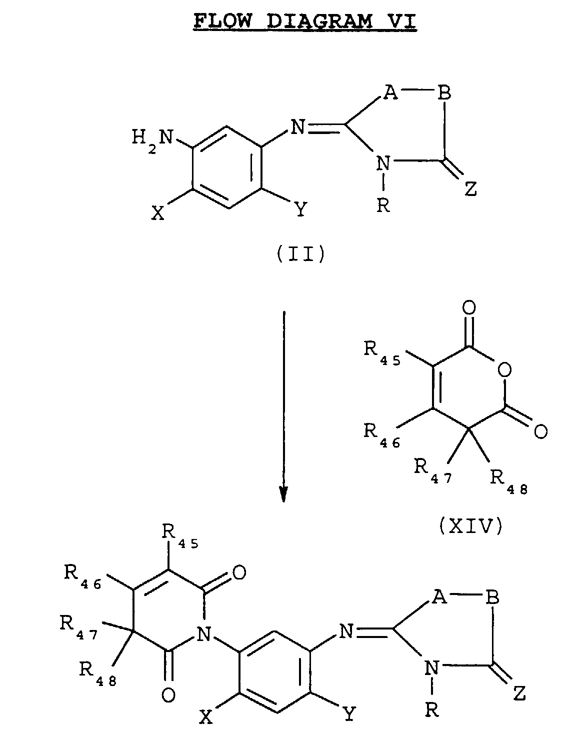

Similarly, formula I compounds wherein Q is Q29 may be prepared by reacting an amine of formula II with a lactone of formula XV. The reaction scheme is shown in Flow Diagram VII.

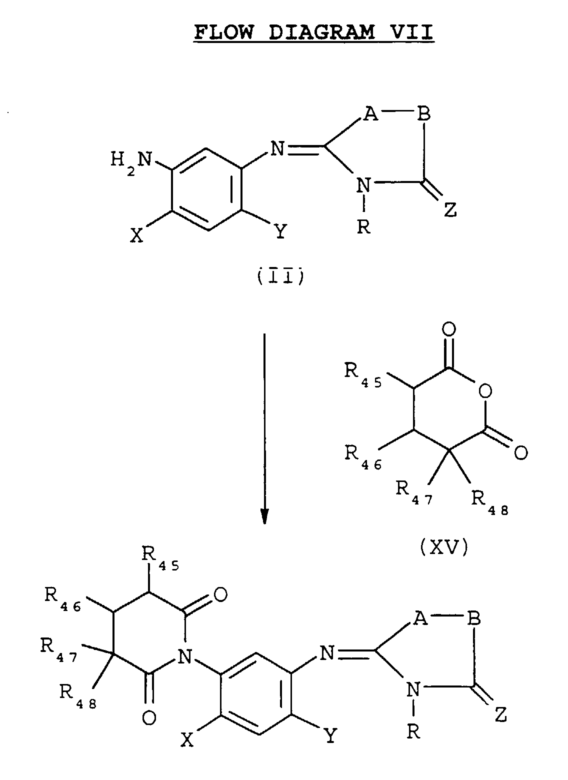

Compounds of formula I wherein Q is Q30 may be prepared, as shown in Flow Diagram VIII, by reacting an isocyanate or isothiocyanate of formula XVI with an unsaturated lactone of formula XVII at an elevated temperature.

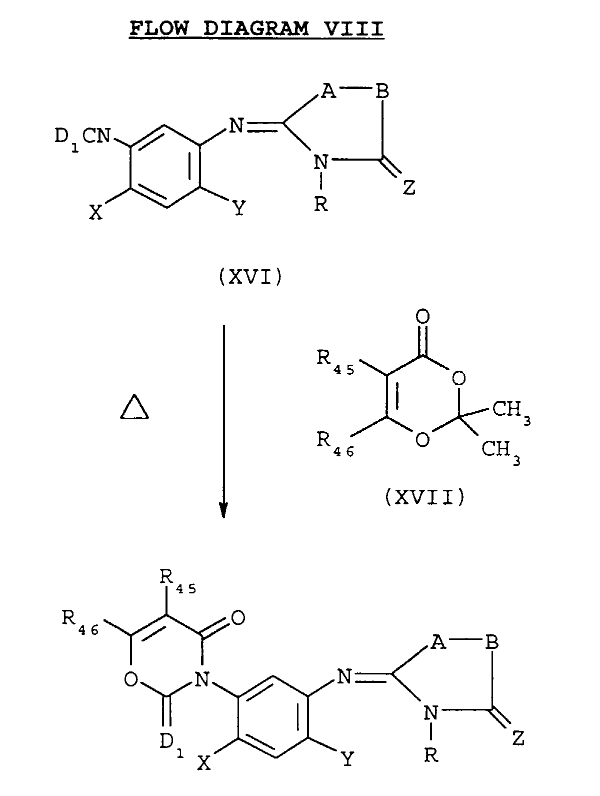

Similarly, formula I compounds wherein Q is Q31 may be prepared by reacting an isocyanate or isothiocyanate of formula XVI with a lactone of formula XVIII at an elevated temperature. The reaction scheme is shown in Flow Diagram IX.

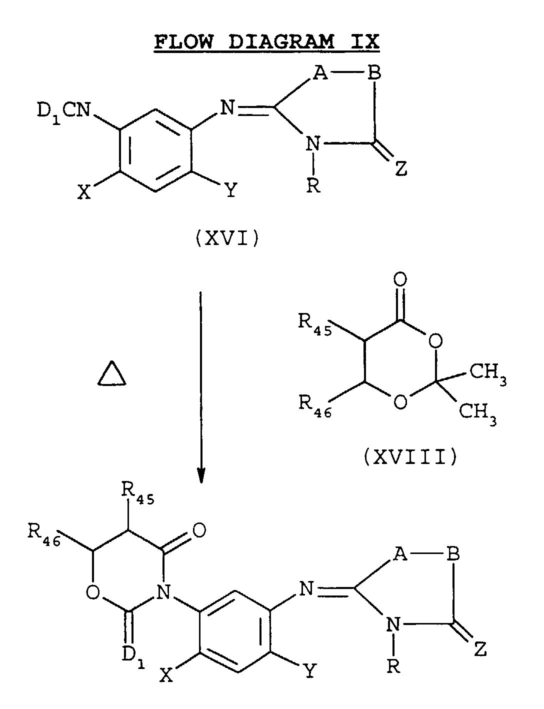

Formula I compounds wherein Q is Q32 may be prepared, as shown in Flow Diagram X, by reacting an amine of formula II with sodium isocyanate or sodium isothiocyanate to form a urea of formula XIX, reacting the urea with a malonic acid diester of formula XX to form an intermediate compound of formula XXI, and reacting the intermediate compound with a phosphorus oxyhalide to form a formula I compound wherein Q is Q32 and E is halogen, and optionally reacting the formula I compound wherein Q is Q32 and E is halogen with a C₁-C₄-alkyl sulfide or a C₁-C₄alkoxide to form a formula I compound wherein Q is Q32 and E is C₁-C₄alkylthio or C₁-C₄alkoxy, respectively.

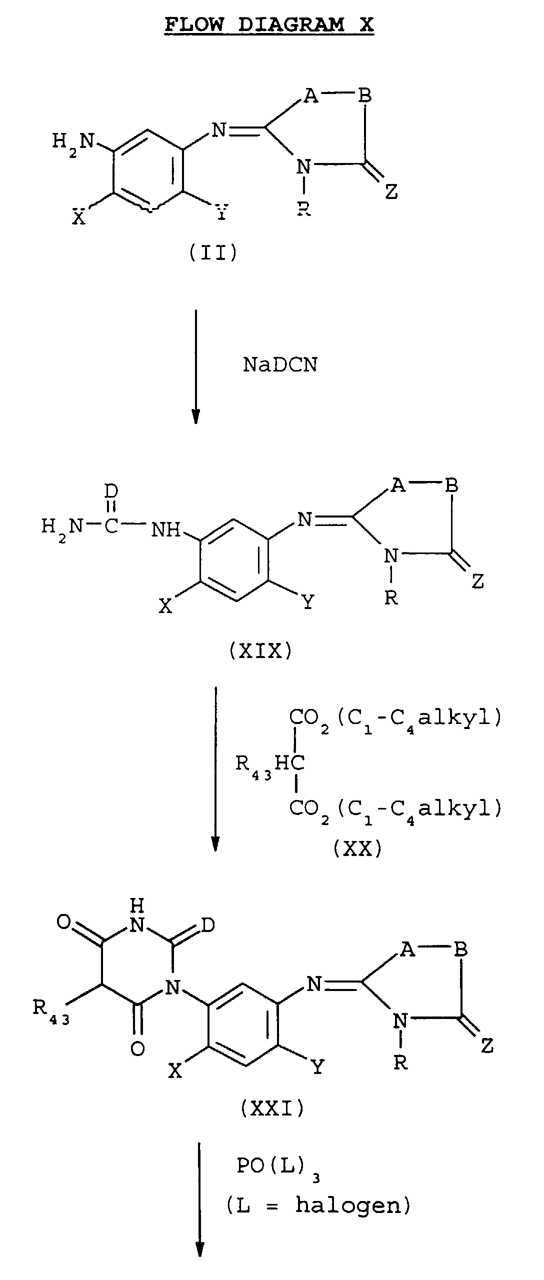

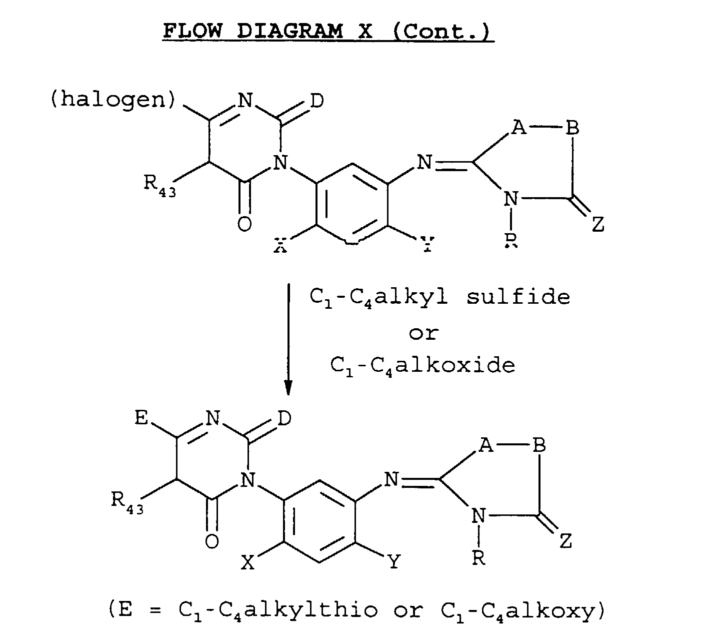

Compounds of formula I wherein Q is Q33 may be prepared, as shown in Flow Diagram XI, by acylating a compound of formula XXII with acetyl chloride and aluminum chloride to form an acetophenone of formula XXIII, reacting the acetophenone compound with an ester of formula XXIV in the presence of a base to form a diketone compound of formula XXV, reacting the formula XXV compound with a substituted hydrazine of formula XXVI to form an intermediate compound of formula XXVII, and halogenating the formula XXVII compound with a halogenating agent such as phosphorus oxychloride to form a formula I compound wherein Q is Q33 and E is halogen, and optionally reacting the formula I compound wherein Q is Q33 and E is halogen with a C₁-C₄alkyl sulfide or a C₁-C₄-alkoxide to form a formula I compound wherein Q is Q33 and E is C₁-C₄alkylthio or C₁-C₄alkoxy, respectively.

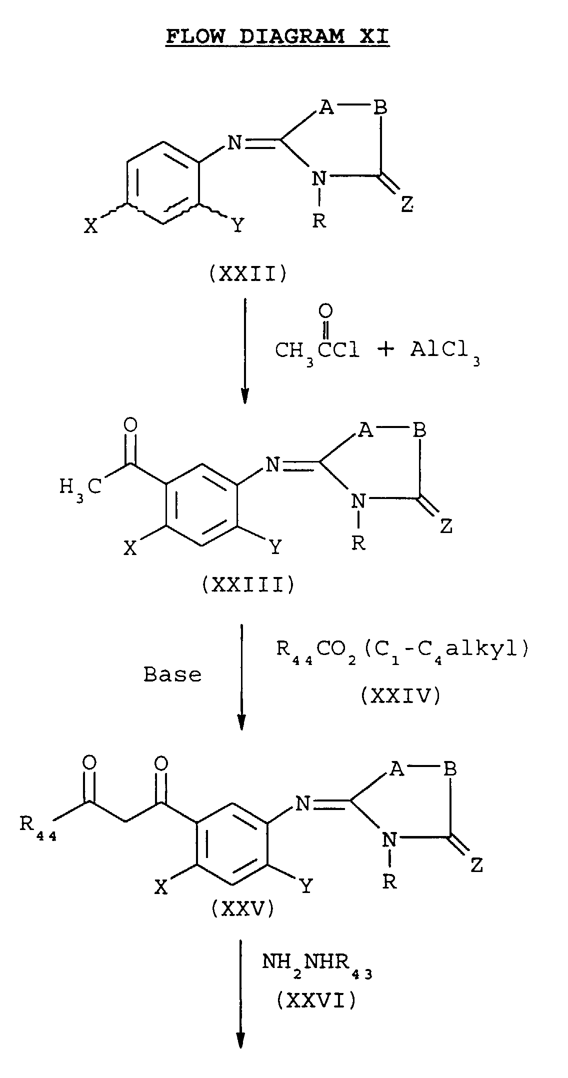

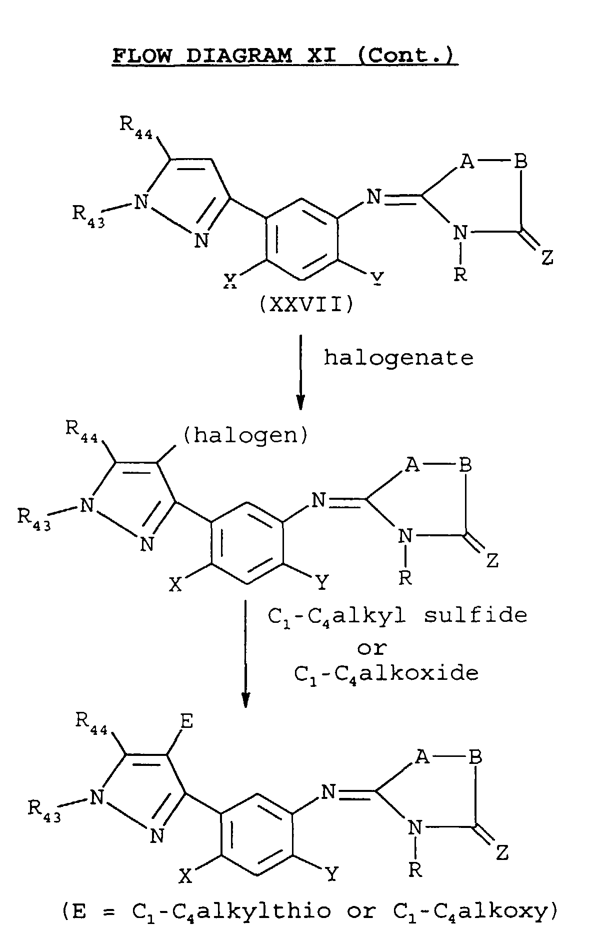

Formula I compounds wherein Q is Q34 may be prepared, as shown in Flow Diagram XII, by reacting an isocyanate or isothiocyanate of formula XVI with a substituted hydrazine of formula XXVIII to form an intermediate compound of formula XXIX, and reacting the formula XXIX compound with an acetal of formula XXX at an elevated temperature.

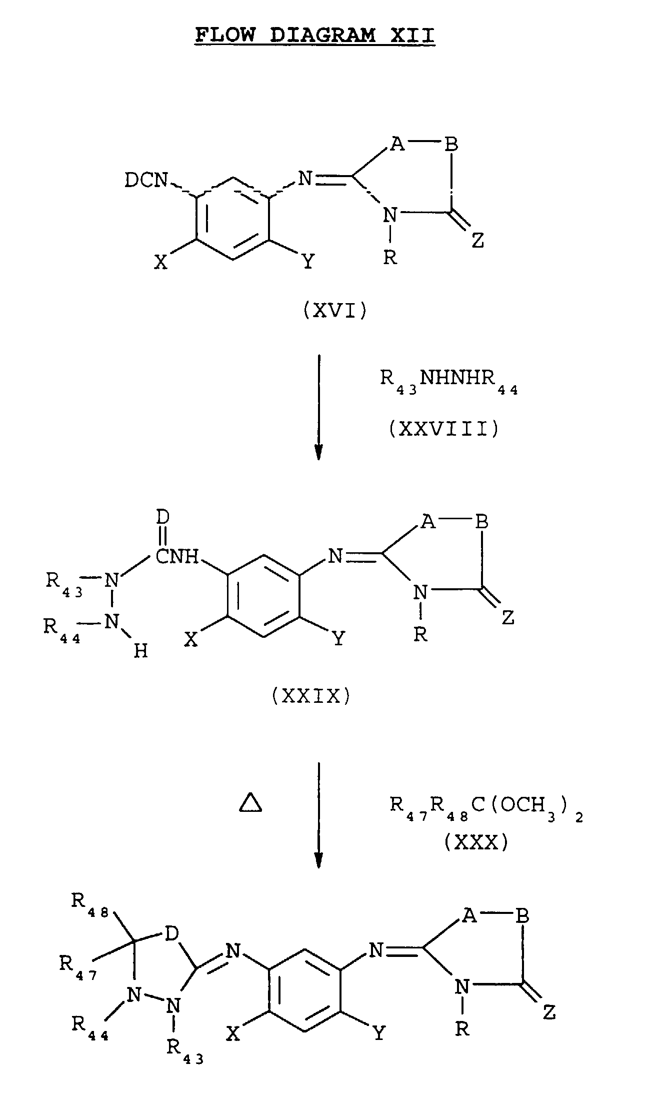

Compounds of formula I wherein Q is Q35 may be prepared by reacting a cyano compound of formula XXXI with a substituted hydrazine of formula XXVI to form an intermediate compound of formula XXXII, and reacting the intermediate compound with an acid chloride of formula XXXIII. The reaction scheme is show in Flow Diagram XIII.

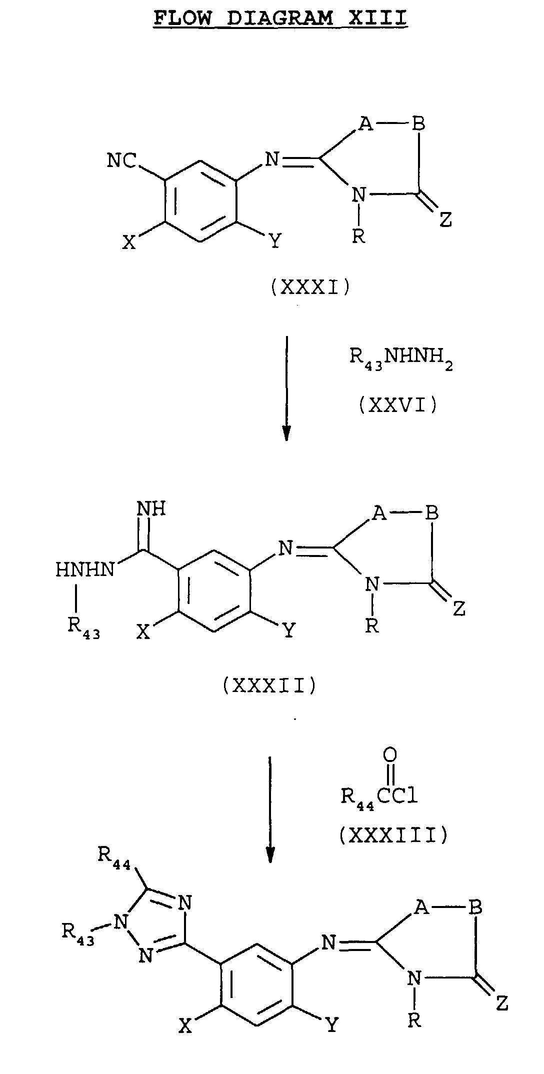

Formula I compounds wherein Q is Q36 may be prepared, as shown in Flow Diagram XIV, by reacting an isothiocyanate of formula XXXIV with an amine of formula XXXV to form a thiourea of formula XXXVI, and reacting the thiourea with an α-haloketone of formula XXXVII.

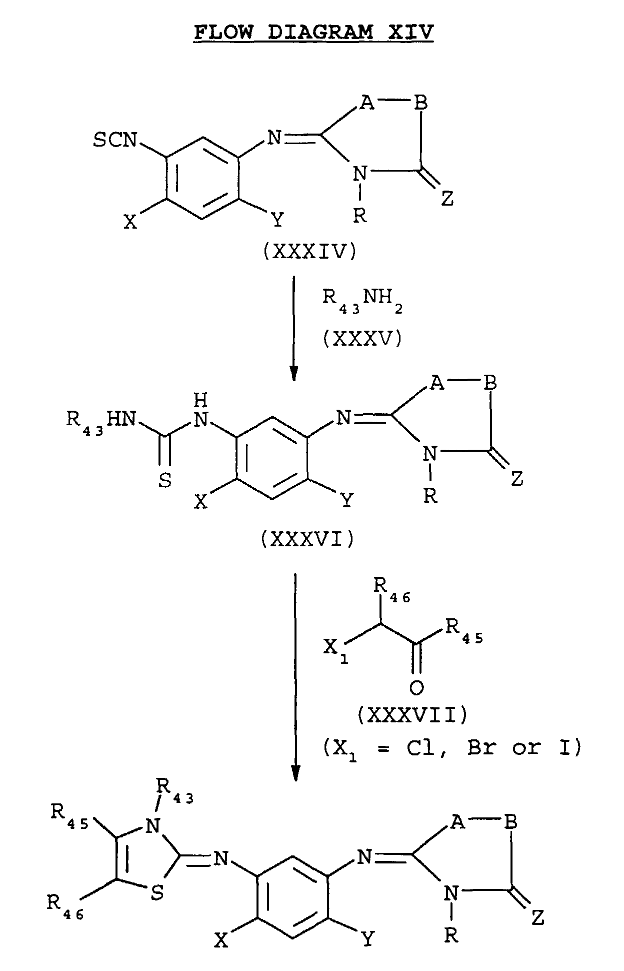

Compounds of formula II wherein Q is Q37 may be prepared, as shown below in Flow Diagram XV, by reacting a urea or thiourea of formula XXXVIII with an acid chloride of formula XXXIX.

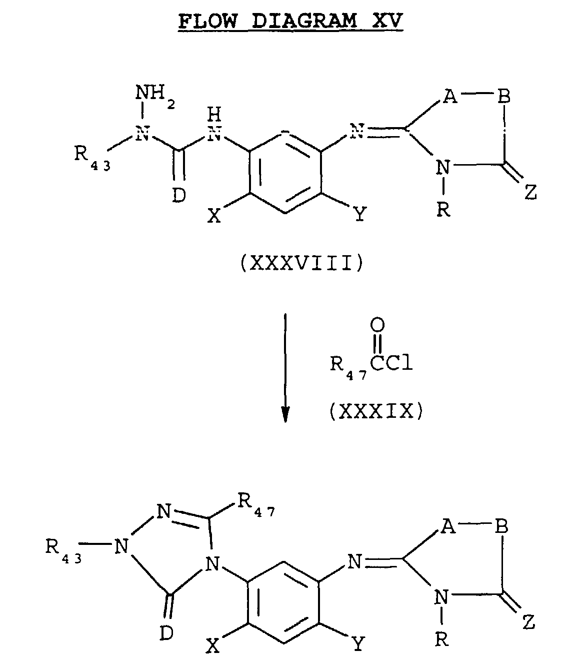

Formula I compounds wherein Q is Q38 may be prepared by reacting an amine of formula II with a chloride compound of formula XL to form an intermediate compound of formula XLI, and reacting the intermediate compound with hydrogen sulfide, hydrogen chloride and sodium periodate. The reaction scheme is shown in Flow Diagram XVI.

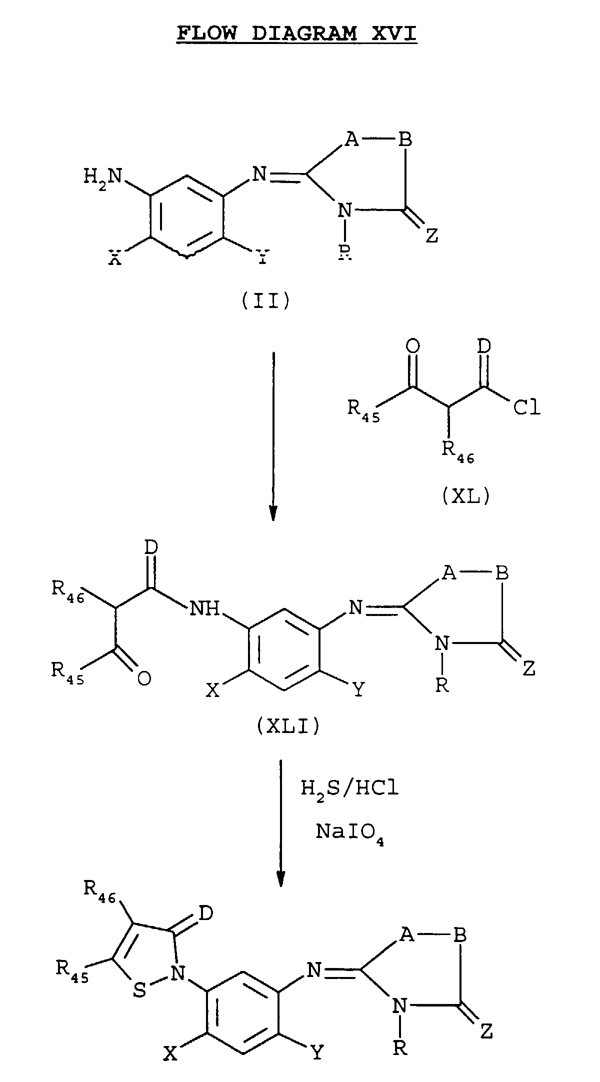

Compounds of formula I wherein Q is Q39 may be prepared by diazotizing an amine of formula II using conventional methods to form an intermediate compound, reducing the intermediate compound with sodium sulfite to form a hydrazine of formula XLII, sequentially reacting the hydrazine with an acid chloride of formula XLIII and phosphorous pentasulfide to form a substituted hydrazine of formula XLIV, and reacting the formula XLIV compound with phosgene or thiophosgene. The reaction scheme is shown in Flow Diagram XVII.

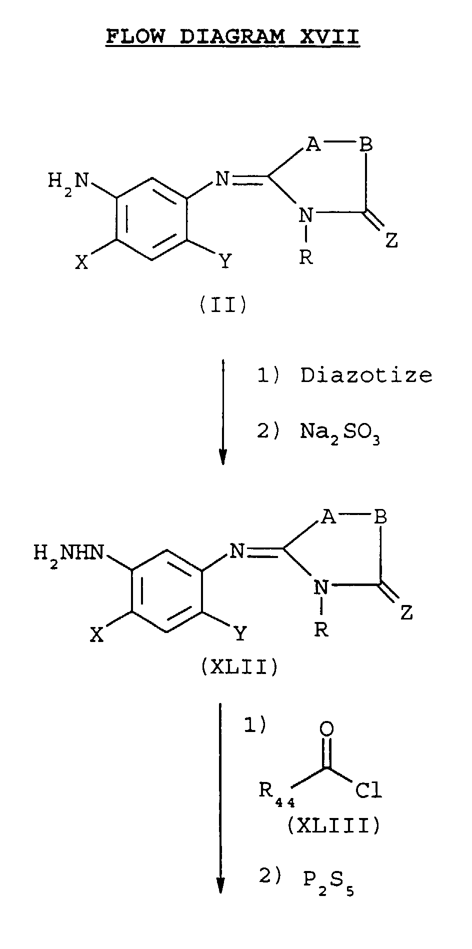

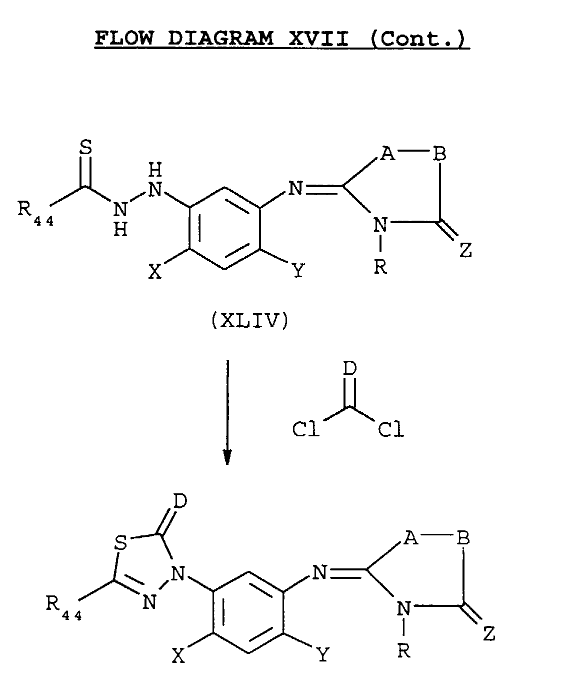

Certain formula II compounds wherein A is S, Z is O, and B is CR₃₇R₃₈ may be prepared by reacting an aniline of formula XLV with nitric acid and sulfuric acid to form a 3-nitroaniline of formula XLVI, reacting the 3-nitroaniline with thiophosgene to form an isothiocyanate of formula XLVII, reacting the isothiocyanate with an amine of formula XLVIII to form a thiourea of formula XLIX, reacting the thiourea with an α-halo ester of formula L optionally in the presence of a base to form an intermediate compound of formula LI, and reducing the formula LI compound using conventional procedures. The reaction scheme is shown below in Flow Diagram XVIII.

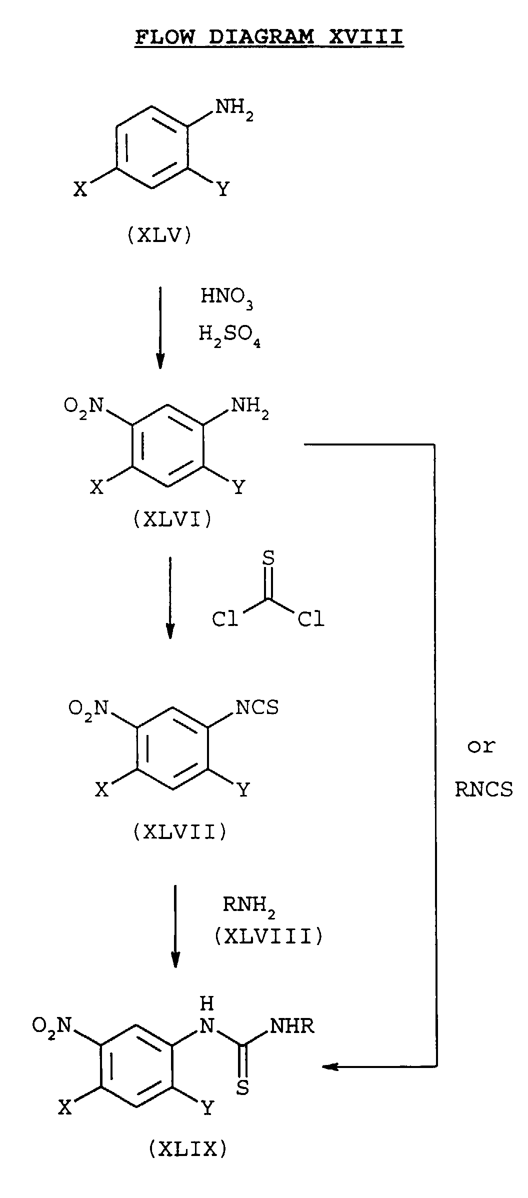

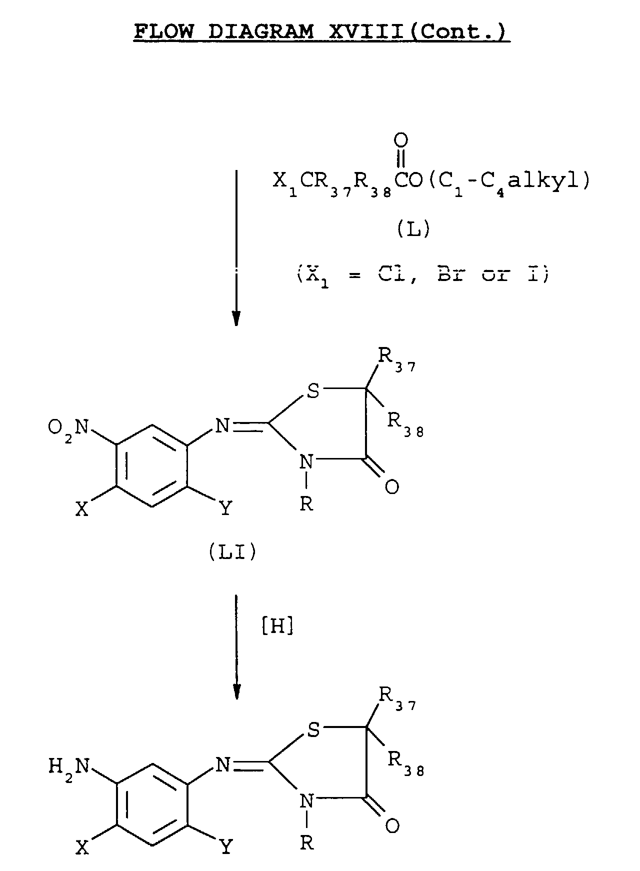

Certain formula II compounds wherein A is S, Z is O, B is -C(=T)-, and T is O may be prepared, as shown in Flow Diagram XIX, by reacting a thiourea of formula XLIX with oxalyl chloride to form an intermediate compound of formula LII, and reducing the formula LII compound using conventional procedures.

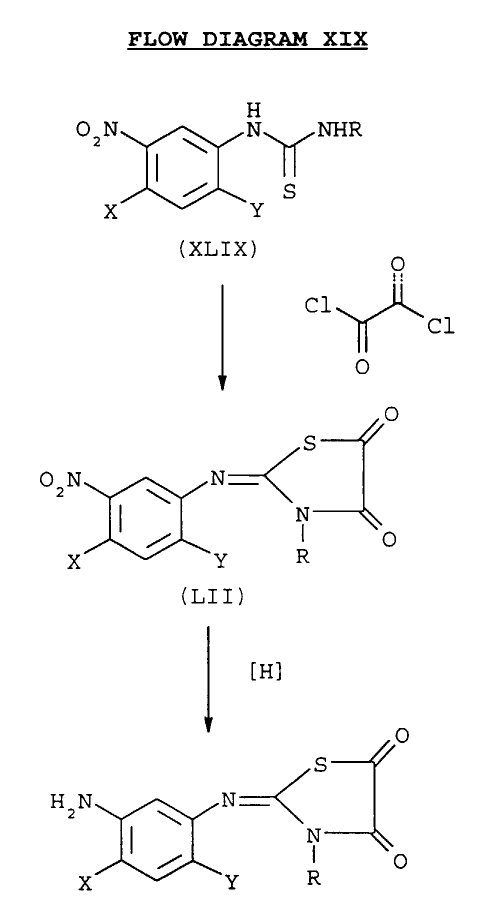

Certain compounds of formula II wherein A is S, B is -C(=CR₄₁R₄₂)-, and Z is O may be prepared, as show in Flow Diagram XX, by reacting a thiourea of formula XLIX with an α-halo ester of formula LIII to form a compound of formula LIV, reacting the formula LIV compound with a ketone or aldehyde of formula LV in the presence of a base to form a compound of formula LVI, and reducing the formula LVI compound using conventional procedures.

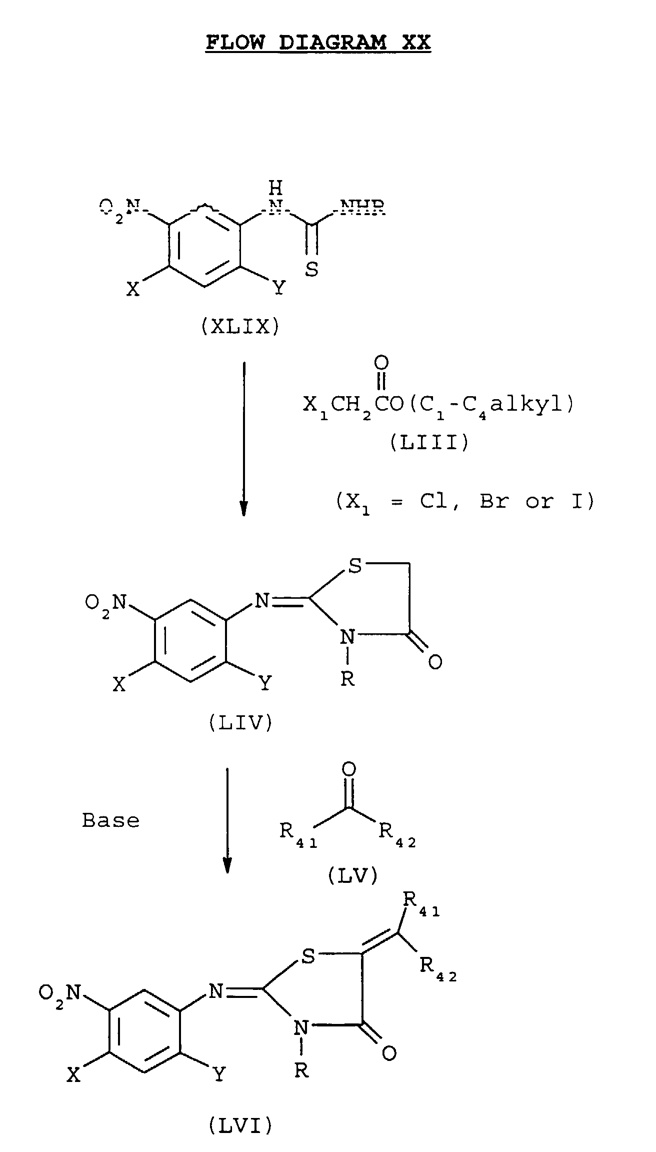

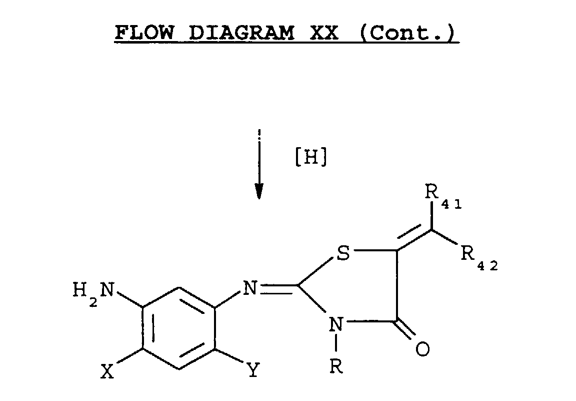

Certain formula II compounds wherein A is O, S or NR₃₄, B is CR₃₇R₃₈ and Z is O may be prepared by reacting a thiourea of formula XLIX with triethylamine, methanesulfonyl chloride and 4-dimethylaminopyridine to form a compound of formula LVII, cyclizing the formula LVII compound with an ester compound of formula LVIII in the presence of copper(I)chloride when A is O to form a compound of formula LIX, and reducing the formula LIX compound using conventional procedures. The reaction scheme is shown below in Flow Diagram XXI.

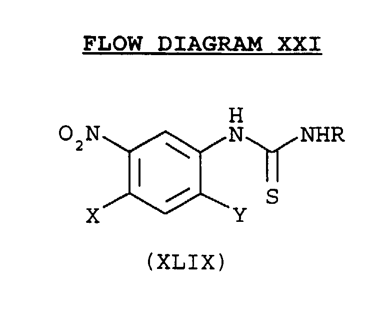

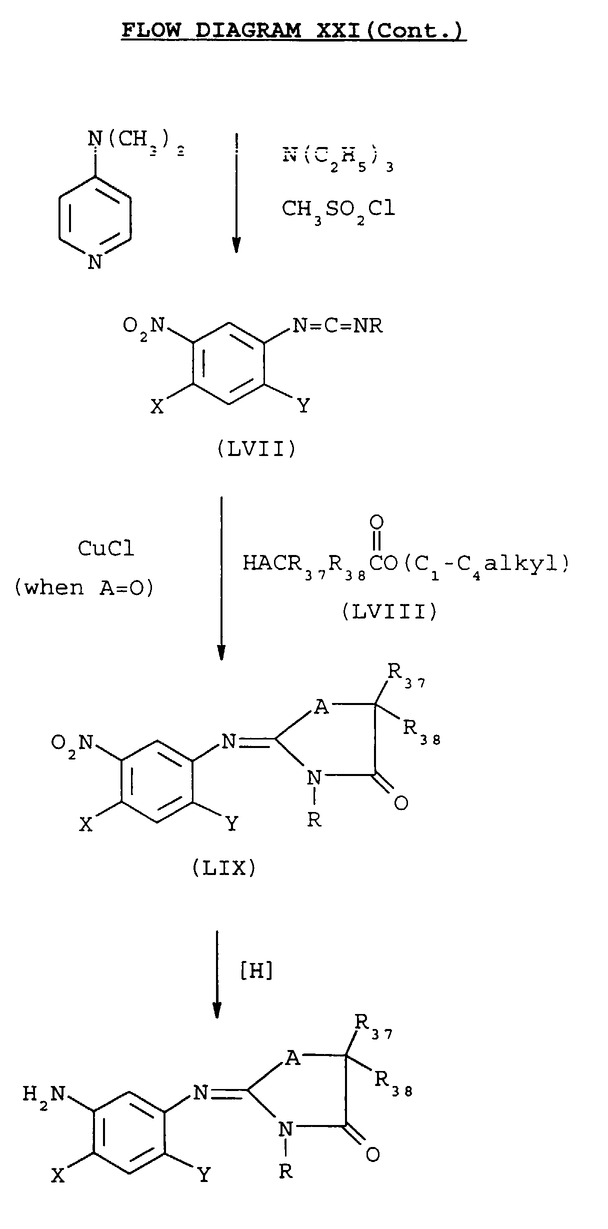

Formula II compounds wherein B is -C(=CR₄₂R₄₃)- and Z is O may be prepared, as shown in Flow Diagram XXII, by reacting a formula LIX compound wherein R₃₇ and R₃₈ are hydrogen with a ketone or aldehyde of formula LV in the presence of a base to form an intermediate compound of formula LX, and reducing the intermediate compound using conventional procedures.

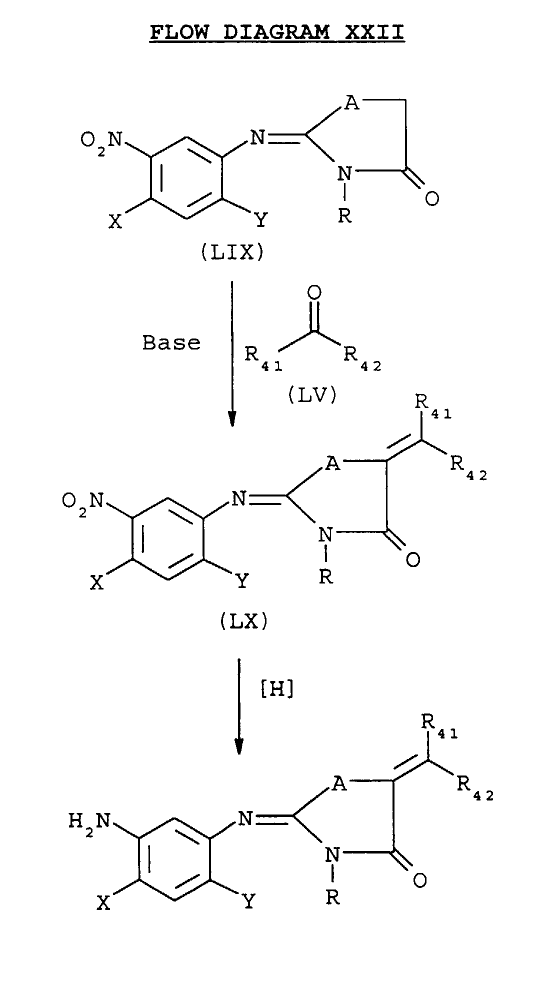

Alternatively, compounds of formula XLIX may be prepared as shown below in Flow Diagram XXIII.

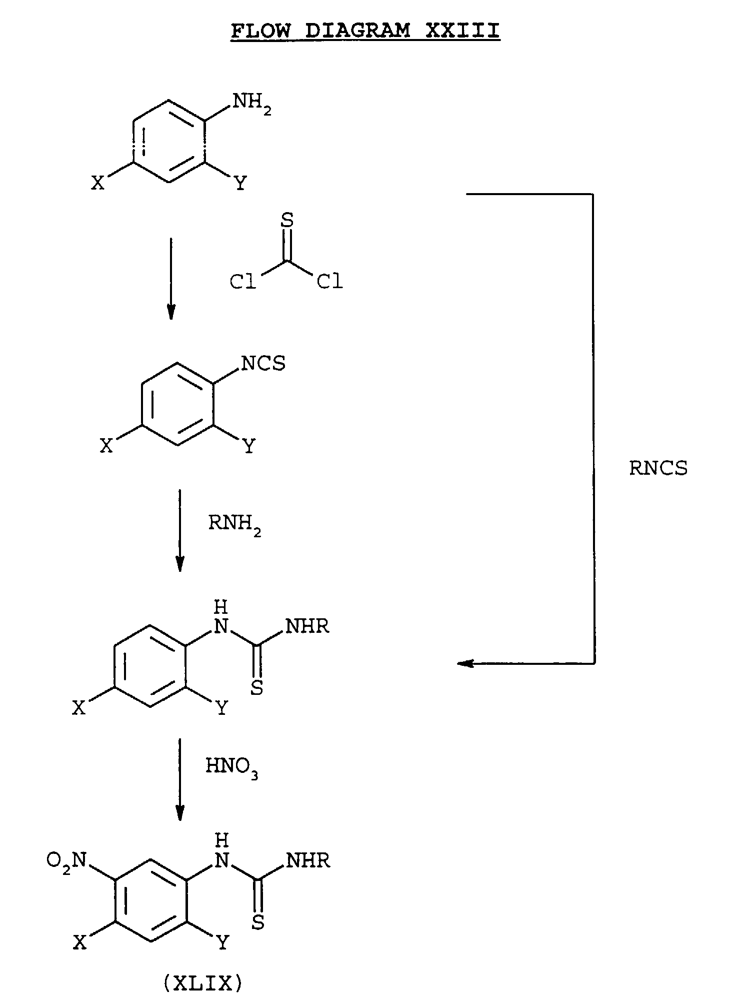

Intermediate compounds of formula LIX may also be prepared as shown below in Flow Diagram XXIV.

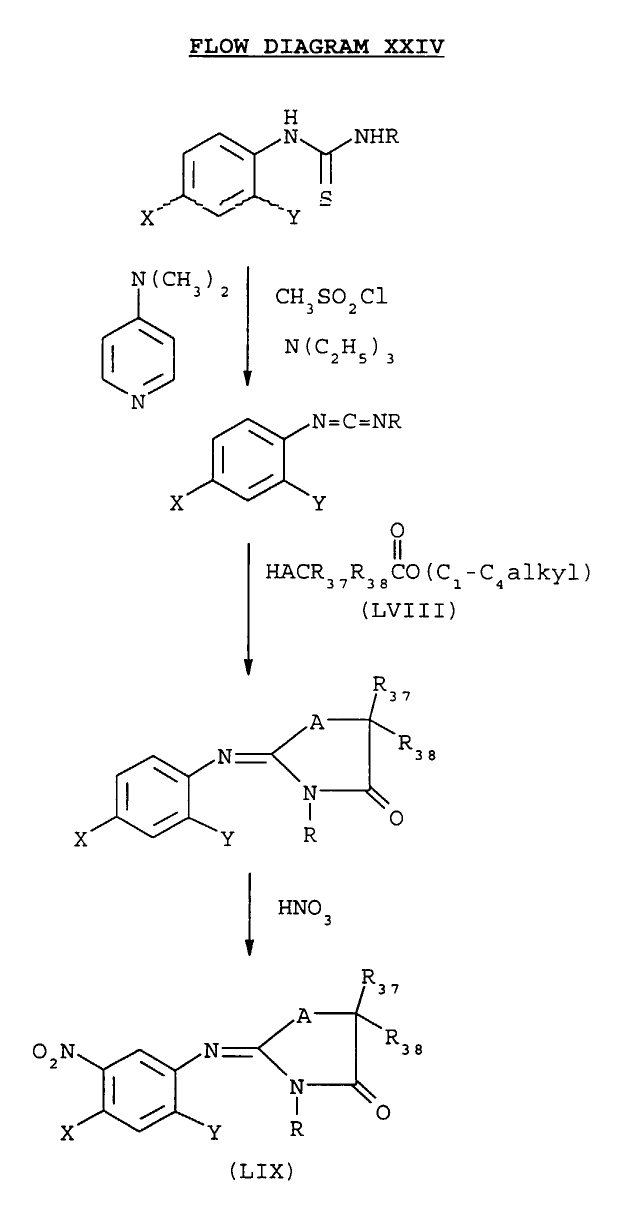

Formula I compounds wherein Q is Q24, A is S, and B is CR₃₇R₃₈ may also be prepared as shown below in Flow Diagram XXV.

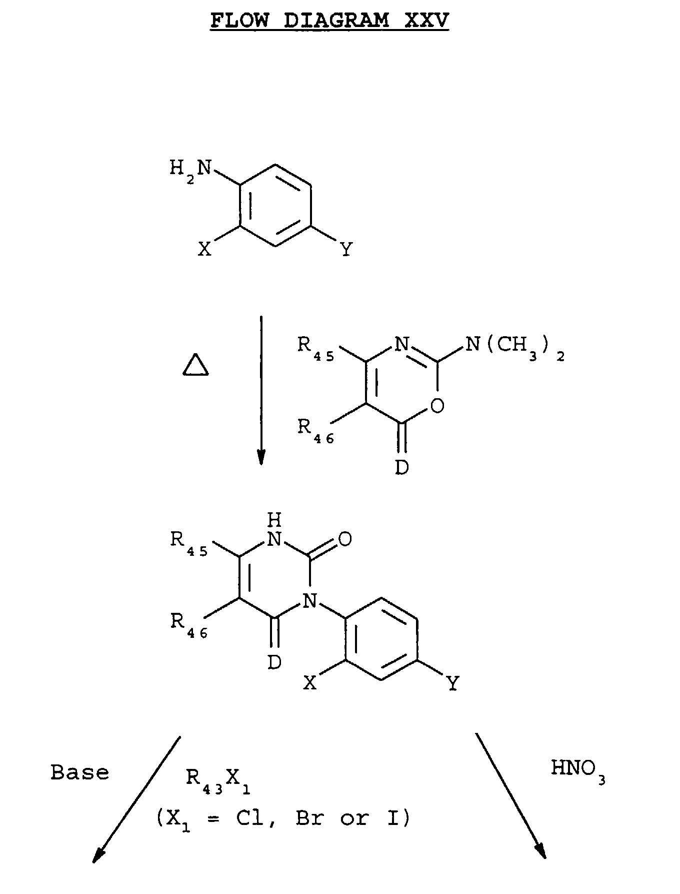

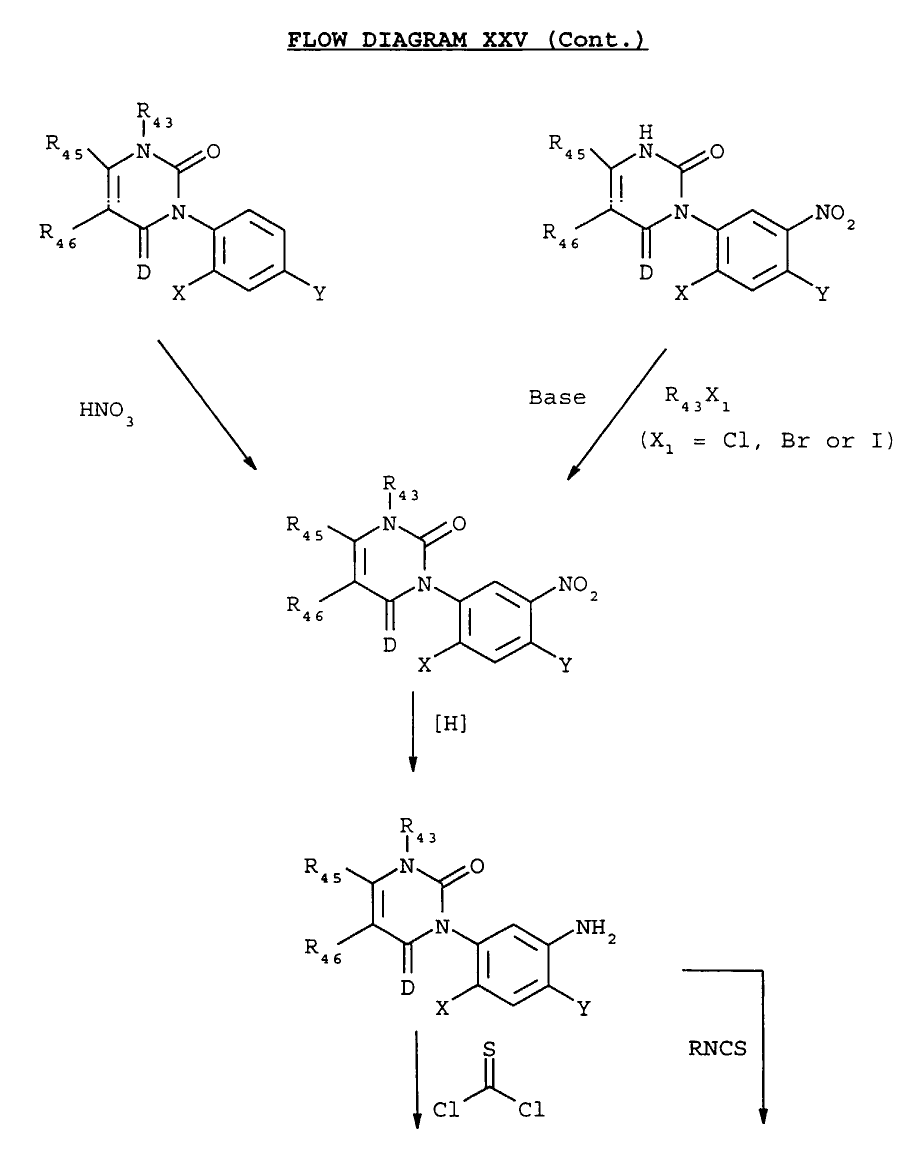

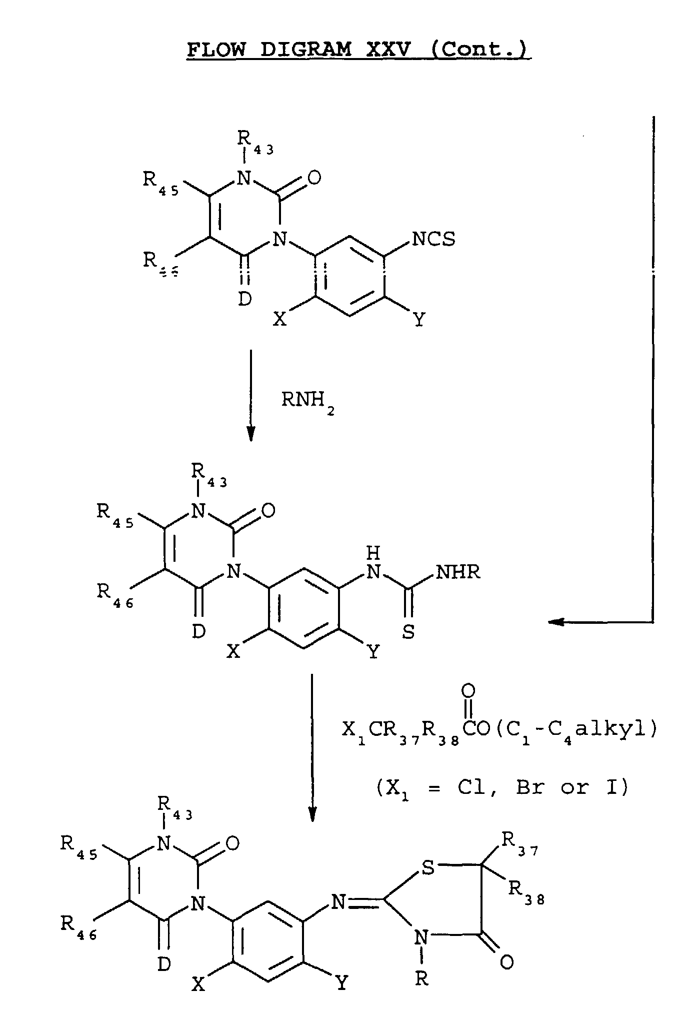

Alternatively, one of the intermediates used in the reaction scheme described in Flow Diagram XXV may be prepared as shown below in Flow Diagram XXVI.

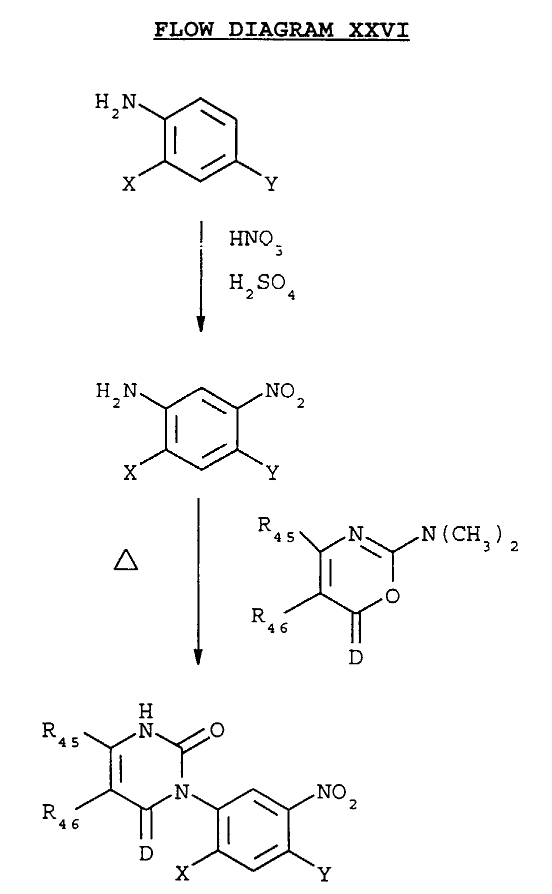

Formula I compounds wherein A is S, B is CR₃₇R₃₈, Z is O, and Q is Q1-Q32, Q34 and Q36-Q39 may also be prepared by substituting an aniline of formula LXI or a 3-nitroaniline of formula LXII for the formula II amines used hereinabove to obtain compounds of formula LXIII, and converting the formula LXIII compounds to the desired formula I compounds as shown below in Flow Diagram XXVII.

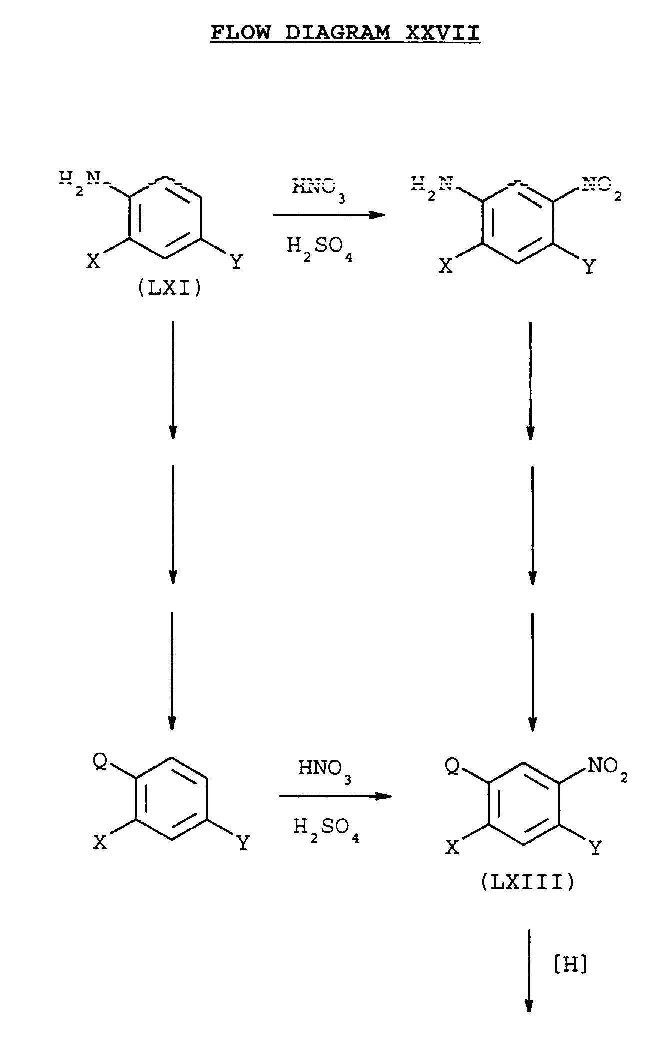

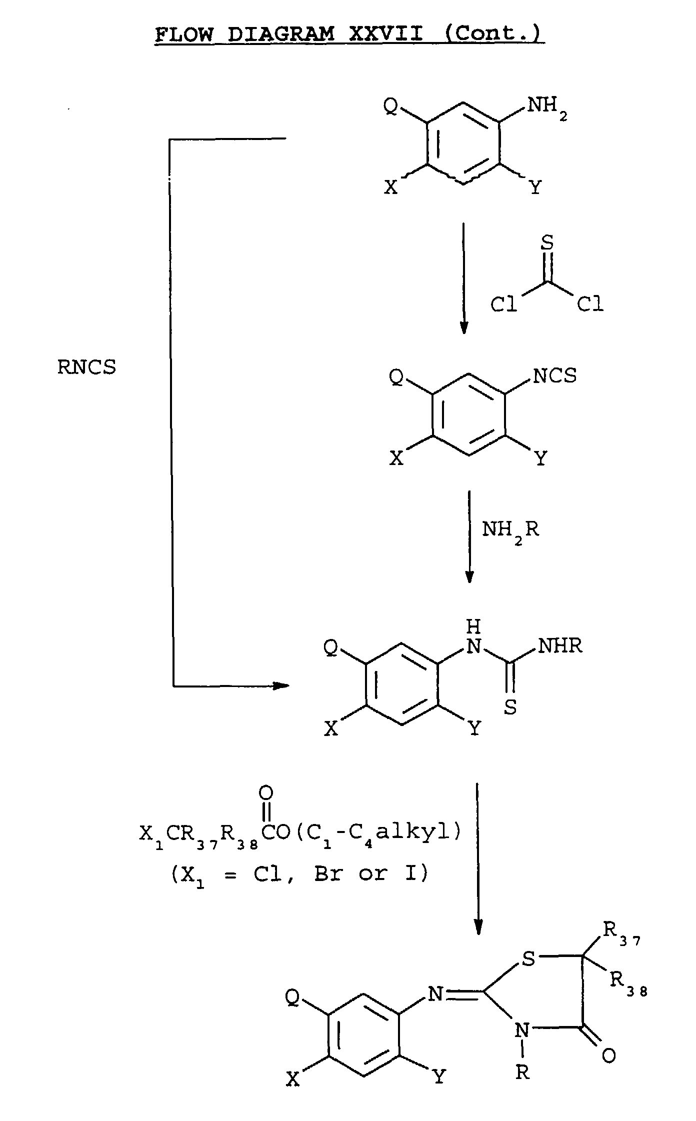

Alternatively, certain formula I compounds wherein Q is Q1-Q32, Q34 and Q36-Q39, B is CR₃₇R₃₈, and Z is O may be prepared, as shown below in Flow Diagram XXVIII, by reacting a thiourea of formula LXIV with methanesulfonyl chloride, triethylamine and 4-dimethylaminopyridine to form an intermediate compound of formula LXV, and reacting the intermediate compound with an ester of formula LVIII and copper(I)chloride when A is O.

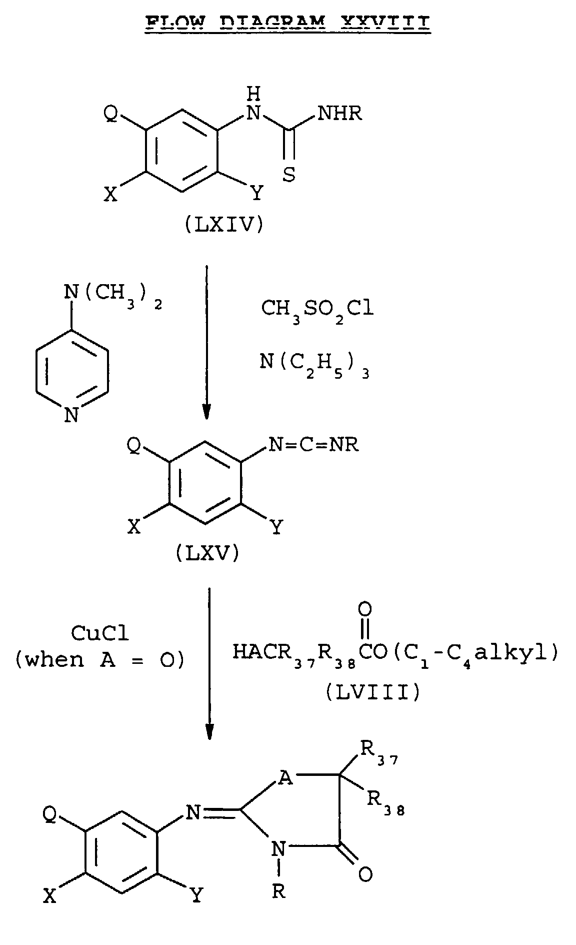

Compounds of formula I wherein A is S, B is -C(=T)-, and T and Z are O may be prepared, as shown in Flow Diagram XXIX, by reacting a thiourea of formula LXIV with oxalyl chloride.

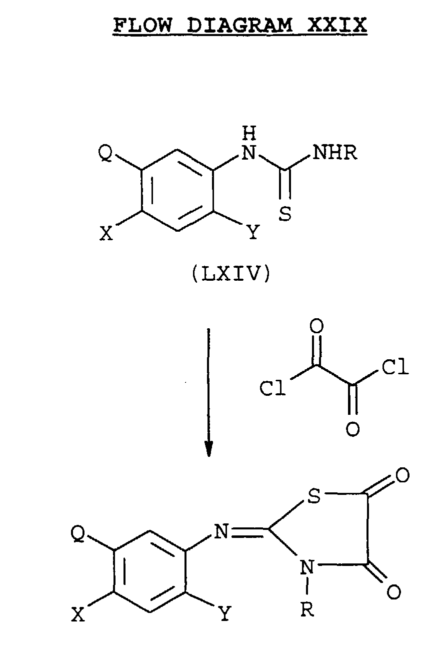

Formula I compounds wherein A is S, B is -C(=CR₄₁R₄₂)-, and Z is O may be prepared as shown below in Flow Diagram XXX.

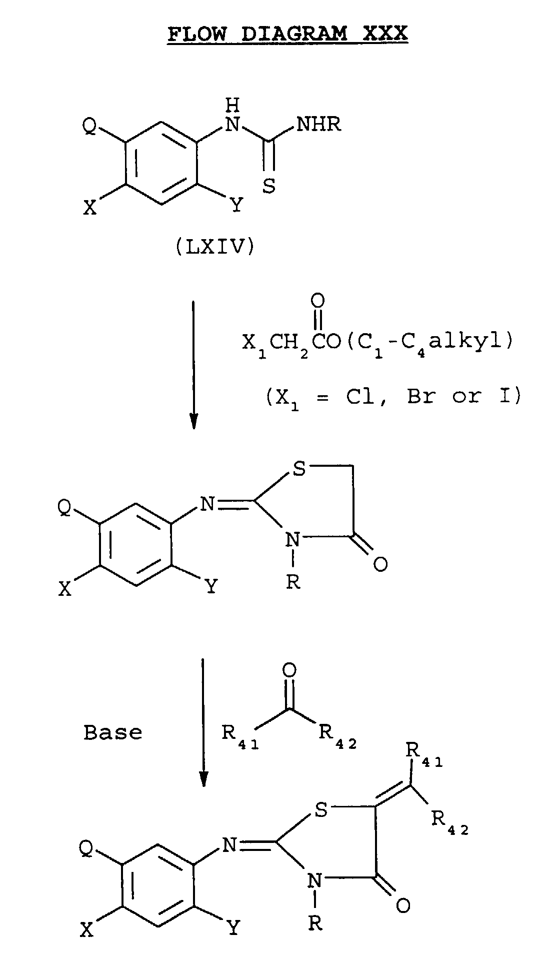

Compounds of formula I wherein R is NR₁₈R₁₉, A is S, B is CR₃₇R₃₈, and Z is O may be prepared, as shown in Flow Diagram XXXI, by reacting an isothiocyanate of formula LXVI with a hydrazine of formula LXVII to form a thiourea of formula LXVIII and reacting the thiourea with an α-halo ester of formula L optionally in the presence of a base.

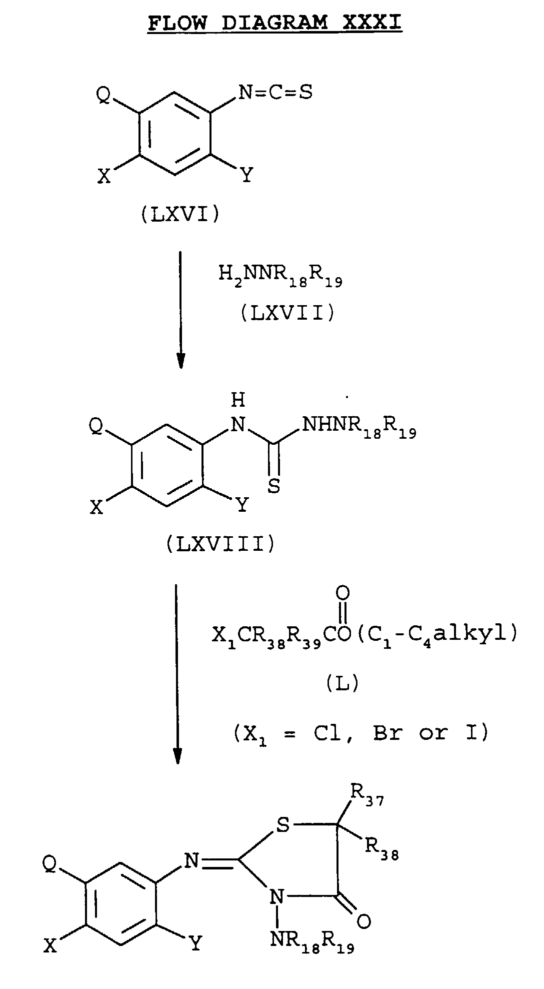

Alternatively, formula I compounds wherein R is NR₁₈R₁₉ may be prepared as shown below in Flow Diagram XXXII.

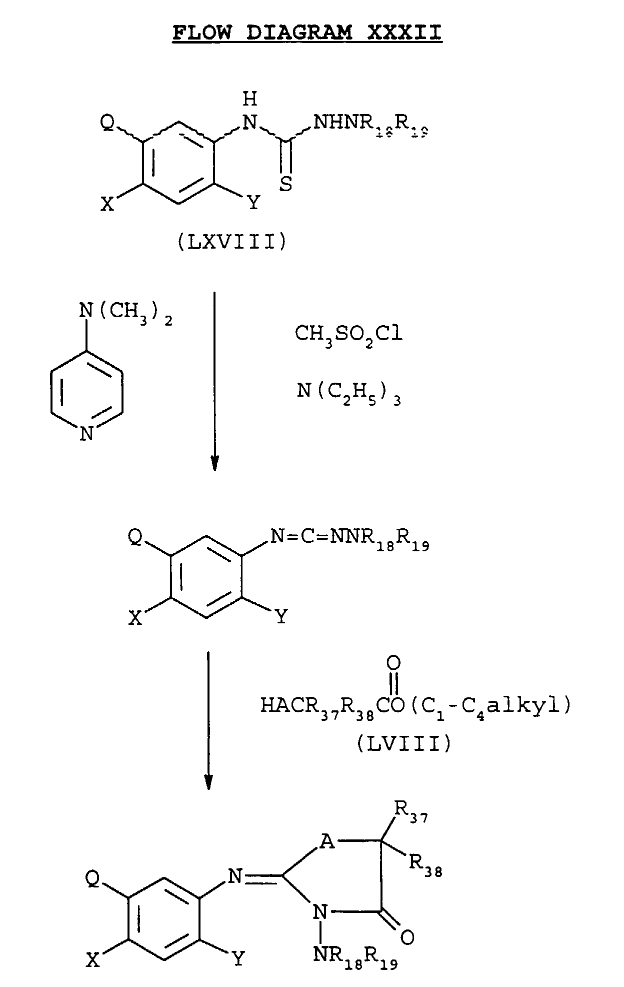

Formula I compounds wherein R is OR₁₇ may be prepared, as shown in Flow Diagram XXXIII, by reacting an isocyanate of formula LXVI with an amine of formula LXIX to form a thiourea of formula LXX, and reacting the thiourea with an α-halo ester of formula L.

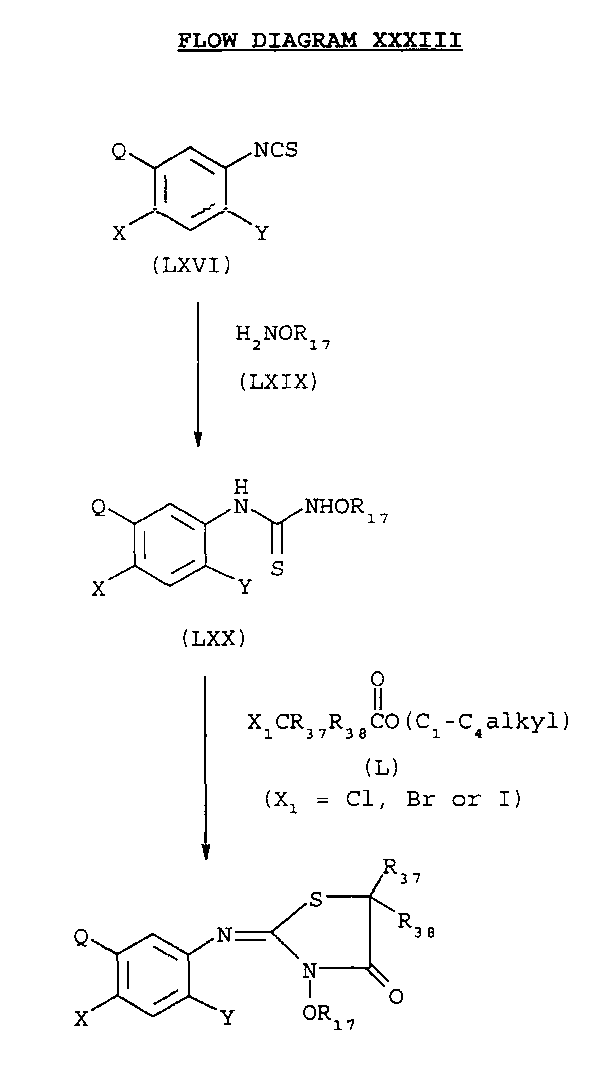

Formula I compounds wherein Q is Q24 and R₄₆ is hydrogen may be prepared as shown below in Flow Diagram XXXIV.

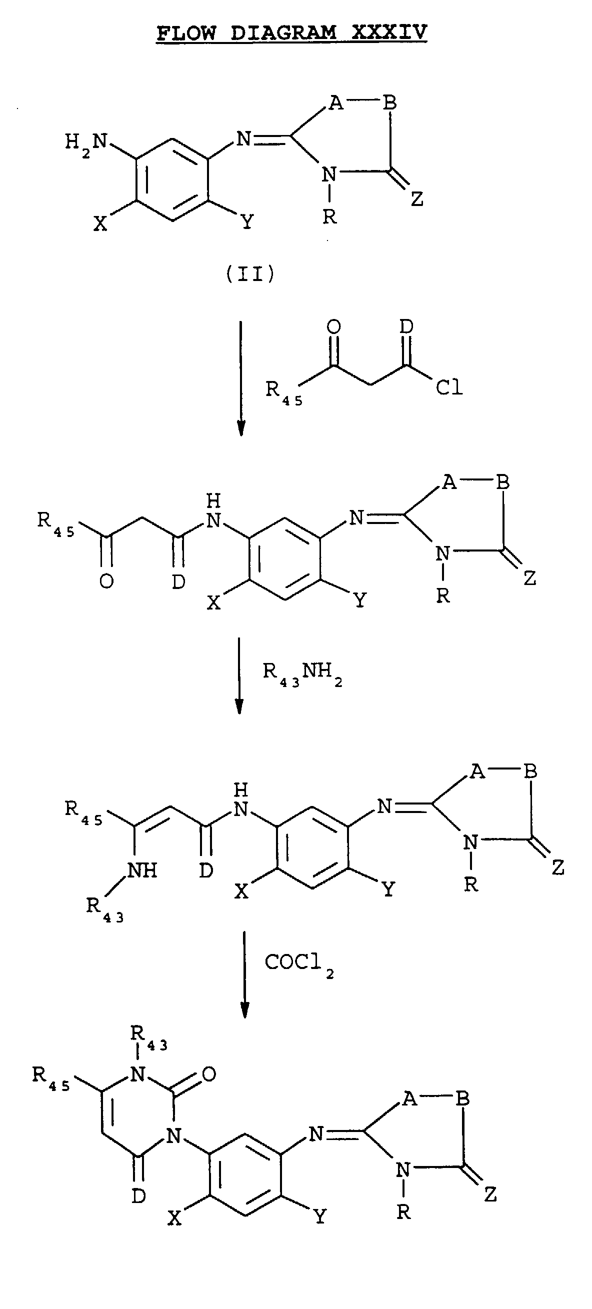

Other methods for the preparation of formula I compounds will become apparent from the examples set forth below. In addition, certain compounds of formula I may be converted into other compounds of formula I by using conventional procedures known to those skilled in the art.

The present invention also relates to intermediate compounds having the structural formula LXXI 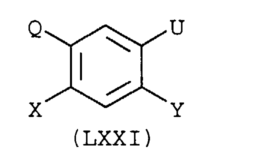 wherein U is -N=C=NR, -N=C=S or 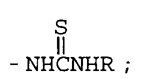 Q is Q5, Q7, Q24 or Q29 as described hereinabove; and R, X and Y are as described hereinabove.

Preferred formula LXXI compounds are those wherein U is -N=C=NR, -N=C=S or 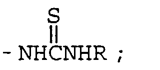 Q is 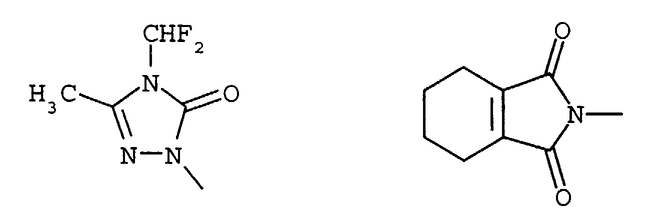 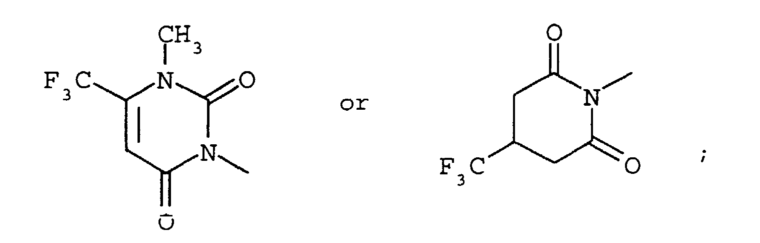
- X is: hydrogen, fluorine or chlorine;
- Y is: fluorine, chlorine, nitro or cyano; and
- R is: hydrogen,
C₁-C₆alkyl optionally substituted with one to three halogen atoms, one or two C(O)OR₃ groups, one cyano group, one OR₇ group, one SR₈ group, one P(O) (OR₆)₂ group, or
a C₃-C₆alkenyl, C₃-C₆alkynyl, phenyl, benzyl or furfural group, wherein each group is optionally substituted with one to three halogen atoms, one C₁-C₃alkoxy group or one C(O)OR₃ group;
- R₃ is: hydrogen, C₁-C₆alkyl or C₁-C₆haloalkyl;
- R₆ is: hydrogen or C₁-C₃alkyl;
- R₇ is: hydrogen, C₁-C₃alkyl or C₃-C₆alkoxycarbonylalkyl; and
- R₈ is: hydrogen, C₁-C₃alkyl or C₃-C₆alkoxycarbonyl.

In order to facilitate a further understanding of the invention, following examples are presented to illustrate more specific details thereof. This invention is not to be limited thereby except as defined in the claims.

### EXAMPLE 1

### Preparation of 4-Chloro-2-fluoro-5-nitroaniline

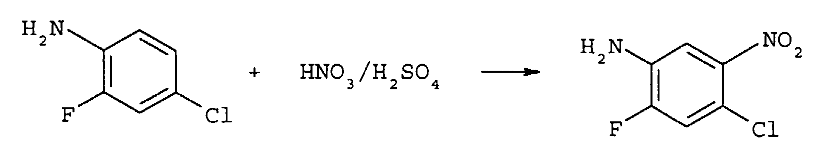

A solution of 4-chloro-2-fluoroaniline (10.0 g, 69 mmol) in concentrated sulfuric acid is cooled to -20°C, treated dropwise with 90% nitric acid (5.0 mL, 107 mmol) over ten minutes, stirred at -15°C for 90 minutes, and poured onto ice. The resultant aqueous mixture is extracted with diethyl ether, neutralized with 50% sodium hydroxide solution, and extracted again with diethyl ether. The organic extracts are combined, washed sequentially with dilute sodium hydroxide solution and brine, dried over anhydrous magnesium sulfate, and concentrated in vacuo to give the title product as a brown solid (11.3 g, 86% yield, mp 82-83.5°C) which is identified by ¹H, ¹⁹F and ¹³C NMR spectral analyses.

Using essentially the same procedure, but substituting 2,4-dichloroaniline for 4-chloro-2-fluoroaniline, 2,4-dichloro-5-nitroaniline is obtained.

### EXAMPLE 2

### Preparation of 3-(4-chloro-2-fluoro-5-nitrophenyl)-6-(trifluoromethyl)-2,4(1H,3H)-pyrimidinedione

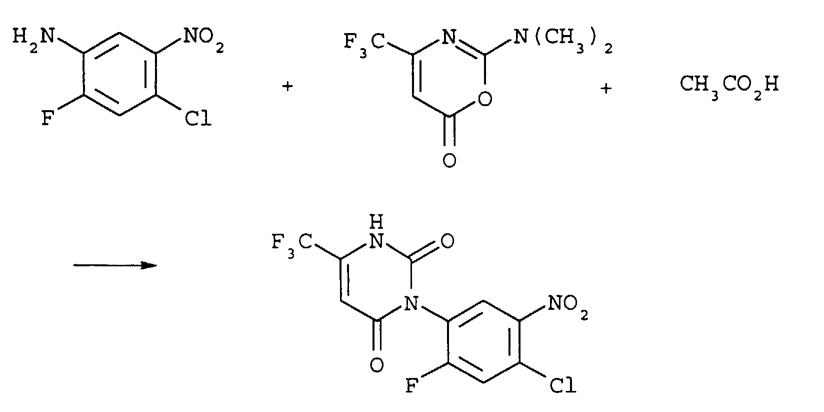

A solution of 4-chloro-2-fluoro-5-nitroaniline (5.0 g, 26.3 mmol) and 2-dimethylamino-4-(trifluoromethyl)-6H-1,3-oxazin-6-one (5.73 g, 27.6 mmol) in acetic acid is refluxed for 4 hours and poured onto ice. The resultant aqueous mixture is filtered to obtain a solid. The solid is washed with water and air-dried to give the title product as a beige solid (7.5 g, 81% yield, mp 230-232°C) which is identified by ¹H, ¹³C and ¹⁹F NMR spectral analyses.

Using essentially the same procedure, but substituting 2,4-dichloro-5-nitroaniline for 4-chloro-2-fluoro-5-nitroaniline, 3-(2,4-dichloro-5-nitrophenyl)-6-(trifluoromethyl)-2,4(1H,3H)-pyrimidinedione, mp 188.5-190°C, is obtained.

### EXAMPLE 3

### Preparation of 3-(4-Chloro-2-fluoro-5-nitrophenyl)-1-methyl-6-(trifluoromethyl)-2,4(1H,3H)-pyrimidinedione

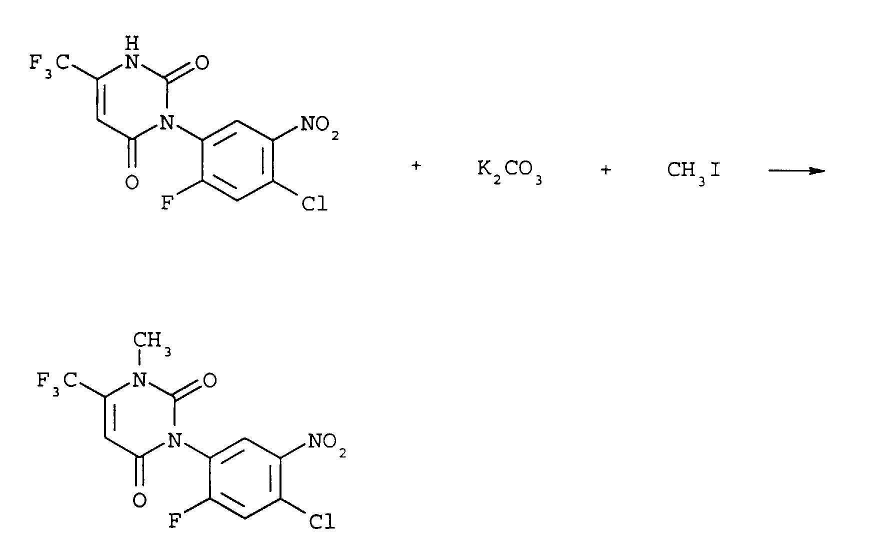

A solution of 3-(4-chloro-2-fluoro-5-nitrophenyl)-6-trifluoromethyl)-2,4(1H,3H)-pyrimidinedione (5.66 g, 16 mmol) in N,N-dimethylformamide is treated sequentially with potassium carbonate (2.43 g, 17.6 mmol) and iodomethane (1.1 mL, 17.6 mmol), stirred overnight at room temperature, and poured into water. After acidifying the resultant aqueous mixture to pH 5, the solids are collected, washed with water and dried to obtain a dark, brown solid. The solid is dissolved in methylene chloride and the resultant solution is washed sequentially with water and brine, passed through a silica gel plug, and concentrated *in vacuo* to give the title product as a light, brown solid (4.7 g, 80% yield, mp 124-126.5°C) which is identified by ¹H and ¹³C NMR spectral analyses.

Using essentially the same procedure, but substituting 3-(2,4-dichloro-5-nitrophenyl)-6-(trifluoromethyl)-2,4(1H,3H)-pyrimidinedione for 3-(4-chloro-2-fluoro-5-nitrophenyl)-6-(trifluoromethyl)-2,4(1H,3H)-pyrimidinedione, 3-(2,4-dichloro-5-nitrophenyl)-1-methyl-6-(trifluoromethyl)-2,4(1H,3H)-pyrimidinedione, mp 127-128.5°C, is obtained.

### EXAMPLE 4

### Preparation of 3-(5-amino-4-chloro-2-fluorophenyl)-1-methyl-6-(trifluoromethyl)-2,4(1H,3H)-pyrimidinedione

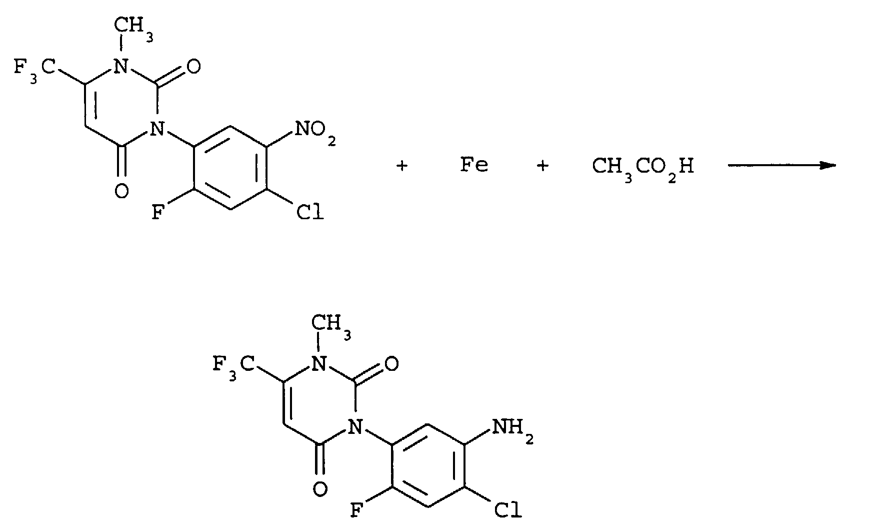

A solution of 3-(4-chloro-2-fluoro-5-nitrophenyl)-1-methyl-6-(trifluoromethyl)-2,4(1H,3H)-pyrimidinedione (3.00 g, 8.16 mmol) in acetic acid is heated to 50-60°C, treated portionwise with iron powder (2.28 g, 40.8 mmol) over 10 minutes, stirred at 50-60°C for 90 minutes, cooled to room temperature, and diluted with water. The resultant aqueous mixture is filtered to remove solids and extracted with diethyl ether. The organic extracts are combined, washed sequentially with 1 M sodium hydroxide solution and brine, dried over anhydrous magnesium sulfate, and concentrated *in vacuo* to give the title product as a brown solid (2.37 g, 86% yield, mp 132-135°C) which is identified by ¹H and ¹³C NMR spectral analyses.

Using essentially the same procedure, but substituting 3-(2,4-dichloro-5-nitrophenyl)-1-methyl-6-(trifluoromethyl)-2,4(1H,3H)-pyrimidinedione for 3-(4-chloro-2-fluoro-5-nitrophenyl)-1-methyl-6-(trifluoromethyl)-2,4(1H,3H)-pyrimidinedione, 3-(5-amino-2,4-dichlorophenyl)-1-methyl-6-(trifluoromethyl)-2,4(1H,3H)-pyrimidinedione, mp 163-165°C, is obtained.

### EXAMPLE 5

### Preparation of N-(4-chloro-2-fluorophenyl)-1-cyclohexene-1,2-dicarboximide

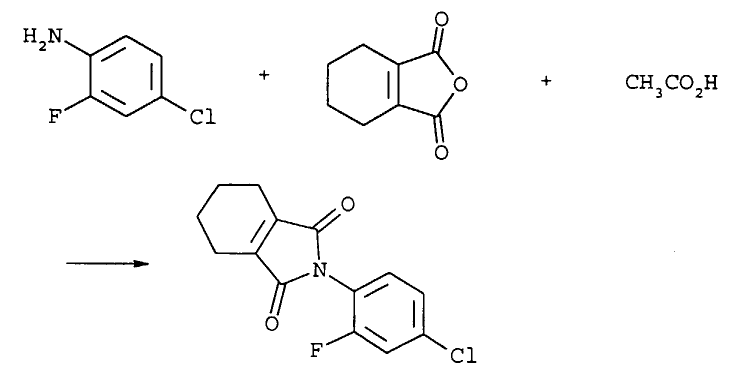

A solution of 4-chloro-2-fluoroaniline (19.0 g, 130.6 mmol) and 3,4,5,6-tetrahydrophthalic anhydride (19.85 g, 130.6 mmol) in acetic acid is refluxed for 2 hours, treated with additional 4-chloro-2-fluoroaniline (3.0 g), refluxed for 90 minutes, stirred at room temperature overnight and poured into water. The resultant aqueous mixture is extracted with ethyl acetate. The organic extract is washed sequentially with water, saturated sodium hydrogen carbonate solution and brine, dried over anhydrous magnesium sulfate, and concentrated *in vacuo* to obtain a purple oil. Flash column chromatography of the oil using silica gel and 15 to 20% ethyl acetate in hexanes solutions gives the title product as an off-white solid (25.3 g, 69% yield, mp 81-82°C) which is identified by ¹H, ¹³C and ¹⁹F NMR spectral analyses.

### EXAMPLE 6

### Preparation of N-(4-chloro-2-fluoro-5-nitrophenyl)-1-cyclohexene-1,2-dicarboximide

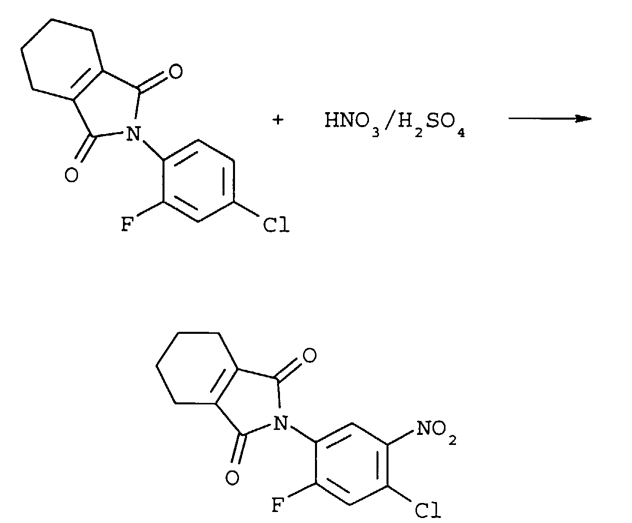

A mixture of N-(4-chloro-2-fluorophenyl)-1-cyclohexene-1,2-dicarboximide (24.4 g, 86.7 mmol) in sulfuric acid is cooled to -3°C, treated dropwise with 70% nitric acid (6.7 mL, 104.0 mmol) while maintaining the temperature from 0° to 2°C, warmed to and stirred at room temperature for 90 minutes, and poured onto ice. The resultant aqueous mixture is filtered to obtain a solid. The solid is washed with water and air-dried overnight to give the title product as a white powder (28.68 g, mp 152-155°C) which is identified by ¹H, ¹³C and ¹⁹F NMR spectral analyses.

### EXAMPLE 7

### Preparation of N-(5-amino-4-chloro-2-fluorophenyl)-1-cyclohexene-1,2-dicarboximide

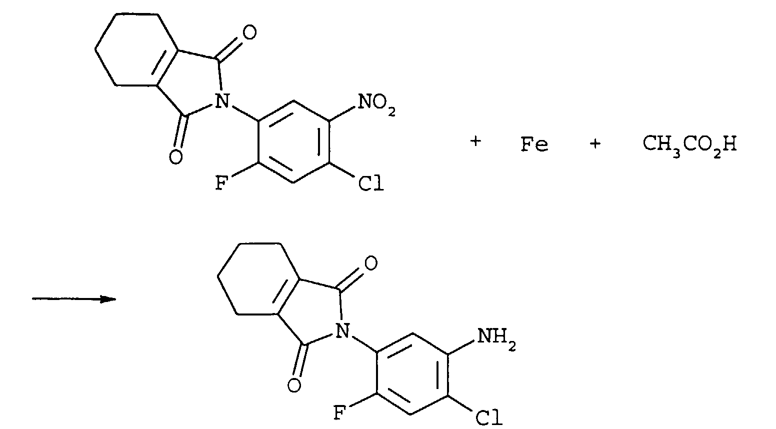

A mixture of N-(4-chloro-2-fluoro-5-nitrophenyl)-1-cyclohexene-1,2-dicarboximide (10.6 g, 32.7 mmol) in acetic acid is heated to 65°C, treated portionwise with iron powder (7.30 g, 130.7 mmol) over 40 minutes, stirred for 10 minutes, and filtered through diatomaceous earth. The resultant filtrate is washed with ethyl acetate, concentrated *in vacuo*, diluted with ethyl acetate and saturated sodium hydrogen carbonate solution, and filtered through a filter pad. The phases are separated and the organic phase is washed sequentially with saturated sodium hydrogen carbonate solution and brine, dried over anhydrous magnesium sulfate, and concentrated *in vacuo* to give the title product as a yellow solid (8.94 g, 94% yield, mp 164-165°C).

### EXAMPLE 8

### Preparation of N-{{2-chloro-5-[3,6-dihydro-3-methyl-2,6-dioxo-4-(trifluoromethyl)-1(2H)-pyrimidinyl]-4-fluoro-phenyl}(thiocarbamoyl)}glycine, ethyl ester

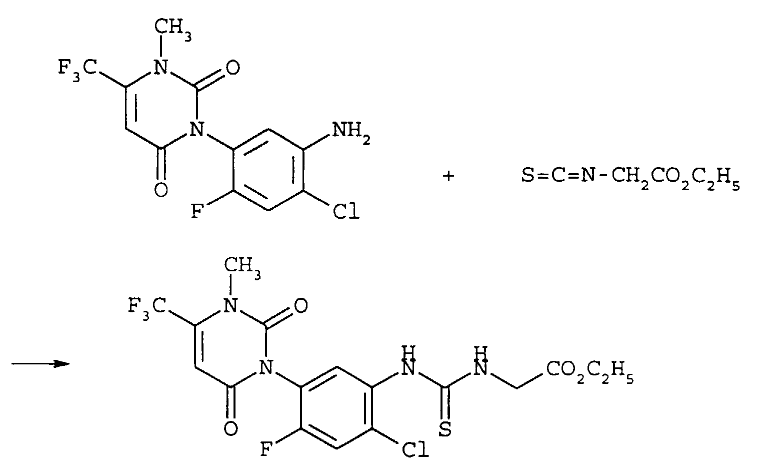

A solution of 3-(5-amino-4-chloro-2-fluorophenyl)-1-methyl-6-(trifluoromethyl)-2,4(1H,3H)-pyrimidinedione (3.0 g, 8.89 mmol) in a tetrahydrofuran/ethanol solution (1:1) is treated with ethyl isothiocyanatoacetate (1.93 g, 13.34 mmol), refluxed for 9 hours, stirred at room temperature overnight, and concentrated *in vacuo* to obtain an orange-brown gum. Flash column chromatography of the gum using silica gel and 25% to 30% ethyl acetate in hexanes solutions gives the title product as a white foam (2.24 g, 52% yield, mp 95-103°C) which is identified by ¹H, ¹³C and ¹⁹F NMR spectral analyses.

Using essentially the same procedure, the following compounds are obtained:

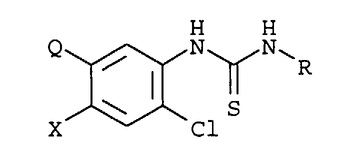

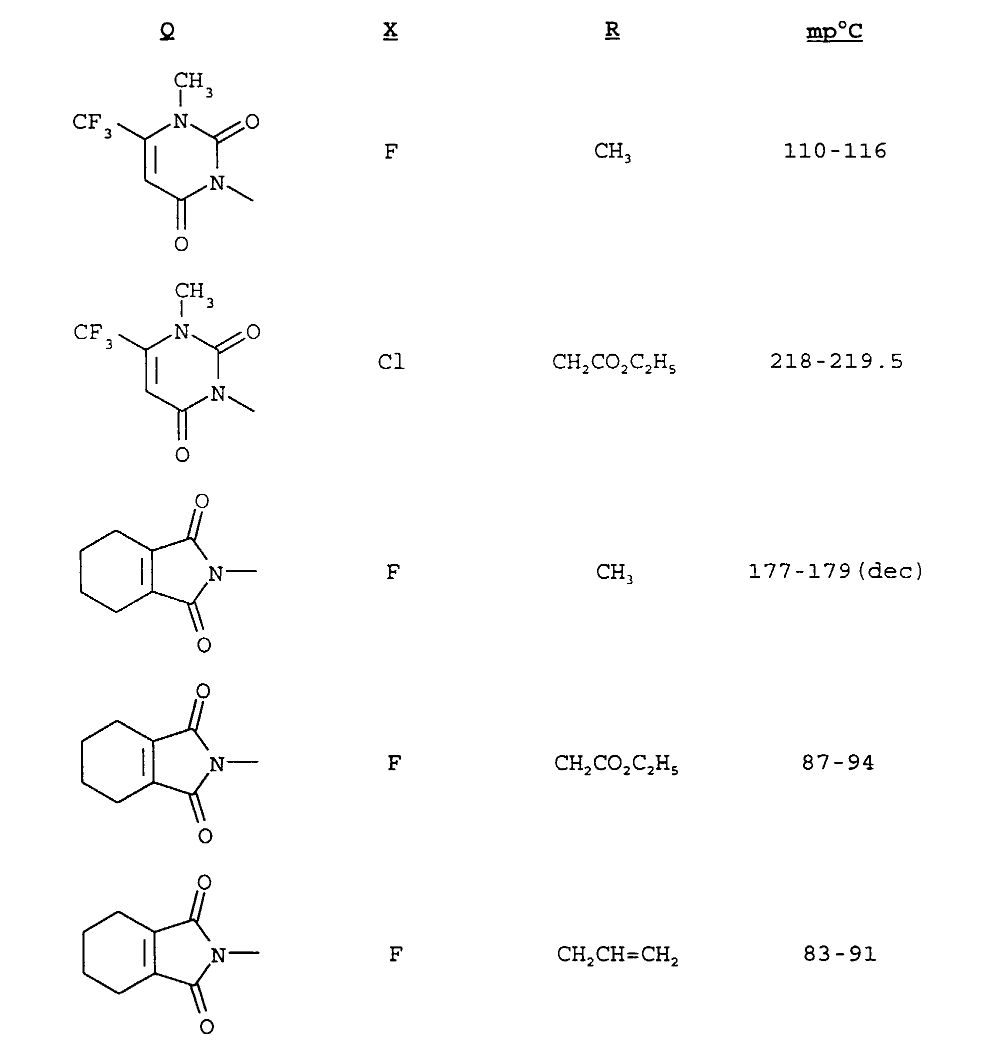

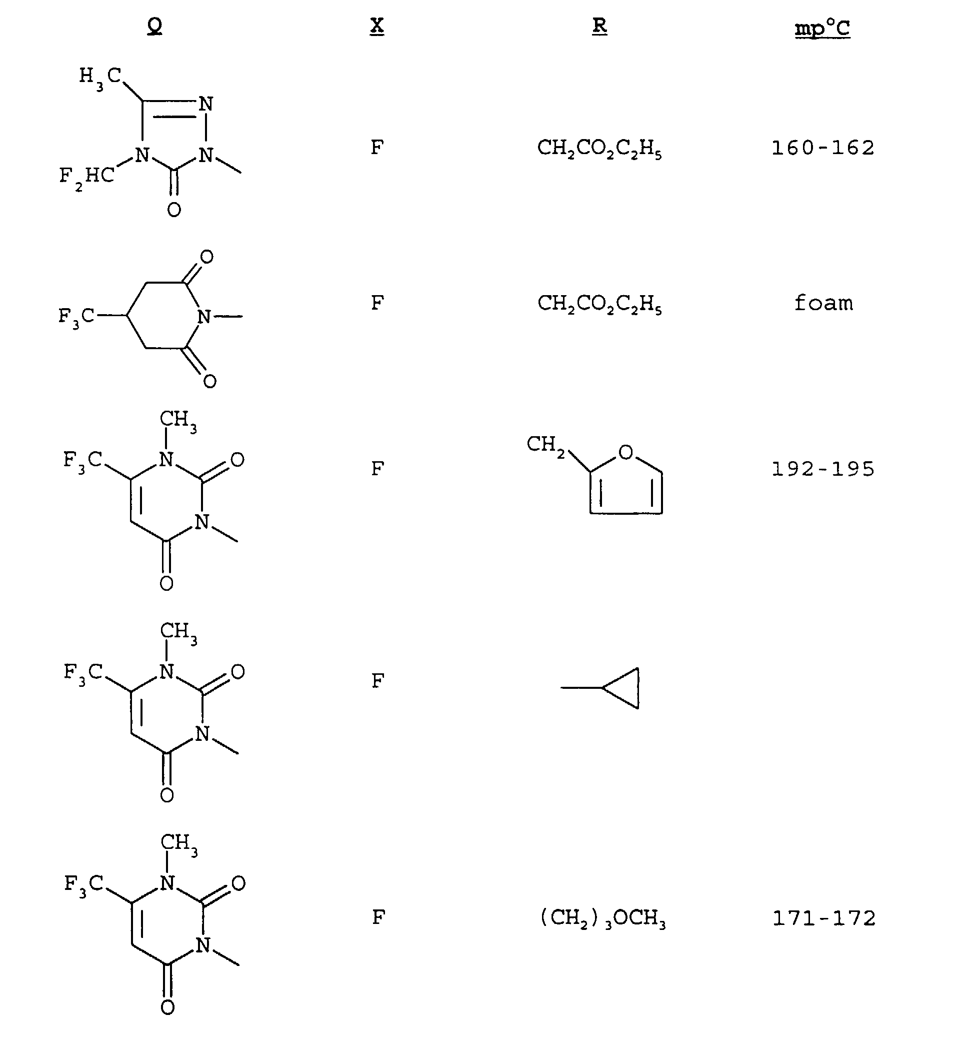

### EXAMPLE 9

### Preparation of 2-Chloro-5-[3,6-dihydro-3-methyl-2,6-dioxo-4-(trifluoromethyl)-1(2H)-pyrimidinyl]-4-fluorophenyl isothiocyanate

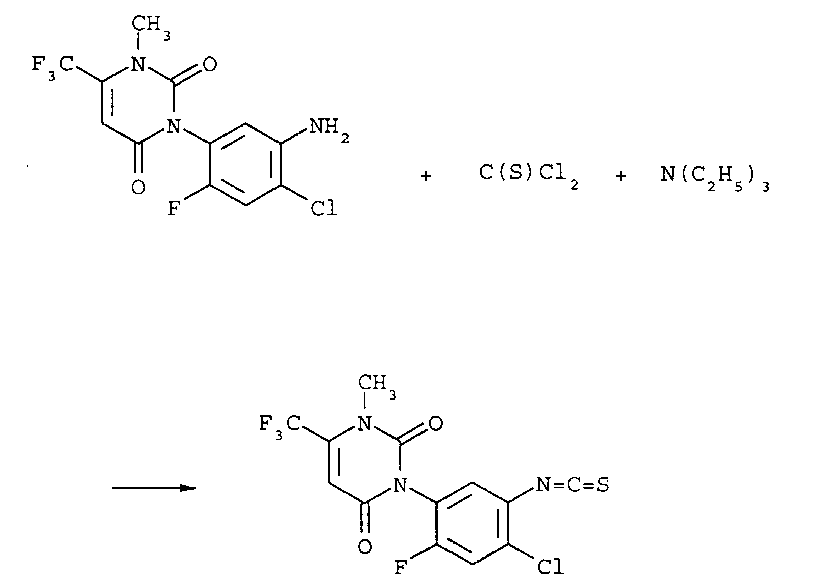

A mixture of 3-(5-amino-4-chloro-2-fluorophenyl)-1-methyl-6-(trifluoromethyl)-2,4(1H,3H)-pyrimidinedione (1.0 g, 2.96 mmol) in toluene is cooled to 0°C, treated sequentially with triethylamine (0.91 mL, 6.52 mmol) and thiophosgene (0.25 mL, 3.26 mmol), warmed to and stirred at room temperature for 5 hours, and filtered to remove solids. The resultant filtrate is concentrated *in vacuo* to give the title product as an orange solid (1.1 g, 98% yield) which is identified by ¹H NMR spectral analysis.

### EXAMPLE 10

### Preparation of N-{{2-chloro-5-[3,6-dihydro-3-methyl-2,6-dioxo-4-(trifluoromethyl)-1(2H)-pyrimidinyl]-4-fluorophenyl}(thiocarbamoyl)}-β-alanine, methyl ester

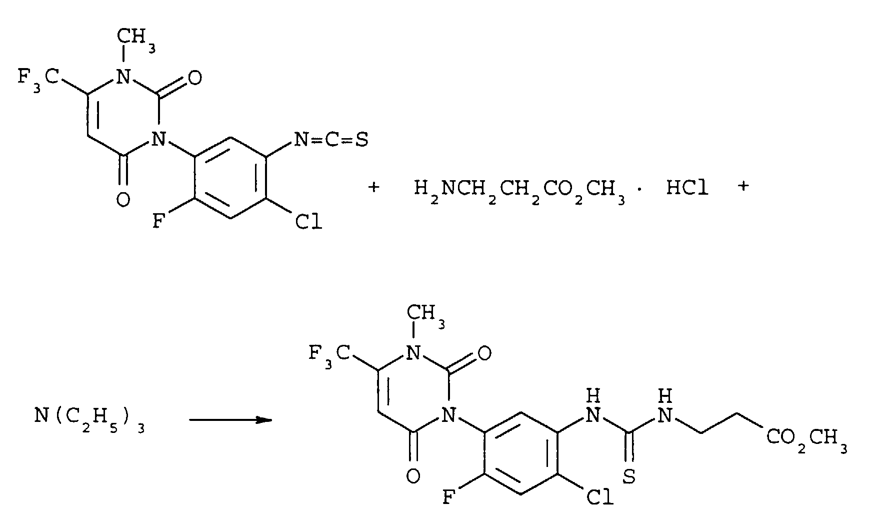

A solution of 2-chloro-5-[3,6-dihydro-3-methyl-2,6-dioxo-4-(trifluoromethyl)-1(2H)-pyrimidinyl]-4-fluorophenyl isothiocyanate (6.0 g, 15.8 mmol) and β-alanine, methyl ester, hydrochloride salt (2.65 g, 19.0 mmol) in acetone is cooled to 0°C, treated dropwise with a solution of triethylamine (1.92 g, 19.0 mmol) in methylene chloride, stirred overnight at room temperature, and poured into water. The resultant aqueous mixture is extracted with ethyl acetate. The organic extracts are combined, washed sequentially with 5% hydrochloric acid and brine, dried over anhydrous magnesium sulfate, and concentrated *in vacuo* to obtain a brown foam. Flash column chromatography of the foam using silica gel and a (9:1) methylene chloride/diethyl ether solution gives the title product as an off-white foam (5.68 g, 74.4% yield, mp 110°C) which is identified by ¹H NMR spectral analysis.

Using essentially the same procedure, but substituting the appropriate amine for β-alanine, methyl ester, hydrochloride salt, the following compounds are obtained:

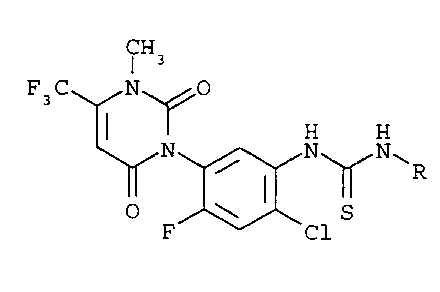

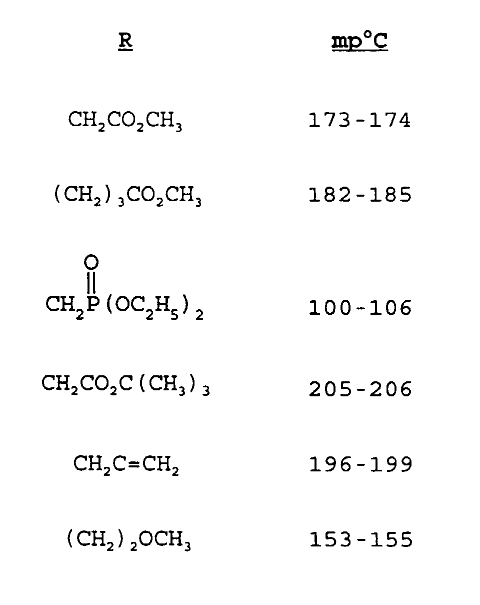

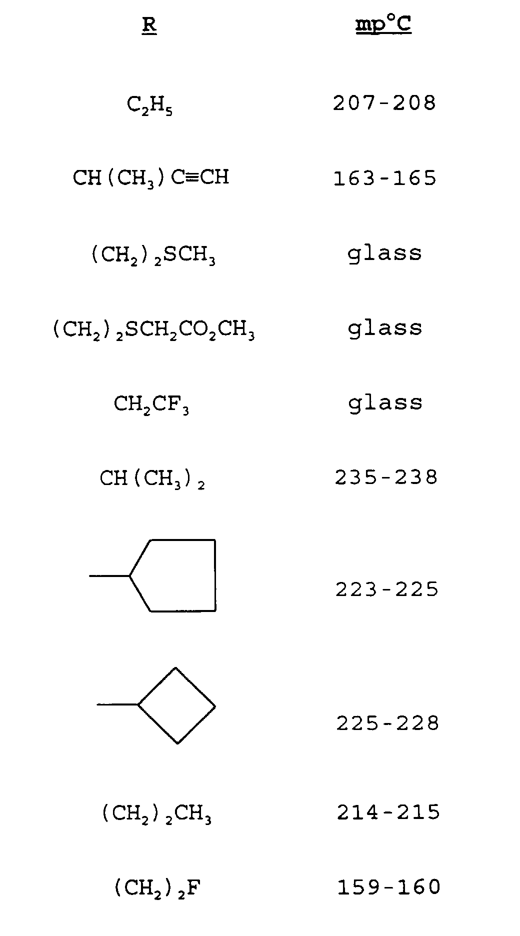

### EXAMPLE 11

### Preparation of 3-{4-Chloro-2-fluoro-5-[3-methyl-4-oxo-2-thiazolidinylidene)amino]phenyl}-1-methyl-6-(trifluoromethyl)-2,4(1H,3H)-pyrimidinedione

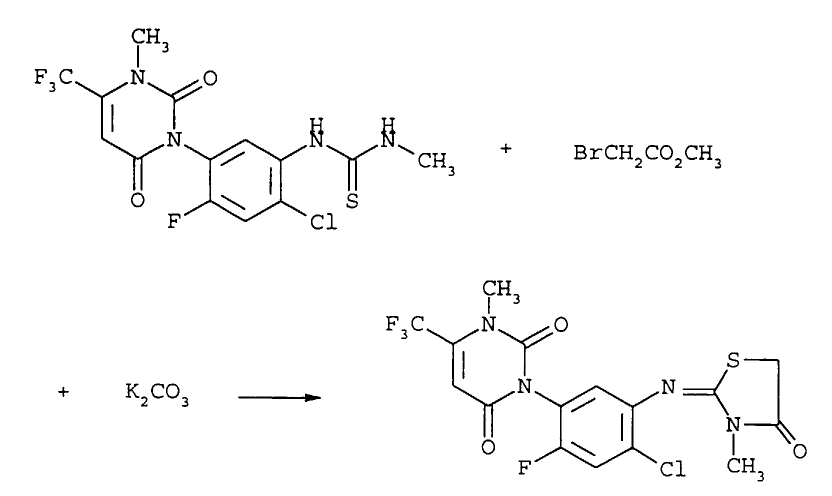

A solution of 1-{2-chloro-5-[3,6-dihydro-3-methyl-2,6-dioxo-4-(trifluoromethyl)-1(2H)-pyrimidinyl]-4-fluorophenyl}-3-methyl-2-thiourea (0.92 g, 2.24 mmol) in N,N-dimethylformamide is treated sequentially with potassium carbonate (0.34 g, 2.47 mmol) and methyl bromoacetate (3.2 mL, 3.36 mmol), stirred at room temperature for 5.5 hours, and poured into water. The resultant aqueous mixture is extracted with diethyl ether and ethyl acetate. The organic extracts are combined, washed sequentially with water and brine, dried over anhydrous magnesium sulfate, and concentrated *in vacuo* to obtain a yellow oil. Flash column chromatography of the oil using silica gel and a (1:2) ethyl acetate/hexanes solution gives the title product as a pale, yellow solid (0.6 g, 59% yield, mp 96-105°C) which is identified by ¹H, ¹³C and ¹⁹F NMR spectral analyses.

Using essentially the same procedure, or alternatively heating the reaction mixture in the absence of base, the following compounds are obtained:

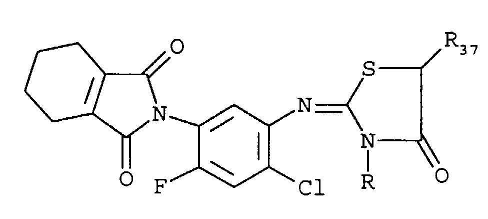

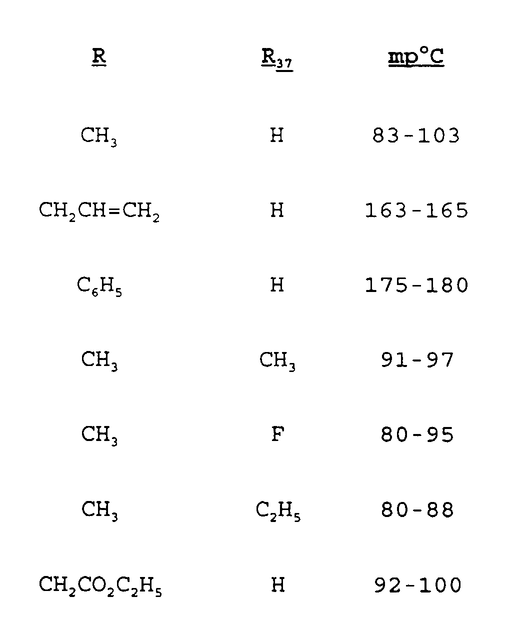

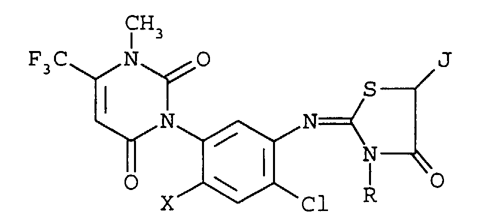

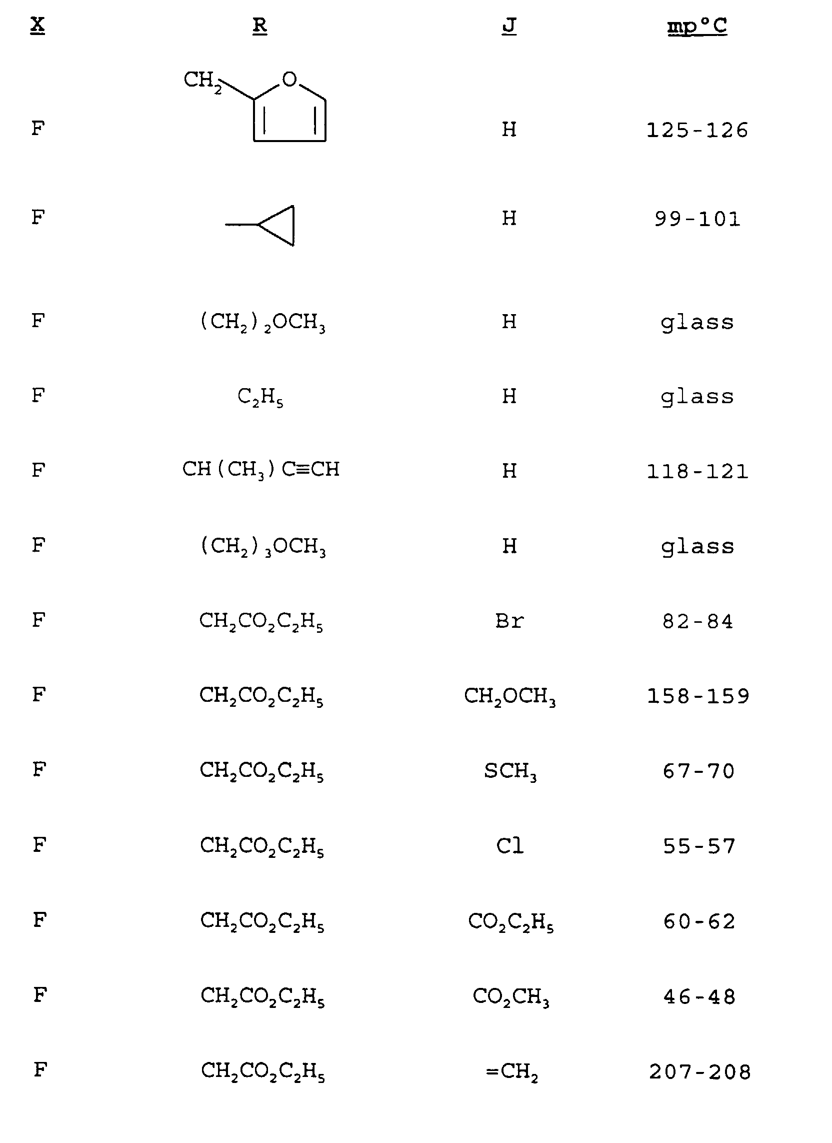

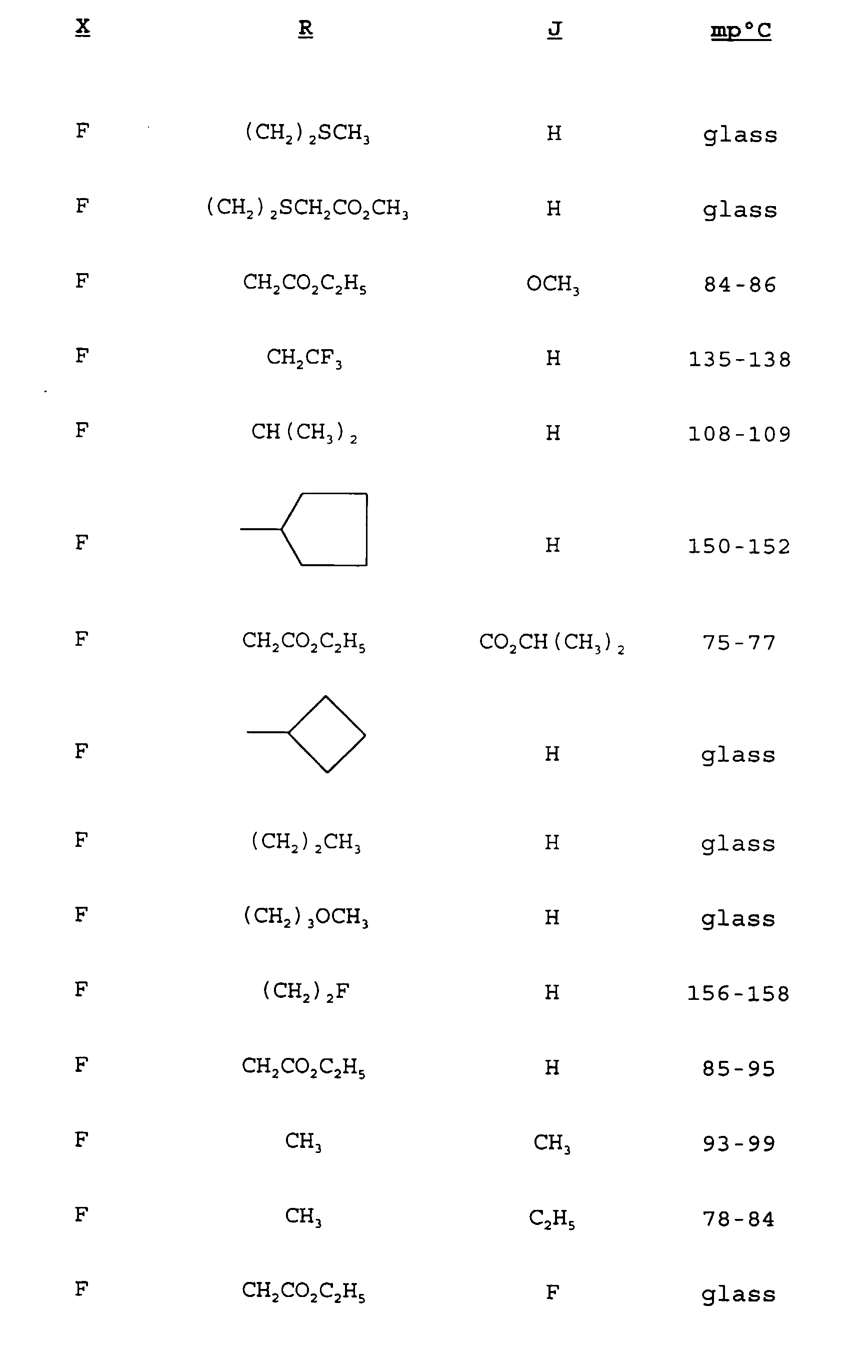

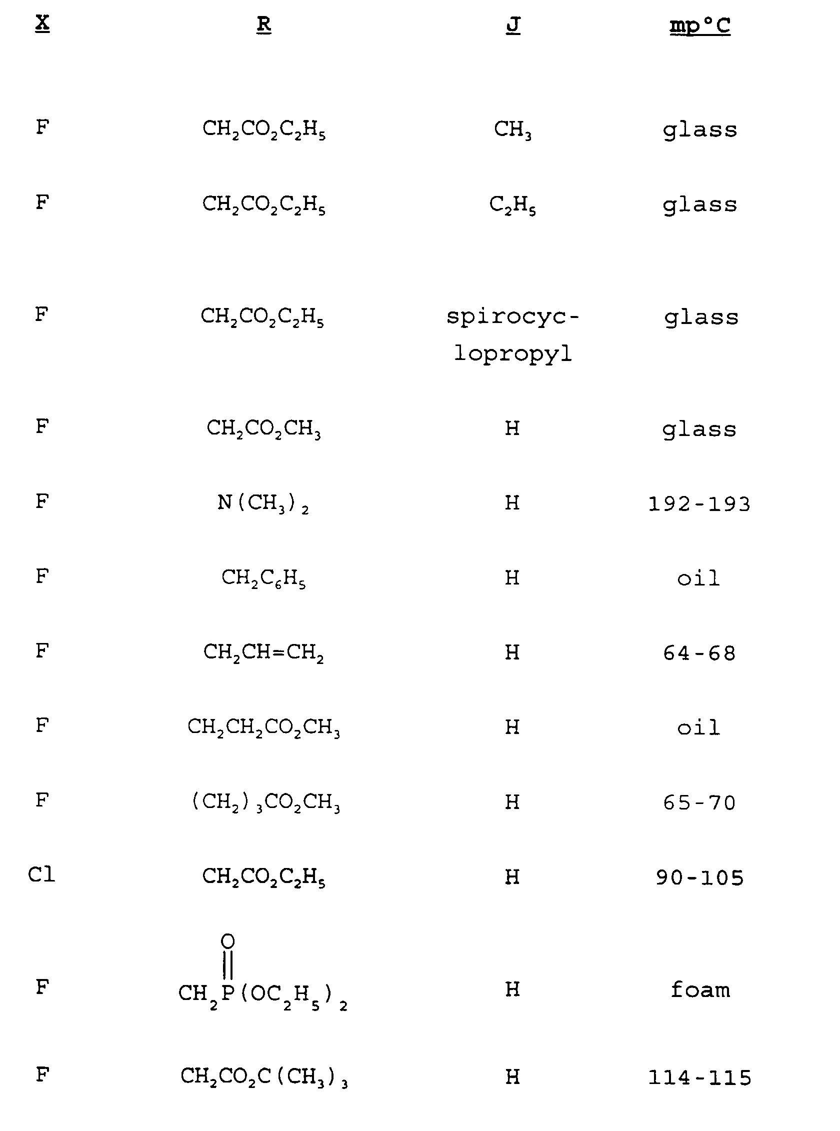

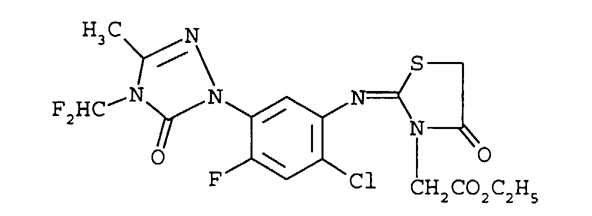

mp 60-70°C
and

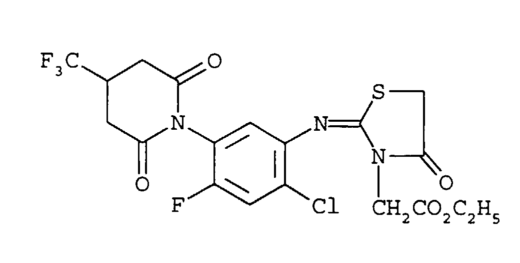

### EXAMPLE 12

### Preparation of 2-{{2-chloro-5-[3,6-dihydro-3-methyl-2,6-dioxo-4-(trifluoromethyl)-1(2H)-pyrimidinyl]-4-fluorophenyl}-4-oxo-3,5-thiazolidinediacetic acid, 3-ethyl ester

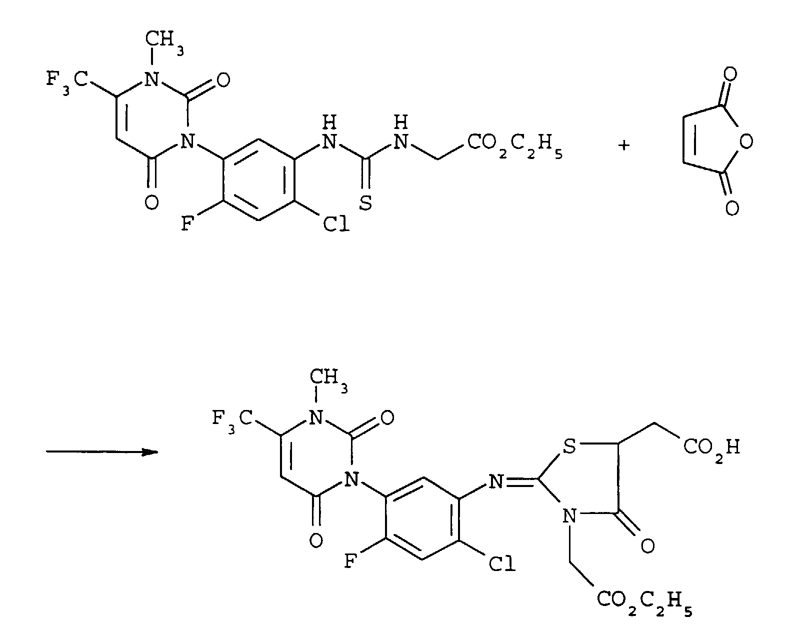

A solution of N-{{2-chloro-5-[3,6-dihydro-3-methyl-2,6-dioxo-4-(trifluoromethyl)-1(2H)-pyrimidinyl]-4-fluorophenyl}(thiocarbamoyl)}glycine, ethyl ester (2.94 g, 6.2 mmol) and maleic anhydride (0.67 g, 6.8 mmol) in N,N-dimethylformamide is stirred at 80°C for 2 days, treated with additional maleic anhydride (0.67 g), stirred overnight at 84°C, and poured into an ice-water mixture. The resultant aqueous mixture is filtered to obtain a solid. Column chromatography of the solid using silica gel and a diethyl ether/methylene chloride/ hexanes acetic acid solution (33:33:33:1) gives the title product as a pale, yellow glass (1.14 g) which is identified by ¹H, ¹³C and ¹⁹F NMR spectral analyses.

Using essentially the same procedure, the following compounds are obtained:

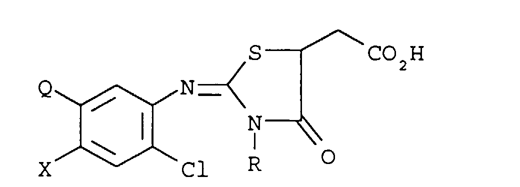

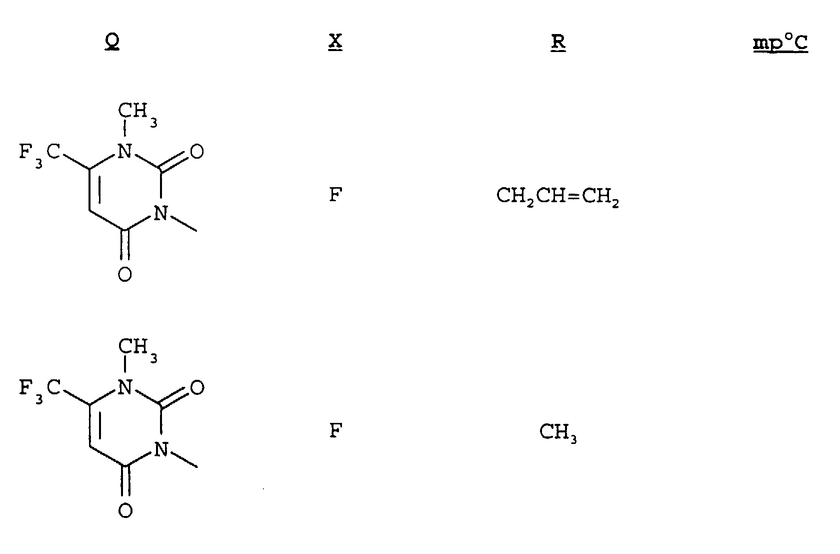

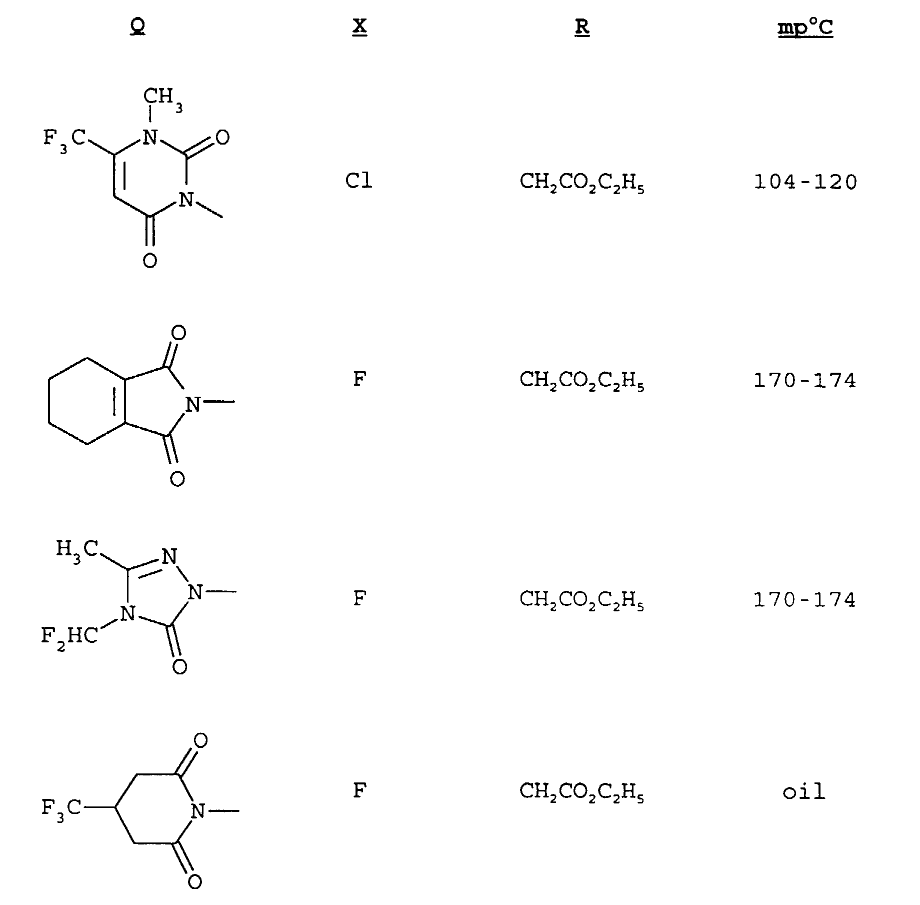

### EXAMPLE 13

### Preparation of 3-(Carboxymethyl)-2-{{2-chloro-5-[3,6-dihydro-3-methyl-2,6-dioxo-4-(trifluoromethyl)-1(2H)pyrimidinyl]-4-fluorophenyl}imino}-4-oxo-delta5,alphathiazolidineacetic acid, diethyl ester

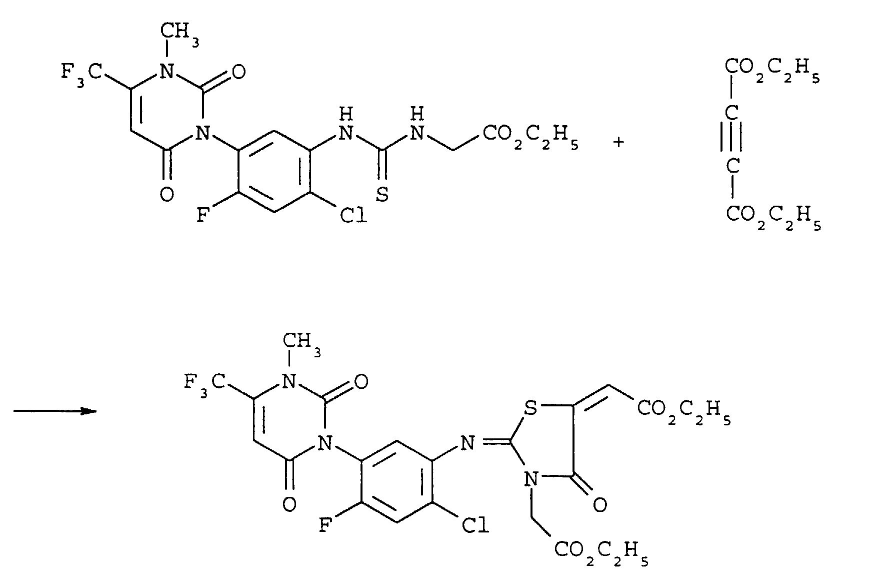

A solution of N-{{2-chloro-5-[3,6-dihydro-3-methyl-2,6-dioxo-4-(trifluoromethyl)-1(2H)-pyrimidinyl]-4-fluorophenyl}(thiocarbamoyl)}glycine, ethyl ester (3.0 g, 6.2 mmol) and diethyl acetylenedicarboxylate (1.06 g, 6.2 mmol) in methanol is refluxed for 3 hours, cooled, and concentrated *in vacuo* to obtain a glass. Column chromatography of the glass using silica gel and a 3% ethyl acetate in methylene chloride solution gives the title product as a yellow glass (2.49 g) which is identified by ¹H, ¹³C and ¹⁹F NMR spectral analyses.

### EXAMPLE 14

### Preparation of N-{{{2-chloro-5-[3,6-dihydro-3-methyl-2,6-dioxo-4-(trifluoromethyl)-1(2H)-pyrimidinyl]-4-fluorophenyl}imino}methylene}glycine, ethyl ester

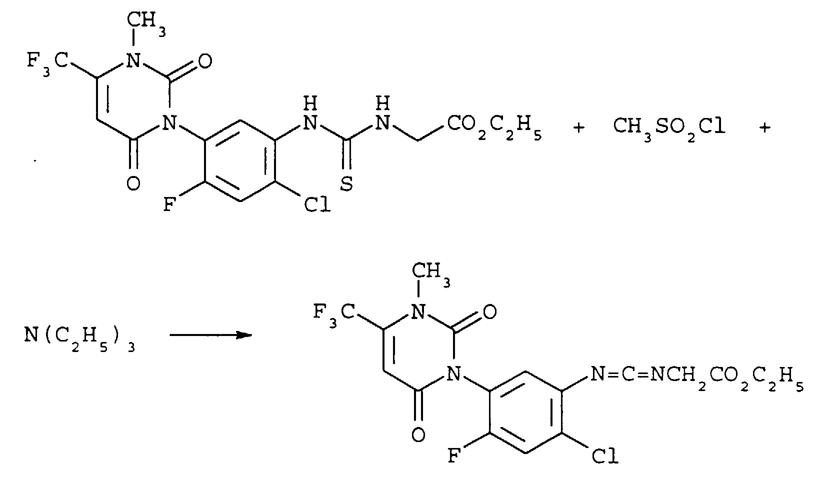

A solution of N-{{2-chloro-5-[3,6-dihydro-3-methyl-2,6-dioxo-4-(trifluoromethyl)-1(2H)-pyrimidinyl]-4-fluorophenyl}(thiocarbamoyl)}glycine, ethyl ester (5.0 g, 10.4 mmol) in methylene chloride is treated sequentially with triethylamine (4.3 mL, 31.1 mmol), 4-dimethylaminopyridine (0.05 g) and methanesulfonyl chloride (1.6 mL, 20.7 mmol) stirred at room temperature for 10 minutes, and partially concentrated *in vacuo* to obtain a heterogeneous mixture. The heterogeneous mixture is placed onto silica gel and eluted with methylene chloride. The resultant solution is concentrated *in vacuo* to give the title product as an amber syrup (4.68 g) which is identified by ¹H and ¹³C NMR spectral analyses.

### EXAMPLE 15

### Preparation of Ethyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate

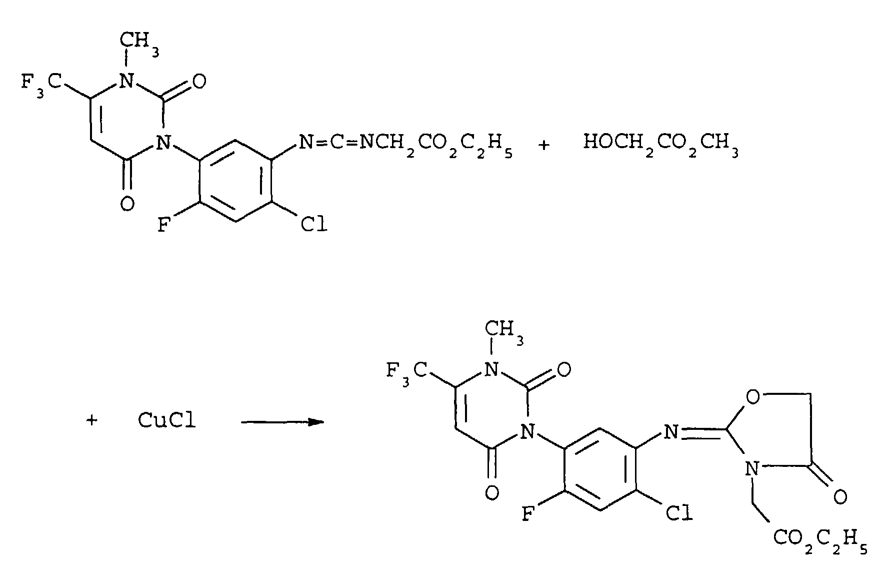

A solution of N-{{{2-chloro-5-[3,6-dihydro-3-methyl-2,6-dioxo-4-(trifluoromethyl)-1(2H)-pyrimidinyl]-4-fluorophenyl}imino}methylene}glycine, ethyl ester (0.63 g, 1.41 mmol)in methylene chloride is treated with methyl glycolate (0.14 g), stirred for 90 minutes, treated with copper(I) chloride (0.05 g), stirred at room temperature for 16 hours, refluxed for 5 hours, and filtered to remove solids. The resultant filtrate is concentrated *in vacuo* to obtain an amber gum. Flash column chromatography of the gum using silica gel and 20% to 25% ethyl acetate in hexanes solutions gives the title product as an off-white foam (0.37 g, mp 67-87°C) which is identified by ¹H and ¹³C NMR spectral analyses.

Using essentially the same procedure, the following compounds are obtained:

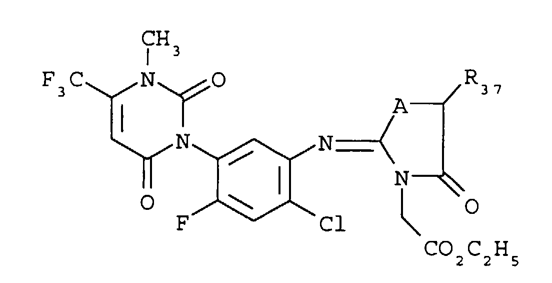

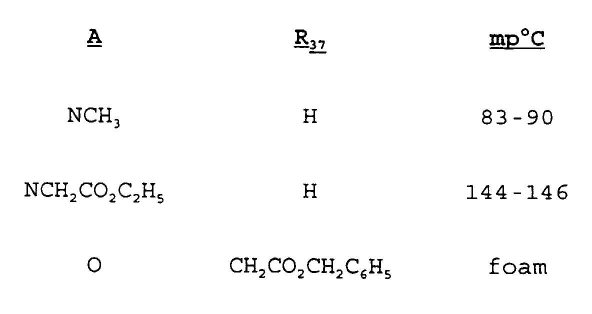

### EXAMPLE 16

### Preparation of Methyl α-benzyl-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate, L-

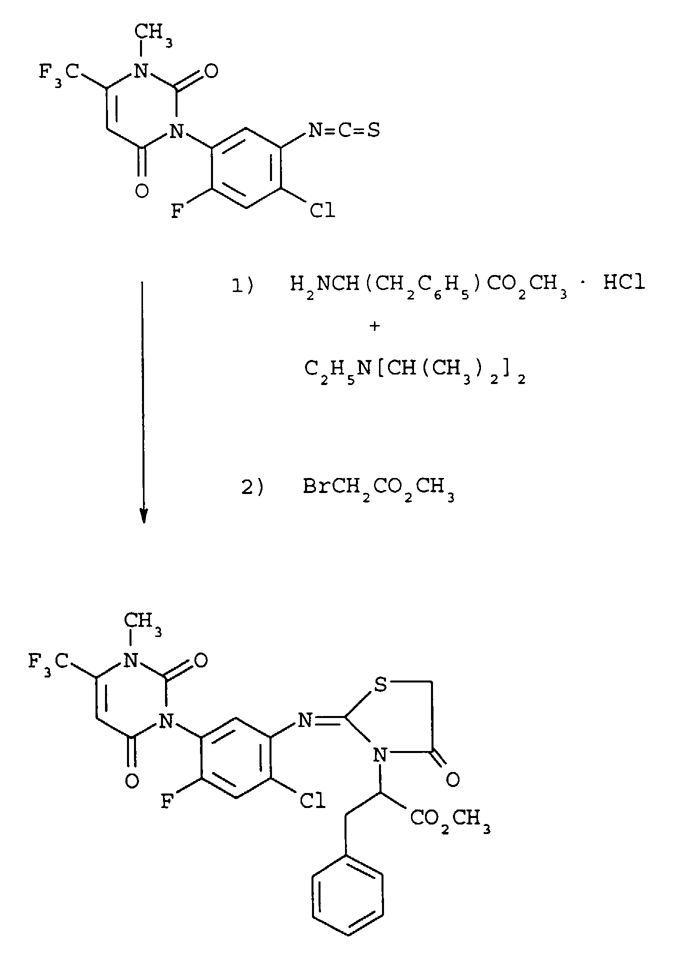

A solution of 2-chloro-5-[3,6-dihydro-3-methyl-2,6-dioxo-4-(trifluoromethyl)-1(2H)-pyrimidinyl]-4-fluorophenyl isothiocyanate (0.38 g, 1.0 mmol) and N,N-diisopropylethylamine (1.0 mL) in tetrahydrofuran is treated with L-phenylalanine, methyl ester, hydrochloride (0.18 g, 1.0 mmol), shaken at 40°C for 1 hour, treated with methyl bromoacetate (0.46 g, 3.0 mmol), shaken at 40°C for 16 hours, and concentrated *in vacuo* to obtain a residue. Column chromatography of the residue using silica gel and 0% to 10% diethyl ether in methylene chloride solutions gives the title product (0.37 g) which is identified by ¹H and ¹³C NMR spectral analyses.

Using essentially the same procedure, the following compounds are obtained:

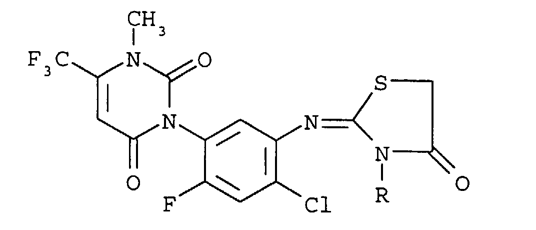

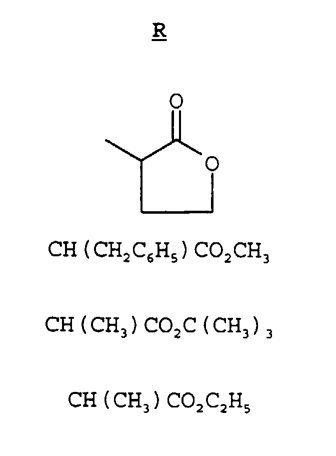

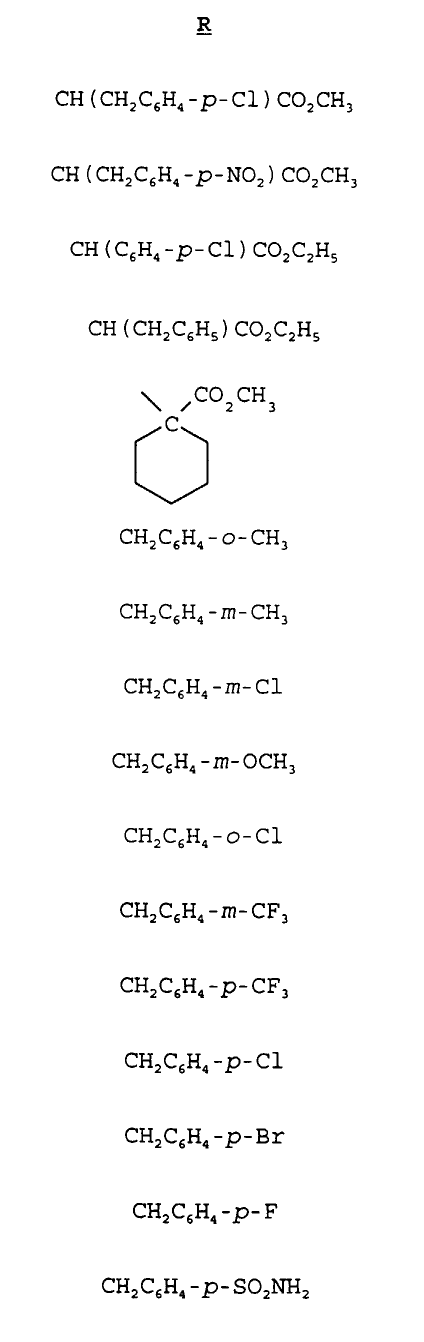

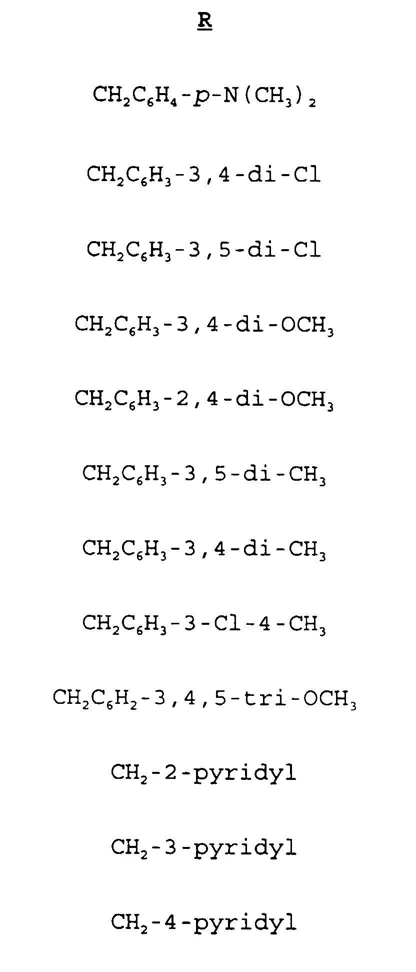

### EXAMPLE 17

### Preparation of 2-{{2-Chloro-5-[3,6-dihydro-3-methyl-2,6-dioxo-4-(trifluoromethyl)-1(2H)-pyrimidinyl]-4-fluorophenol}imino}-α-methyl-4-oxo-3-thiazolidineacetic acid,

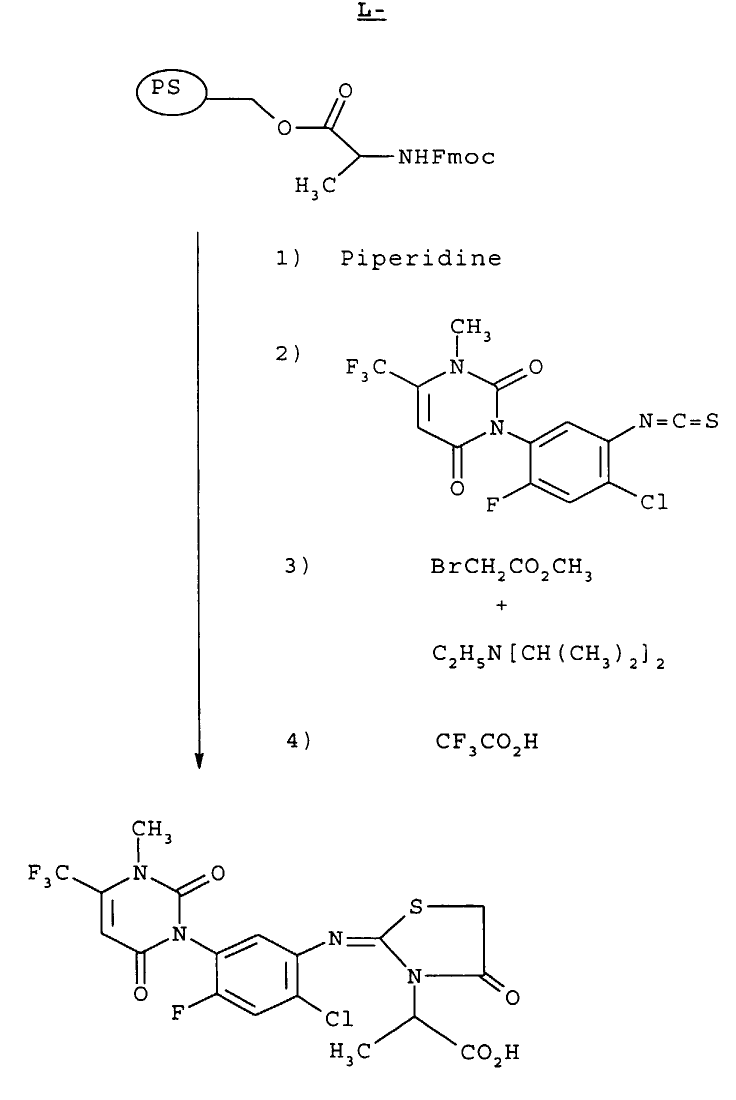

An alanine substituted resin (1.0 mmol) which is N-protected with Fmoc and supported on polystyrene (commercially available as Fmoc-gly-Wang resin from Midwest Bio-tech Inc., Fishers, Indiana) is treated with a 20% piperdine in N,N-dimethylformamide solution to remove the Fmoc protecting group. The solvents are removed and the resin is washed with N,N-dimethylformamide, methanol and methylene chloride, treated with a solution of 2-chloro-5-[3,6-dihydro-3-methyl-2,6-dioxo-4-(trifluoromethyl)-1(2H)-pyrimidinyl]-4-fluorophenyl isothiocyanate (0.57 g, 1.5 mmol) in methylene chloride, and stirred at room temperature for 6 hours. The solvents are removed and the resin is washed with N,N-dimethylformamide, methanol and methylene chloride, diluted with N,N-dimethylformamide, treated with methyl bromoacetate (0.46 g, 3 mmol) and N,N-diisopropylethylamine (1 ml), and stirred overnight at room temperature. The solvents are removed and the resultant resin is washed sequentially with N,N-dimethylformamide, methanol and methylene chloride, air-dried, and cleaved with trifluoroacetic acid. Column chromatography of the resultant reaction mixture using silica gel and a 15% methanol in methylene chloride solution gives the title product (0.55 g) which is identified by ¹H and ¹³C NMR spectral analyses.

Using essentially the same procedure, the following compounds are obtained:

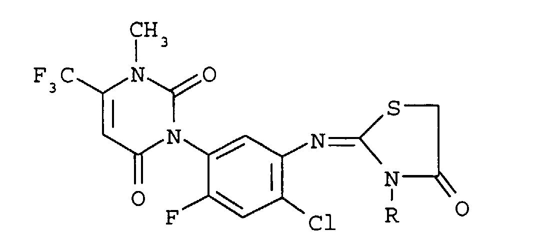

| **R** | **Stereochemistry** |
|---|---|
| CH₂CO₂H | |
| CH(CH₂C₆H₅)CO₂H | L- |
| CH(CH₂C₆H₅)CO₂H | D- |
| CH(CH₃)CO₂H | D- |
| CH[CH₂CH(CH₃)₂]CO₂H | L- |

### EXAMPLE 18

### Preparation of 2-{{2-Chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid

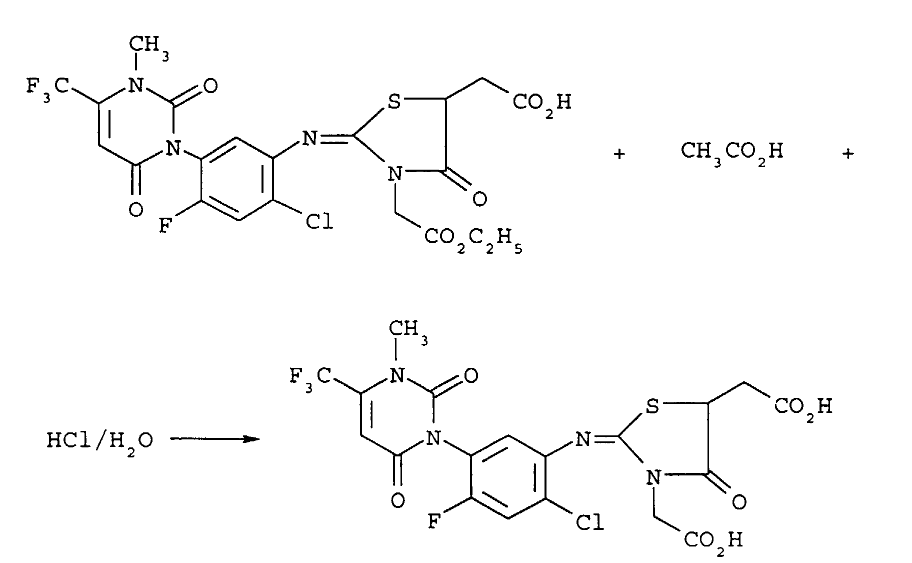

A solution of 2-{{2-chloro-5-[3,6-dihydro-3-methyl-2,6-dioxo-4-(trifluoromethyl)-1(2H)-pyrimidinyl]-4-fluorophenyl}-4-oxo-3,5-thiazolidinediacetic acid, 3-ethyl ester (0.5 g, 0.9 mmol) in 10% hydrochloric acid (1.4 mL) and acetic acid (5.6 mL) is stirred at 55°C for 5 days, and concentrated *in vacuo* to obtain a yellow glass. Column chromatography of the glass using silica gel and a 5% methanol in methylene chloride solution gives the title product as a white solid which is identified by ¹H NMR spectral analysis.

Using essentially the same procedure, the following compounds are obtained:

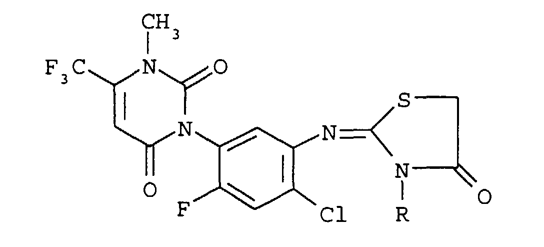

| **R** | **mp°C** |
|---|---|
| CH₂CH₂CH₂CO₂H | 140(dec) |
| CH₂CH₂CO₂H | 135(dec) |

### EXAMPLE 19

### Preparation of Ethyl 5-(carbamoylmethyl)-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate

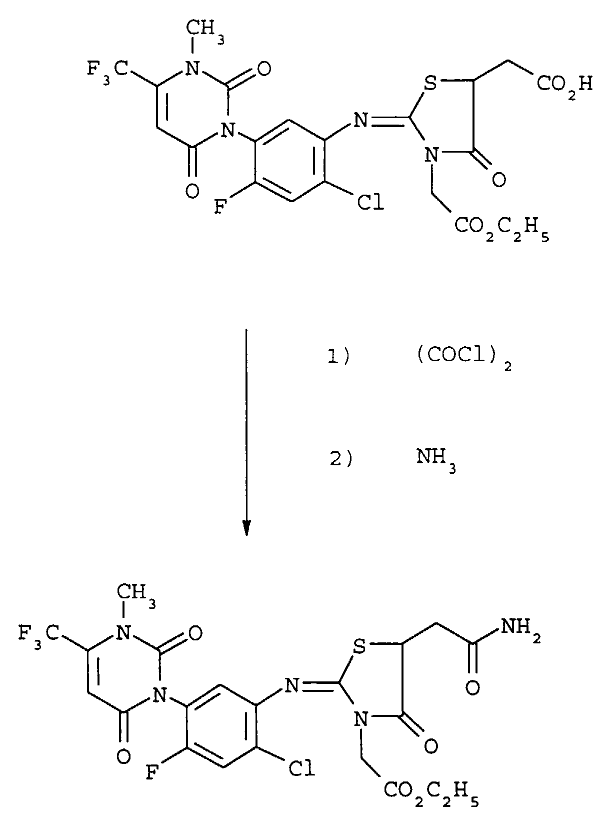

A solution of 2-{{2-chloro-5-[3,6-dihydro-3-methyl-2,6-dioxo-4-(trifluoromethyl)-1(2H)-pyrimidinyl]-4-fluorophenyl}-4-oxo-3,5-thiazolidinediacetic acid, 3-ethyl ester (0.5 g, 0.9 mmol) in methylene chloride is treated with oxalyl chloride (0.5 mL), stirred at room temperature for 2 hours, concentrated *in vacuo,* treated with a 0.5 M ammonia solution in dioxane (2 mL), stirred overnight at room temperature, and concentrated *in vacuo* to obtain a residue. Chromatography of the residue gives the title product which is identified by ¹H NMR spectral analysis.

Using essentially the same procedure, the following compounds are obtained:

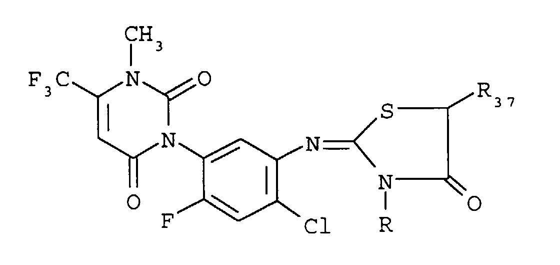

| **R** | **R**_{**37**} |
|---|---|
| CH₂CO₂C₂H₅ | CH₂C(O)NHCH₃ |
| CH₂CO₂C₂H₅ | CH₂C(O)NHCH(CH₃)₂ |
| CH₂CO₂C₂H₅ | CH₂C(O)NHC₆H₅ |
| CH₃ | CH₂C(O)NHC₆H₅ |
| CH₃ | CH₂C(O)NH₂ |
| CH₂CO₂C₂H₅ | CH₂C(O)OCH₂C≡CH |
| CH₂CO₂C₂H₅ | CH₂C(O)OCH₂CH₂F |
| CH₂CH=CH₂ | CH₂C(O)NHCH₃ |
| CH₂CH=CH₂ | CH₂C(O)OCH₂CH₂F |
| CH₃ | CH₂C(O)OCH₃ |
| CH₃ | CH₂C(O)OCH(CH₃)₂ |
| CH₃ | CH₂C(O)OCH₂C≡CH |
| CH₃ | CH₂C(O)OCH₂CH₂F |
| CH₂CH=CH₂ | CH₂C(O)NHC₆H₅ |
| CH₂CH=CH₂ | CH₂C(O)NH₂ |

### EXAMPLE 20

### Preparation of Ethyl 2-{{2-chloro-5-[3,6-dihydro-3-methyl-2,6-dioxo-4-(trifluoromethyl)-1(2H)-pyrimidinyl]-4-fluorophenyl}imino}-5-{[(methylsulfonyl)carbamoyl]-methyl}-4-oxo-3-thiazolidineacetate

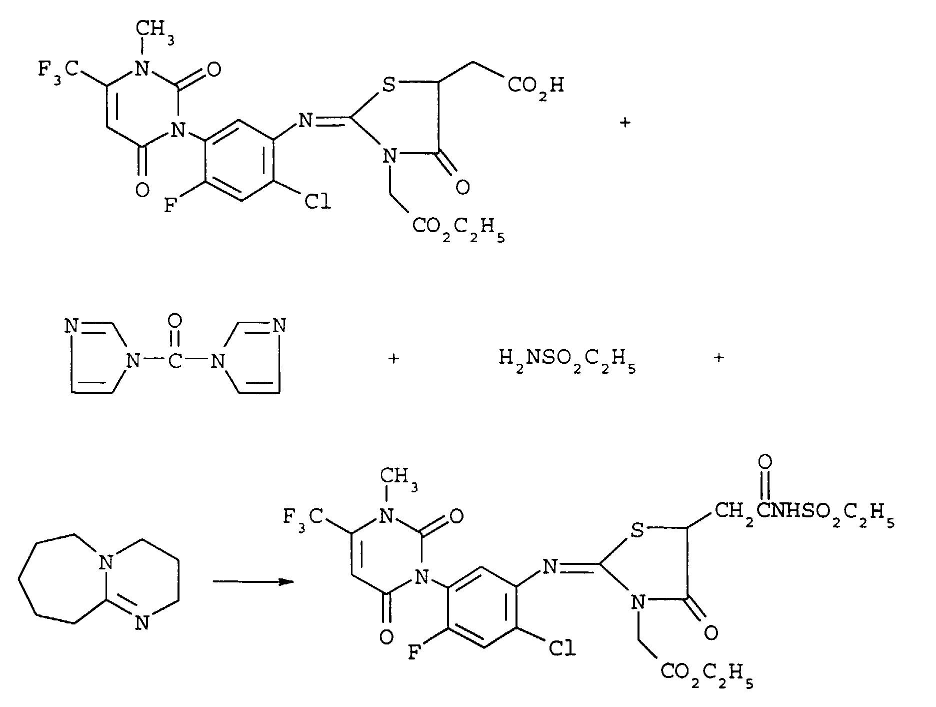

A solution of 2-{{2-chloro-5-[3,6-dihydro-3-methyl-2,6-dioxo-4-(trifluoromethyl)-1(2H)-pyrimidinyl]-4-fluorophenyl}-4-oxo-3,5-thiazolidinediacetic acid, 3-ethyl ester (0.5 g, 0.9 mmol) and 1,1'-carbonyldiimidazole (0.23 g, 1.3 mmol) in tetrahydrofuran is refluxed for 2 hours, cooled to room temperature, treated portionwise with a solution of ethanesulfonamide (0.22 g, 1.1 mmol) and 1,8-diazabicyclo[5.4.0]undec-7-ene (0.21 g, 1.3 mmol) in tetrahydrofuran, stirred overnight at room temperature, and poured into dilute hydrochloric acid. The resultant aqueous mixture is extracted with methylene chloride. The organic extracts are combined, washed with water, dried over anhydrous sodium sulfate, and concentrated *in vacuo* to obtain a tan liquid. Column chromatography of the liquid using silica gel and a 7.5% methanol in methylene chloride solution gives the title product which is identified by ¹H NMR spectral analyses.

Using essentially the same procedure, the following compounds are obtained:

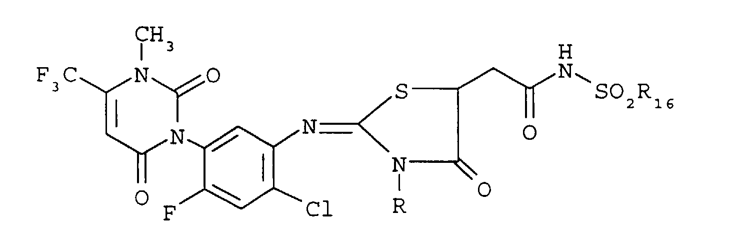

| **R** | **R**_{**16**} |
|---|---|
| CH₂CH=CH₂ | CH₃ |
| CH₃ | CH₃ |
| CH₂CO₂C₂H₅ | C₆H₅ |
| CH₂CH=CH₂ | C₆H₅ |
| CH₃ | C₆H₅ |
| CH₂CO₂C₂H₅ | C₂H₅ |
| CH₂CH=CH₂ | C₂H₅ |
| CH₃ | C₂H₅ |
| CH₂CO₂C₂H₅ | CH(CH₃)₂ |
| CH₂CH=CH₂ | CH(CH₃)₂ |
| CH₃ | CH(CH₃)₂ |

and

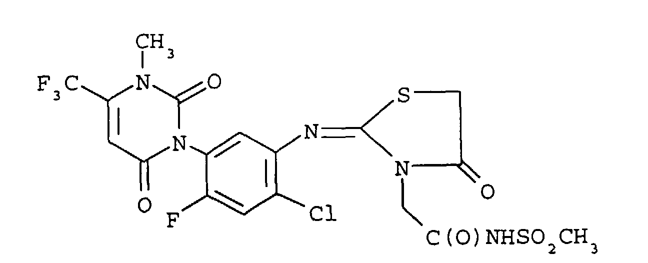

### EXAMPLE 21

### Preparation of 2-{{2-Chloro-5-[3,6-dihydro-3-methyl-2,6-dioxo-1(2H)-pyrimidinyl]-4-fluorophenol}imino}-4-oxo-3,5-thiazolidinediacetic acid, 3-ethyl ester, 5-ester with ethyl lactate

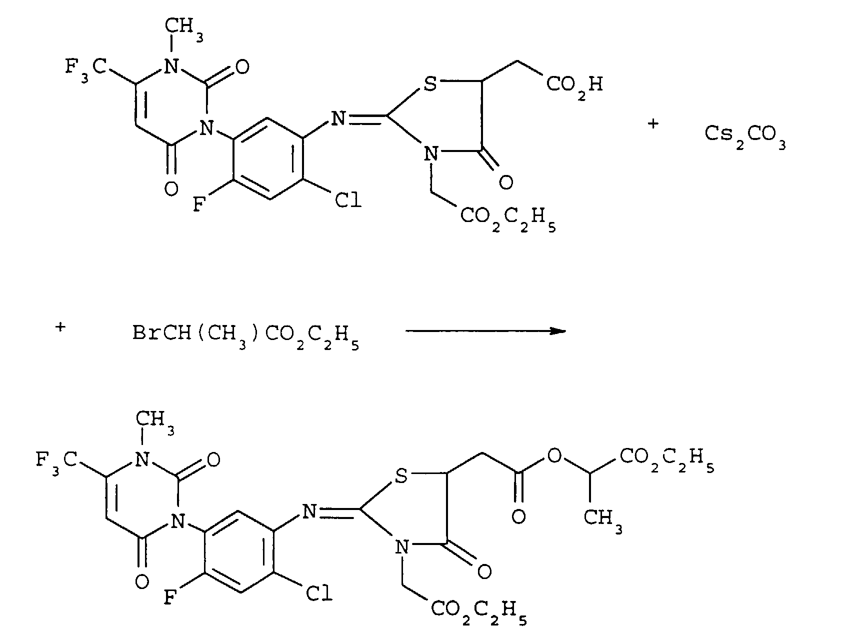

A mixture of 2-{{2-chloro-5-[3,6-dihydro-3-methyl-2,6-dioxo-4-(trifluoromethyl)-1(2H)-pyrimidinyl]-4-fluorophenyl}-4-oxo-3,5-thiazolidinediacetic acid, 3-ethyl ester (0.5 g, 0.9 mmol) and cesium carbonate (0.28 g, 0.9 mmol) in N,N-dimethylformamide is heated to 40°C, treated with ethyl 2-bromopropionate (0.16 g, 0.9 mmol), stirred at 40°C for 45 minutes, concentrated *in vacuo,* and diluted with methylene chloride. The resultant organic solution is washed with water, dried over anhydrous sodium sulfate, and concentrated *in vacuo* to obtain a brown oil. Column chromatography of the oil using silica gel and a methylene chloride/diethyl ether/hexanes/acetic acid solution (33:33:33:1) gives the title product as a clear, yellow oil which is identified by ¹H NMR spectral analyses.

Using essentially the same procedure, the following compounds are obtained:

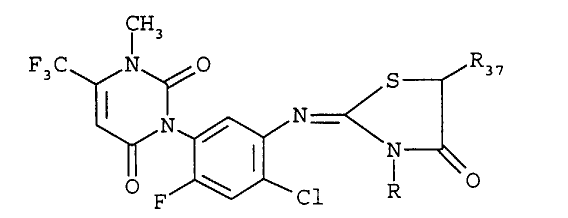

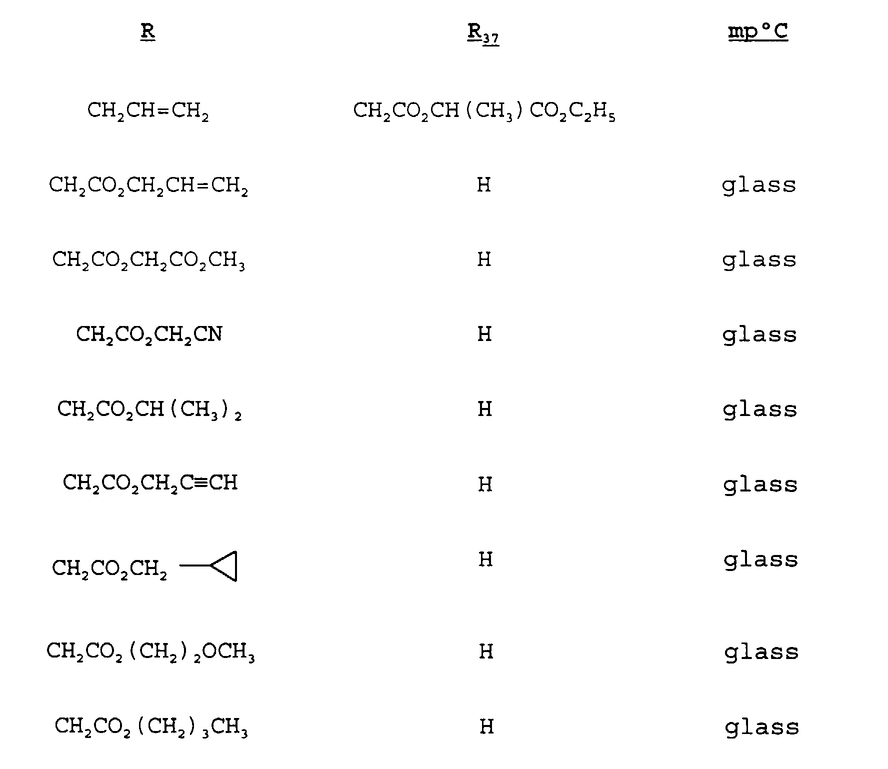

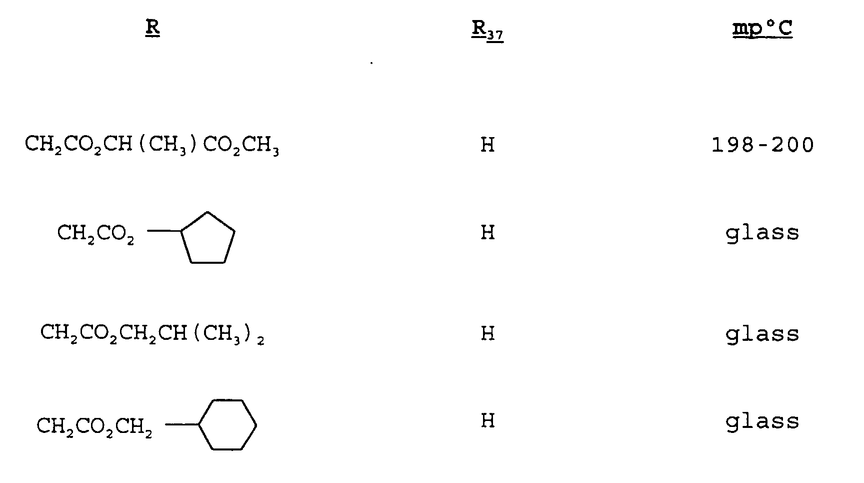

### EXAMPLE 22

### Preparation of 2-{{2-Chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid, 3-ethyl methyl ester

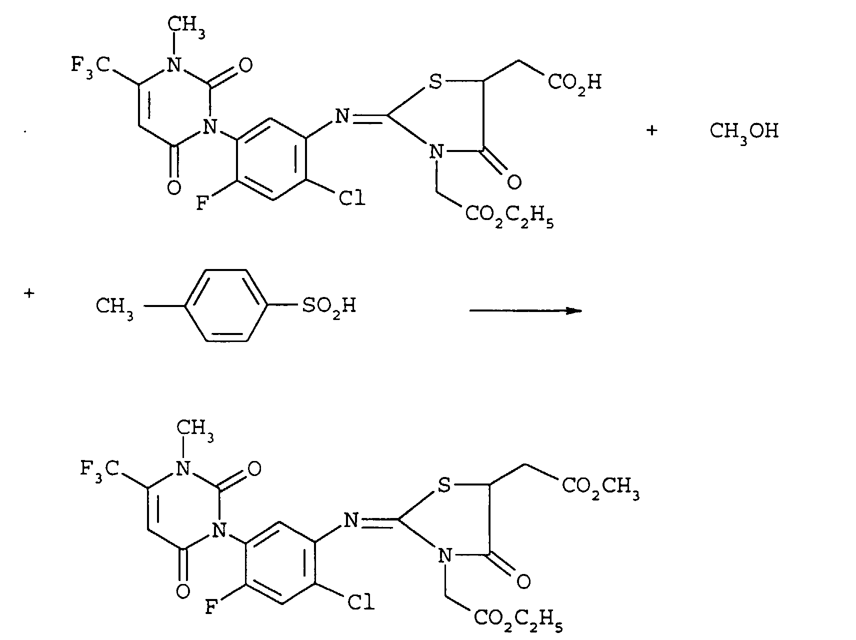

A mixture of 2-{{2-chloro-5-[3,6-dihydro-3-methyl-2,6-dioxo-4-(trifluoromethyl)-1(2H)-pyrimidinyl]-4-fluorophenyl}-4-oxo-3,5-thiazolidinediacetic acid, 3-ethyl ester (0.5 g, 0.9 mmol), methanol (1 mL) and a catalytic amount of *p*-toluenesulfonic acid in methylene chloride is stirred overnight at 40°C, and concentrated *in vacuo* to obtain a residue. Column chromatography of the residue using silica gel and a methylene chloride/diethyl ether/hexanes/acetic acid solution (33:33:33:1) gives the title product which is identified by ¹H NMR spectral analyses.

Using essentially the same procedure, the following compounds are obtained:

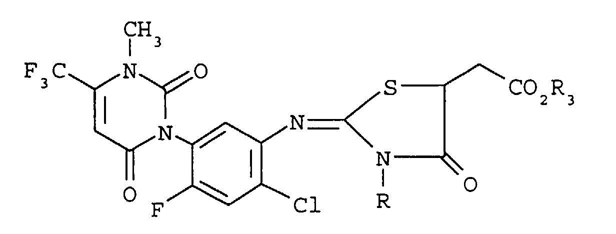

| **R**_{**3**} | **R** |
|---|---|
| CH(CH₃)₂ | CH₂CO₂C₂H₅ |
| CH₃ | CH₂CH=CH₂ |
| CH₂CH=CH₂ | CH₂CH=CH₂ |
| CH(CH₃)₂ | CH₂CH=CH₂ |
| CH₂C≡CH | CH₂CH=CH₂ |

### EXAMPLE 23

### Preparation of Ethyl 2-{{2-chloro-5-[3,6-dihydro-3-methyl-2,6-dioxo-4-(trifluoromethyl)-1(2H)-pyrimidinyl]-4-fluorophenyl}imino}-4-oxo-5-{[(trityloxy)carbamoyl]methyl}-3-thiazolidineacetate

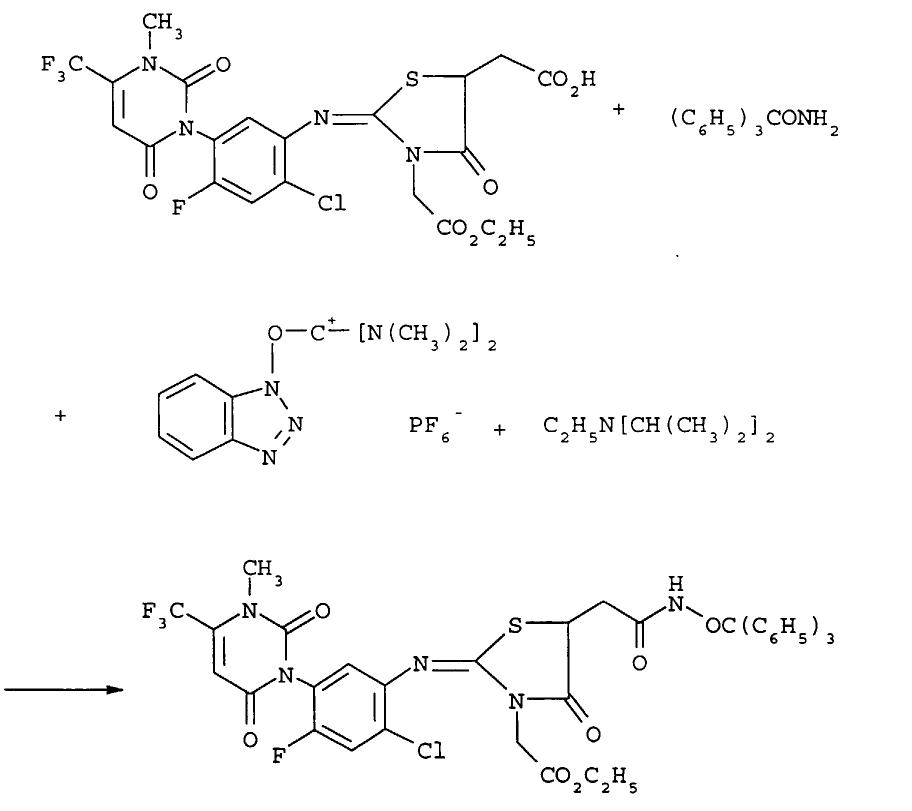

A mixture of 2-{{2-chloro-5-[3,6-dihydro-3-methyl-2,6-dioxo-4-(trifluoromethyl)-1(2H)-pyrimidinyl]-4-fluorophenyl}-4-oxo-3,5-thiazolidinediacetic acid, 3-ethyl ester (0.1 g, 0.17 mmol), O-tritylhydroxylamine (0.047 g, 0.17 mmol) and (1H-benzotriazol-1-yloxy)bis-(dimethylamino)methylium hexafluorophosphate (0.065 g, 0.17 mmol) is treated with a solution of N,N-diisopropylethylamine (0.04 mL, 0.17 mmol) in N,N-dimethylformamide, stirred at room temperature overnight, and concentrated *in vacuo* to obtain a residue. Column chromatography of the residue using silica gel and a 5% methanol in methylene chloride solution gives the title product which is identified by ¹H NMR spectral analysis.

Using essentially the same procedure, but substituting methoxylamine hydrochloride for O-tritylhydroxylamine, the following compounds are obtained:

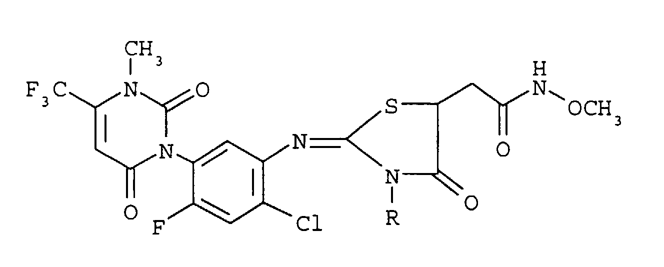

| **R** |
|---|
| CH₂CO₂C₂H₅ |
| CH₂CH=CH₂ |

### EXAMPLE 24

### Preparation of Ethyl 2-{{2-chloro-5-[3,6-dihydro-3-methyl-2,6-dioxo-4-(trifluoromethyl)-1(2H)-pyrimidinyl]-4-fluorophenyl}imino}-5-[(hydroxycarbamoyl)methyl]-4-oxo-3-thiazolidineacetate

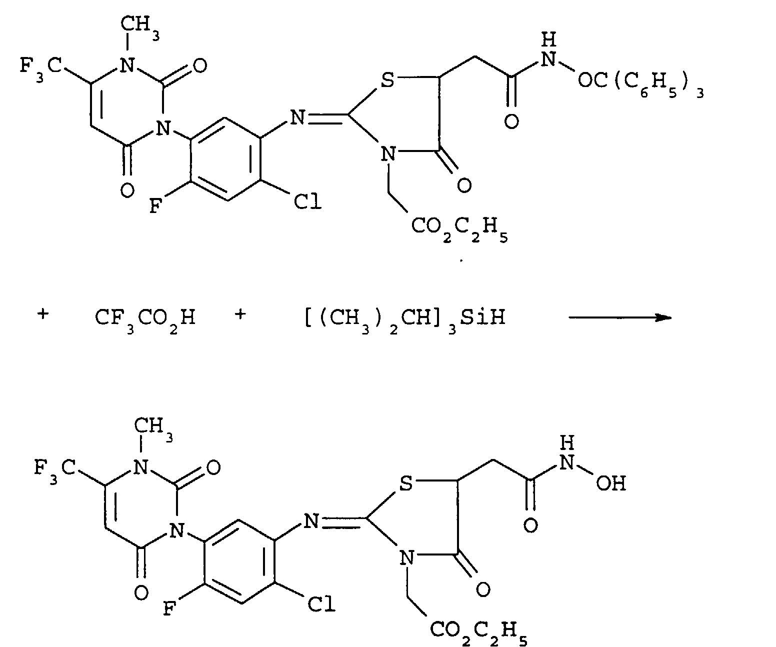

A solution of ethyl 2-{{2-chloro-5-[3,6-dihydro-3-methyl-2,6-dioxo-4-(trifluoromethyl)-1(2H)-pyrimidinyl]-4-fluorophenyl}imino}-4-oxo-5-{[(trityloxy)carbamoyl]-methyl)-3-thiazolidineacetate (entire sample from Example 23) and triisopropylsilane (1 mL) in trifluoroacetic acid (19 mL) is stirred at room temperature for 4 hours, cooled with an ice-water bath, and filtered. The resultant filtrate is concentrated *in vacuo* and chased three times with toluene to obtain a solid. Column chromatography of the solid using silica gel and a 7.5% methanol in methylene chloride solution gives the title product as a yellow solid which is identified by ¹H NMR spectral analysis.

Using essentially the same procedure, the following compounds are obtained:

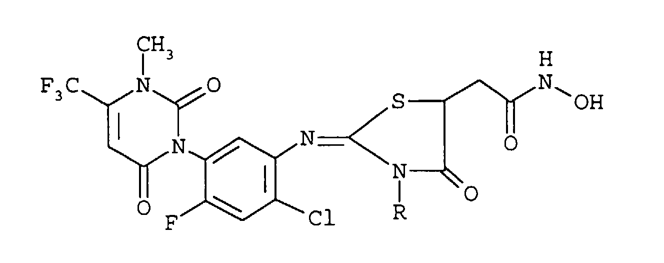

| **R** |
|---|
| CH₂CH=CH₂ |
| CH₃ |

### EXAMPLE 25

### Preparation of 2'-Chloro-4'-fluoro-5'-nitroacetanilide

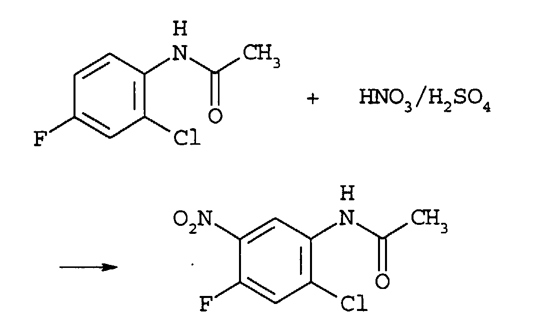

A solution of 2'-chloro-4'-fluoroacetanilide (85.5 g, 456 mmol) in concentrated sulfuric acid is cooled to 0°C, treated slowly with 70% nitric acid (32 mL, 502 mmol) while maintaining the temperature from 5-8°C, stirred at room temperature overnight and poured into water. The resultant aqueous mixture is filtered to obtain a solid which is recrystallized from ethanol to give the title product as pale, yellow plates (27.0 g, mp 158-160°C).

### EXAMPLE 26

### Preparation of 5'-Amino-2'-chloro-4'-fluoroacetanilide

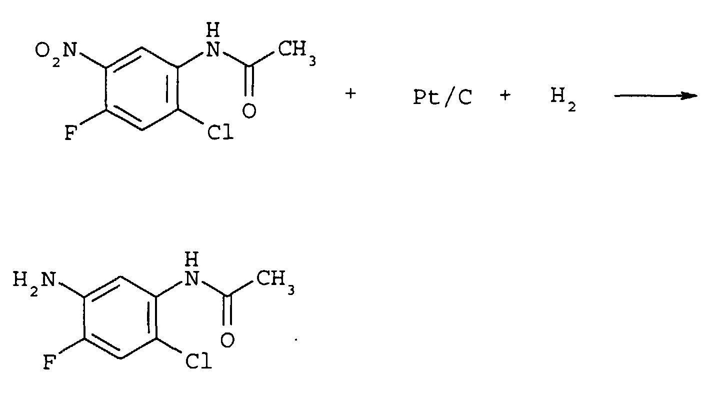

A mixture of 2'-chloro-4'-fluoro-5'-nitroacetanilide (26.1 g, 112 mmol) and 5% platinum on carbon (7.8 g, 30 wt%) in a tetrahydrofuran/ethyl acetate/ethanol solution (1:1:1) is hydrogenated at 50 psi for 22 hours and filtered. The resultant filtrate is concentrated *in vacuo* to obtain a solid which is recrystallized from an ethanol/water solution to give the title product as off-white plates (14.5 g, mp 176-178°C).

### EXAMPLE 27

### Preparation of 2'-Chloro-5'-[3,6-dihydro-2,6-dioxo-4-(trifluoromethyl)-1(2H)-pyrimidinyl]-4'-fluoroacetanilide

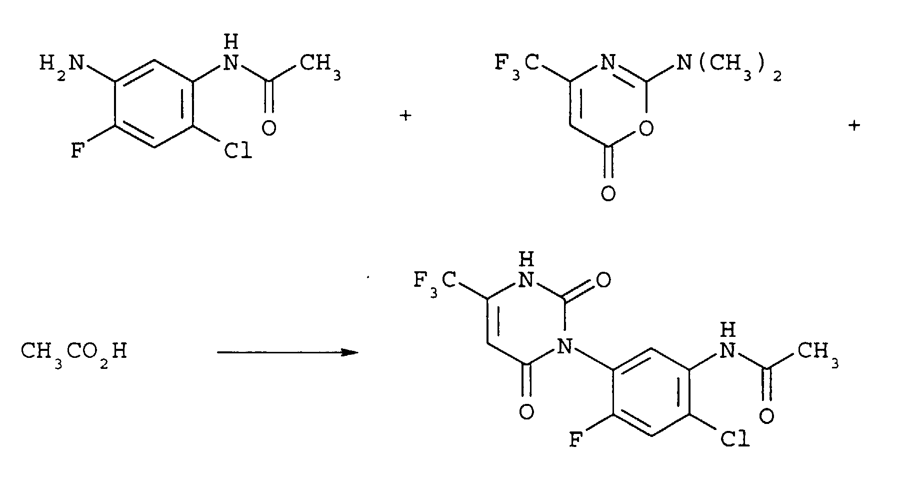

A mixture of 5'-amino-2'-chloro-4'-fluoroacetanilide (1.0 g, 4.9 mmol) and 2-dimethylamino-4-(trifluoromethyl)-6H-1,3-oxazin-6-one (1.0 g, 4.9 mmol) in acetic acid is refluxed for 90 minutes and diluted with water. The resultant aqueous mixture is filtered to obtain the title product as an off-white solid (1.4 g, mp 264-266°C) which is identified by ¹H, ¹³C and ¹⁹F NMR spectral analyses.

### EXAMPLE 28

### Preparation of 2'-Chloro-5'-[3,6-dihydro-3-methyl-2,6-dioxo-4-(trifluoromethyl)-1(2H)-pyrimidinyl]-4'-fluoroacetanilide

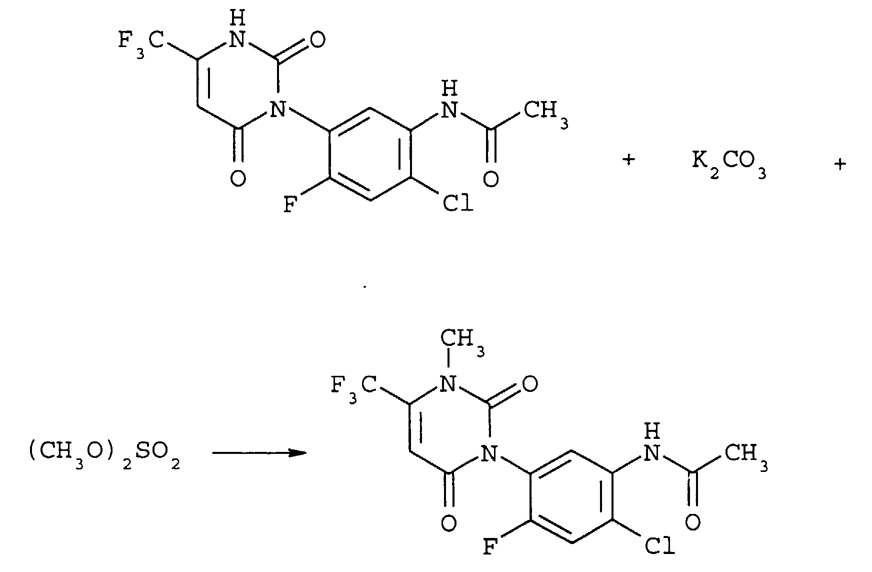

A mixture of 2'-chloro-5'-[3,6-dihydro-2,6-dioxo-4-(trifluoromethyl)-1(2H)-pyrimidinyl]-4'-fluoroacetanilide (0.85 g, 2.3 mmol), potassium carbonate (0.32 g, 2.3 mmol) and dimethylsulfate (0.22 mL, 2.3 mmol) in tetrahydrofuran is refluxed for 90 minutes, cooled to room temperature, and diluted with water and chloroform. The resultant mixture is concentrated *in vacuo* to obtain an aqueous suspension which is filtered to give the title product as an off-white solid (0.75 g, mp 257-258.5°C).

### EXAMPLE 29

### Preparation of 3-(5-Amino-4-chloro-2-fluorophenyl)-1-methyl-6-(trifluoromethyl)-2,4(1H,3H)-pyrimidinedione

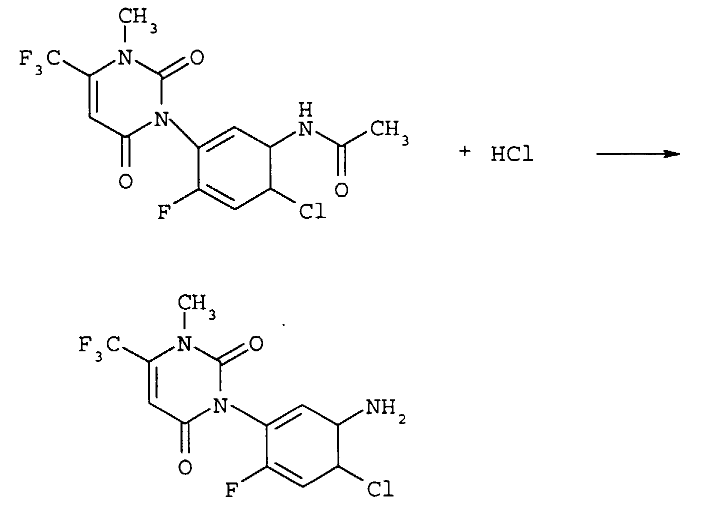

A mixture of 2'-chloro-5'-[3,6-dihydro-3-methyl-2,6-dioxo-4-(trifluoromethyl)-1(2H)-pyrimidinyl]-4'-fluoro-acetanilide (24.42 g, 64.3 mmol) in ethanol is treated with 2N hydrochloric acid (200 ml), diluted with tetrahydrofuran, refluxed overnight, cooled to room temperature, partially concentrated *in vacuo* to remove ethanol and tetrahydrofuran, neutralized with 3N sodium hydroxide solution, and extracted with ethyl acetate. The organic extracts are combined, washed sequentially with saturated sodium hydrogen carbonate solution and brine, dried over anhydrous magnesium sulfate, and concentrated *in vacuo* to give the title product as a light, brown solid (20.3 g, mp 132-135°C).

### EXAMPLE 30

### Preparation of Ethyl 2-{{2-chloro-5-[3,6-dihydro-3-methyl-2,6-dioxo-4-(trifluoromethyl)-1(2H)-pyrimidinyl]-4-fluorophenyl}imino}-5-isopropylidene-4-oxo-3-thiazol-idineacetate

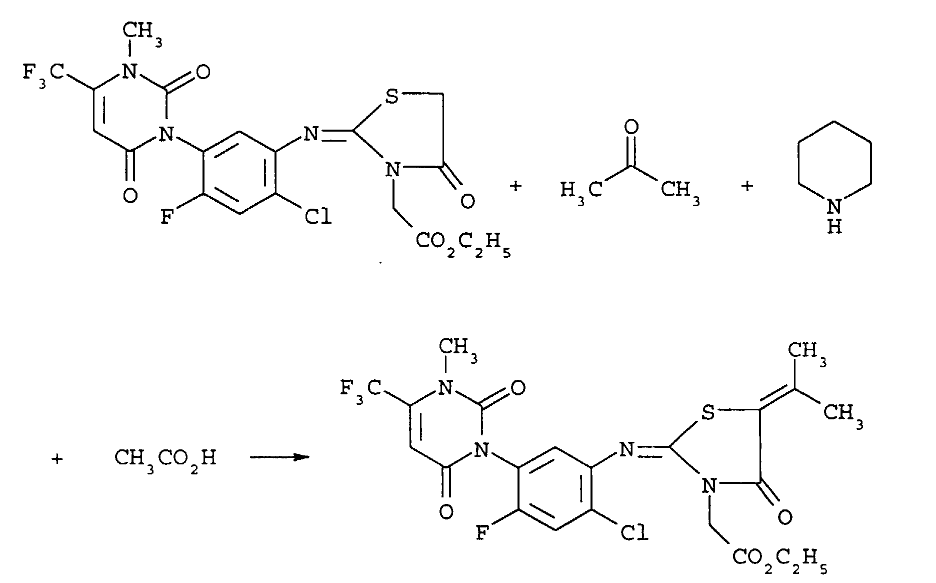

A mixture of ethyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate (2.0 g, 3.83 mmol), acetone (0.562 mL, 7.65 mmol), piperidine (0.20 mL, 2.02 mmol) and acetic acid (20 µL) in ethanol is refluxed for 90 minutes, treated with additional acetone (0.562 mL), refluxed for 3.5 hours, stirred at room temperature overnight, concentrated *in vacuo,* and chased with ethyl acetate to obtain a residue. Column chromatography of the residue using silica gel and an ethyl acetate/hexanes solution (1:3) gives the title product as a white solid (1.5 g, mp 67-80°C).

Using essentially the same procedure, the following compounds are obtained:

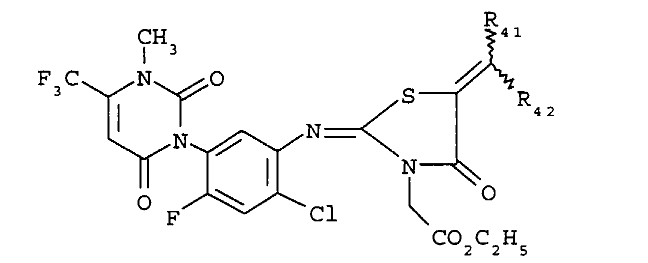

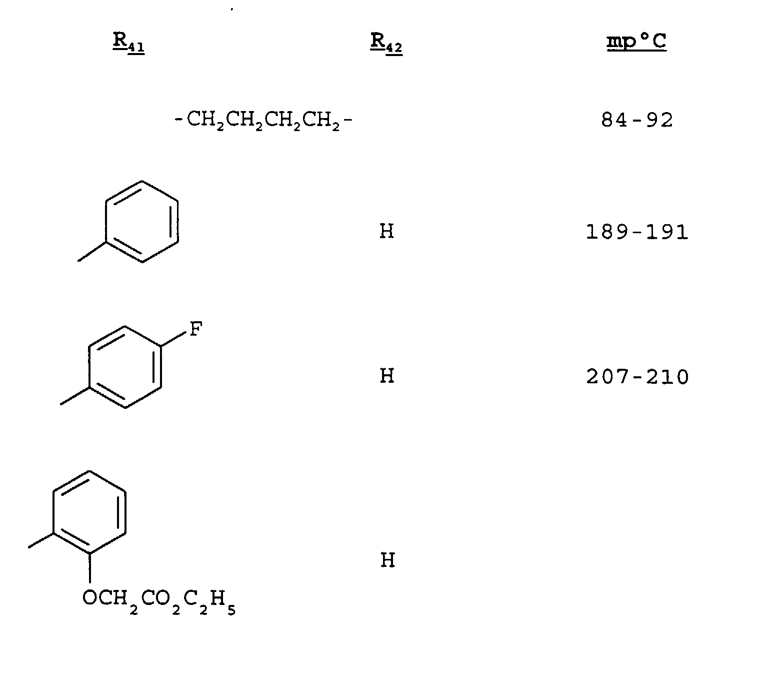

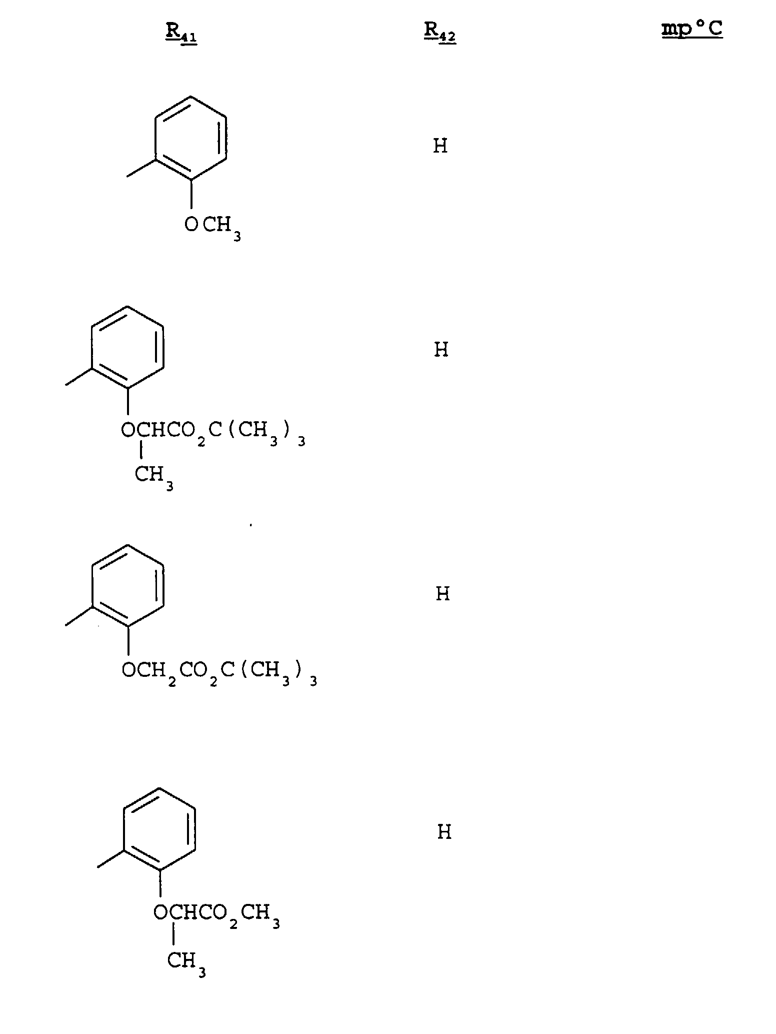

### EXAMPLE 31

### Preparation of 2-{{2-Chloro-5-[3,6-dihydro-3-methyl-2,6-dioxo-4-(trifluoromethyl)-1(2H)-pyrimidinyl]-4-fluorophenyl}imino}-5-(o-hydroxybenzyl)-4-oxo-3-thiazolidine-acetic acid, ethyl ester, ester with ethyl glycolate

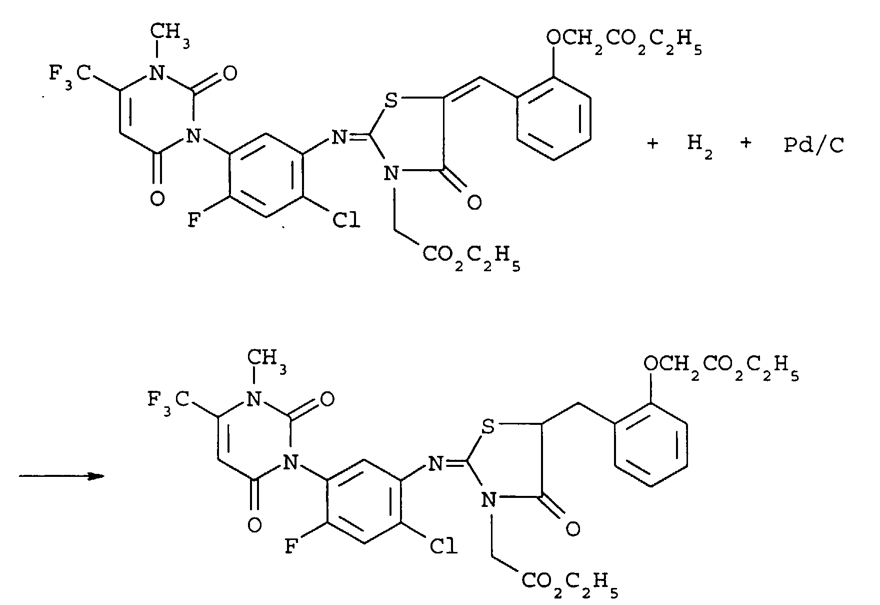

A mixture of 5-[*o*-(carboxymethoxy)benzylidene]-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetic acid, diethyl ester (2.1 g, 2.95 mmol) and 10% palladium on carbon (2.0 g) in ethyl acetate is hydrogenated at 50 psi for 72 hours at room temperature, and filtered to remove solids. The resultant filtrate is concentrated *in vacuo* to give a residue. Column chromatography of the residue using silica gel and an ethyl acetate/hexanes solution (1:2) gives the title product as a white solid (0.79 g, mp 47-60°C) which is identified by ¹H NMR spectral analysis.

Using essentially the same procedure, the following compounds are obtained:

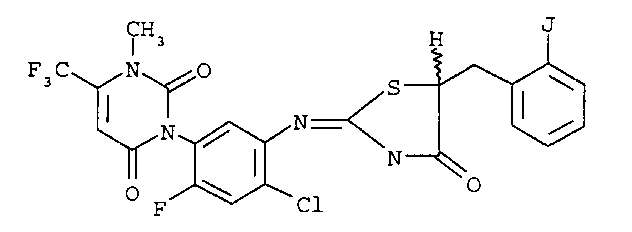

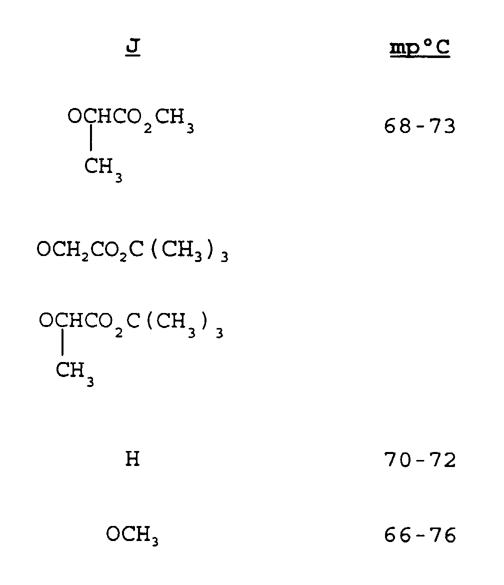

### EXAMPLE 32

### Preparation of 5-[o-(Carboxymethoxy)benzyl]-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetic acid, 3-ethyl ester

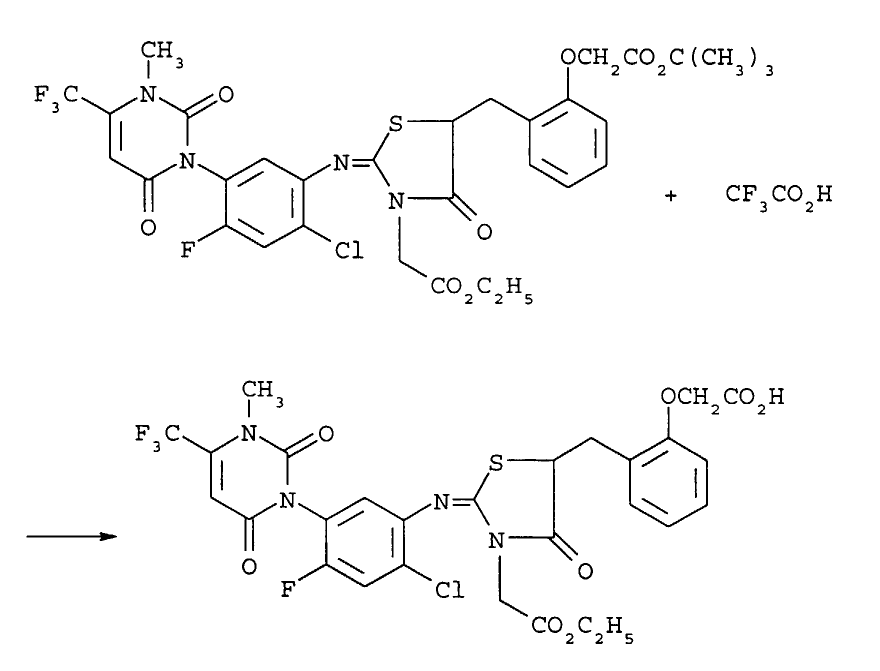

A solution of 5-[*o*-(carboxymethoxy)benzyl]-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetic acid, 5-tert-butyl ethyl ester (2.2 g, 2.96 mmol) in methylene chloride is treated with trifluoroacetic acid (5.5 mL, 71.4 mmol), stirred at room temperature for 7.5 hours, concentrated *in vacuo,* and chased with toluene and hexanes to obtain a residue. The residue is disolved in diethyl ether, diluted with hexanes, held overnight at 0°C, and filtered to give the title product as a white solid (2.0 g, mp 93-98°C).

Using essentially the same procedure, the following compound is obtained:

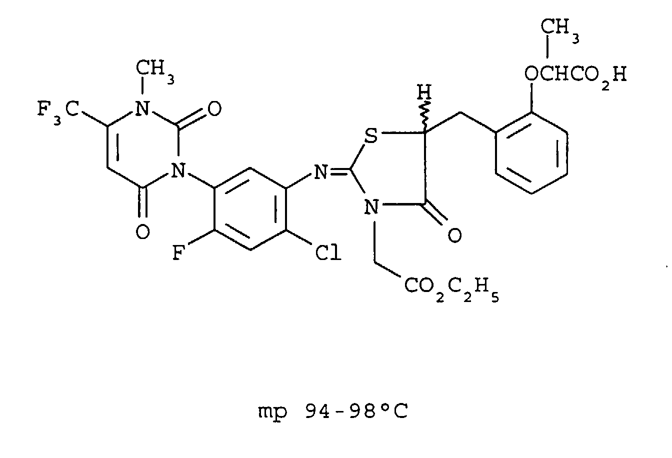

### EXAMPLE 33

### Preparation of 2-{{2-Chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetic acid, ethyl ester, 1-oxide

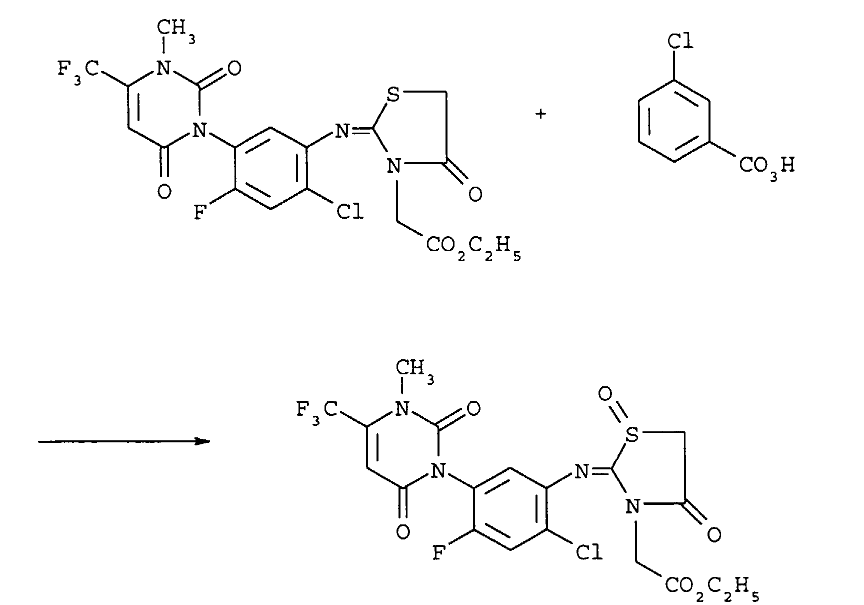

A solution of ethyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate (3.00 g, 5.74 mmol) in methylene chloride is treated with 3-chloroperoxybenzoic acid (1.23 g, 57%-86% real), stirred at room temperature for 3 hours, and poured into saturated sodium hydrogen carbonate solution. After separating the phases, the organic phase is washed with saturated sodium hydrogen carbonate solution, dried over anhydrous magnesium sulfate, and concentrated *in vacuo* to obtain a residue. Column chromatography of the residue using silica gel and an ethyl acetate/hexanes solution (2:3) gives the title product as a yellow solid (1.0 g, mp 105-115°C) which is identified by ¹H and ¹³C NMR spectral analyses.

Following essentially the same procedure, but using at least 2 equivalents of 3-chloroperoxybenzoic acid, 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetic acid, ethyl ester, 1,1-dioxide is obtained as a white solid (mp 75-88°C).

### EXAMPLE 34

### Preparation of Ethyl 2-{{2-chloro-5-[3,6-dihydro-3-methyl-2,6-dioxo-4-(trifluoromethyl)-1(2H)-pyrimidinyl]-4-fluorophenyl}imino}-5-(hydroxyethyl)-4-oxo-3-thiazolidineacetate

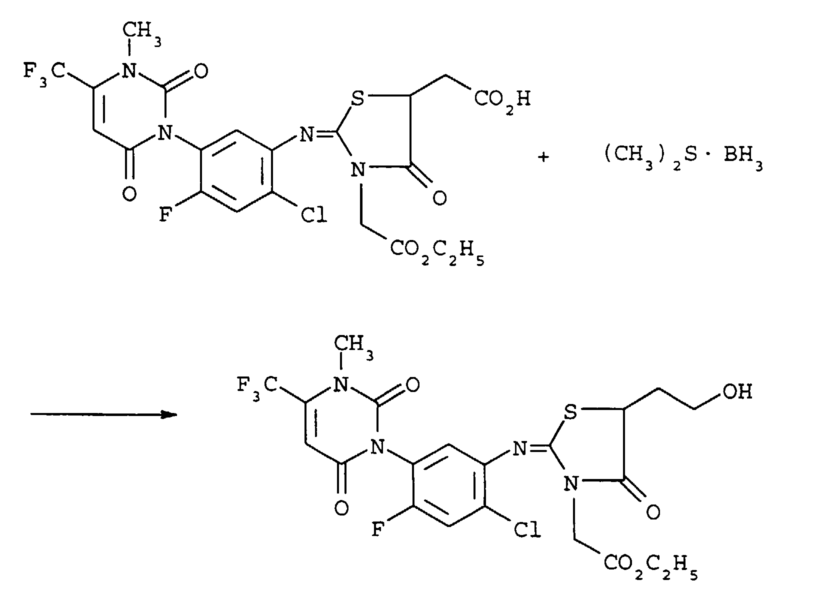

A mixture of 2-{{2-chloro-5-[3,6-dihydro-3-methyl-2,6-dioxo-4-(trifluoromethyl)-1(2H)-pyrimidinyl]-4-fluorophenyl}-4-oxo-3,5-thiazolidinediacetic acid, 3-ethyl ester (2.0 g, 3.44 mmol) and borane-methyl sulfide complex (0.36 g, 3.8 mmol) in tetrahydrofuran is stirred at 40-45°C for 2 days with additional borane-methyl sulfide complex (2 x 0.36 g) being added at equal intervals. The reaction mixture is then cooled to room temperature, treated with methanol (5 mL), stirred overnight at room temperature, and concentrated *in vacuo* to obtain a glass. Column chromatography of the glass using silica gel and 2% to 10% ethyl acetate in methylene chloride solutions gives the title product (0.46 g, mp 137-139°C).

### EXAMPLE 35

### Preparation of 2-{{2-Chloro-5-[3,6-dihydro-3-methyl-2,6-dioxo-4-(trifluoromethyl)-1(2H)-pyrimidinyl]-4-fluorophenyl}imino}-4-oxo-3,5-oxazolidinediacetic acid, 3-ethyl ester

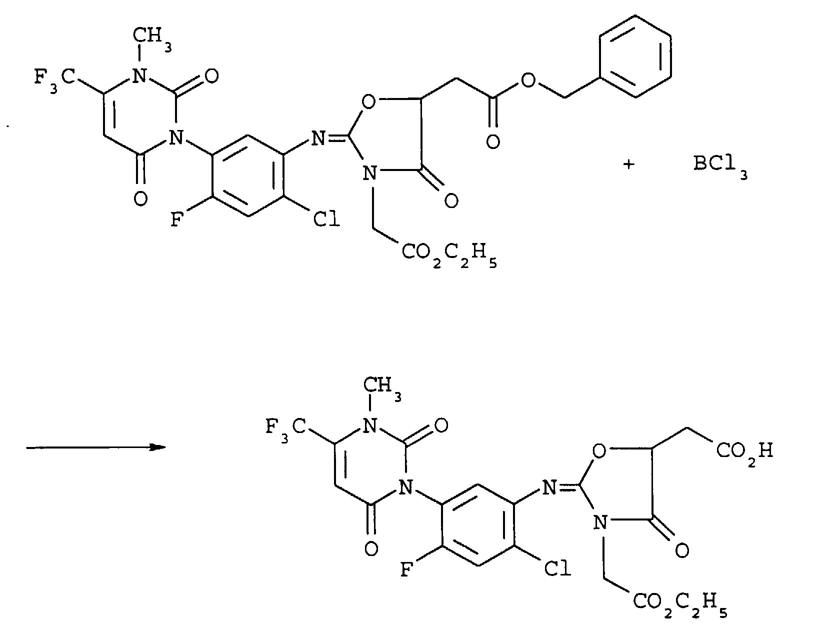

A solution of 2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-oxazolidinediacetic acid, 5-benzyl ethyl ester (0.65 g, 1.0 mmol) in methylene chloride is cooled to 0°C, treated with boron trichloride (1 mL of a 1M solution in methylene chloride), stirred at 0°C for 30 minutes, treated with additional boron trichloride (1 mL of a 1M solution in methylene chloride), stirred at room temperature for 90 minutes, and poured into an ice-water mixture. The resultant aqueous mixture is extracted with methylene chloride. The organic extracts are combined, washed with brine, dried over anhydrous magnesium sulfate, and concentrated *in vacuo* to give the title product (0.2 g) which is identified by ¹H and ¹³C NMR spectral analyses.

### EXAMPLE 36

### Preparation of 2-{{2-Chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetic acid

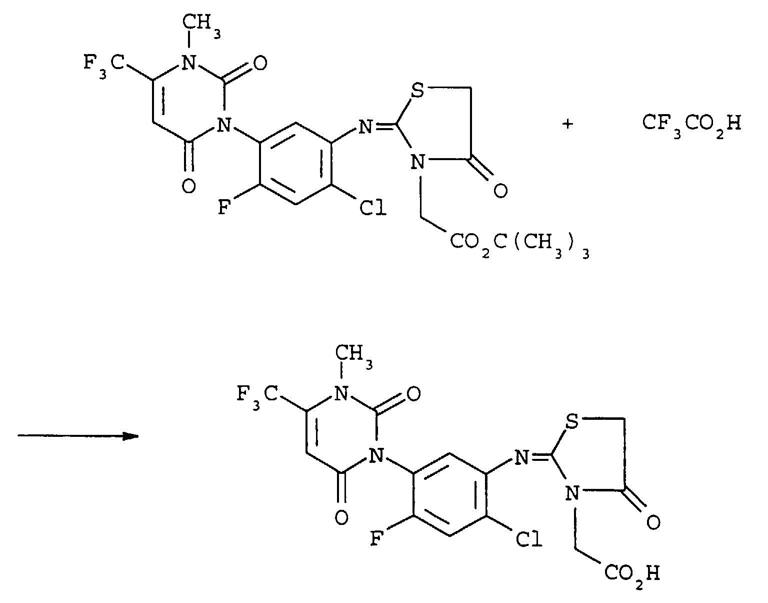

A solution of tert-butyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate (41.93 g, 76.1 mmol) in trifluoroacetic acid is stirred at room temperature for 3 hours, stirred at 35°C for 1 hour, and concentrated *in vacuo* to obtain a yellow liquid. A solution of the yellow liquid in diethyl ether is cooled and filtered to obtain a solid. The solid is washed with diethyl ether and air-dried to give the title product as a white solid (31.6 g) which is identified by ¹H and ¹³C NMR spectral analyses.

### EXAMPLE 37

### Postemergence herbicidal evaluation of test compounds

The postemergence herbicidal activity of the compounds of the present invention is demonstrated by the following tests wherein a variety of dicotyledonous and monocotyledonous plants are treated with test compounds. In the tests, seedling plants are grown in jiffy flats for about two weeks. The test compounds are dispersed in 50/50 acetone/water mixtures containing 0.5% TWEEN®20, a polyoxyethylene sorbitan monolaurate surfactant of Atlas Chemical Industries, in sufficient quantities to provide the equivalent of about 0.0156 kg to 0.500 kg per hectare of active compound when applied to the plants through a spray nozzle operating at 40 psi for a predetermined time. After spraying, the plants are placed on greenhouse benches and are cared for in accordance with conventional greenhouse procedures. From four to five weeks after treatment, the seedling plants are examined and rated according to the rating system set forth below. Data obtained are reported in Table I below. Where more than one test is involved for a given compound, the data are averaged.

Plant species employed in these evaluations are reported by header abbreviation, common name and scientific name.

Compounds employed in this postemergence herbicidal evaluation are given a compound number and identified by name. Data in Table I are reported by compound number.

### Herbicide Rating Scale

Results of herbicide evaluation are expressed on a rating scale (0-9). The scale is based upon a visual observation of plant stand, vigor, malformation, size, chlorosis and overall plant appearance as compared with a control.

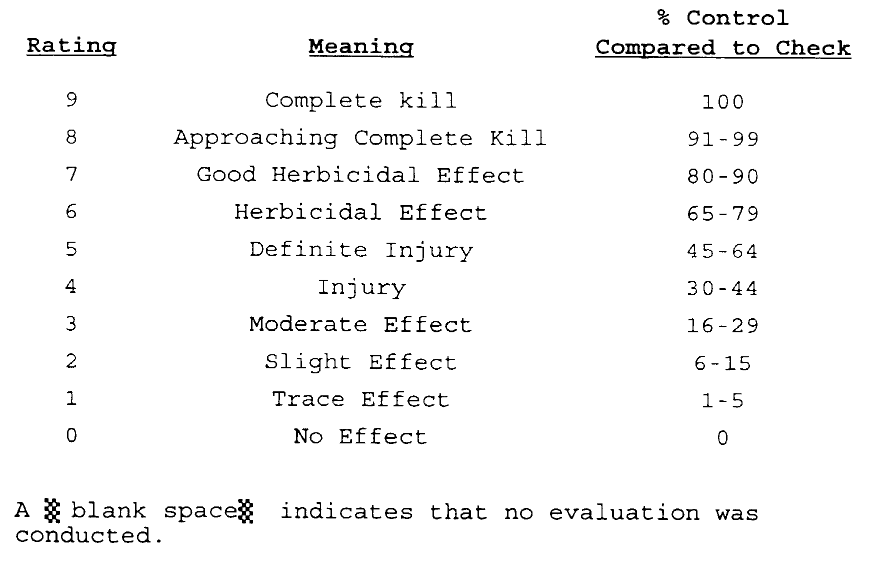

| **PLANT SPECIES EMPLOYED IN HERBICIDAL EVALUATIONS** | | |
|---|---|---|
| **Header Abbr.** | **Common Name** | **Scientific Name** |
| ABUTH | Velvetleaf | *Abutilon theophrasti,* Medic. |
| | | |
| AMBEL | Ragweed, Common | *Ambrosia artemisifolia,* L. |
| | | |
| CASOB | Sicklepod | *Cassia obtusifolia,* L. |
| | | |
| CHEAL | Lambsquarters, Common | *Chenopodium album,* L. |
| | | |
| GALAP | Galium | *Galium aparine* |
| | | |
| IPOHE | Morningglory, Ivyleaf | *Ipomoea hederacea,* (L.) Jacq. |
| | | |
| IPOSS | Morningglory Spp. | *Ipomoea* Spp. |
| | | |
| ECHCG | Barnyardgrass | *Echinochloa crus-galli,* (L.) Beau |
| | | |
| SETVI | Foxtail, Green | *Setaria viridis,* (L.) Beau |
| | | |
| GLXMAW | Soybean, Williams | *Glycine max,* (L.) Merr. cv Williams |
| | | |
| GLXMA | Soybean | *Glycine max,* (L.) Merr. |
| | | |
| ORYSAT | Rice, Tebonnet | *Oryza sativa,* (L.) Tebonnet |
| | | |
| TRZAWR | Wheat, Winter, cv Riband | *Triticum aestivum,* cv Riband |
| | | |
| ZEAMX | Corn, Field | *Zea mays,* L. |

| **COMPOUNDS EVALUATED AS HERBICIDAL AGENTS** | |
|---|---|
| **Compound Number** | |
| 1 | N-{4-chloro-2-fluoro-5-[(3-methyl-4-oxo-2-thiazolidinylidene)amino]phenyl}-1-cyclohexene-1,2-dicarboximide |
| | |
| 2 | N-{5-[(3-allyl-4-oxo-2-thiazolidinylidene)amino]-4-chloro-2-fluorophenyl}-1-cyclohexene-1,2-dicarboximide |
| | |
| 3 | N-{4-chloro-2-fluoro-5-[(4-oxo-3-phenyl-2-thiazolidinylidene)amino]phenyl}-1-cyclohexene-1,2-dicarboximide |
| | |
| 4 | N-{4-chloro-2-fluoro-5-(3,5-dimethyl-4-oxo-2-thiazolidinylidene)amino]phenyl}-1-cyclohexene-1,2-dicarboximide |
| | |
| 5 | N-{4-chloro-2-fluoro-5-[(5-fluoro-3-methyl-4-oxo-2-thiazolidinylidene)amino]phenyl}-1-cyclohexene-1,2-dicarboximide |
| | |
| 6 | N-{4-chloro-5-[(5-ethyl-3-methyl-4-oxo-2-thiazolidinylidene)amino]-2-fluorophenyl}-1-cyclohexene-1,2-dicarboximide |
| | |
| 7 | 3-{4-Chloro-2-fluoro-5-[3-methyl-4-oxo-2-thiazolidinylidene)amino]phenyl}-1-methyl-6-(trifluoromethyl)-2,4(1H,3H)-pyrimidinedione |
| | |
| 8 | Ethyl 2-{{2-chloro-5-[3,6-dihydro-3-methyl-2,6-dioxo-4-(trifluoromethyl)-1(2H)pyrimidinyl]-4-fluorophenyl}imino}-4-oxo-3-thiazolidineacetate |
| | |
| 9 | 3-{4-Chloro-5-[(3,5-dimethyl-4-oxo-2-thiazolidinyl-idene)amino]-2-fluorophenyl}-1-methyl-6-trifluoromethyl)-2,4(1H,3H)-pyrimidinedione |
| | |
| 10 | 3-{4-Chloro-5-[(5-ethyl-3-methyl-4-oxo-2-thiazolidinylidene)amino]-2-fluorophenyl}-1-methyl-6-trifluoromethyl)-2,4(1H,3H)-pyrimidinedione |
| | |
| 11 | Ethyl 2-{{2-chloro-5-[3,6-dihydro-3-methyl-2,6-dioxo-4-(trifluoromethyl)-1(2H)-pyrimidinyl]-4-fluorophenyl}imino}-5-fluoro-4-oxo-3-thiazolidineacetate |
| | |
| 12 | Ethyl 2-{{2-chloro-5-[3,6-dihydro-3-methyl-2,6-dioxo-4-(trifluoromethyl)-1(2H)-pyrimidinyl]-4-fluorophenyl}imino-5-methyl-4-oxo-3-thiazolidineacetate |
| | |
| 13 | Ethyl 2-{{2-chloro-5-[3,6-dihydro-3-methyl-2,6-dioxo-4-(trifluoromethyl)-1(2H)-pyrimidinyl]-4-fluorophenyl}imino}-5-ethyl-4-oxo-3-thiazolidineacetate |
| | |
| 14 | 3-(Carboxymethyl)-2-{{2-chloro-5-[3,6-dihydro-3-methyl-2,6-dioxo-4-(trifluoromethyl)-1(2H)-pyrimidinyl]-4-fluorophenyl}imino}-4-oxo-delta5,alpha-thiazolidineacetic acid, diethyl ester |
| | |
| 15 | Ethyl 5-{{2-chloro-5-[3,6-dihydro-3-methyl-2,6-dioxo-4-(trifluoromethyl)-1(2H)-pyrimidinyl]-4-fluorophenyl}imino}-7-oxo-4-thia-6-azaspiro[2.4]-heptane-6-acetate |
| | |
| 16 | 2-{{2-Chloro-5-[3,6-dihydro-3-methyl-2,6-dioxo-4-(trifluoromethyl)-1(2H)-pyrimidinyl]-4-fluorophenyl}-4-oxo-3,5-thiazolidinediacetic acid, 3-ethyl ester |
| | |
| 17 | Methyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate |
| | |
| 18 | Ethyl 2-{{2-chloro-5-[3,6-dihydro-3-methyl-2,6-dioxo-4-(trifluoromethyl)-1(2H)-pyrimidinyl]-4-fluorophenyl}imino}-5-cyclopentylidene-4-oxo-3-thiazolidineacetate |
| | |
| 19 | Ethyl 5-benzylidene-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate |
| | |
| 20 | Ethyl 2-{{2-chloro-5-[3,6-dihydro-3-methyl-2,6-dioxo-4-(trifluoromethyl)-1(2H)-pyrimidinyl]-4-fluorophenyl}imino}-5-isopropylidene-4-oxo-3-thiazolidineacetate |
| | |
| 21 | Ethyl 2-{{2-chloro-5-[3,6-dihydro-3-methyl-2,6-dioxo-4-(trifluoromethyl)-1(2H)-pyrimidinyl]-4-fluorophenyl}imino}-5-(*p*-fluorobenzylidene)-4-oxo-3-thiazolidineacetate |
| | |
| 22 | Ethyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-oxazolidineacetate |
| | |
| 23 | 3-{4-Chloro-5-{[3-(dimethylamino)-4-oxo-2-thiazolidinylidine]amino}-2-fluorophenyl}-1-methyl 6-(trifluoromethyl)-2,4(1H,3H)-pyrimidinedione |
| | |
| 24 | Ethyl 5-benzyl-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate |
| | |
| 25 | Ethyl 2-{{2-chloro-5-[3,6-dihydro-3-methyl-2,6-dioxo-4-(trifluoromethyl)-1(2H)-pyrimidinyl]-4-fluorophenyl}imino}-3-methyl-5-oxo-1-imidazolidineacetate |
| | |
| 26 | 3-{5-[(3-Benzyl-4-oxo-2-thiazolidinylidene)amino]-4-chloro-2-fluorophenyl}-1-methyl-6-(trifluoromethyl)-2,4(1H,3H)-pyrimidinedione |
| | |
| 27 | 3-{5-[(3-Allyl-4-oxo-2-thiazolidinylidene)amino]-4-chloro-2-fluorophenyl}-1-methyl-6-(trifluoromethyl)-2,4(1H,3H)-pyrimidinedione |
| | |
| 28 | Methyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidinepropionate |
| | |
| 29 | Methyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidinebutyrate |
| | |
| 30 | 2-{{2-Chloro-5-[3,6-dihydro-3-methyl-2,6-dioxo-4-(trifluoromethyl)-1(2H)-pyrimidinyl]-4-fluorophenyl}imino}-4-oxo-3-thiazolidinepropionic acid |
| | |
| 31 | 2-{{2-Chloro-5-[3,6-dihydro-3-methyl-2,6-dioxo-4-(trifluoromethyl)-1(2H)-pyrimidinyl]-4-fluorophenyl}imino}-4-oxo-3-thiazolidinepropionic acid |
| | |
| 32 | Ethyl 2-{{2,4-dichloro-5-[3,6-dihydro-3-methyl-2,6-dioxo-4-(trifluoromethyl)-1(2H)-pyrimidinyl]-phenyl}imino}-4-oxo-3-thiazolidinepropionate |
| | |
| 33 | 2-{{2,4-Dichloro-5-[3,6-dihydro-3-methyl-2,6-dioxo-4-(trifluoromethyl)-1(2H)-pyrimidinyl]phenyl}-imino}-4-oxo-3,5-thiazolidinediacetic acid, 3-ethyl ester |
| | |
| 34 | 2-{{2-Chloro-5-[3,6-dihydro-3-methyl-2,6-dioxo-4-(trifluoromethyl)-1(2H)-pyrimidinyl]-4-fluorophenyl}imino}-4-oxo-1,3-imidazolidinediacetic acid, diethyl ester |
| | |
| 35 | Diethyl {{2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidinyl}methyl}phosphonate |
| | |
| 36 | Ethyl 2-{{2-chloro-5-[4-(difluoromethyl-4,5-dihydro-3-methyl-5-oxo-1H-1,2,4-triazol-1-yl]-4-fluorophenyl}imino}-3-thiazolidineacetate |
| | |
| 37 | Ethyl 2-{{2-chloro-5-[3,6-dihydro-3-methyl-2,6dioxo-4-(trifluoromethyl)-1(2H)-pyrimidinyl]-4-fluorophenyl}imino}-5-(*o*-methoxybenzyl)-4-oxo-3-thiazolidineacetate |
| | |
| 38 | 2-{{2-Chloro-5-[3,6-dihydro-3-methyl-2,6-dioxo-4-(trifluoromethyl)-1(2H)-pyrimidinyl]-4-fluorophenyl}imino}-N-(methylsulfonyl)-4-oxo-3-thiazolidineacetamide |
| | |
| 39 | 3-{4-Chloro-2-fluoro-5-[(4-oxo-2-thiazolidinylidene)amino]phenyl}-1-methyl-6-(trifluoromethyl)-2,4(1H,3H)-pyrimidinedione |
| | |
| 40 | 2-{{2-Chloro-5-[3,6-dihydro-3-methyl-2,6-dioxo-4-(trifluoromethyl)-1(2H)-pyrimidinyl]-4-fluorophenyl}imino)-4-oxo-3,5-oxazolidinediacetic acid, 5-benzyl ethyl ester |
| | |
| 41 | 2-{{2-Chloro-5-[3,6-dihydro-3-methyl-2,6-dioxo-4-(trifluoromethyl)-1(2H)-pyrimidinyl]-4-fluorophenyl}imino}-4-oxo-3,5-oxazolidinediacetic acid, 3-ethyl ester |
| | |
| 42 | 2-{{2-Chloro-5-[3,6-dihydro-3-methyl-2,6-dioxo-4-(trifluoromethyl)-1(2H)-pyrimidinyl]-4-fluorophenyl}imino}-5-(*o*-hydroxybenzyl)-4-oxo-3-thiazolidineacetic acid, ethyl ester, ester with ethyl glycolate |
| | |
| 43 | *tert*-Butyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate |
| | |
| 44 | 3-Allyl-2-{{2-chloro-5-[3,6-dihydro-3-methyl-2,6-dioxo-4-(trifluoromethyl)-1(2H)-pyrimidinyl]-4-fluorophenyl}imino}-4-oxo-5-thiazolidineacetanilide |
| | |
| 45 | 2-{{2-Chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetic acid, ethyl ester, 1-oxide |
| | |
| 46 | 2-{{2-Chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetic acid, ethyl ester, 1,1-dioxide |
| | |
| 47 | Ethyl 2-{[2-chloro-5-(1-cyclohexene-1,2-dicarboximido)-4-fluorophenyl]imino}-4-oxo-3-thiazolidineacetate |
| | |
| 48 | 2-{[2-Chloro-5-(1-cyclohexene-1,2-dicarboximido)-4-fluorophenyl]imino}-4-oxo-3,5-thiazolidinediacetic acid |
| | |
| 49 | Ethyl 2-((2-chloro-5-[3,6-dihydro-3-methyl-2,6-dioxo-4-(trifluoromethyl)-1(2H)-pyrimidinyl]-4-fluorophenyl}imino}-5-(hydroxyethyl)-4-oxo-3-thiazolidineacetate |
| | |
| 50 | 2-{{2-Chloro-5-[3,6-dihydro-3-methyl-2,6-dioxo-4-(trifluoromethyl)-1(2H)-pyrimidinyl]-4-fluorophenyl}imino}-5-(*o*-hydroxybenzyl)-4-oxo-3-thiazolidineacetic acid, ethyl ester, ester with methyl lactate |
| | |
| 51 | 2-{{2-Chloro-5-[3,6-dihydro-3-methyl-2,6-dioxo-4-(trifluoromethyl)-1(2H)-pyrimidinyl]-4-fluorophenyl}imino}-α-methyl-4-oxo-3-thiazolidineacetic acid, L- |
| | |
| 52 | 2-{{2-Chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetic acid |
| | |
| 53 | α-Benzyl-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetic acid, L- |
| | |
| 54 | 2-{{2-Chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid, 3-ethyl methyl ester |
| | |
| 55 | 2-{{2-Chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid, 3-ethyl isopropyl ester |
| | |
| 56 | Ethyl 5-(carbamoylmethyl)-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate |
| | |
| 57 | Ethyl 2-{{2-chloro-5-[3,6-dihydro-3-methyl-2,6-dioxo-4-(trifluoromethyl)-1(2H)-pyrimidinyl]-4-fluorophenyl}imino}-5-[(methylcarbamoyl)methyl]-4-oxo-3-thiazolidineacetate |
| | |
| 58 | 2-{{2-Chloro-5-[3,6-dihydro-3-methyl-2,6-dioxo-4-(trifluoromethyl)-1(2H)-pyrimidinyl]-4-fluorophenyl}imino}-4-oxo-3,5-thiazolidenediacetic acid, 3-ethyl (2-propynyl) ester, (2:1) mixture with di-2-propynyl ester |
| | |
| 59 | 2-{{2-Chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid, 3-ethyl (2-propynyl) ester |
| | |
| 60 | 2-{{2-Chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid, 3-ethyl isopropyl ester |
| | |
| 61 | 2-{{2-Chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid |
| | |
| 62 | 3-Allyl-2-{{2-chloro-5-[3,6-dihydro-3-methyl-2,6-dioxo-4-(trifluoromethyl)-1(2H)-pyrimidinyl]-4-fluorophenyl}imino}-4-oxo-5-thiazolidineacetic acid |
| | |
| 63 | 2-{{2-Chloro-5-[3,6-dihydro-3-methyl-2,6-dioxo-4-(trifluoromethyl)-1(2H)-pyrimidinyl]-4-fluorophenyl}imino}-3-methyl-4-oxo-5-thiazolidineacetic acid |
| | |
| 64 | Methyl 3-allyl-2-{{2-chloro-5-[3,6-dihydro-3-methyl-2,6-dioxo-4-(trifluoromethyl)-1(2H)-pyrimidinyl]-4-fluorophenyl}imino}-4-oxo-5-thiazolidineacetate |
| | |
| 65 | Allyl 3-allyl-2-{{2-chloro-5-[3,6-dihydro-3-methyl-2,6-dioxo-4-(trifluoromethyl)-1(2H)-pyrimidinyl]-4-fluorophenyl}imino}-4-oxo-5-thiazolidineacetate |
| | |
| 66 | Isopropyl 3-allyl-2-{{2-chloro-5-[3,6-dihydro-3-methyl-2,6-dioxo-4-(trifluoromethyl)-1(2H)-pyrimidinyl]-4-fluorophenyl}imino}-4-oxo-5-thiazolidineacetate |
| | |
| 67 | 2-Propynyl 3-allyl-2-{{2-chloro-5-[3,6-dihydro-3-methyl-2,6-dioxo-4-(trifluoromethyl)-1(2H)-pyrimidinyl]-4-fluorophenyl}imino}-4-oxo-5-thiazolidineacetate |
| | |
| 68 | Ethyl 2-{{2-chloro-5-[3,6-dihydro-3-methyl-2,6-dioxo-4-(trifluoromethyl)-1(2H)-pyrimidinyl]-4-fluorophenyl}imino}-4-oxo-5-[(phenylcarbamoyl)-methyl]-3-thiazolidineacetate |
| | |
| 69 | α-Benzyl-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetic acid, D- |
| | |
| 70 | 2-{{2-Chloro-5-[3,6-dihydro-3-methyl-2,6-dioxo-4-(trifluoromethyl)-1(2H)-pyrimidinyl]-4-fluorophenyl}imino}-α-methyl-4-oxo-3-thiazolidineacetic acid, D- |
| | |
| 71 | α-Isobutyl-2-{{2-chloro-5-[3,6-dihydro-3-methyl2,6-dioxo-4-(trifluoromethyl)-1(2H)-pyrimidinyl]-4-fluorophenyl}imino}-4-oxo-3-thiazolidineacetic acid, L- |
| | |
| 72 | Methyl α-benzyl-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate, L- |
| | |
| 73 | 3-{4-Chloro-2-fluoro-5-{[4-oxo-3-(tetrahydro-2-oxo-3-furyl)-2-thiazolidinylidene]amino}phenyl-1-methyl-6-(trifluoromethyl)-2,4(1H,3H)-pyrimidinedione |
| | |
| 74 | Methyl α-benzyl-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate, D- |
| | |
| 75 | *tert*-Butyl 2-{{2-chloro-5-[3,6-dihydro-3-methyl-2,6-dioxo-4-(trifluoromethyl)-1(2H)-pyrimidinyl]-4-fluorophenyl}imino}-α-methyl-4-oxo-3-thiazolidineacetate, L- |
| | |
| 76 | Ethyl 2-{{2-chloro-5-[3,6-dihydro-3-methyl-2,6-dioxo-4-(trifluoromethyl)-1(2H)-pyrimidinyl]-4-fluorophenyl}imino}-α-methyl-4-oxo-3-thiazolidineacetate, L- |
| | |
| 77 | Methyl α-(*p*-chlorobenzyl)-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate, DL- |
| | |
| 78 | Methyl 2-{{2-chloro-5-[3,6-dihydro-3-methyl-2,6-dioxo-4-(trifluoromethyl)-1(2H)-pyrimidinyl]-4-fluorophenyl}imino}-4-oxo-α-(*p*-nitrobenzyl)-3-thiazolidineacetate, L- |
| | |
| 79 | Ethyl α-(*p*-chlorophenyl)-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate, DL- |
| | |
| 80 | Ethyl α-benzyl-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate, L- |
| | |
| 81 | Methyl 1-{2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidinyl}-cyclohexanecarboxylate |
| | |
| 82 | 2-{{2-Chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid, 3-ethyl 2-fluoroethyl ester |
| | |
| 83 | Ethyl 2-{{2-chloro-5-[3,6-dihydro-3-methyl-2,6-dioxo-4-(trifluoromethyl)-1(2H)-pyrimidinyl]-4-fluorophenyl}imino}-5-[(methoxycarbamoyl)methyl]-4-oxo-3-thiazolidineacetate |
| | |
| 84 | 3-Allyl-2-{{2-chloro-5-[3,6-dihydro-3-methyl-2,6-dioxo-4-(trifluoromethyl)-1(2H)-pyrimidinyl]-4-fluorophenyl}imino}-N-methyl-4-oxo-5-thiazolidineacetamide |
| | |
| 85 | 3-Allyl-2-{{2-chloro-5-[3,6-dihydro-3-methyl-2,6-dioxo-4-(trifluoromethyl)-1(2H)-pyrimidinyl]-4-fluorophenyl}imino}-N-methoxy-4-oxo-5-thiazolidineacetamide |
| | |
| 86 | 2-Fluoroethyl 3-allyl-2-{{2-chloro-5-[3,6-dihydro-3-methyl-2,6-dioxo-4-(trifluoromethyl)-1(2H)-pyrimidinyl]-4-fluorophenyl}imino}-4-oxo-5-thiazolidineacetate |
| | |
| 87 | 2-{{2-Chloro-5-[3,6-dihydro-3-methyl-2,6-dioxo-1(2H)-pyrimidinyl]-4-fluorophenyl}imino}-4-oxo-3,5-thiazolidinediacetic acid, 3-ethyl ester, 5-ester with ethyl lactate |
| | |
| 88 | Methyl 2-{{2-chloro-5-[3,6-dihydro-3-methyl-2,6-dioxo-4-(trifluoromethyl)-1(2H)-pyrimidinyl]-4-fluorophenyl}imino}-3-methyl-4-oxo-5-thiazolidineacetate |
| | |
| 89 | Isopropyl 2-{{2-chloro-5-[3,6-dihydro-3-methyl-2,6-dioxo-4-(trifluoromethyl)-1(2H)-pyrimidinyl]-4-fluorophenyl}imino}-3-methyl-4-oxo-5-thiazolidineacetate |
| | |
| 90 | 2-Propynyl 2-{{2-chloro-5-[3,6-dihydro-3-methyl-2,6-dioxo-4-(trifluoromethyl)-1(2H)-pyrimidinyl]-4-fluorophenyl}imino}-3-methyl-4-oxo-5-thiazolidineacetate |
| | |
| 91 | 2-{{2-Chloro-5-[3,6-dihydro-3-methyl-2,6-dioxo-4-(trifluoromethyl)-1(2H)-pyrimidinyl]-4-fluorophenyl}imino}-3-methyl-4-oxo-5-thiazolidineacetanilide |
| | |
| 92 | 2-{{2-Chloro-5-[3,6-dihydro-3-methyl-2,6-dioxo-4-(trifluoromethyl)-1(2H)-pyrimidinyl]-4-fluorophenyl}imino}-3-methyl-4-oxo-5-thiazolidineacetamide |
| | |
| 93 | 2-Fluoroethyl 2-{{2-chloro-5-[3,6-dihydro-3-methyl-2,6-dioxo-4-(trifluoromethyl)-1(2H)-pyrimidinyl]-4-fluorophenyl}imino}-3-methyl-4-oxo-5-thiazolidineacetate |
| | |
| 94 | 3-Allyl-2-{{2-chloro-5-[3,6-dihydro-3-methyl-2,6-dioxo-1(2H)-pyrimidinyl]-4-fluorophenyl}imino}-4 oxo-5-thiazolidineacetic acid, ester with ethyl lactate, (R,S)- |
| | |
| 95 | Ethyl 2-{{2-chloro-5-[3,6-dihydro-3-methyl-2,6-dioxo-4-(trifluoromethyl)-1(2H)-pyrimidinyl]-4-fluorophenyl}imino}-5-{[(methylsulfonyl)carbamoyl]-methyl}-4-oxo-3-thiazolidineacetate |
| | |
| 96 | 3-Allyl-2-{{2-chloro-5-[3,6-dihydro-3-methyl-2,6-dioxo-4-(trifluoromethyl)-1(2H)-pryimidinyl]-4-fluorophenyl}imino}-N-(methylsulfonyl)-4-oxo-5-thiazolidineacetamide |
| | |
| 97 | 2-{{2-Chloro-5-[3,6-dihydro-3-methyl-2,6-dioxo-4-(trifluoromethyl)-1(2H)-pyrimidinyl]-4-fluorophenyl}imino}-3-methyl-N-(methylsulfonyl)-4-oxo-5-thiazolidineacetamide |
| | |
| 98 | Ethyl 2-{{2-chloro-5-[3,6-dihydro-3-methyl-2,6-dioxo-4-(trifluoromethyl)-1(2H)-pyrimidinyl]-4-fluorophenyl}imino}-5-[(hydroxycarbamoyl)methyl]-4-oxo-3-thiazolidineacetate |
| | |
| 99 | 3-Allyl-2-{{2-chloro-5-[3,6-dihydro-3-methyl-2,6-dioxo-4-(trifluoromethyl)-1(2H)-pyrimidinyl]-4-fluorophenyl}imino}-4-oxo-5-thiazolidineacetamide |
| | |
| 100 | Ethyl 2-{{2-chloro-5-[3,6-dihydro-3-methyl-2,6-dioxo-4-(trifluoromethyl)-1(2H)-pyrimidinyl]-4-fluorophenyl}imino}-4-oxo-5-{[(phenylsulfonyl)-carbamoyl]methyl}-3-thiazolidineacetate |
| | |
| 101 | 3-Allyl-2-{{2-chloro-5-[3,6-dihydro-3-methyl-2,6-dioxo-4-(trifluoromethyl)-1(2H)-pyrimidinyl]-4-fluorophenyl}imino}-4-oxo-N-(phenylsulfonyl)-5-thiazolidineacetamide |
| | |
| 102 | 2-{{2-Chloro-5-[3,6-dihydro-3-methyl-2,6-dioxo-4-(trifluoromethyl)-1(2H)-pyrimidinyl]-4-fluoro-phenyl}imino}-3-methyl-4-oxo-N-(phenylsulfonyl)-5-thiazolidineacetamide |
| | |
| 103 | Ethyl 2-{{2-chloro-5-[3,6-dihydro-3-methyl-2,6-dioxo-4-(trifluoromethyl)-1(2H)-pyrimidinyl]-4-fluorophenyl}imino}-5-{[(ethylsulfonyl)carbamoyl]-methyl}-4-oxo-3-thiazolidineacetate |
| | |
| 104 | 3-Allyl-2-{{2-chloro-5-[3,6-dihydro-3-methyl-2,6-dioxo-4-(trifluoromethyl)-1(2H)-pyrimidinyl]-4-fluorophenyl}imino}-N-(ethylsulfonyl)-4-oxo-5-thiazolidineacetamide |
| | |
| 105 | 2-{{2-Chloro-5-[3,6-dihydro-3-methyl-2,6-dioxo-4-(trifluoromethyl)-1(2H)-pyrimidinyl]-4-fluorophenyl}imino}-N-(ethylsulfonyl)-3-methyl-4-oxo-5-thiazolidineacetamide |
| | |
| 106 | Allyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate |
| | |
| 107 | 2-Methoxyethyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate |
| | |
| 108 | 2-{{2-Chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetic acid, ester with methyl glycolate |
| | |
| 109 | 2-{{2-Chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetic acid, ester with glycolonitrile |
| | |
| 110 | Isopropyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate |
| | |
| 111 | Butyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate |
| | |
| 112 | 2-{{2-Chloro-5-[4-(difluoromethyl)-4,5-dihydro-3-methyl-5-oxo-1H-1,2,4-triazol-1-yl]-4-fluorophenyl}imino}-4-oxo-3,5-thiazolidinediacetic acid, 3-ethyl ester |
| | |
| 113 | 5-[*o*-(Carboxymethoxy)benzyl]-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetic acid, 3-ethyl ester |
| | |
| 114 | 5-[*o*-(1-Carboxyethoxy)benzyl]-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetic acid, 3-ethyl ester |
| | |
| 115 | 2-Propynyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate |
| | |
| 116 | 2-{{2-Chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetic acid, ester with methyl lactate |
| | |
| 117 | Ethyl 2-{{2-chloro-5-[3,6-dihydro-3-methyl-2,6-dioxo-4-(trifluoromethyl)-1(2H)-pyrimidinyl]-4-fluorophenyl}imino}-5-{[(isopropylsulfonyl)-carbamoyl]methyl}-4-oxo-3-thiazolidineacetate |
| | |
| 118 | 3-Allyl-2-{{2-chloro-5-[3,6-dihydro-3-methyl-2,6-dioxo-4-(trifluoromethyl)-1(2H)-pyrimidinyl]-4-fluorophenyl}imino}-N-(isopropylsulfonyl)-4-oxo-5-thiazolidineacetamide |
| | |
| 119 | 2-{{2-Chloro-5-[3,6-dihydro-3-methyl-2,6-dioxo-4-(trifluoromethyl)-1(2H)-pyrimidinyl]-4-fluorophenyl}imino}-N-(isopropylsulfonyl)-3-methyl-4-oxo-5-thiazolidineacetamide |
| | |
| 120 | Cyclopentyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate |
| | |
| 121 | Isobutyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate |
| | |
| 122 | 3-{4-Chloro-2-fluoro-5-[(3-furfuryl-4-oxo-2-thiazolidinylidene)amino]phenyl}-1-methyl-6-(trifluoromethyl)-2,4(1H,3H)-pyrimidinedione |
| | |
| 123 | Cyclohexylmethyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate |
| | |
| 124 | Cyclopropylmethyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate |
| | |
| 125 | Ethyl 2-{{2-chloro-5-[3,6-dihydro-3-methyl-2,6-dioxo-4-(trifluoromethyl)-1(2H)-pyrimidinyl]-4-fluorophenyl}imino}-5-(methylthio)-4-oxo-3-thiazolidineacetate |
| | |
| 126 | Ethyl 5-chloro-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate |
| | |
| 127 | 5-Carboxy-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetic acid, diethyl ester |
| | |
| 128 | 5-Carboxy-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetic acid, 3-ethyl methyl ester |
| | |
| 129 | 3-{4-Chloro-2-fluoro-5-{[3-(*o*-methylbenzyl)-4-oxo-2-thiazolidinylidene]amino}phenyl}-1-methyl-6-(trifluoromethyl)-2,4(1H,3H)-pyrimidinedione |
| | |
| 130 | 3-{4-Chloro-2-fluoro-5-{[3-(*m*-methylbenzyl)-4-oxo-2-thiazolidinylidene]amino}phenyl}-1-mehyl-6-(trifluoromethyl)-2,4(1H,3H)-pyrimidinedione |
| | |
| 131 | 3-{4-Chloro-2-fluoro-5-{[4-oxo-3-(2-pyridylmethyl)-2-thiazolidinylidene]amino}phenyl}-1-methyl-6-(trifluoromethyl)-2,4(1H,3H)-pyrimidinedione |
| | |
| 132 | 3-{4-Chloro-2-fluoro-5-{[4-oxo-3-(3-pyridylmethyl)-2-thiazolidinylidene]amino}phenyl}-1-methyl-6-(trifluoromethyl)-2,4(1H,3H)-pyrimidinedione |
| | |
| 133 | 3-{4-Chloro-2-fluoro-5-{[4-oxo-3-(4-pyridylmethyl)-2-thiazolidinylidene]amino}phenyl}-1-methyl-6-(trifluoromethyl)-2,4(1H,3H)-pyrimidinedione |
| | |
| 134 | 3-{4-Chloro-5-{[3-(3,4-dimethoxybenzyl)-4-oxo-2-thiazolidinylidene]amino}-2-fluorophenyl}-1-methyl-6-(trifluoromethyl)-2,4(1H,3H)-pyrimidinedione |
| | |
| 135 | Alpha-{2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidinyl}-*p*-toluenesulfonamide |
| | |
| 136 | 3-{4-Chloro-5-{[3-(*m*-chlorobenzyl)-4-oxo-2-thiazolidinylidene]amino}-2-fluorophenyl}-1-methyl-6-(trifluoromethyl)-2,4(1H,3H)-pyrimidinedione |
| | |
| 137 | 3-{4-Chloro-2-fluoro-5-{[3-(*m*-methoxybenzyl)-4-oxo-2-thiazolidinylidene]amino}phenyl}-1-methyl-6-(trifluoromethyl)-2,4(1H,3H)-pyrimidinedione |
| | |
| 138 | 3-{4-Chloro-5-{{3-[p-(dimethylamino)benzyl]-4-oxo-2-thiazolidinylidene}amino}-2-fluorophenyl}-1-methyl-6-(trifluoromethyl)-2,4(1H,3H)-pyrimidinedione |
| | |
| 139 | 3-{4-Chloro-5-{[3-(2,4-dimethoxybenzyl)-4-oxo-2-thiazolidinylidene]amino}-2-fluorophenyl}-1-methyl-6-(trifluoromethyl)-2,4(1H,3H)-pyrimidinedione |
| | |
| 140 | 3-{4-Chloro-2-fluoro-5-{[3-(3,4,5-trimethoxybenzyl)-4-oxo-2-thiazolidinylidene]amino}phenyl}-1-methyl-6-(trifluoromethyl)-2,4(1H,3H)-pyrimidinedione |
| | |
| 141 | 3-{4-Chloro-2-fluoro-5-{{3-[*p*-(trifluoromethyl)benzyl]-4-oxo-2-thiazolidinylidene}amino}phenyl}-1-methyl-6-(trifluoromethyl)-2,4(1H,3H)-pyrimidinedione |
| | |
| 142 | 3-{4-Chloro-2-fluoro-5-{{3-[m-(trifluoromethyl)benzyl]-4-oxo-2-thiazolidinylidene}-amino}phenyl}-1-methyl-6-(trifluoromethyl)-2,4(1H,3H)-pyrimidinedione |
| | |
| 143 | 3-{4-Chloro-5-{[3-(*o*-chlorobenzyl)-4-oxo-2-thiazolidinylidene]amino}-2-fluorophenyl}-1-methyl-6-(trifluoromethyl)-2,4(1H,3H)-pyrimidinedione |
| | |
| 144 | 3-{4-Chloro-5-{[3-(3,4-dichlorobenzyl)-4-oxo-2-thiazolidinylidene]amino}-2-fluorophenyl}-1-methyl-6-(trifluoromethyl)-2,4(1H,3H)-pyrimidinedione |
| | |
| 145 | 3-{4-Chloro-5-{[3-(*p*-chlorobenzyl)-4-oxo-2-thiazolidinylidene]amino}-2-fluorophenyl}-1-methyl-6-(trifluoromethyl)-2,4(1H,3H)-pyrimidinedione |
| | |
| 146 | 3-{4-Chloro-5-{[3-(3,5-dimethylbenzyl)-4-oxo-2-thiazolidinylidene]amino}-2-fluorophenyl}-1-methyl-6-(trifluoromethyl)-2,4(1H,3H)-pyrimidinedione |
| | |
| 147 | 3-{4-Chloro-5-{[3-(3,4-dimethylbenzyl)-4-oxo-2-thiazolidinylidene]amino}-2-fluorophenyl}-1-methyl-6-(trifluoromethyl)-2,4(1H,3H)-pyrimidinedione |
| | |
| 148 | 3-{5-{[3-(*p*-Bromobenzyl)-4-oxo-2-thiazolidinylidene]amino}-4-chloro-2-fluorophenyl}-1-methyl-6-(trifluoromethyl)-2,4(1H,3H)-pyrimidinedione |
| | |
| 149 | 3-{4-Chloro-5-{[3-(3-chloro-4-methylbenzyl)-4-oxo-2-thiazolidinylidene]amino}-2-fluorophenyl}-1-methyl-6-(trifluoromethyl)-2,4(1H,3H)-pyrimidinedione |
| | |
| 150 | 3-{4-Chloro-5-{[3-(3,5-dichlorobenzyl)-4-oxo-2-thiazolidinylidene]amino}-2-fluorophenyl}-1-methyl-6-(trifluoromethyl)-2,4(1H,3H)-pyrimidinedione |
| | |
| 151 | 3-(4-Chloro-2-fluoro-5-{[3-(*p*-fluorobenzyl)-4-oxo-2-thiazolidinylidene]amino}phenyl}-1-methyl-6-(trifluoromethyl)-2,4(1H,3H)-pyrimidinedione |
| | |
| 152 | 3-{4-Chloro-5-[(3-cyclopropyl-4-oxo-2-thiazolidinylidene)amino]-2-fluorophenyl}-1-methyl-6-(trifluoromethyl)-2,4(1H,3H)-pyrimidinedione |
| | |
| 153 | Ethyl 5-bromo-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate |
| | |
| 154 | Ethyl 2-{{2-chloro-5-[3,6-dihydro-3-methyl-2,6-dioxo-4-(trifluoromethyl)-1(2H)-pyrimidinyl]-4-fluorophenyl}imino}-5-methylene-4-oxo-3-thiazolidineacetate |
| | |
| 155 | 3-{4-Chloro-2-fluoro-5-{[3-(2-methoxyethyl)-4-oxo-2-thiazolidinylidene]amino}phenyl}-1-methyl-6-(trifluoromethyl)-2,4(1H,3H)-pyrimidinedione |
| | |
| 156 | Ethyl 2-{{2-chloro-5-[3,6-dihydro-3-methyl-2,6-dioxo-4-(trifluoromethyl)-1(2H)-pyrimidinyl]-4-fluorophenyl}imino}-5-(methoxymethyl)-4-oxo-3-thiazolidineacetate |
| | |
| 157 | 3-{4-Chloro-2-fluoro-5-{{3-[2-(methylthio)ethyl]-4-oxo-2-thiazolidinylidene}amino}phenyl}-1-methyl-6-(trifluoromethyl)-2,4(1H,3H)-pyrimidinedione |
| | |
| 158 | Methyl {{2-{2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidinyl}ethyl}-thio}acetate |
| | |
| 159 | 3-{4-Chloro-5-[(3-ethyl-4-oxo-2-thiazolidinylidene)amino]-2-fluorophenyl}-1-methyl-6-(trifluoromethyl)-2,4(1H,3H)-pyrimidinedione |
| | |
| 160 | Ethyl 2-{{2-chloro-5-[3,6-dihydro-3-methyl-2,6-dioxo-4-(trifluoromethyl)-1(2H)-pyrimidinyl]-4-fluorophenyl}imino}-5-methoxy-4-oxo-3-thiazolidineacetate |
| | |
| 161 | 3-Allyl-2-{{2-chloro-5-[3,6-dihydro-3-methyl-2,6-dioxo-4-(trifluoromethyl)-1(2H)-pyrimidinyl]-4-fluorophenyl}imino}-4-oxo-5-thiazolidineacetohydroxamic acid |
| | |
| 162 | 2-{{2-Chloro-5-[3,6-dihydro-3-methyl-2,6-dioxo-4-(trifluoromethyl)-1(2H)-pyrimidinyl]-4-fluorophenyl}imino}-3-methyl-4-oxo-5-thiazolidineacetohydroxamic acid |
| | |
| 163 | 3-{4-Chloro-2-fluoro-5-{[4-oxo-3-(2,2,2-trifluoroethyl)-2-thiazolidinylidene]amino}phenyl}-1-methyl-6-(trifluoromethyl)-2,4(1H,3H)-pyrimidinedione |
| | |
| 164 | 3-{4-Chloro-2-fluoro-5-[(3-isopropyl-4-oxo-2-thiazolidinylidene)amino]phenyl}-1-methyl-6-(trifluoromethyl)-2,4(1H,3H)-pyrimidinedione |
| | |
| 165 | 3-{4-Chloro-2-fluoro-5-{[3-(1-methyl-2-propynyl)-4-oxo-2-thiazolidinylidene]amino}phenyl}-1-methyl-6-(trifluoromethyl)-2,4(1H,3H)-pyrimidinedione |
| | |
| 166 | 3-{4-Chloro-5-[(3-cyclopentyl-4-oxo-2-thiazolidinylidene)amino]-2-fluorophenyl}-1-methyl-6-(trifluoromethyl)-2,4(1H,3H)-pyrimidinedione |
| | |
| 167 | 5-Carboxy-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetic acid, 5-*tert*-butyl ethyl ester |
| | |
| 168 | 3-{4-Chloro-5-[(3-cyclobutyl-4-oxo-2-thiazolidinylidene)amino]-2-fluorophenyl}-1-methyl-6-(trifluoromethyl)-2,4(1H,3H)-pyrimidinedione |
| | |
| 169 | Ethyl 2-{{2-chloro-5-[2,6-dioxo-4-(trifluoromethyl)piperidino]-4-fluorophenyl}imino}-4-oxo-3-thiazolidineacetate |
| | |
| 170 | 2-{{2-Chloro-5-[2,6-dioxo-4-(trifluoromethyl)piperidino]-4-fluorophenyl}imino}-4-oxo-3,5-thiazolidinediacetic acid, 3-ethyl ester |
| | |
| 171 | 3-{4-Chloro-2-fluoro-5-[(4-oxo-3-propyl-2-thiazolidinylidene)amino]phenyl}-1-methyl-6-(trifluoromethyl)-2,4(1H,3H)-pyrimidinedione |
| | |
| 172 | 3-{4-Chloro-2-fluoro-5-{[3-(3-methoxypropyl)-4-oxo-2-thiazolidinylidene]amino}phenyl}-1-methyl-6-(trifluoromethyl)-2,4(1H,3H)-pyrimidinedione |
| | |
| 173 | 3-{4-Chloro-2-fluoro-5-{[3-(2-fluoroethyl)-4-oxo-2-thiazolidinylidene]amino}phenyl}-1-methyl-6-(trifluoromethyl)-2,4(1H,3H)-pyrimidinedione |

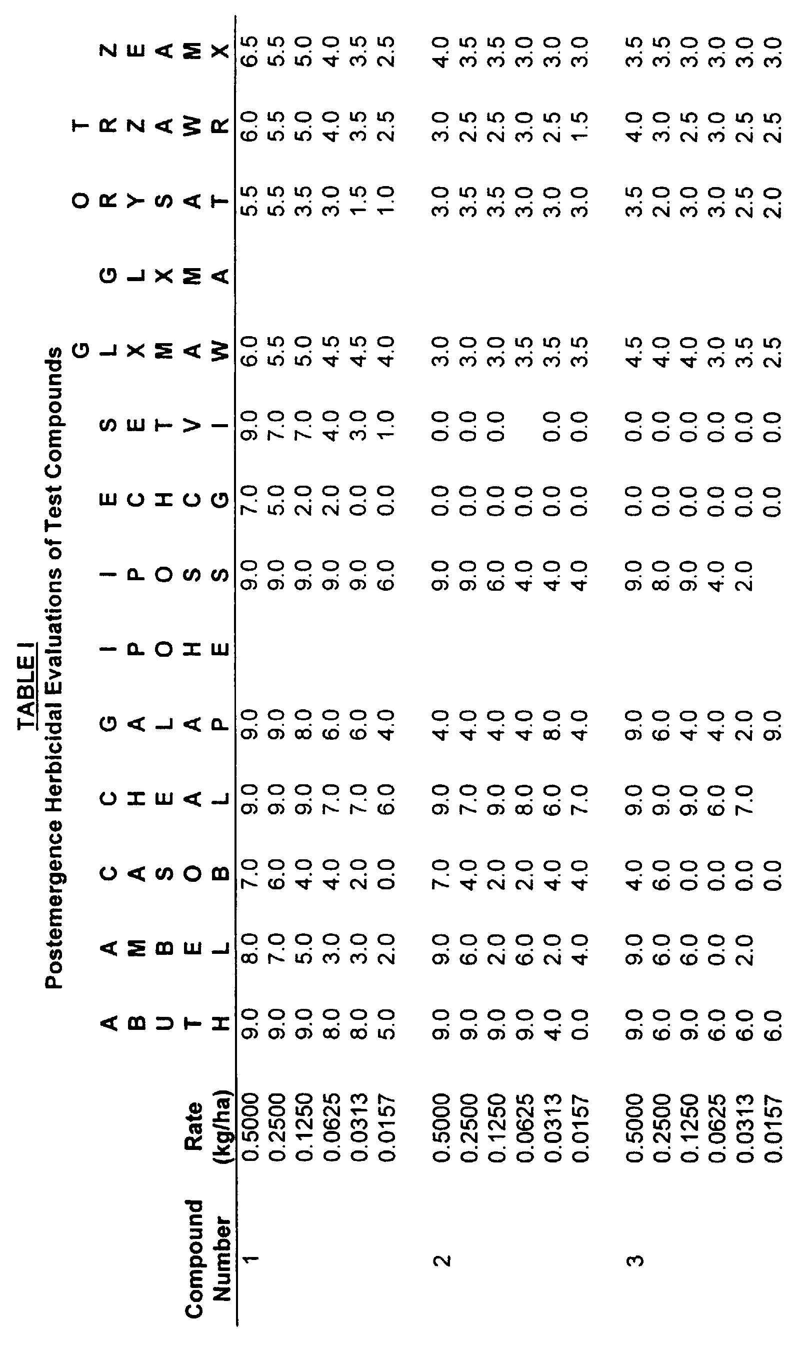

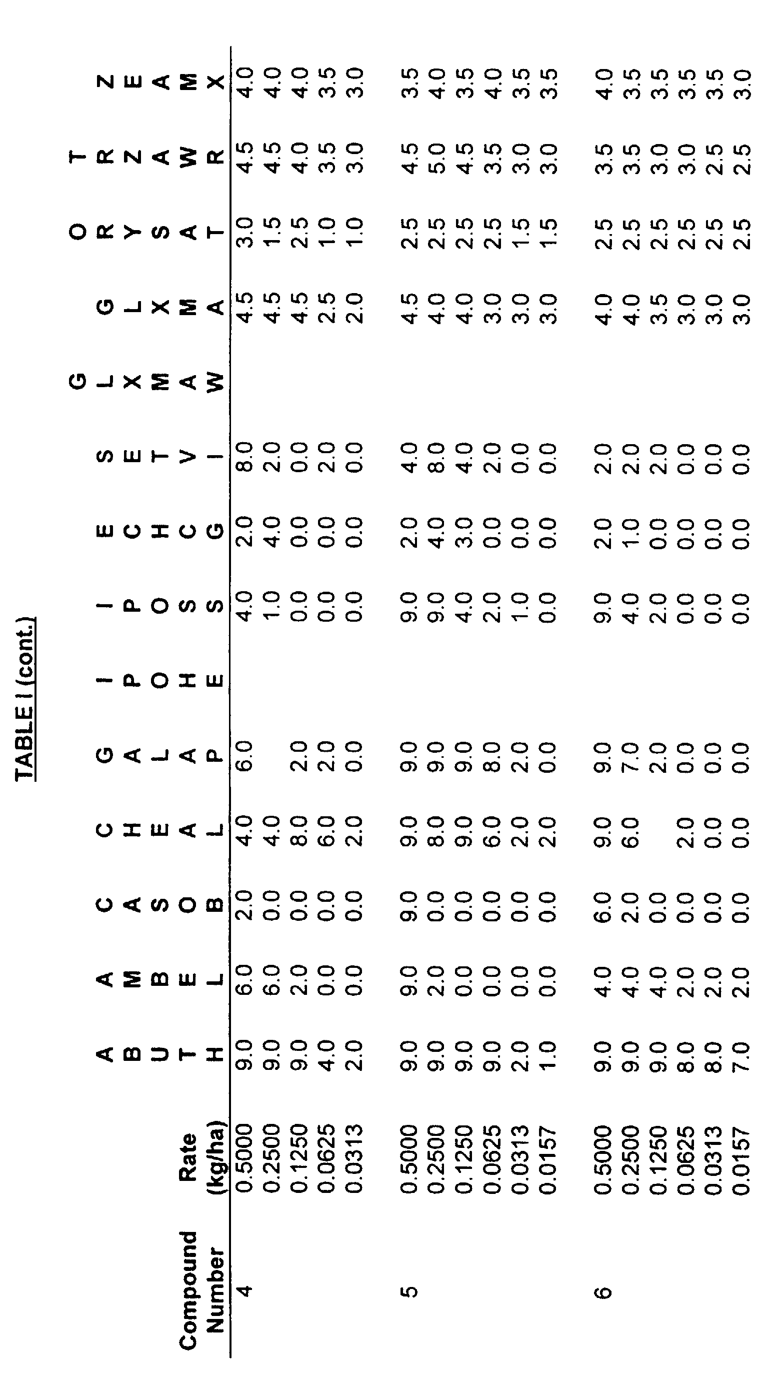

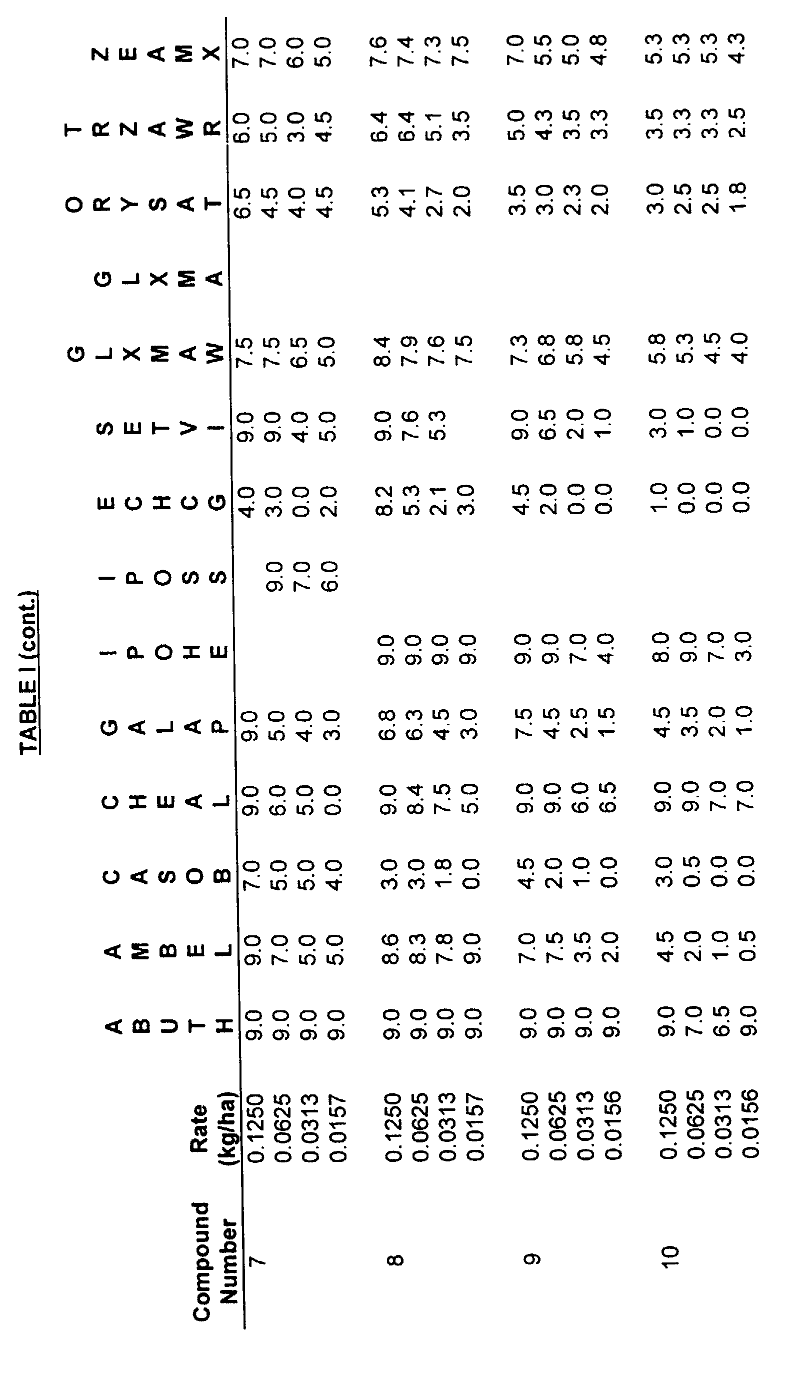

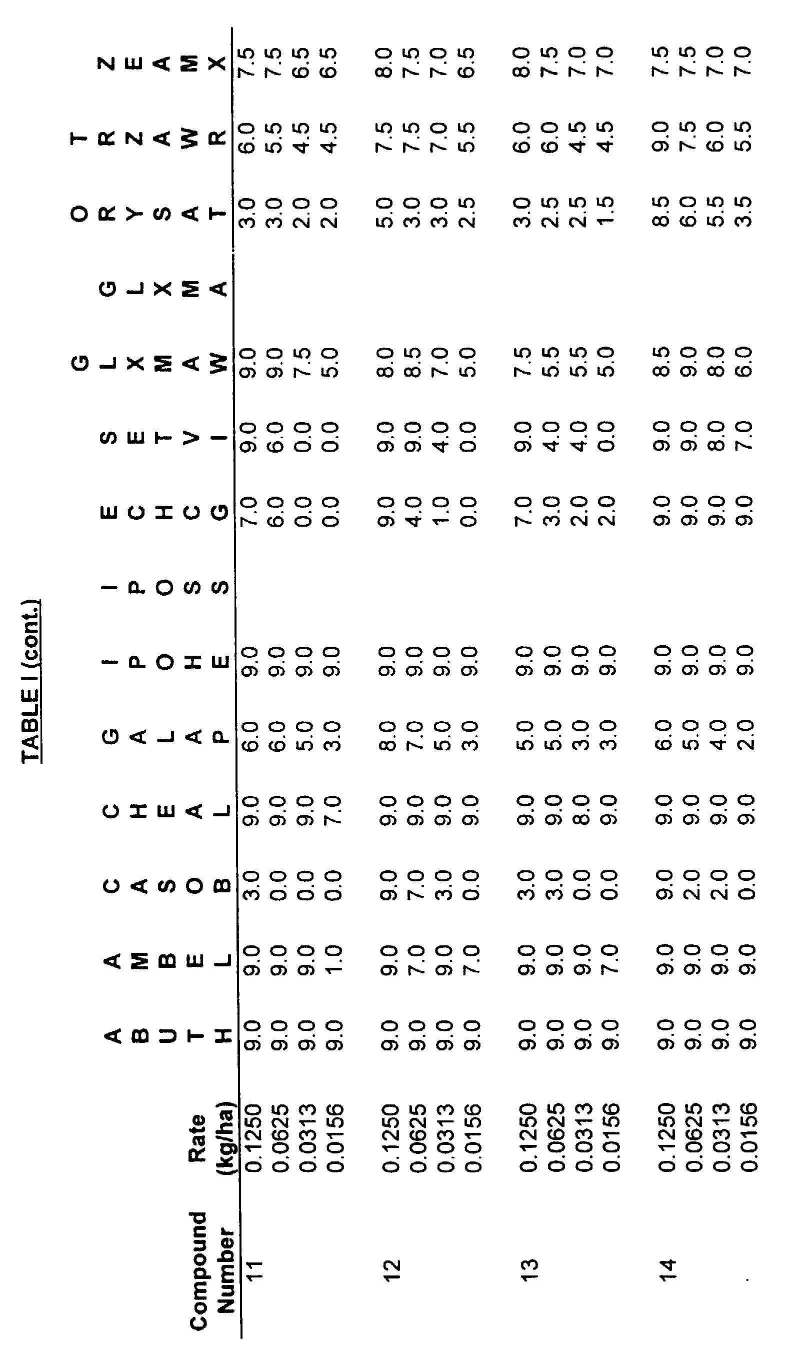

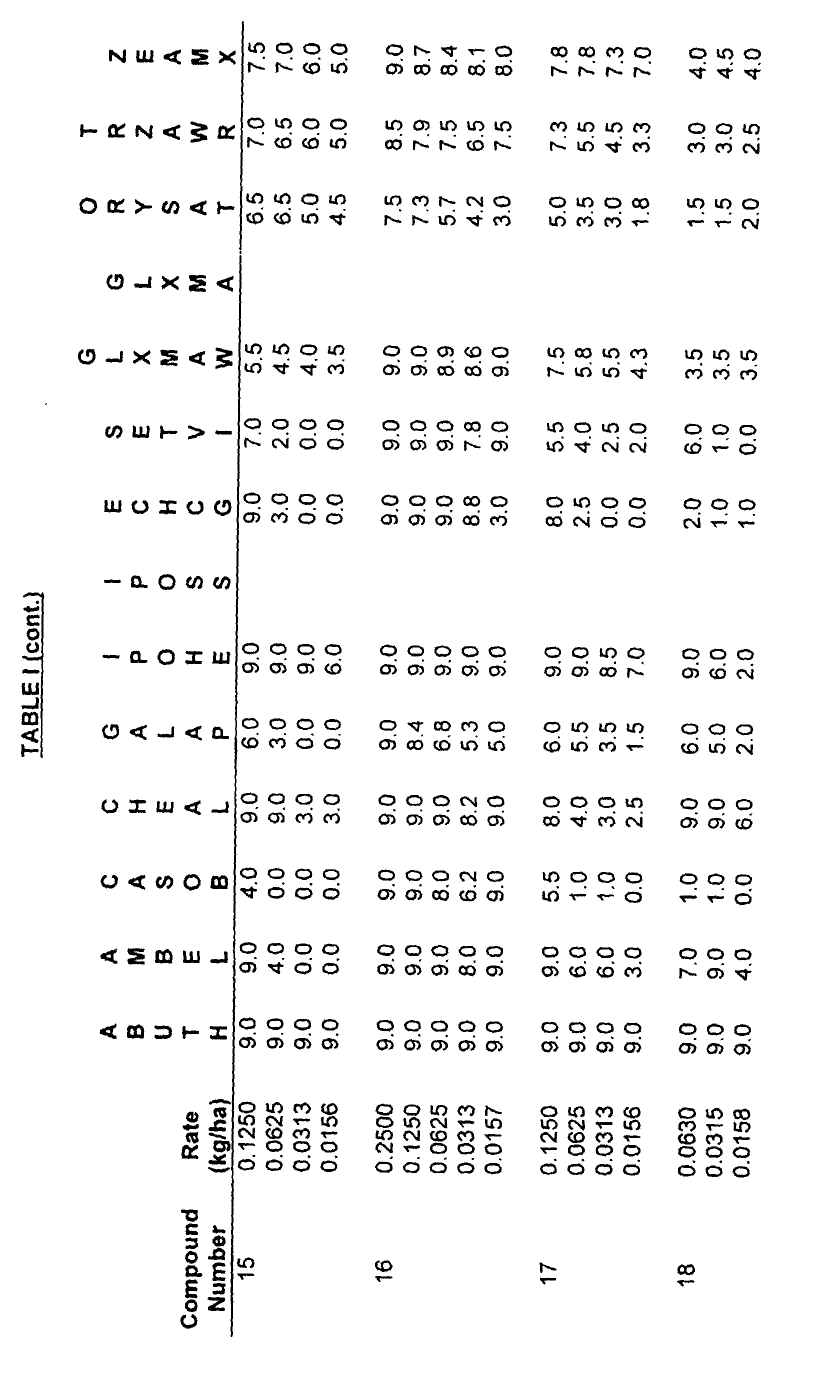

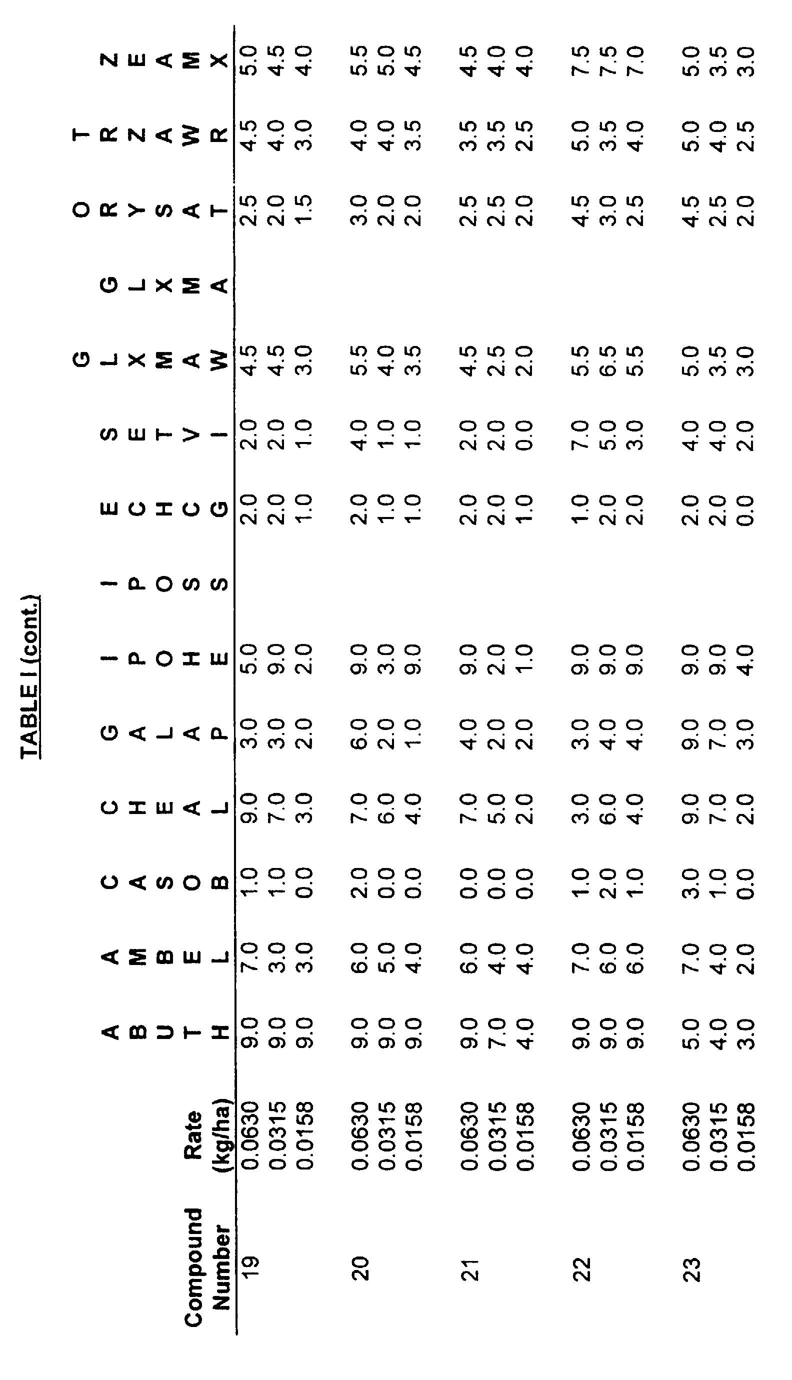

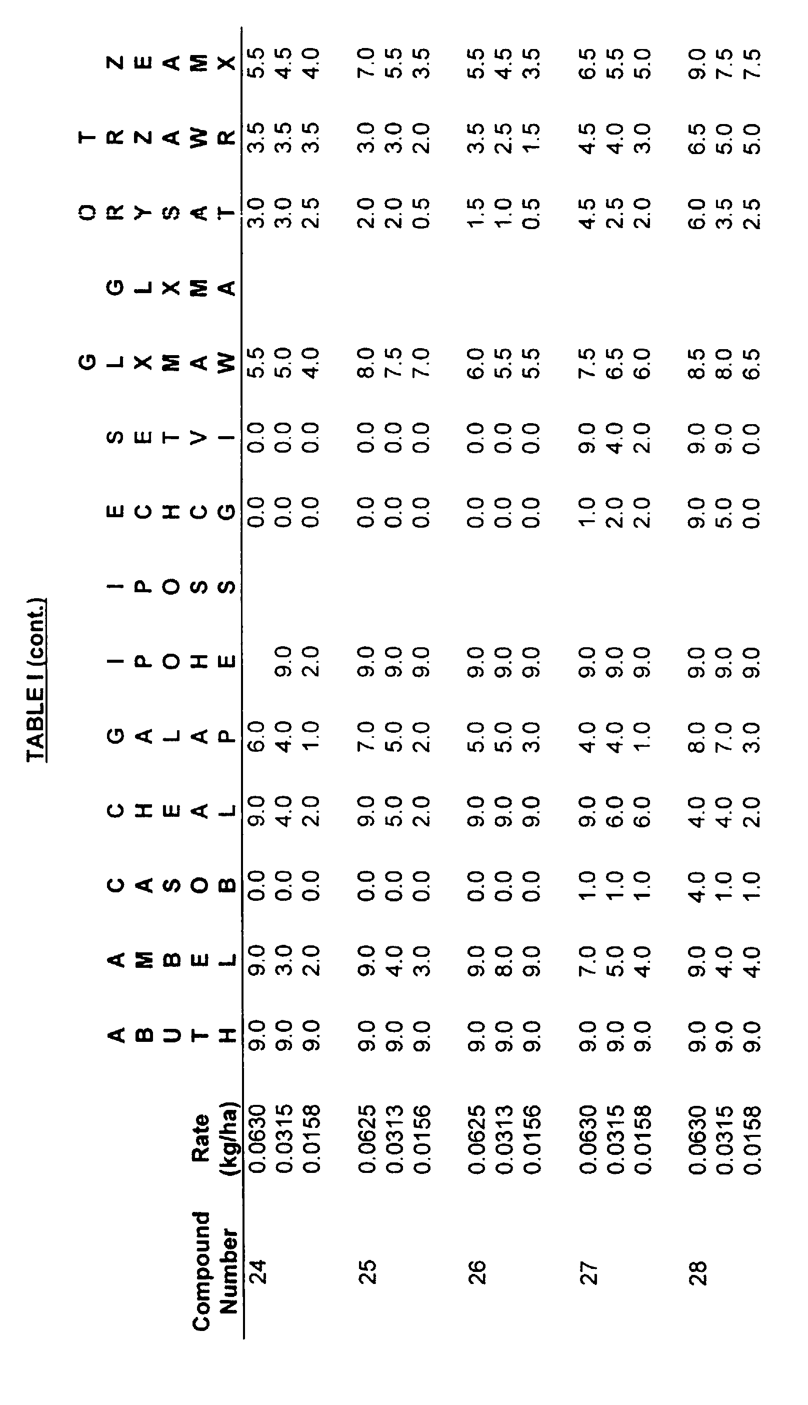

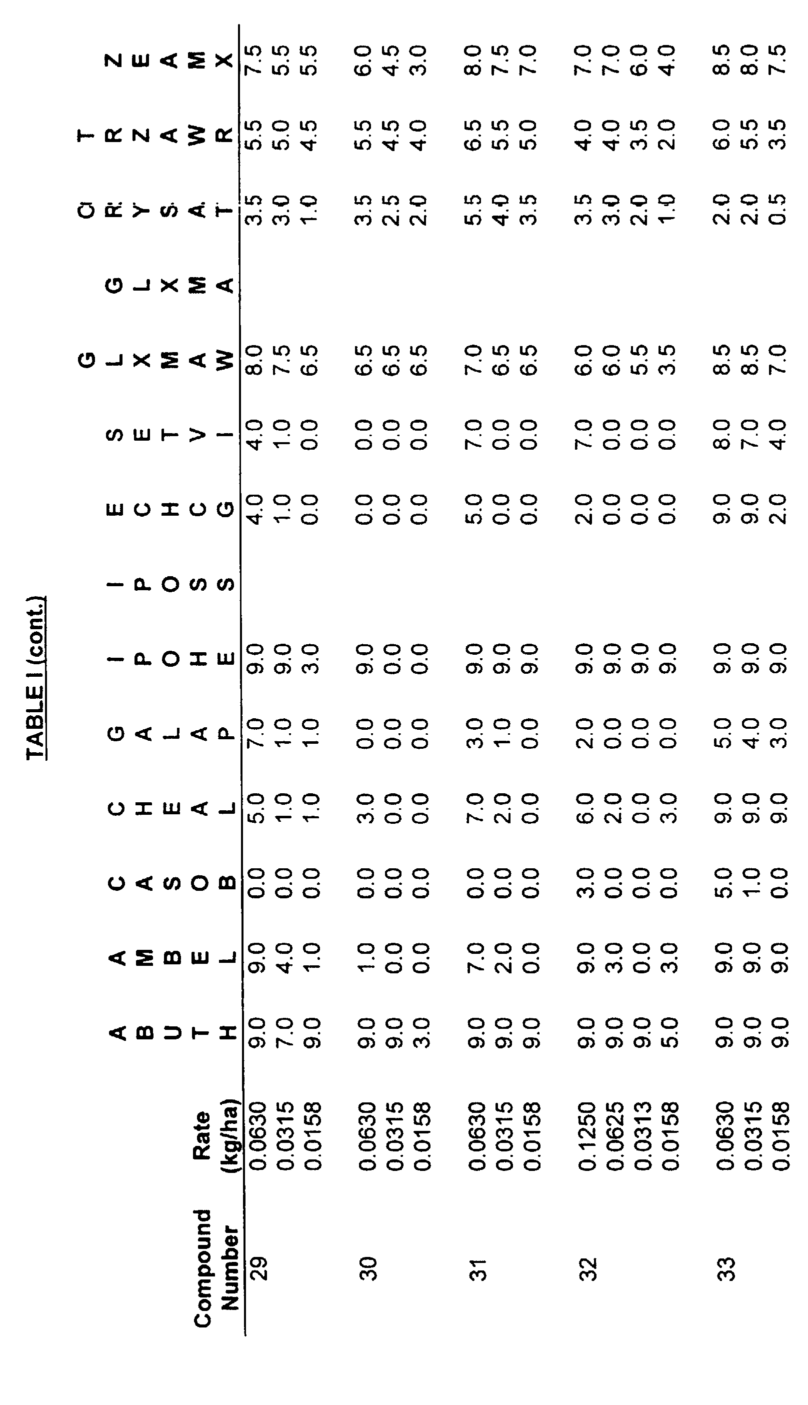

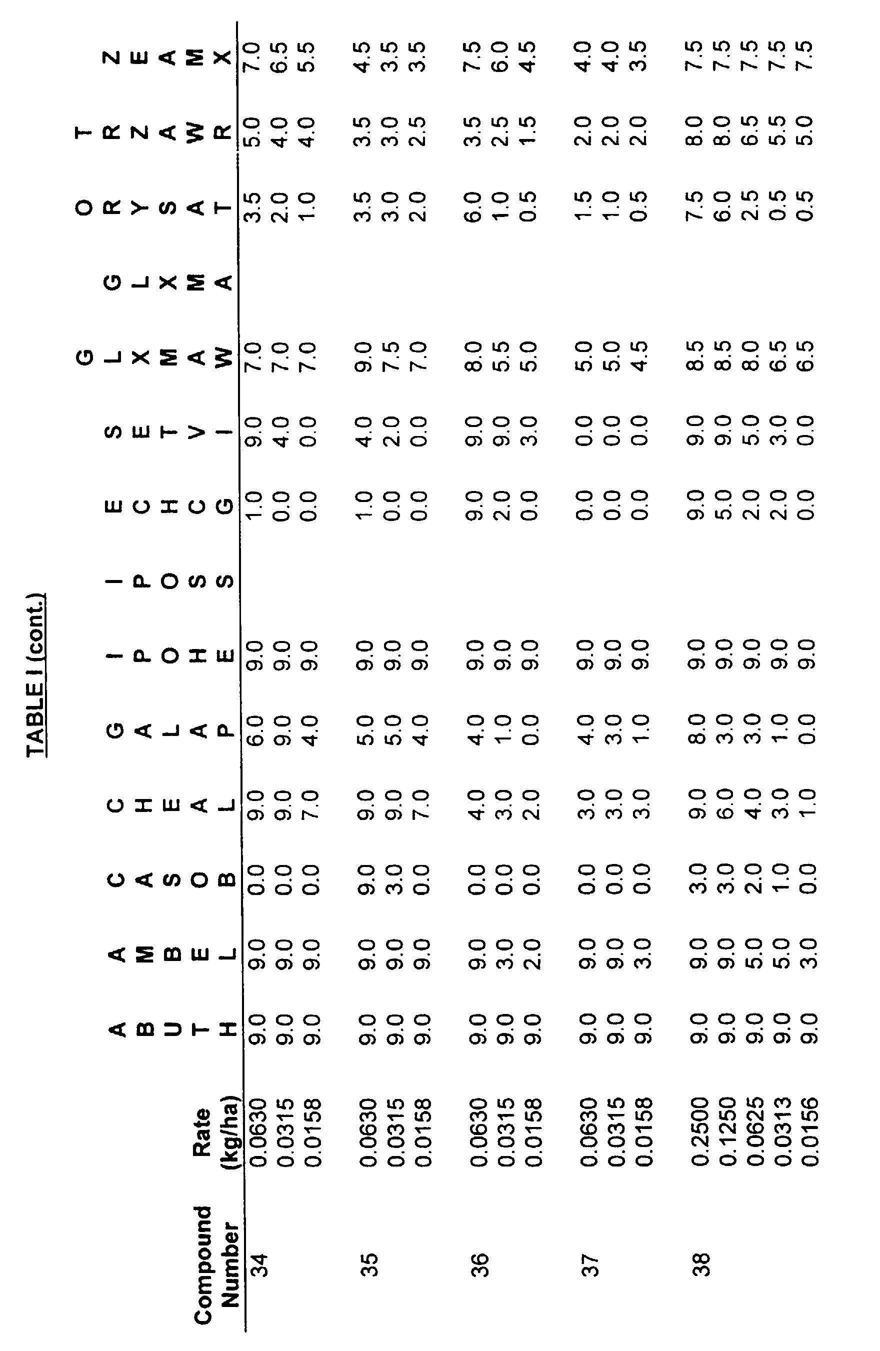

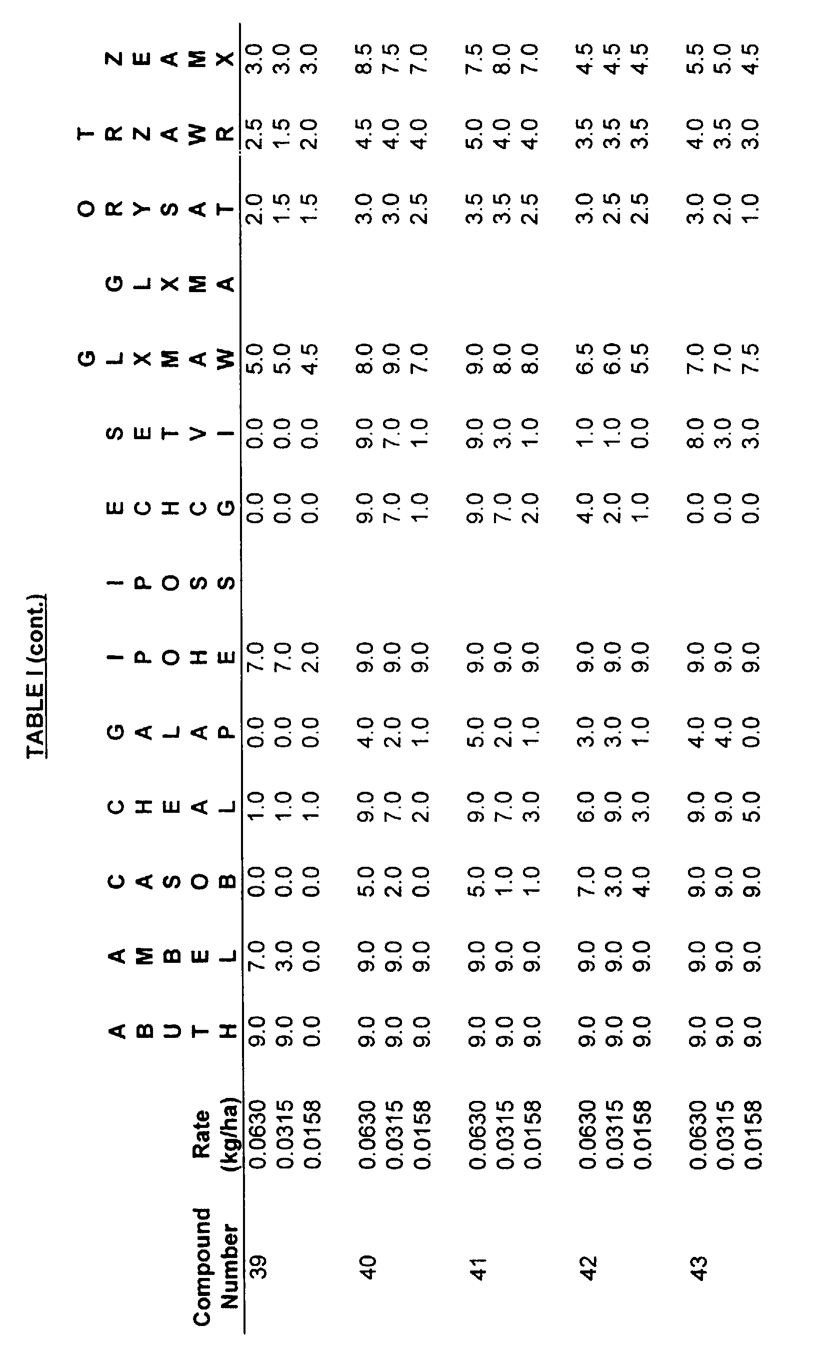

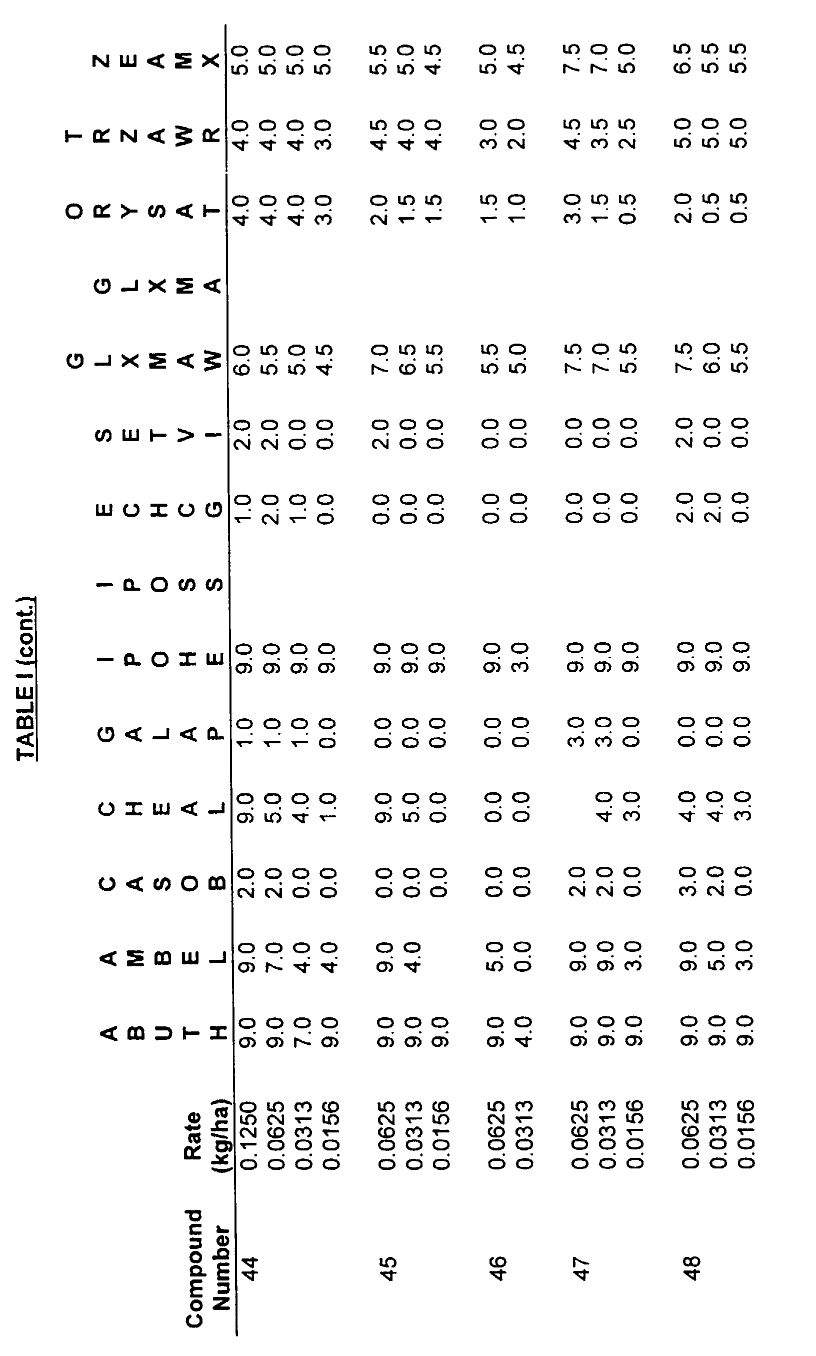

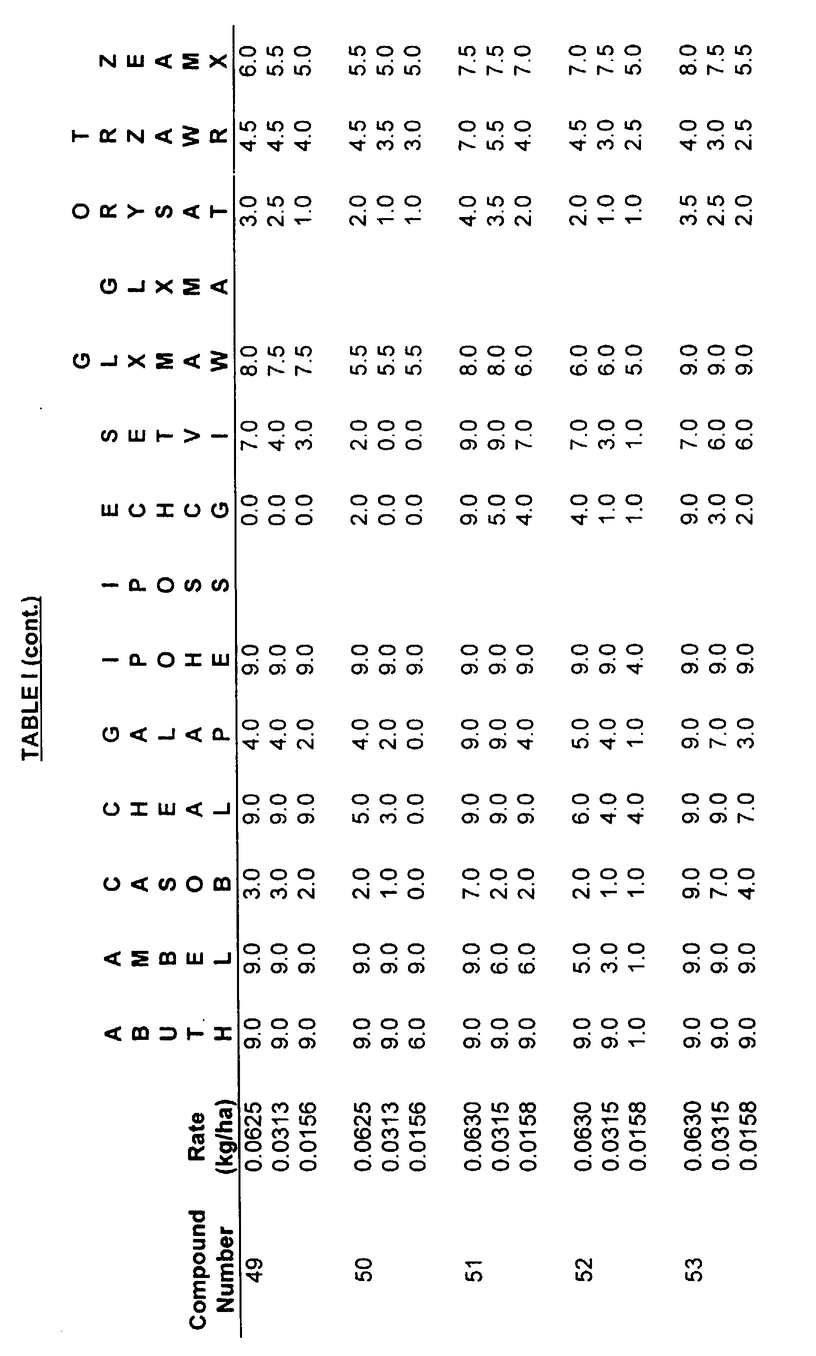

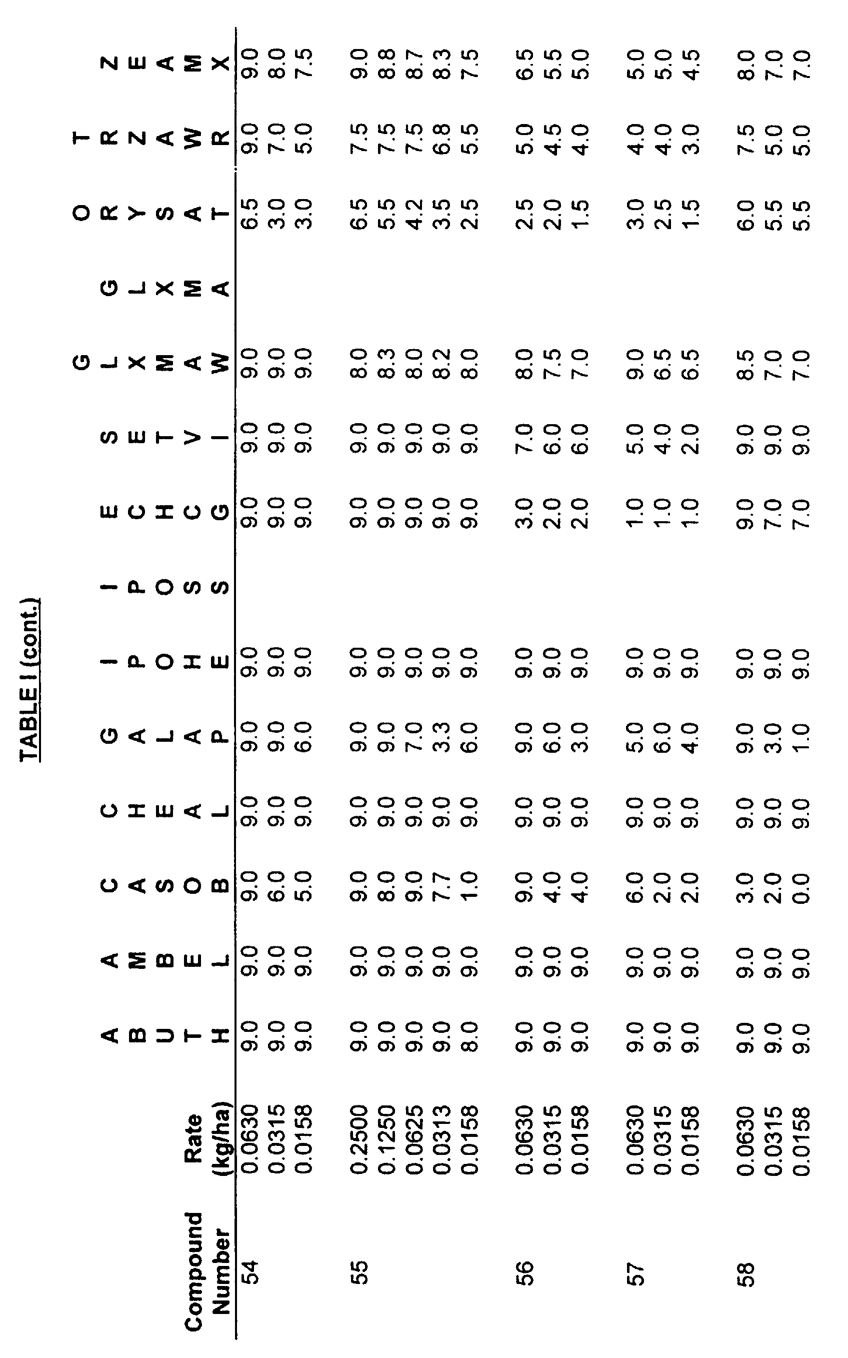

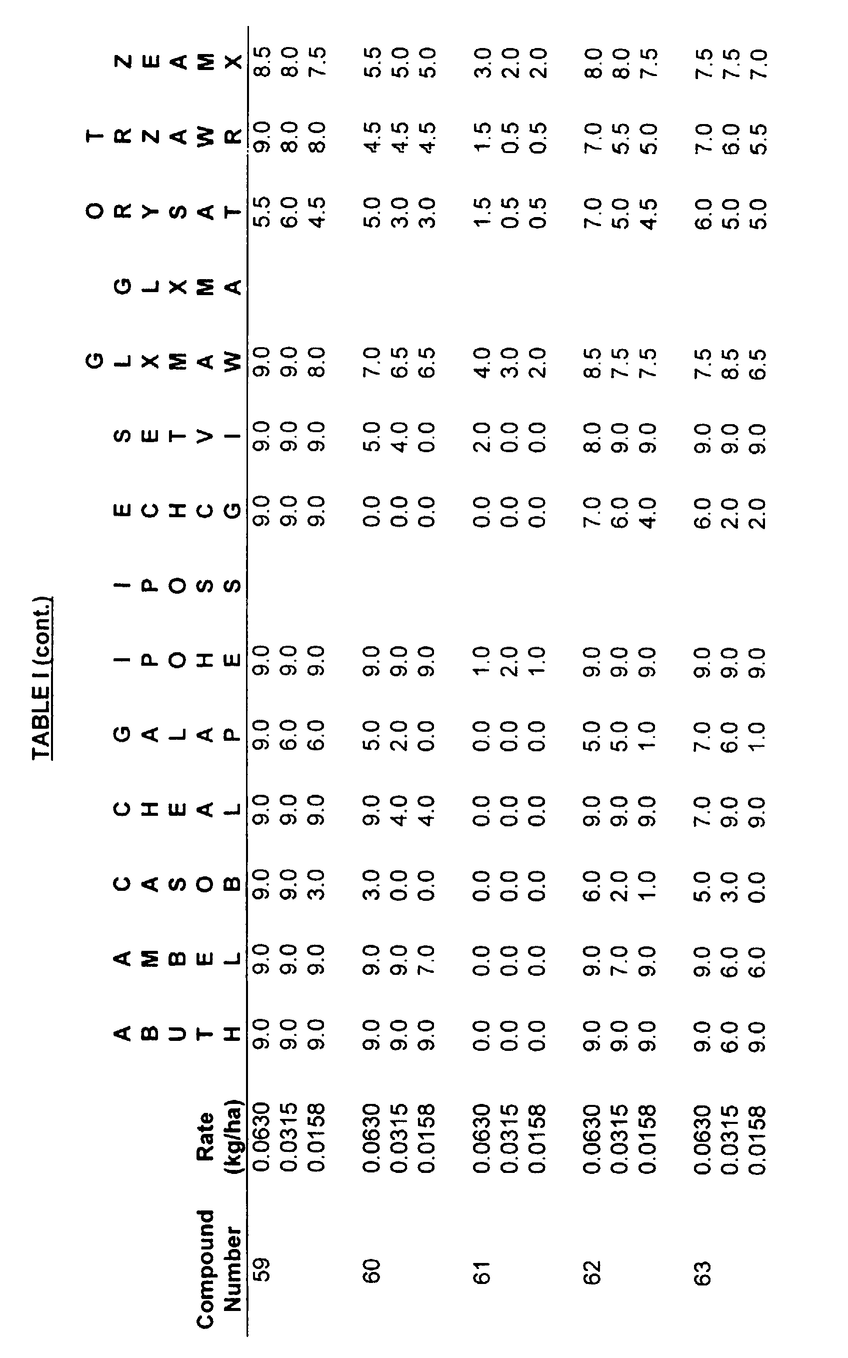

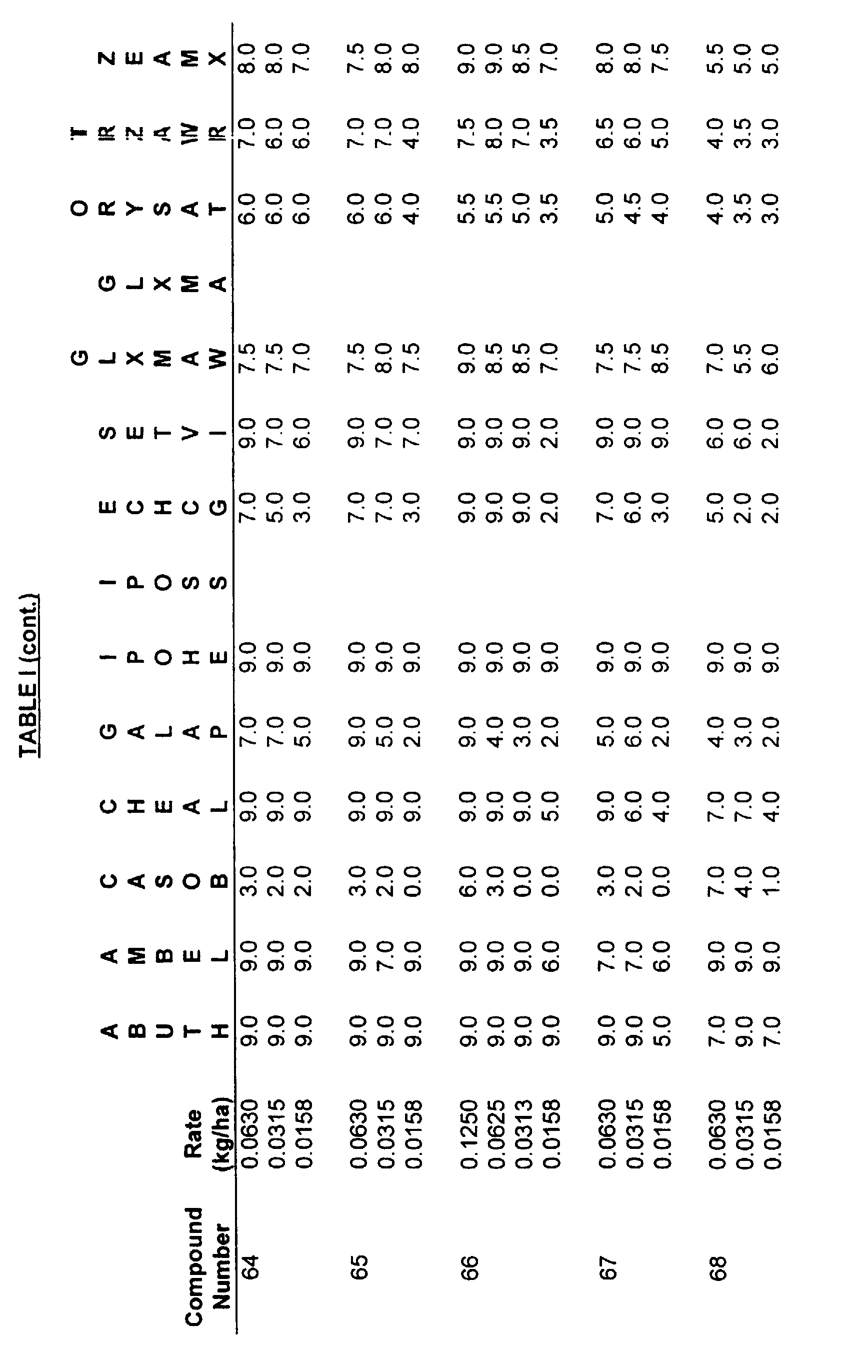

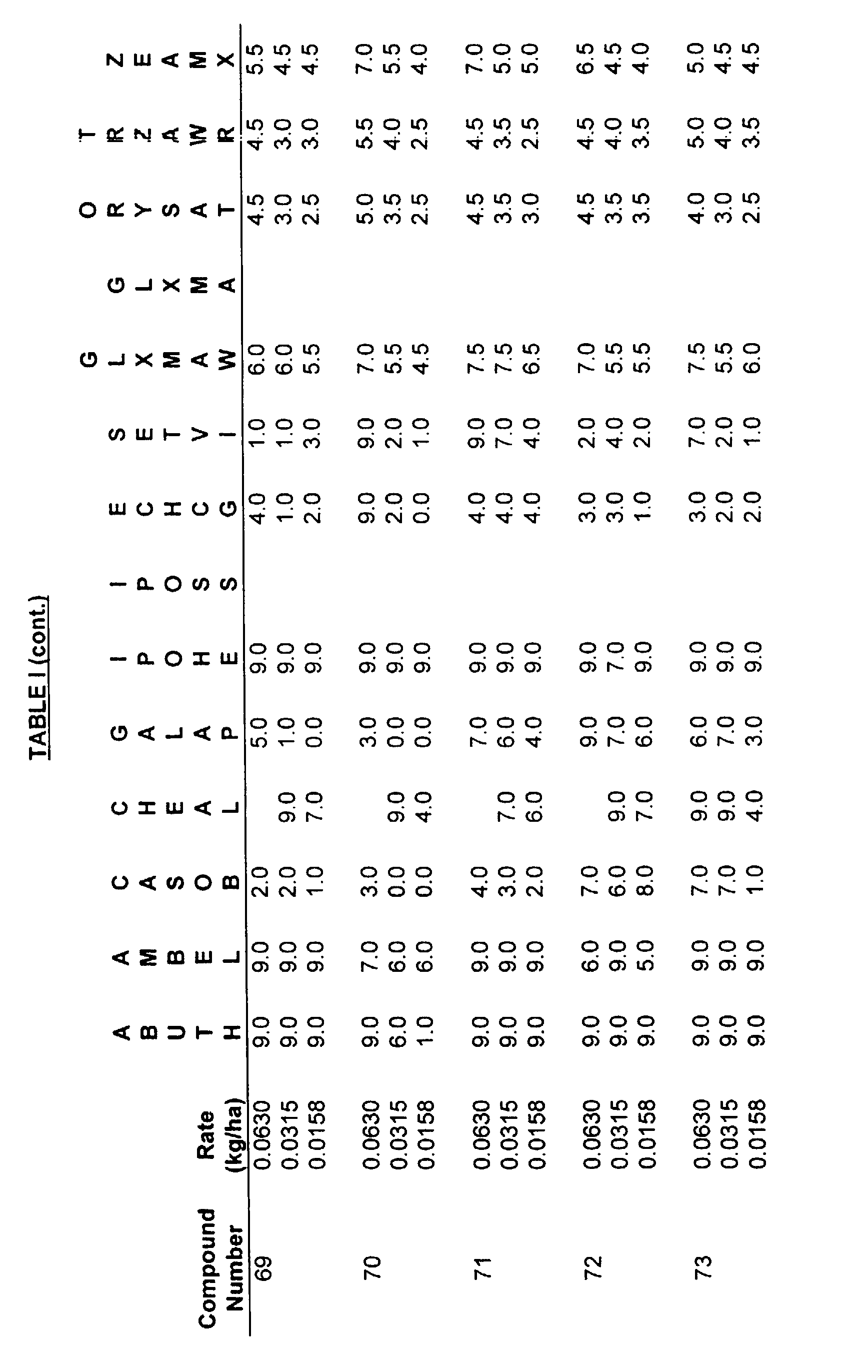

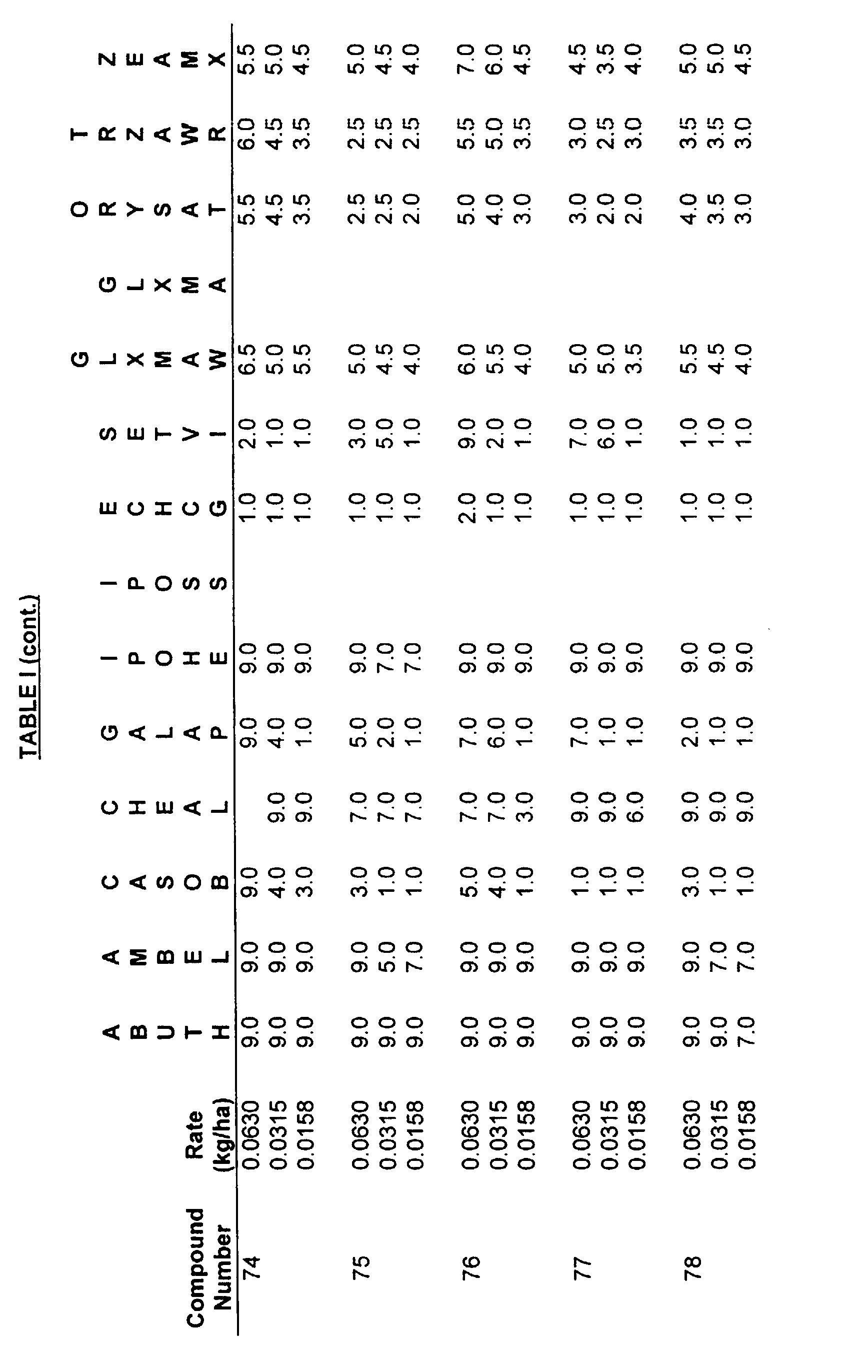

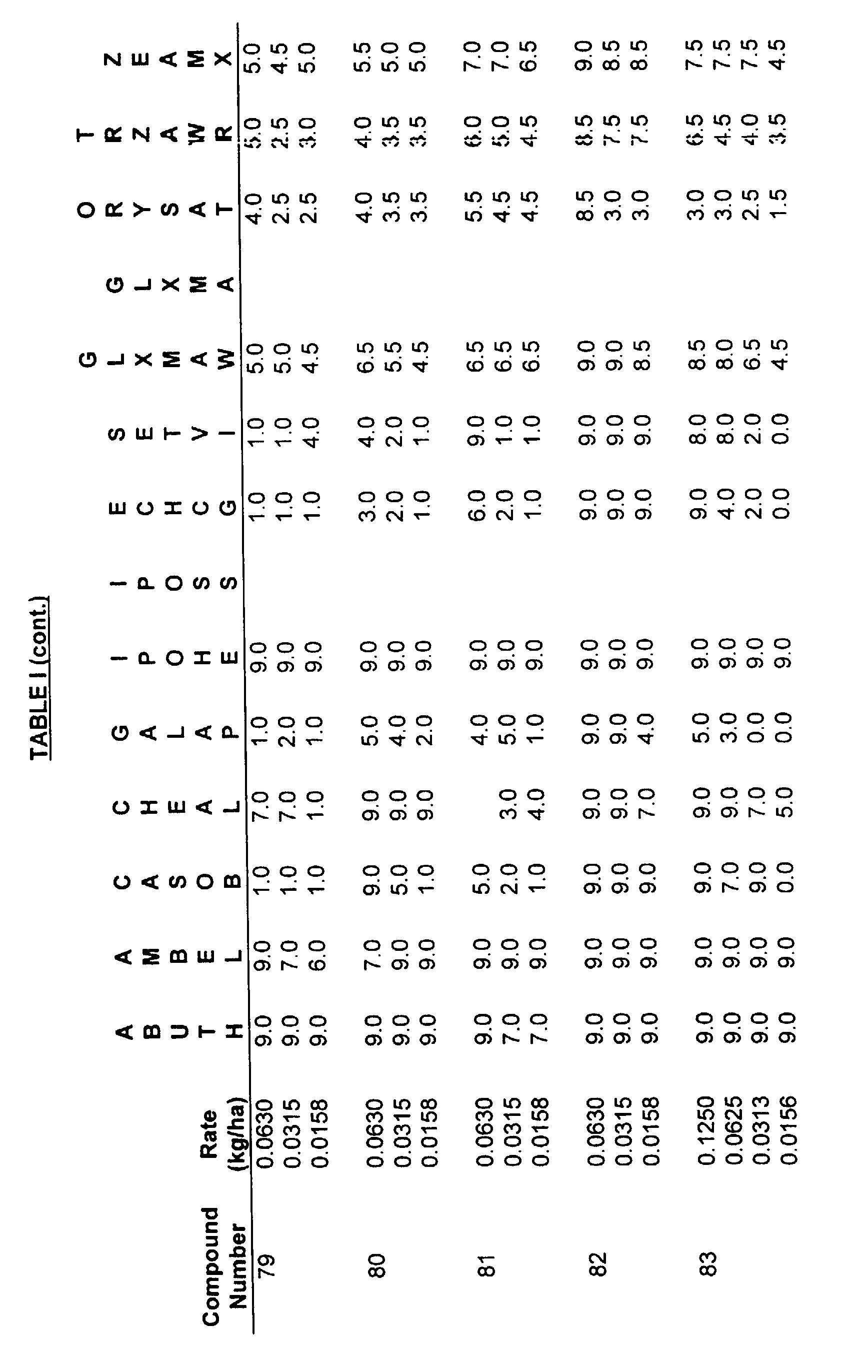

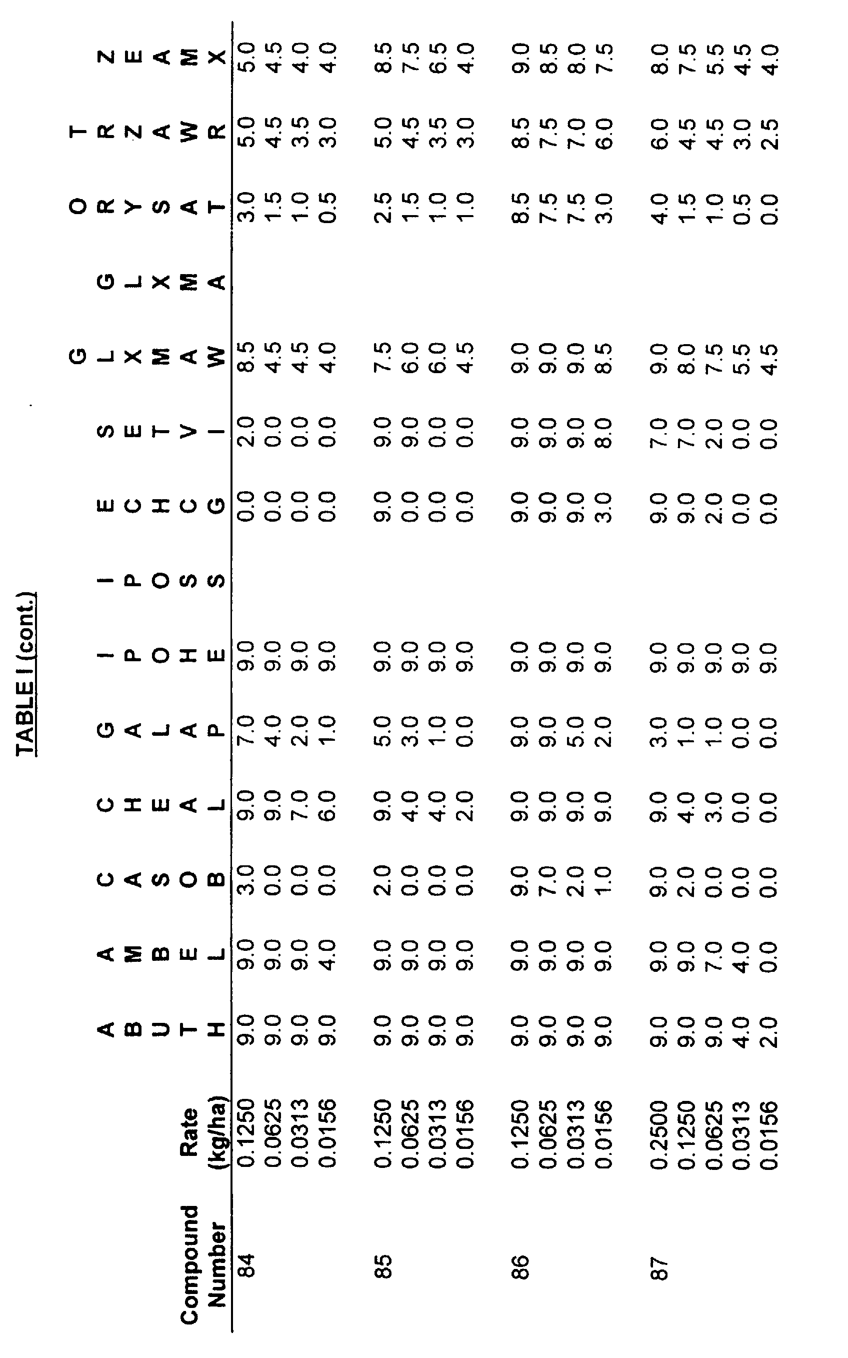

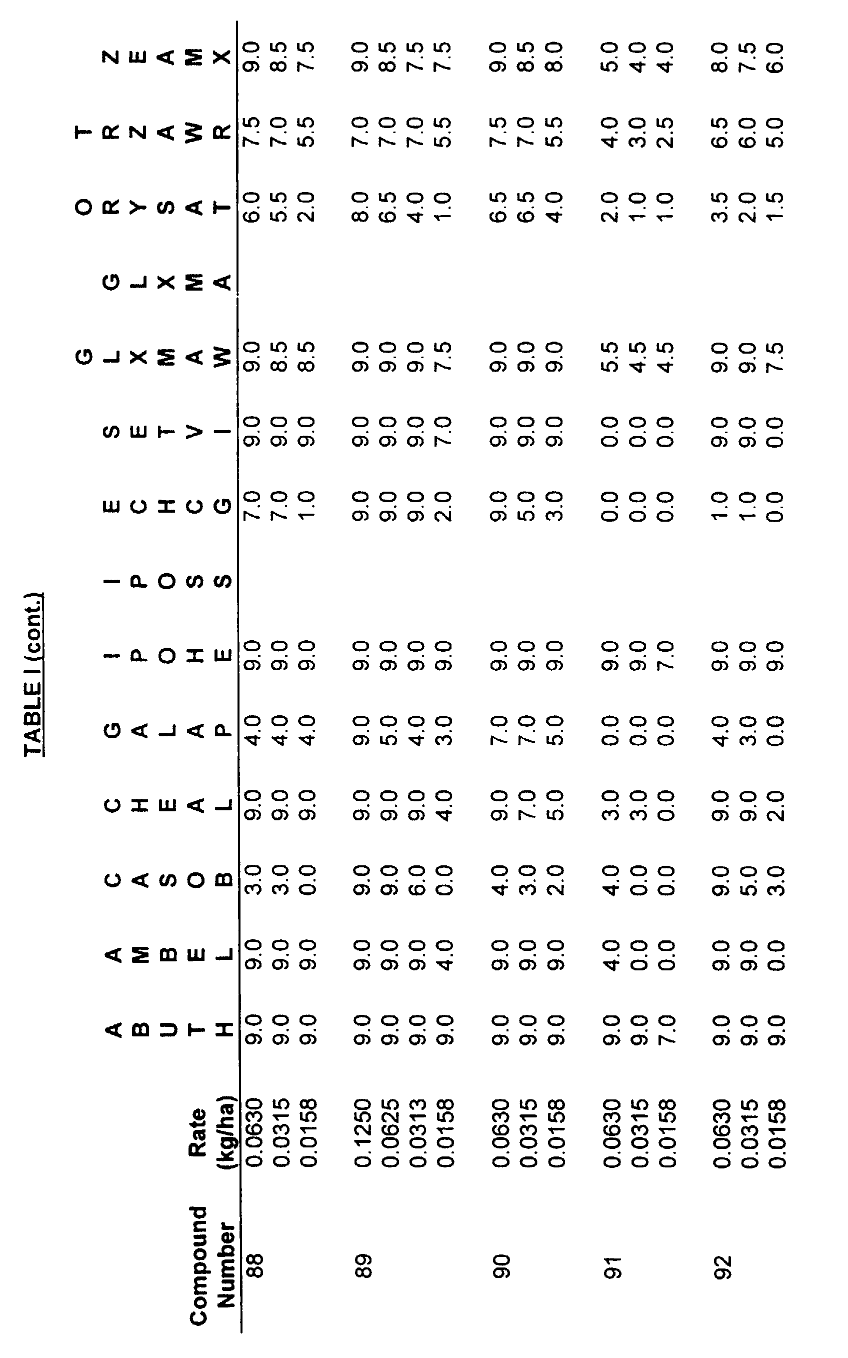

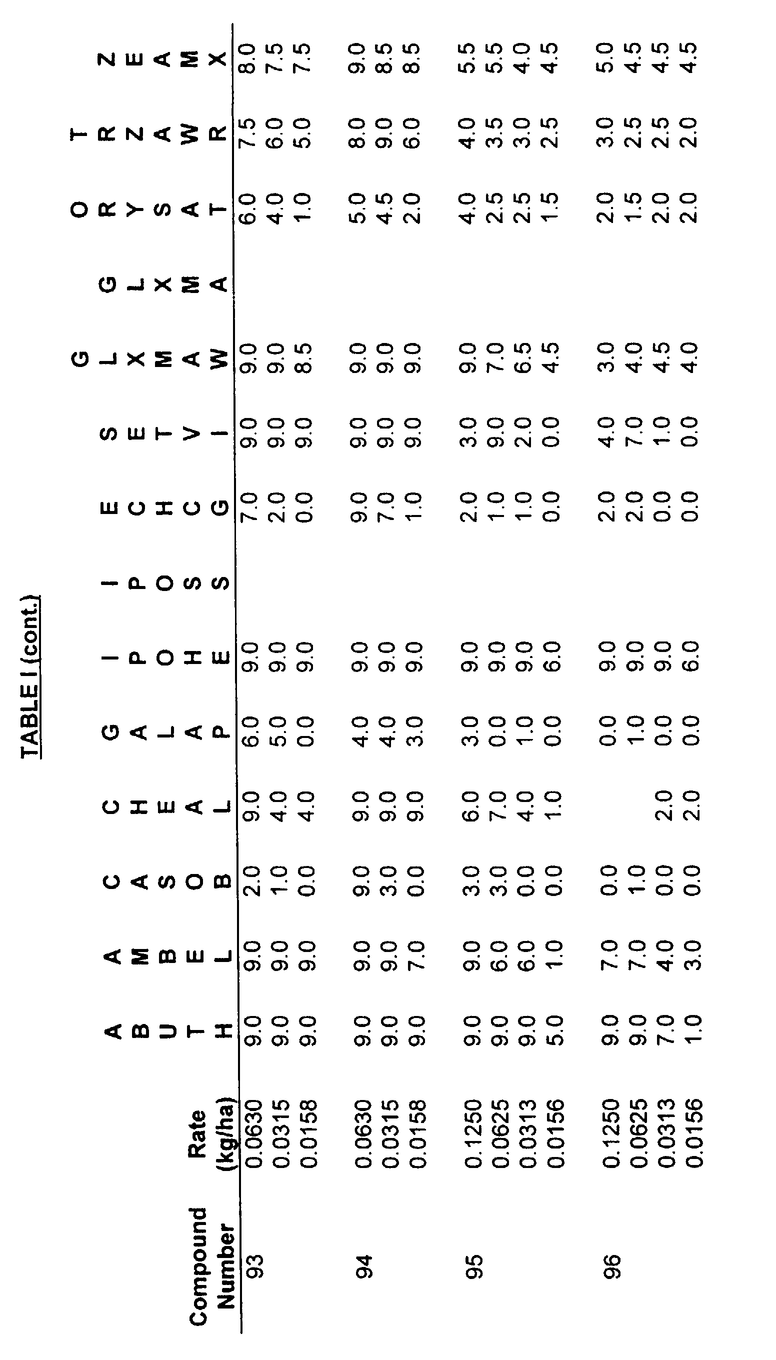

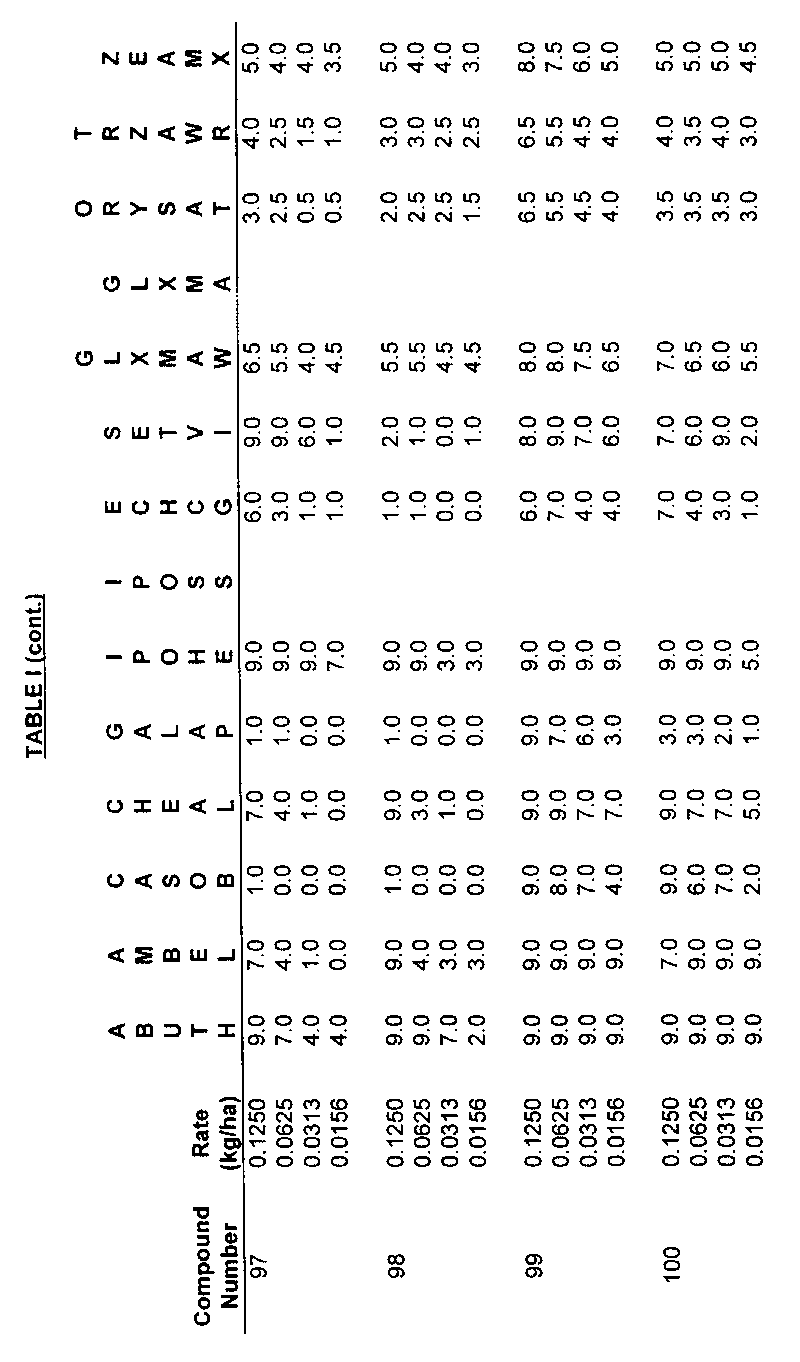

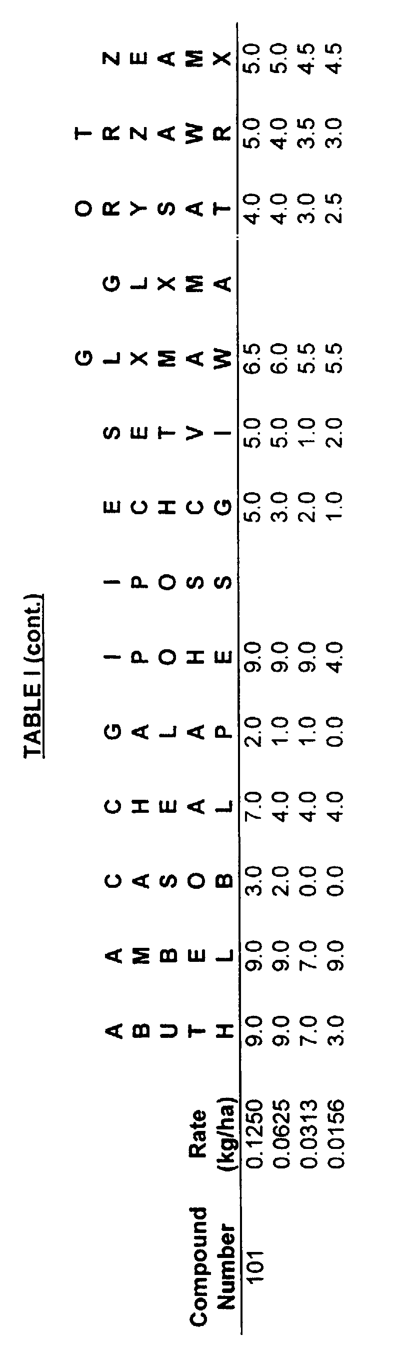

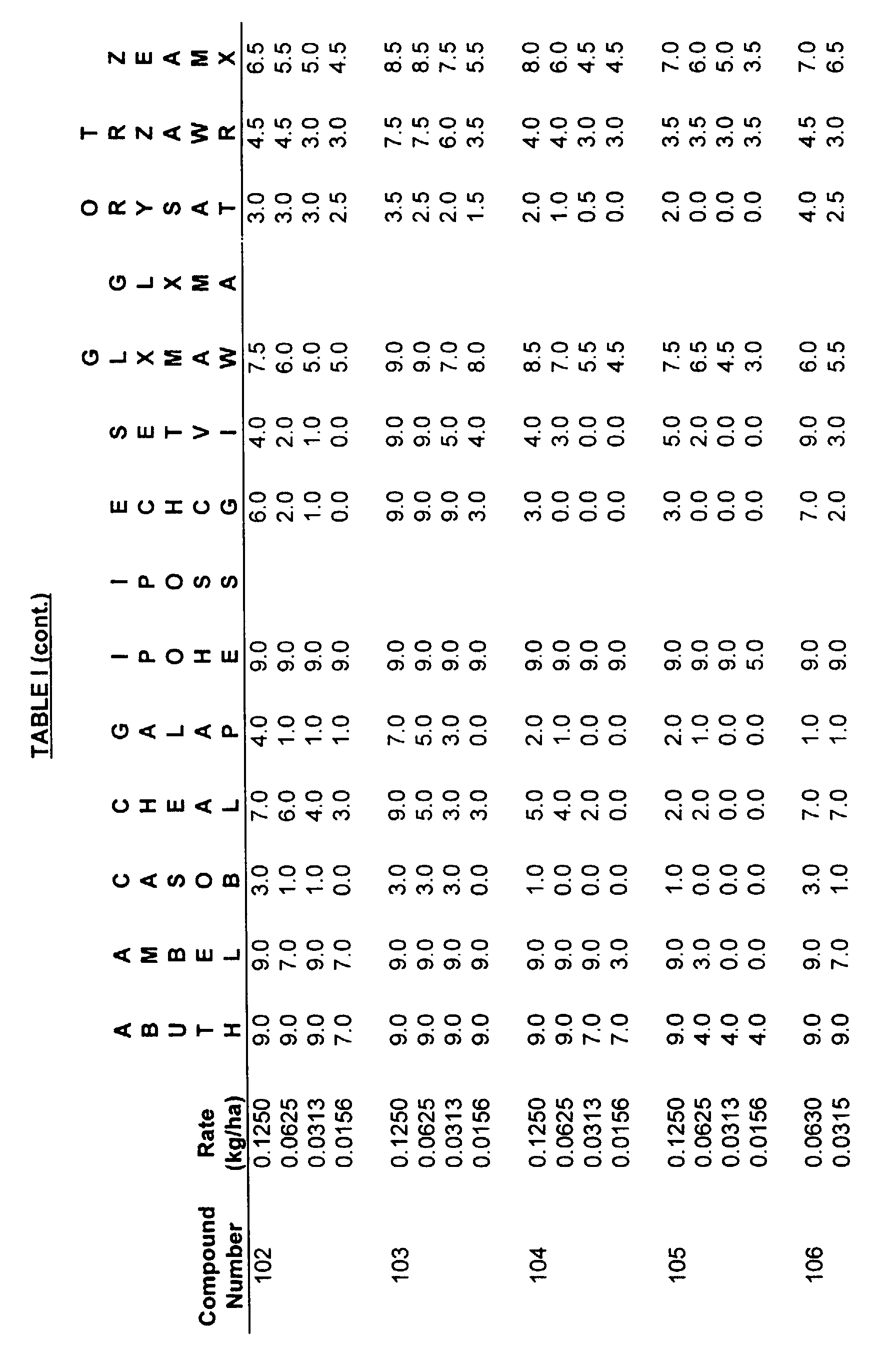

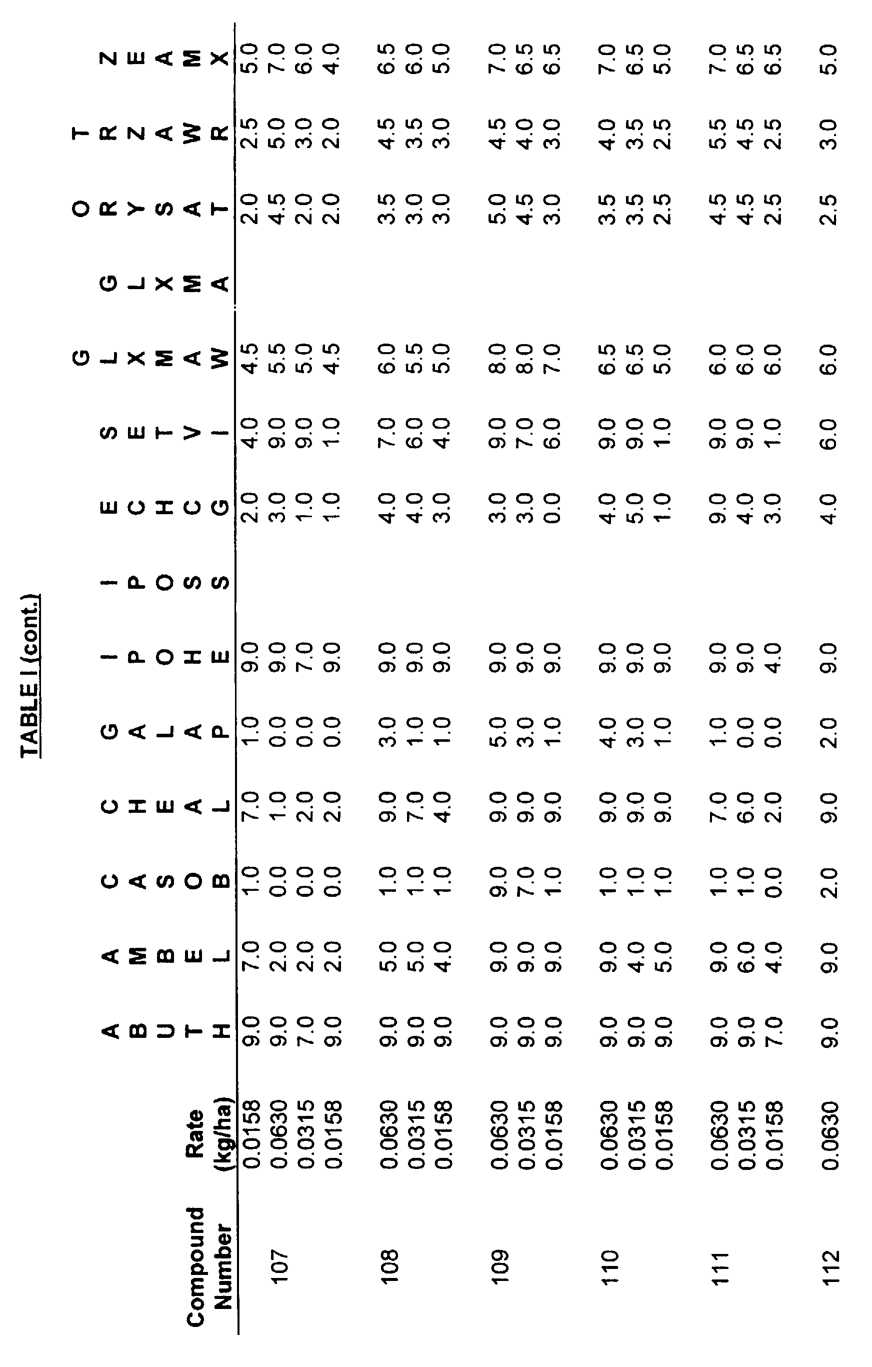

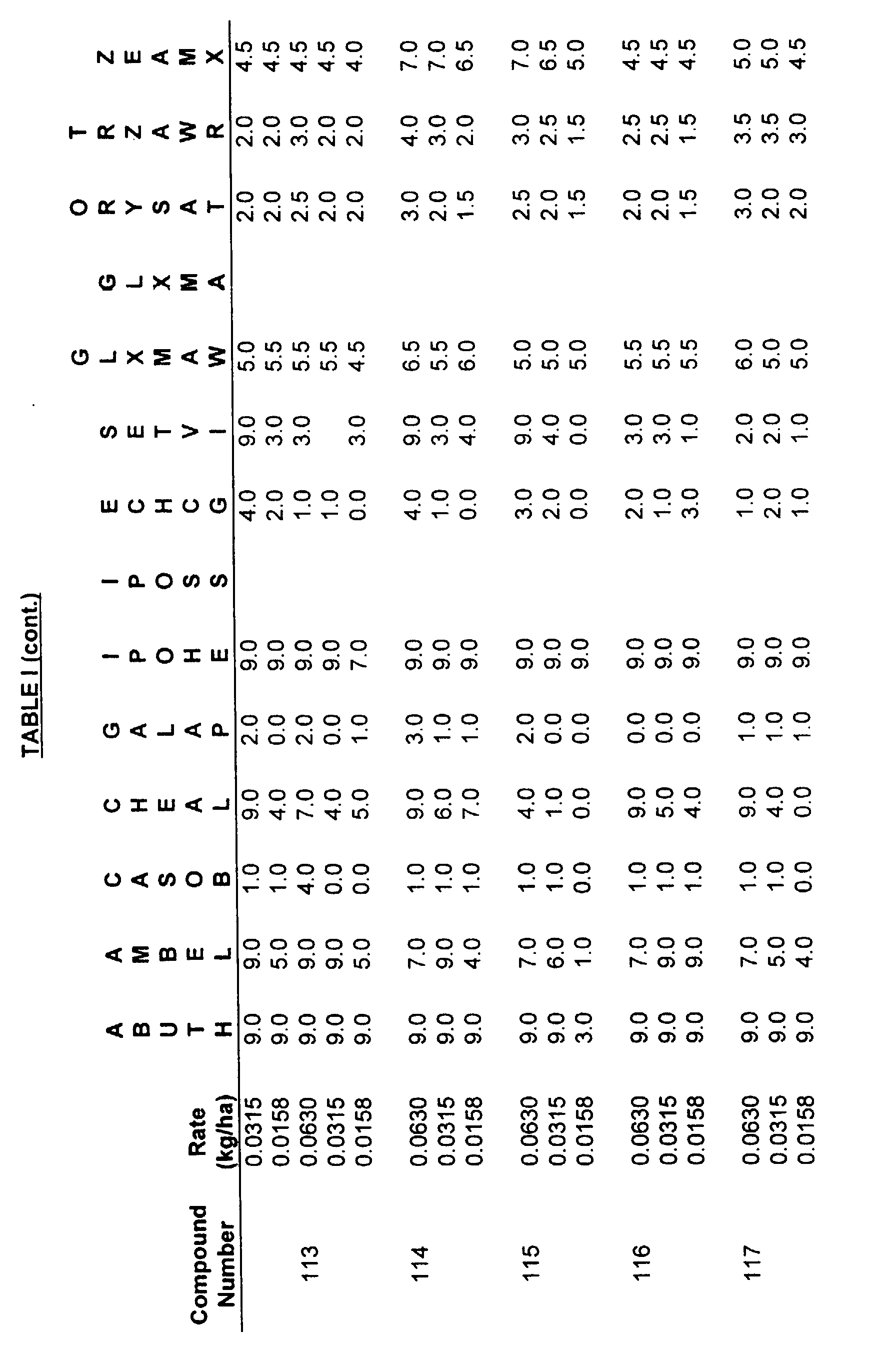

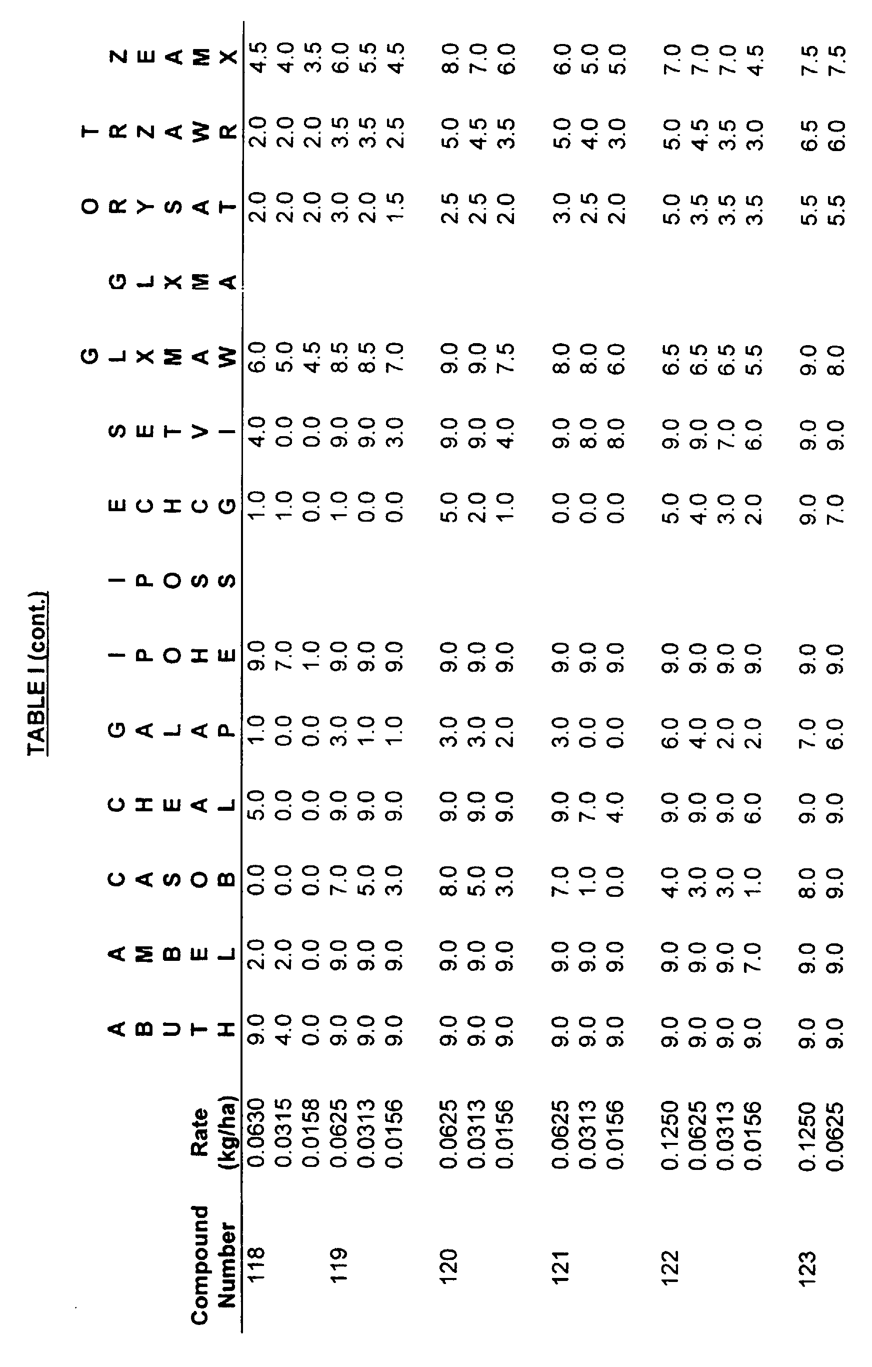

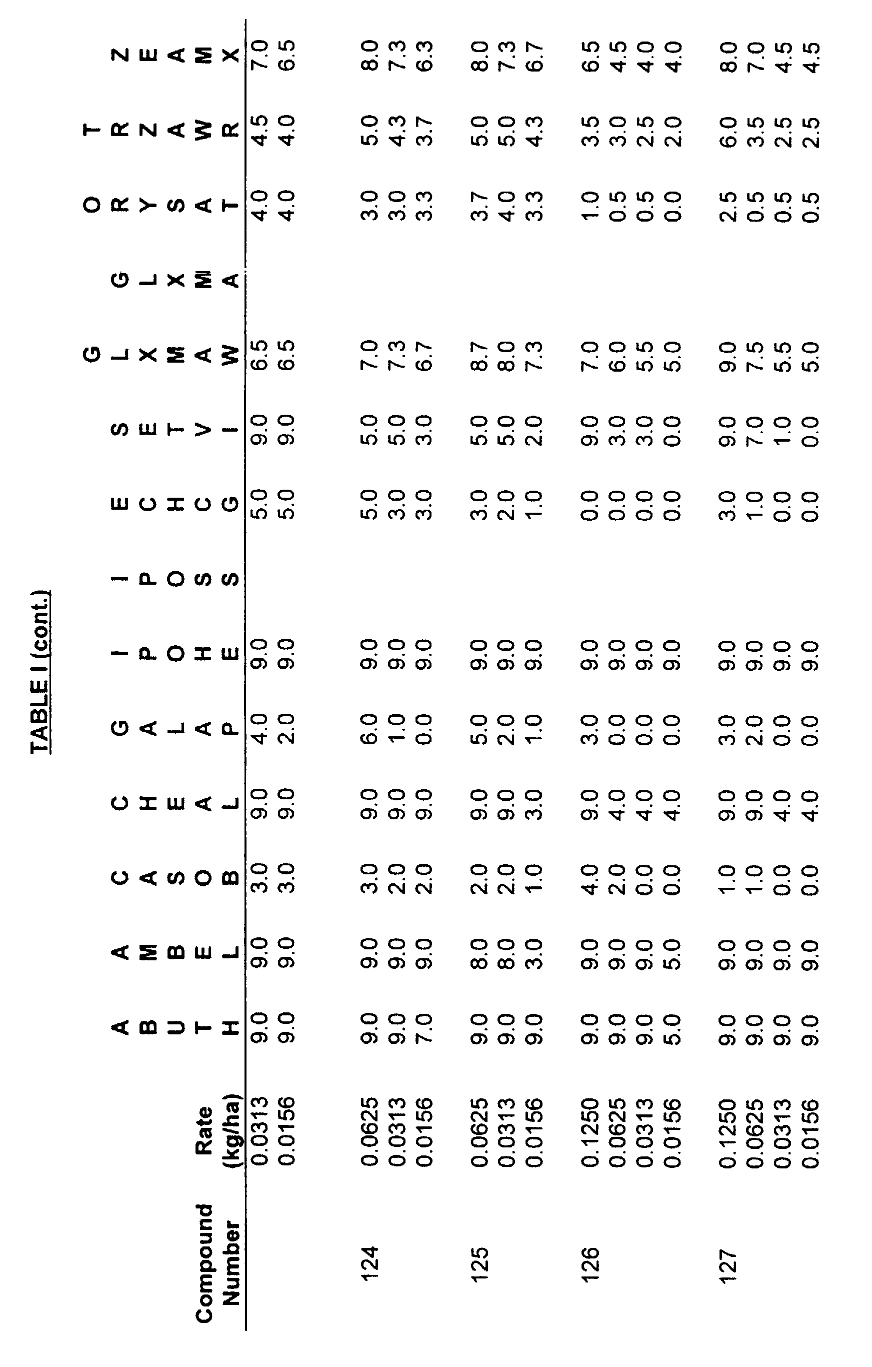

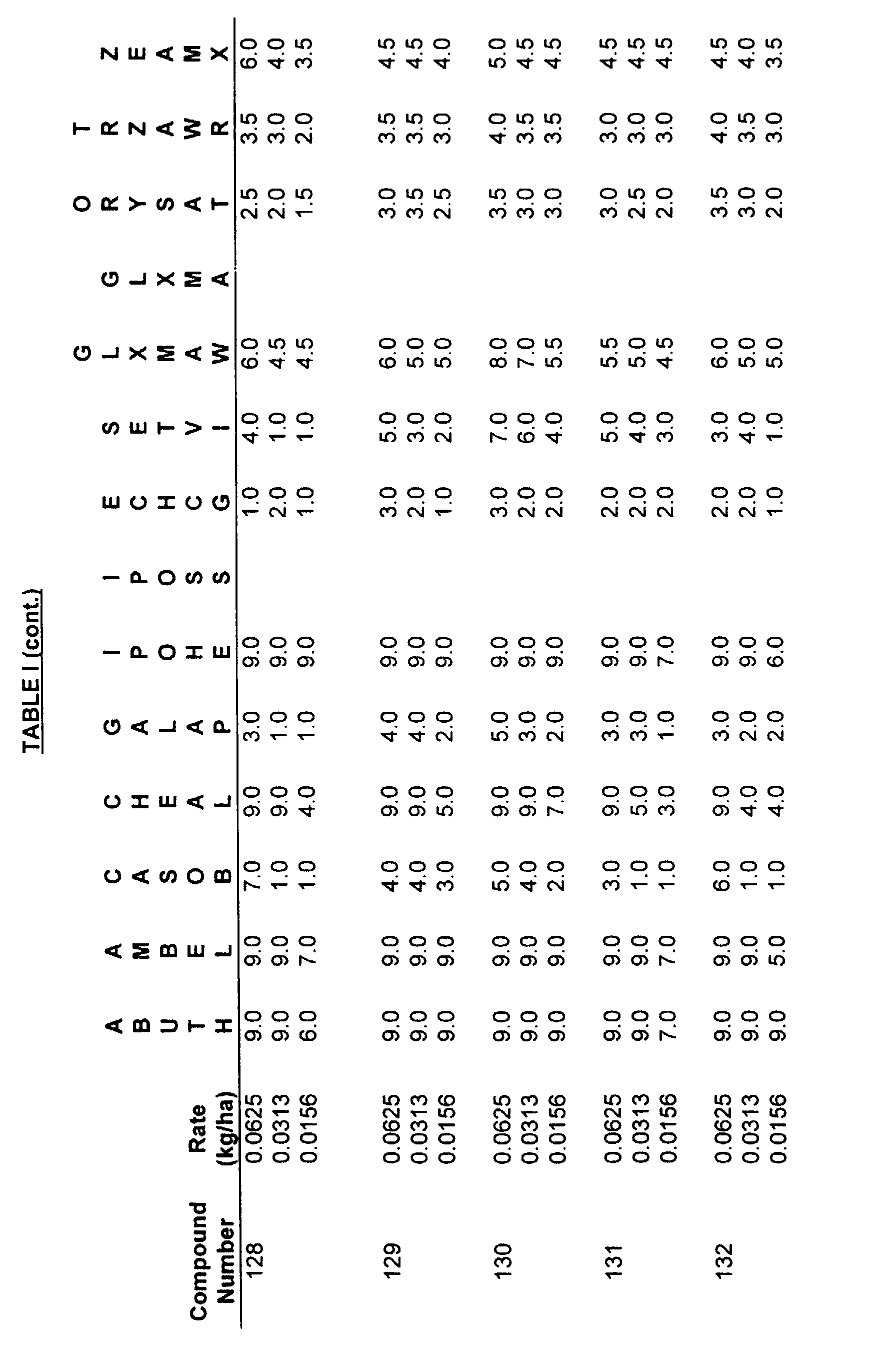

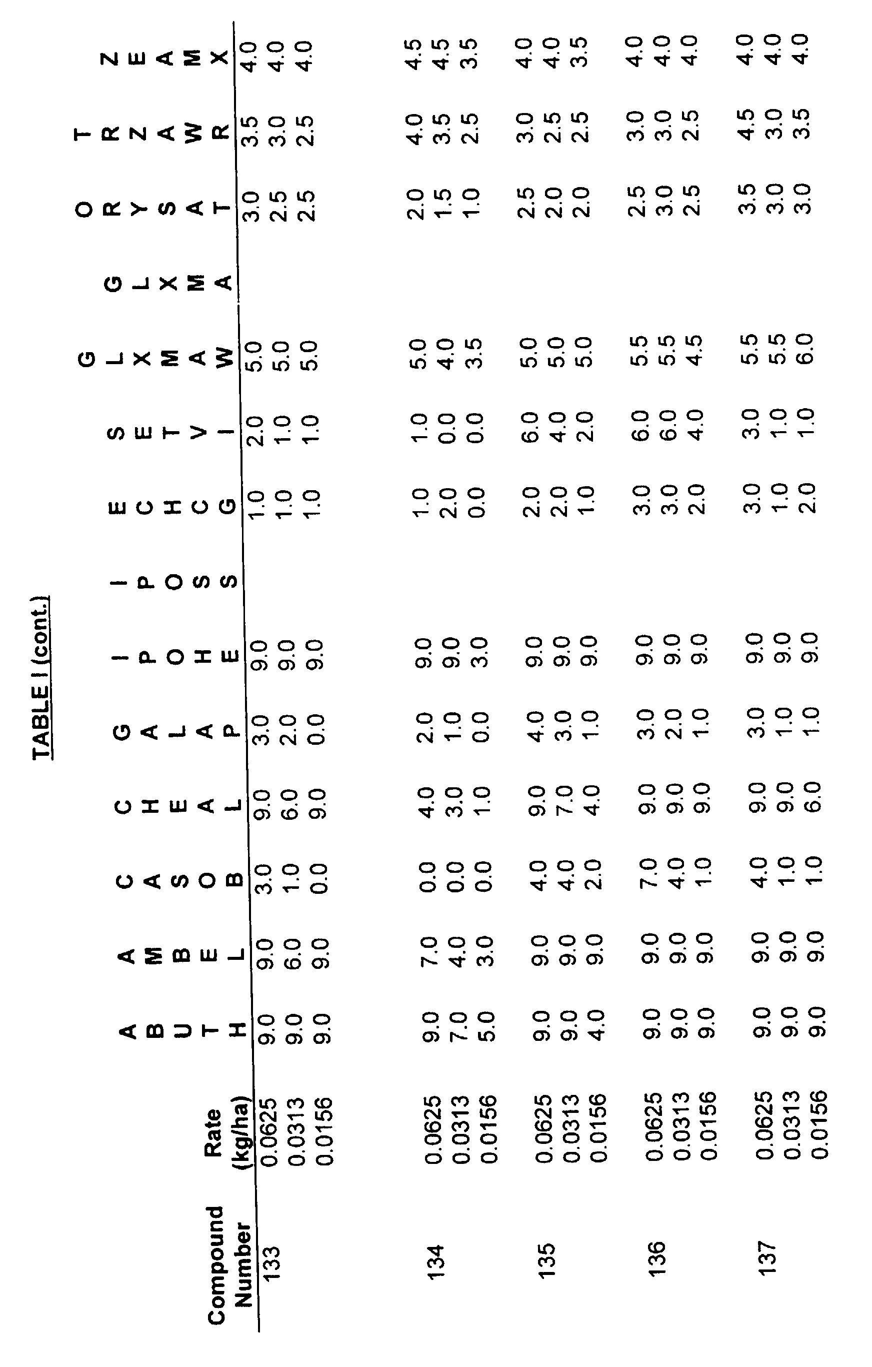

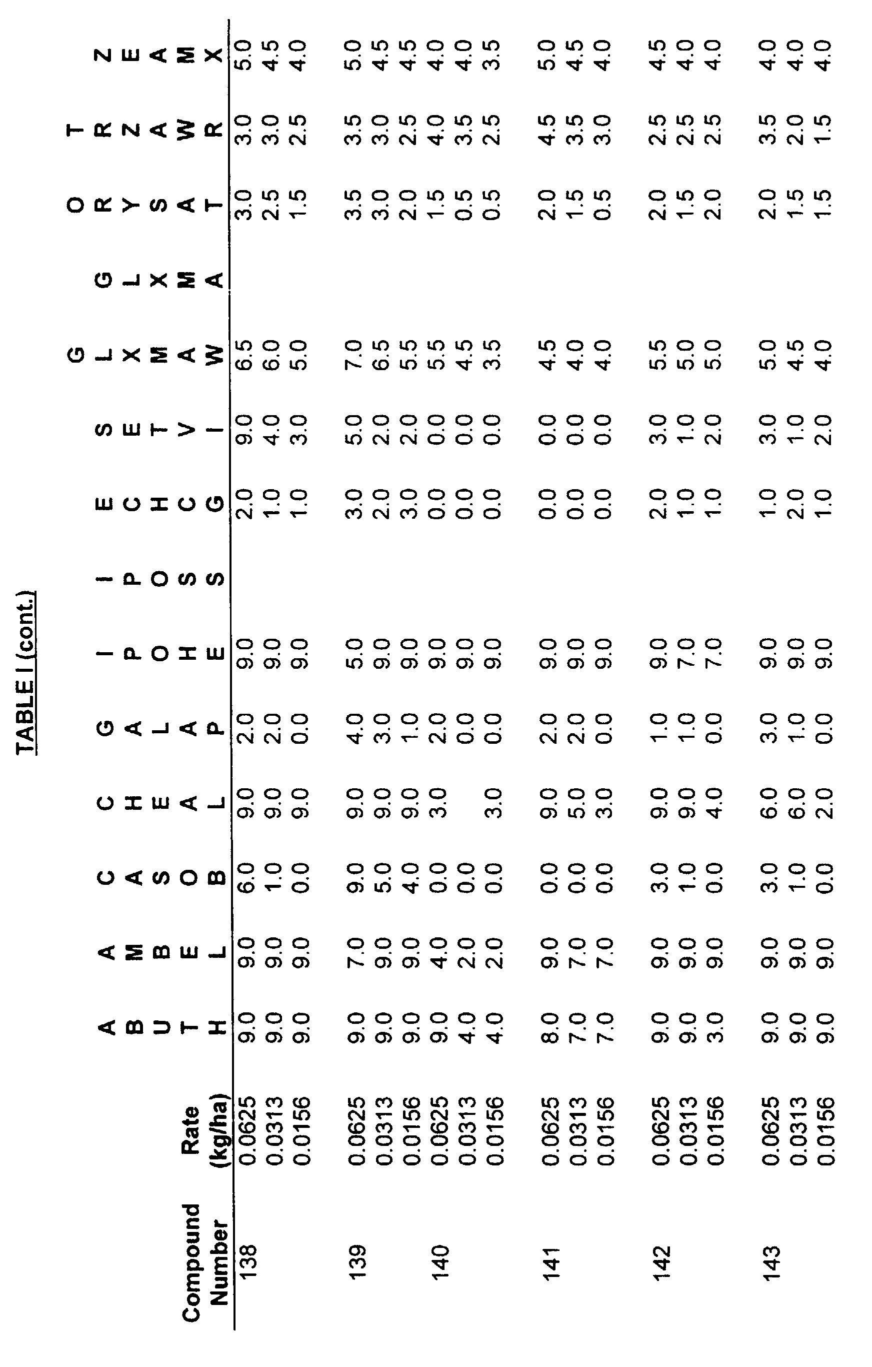

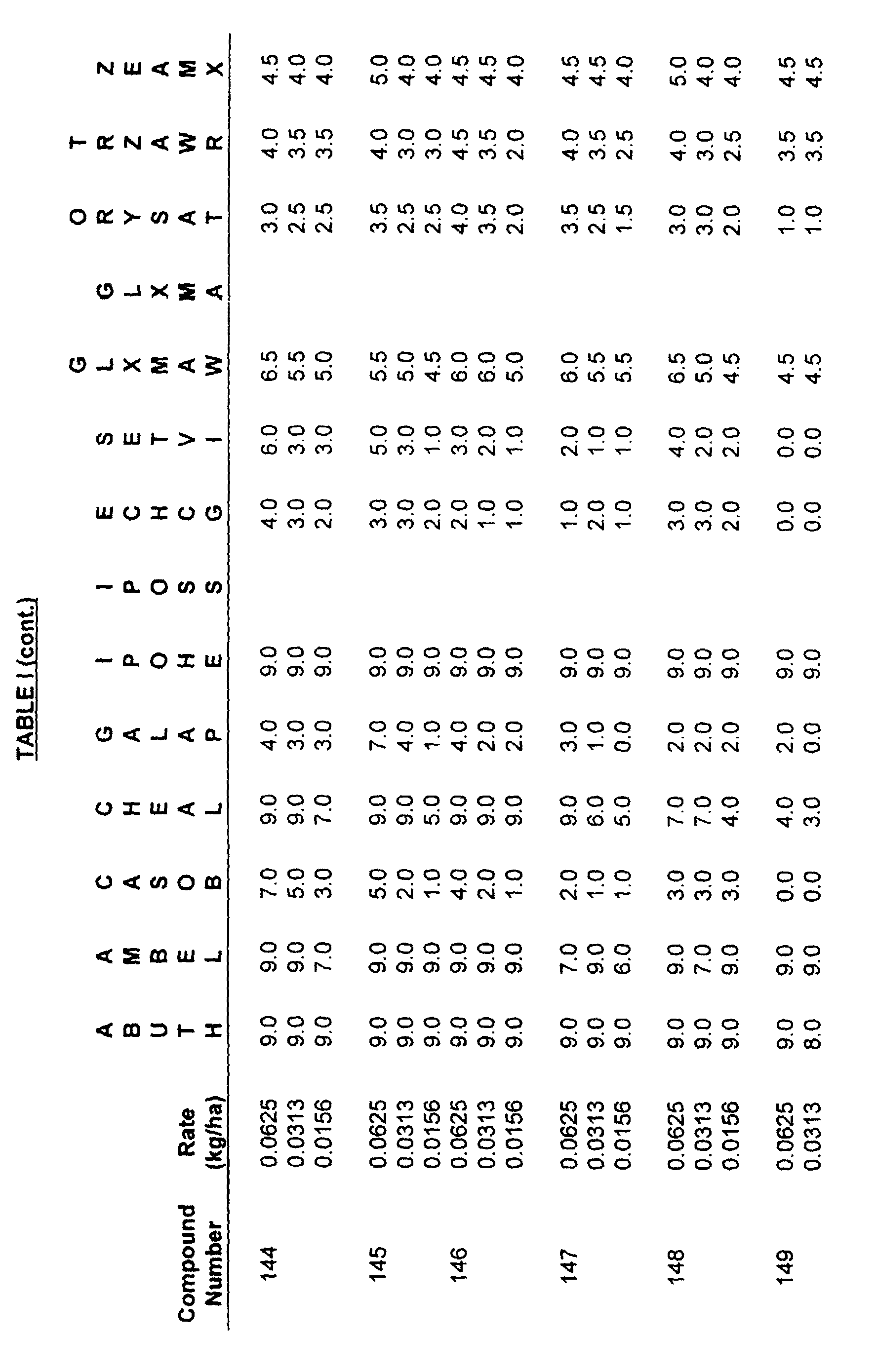

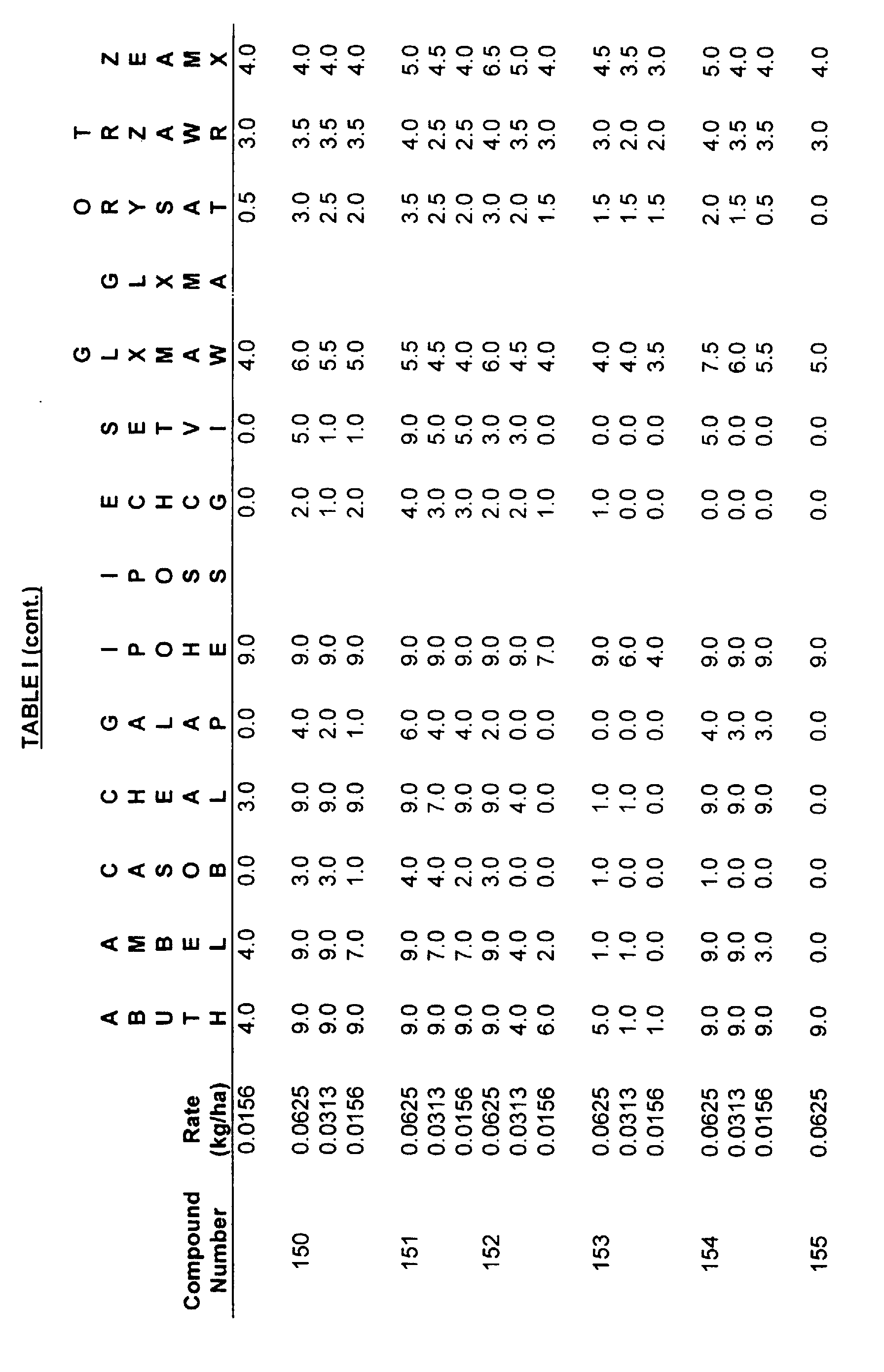

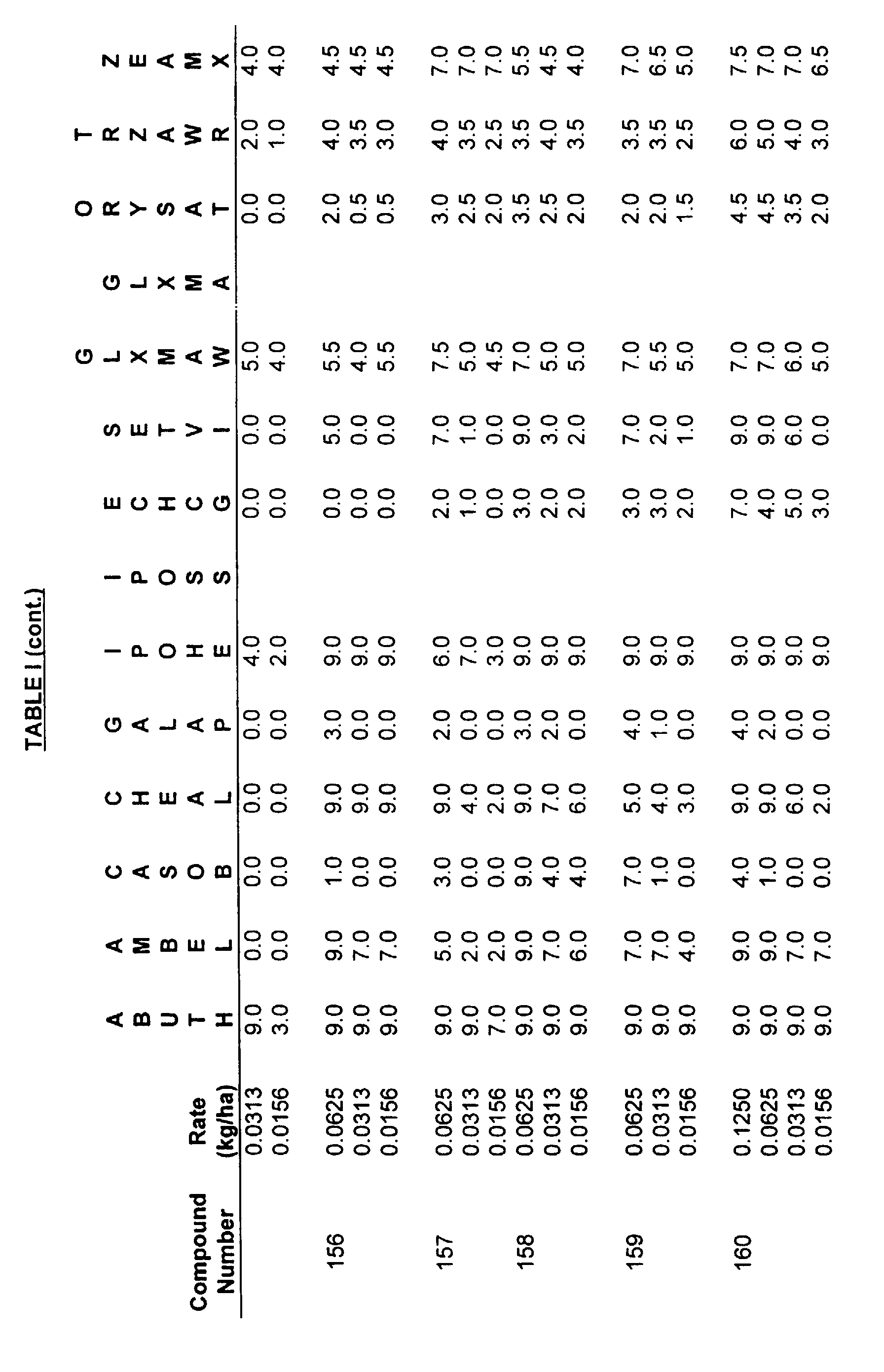

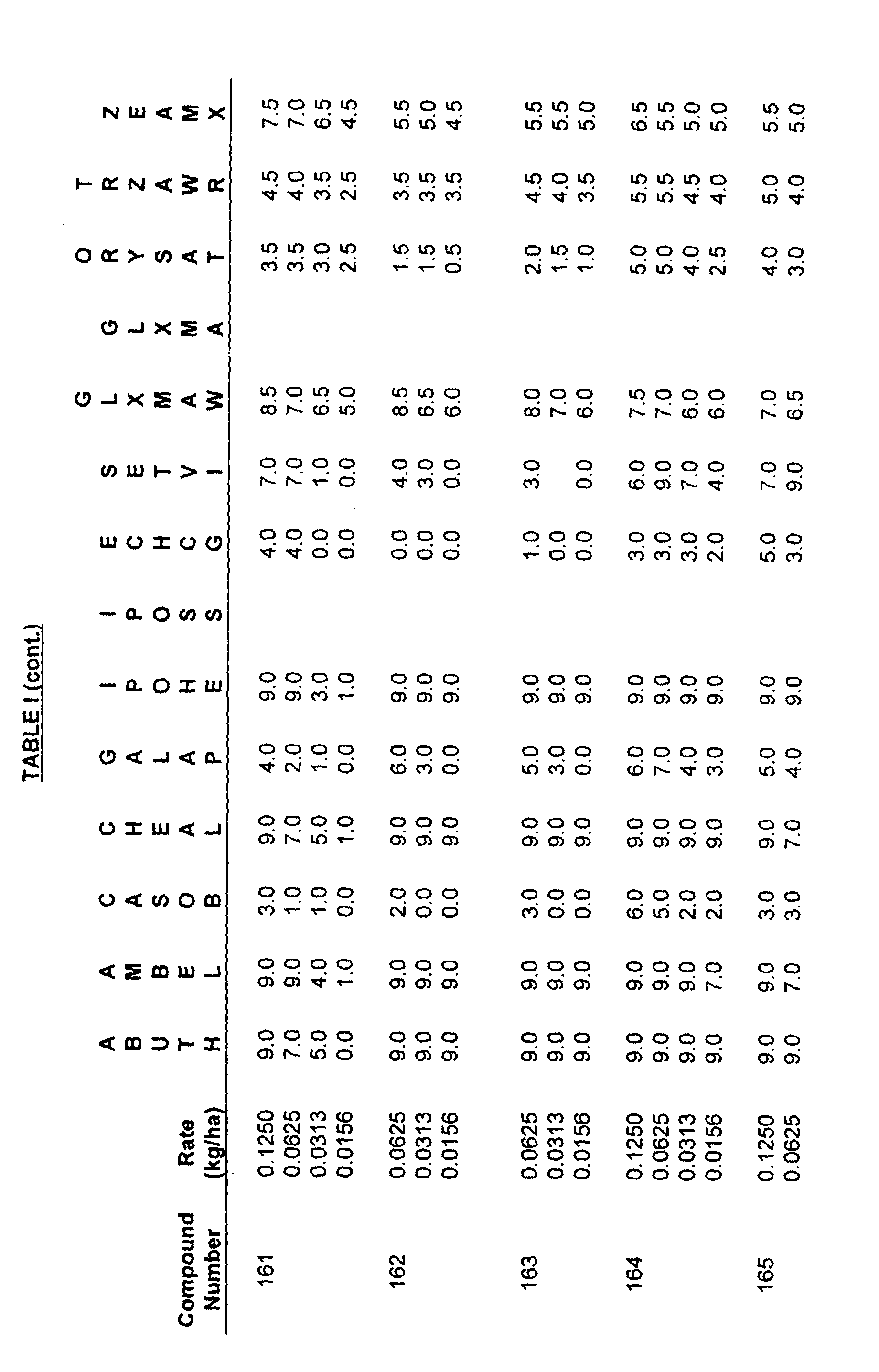

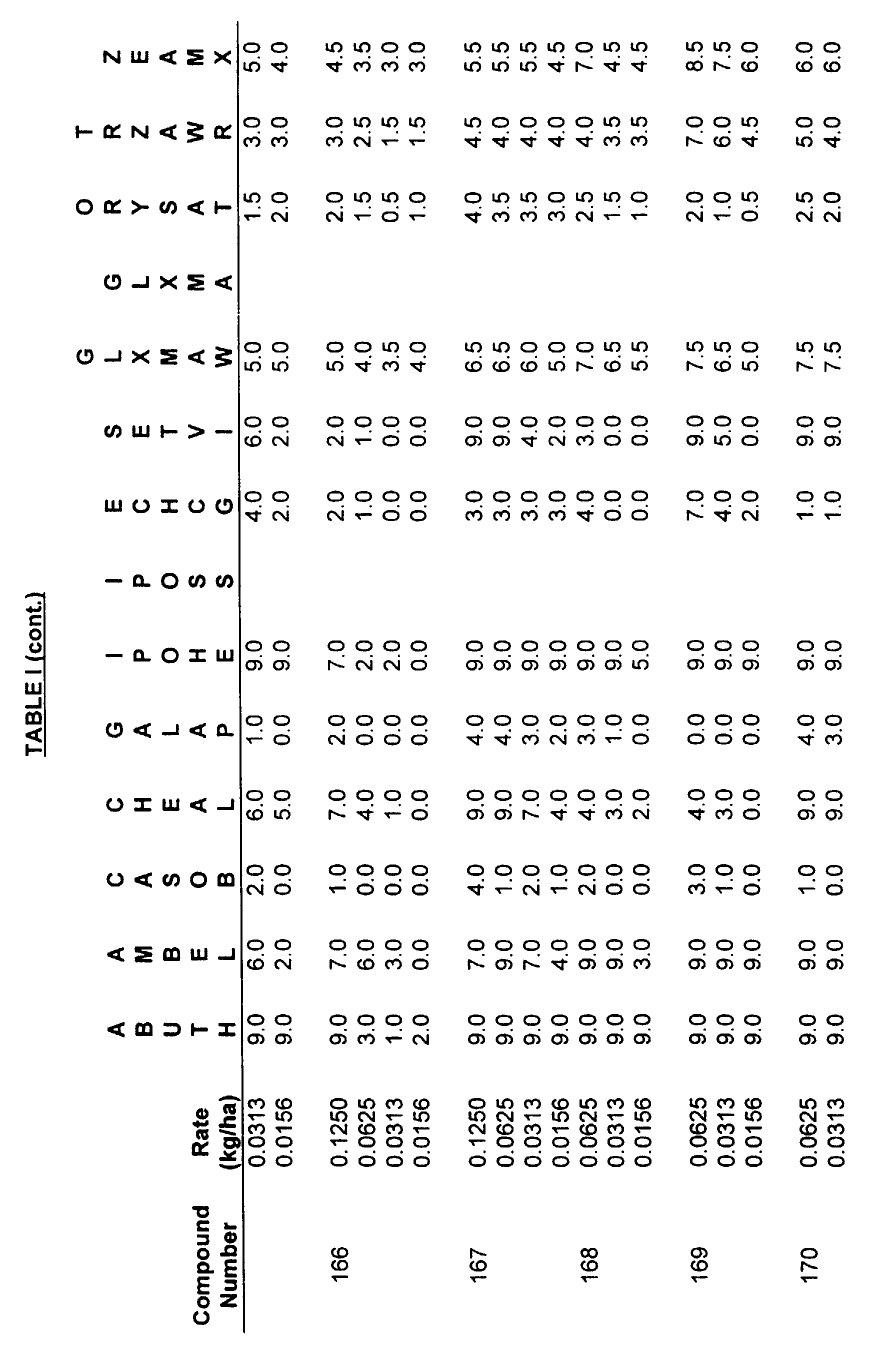

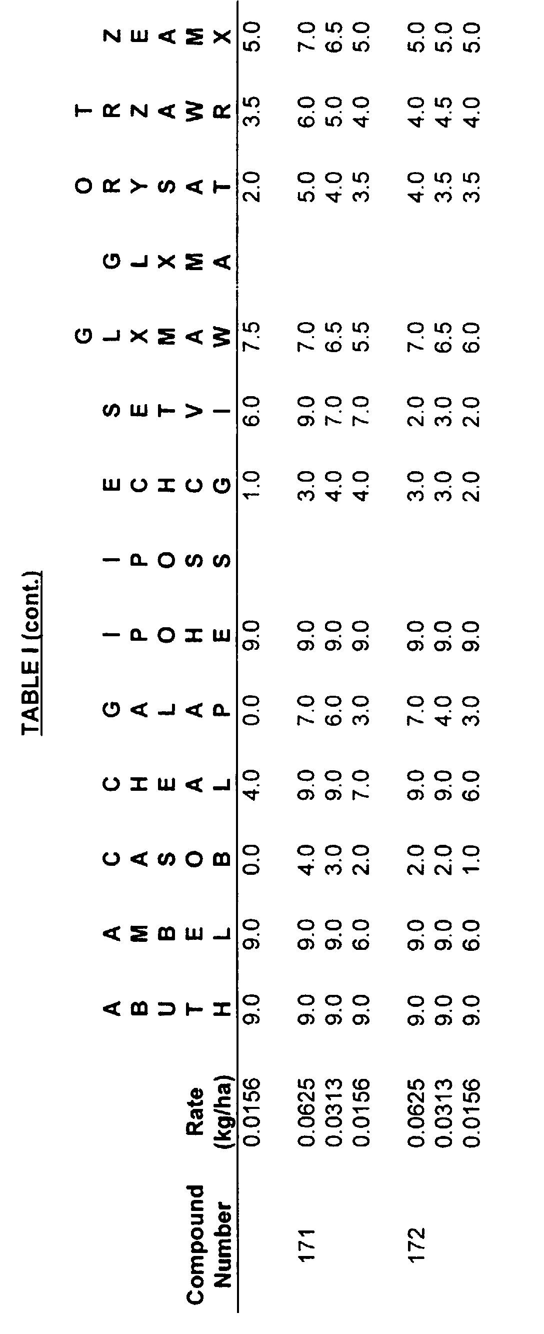

### EXAMPLE 38

### Preemergence herbicidal evaluation of test compounds

The preemergence herbicidal activity of the test compounds of the present invention is exemplified by the following tests in which the seeds of a variety of monocotyledonous and dicotyledonous plants are separately mixed with potting soil and planted on top of approximately one inch of soil in separate pint cups. After planting, the cups are sprayed with the selected aqueous acetone solution containing test compound in sufficient quantity to provide the equivalent of about 0.0156 to 0.500 kg per hectare of test compound per cup. The treated cups are then placed on greenhouse benches, watered and cared for in accordance with conventional greenhouse procedures. From four to five weeks after treatment, the tests are terminated and each cup is examined and rated according to the rating system provided in Example 37.
The data obtained are reported in Table II below. The compounds evaluated are reported by compound number given in Example 37.

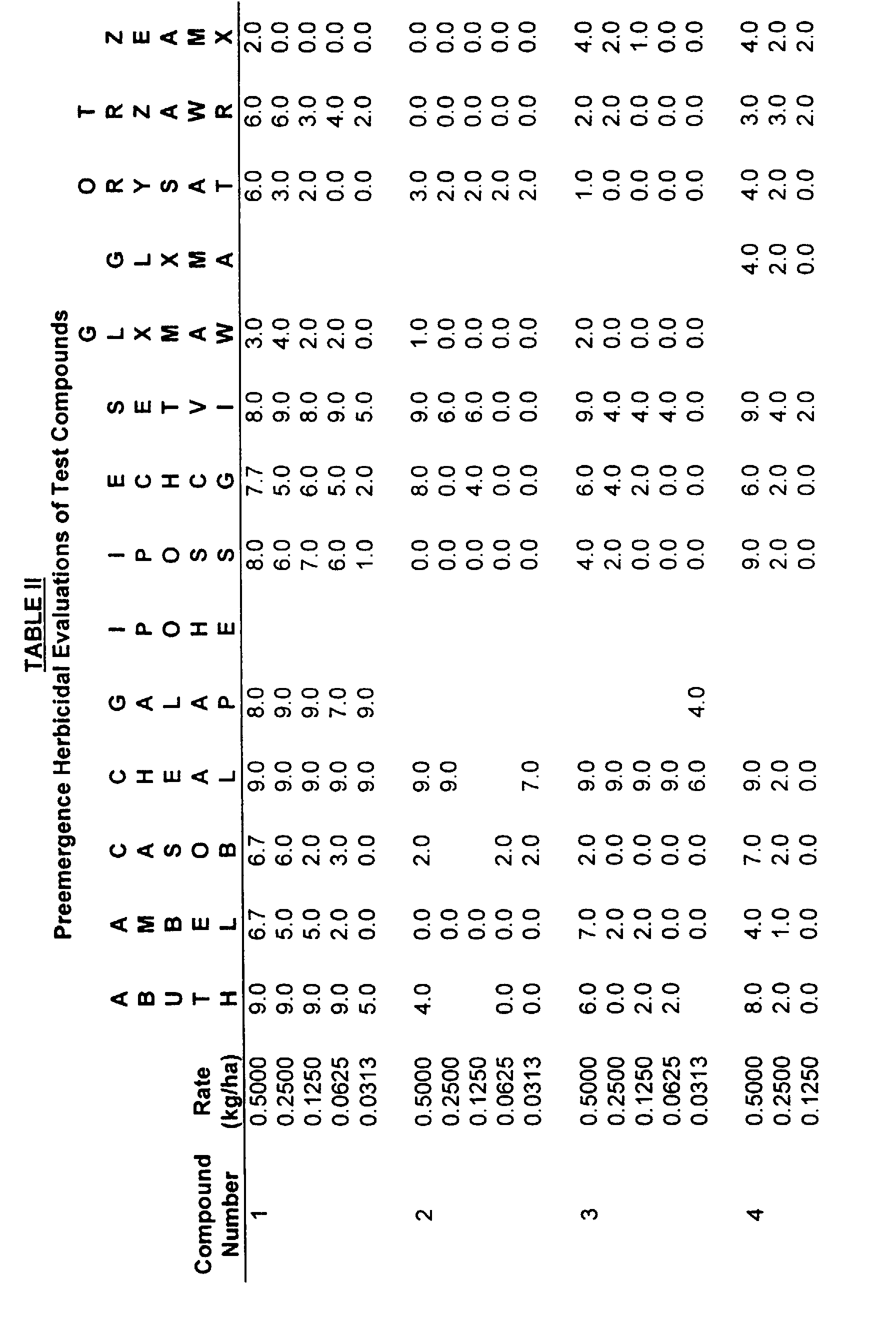

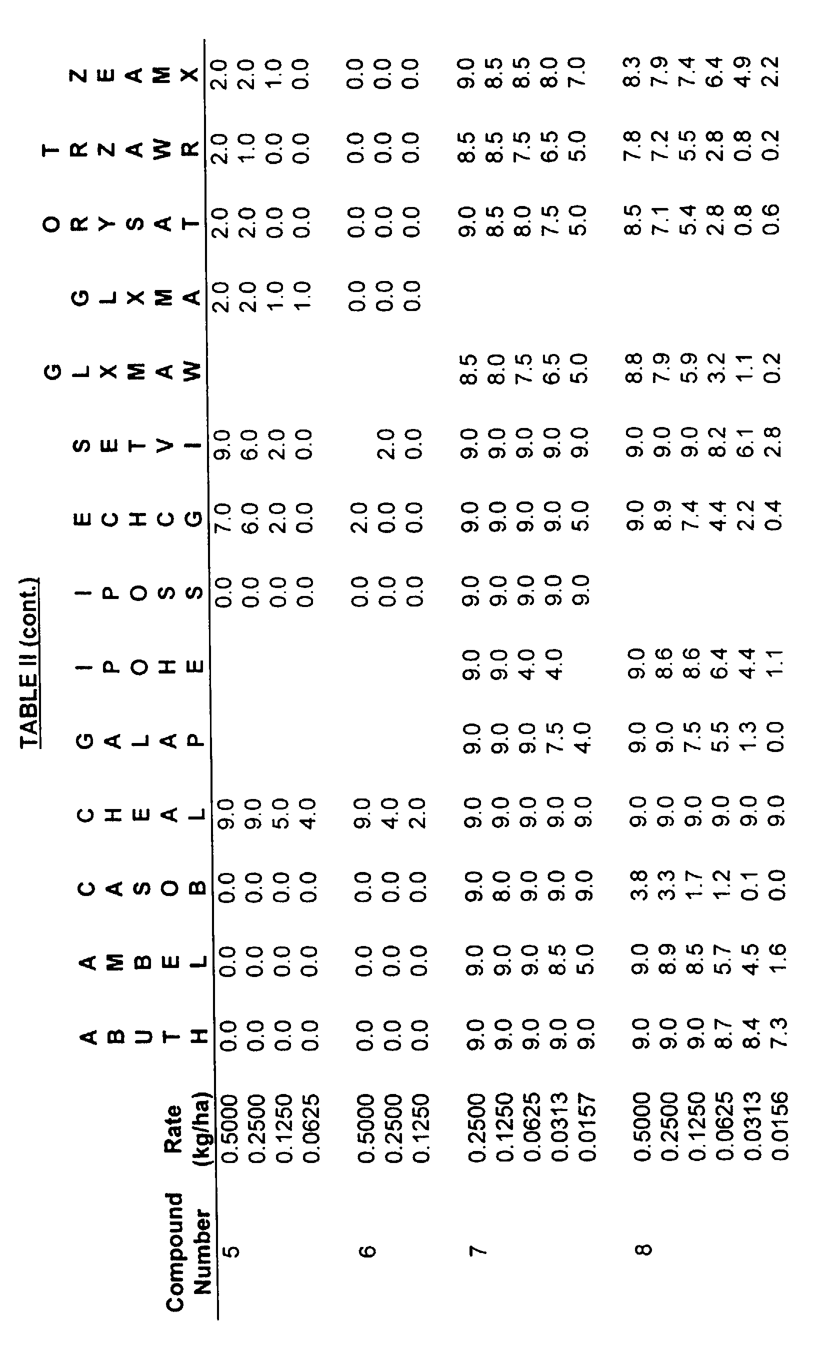

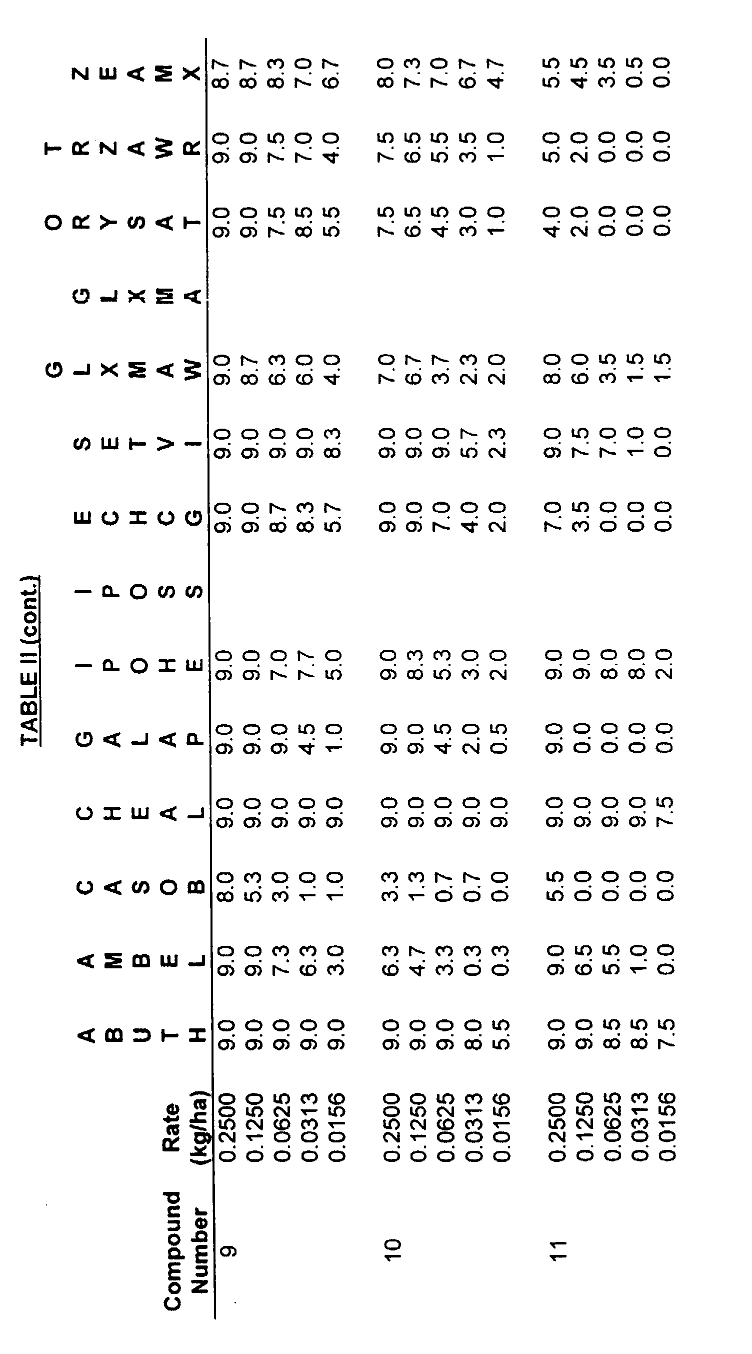

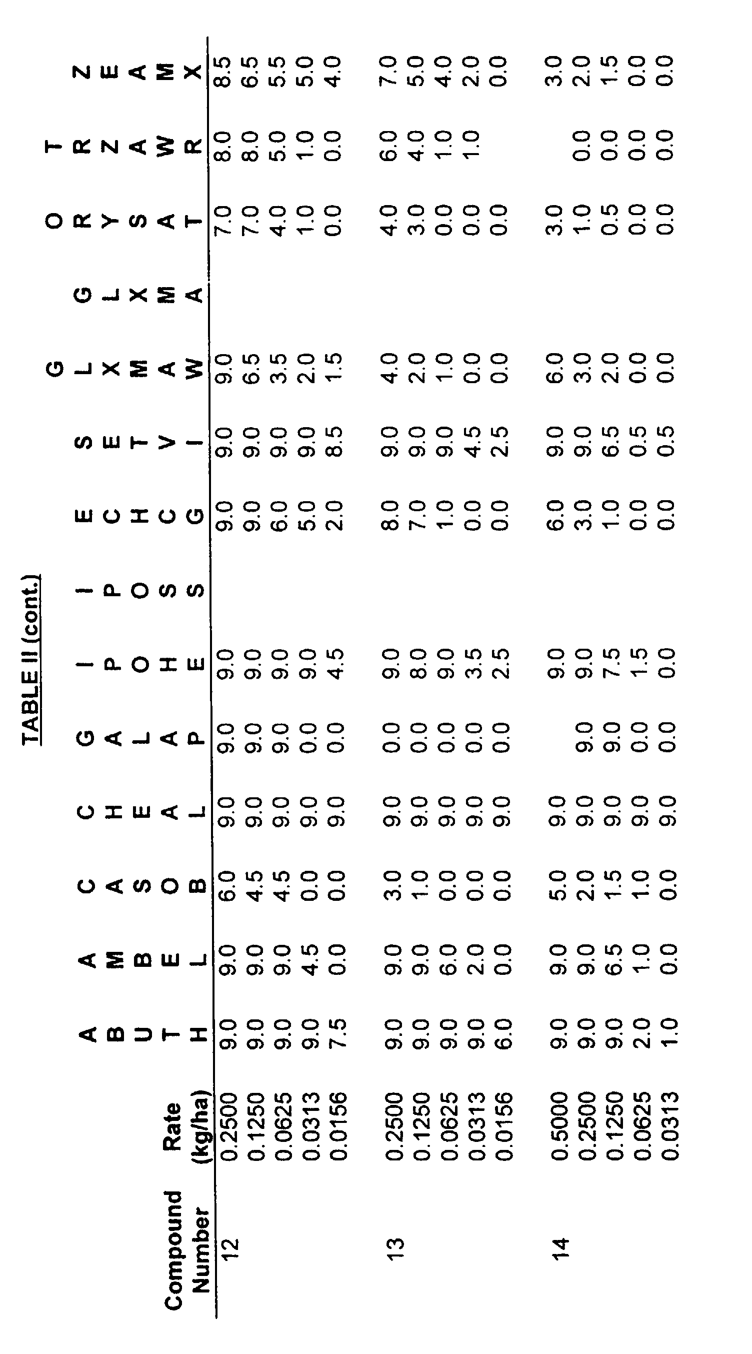

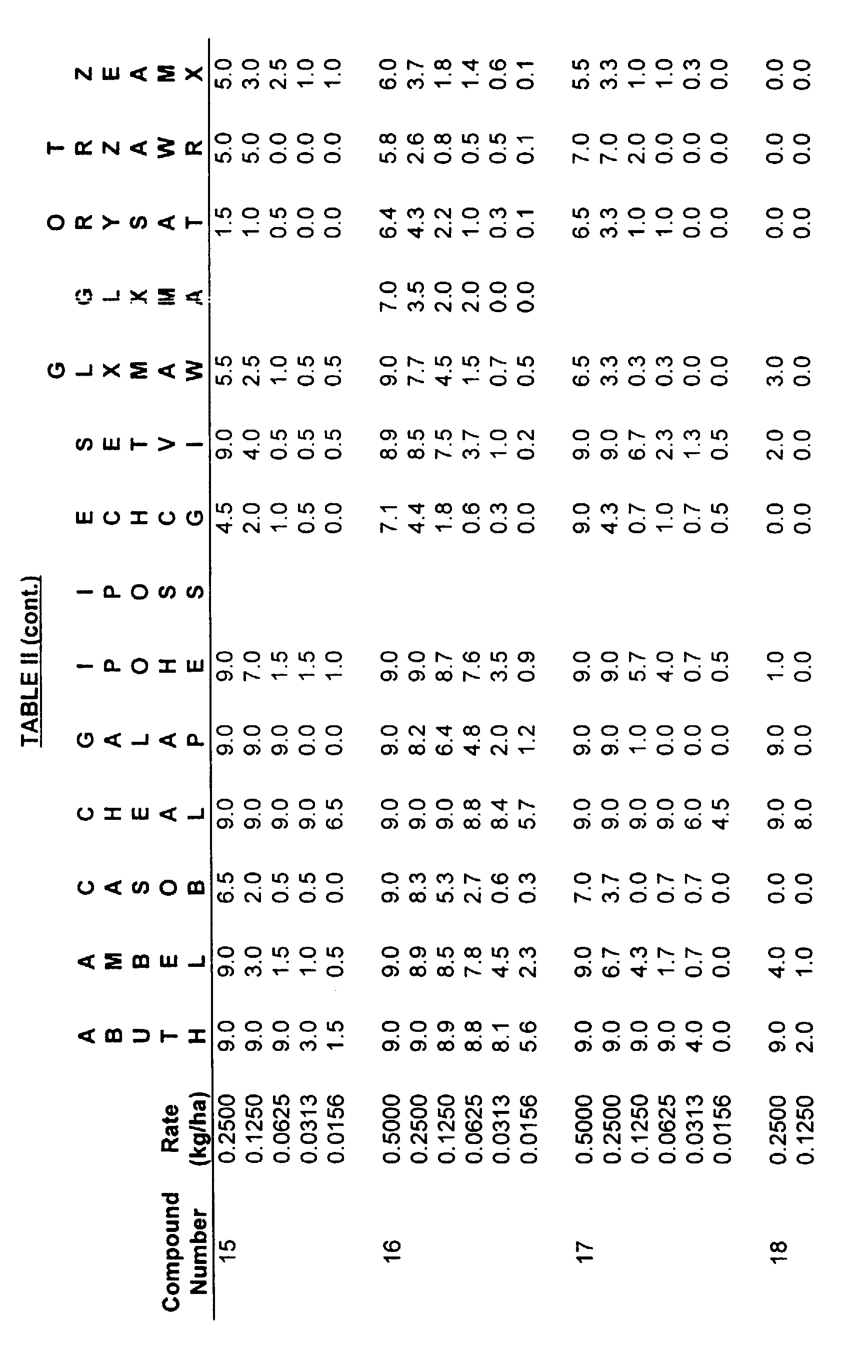

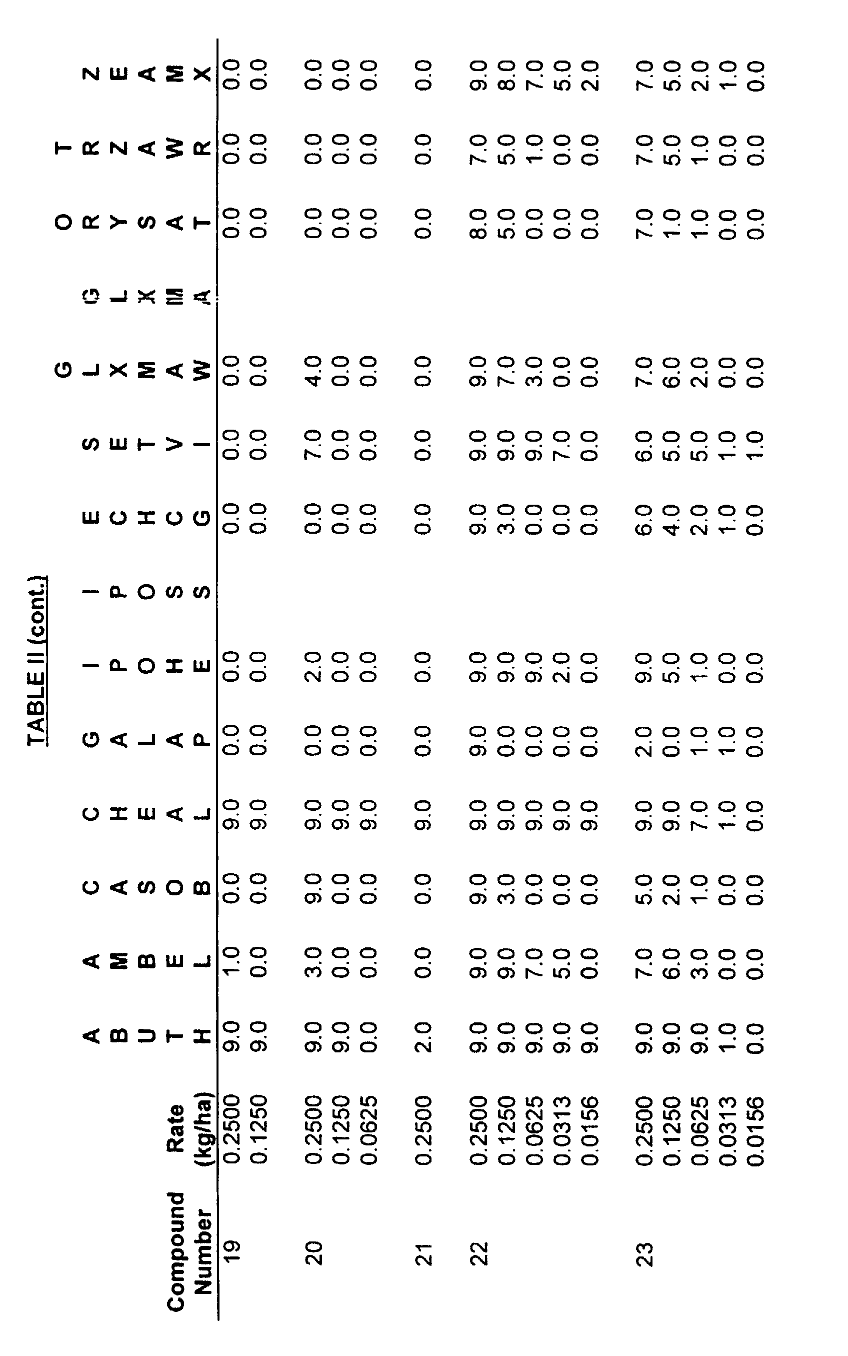

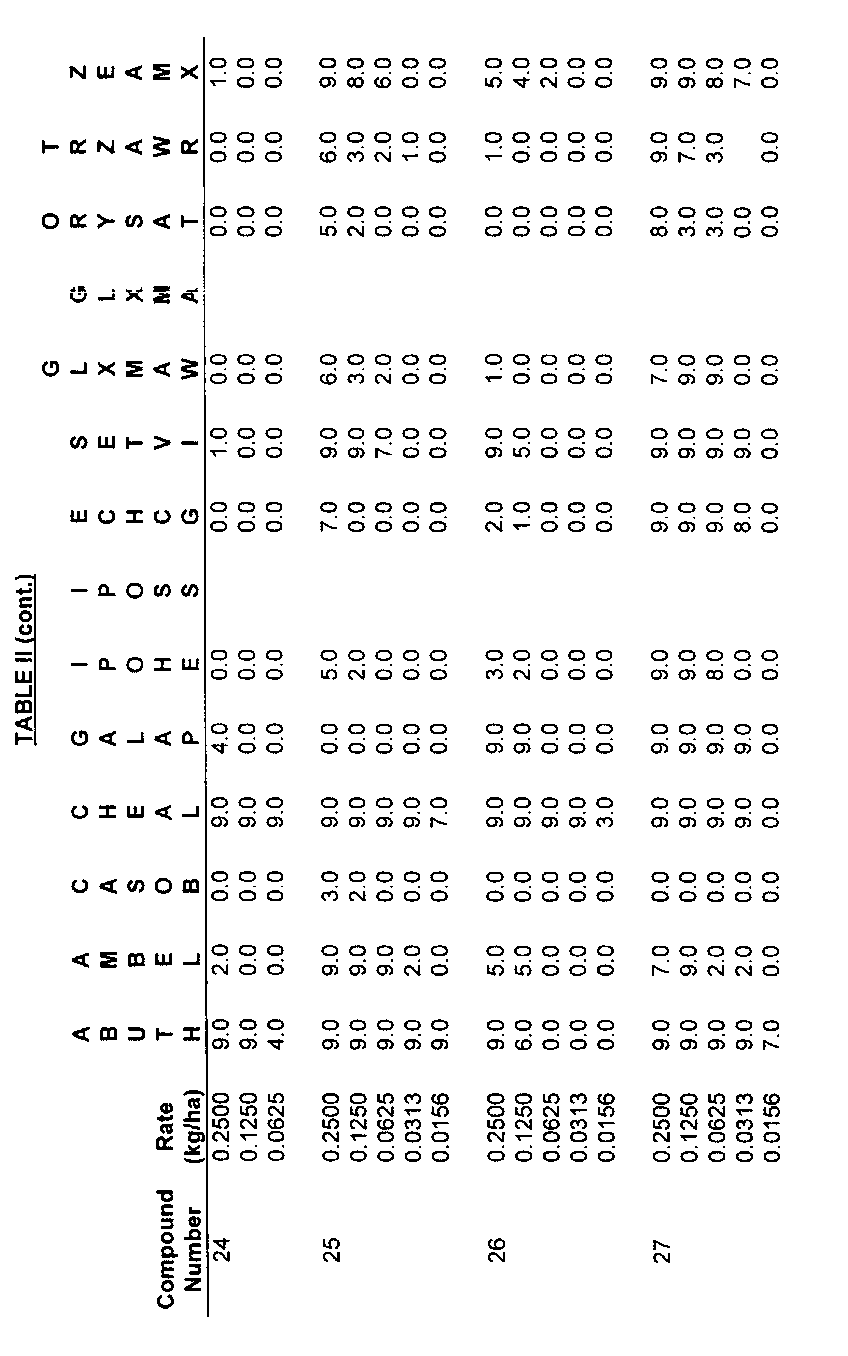

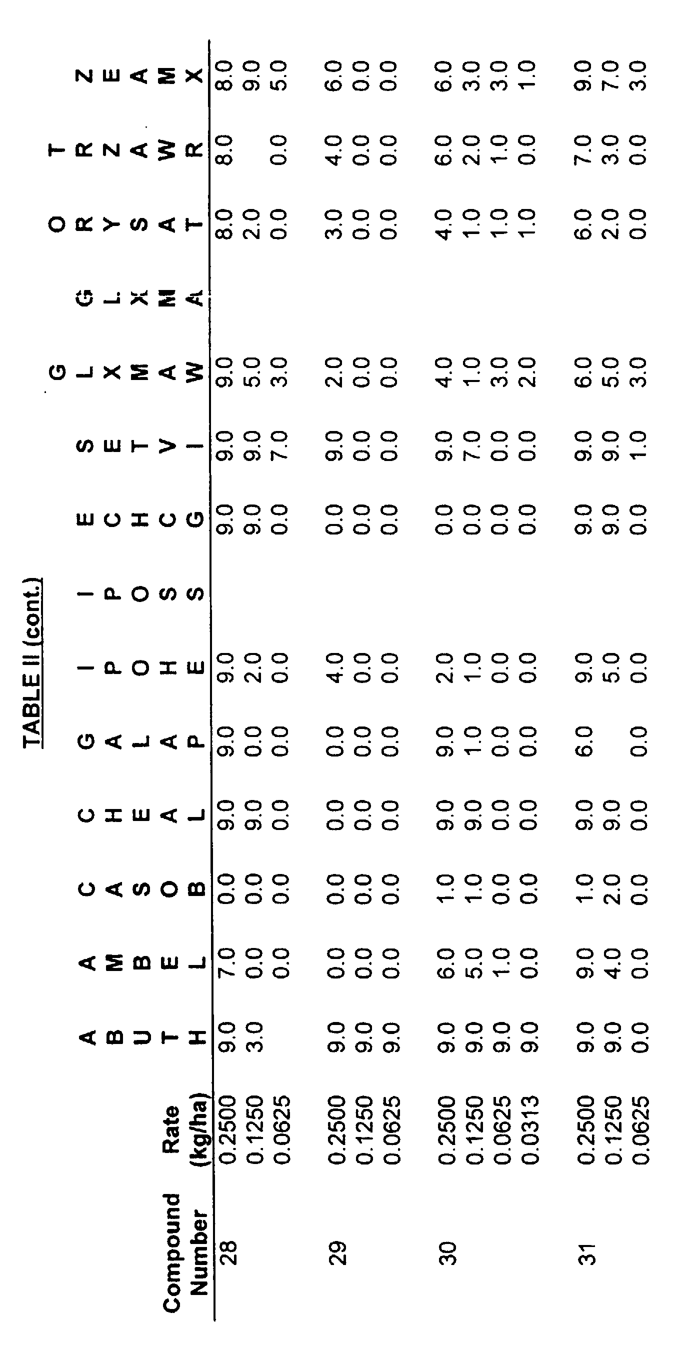

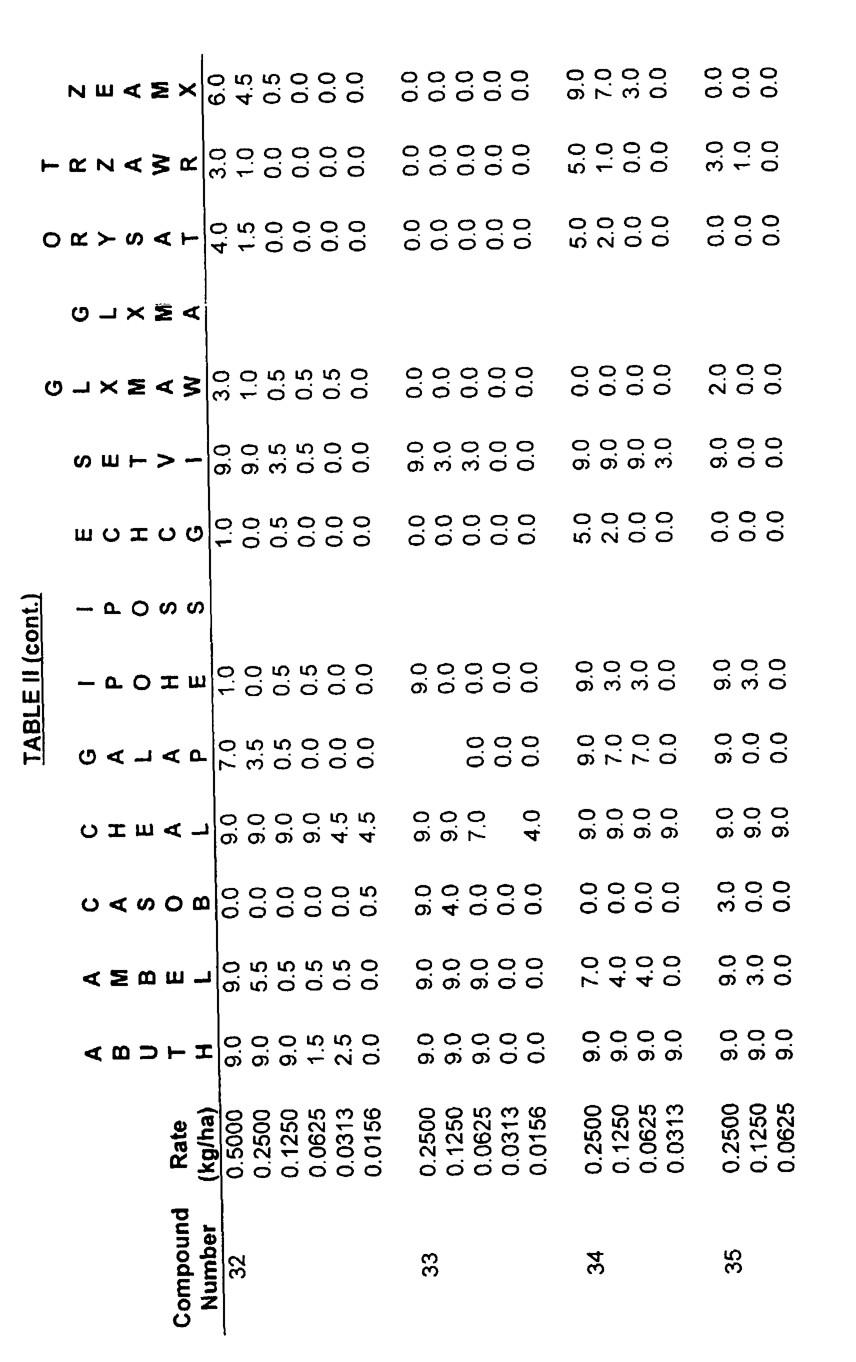

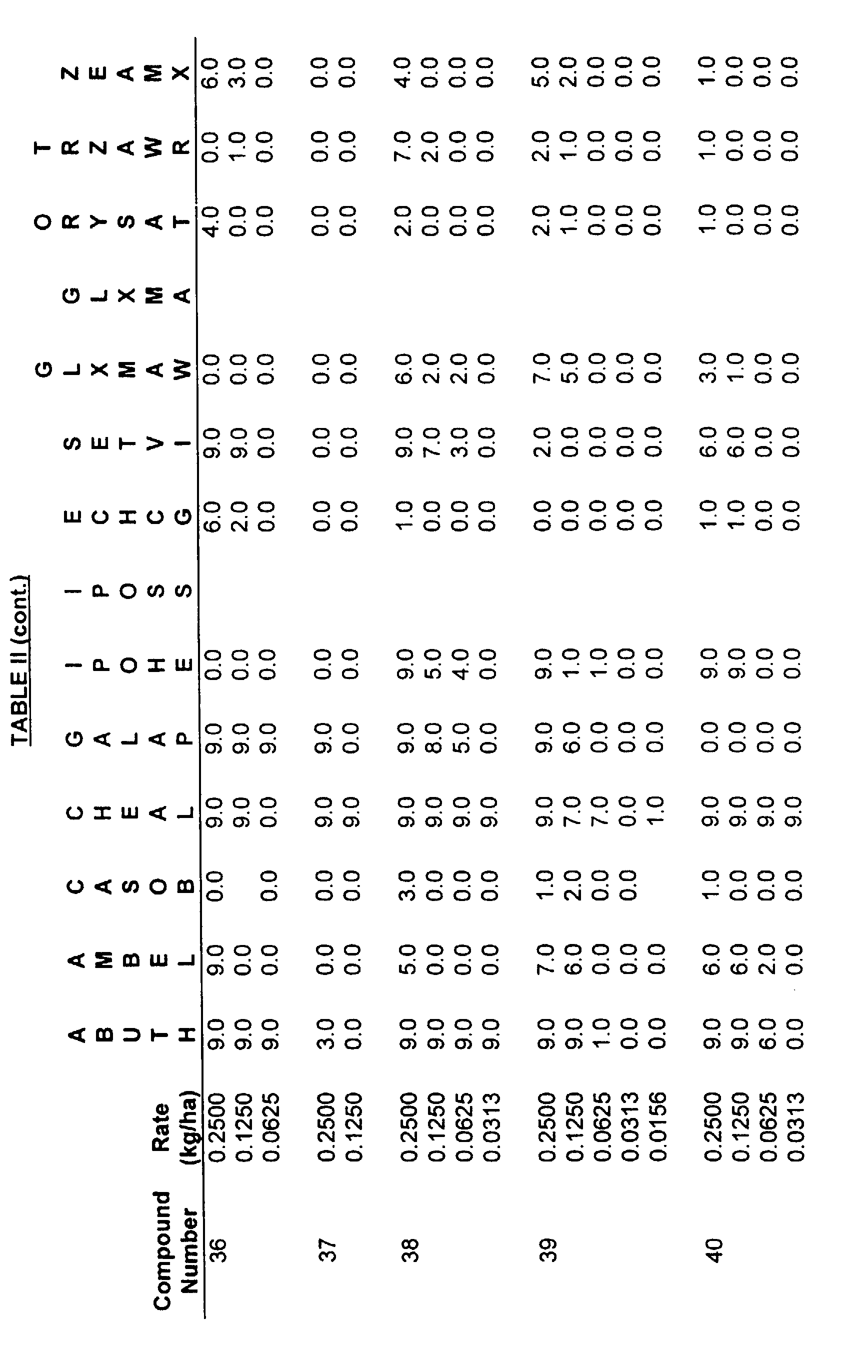

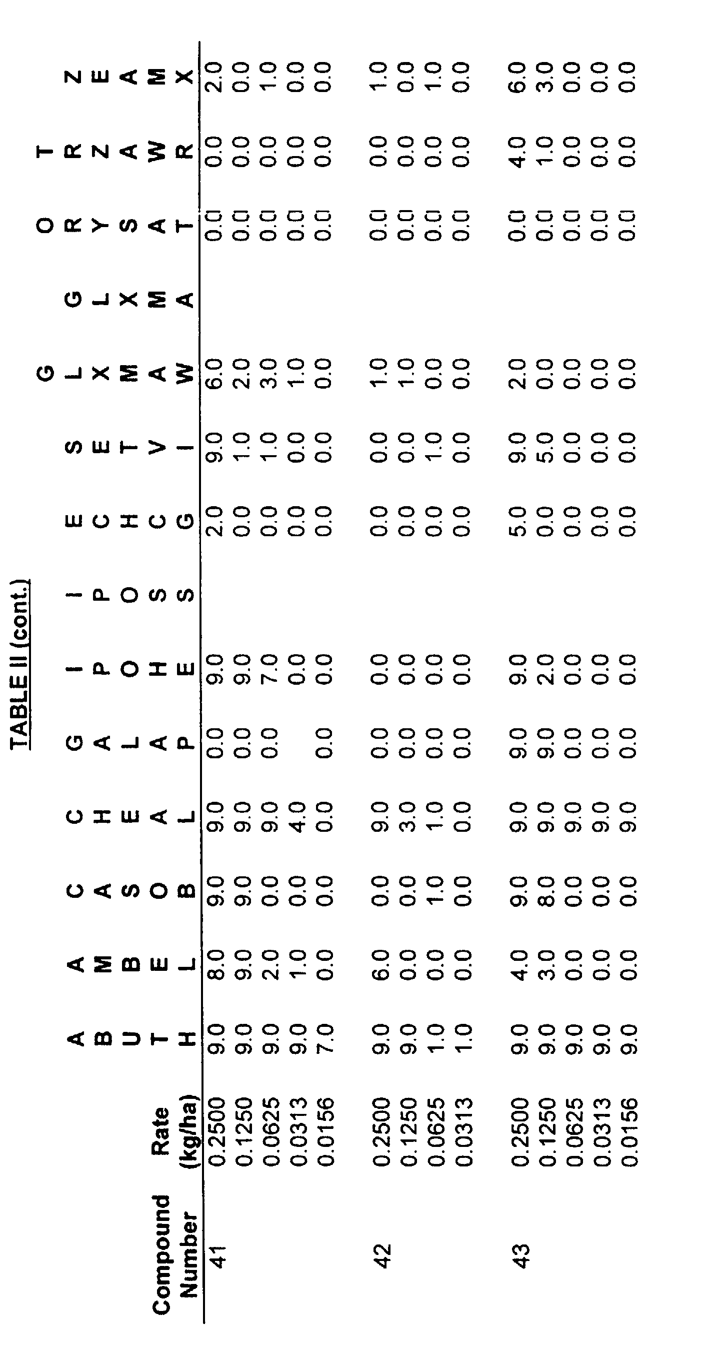

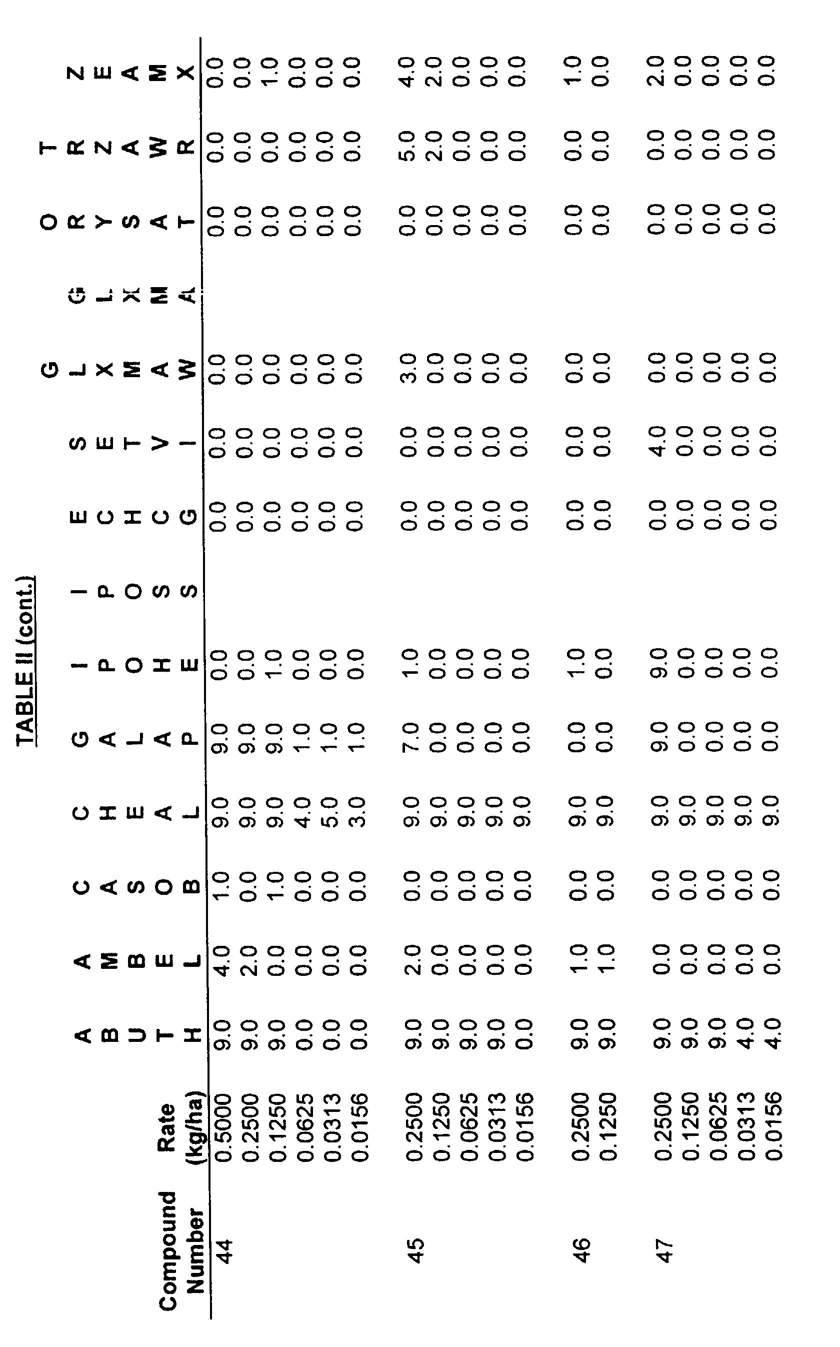

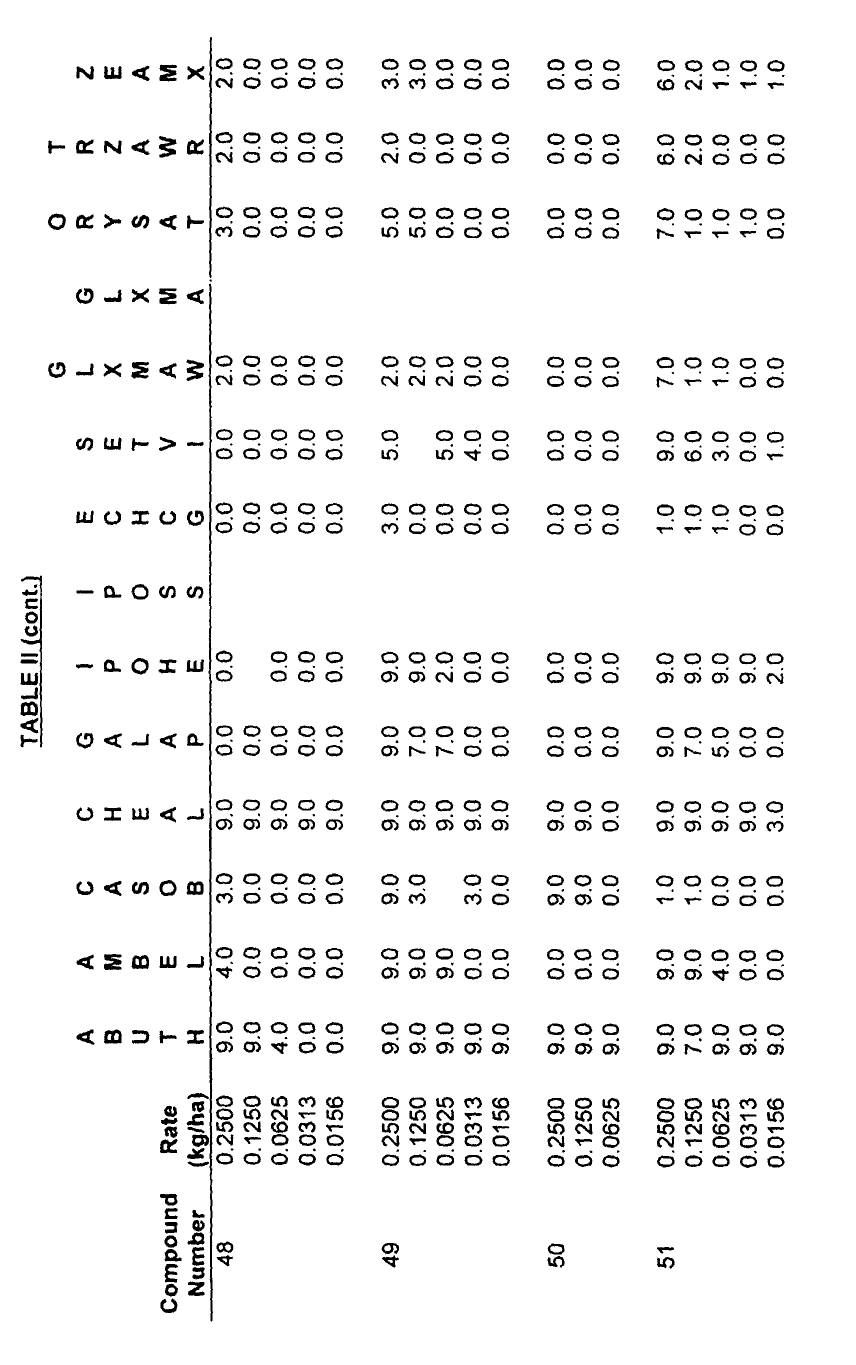

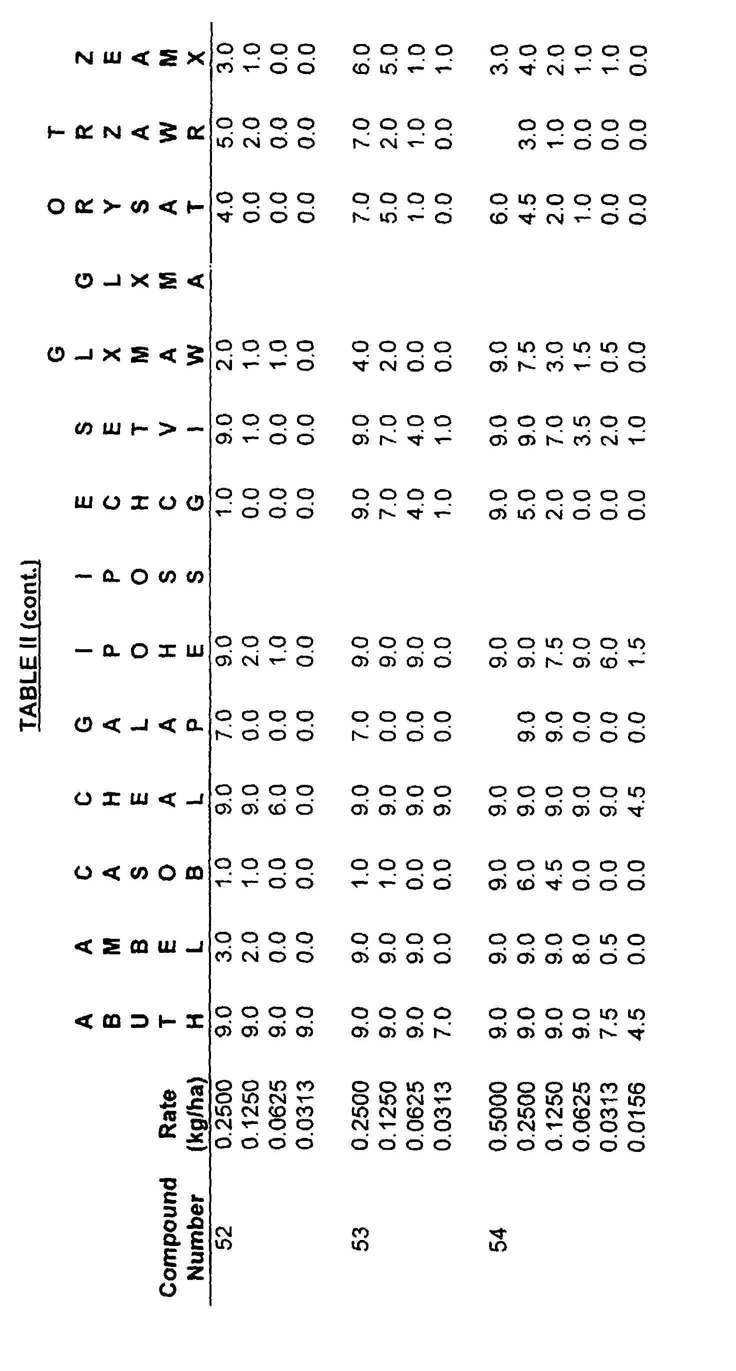

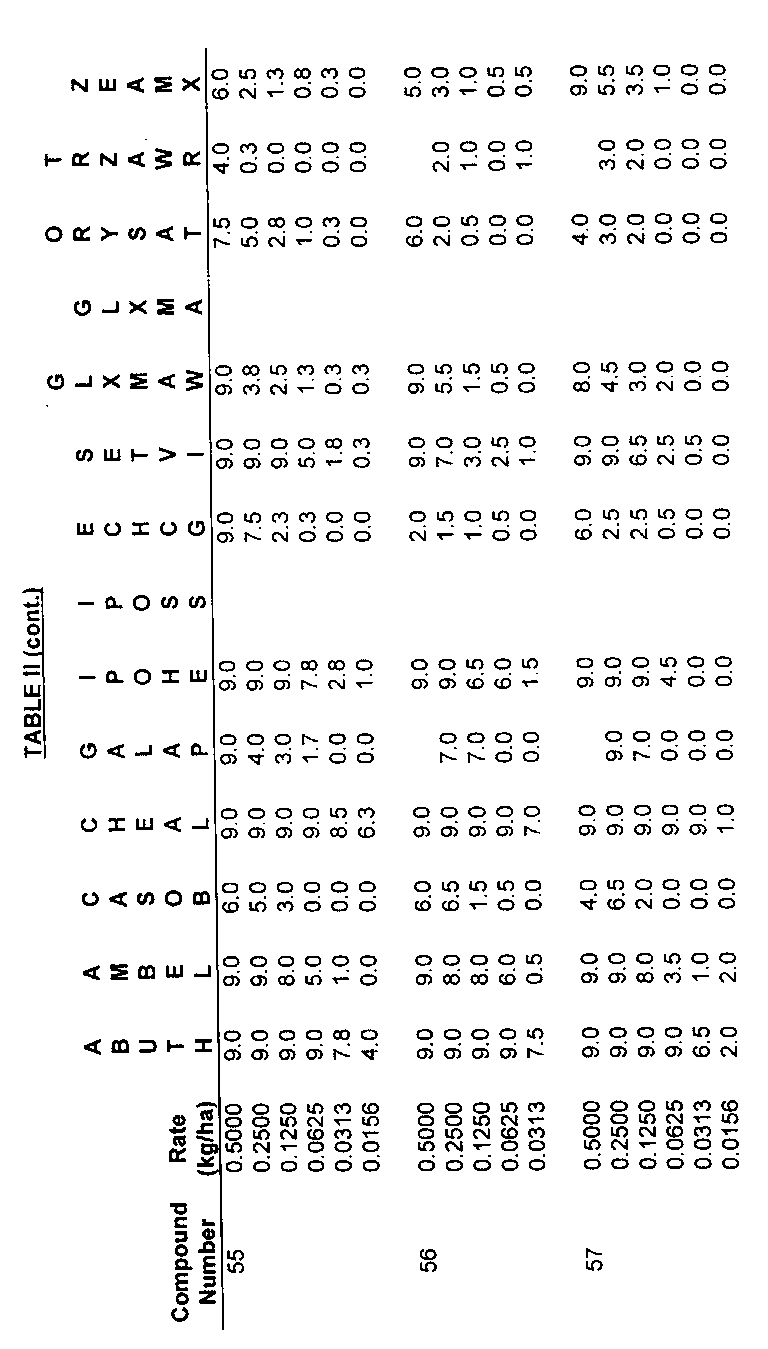

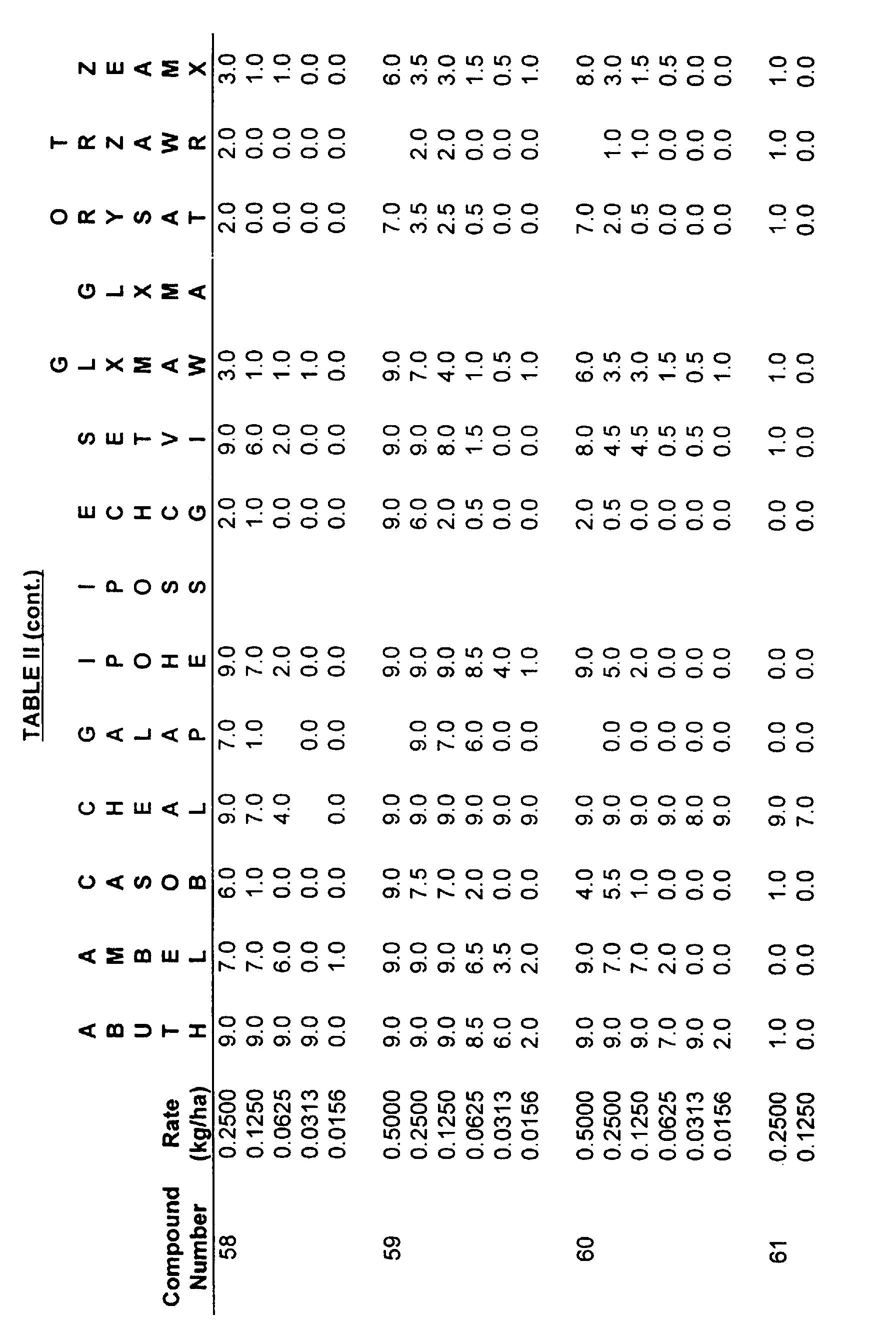

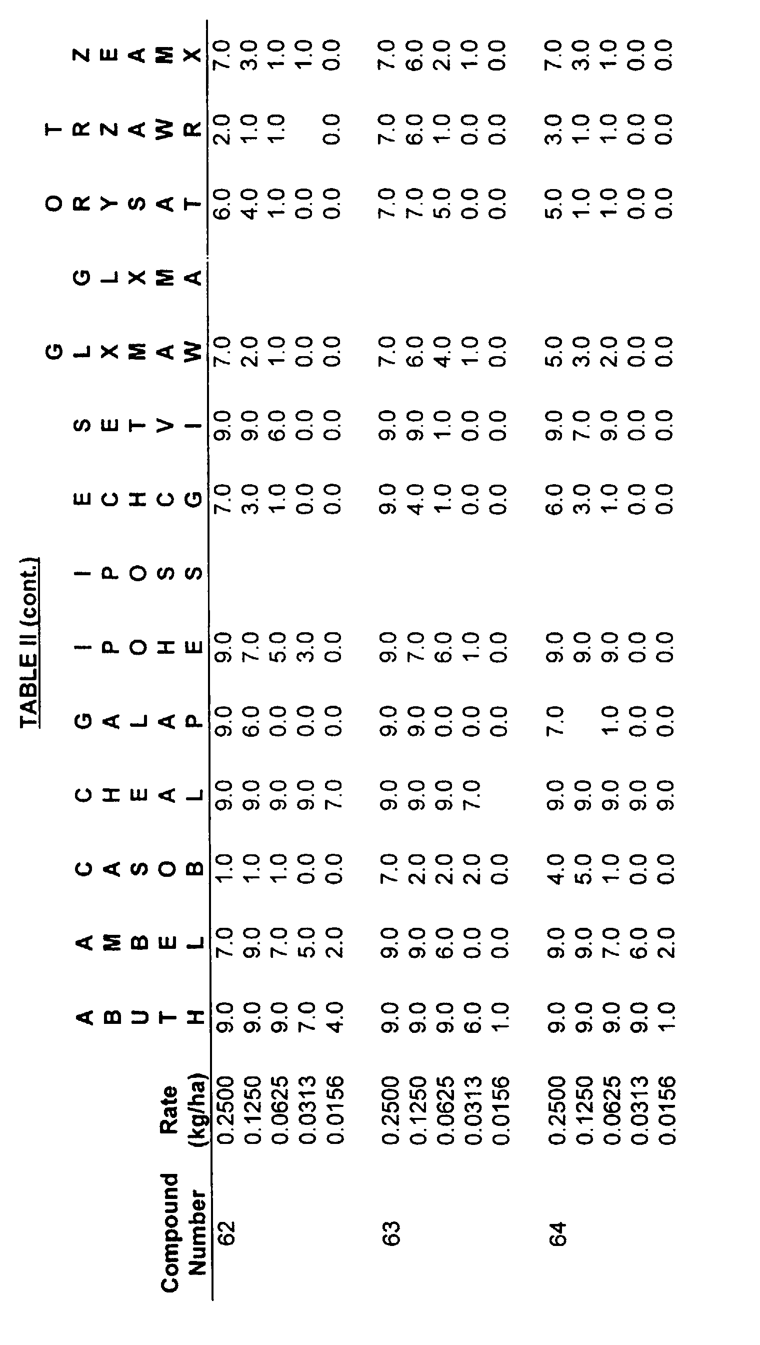

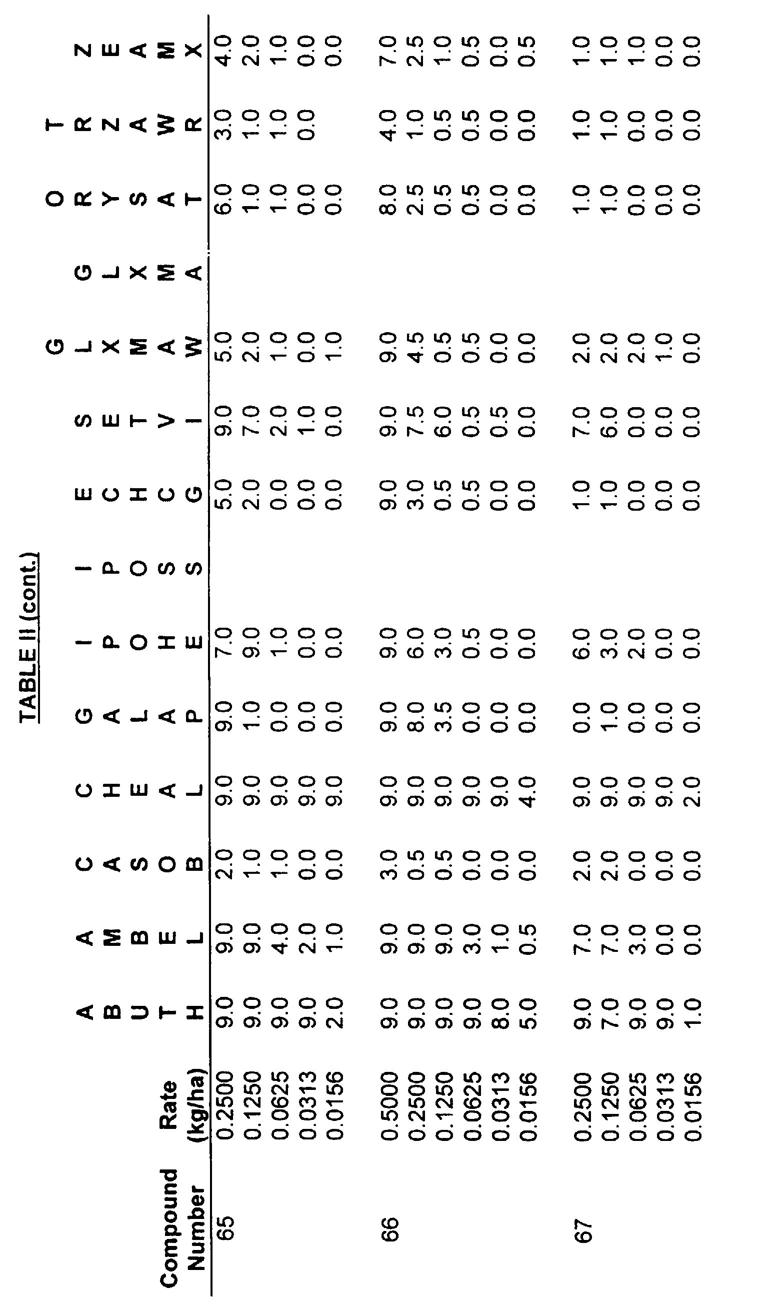

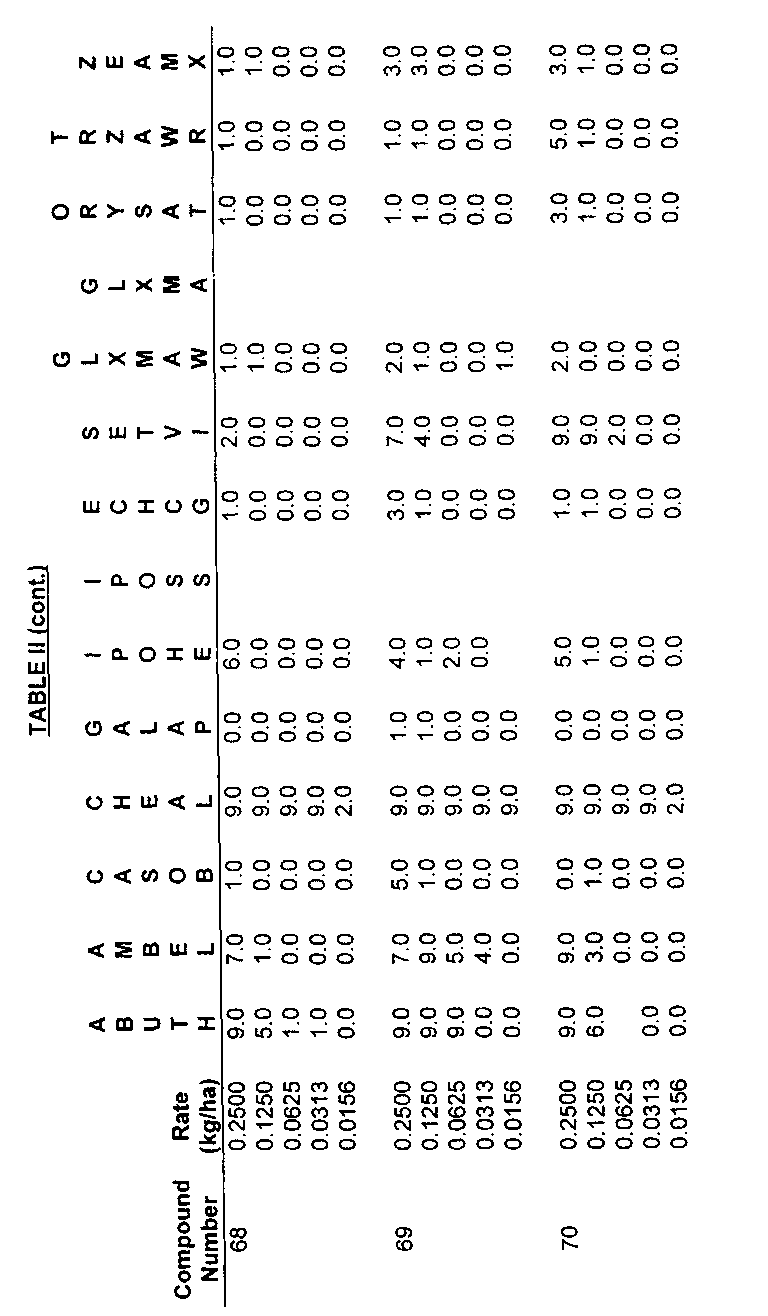

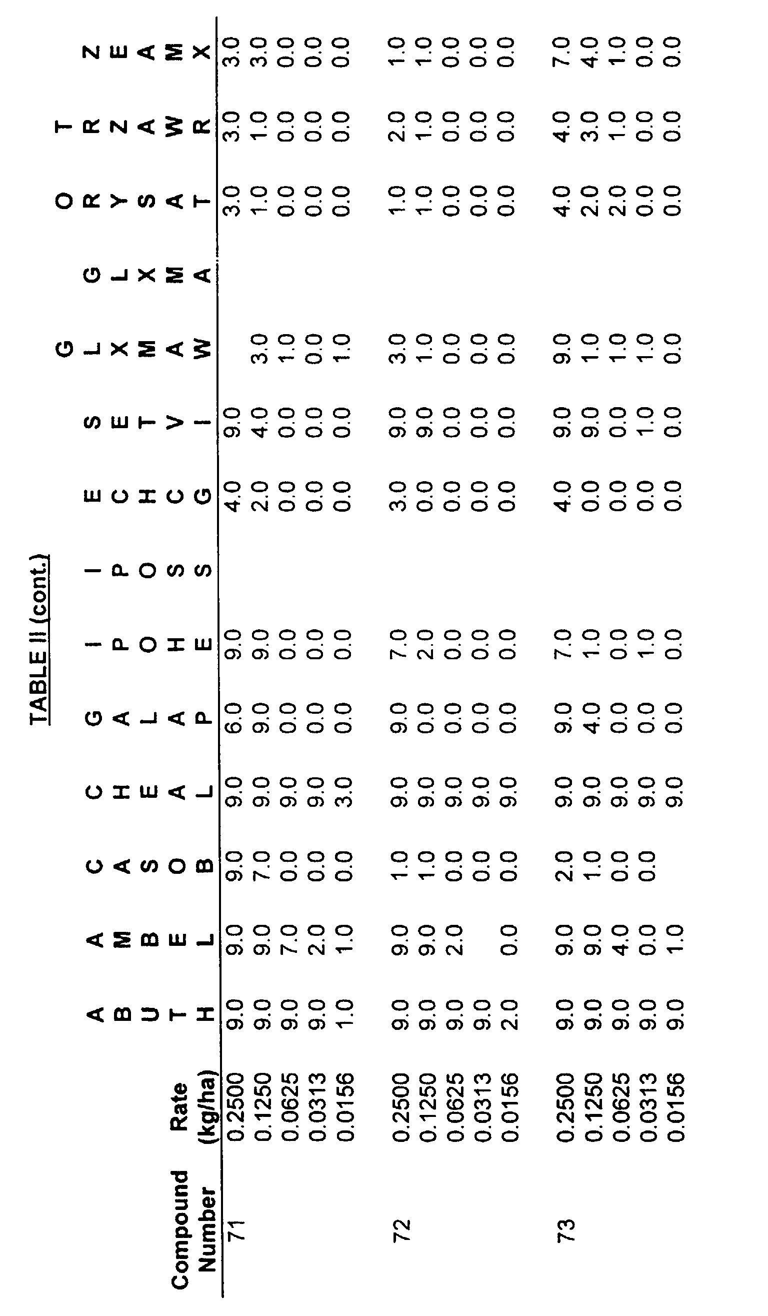

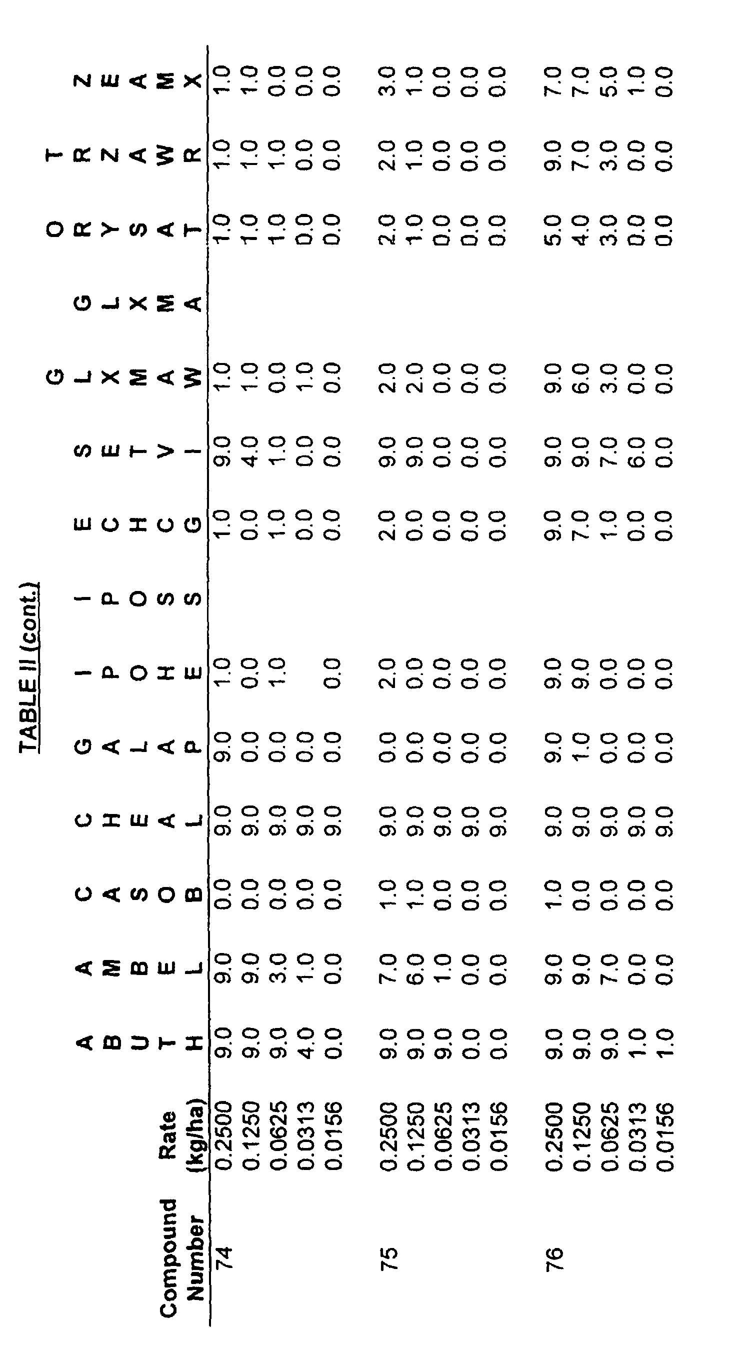

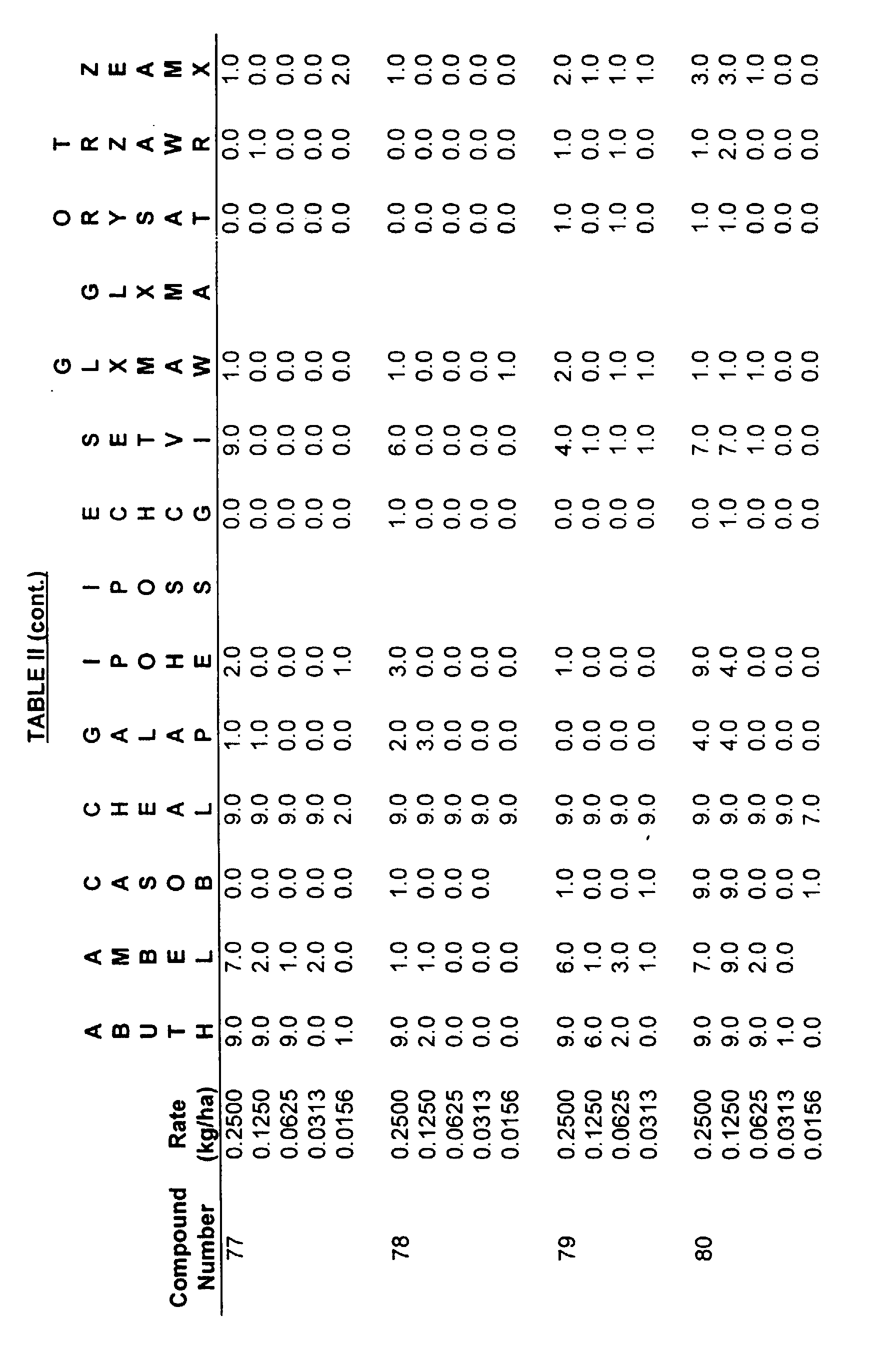

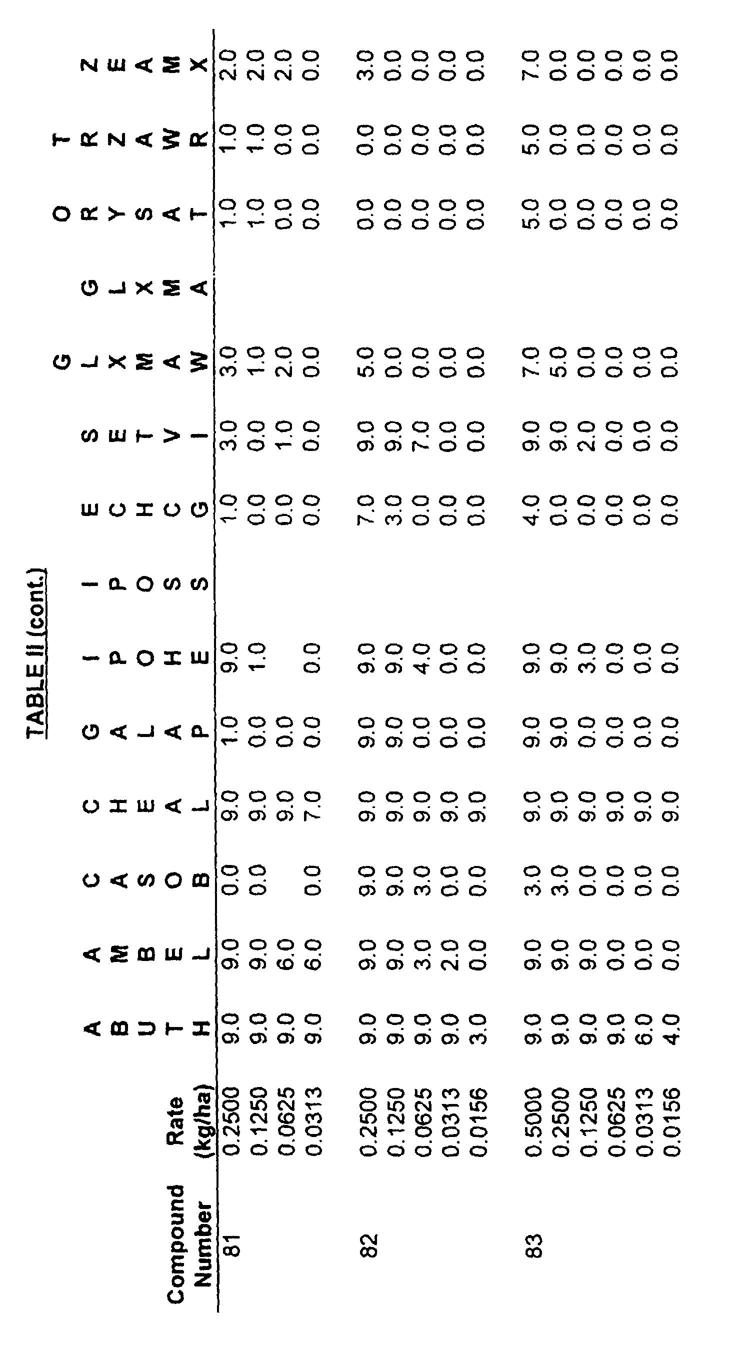

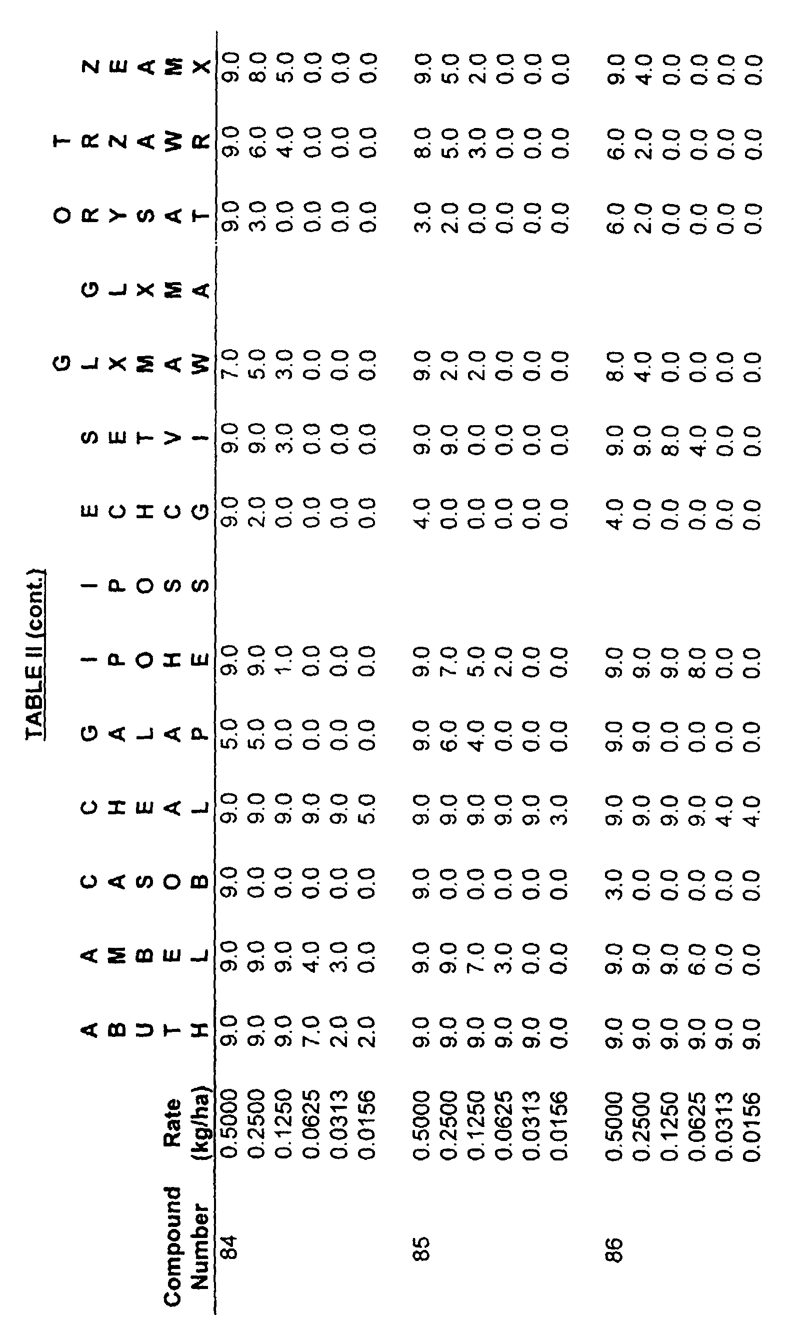

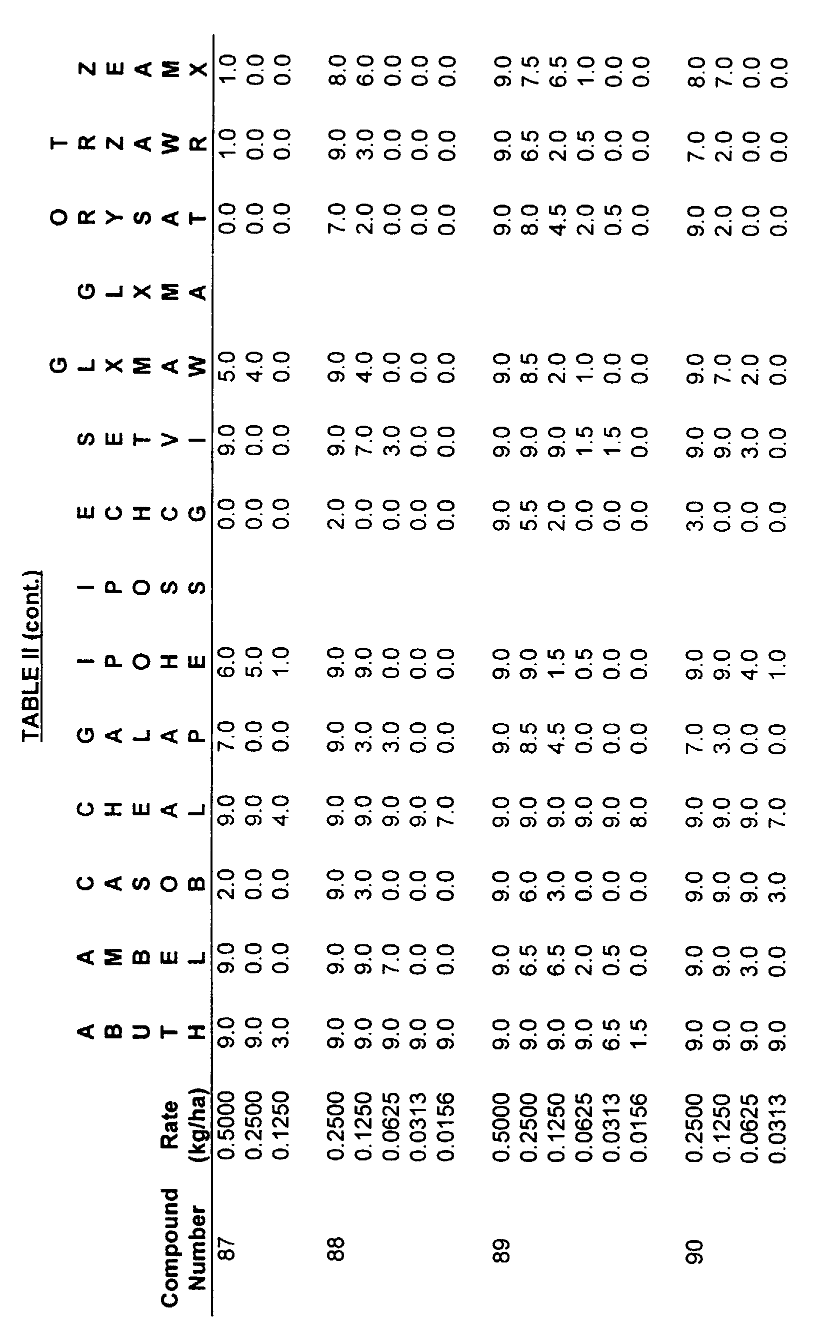

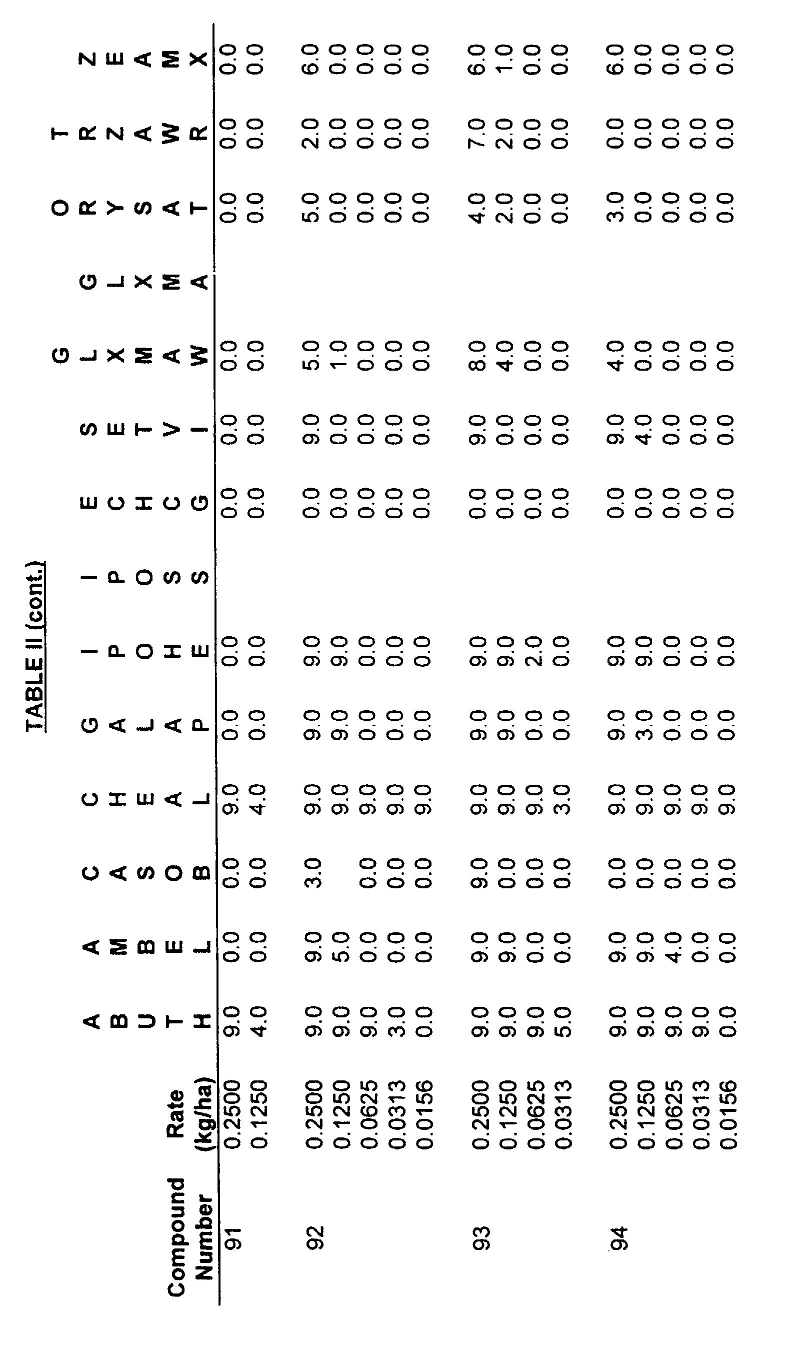

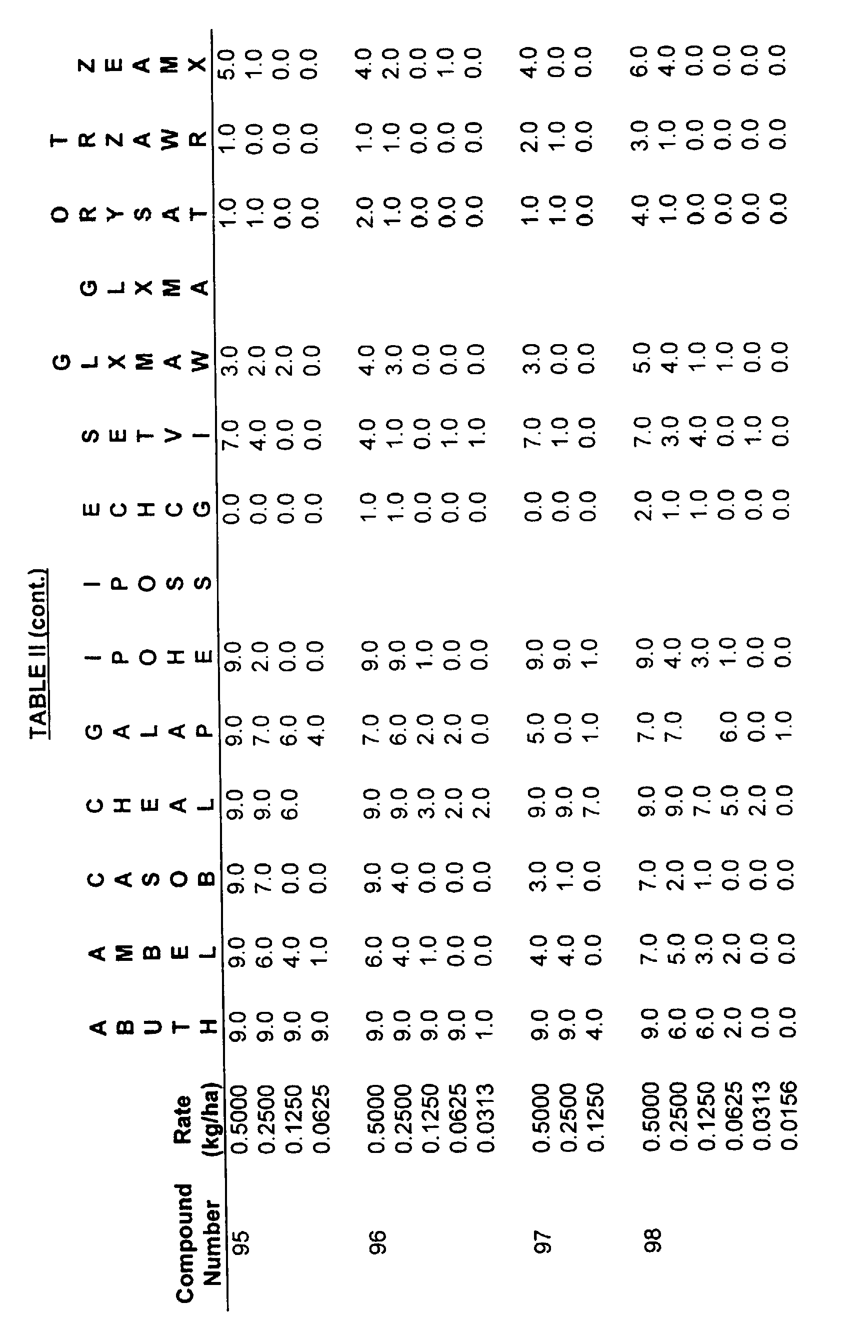

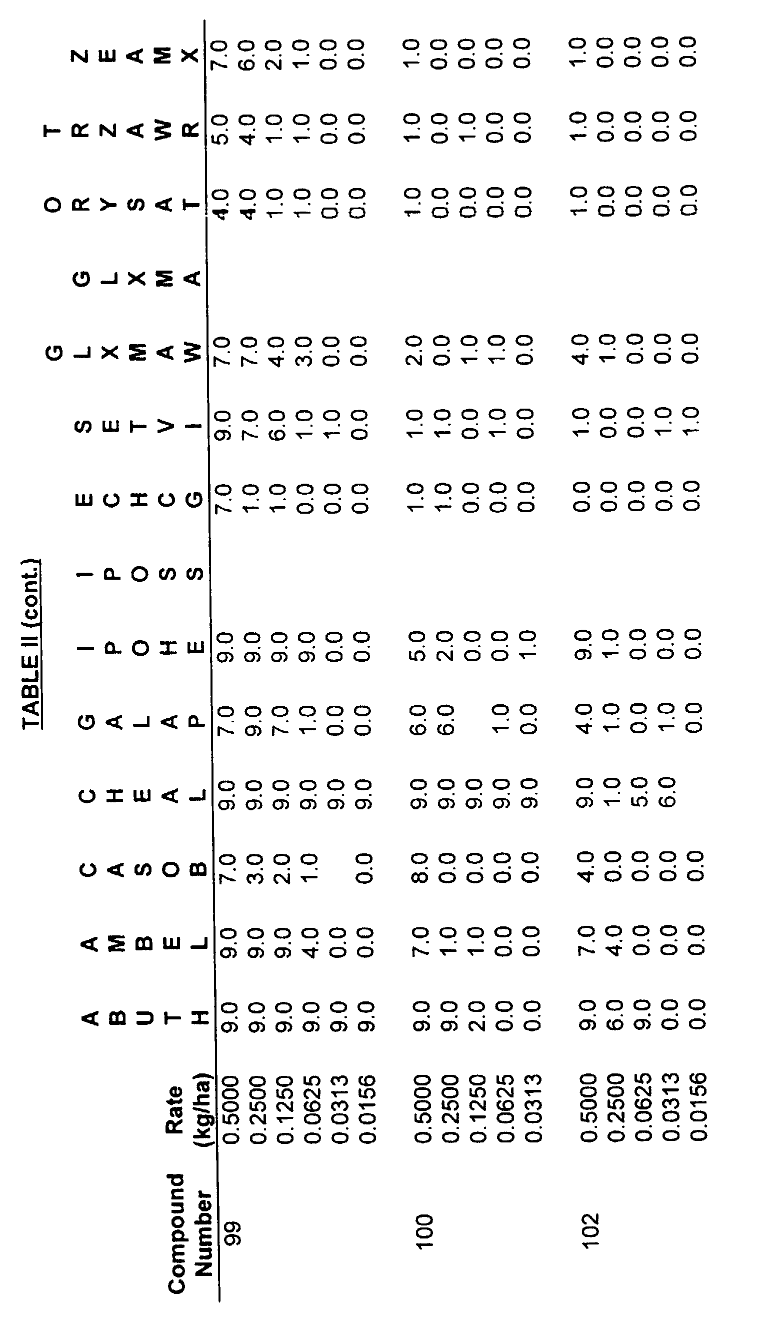

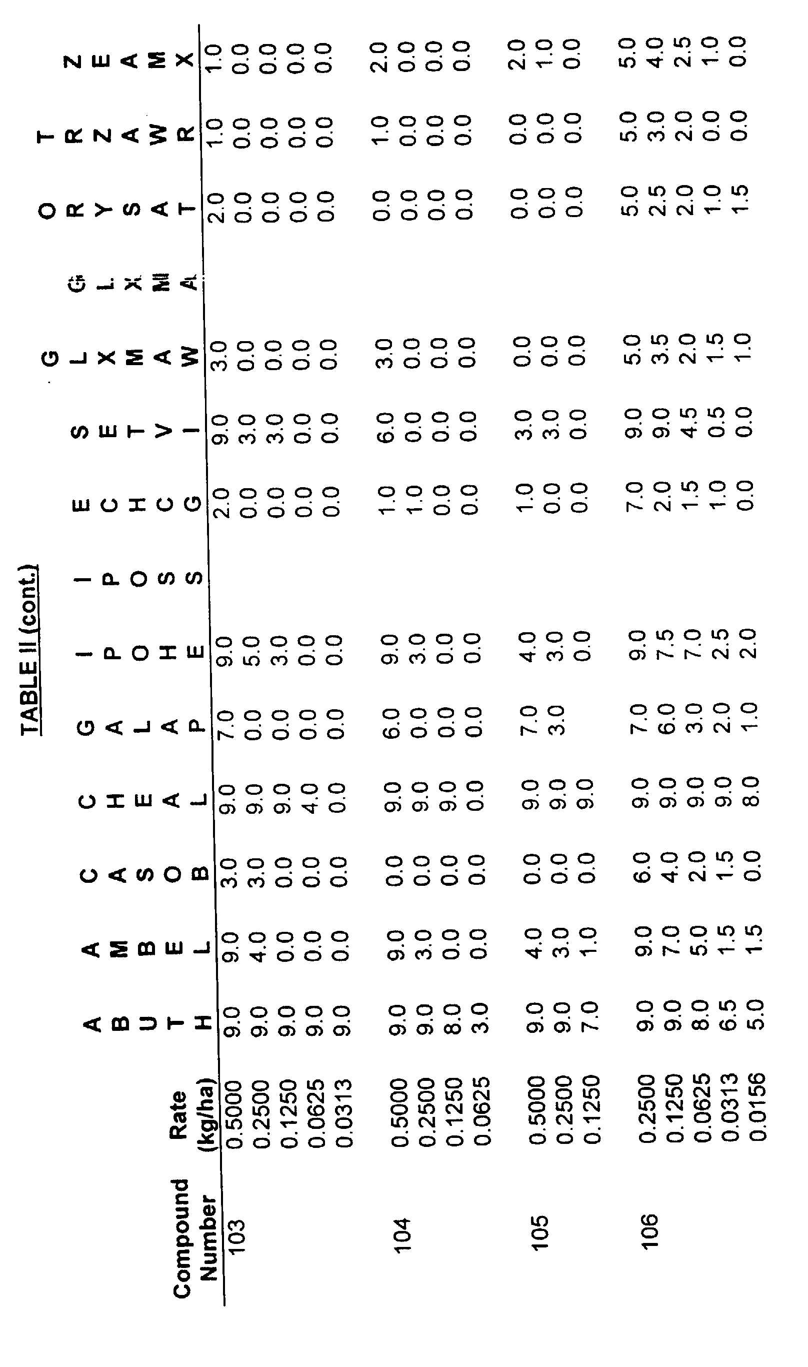

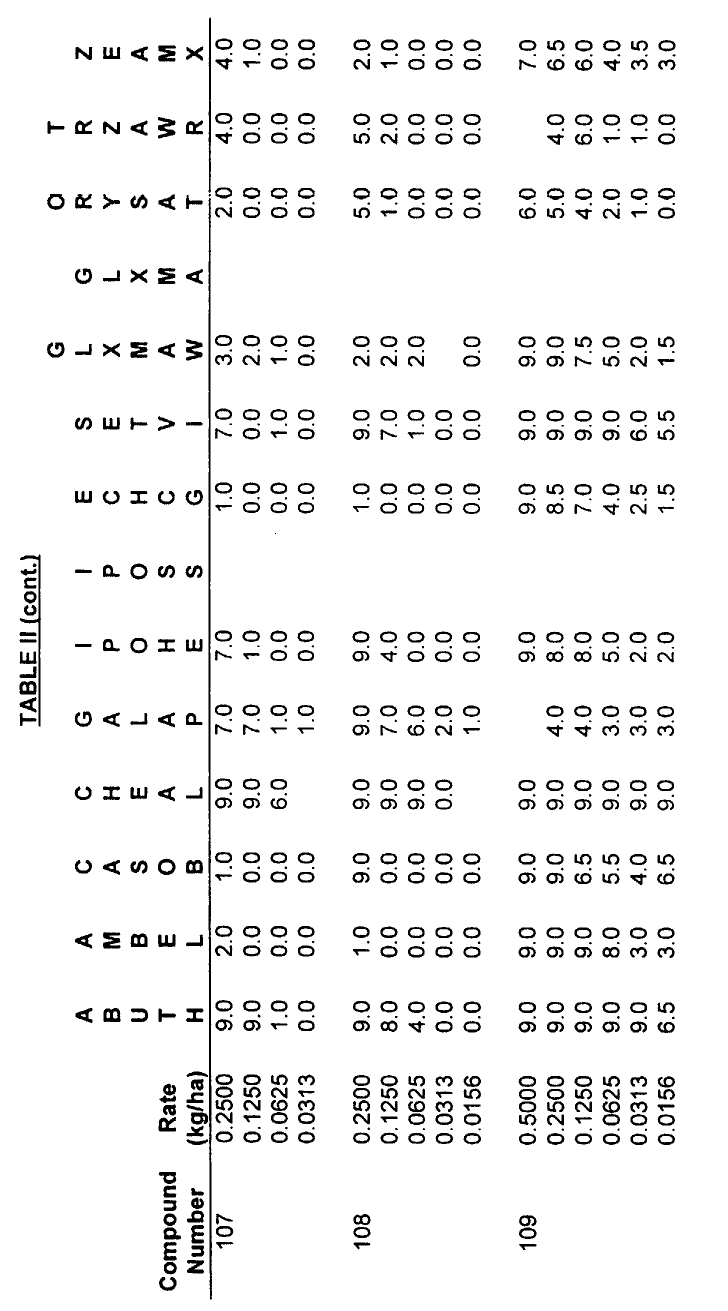

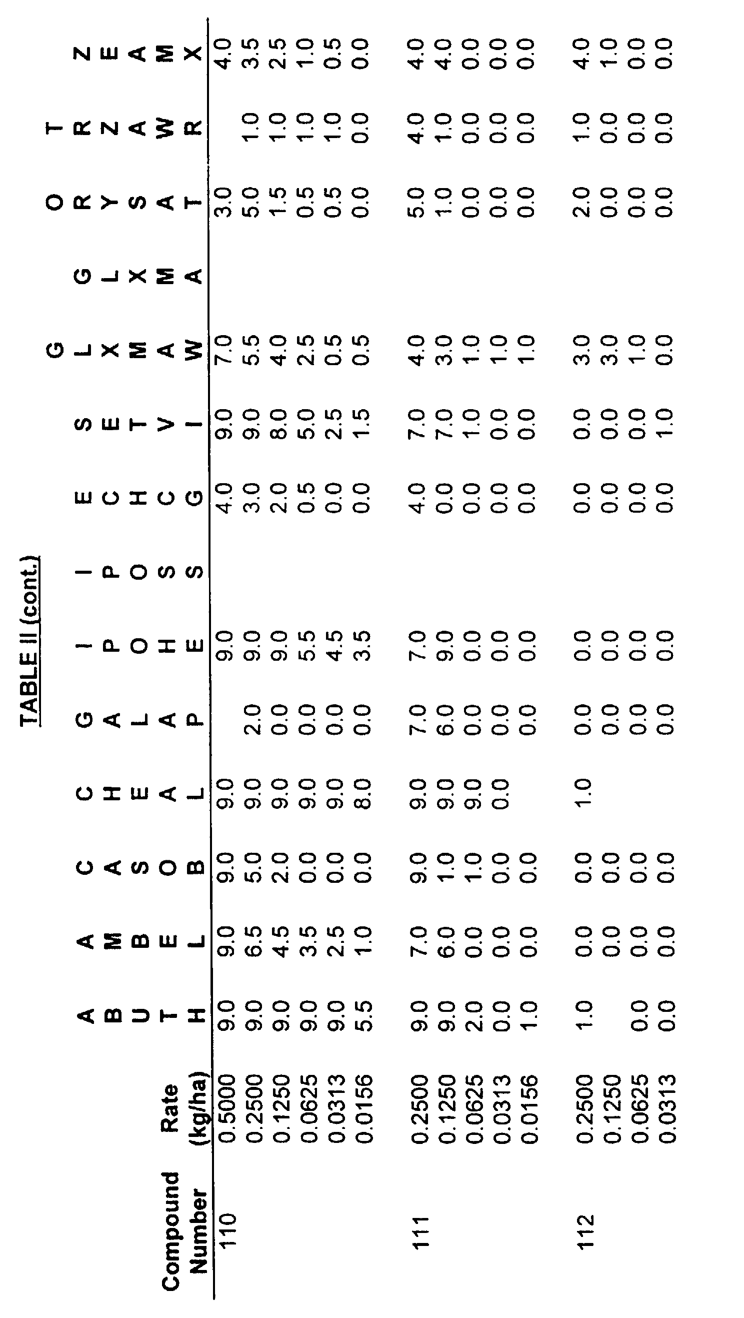

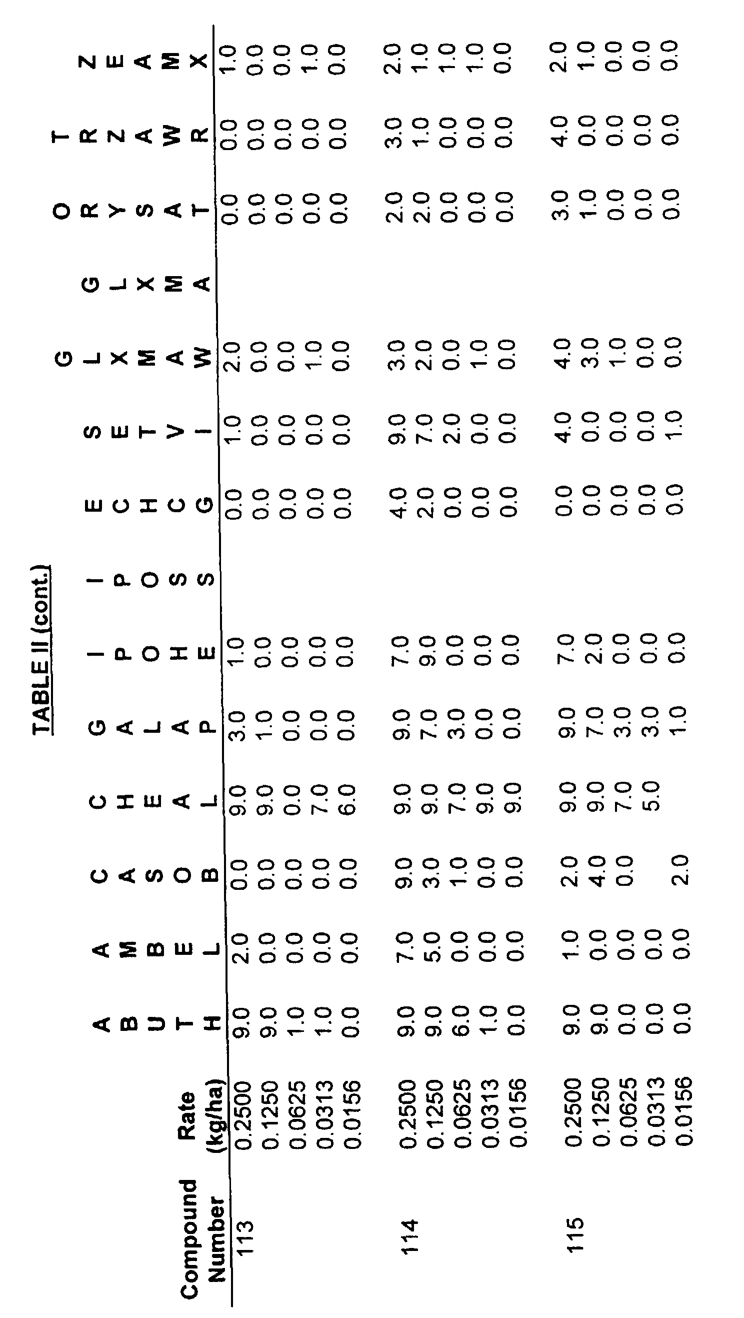

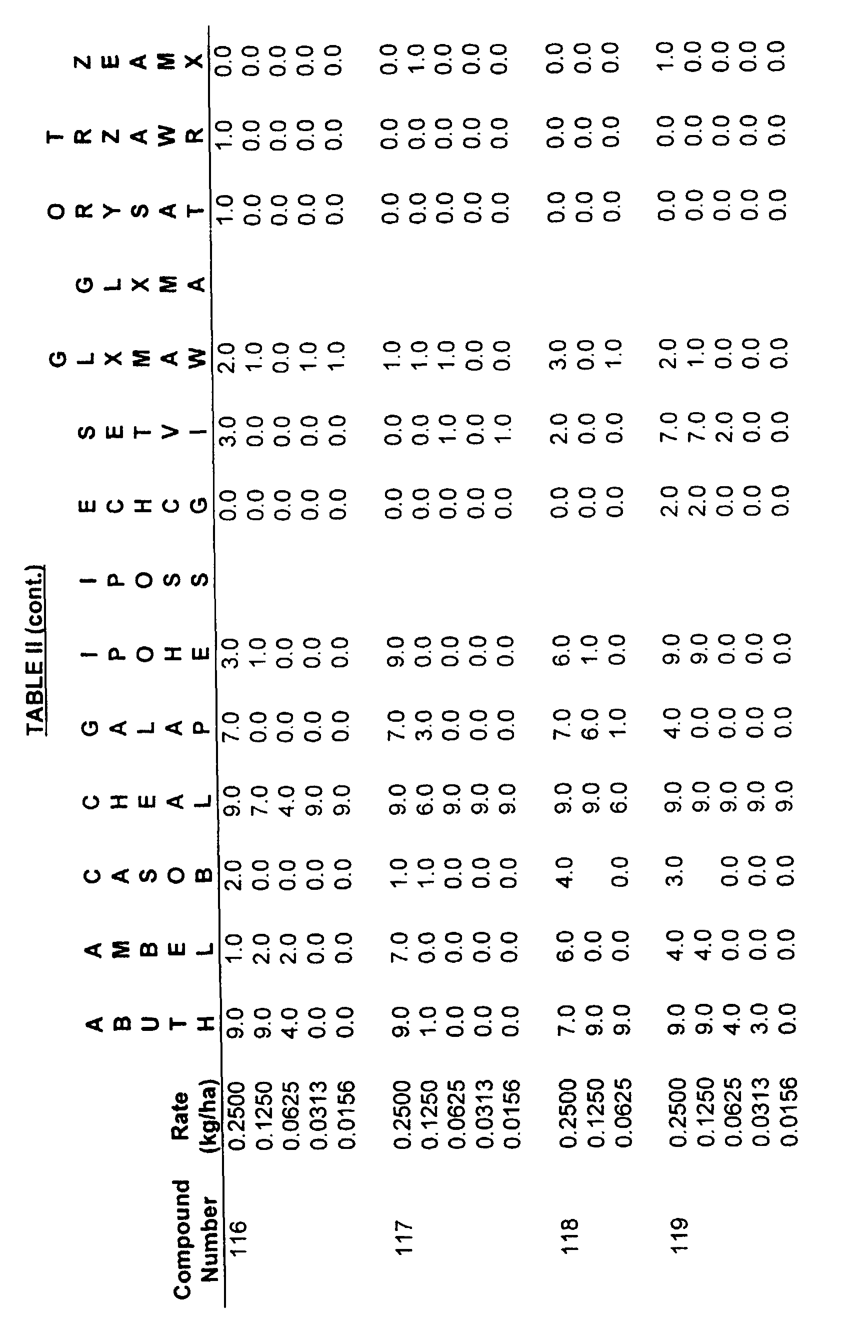

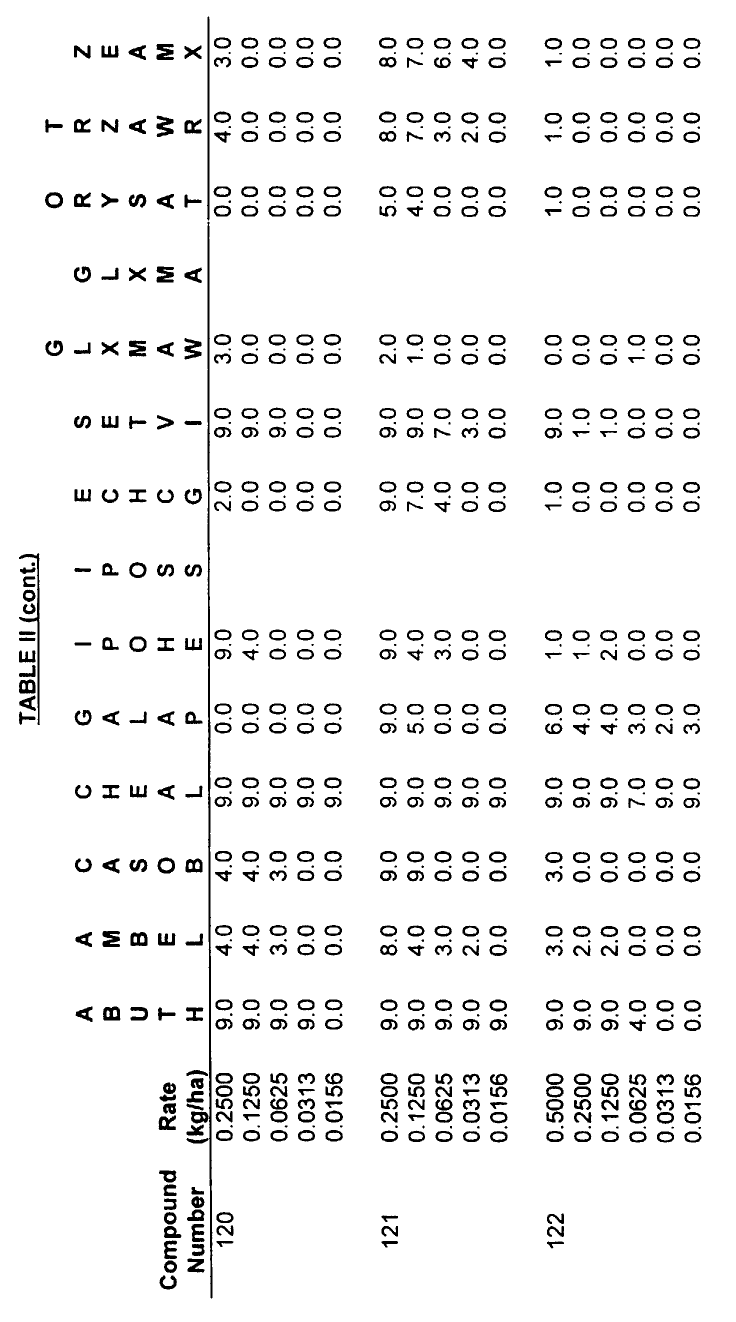

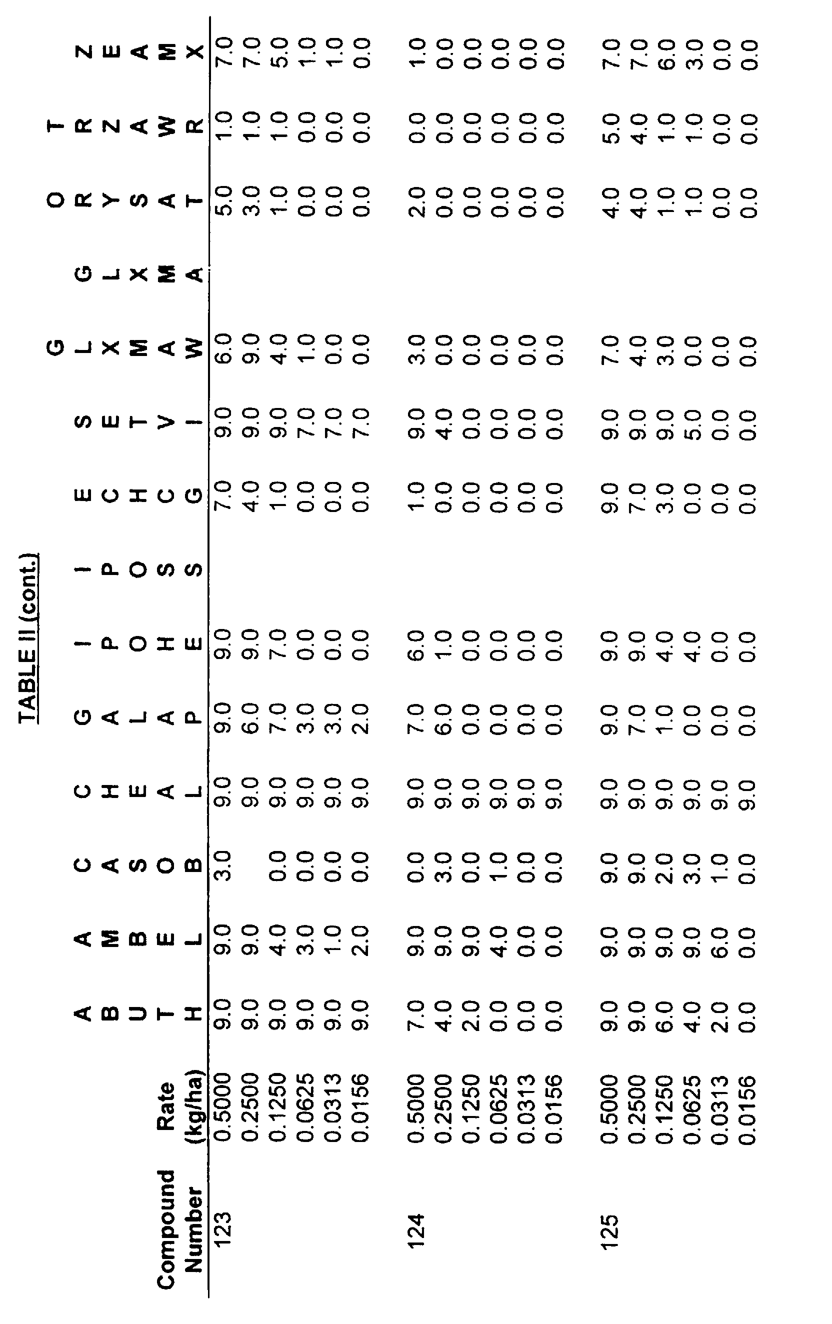

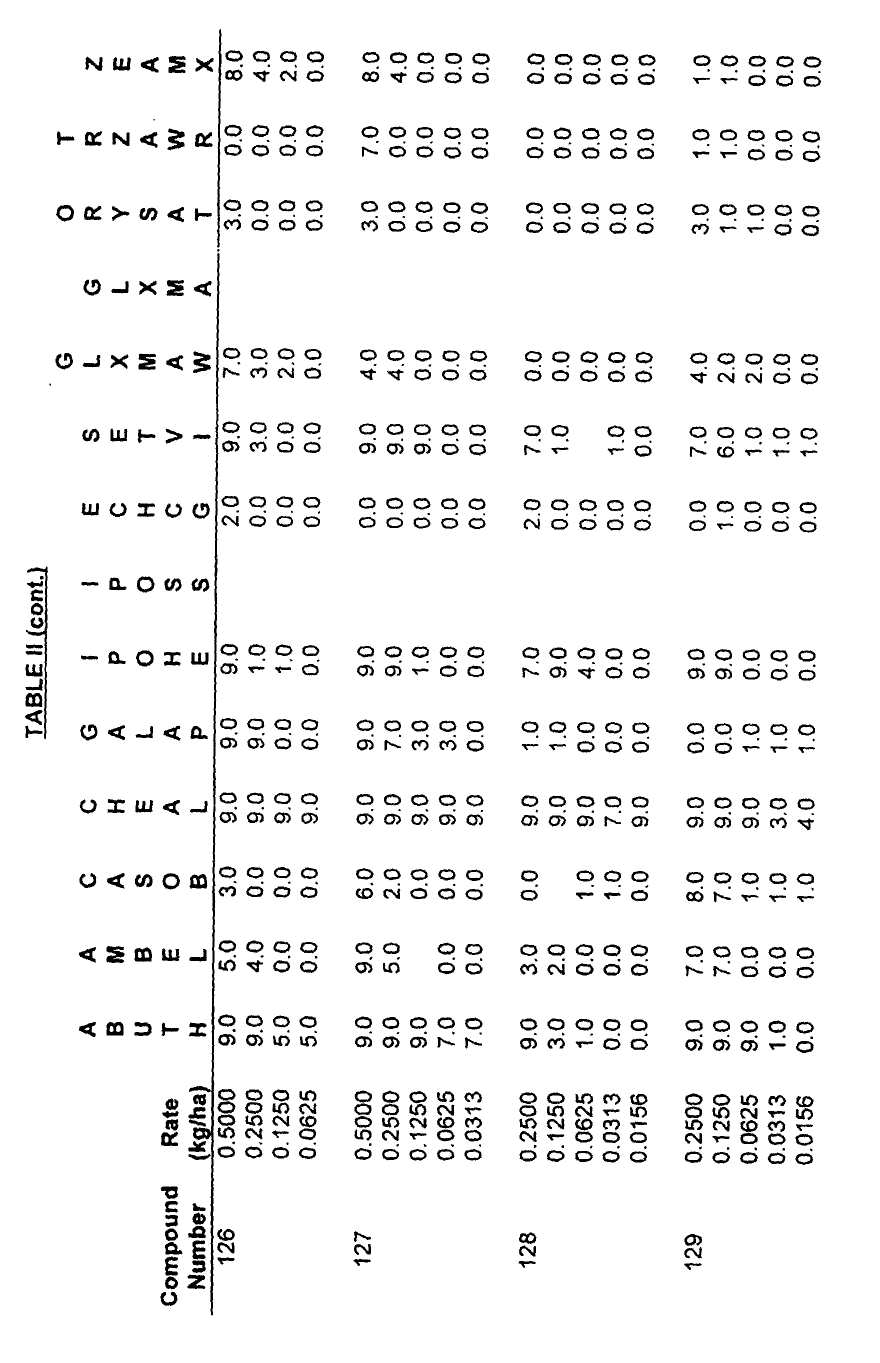

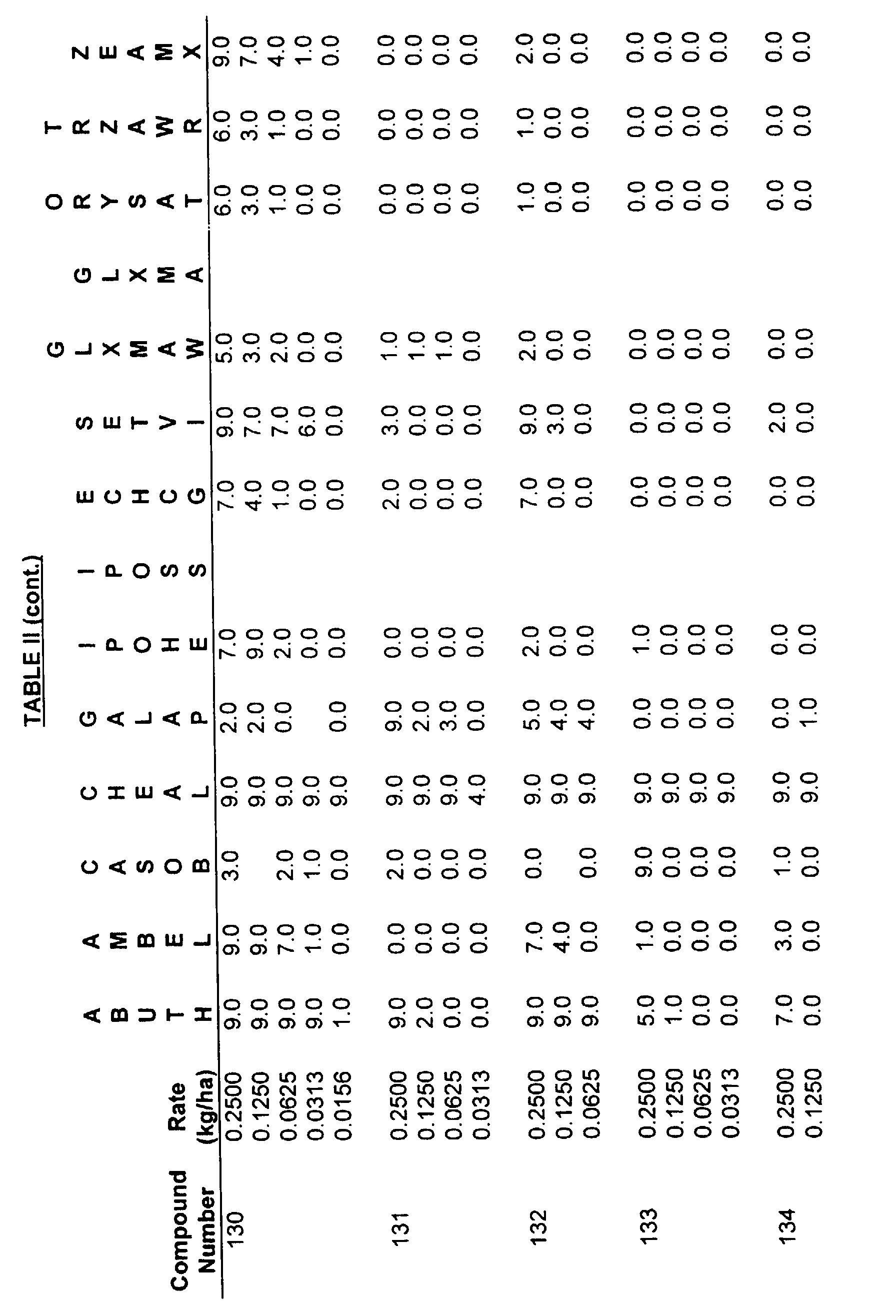

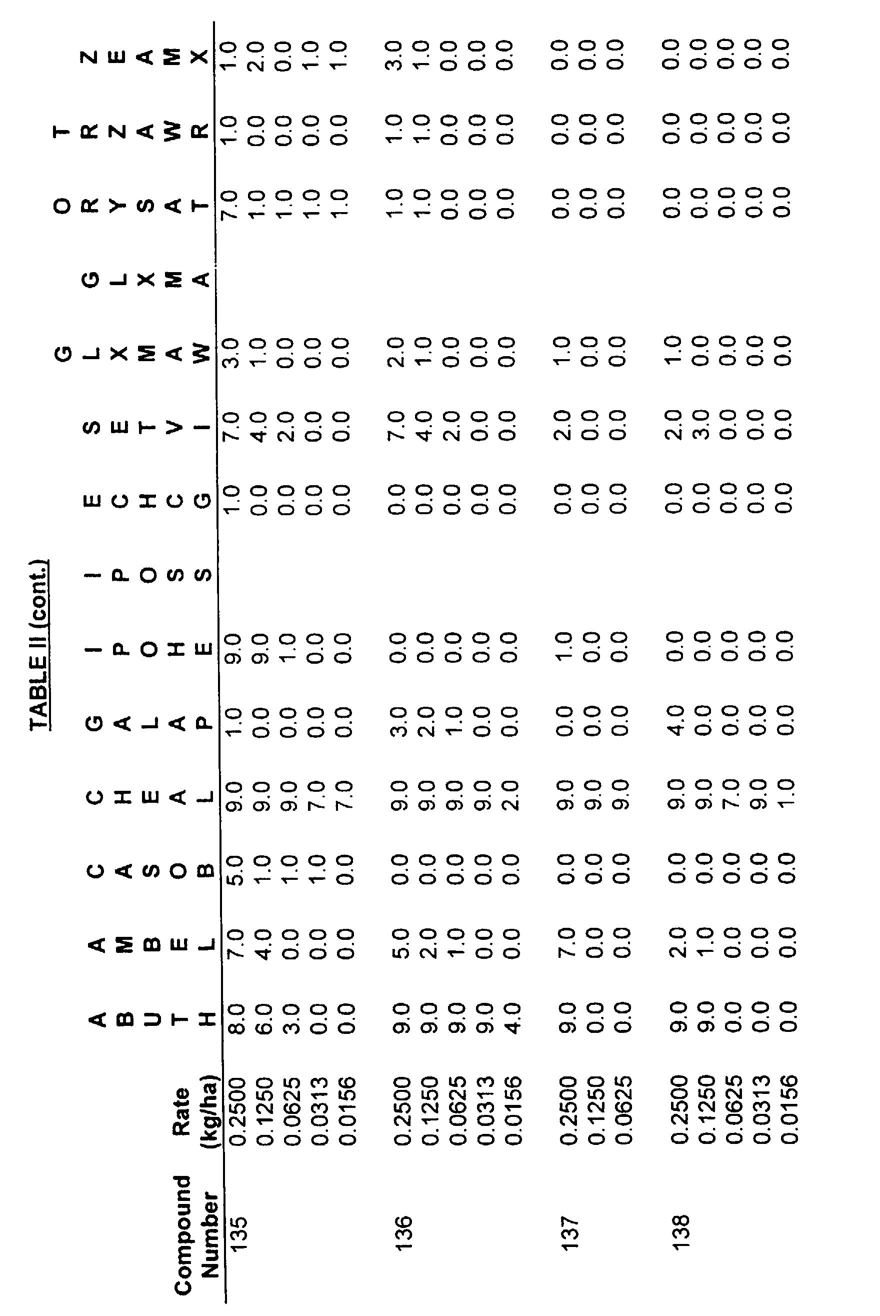

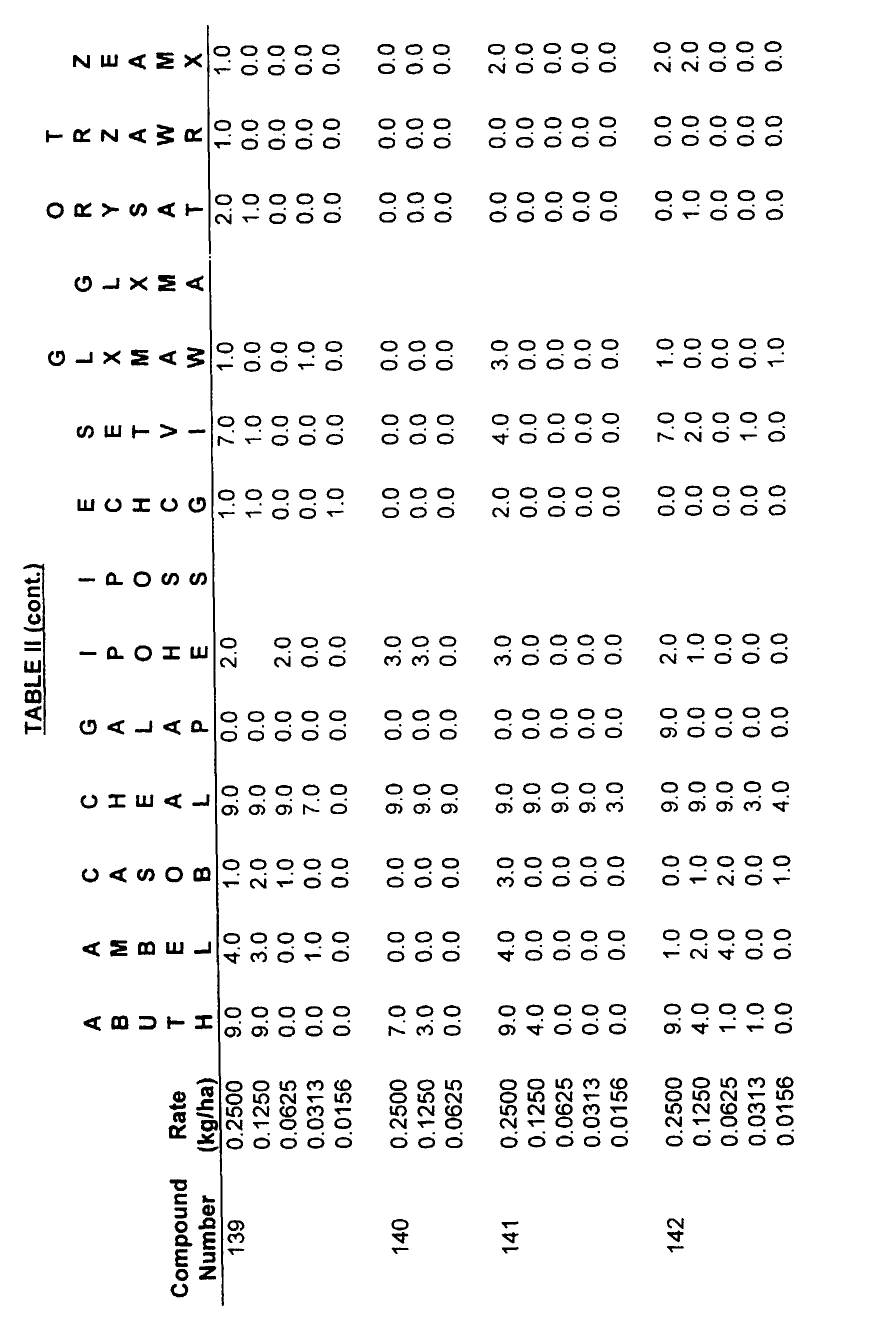

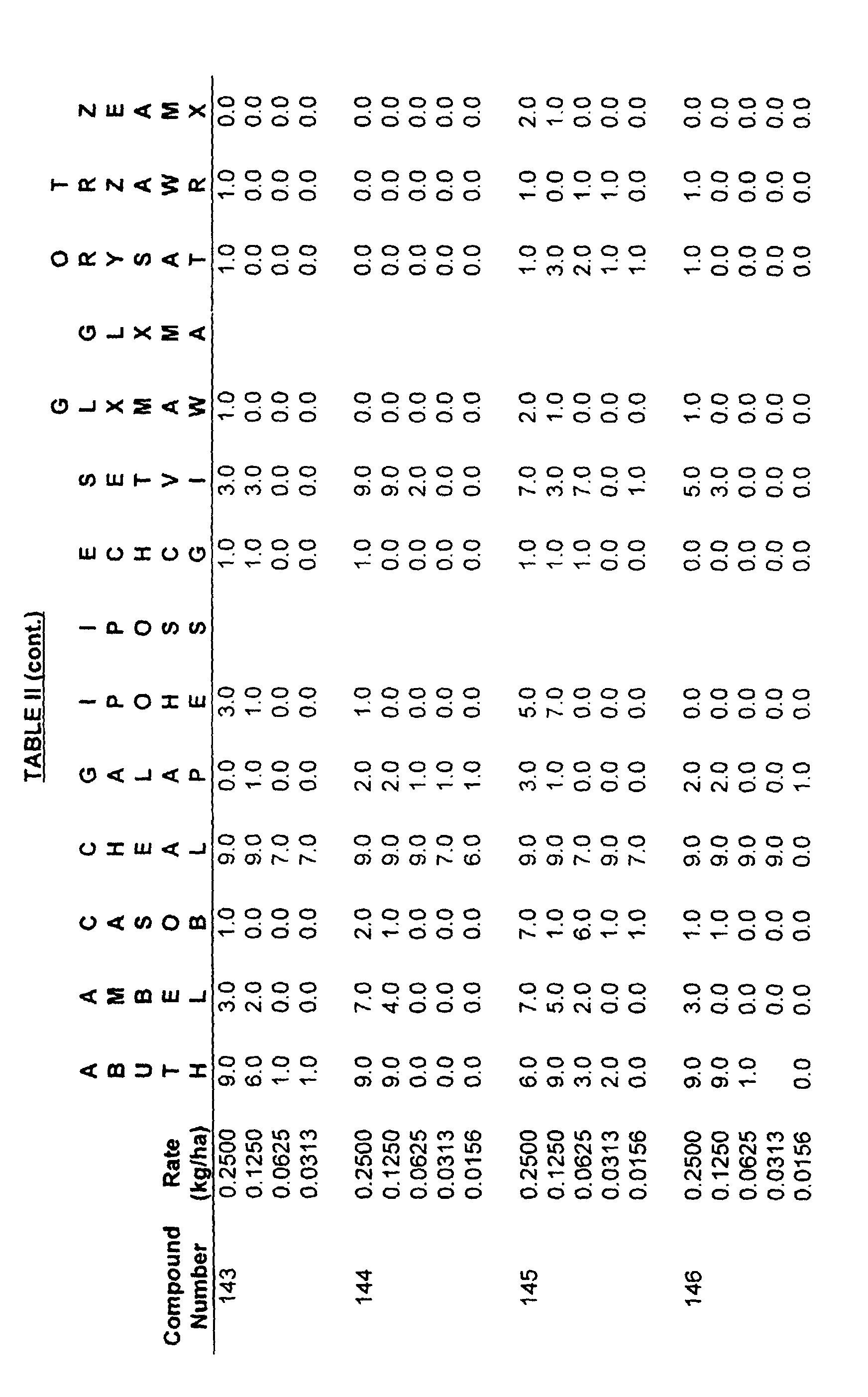

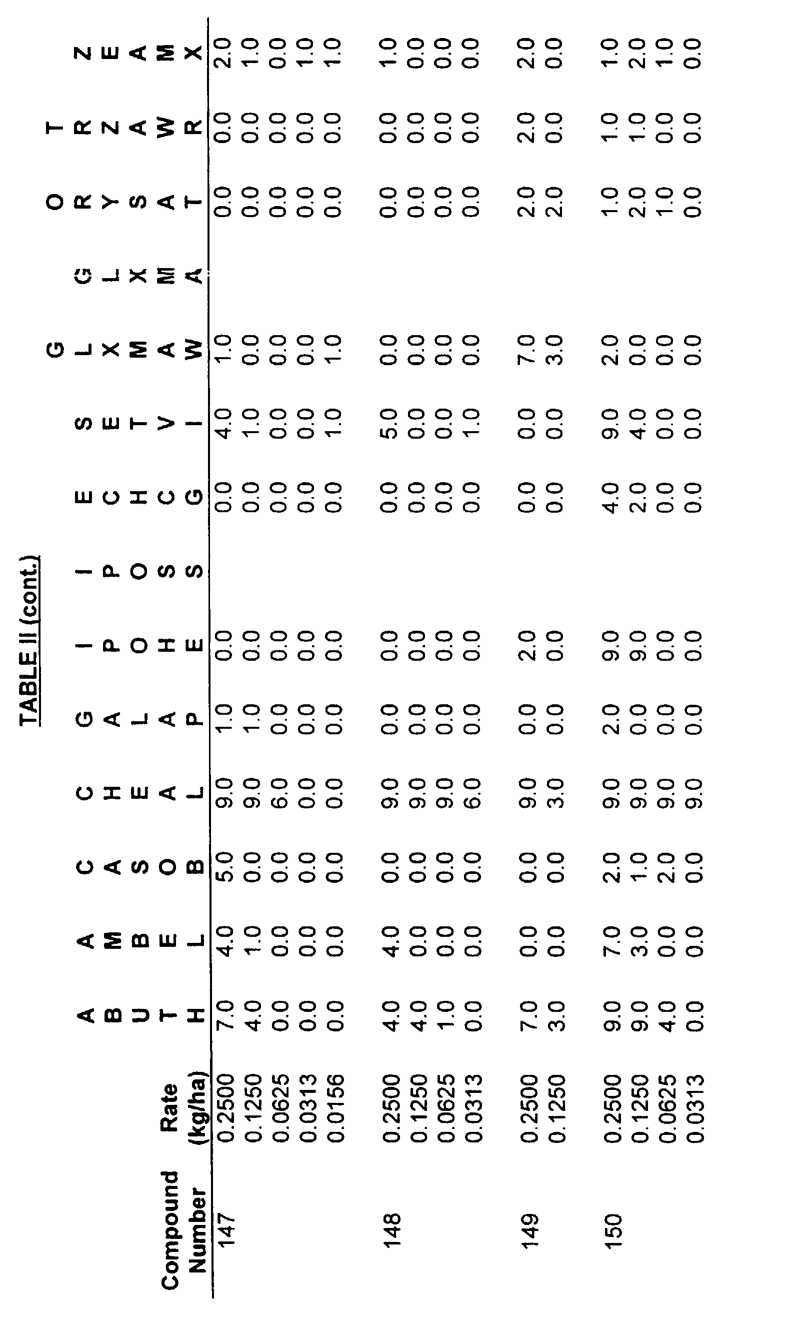

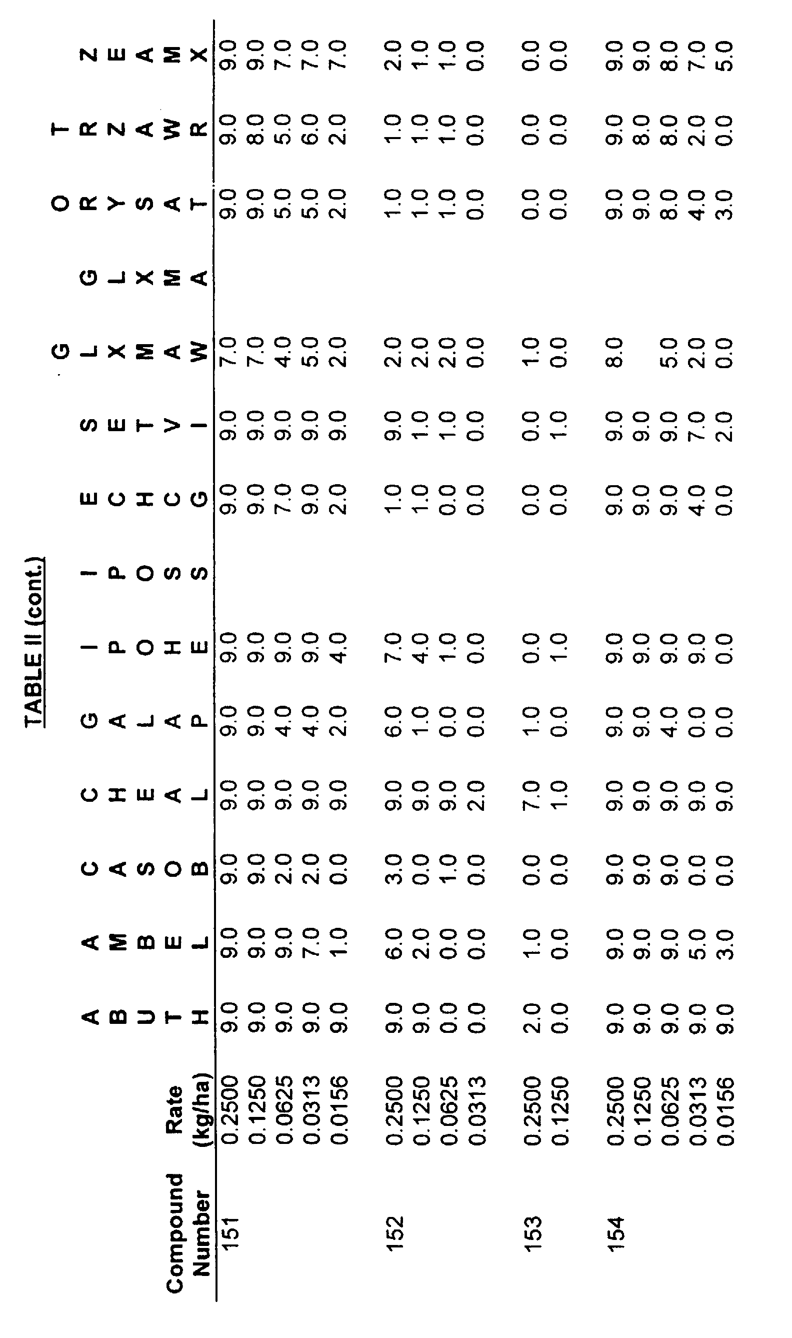

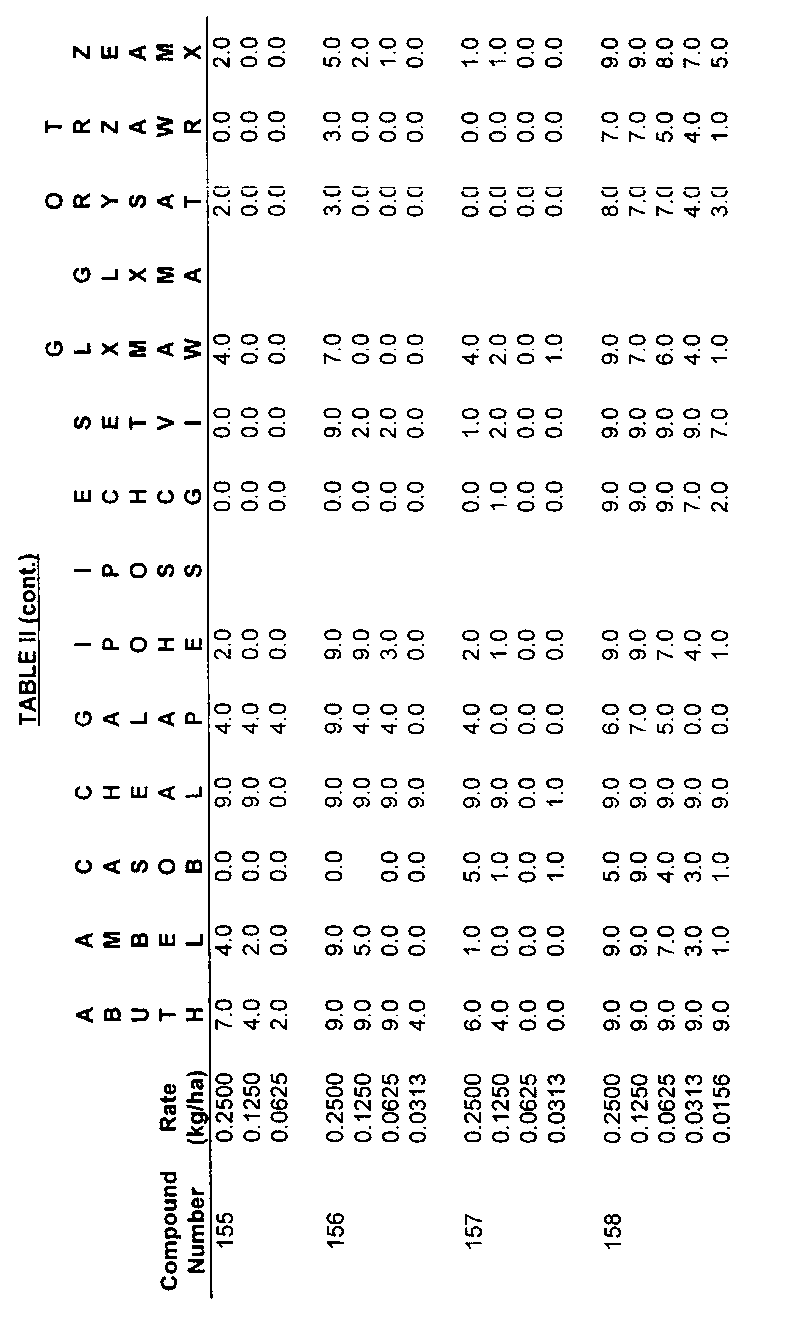

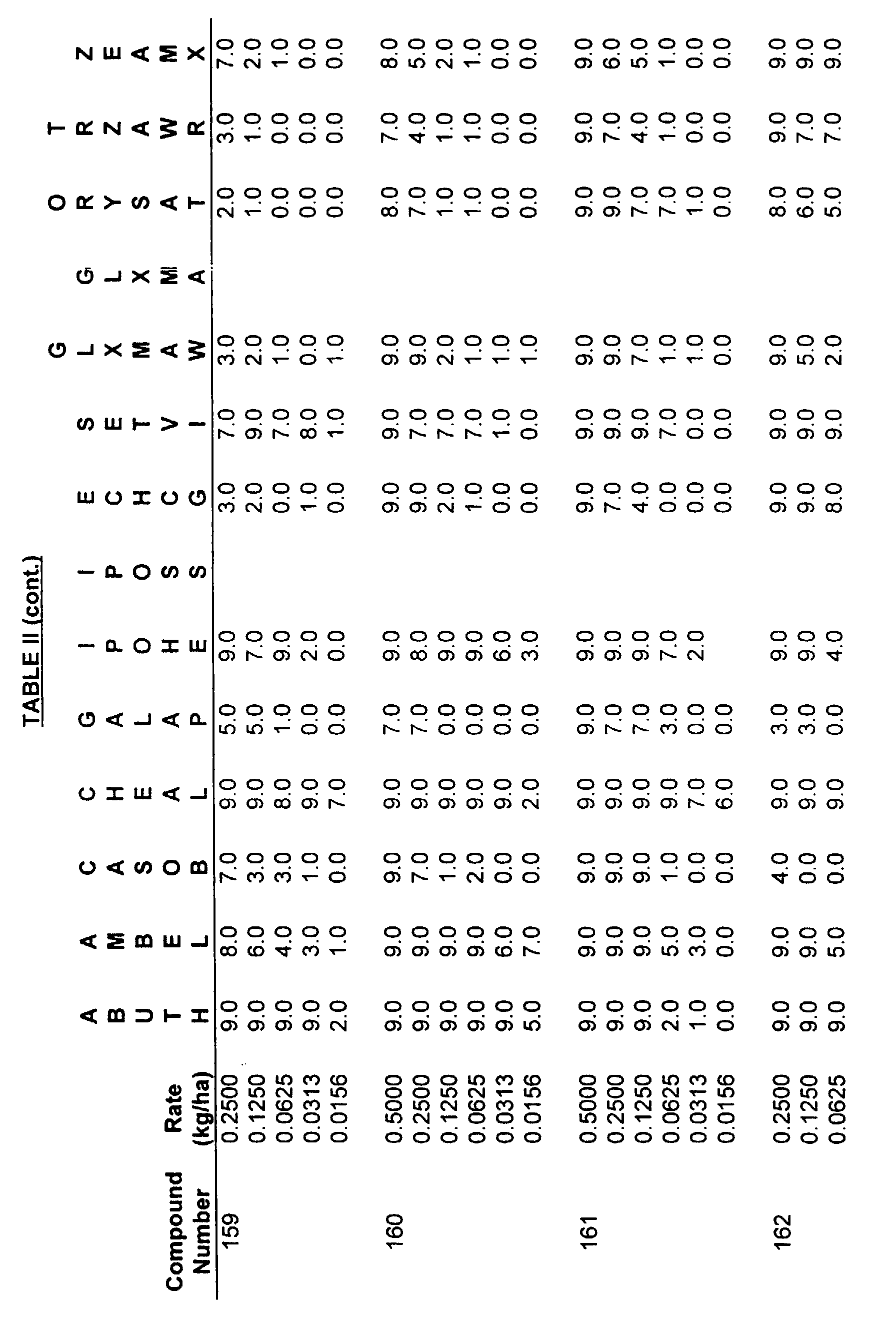

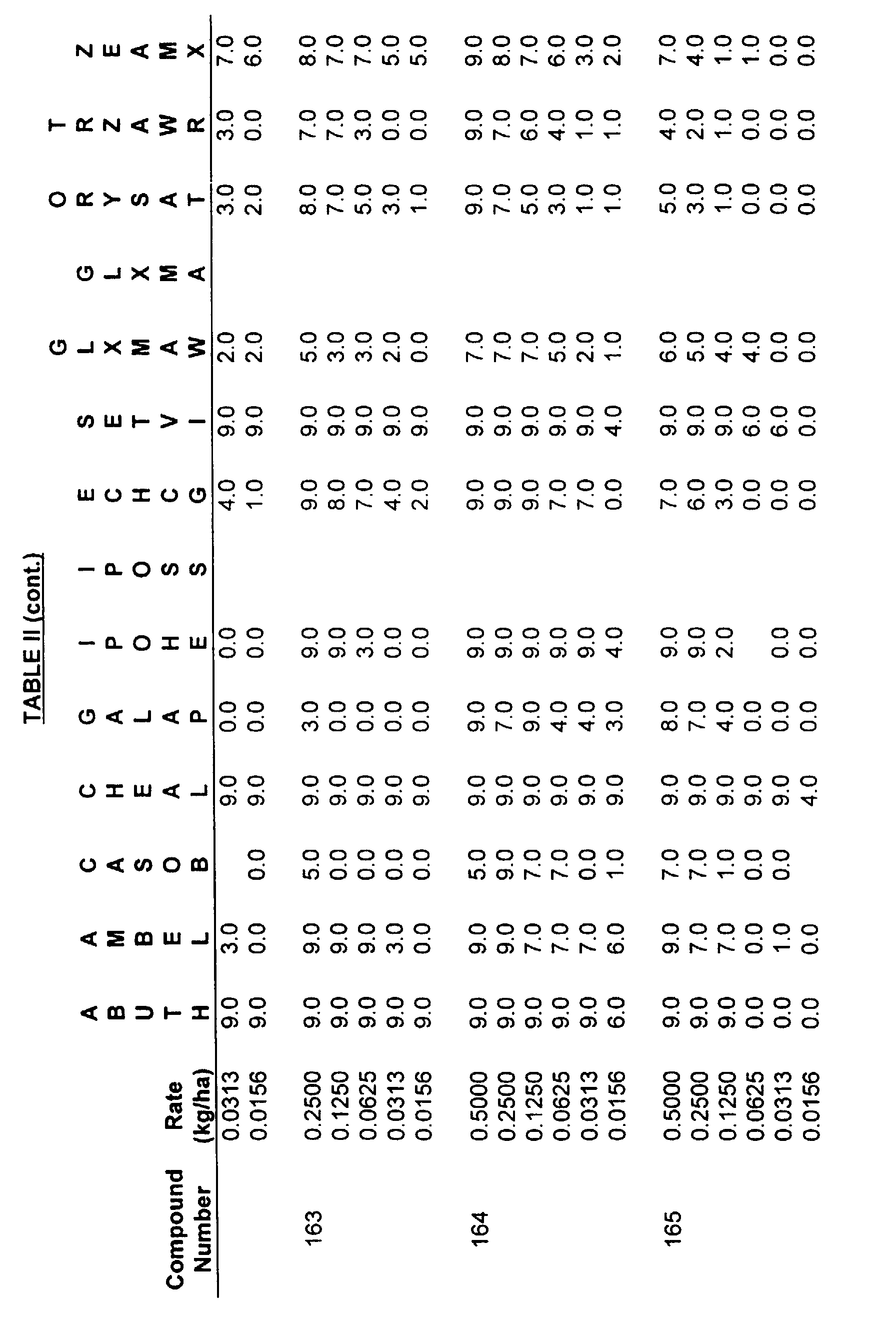

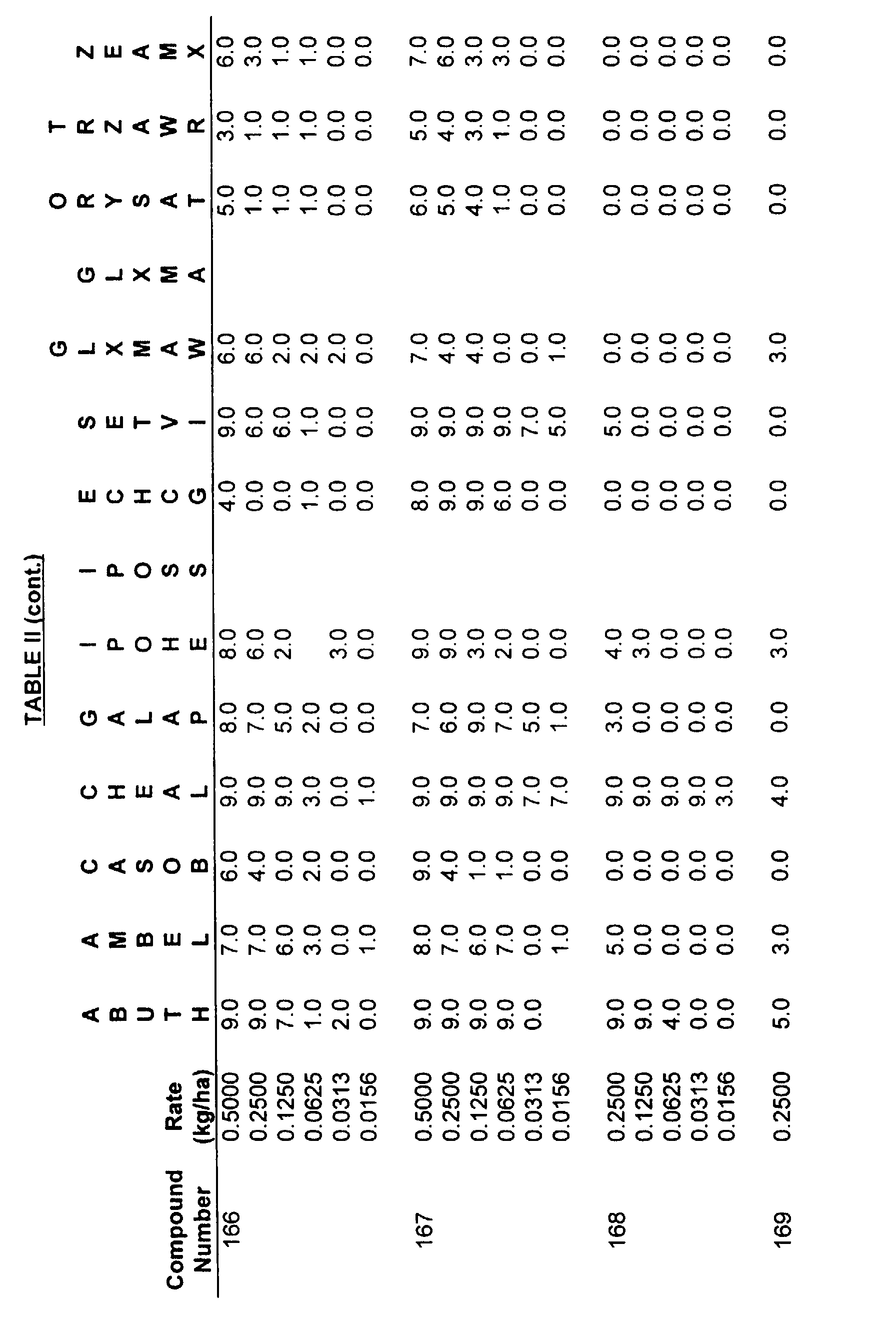

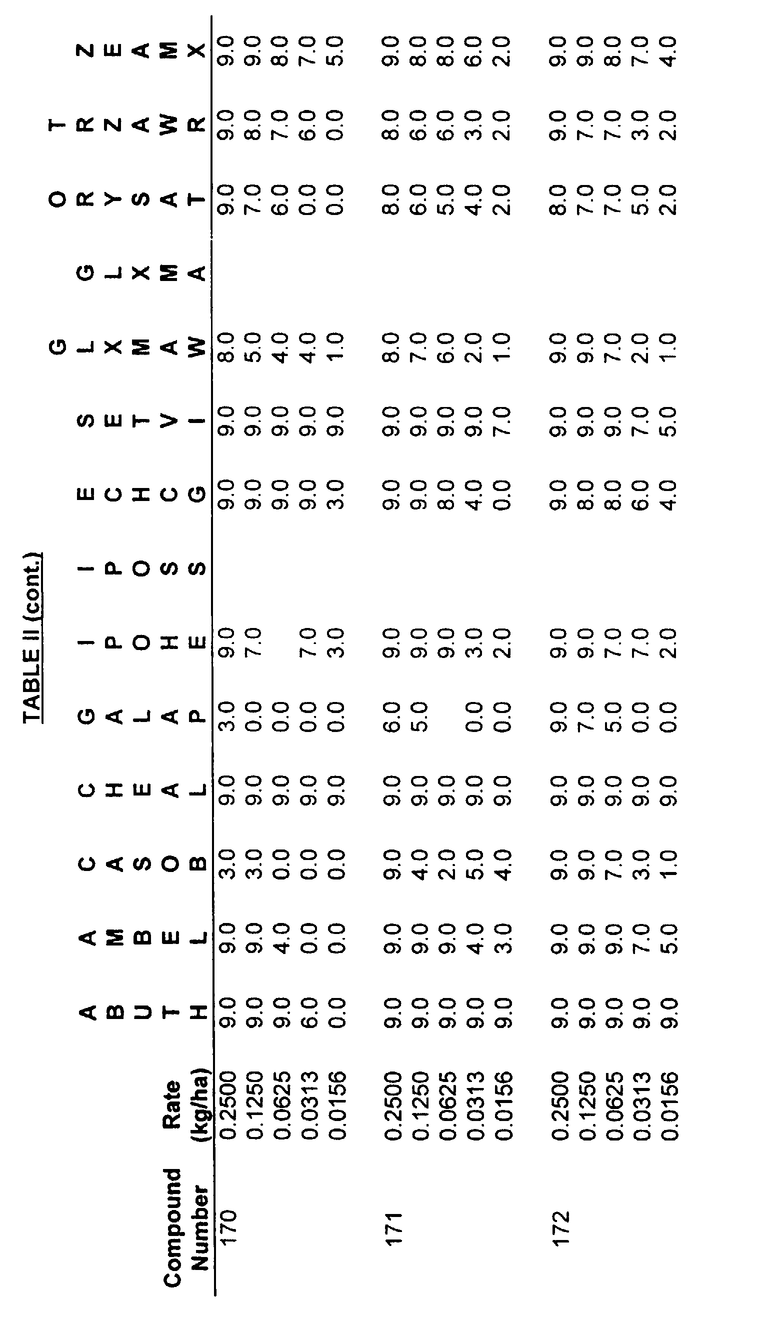

### EXAMPLE 39

### Rice tolerance to post-transplant applications and preemergence weed control under flooded paddy conditions

The tolerance of transplanted rice to post-transplanted herbicide applications is determined as follows: two ten-day-old rice seedlings (cv. Tebonnet) are transplanted into silt loam soil in 32 oz. plastic containers with a diameter of 10.5 cm and no drainage holes. After transplanting, the containers are flooded and the water level is maintained at 1.5 to 3 cm above the soil surface. Three days after transplanting, the flooded soil surface of the containers is treated with the selected aqueous/acetone 50/50 v/v mixture containing the test compounds to provide the equivalent of about 0.0313 to 0.500 kg/ha of active ingredient. The treated containers are placed on greenhouse benches, watered such that the water level is maintained as stated above, and cared for in accordance with conventional greenhouse procedures. Three to four weeks after treatment, the test is terminated and each container is examined and herbicidal effect rated according to the rating system provided in Example 37. The data obtained are reported in Table III. The compounds evaluated are reported by compound number given in Example 37.

Preemergence herbicidal activity under flooded paddy conditions is determined as follows: plant seeds or propagating organs are planted in the top 0.5 cm of silt loam soil in 32 oz. plastic containers with a diameter of 10.5 cm and no drainage holes. Water is added to these containers and maintained at 1.5 to 3 cm above the soil surface for the duration of the experiment. The test compounds are applied as aqueous/acetone mixtures 50/50 v/v pipetted directly into the flood water to give the equivalent of about 0.0313 to 0.500 kg/ha of active ingredient. The treated containers are placed on greenhouse benches and cared for in accordance with conventional greenhouse procedures. Three to four weeks after treatment, the test is terminated and each container is examined and herbicidal effect rated according to the rating system provided in Example 37. The data obtained are reported in Table III. The compounds evaluated are reported by compound number given in Example 37.

Plant species employed in this example are reported by header abbreviation, common name and scientific name.

| **PLANT SPECIES EMPLOYED IN RICE TOLERANCE/PREEMERGENCE** **WEED CONTROL EVALUATIONS** | | |
|---|---|---|
| **Header Abbr.** | **Common Name** | **Scientific Name** |
| ECHORC | Watergrass (Calif.) | *Echinochloa oryzoides* (Ard.) Fritsch. |
| CYPIR | Rice Flatsedge | *Cyperus iria* |
| CYPSE | Flatsedge | *Cyperus serotinus,* Rottb. |
| MOOVA | Monochoria | *Monochoria vaginalis,* Presl. |
| SAGPY | Arrowhead (Pygmaea) | *Sagittaria pygmaea,* L. |
| ORYSAT | Rice, Tebonnet | *Oryza sativa,* (L.) Tebonnet |

**TABLE III**

| **Paddy Conditions - Post-Transplant Rice** **Preemergence Weeds** | | | | | | | |
|---|---|---|---|---|---|---|---|
| **Compound** **Number** | **Rate** **(kg/ha)** | **E** **C** **H** **O** **R** **C** | **C** **Y** **P** **I** **R** | **C** **Y** **P** **S** **E** | **M** **O** **O** **V** **A** | **S** **A** **G** **P** **Y** | **O** **R** **Y** **S** **A** **T** |
| 1 | 0.5000 | 9.0 | 9.0 | 8.0 | 9.0 | 0.0 | 4.0 |
| | 0.2500 | 7.0 | 9.0 | 2.0 | 9.0 | 0.0 | 3.0 |
| | 0.1250 | 6.0 | 9.0 | 0.0 | 9.0 | 0.0 | 2.0 |
| | 0.0625 | 4.0 | 7.0 | 0.0 | 9.0 | 0.0 | 2.0 |
| | 0.0313 | 2.0 | 2.0 | 0.0 | 8.0 | 0.0 | 1.0 |
| | | | | | | | |
| 2 | 0.5000 | 9.0 | 1.0 | 0.0 | 9.0 | 0.0 | 3.0 |
| | 0.2500 | 8.0 | 0.0 | 0.0 | 9.0 | 0.0 | 2.0 |
| | 0.1250 | 7.0 | 0.0 | 0.0 | 9.0 | 0.0 | 1.0 |
| | 0.0625 | 2.0 | 0.0 | 0.0 | 9.0 | 0.0 | 0.0 |
| | 0.0313 | 2.0 | 0.0 | 0.0 | 4.0 | 0.0 | 0.0 |
| | | | | | | | |
| 3 | 0.5000 | 4.0 | 0.0 | 0.0 | 9.0 | 0.0 | 3.0 |
| | 0.2500 | 2.0 | 0.0 | 0.0 | 9.0 | 0.0 | 2.0 |
| | 0.1250 | 0.0 | 0.0 | 0.0 | 9.0 | 0.0 | 2.0 |
| | 0.0625 | 0.0 | 0.0 | 0.0 | 2.0 | 0.0 | 0.0 |
| | | | | | | | |
| 4 | 0.5000 | 9.0 | 9.0 | 0.0 | 9.0 | 0.0 | 3.0 |
| | 0.2500 | 8.0 | 9.0 | 0.0 | 9.0 | 0.0 | 3.0 |
| | 0.1250 | 7.0 | 8.0 | 0.0 | 8.0 | 0.0 | 2.0 |
| | 0.0625 | 4.0 | 4.0 | 0.0 | 7.0 | 0.0 | 2.0 |
| | 0.0313 | 2.0 | 0.0 | 0.0 | 2.0 | 0.0 | 0.0 |
| | | | | | | | |
| 5 | 0.5000 | 4.0 | 0.0 | 1.0 | 8.0 | 0.0 | 2.0 |
| | 0.2500 | 2.0 | 0.0 | 0.0 | 4.0 | 0.0 | 1.0 |
| | 0.1250 | 1.0 | 0.0 | 0.0 | 2.0 | 0.0 | 1.0 |
| | | | | | | | |
| 6 | 0.5000 | 7.0 | 7.0 | 0.0 | 9.0 | 0.0 | 0.0 |
| | 0.2500 | 7.0 | 6.0 | 0.0 | 9.0 | 0.0 | 0.0 |
| | 0.1250 | 2.0 | 4.0 | 0.0 | 8.0 | 0.0 | 0.0 |
| | 0.0625 | 0.0 | 2.0 | 0.0 | 8.0 | 0.0 | 0.0 |
| | 0.0313 | 0.0 | 0.0 | 0.0 | 2.0 | 0.0 | 0.0 |
| | | | | | | | |
| 7 | 0.2500 | 9.0 | 9.0 | 9.0 | 9.0 | 9.0 | 9.0 |
| | 0.1250 | 9.0 | 9.0 | 7.0 | 9.0 | 8.0 | 8.0 |
| | 0.0625 | 9.0 | 9.0 | 7.0 | 9.0 | 4.0 | 7.0 |
| | 0.0313 | 8.0 | 8.0 | 4.0 | 9.0 | 2.0 | 7.0 |
| | | | | | | | |
| 8 | 0.2500 | 8.5 | 4.0 | 0.0 | 9.0 | 0.0 | 6.0 |
| | 0.1250 | 7.5 | 0.0 | 0.0 | 9.0 | 0.0 | 5.0 |
| | 0.0625 | 6.5 | 0.0 | 0.0 | 9.0 | 0.0 | 3.5 |
| | 0.0313 | 6.5 | 0.0 | 0.0 | 9.0 | 0.0 | 2.5 |
| | | | | | | | |
| 9 | 0.2500 | 7.0 | 6.0 | 7.0 | 9.0 | 2.0 | 9.0 |
| | 0.1250 | 4.0 | 4.0 | 2.0 | 9.0 | 1.0 | 9.0 |
| | 0.0625 | 2.0 | 2.0 | 0.0 | 9.0 | 0.0 | 8.0 |
| | 0.0313 | 0.0 | 0.0 | 0.0 | 9.0 | 0.0 | 7.0 |
| | | | | | | | |
| 10 | 0.2500 | 2.0 | 4.0 | 0.0 | 9.0 | 0.0 | 7.0 |
| | 0.1250 | 2.0 | 2.0 | 0.0 | 9.0 | 0.0 | 6.0 |
| | 0.0625 | 1.0 | 0.0 | 0.0 | 9.0 | 0.0 | 4.0 |
| | 0.0313 | 0.0 | 0.0 | 0.0 | 9.0 | 0.0 | 3.0 |
| | | | | | | | |
| 11 | 0.2500 | 3.0 | 0.0 | 0.0 | 7.0 | 0.0 | 3.0 |
| | 0.1250 | 2.0 | 0.0 | 0.0 | 4.0 | 0.0 | 3.0 |
| | 0.0625 | 2.0 | 0.0 | 0.0 | 1.0 | 0.0 | 2.0 |
| | 0.0313 | 1.0 | 0.0 | 0.0 | 0.0 | 0.0 | 2.0 |
| | | | | | | | |
| 12 | 0.2500 | 9.0 | 0.0 | 6.0 | 9.0 | 0.0 | 7.0 |
| | 0.1250 | 8.5 | 1.0 | 1.0 | 9.0 | 4.0 | 5.5 |
| | 0.0625 | 9.0 | 0.0 | 3.0 | 9.0 | 1.0 | 3.5 |
| | 0.0313 | 5.0 | 0.0 | 1.0 | 9.0 | 0.0 | 3.0 |
| | | | | | | | |
| 13 | 0.2500 | 9.0 | 1.0 | 0.0 | 9.0 | 0.0 | 5.0 |
| | 0.1250 | 8.0 | 0.5 | 0.0 | 9.0 | 0.0 | 3.0 |
| | 0.0625 | 7.0 | 0.0 | 0.0 | 9.0 | 0.0 | 2.5 |
| | 0.0313 | 5.5 | 0.0 | 0.0 | 9.0 | 0.0 | 1.5 |
| | 0.0156 | 3.5 | 0.0 | 0.0 | 7.5 | 0.0 | 1.0 |
| | | | | | | | |
| 14 | 0.2500 | 9.0 | 0.0 | 0.0 | 0.0 | 0.0 | 0.0 |
| | 0.1250 | 7.5 | 4.5 | 3.0 | 4.5 | 0.0 | 1.0 |
| | 0.0625 | 4.5 | 2.5 | 0.0 | 4.5 | 0.0 | 0.5 |
| | 0.0313 | 1.5 | 2.0 | 0.0 | 4.5 | 0.0 | 0.0 |
| | | | | | | | |
| 15 | 0.2500 | 9.0 | 7.0 | 0.0 | 9.0 | 0.0 | 3.0 |
| | 0.1250 | 9.0 | 6.0 | 0.0 | 9.0 | 0.0 | 3.0 |
| | 0.0625 | 9.0 | 2.0 | 0.0 | 9.0 | 0.0 | 2.0 |
| | 0.0313 | 6.0 | 2.0 | 0.0 | 9.0 | 0.0 | 0.0 |
| | | | | | | | |
| 16 | 0.2500 | 0.0 | 9.0 | 0.0 | 3.0 | 0.0 | 0.0 |
| | 0.1250 | 0.0 | 4.0 | 0.0 | 9.0 | 0.0 | 0.0 |
| | 0.0625 | 0.0 | 0.0 | 0.0 | 9.0 | 0.0 | 0.0 |
| | 0.0313 | 0.0 | 0.0 | 0.0 | 9.0 | 0.0 | 0.0 |
| | | | | | | | |
| 17 | 0.2500 | 9.0 | 9.0 | 9.0 | 9.0 | 4.0 | 9.0 |
| | 0.1250 | 9.0 | 6.0 | 9.0 | 9.0 | 4.0 | 9.0 |
| | 0.0625 | 9.0 | 0.0 | 9.0 | 9.0 | 0.0 | 6.0 |
| | 0.0313 | 4.0 | 0.0 | 9.0 | 9.0 | 0.0 | 4.0 |
| | | | | | | | |
| 18 | 0.4630 | 7.0 | 0.0 | 4.0 | 9.0 | 6.0 | 3.0 |
| | 0.2310 | 7.0 | 0.0 | 0.0 | 9.0 | 0.0 | 3.0 |
| | 0.1160 | 7.0 | 0.0 | 0.0 | 9.0 | 0.0 | 3.0 |
| | | | | | | | |
| 19 | 0.4630 | 9.0 | 0.0 | 4.0 | 9.0 | 3.0 | 3.0 |
| | 0.2310 | 7.0 | 0.0 | 4.0 | 9.0 | 3.0 | 3.0 |
| | 0.1160 | 7.0 | 0.0 | 4.0 | 9.0 | 0.0 | 3.0 |
| | | | | | | | |
| 20 | 0.4630 | 7.0 | 0.0 | 5.0 | 9.0 | 3.0 | 2.0 |
| | 0.2310 | 7.0 | 0.0 | 5.0 | 9.0 | 0.0 | 2.0 |
| | 0.1160 | 7.0 | 0.0 | 5.0 | 9.0 | 0.0 | 2.0 |
| | | | | | | | |
| 21 | 0.4630 | 5.0 | 0.0 | 0.0 | 0.0 | 0.0 | 0.0 |
| | 0.2310 | 4.0 | 0.0 | 0.0 | 0.0 | 0.0 | 0.0 |
| | 0.1160 | 2.0 | 0.0 | 0.0 | 0.0 | 0.0 | 0.0 |
| | | | | | | | |
| 22 | 0.4630 | 9.0 | 5.0 | 4.0 | 9.0 | 5.0 | 7.0 |
| | 0.2310 | 9.0 | 0.0 | 4.0 | 9.0 | 4.0 | 6.0 |
| | 0.1160 | 6.0 | 0.0 | 4.0 | 9.0 | 4.0 | 3.0 |
| | | | | | | | |
| 23 | 0.4630 | 4.0 | 0.0 | 0.0 | 9.0 | 3.0 | 5.0 |
| | 0.2310 | 4.0 | 0.0 | 0.0 | 9.0 | 0.0 | 4.0 |
| | 0.1160 | 4.0 | 0.0 | 0.0 | 9.0 | 0.0 | 4.0 |
| | | | | | | | |
| 24 | 0.2500 | 9.0 | 0.0 | | 9.0 | 0.0 | 0.0 |
| | 0.1250 | 9.0 | 0.0 | | 9.0 | 0.0 | 0.0 |
| | 0.0625 | 9.0 | 0.0 | | 9.0 | 0.0 | 0.0 |
| | 0.0313 | 4.0 | 0.0 | | 0.0 | 0.0 | 0.0 |
| | | | | | | | |
| 25 | 0.2500 | 9.0 | 0.0 | | 9.0 | 5.0 | 5.0 |
| | 0.1250 | 7.0 | 0.0 | | 9.0 | 5.0 | 4.0 |
| | 0.0625 | 6.0 | 0.0 | | 9.0 | 0.0 | 4.0 |
| | 0.0313 | 4.0 | 0.0 | | 9.0 | 0.0 | 3.0 |
| | | | | | | | |
| 26 | 0.2500 | 9.0 | 9.0 | | 9.0 | 7.0 | 2.0 |
| | 0.1250 | 9.0 | 7.0 | | 9.0 | 5.0 | 1.0 |
| | 0.0625 | 9.0 | 4.0 | | 9.0 | 4.0 | 0.0 |
| | 0.0313 | 7.0 | 2.0 | | 9.0 | 0.0 | 0.0 |
| | | | | | | | |
| 27 | 0.2500 | 9.0 | 9.0 | 9.0 | 9.0 | 4.0 | 4.0 |
| | 0.1250 | 9.0 | 6.0 | 0.0 | 9.0 | 4.0 | 4.0 |
| | 0.0625 | 7.0 | 4.0 | 0.0 | 9.0 | 0.0 | 2.0 |
| | 0.0313 | 6.0 | 3.0 | 0.0 | 9.0 | 0.0 | 1.0 |
| | | | | | | | |
| 28 | 0.2500 | 9.0 | 9.0 | 0.0 | 9.0 | 0.0 | 1.0 |
| | 0.1250 | 7.0 | 9.0 | 0.0 | 9.0 | 0.0 | 0.0 |
| | 0.0625 | 4.0 | 9.0 | 0.0 | 9.0 | 0.0 | 0.0 |
| | 0.0313 | 0.0 | 9.0 | 0.0 | 9.0 | 0.0 | 0.0 |
| | | | | | | | |
| 29 | 0.2500 | 7.0 | 0.0 | 0.0 | 0.0 | 0.0 | 3.0 |
| | 0.1250 | 7.0 | 0.0 | 0.0 | 0.0 | 0.0 | 2.0 |
| | 0.0625 | 7.0 | 0.0 | 0.0 | 0.0 | 0.0 | 1.0 |
| | 0.0313 | 4.0 | 0.0 | 0.0 | 0.0 | 0.0 | 0.0 |
| | | | | | | | |
| 30 | 0.2500 | 0.0 | 4.5 | 0.0 | 4.5 | 2.0 | 0.0 |
| | 0.1250 | 0.0 | 2.0 | 0.0 | 1.5 | 0.0 | 0.0 |
| | | | | | | | |
| 31 | 0.2500 | 5.0 | 2.5 | 0.0 | 2.5 | 0.0 | 0.0 |
| | 0.1250 | 2.0 | 0.0 | 0.0 | 0.0 | 0.0 | 0.0 |
| | 0.0625 | 1.0 | 0.0 | 0.0 | 0.0 | 0.0 | 0.0 |
| | | | | | | | |
| 32 | 0.2500 | 6.0 | 0.0 | 0.0 | | 0.0 | 0.0 |
| | 0.1250 | 3.0 | 0.0 | 0.0 | | 0.0 | 0.0 |
| | | | | | | | |
| 33 | 0.2500 | 0.0 | 0.0 | 0.0 | | 0.0 | 0.0 |
| | | | | | | | |
| 34 | 0.2500 | 9.0 | 0.0 | 0.0 | | 0.0 | 6.0 |
| | 0.1250 | 4.0 | 0.0 | 0.0 | | 0.0 | 4.0 |
| | 0.0625 | 4.0 | 0.0 | 0.0 | | 0.0 | 3.0 |
| | 0.0313 | 3.0 | 0.0 | 0.0 | | 0.0 | 3.0 |
| | | | | | | | |
| 35 | 0.2500 | 6.0 | 0.0 | 0.0 | | 3.0 | 3.0 |
| | 0.1250 | 3.0 | 0.0 | 0.0 | | 0.0 | 2.0 |
| | | | | | | | |
| 36 | 0.2500 | 9.0 | 9.0 | 4.0 | | 9.0 | 5.0 |
| | 0.1250 | 7.0 | 5.0 | 2.0 | | 3.0 | 3.0 |
| | 0.0625 | 6.0 | 5.0 | 0.0 | | 0.0 | 3.0 |
| | 0.0313 | 4.0 | 0.0 | 0.0 | | 0.0 | 3.0 |
| | | | | | | | |
| 37 | 0.2500 | 7.0 | 0.0 | 0.0 | | 3.0 | 3.0 |
| | 0.1250 | 7.0 | 0.0 | 0.0 | | 0.0 | 0.0 |
| | 0.0625 | 6.0 | 0.0 | 0.0 | | 0.0 | 0.0 |
| | 0.0313 | 4.0 | 0.0 | 0.0 | | 0.0 | 0.0 |
| | | | | | | | |
| 38 | 0.2500 | 7.0 | 5.0 | 3.0 | 9.0 | 9.0 | 7.0 |
| | 0.1250 | 4.0 | 5.0 | 0.0 | 9.0 | 4.0 | 5.0 |
| | 0.0625 | 2.0 | 3.0 | 0.0 | 9.0 | 3.0 | 5.0 |
| | 0.0313 | 0.0 | 3.0 | 0.0 | 9.0 | 0.0 | 2.0 |
| | | | | | | | |
| 39 | 0.2500 | 3.0 | 0.0 | 0.0 | 9.0 | 4.0 | 3.0 |
| | 0.1250 | 2.0 | 0.0 | 0.0 | 9.0 | 0.0 | 2.0 |
| | 0.0625 | 0.0 | 0.0 | 0.0 | 9.0 | 0.0 | 0.0 |
| | 0.0313 | 0.0 | 0.0 | 0.0 | 9.0 | 0.0 | 0.0 |
| | | | | | | | |
| 40 | 0.2500 | 9.0 | 0.0 | 6.0 | 9.0 | 0.0 | 3.0 |
| | 0.1250 | 5.0 | 0.0 | 0.0 | 9.0 | 0.0 | 3.0 |
| | 0.0625 | 5.0 | 0.0 | 0.0 | 9.0 | 0.0 | 3.0 |
| | 0.0313 | 5.0 | 0.0 | 0.0 | 9.0 | 0.0 | 2.0 |
| | | | | | | | |
| 41 | 0.2500 | 7.0 | 4.0 | 7.0 | 9.0 | 6.0 | 5.0 |
| | 0.1250 | 6.0 | 0.0 | 2.0 | 9.0 | 0.0 | 5.0 |
| | 0.0625 | 4.0 | 0.0 | 0.0 | 9.0 | 0.0 | 5.0 |
| | 0.0313 | 3.0 | 0.0 | 0.0 | 6.0 | 0.0 | 4.0 |
| | | | | | | | |
| 42 | 0.2500 | 6.0 | 0.0 | 0.0 | 9.0 | 3.0 | 0.0 |
| | 0.1250 | 5.0 | 0.0 | 0.0 | 9.0 | 0.0 | 0.0 |
| | 0.0625 | 5.0 | 0.0 | 0.0 | 9.0 | 0.0 | 0.0 |
| | 0.0313 | 5.0 | 0.0 | 0.0 | 7.0 | 0.0 | 0.0 |
| | | | | | | | |
| 43 | 0.2500 | 9.0 | 5.0 | 9.0 | 9.0 | 9.0 | 6.0 |
| | 0.1250 | 7.0 | 4.0 | 8.0 | 9.0 | 7.0 | 4.0 |
| | 0.0625 | 6.0 | 2.0 | 5.0 | 9.0 | 7.0 | 3.0 |
| | 0.0313 | 5.0 | 0.0 | 5.0 | 9.0 | 7.0 | 2.0 |
| | | | | | | | |
| 44 | 0.2500 | 8.0 | 9.0 | 0.0 | 9.0 | | 5.0 |
| | 0.1250 | 7.0 | 6.0 | 0.0 | 7.0 | | 3.0 |
| | 0.0625 | 6.0 | 6.0 | 0.0 | 4.0 | | 3.0 |
| | 0.0313 | 6.0 | 5.0 | 0.0 | 4.0 | | 0.0 |
| | | | | | | | |
| 45 | 0.2500 | 7.0 | 7.0 | 0.0 | 9.0 | 4.0 | 3.0 |
| | 0.1250 | 7.0 | 7.0 | | 9.0 | 0.0 | 2.0 |
| | 0.0625 | 4.0 | 3.0 | 0.0 | 7.0 | 0.0 | 2.0 |
| | 0.0313 | 3.0 | 0.0 | 0.0 | 7.0 | 0.0 | 3.0 |
| | | | | | | | |
| 46 | 0.2500 | 4.0 | 0.0 | 6.0 | 7.0 | 3.0 | 3.0 |
| | 0.1250 | 0.0 | 0.0 | 0.0 | 6.0 | 0.0 | 2.0 |
| | 0.0625 | 0.0 | 0.0 | 0.0 | 4.0 | 0.0 | 2.0 |
| | 0.0313 | 0.0 | 0.0 | 0.0 | 3.0 | 0.0 | 0.0 |
| | | | | | | | |
| 47 | 0.2500 | 7.0 | 6.0 | 0.0 | 9.0 | 7.0 | 3.0 |
| | 0.1250 | 4.0 | 4.0 | 0.0 | 9.0 | 4.0 | 0.0 |
| | 0.0625 | 2.0 | 0.0 | 0.0 | 6.0 | 0.0 | 0:0 |
| | 0.0313 | 0.0 | 0.0 | 0.0 | 5.0 | 0.0 | 0.0 |
| | | | | | | | |
| 48 | 0.2500 | 6.0 | 5.0 | 0.0 | 6.0 | 6.0 | 6.0 |
| | 0.1250 | 3.0 | 2.0 | 0.0 | 3.0 | 3.0 | 4.0 |
| | | | | | | | |
| 49 | 0.2500 | 7.0 | 7.0 | 4.0 | 9.0 | 7.0 | 7.0 |
| | 0.1250 | 5.0 | 2.0 | | 9.0 | 6.0 | 7.0 |
| | 0.0625 | 2.0 | 0.0 | 3.0 | 9.0 | 4.0 | 5.0 |
| | 0.0313 | 0.0 | 0.0 | 2.0 | 9.0 | 2.0 | 5.0 |
| | | | | | | | |
| 50 | 0.2500 | 7.0 | 0.0 | 0.0 | 7.0 | 3.0 | 1.0 |
| | 0.1250 | 7.0 | 0.0 | 0.0 | 3.0 | 3.0 | 0.0 |
| | 0.0625 | 7.0 | 0.0 | 0.0 | 0.0 | 2.0 | 0.0 |
| | 0.0313 | 4.0 | 0.0 | 0.0 | 0.0 | 2.0 | 0.0 |
| | | | | | | | |
| 51 | 0.2500 | 0.0 | 6.0 | 0.0 | 4.0 | 0.0 | 0.0 |
| | 0.1250 | 0.0 | 3.0 | 0.0 | 4.0 | 0.0 | 0.0 |
| | 0.0625 | 0.0 | 2.0 | 0.0 | 3.0 | 0.0 | 0.0 |
| | | | | | | | |
| 52 | 0.2500 | 0.0 | 2.0 | 0.0 | 4.0 | 0.0 | 0.0 |
| | | | | | | | |
| 53 | 0.2500 | 3.0 | 9.0 | 0.0 | 9.0 | 0.0 | 3.0 |
| | 0.1250 | 2.0 | 9.0 | 0.0 | 9.0 | 0.0 | 2.0 |
| | 0.0625 | 0.0 | 2.0 | 0.0 | 2.0 | 0.0 | 0.0 |
| | 0.0313 | 0.0 | 2.0 | 0.0 | 2.0 | 0.0 | 0.0 |
| | | | | | | | |
| 54 | 0.2500 | 9.0 | 4.0 | 4.0 | 6.0 | 2.0 | 4.0 |
| | 0.1250 | 7.0 | 4.0 | 0.0 | 6.0 | 0.0 | 3.0 |
| | 0.0625 | 5.0 | 4.0 | 0.0 | 0.0 | 0.0 | 2.0 |
| | 0.0313 | 5.0 | 0.0 | 0.0 | 0.0 | 0.0 | 0.0 |
| | | | | | | | |
| 55 | 0.2500 | 7.0 | 9.0 | 0.0 | 9.0 | 0.0 | 4.0 |
| | 0.1250 | 7.0 | 9.0 | 0.0 | 9.0 | 0.0 | 1.0 |
| | 0.0625 | 7.0 | 9.0 | 0.0 | 9.0 | 0.0 | 1.0 |
| | 0.0313 | 7.0 | 9.0 | 0.0 | 9.0 | 0.0 | 0.0 |
| | | | | | | | |
| 56 | 0.2500 | 5.0 | 0.0 | 0.0 | 9.0 | 0.0 | 2.0 |
| | 0.1250 | 3.0 | 0.0 | 0.0 | 9.0 | 0.0 | 0.0 |
| | 0.0625 | 0.0 | 0.0 | 0.0 | 6.0 | 0.0 | 0.0 |
| | 0.0313 | 0.0 | 0.0 | 0.0 | 6.0 | 0.0 | 0.0 |
| | | | | | | | |
| 57 | 0.2500 | 5.0 | 9.0 | 3.0 | 9.0 | 6.0 | 4.0 |
| | 0.1250 | 2.0 | 9.0 | 0.0 | 9.0 | 0.0 | 0.0 |
| | 0.0625 | 2.0 | 9.0 | 0.0 | 9.0 | 0.0 | 0.0 |
| | 0.0313 | 0.0 | 0.0 | 0.0 | 9.0 | 0.0 | 0.0 |
| | | | | | | | |
| 58 | 0.2500 | 9.0 | 3.0 | 5.0 | 9.0 | 4.0 | 5.0 |
| | 0.1250 | 7.0 | 2.0 | 5.0 | 3.0 | 0.0 | 4.0 |
| | 0.0625 | 7.0 | 0.0 | 5.0 | 0.0 | 0.0 | 4.0 |
| | 0.0313 | 7.0 | 0.0 | 5.0 | 0.0 | 0.0 | 4.0 |
| | | | | | | | |
| 59 | 0.2500 | 7.0 | 9.0 | 7.0 | 9.0 | 3.0 | 2.0 |
| | 0.1250 | 5.0 | 0.0 | 0.0 | 0.0 | 0.0 | 2.0 |
| | 0.0625 | 4.0 | 0.0 | 0.0 | 0.0 | 0.0 | 0.0 |
| | | | | | | | |
| 60 | 0.2500 | 9.0 | 6.0 | 4.0 | 9.0 | 9.0 | 5.0 |
| | 0.1250 | 9.0 | 6.0 | 4.0 | 9.0 | 9.0 | 5.0 |
| | 0.0625 | 9.0 | 3.0 | 2.0 | 9.0 | 4.0 | 4.0 |
| | 0.0313 | 7.0 | 3.0 | 2.0 | 9.0 | 3.0 | 4.0 |
| | | | | | | | |
| 61 | 0.2500 | 0.0 | 0.0 | 0.0 | 0.0 | 0.0 | 0.0 |
| | | | | | | | |
| 62 | 0.2500 | 5.0 | 9.0 | 0.0 | 9.0 | 6.0 | 4.0 |
| | 0.1250 | 2.0 | 9.0 | 0.0 | 9.0 | 3.0 | 3.0 |
| | 0.0625 | 0.0 | 2.0 | 0.0 | 9.0 | 0.0 | 3.0 |
| | 0.0313 | 0.0 | 0.0 | 0.0 | 4.0 | 0.0 | 2.0 |
| | | | | | | | |
| 63 | 0.2500 | 5.0 | 7.0 | 0.0 | 9.0 | 9.0 | 3.0 |
| | 0.1250 | 5.0 | 7.0 | 0.0 | 9.0 | 9.0 | 3.0 |
| | 0.0625 | 0.0 | 6.0 | 0.0 | 9.0 | 7.0 | 3.0 |
| | 0.0313 | 0.0 | 2.0 | 0.0 | 9.0 | 4.0 | 3.0 |
| | | | | | | | |
| 64 | 0.2500 | 9.0 | 7.0 | 0.0 | 9.0 | 8.0 | 6.0 |
| | 0.1250 | 6.0 | 2.0 | 0.0 | 9.0 | 5.0 | 5.0 |
| | 0.0625 | 6.0 | 2.0 | 0.0 | 9.0 | 3.0 | 4.0 |
| | 0.0313 | 5.0 | 0.0 | 0.0 | 7.0 | 0.0 | 3.0 |
| | | | | | | | |
| 65 | 0.2500 | 7.0 | 8.0 | 0.0 | 9.0 | 7.0 | 6.0 |
| | 0.1250 | 6.0 | 7.0 | 0.0 | 9.0 | 5.0 | 6.0 |
| | 0.0625 | 5.0 | 7.0 | 0.0 | 9.0 | 4.0 | 3.0 |
| | 0.0313 | 5.0 | 6.0 | 0.0 | 9.0 | 2.0 | 2.0 |
| | | | | | | | |
| 66 | 0.2500 | 9.0 | 7.0 | 4.0 | 9.0 | 4.0 | 3.0 |
| | 0.1250 | 9.0 | 7.0 | 4.0 | 9.0 | 4.0 | 3.0 |
| | 0.0625 | 7.0 | 6.0 | 0.0 | 9.0 | 4.0 | 2.0 |
| | 0.0313 | 7.0 | 6.0 | 0.0 | 9.0 | 3.0 | 2.0 |
| | | | | | | | |
| 67 | 0.2500 | 5.0 | 8.0 | 3.0 | 9.0 | 6.0 | 6.0 |
| | 0.1250 | 5.0 | 5.0 | 3.0 | 9.0 | 4.0 | 4.0 |
| | 0.0625 | 4.0 | 5.0 | 3.0 | 9.0 | 4.0 | 3.0 |
| | 0.0313 | 0.0 | 5.0 | 0.0 | 9.0 | 0.0 | 2.0 |
| | | | | | | | |
| 68 | 0.2500 | 7.0 | 8.0 | 0.0 | 9.0 | 3.0 | 4.0 |
| | 0.1250 | 7.0 | 5.0 | 0.0 | 9.0 | 0.0 | 2.0 |
| | 0.0625 | 5.0 | 0.0 | 0.0 | 9.0 | 0.0 | 0.0 |
| | 0.0313 | 0.0 | 0.0 | 0.0 | 3.0 | 0.0 | 0.0 |
| | | | | | | | |
| 69 | 0.2500 | 7.0 | 3.0 | 9.0 | | 3.0 | 3.0 |
| | 0.1250 | 5.0 | 3.0 | 9.0 | | 3.0 | 2.0 |
| | 0.0625 | 4.0 | 3.0 | 3.0 | | 0.0 | 2.0 |
| | 0.0313 | 0.0 | 3.0 | 3.0 | | 0.0 | 0.0 |
| | | | | | | | |
| 70 | 0.2500 | 0.0 | 2.0 | 3.0 | | 4.0 | 2.0 |
| | 0.1250 | 0.0 | 2.0 | 0.0 | | 4.0 | 2.0 |
| | 0.0625 | 0.0 | 2.0 | 0.0 | | 4.0 | 0.0 |
| | 0.0313 | 0.0 | 0.0 | 0.0 | | 4.0 | 0.0 |
| | | | | | | | |
| 71 | 0.2500 | 7.0 | 7.0 | 3.0 | | 0.0 | 4.0 |
| | 0.1250 | 0.0 | 3.0 | 3.0 | | 0.0 | 4.0 |
| | | | | | | | |
| 72 | 0.2500 | 9.0 | 9.0 | 9.0 | | 4.0 | 3.0 |
| | 0.1250 | 9.0 | 7.0 | 9.0 | | 4.0 | 3.0 |
| | 0.0625 | 9.0 | 6.0 | 0.0 | | 4.0 | 2.0 |
| | 0.0313 | 9.0 | 6.0 | 0.0 | | 4.0 | 1.0 |
| | | | | | | | |
| 73 | 0.2500 | 7.0 | 6.0 | 4.0 | | 4.0 | 6.0 |
| | 0.1250 | 3.0 | 0.0 | 2.0 | | 3.0 | 5.0 |
| | | | | | | | |
| 74 | 0.2500 | 9.0 | 0.0 | 9.0 | | 9.0 | 3.0 |
| | 0.1250 | 9.0 | 0.0 | 5.0 | | 3.0 | 2.0 |
| | 0.0625 | 9.0 | 0.0 | 5.0 | | 3.0 | 0.0 |
| | 0.0313 | 7.0 | 0.0 | 0.0 | | 3.0 | 0.0 |
| | | | | | | | |
| 75 | 0.2500 | 9.0 | 6.0 | 3.0 | | 3.0 | 2.0 |
| | 0.1250 | 9.0 | 6.0 | 3.0 | | 0.0 | 1.0 |
| | 0.0625 | 6.0 | 0.0 | 3.0 | | 0.0 | 0.0 |
| | 0.0313 | 6.0 | 0.0 | 2.0 | | 0.0 | 0.0 |
| | | | | | | | |
| 76 | 0.2500 | 9.0 | 6.0 | 3.0 | | 4.0 | 4.0 |
| | 0.1250 | 9.0 | 0.0 | 2.0 | | 4.0 | 3.0 |
| | 0.0625 | 7.0 | 0.0 | 2.0 | | 0.0 | 2.0 |
| | 0.0313 | 4.0 | 0.0 | 2.0 | | 0.0 | 2.0 |
| | | | | | | | |
| 77 | 0.2500 | 9.0 | 0.0 | 4.0 | | 0.0 | 3.0 |
| | 0.1250 | 9.0 | 0.0 | 0.0 | | 0.0 | 3.0 |
| | 0.0625 | 9.0 | 0.0 | 0.0 | | 0.0 | 2.0 |
| | 0.0313 | 9.0 | 0.0 | 0.0 | | 0.0 | 2.0 |
| | | | | | | | |
| 78 | 0.2500 | 9.0 | 9.0 | 9.0 | | 4.0 | 1.0 |
| | 0.1250 | 9.0 | 6.0 | 7.0 | | 2.0 | 0.0 |
| | 0.0625 | 7.0 | 5.0 | 6.0 | | 0.0 | 0.0 |
| | 0.0313 | 7.0 | 5.0 | 6.0 | | 0.0 | 0.0 |
| | | | | | | | |
| 79 | 0.2500 | 8.0 | 6.0 | 9.0 | | 4.0 | 3.0 |
| | 0.1250 | 7.0 | 2.0 | 0.0 | | 4.0 | 3.0 |
| | 0.0625 | 6.0 | 0.0 | 0.0 | | 0.0 | 2.0 |
| | 0.0313 | 5.0 | 0.0 | 0.0 | | 0.0 | 0.0 |
| | | | | | | | |
| 80 | 0.2500 | 9.0 | 7.0 | 4.0 | | 0.0 | 2.0 |
| | 0.1250 | 9.0 | 7.0 | 3.0 | | 0.0 | 0.0 |
| | 0.0625 | 9.0 | 7.0 | 3.0 | | 0.0 | 0.0 |
| | 0.0313 | 9.0 | 5.0 | 3.0 | | 0.0 | 0.0 |
| | | | | | | | |
| 81 | 0.2500 | 3.0 | 5.0 | 0.0 | | 0.0 | 0.0 |
| | 0.1250 | 3.0 | 5.0 | 0.0 | | 0.0 | 0.0 |
| | 0.0625 | 0.0 | 4.0 | 0.0 | | 0.0 | 0.0 |
| | | | | | | | |
| 82 | 0.2500 | 9.0 | 3.0 | 4.0 | | 7.0 | 3.0 |
| | 0.1250 | 6.0 | 3.0 | 2.0 | | 4.0 | 3.0 |
| | 0.0625 | 4.0 | 0.0 | 0.0 | | 4.0 | 0.0 |
| | 0.0313 | 4.0 | 0.0 | 0.0 | | 4.0 | 0.0 |
| | | | | | | | |
| 83 | 0.2500 | 7.0 | 7.0 | 5.0 | 9.0 | 9.0 | 6.0 |
| | 0.1250 | 7.0 | 6.0 | 3.0 | 9.0 | 7.0 | 5.0 |
| | 0.0625 | 6.0 | 6.0 | | 9.0 | 7.0 | 3.0 |
| | 0.0313 | 5.0 | 5.0 | 0.0 | 9.0 | 7.0 | 3.0 |
| | | | | | | | |
| 84 | 0.2500 | 9.0 | 9.0 | 9.0 | 9.0 | 9.0 | 7.0 |
| | 0.1250 | 7.0 | 9.0 | 9.0 | 9.0 | 7.0 | 6.0 |
| | 0.0625 | 5.0 | 7.0 | 3.0 | 9.0 | 6.0 | 4.0 |
| | 0.0313 | 4.0 | 3.0 | 0.0 | 9.0 | 2.0 | 3.0 |
| | | | | | | | |
| 85 | 0.2500 | 9.0 | 9.0 | 9.0 | 9.0 | 9.0 | 5.0 |
| | 0.1250 | 7.0 | 9.0 | 2.0 | 9.0 | 9.0 | 4.0 |
| | 0.0625 | 5.0 | 7.0 | 2.0 | 9.0 | 3.0 | 4.0 |
| | 0.0313 | 4.0 | 3.0 | 0.0 | 9.0 | 3.0 | 3.0 |
| | | | | | | | |
| 86 | 0.2500 | 9.0 | 9.0 | 9.0 | 9.0 | 9.0 | 5.0 |
| | 0.1250 | 9.0 | 8.0 | 3.0 | 9.0 | 9.0 | 4.0 |
| | 0.0625 | 6.0 | 7.0 | 0.0 | 9.0 | 9.0 | 4.0 |
| | 0.0313 | 5.0 | 3.0 | 0.0 | 9.0 | 5.0 | 3.0 |
| | | | | | | | |
| 87 | 0.5000 | 3.0 | 7.0 | 5.0 | 9.0 | 7.0 | 6.0 |
| | 0.2500 | 3.0 | 5.0 | 5.0 | 9.0 | 5.0 | 4.0 |
| | 0.1250 | 2.0 | 5.0 | 5.0 | 9.0 | 4.0 | 3.0 |
| | 0.0625 | 0.0 | 5.0 | 0.0 | 9.0 | 4.0 | 3.0 |
| | 0.0313 | 0.0 | 5.0 | 0.0 | 9.0 | 0.0 | 2.0 |
| | | | | | | | |
| 88 | 0.2500 | 7.0 | 0.0 | 7.0 | 9.0 | 9.0 | 4.0 |
| | 0.1250 | 6.0 | 0.0 | 7.0 | 9.0 | 9.0 | 4.0 |
| | 0.0625 | 6.0 | 0.0 | 5.0 | 9.0 | 9.0 | 3.0 |
| | 0.0313 | 6.0 | 0.0 | 3.0 | 9.0 | 6.0 | 2.0 |
| | | | | | | | |
| 89 | 0.2500 | 6.0 | 4.0 | 6.0 | 9.0 | 9.0 | 6.0 |
| | 0.1250 | 5.0 | 2.0 | 4.0 | 9.0 | 9.0 | 5.0 |
| | 0.0625 | 4.0 | 0.0 | 0.0 | 9.0 | 5.0 | 4.0 |
| | 0.0313 | 3.0 | 0.0 | 0.0 | 9.0 | 4.0 | 2.0 |
| | | | | | | | |
| 90 | 0.2500 | 3.0 | 3.0 | 3.0 | 9.0 | 9.0 | 3.0 |
| | 0.1250 | 0.0 | 1.0 | 0.0 | 9.0 | 9.0 | 1.0 |
| | 0.0625 | 0.0 | 0.0 | 0.0 | 9.0 | 6.0 | 0.0 |
| | 0.0313 | 0.0 | 0.0 | 0.0 | 9.0 | 4.0 | 0.0 |
| | | | | | | | |
| 91 | 0.2500 | 4.0 | 0.0 | 0.0 | 9.0 | 0.0 | 2.0 |
| | 0.1250 | 2.0 | 0.0 | 0.0 | 9.0 | 0.0 | 0.0 |
| | 0.0625 | 0.0 | 0.0 | 0.0 | 9.0 | 0.0 | 0.0 |
| | 0.0313 | 0.0 | 0.0 | 0.0 | 9.0 | 0.0 | 0.0 |
| | | | | | | | |
| 92 | 0.2500 | 3.0 | 7.0 | 9.0 | 9.0 | 9.0 | 3.0 |
| | 0.1250 | 2.0 | 5.0 | 9.0 | 9.0 | 4.0 | 3.0 |
| | 0.0625 | 0.0 | 3.0 | 9.0 | 9.0 | 0.0 | 2.0 |
| | 0.0313 | 0.0 | 3.0 | 9.0 | 9.0 | 0.0 | 1.0 |
| | | | | | | | |
| 93 | 0.2500 | 7.0 | 0.0 | 9.0 | 9.0 | 9.0 | 3.0 |
| | 0.1250 | 4.0 | 0.0 | 5.0 | 9.0 | 3.0 | 0.0 |
| | 0.0625 | 3.0 | 0.0 | 0.0 | 9.0 | 0.0 | 0.0 |
| | 0.0313 | 0.0 | 0.0 | 0.0 | 9.0 | 0.0 | 0.0 |
| | | | | | | | |
| 94 | 0.2500 | 7.0 | 4.0 | 9.0 | 9.0 | 9.0 | 3.0 |
| | 0.1250 | 2.0 | 4.0 | 6.0 | 9.0 | 5.0 | 3.0 |
| | 0.0625 | 0.0 | 3.0 | | 9.0 | 0.0 | 2.0 |
| | 0.0313 | 0.0 | 0.0 | | 9.0 | 0.0 | 0.0 |
| | | | | | | | |
| 95 | 0.2500 | 3.0 | 7.0 | 3.0 | 9.0 | | 8.0 |
| | 0.1250 | 3.0 | 7.0 | 0.0 | 9.0 | | 8.0 |
| | 0.0625 | 0.0 | 4.0 | 0.0 | 5.0 | | 4.0 |
| | 0.0313 | 0.0 | 4.0 | 0.0 | 5.0 | | 3.0 |
| | | | | | | | |
| 96 | 0.2500 | 3.0 | 6.0 | 0.0 | 3.0 | | 5.0 |
| | 0.1250 | 0.0 | 6.0 | 0.0 | 3.0 | | 4.0 |
| | 0.0625 | 0.0 | 5.0 | 0.0 | 2.0 | | 4.0 |
| | 0.0313 | 0.0 | 0.0 | 0.0 | 2.0 | | 3.0 |
| | | | | | | | |
| 97 | 0.2500 | 3.0 | 4.0 | 3.0 | 9.0 | | 5.0 |
| | 0.1250 | 2.0 | 3.0 | 0.0 | 9.0 | | 5.0 |
| | 0.0625 | 0.0 | 0.0 | 0.0 | 9.0 | | 4.0 |
| | 0.0313 | 0.0 | 0.0 | 0.0 | 9.0 | | 1.0 |
| | | | | | | | |
| 98 | 0.2500 | 3.0 | 9.0 | 4.0 | 9.0 | | 7.0 |
| | 0.1250 | 1.0 | 9.0 | 4.0 | 9.0 | | 7.0 |
| | 0.0625 | 0.0 | 9.0 | 3.0 | 9.0 | | 6.0 |
| | 0.0313 | 0.0 | 9.0 | 2.0 | 9.0 | | 2.0 |
| | | | | | | | |
| 99 | 0.5000 | 4.0 | 0.0 | 0.0 | 9.0 | 4.0 | 4.0 |
| | 0.2500 | 0.0 | 0.0 | 0.0 | 9.0 | 4.0 | 4.0 |
| | 0.1250 | 0.0 | 0.0 | 0.0 | 9.0 | 3.0 | 4.0 |
| | 0.0625 | 0.0 | 0.0 | 0.0 | 9.0 | 0.0 | 2.0 |
| | 0.0313 | 0.0 | 0.0 | 0.0 | 9.0 | 0.0 | 2.0 |
| | | | | | | | |
| 100 | 0.5000 | 4.0 | 9.0 | | 9.0 | 6.0 | 4.0 |
| | 0.2500 | 3.0 | 4.0 | 0.0 | 9.0 | 4.0 | 3.0 |
| | 0.1250 | 0.0 | 0.0 | 0.0 | 9.0 | 0.0 | 3.0 |
| | 0.0625 | 0.0 | 0.0 | 0.0 | 9.0 | 0.0 | 3.0 |
| | 0.0313 | 0.0 | 0.0 | 0.0 | 9.0 | 0.0 | 3.0 |
| | | | | | | | |
| 101 | 0.5000 | 0.0 | 4.0 | 0.0 | 9.0 | 4.0 | 3.0 |
| | 0.2500 | 0.0 | 3.0 | 0.0 | 9.0 | 2.0 | 0.0 |
| | 0.1250 | 0.0 | 0.0 | 0.0 | 7.0 | 0.0 | 0.0 |
| | 0.0625 | 0.0 | 0.0 | 0.0 | 5.0 | 0.0 | 0.0 |
| | 0.0313 | 0.0 | 0.0 | 0.0 | 5.0 | 0.0 | 0.0 |
| | | | | | | | |
| 102 | 0.5000 | 2.0 | 6.0 | 0.0 | 4.0 | 0.0 | 4.0 |
| | 0.2500 | 0.0 | 2.0 | 0.0 | 0.0 | 0.0 | 2.0 |
| | | | | | | | |
| 103 | 0.2500 | 6.0 | 9.0 | 6.0 | 9.0 | 9.0 | 6.0 |
| | 0.1250 | 5.0 | 9.0 | 6.0 | 9.0 | 9.0 | 5.0 |
| | 0.0625 | 4.0 | 6.0 | 5.0 | 7.0 | 9.0 | 5.0 |
| | 0.0313 | 4.0 | 4.0 | 5.0 | 5.0 | 7.0 | 4.0 |
| | | | | | | | |
| 104 | 0.2500 | 6.0 | 9.0 | 0.0 | 9.0 | 9.0 | 6.0 |
| | 0.1250 | 6.0 | 9.0 | 0.0 | 9.0 | 9.0 | 7.0 |
| | 0.0625 | 5.0 | 9.0 | | 9.0 | 9.0 | 5.0 |
| | 0.0313 | 4.0 | 5.0 | 0.0 | 7.0 | 6.0 | 3.0 |
| | | | | | | | |
| 105 | 0.2500 | 5.0 | 9.0 | 9.0 | 9.0 | 7.0 | 6.0 |
| | 0.1250 | 4.0 | 7.0 | 7.0 | 7.0 | 5.0 | 3.0 |
| | 0.0625 | 3.0 | 7.0 | 5.0 | 7.0 | 5.0 | 5.0 |
| | 0.0313 | 0.0 | 5.0 | 2.0 | 6.0 | 3.0 | 5.0 |
| | | | | | | | |
| 106 | 0.2500 | 7.0 | 9.0 | 6.0 | 9.0 | 7.0 | 5.0 |
| | 0.1250 | 5.0 | 6.0 | 2.0 | 9.0 | 5.0 | 3.0 |
| | 0.0625 | 4.0 | 6.0 | 0.0 | 9.0 | 4.0 | 2.0 |
| | 0.0313 | 2.0 | 5.0 | 0.0 | 9.0 | 3.0 | 2.0 |
| | | | | | | | |
| 107 | 0.2500 | 4.0 | 7.0 | | 9.0 | 3.0 | 3.0 |
| | 0.1250 | 4.0 | 4.0 | 0.0 | 7.0 | 0.0 | 0.0 |
| | 0.0625 | 4.0 | 0.0 | 0.0 | 6.0 | 0.0 | 0.0 |
| | 0.0313 | 0.0 | 0.0 | 0.0 | 4.0 | 0.0 | 0.0 |
| | | | | | | | |
| 108 | 0.2500 | 0.0 | 7.0 | | 9.0 | 6.0 | 3.0 |
| | 0.1250 | 0.0 | 7.0 | 0.0 | 9.0 | 3.0 | 2.0 |
| | 0.0625 | 0.0 | 6.0 | 0.0 | 9.0 | 0.0 | 2.0 |
| | 0.0313 | 0.0 | 4.0 | 0.0 | 9.0 | 0.0 | 0.0 |
| | | | | | | | |
| 109 | 0.2500 | 9.0 | 9.0 | 6.5 | 9.0 | 9.0 | 7.5 |
| | 0.1250 | 9.0 | 9.0 | 9.0 | 9.0 | 9.0 | 7.0 |
| | 0.0625 | 9.0 | 7.0 | 5.5 | 9.0 | 7.5 | 8.0 |
| | 0.0313 | 9.0 | 5.0 | 4.5 | 7.0 | 4.5 | 5.0 |
| | | | | | | | |
| 110 | 0.2500 | 9.0 | 8.0 | 0.0 | 9.0 | 8.0 | 4.0 |
| | 0.1250 | 7.0 | 7.0 | 0.0 | 9.0 | 7.0 | 2.0 |
| | 0.0625 | 7.0 | 4.0 | 0.0 | 9.0 | 7.0 | 0.0 |
| | 0.0313 | 7.0 | 3.0 | 0.0 | 9.0 | 6.0 | 0.0 |
| | | | | | | | |
| 111 | 0.2500 | 7.0 | 5.0 | 9.0 | 4.0 | 6.0 | 3.0 |
| | 0.1250 | 6.0 | 4.0 | 9.0 | 4.0 | 5.0 | 4.0 |
| | 0.0625 | 3.0 | 3.0 | 4.0 | 4.0 | 4.0 | 3.0 |
| | 0.0313 | 0.0 | 0.0 | | 3.0 | 3.0 | 0.0 |
| | | | | | | | |
| 112 | 0.2500 | 3.0 | 7.0 | | 9.0 | 7.0 | 5.0 |
| | 0.1250 | 3.0 | 6.0 | | 9.0 | 7.0 | 3.0 |
| | 0.0625 | 3.0 | 3.0 | | 9.0 | 7.0 | 0.0 |
| | 0.0313 | 0.0 | 0.0 | 0.0 | 9.0 | 6.0 | 0.0 |
| | | | | | | | |
| 113 | 0.2500 | 7.0 | 6.0 | 0.0 | 9.0 | 9.0 | 0.0 |
| | 0.1250 | 5.0 | 5.0 | 0.0 | 9.0 | 9.0 | 0.0 |
| | 0.0625 | 4.0 | 5.0 | 0.0 | 9.0 | 6.0 | 0.0 |
| | 0.0313 | 4.0 | 3.0 | 0.0 | 9.0 | 4.0 | 0.0 |
| | | | | | | | |
| 114 | 0.2500 | 8.0 | 9.0 | 4.5 | 9.0 | 4.5 | 4.5 |
| | 0.1250 | 7.0 | 8.5 | 3.5 | 9.0 | 3.5 | 4.5 |
| | 0.0625 | 7.0 | 6.5 | 4.5 | 9.0 | 3.5 | 4.5 |
| | 0.0313 | 6.0 | 4.5 | 3.5 | 9.0 | 3.5 | 2.0 |
| | | | | | | | |
| 115 | 0.2500 | 5.0 | 9.0 | 6.0 | 9.0 | 6.0 | 4.0 |
| | 0.1250 | 5.0 | 9.0 | | 9.0 | 5.0 | 4.0 |
| | 0.0625 | 4.0 | 6.0 | 0.0 | 9.0 | 3.0 | 3.0 |
| | 0.0313 | 4.0 | 5.0 | 0.0 | 7.0 | 2.0 | 3.0 |
| | | | | | | | |
| 116 | 0.2500 | 5.0 | 7.0 | 8.0 | 9.0 | 6.0 | 4.0 |
| | 0.1250 | 6.0 | 7.0 | 7.0 | 9.0 | 5.0 | 4.0 |
| | 0.0625 | 5.0 | 6.0 | 6.0 | 9.0 | 3.0 | 2.0 |
| | 0.0313 | 0.0 | 3.0 | 6.0 | 7.0 | 2.0 | 2.0 |
| | | | | | | | |
| 117 | 0.2500 | 0.0 | 6.0 | 9.0 | 9.0 | 0.0 | 3.0 |
| | 0.1250 | 0.0 | 5.0 | 9.0 | 9.0 | 0.0 | 3.0 |
| | 0.0625 | 0.0 | 2.0 | 7.0 | 7.0 | 0.0 | 0.0 |
| | 0.0313 | 0.0 | 0.0 | | 6.0 | 0.0 | 2.0 |
| | | | | | | | |
| 118 | 0.2500 | 6.0 | 0.0 | 0.0 | 7.0 | 3.0 | 3.0 |
| | 0.1250 | 4.0 | 0.0 | 0.0 | 6.0 | 3.0 | 3.0 |
| | 0.0625 | 0.0 | 0.0 | 0.0 | 4.0 | 0.0 | 1.0 |
| | 0.0313 | 0.0 | 0.0 | 0.0 | 4.0 | 0.0 | 0.0 |
| | | | | | | | |
| 119 | 0.2500 | 9.0 | 8.0 | 1.0 | 9.0 | 4.5 | 5.5 |
| | 0.1250 | 9.0 | 8.5 | 0.0 | 9.0 | 3.5 | 3.0 |
| | 0.0625 | 9.0 | 7.0 | 0.0 | 8.0 | 3.0 | 2.0 |
| | 0.0313 | 8.0 | 4.5 | 0.0 | 7.5 | 2.5 | 1.0 |
| | | | | | | | |
| 120 | 0.2500 | 9.0 | 9.0 | 3.5 | 9.0 | 7.5 | 4.0 |
| | 0.1250 | 9.0 | 3.5 | 0.0 | 9.0 | 2.0 | 3.0 |
| | 0.0625 | 8.0 | 2.5 | 0.0 | 9.0 | 0.0 | 1.0 |
| | 0.0313 | 7.0 | 2.0 | 0.0 | 8.5 | 0.0 | 2.0 |
| | | | | | | | |
| 121 | 0.2500 | 6.0 | 8.0 | 5.0 | 9.0 | 6.0 | 7.0 |
| | 0.1250 | 6.0 | 8.0 | 5.0 | 9.0 | 4.0 | 6.0 |
| | 0.0625 | 5.0 | 4.0 | | 9.0 | 4.0 | 4.0 |
| | 0.0313 | 4.0 | 4.0 | 2.0 | 9.0 | 0.0 | 3.0 |
| | | | | | | | |
| 122 | 0.2500 | 7.0 | 8.0 | 3.0 | 9.0 | 6.0 | 3.0 |
| | 0.1250 | 7.0 | 7.0 | | 9.0 | 5.0 | 3.0 |
| | 0.0625 | 7.0 | 7.0 | | 9.0 | 3.0 | 2.0 |
| | 0.0313 | 6.0 | 4.0 | | 8.0 | 2.0 | 2.0 |
| | | | | | | | |
| 123 | 0.2500 | 9.0 | 9.0 | 9.0 | 9.0 | 8.0 | 3.0 |
| | 0.1250 | 9.0 | 9.0 | 9.0 | 9.0 | 6.0 | 3.0 |
| | 0.0625 | 9.0 | 5.0 | 9.0 | 7.0 | 6.0 | 2.0 |
| | 0.0313 | 7.0 | 6.0 | 5.0 | 6.0 | 5.0 | 2.0 |
| | | | | | | | |
| 124 | 0.2500 | 8.0 | 6.0 | 3.0 | 7.0 | 5.0 | 5.0 |
| | 0.1250 | 9.0 | 0.0 | 2.0 | 0.0 | 4.0 | 2.0 |
| | 0.0625 | 5.0 | 0.0 | 0.0 | 0.0 | 0.0 | 0.0 |
| | 0.0313 | 5.0 | 0.0 | 0.0 | 0.0 | 0.0 | 0.0 |
| | | | | | | | |
| 125 | 0.2500 | 7.0 | 9.0 | 9.0 | 9.0 | 9.0 | 7.0 |
| | 0.1250 | 7.0 | 7.0 | 9.0 | 7.0 | 4.0 | 3.0 |
| | 0.0625 | 4.0 | 6.0 | 4.0 | 7.0 | 4.0 | 2.0 |
| | 0.0313 | 2.0 | 3.0 | 2.0 | 5.0 | 3.0 | 0.0 |
| | | | | | | | |
| 126 | 0.2500 | 7.0 | 2.0 | 0.0 | 7.0 | 3.0 | 4.0 |
| | 0.1250 | 6.0 | 0.0 | 0.0 | 6.0 | 3.0 | 3.0 |
| | 0.0625 | 3.0 | 0.0 | 0.0 | 4.0 | 0.0 | 3.0 |
| | 0.0313 | 0.0 | 0.0 | 0.0 | 3.0 | 0.0 | 0.0 |
| | | | | | | | |
| 127 | 0.2500 | 9.0 | 4.0 | 0.0 | 7.0 | 3.0 | 4.0 |
| | 0.1250 | 9.0 | 4.0 | 0.0 | 5.0 | 0.0 | 4.0 |
| | 0.0625 | 7.0 | 2.0 | 0.0 | 3.0 | 0.0 | 2.0 |
| | 0.0313 | 4.0 | 2.0 | 0.0 | 3.0 | 0.0 | 0.0 |
| | | | | | | | |
| 128 | 0.2500 | 9.0 | 4.0 | 3.0 | 9.0 | 6.0 | 3.0 |
| | 0.1250 | 7.0 | 4.0 | | 9.0 | 4.0 | 0.0 |
| | 0.0625 | 7.0 | 4.0 | 0.0 | 7.0 | 3.0 | 0.0 |
| | 0.0313 | 5.0 | 0.0 | | 6.0 | 0.0 | 0.0 |
| | | | | | | | |
| 129 | 0.2500 | 9.0 | 9.0 | 9.0 | 9.0 | 7.0 | 2.0 |
| | 0.1250 | 9.0 | 7.0 | 7.0 | 9.0 | 2.0 | 0.0 |
| | 0.0625 | 9.0 | 5.0 | | 7.0 | 0.0 | 0.0 |
| | 0.0313 | 9.0 | 4.0 | | 6.0 | 0.0 | 0.0 |
| | | | | | | | |
| 130 | 0.2500 | 4.0 | 6.0 | 7.0 | 6.0 | 3.0 | 6.0 |
| | 0.1250 | 3.0 | 4.0 | 0.0 | 6.0 | 0.0 | 4.0 |
| | 0.0625 | 2.0 | 2.0 | | 5.0 | 0.0 | 4.0 |
| | 0.0313 | 0.0 | 0.0 | | 5.0 | 0.0 | 4.0 |
| | | | | | | | |
| 131 | 0.2500 | 4.0 | 4.0 | 0.0 | 2.0 | 2.0 | 5.0 |
| | 0.1250 | 2.0 | 0.0 | 0.0 | 0.0 | 0.0 | 2.0 |
| | | | | | | | |
| 132 | 0.2500 | 6.0 | 3.0 | 0.0 | 9.0 | 0.0 | 4.0 |
| | 0.1250 | 4.0 | 0.0 | 0.0 | 9.0 | 0.0 | 4.0 |
| | 0.0625 | 4.0 | 0.0 | 0.0 | 9.0 | 0.0 | 2.0 |
| | 0.0313 | 3.0 | 0.0 | 0.0 | 6.0 | 0.0 | 0.0 |
| | | | | | | | |
| 133 | 0.2500 | 9.0 | 3.0 | 1.0 | 6.0 | 3.0 | 4.0 |
| | 0.1250 | 9.0 | 2.0 | 0.0 | 4.0 | 3.0 | 4.0 |
| | 0.0625 | 6.0 | 0.0 | 0.0 | 4.0 | 3.0 | 2.0 |
| | 0.0313 | 6.0 | 0.0 | 0.0 | 3.0 | 0.0 | 2.0 |
| | | | | | | | |
| 134 | 0.2500 | 2.0 | 3.0 | | 2.0 | 0.0 | 2.0 |
| | | | | | | | |
| 135 | 0.2500 | 9.0 | 7.0 | 0.0 | 8.0 | 6.0 | 0.0 |
| | 0.1250 | 7.0 | 7.0 | 0.0 | 9.0 | 5.0 | 0.0 |
| | 0.0625 | 7.0 | 6.0 | | 9.0 | 3.0 | 0.0 |
| | 0.0313 | 7.0 | 5.0 | | 6.0 | 0.0 | 0.0 |
| | | | | | | | |
| 136 | 0.2500 | 9.0 | 7.0 | 0.0 | 9.0 | 3.0 | 3.0 |
| | 0.1250 | 7.0 | 6.0 | 0.0 | 9.0 | 3.0 | 3.0 |
| | 0.0625 | 7.0 | 5.0 | 0.0 | 4.0 | 0.0 | 3.0 |
| | 0.0313 | 7.0 | 0.0 | 0.0 | 3.0 | 0.0 | 2.0 |
| | | | | | | | |
| 137 | 0.2500 | 5.0 | 4.0 | 0.0 | 8.0 | 3.0 | 4.0 |
| | 0.1250 | 3.0 | 2.0 | 0.0 | 5.0 | 0.0 | 1.0 |
| | 0.0625 | 3.0 | 2.0 | 0.0 | 5.0 | 0.0 | 0.0 |
| | 0.0313 | 3.0 | 3.0 | 0.0 | 4.0 | 0.0 | 0.0 |
| | | | | | | | |
| 138 | 0.2500 | 9.0 | 5.0 | 0.0 | 7.0 | 3.0 | 3.0 |
| | 0.1250 | 9.0 | 3.0 | 0.0 | 6.0 | 0.0 | 3.0 |
| | 0.0625 | 9.0 | 2.0 | 0.0 | 6.0 | 0.0 | 3.0 |
| | 0.0313 | 7.0 | 2.0 | 0.0 | 5.0 | 0.0 | 3.0 |
| | | | | | | | |
| 139 | 0.2500 | 7.0 | 6.0 | 0.0 | 7.0 | 0.0 | 2.0 |
| | 0.1250 | 7.0 | 5.0 | 0.0 | 7.0 | 0.0 | 0.0 |
| | 0.0625 | 7.0 | 4.0 | | 5.0 | 0.0 | 0.0 |
| | 0.0313 | 6.0 | 2.0 | | 4.0 | 0.0 | 0.0 |
| | | | | | | | |
| 140 | 0.2500 | 7.0 | 7.0 | 0.0 | 9.0 | 4.0 | 2.0 |
| | 0.1250 | 7.0 | 9.0 | 0.0 | 9.0 | 4.0 | 0.0 |
| | 0.0625 | 5.0 | 7.0 | 0.0 | 7.0 | 3.0 | 0.0 |
| | 0.0313 | 5.0 | 4.0 | 0.0 | 4.0 | 2.0 | 0.0 |
| | | | | | | | |
| 141 | 0.2500 | 7.0 | 6.0 | 0.0 | 9.0 | 6.0 | 0.0 |
| | 0.1250 | 7.0 | 6.0 | 0.0 | 8.0 | 5.0 | 0.0 |
| | 0.0625 | 5.0 | 5.0 | 0.0 | 8.0 | 5.0 | 0.0 |
| | 0.0313 | 5.0 | 4.0 | 0.0 | 7.0 | 4.0 | 0.0 |
| | | | | | | | |
| 142 | 0.2500 | 7.0 | 7.0 | 0.0 | 8.0 | 2.0 | 1.0 |
| | 0.1250 | 7.0 | 6.0 | 0.0 | 9.0 | 0.0 | 0.0 |
| | 0.0625 | 5.0 | 2.0 | | 7.0 | 0.0 | 0.0 |
| | 0.0313 | 4.0 | 0.0 | | 6.0 | 0.0 | 0.0 |
| | | | | | | | |
| 143 | 0.2500 | 9.0 | 4.0 | 0.0 | 6.0 | 5.0 | 3.0 |
| | 0.1250 | 9.0 | 4.0 | 0.0 | 6.0 | 0.0 | 3.0 |
| | 0.0625 | 7.0 | 3.0 | 0.0 | 6.0 | 0.0 | 3.0 |
| | 0.0313 | 5.0 | 0.0 | 0.0 | 5.0 | 0.0 | 1.0 |
| | | | | | | | |
| 144 | 0.2500 | 3.0 | 9.0 | 9.0 | 9.0 | 4.0 | 3.0 |
| | 0.1250 | 1.0 | 9.0 | 9.0 | 9.0 | 2.0 | 3.0 |
| | 0.0625 | 0.0 | 3.0 | 7.0 | 9.0 | 0.0 | 0.0 |
| | 0.0313 | 0.0 | 3.0 | 7.0 | 6.0 | 0.0 | 0.0 |
| | | | | | | | |
| 145 | 0.2500 | 7.0 | 9.0 | 9.0 | 9.0 | 5.0 | 2.0 |
| | 0.1250 | 7.0 | 7.0 | 9.0 | 9.0 | 5.0 | 0.0 |
| | 0.0625 | 6.0 | 5.0 | | 6.0 | 2.0 | 0.0 |
| | 0.0313 | 6.0 | 4.0 | | 4.0 | 0.0 | 0.0 |
| | | | | | | | |
| 146 | 0.2500 | 9.0 | 7.0 | 0.0 | 9.0 | 4.0 | 2.0 |
| | 0.1250 | 9.0 | 6.0 | 0.0 | 6.0 | 3.0 | 0.0 |
| | 0.0625 | 9.0 | 6.0 | 0.0 | 6.0 | 0.0 | 0.0 |
| | 0.0313 | 9.0 | 4.0 | 0.0 | 4.0 | 0.0 | 0.0 |
| | | | | | | | |
| 147 | 0.2500 | 9.0 | 7.0 | 0.0 | 9.0 | 2.0 | 5.0 |
| | 0.1250 | 9.0 | 7.0 | | 9.0 | 0.0 | 4.0 |
| | 0.0625 | 7.0 | 4.0 | | 9.0 | 0.0 | 3.0 |
| | 0.0313 | 5.0 | 4.0 | | 7.0 | 0.0 | 2.0 |
| | | | | | | | |
| 148 | 0.2500 | 9.0 | 7.0 | 5.0 | 9.0 | 4.0 | 0.0 |
| | 0.1250 | 9.0 | 6.0 | 0.0 | 9.0 | 4.0 | 0.0 |
| | 0.0625 | 7.0 | 4.0 | | 7.0 | 3.0 | 0.0 |
| | 0.0313 | 6.0 | 4.0 | 0.0 | 7.0 | 0.0 | 0.0 |
| | | | | | | | |
| 149 | 0.2500 | 7.0 | 4.0 | 0.0 | 7.0 | 3.0 | 0.0 |
| | 0.1250 | 6.0 | 7.0 | 0.0 | 7.0 | 3.0 | 0.0 |
| | 0.0625 | 3.0 | 4.0 | 0.0 | 7.0 | 2.0 | 0.0 |
| | 0.0313 | 2.0 | 3.0 | 0.0 | 5.0 | 0.0 | 0.0 |
| | | | | | | | |
| 150 | 0.2500 | 9.0 | 9.0 | 9.0 | 9.0 | 2.0 | 5.0 |
| | 0.1250 | 9.0 | 9.0 | 9.0 | 9.0 | 2.0 | 3.0 |
| | 0.0625 | 7.0 | 9.0 | | 9.0 | 2.0 | 2.0 |
| | 0.0313 | 6.0 | 9.0 | | 6.0 | 0.0 | 0.0 |
| | | | | | | | |
| 151 | 0.2500 | 9.0 | 8.0 | 7.0 | 9.0 | 6.0 | 9.0 |
| | 0.1250 | 9.0 | 6.0 | | 9.0 | 8.0 | 8.0 |
| | 0.0625 | 5.0 | 2.0 | 7.0 | 9.0 | 2.0 | 9.0 |
| | 0.0313 | 3.0 | 0.0 | | 6.0 | 0.0 | 6.0 |
| | | | | | | | |
| 152 | 0.2500 | 0.0 | 0.0 | 0.0 | 3.0 | 0.0 | 3.0 |
| | 0.1250 | 0.0 | 0.0 | 0.0 | 3.0 | 0.0 | 2.0 |
| | 0.0625 | 0.0 | 0.0 | 0.0 | 3.0 | 0.0 | 0.0 |
| | | | | | | | |
| 153 | 0.2500 | 0.0 | 3.0 | 0.0 | 5.0 | 0.0 | 4.0 |
| | 0.1250 | 0.0 | 0.0 | 0.0 | 4.0 | 0.0 | 2.0 |
| | 0.0625 | 0.0 | 0.0 | 0.0 | 3.0 | 0.0 | 2.0 |
| | | | | | | | |
| 154 | 0.2500 | 7.0 | 6.0 | 9.0 | 9.0 | 6.0 | 8.0 |
| | 0.1250 | 7.0 | 4.0 | 9.0 | 8.0 | 5.0 | 9.0 |
| | 0.0625 | 4.0 | 3.0 | 6.0 | 7.0 | 4.0 | 8.0 |
| | 0.0313 | 3.0 | 2.0 | 4.0 | 6.0 | 2.0 | 9.0 |
| | | | | | | | |
| 155 | 0.2500 | 4.0 | 0.0 | | 0.0 | 0.0 | 3.0 |
| | 0.1250 | 4.0 | 0.0 | 0.0 | 0.0 | 0.0 | 1.0 |
| | 0.0625 | 4.0 | 0.0 | 0.0 | 0.0 | 0.0 | 0.0 |
| | 0.0313 | 4.0 | 0.0 | 0.0 | 0.0 | 0.0 | 0.0 |
| | | | | | | | |
| 156 | 0.2500 | 0.0 | 0.0 | 0.0 | 0.0 | 0.0 | 3.0 |
| | | | | | | | |
| 157 | 0.2500 | 0.0 | 0.0 | | 0.0 | 0.0 | 0.0 |
| | | | | | | | |
| 158 | 0.2500 | 9.0 | 9.0 | 9.0 | 9.0 | 9.0 | 7.0 |
| | 0.1250 | 9.0 | 9.0 | 9.0 | 9.0 | 6.0 | 9.0 |
| | 0.0625 | 9.0 | 9.0 | 9.0 | 9.0 | 5.0 | 6.0 |
| | 0.0313 | 9.0 | 9.0 | 5.0 | 9.0 | 4.0 | 6.0 |
| | | | | | | | |
| 159 | 0.2500 | 7.0 | 4.0 | | 9.0 | 6.0 | 8.0 |
| | 0.1250 | 5.0 | 3.0 | 0.0 | 6.0 | 3.0 | 2.0 |
| | 0.0625 | 4.0 | 0.0 | 0.0 | 3.0 | 3.0 | 0.0 |
| | 0.0313 | 2.0 | 0.0 | | 0.0 | 0.0 | 0.0 |
| | | | | | | | |
| 160 | 0.2500 | 4.0 | 9.0 | 3.0 | 7.0 | 8.0 | 6.0 |
| | 0.1250 | 2.0 | 6.0 | 3.0 | 6.0 | 5.0 | 5.0 |
| | 0.0625 | 0.0 | 3.0 | 3.0 | 6.0 | 2.0 | 3.0 |
| | 0.0313 | 0.0 | 0.0 | 3.0 | 5.0 | 0.0 | 3.0 |
| | | | | | | | |
| 161 | 0.2500 | 4.0 | 6.0 | 3.0 | 9.0 | 9.0 | 4.0 |
| | 0.1250 | 2.0 | 5.0 | 0.0 | 7.0 | 6.0 | 3.0 |
| | 0.0625 | 0.0 | 4.0 | 0.0 | 7.0 | | 2.0 |
| | 0.0313 | 0.0 | 3.0 | 0.0 | 7.0 | 2.0 | 0.0 |
| | | | | | | | |
| 162 | 0.2500 | 8.0 | 9.0 | 9.0 | 9.0 | 3.0 | 7.0 |
| | 0.1250 | 8.0 | 6.0 | 9.0 | 7.0 | 3.0 | 6.0 |
| | 0.0625 | 7.0 | 6.0 | | 7.0 | 3.0 | 3.0 |
| | 0.0313 | 6.0 | 5.0 | | 7.0 | 0.0 | 2.0 |
| | | | | | | | |
| 163 | 0.2500 | 9.0 | 9.0 | 9.0 | 9.0 | 7.0 | 7.0 |
| | 0.1250 | 9.0 | 9.0 | 9.0 | 9.0 | 6.0 | 6.0 |
| | 0.0625 | 9.0 | 7.0 | | 9.0 | 3.0 | 3.0 |
| | 0.0313 | 9.0 | 6.0 | | 9.0 | 2.0 | 2.0 |
| | | | | | | | |
| 164 | 0.2500 | 9.0 | 9.0 | 7.0 | 9.0 | 6.0 | 6.0 |
| | 0.1250 | 9.0 | 6.0 | 3.0 | 9.0 | 5.0 | 3.0 |
| | 0.0625 | 7.0 | 5.0 | 0.0 | 9.0 | 4.0 | 2.0 |
| | 0.0313 | 6.0 | 3.0 | 0.0 | 8.0 | 4.0 | 2.0 |
| | | | | | | | |
| 165 | 0.2500 | 9.0 | 9.0 | 3.0 | 9.0 | 7.0 | 3.0 |
| | 0.1250 | 9.0 | 9.0 | 0.0 | 9.0 | 4.0 | 1.0 |
| | 0.0625 | 9.0 | 7.0 | | 9.0 | 5.0 | 1.0 |
| | 0.0313 | 9.0 | 6.0 | 0.0 | 9.0 | 4.0 | 0.0 |
| | | | | | | | |
| 166 | 0.2500 | 4.0 | 9.0 | 0.0 | 9.0 | 6.0 | 3.0 |
| | 0.1250 | 2.0 | 6.0 | 0.0 | 9.0 | 5.0 | 3.0 |
| | 0.0625 | 2.0 | 2.0 | 0.0 | 9.0 | 4.0 | 0.0 |
| | 0.0313 | 2.0 | 2.0 | 0.0 | 9.0 | 2.0 | 0.0 |
| | | | | | | | |
| 167 | 0.2500 | 9.0 | 9.0 | 5.0 | 9.0 | 7.0 | 4.0 |
| | 0.1250 | 9.0 | 9.0 | 3.0 | 9.0 | 6.0 | 4.0 |
| | 0.0625 | 9.0 | 9.0 | 0.0 | 9.0 | 5.0 | 4.0 |
| | 0.0313 | 9.0 | 7.0 | 0.0 | 9.0 | 4.0 | 4.0 |
| | | | | | | | |
| 168 | 0.2500 | 0.0 | 0.0 | | 7.0 | 2.0 | 2.0 |
| | 0.1250 | 0.0 | 0.0 | 0.0 | 6.0 | 0.0 | 0.0 |
| | 0.0625 | 0.0 | 0.0 | 0.0 | 6.0 | 0.0 | 0.0 |
| | 0.0313 | 0.0 | 0.0 | 0.0 | 6.0 | 0.0 | 0.0 |
| | | | | | | | |
| 169 | 0.2500 | 2.0 | 2.0 | | 7.0 | 3.0 | 2.0 |
| | 0.1250 | 0.0 | 0.0 | 0.0 | 6.0 | 0.0 | 0.0 |
| | 0.0625 | 0.0 | 0.0 | | 6.0 | 0.0 | 0.0 |
| | 0.0313 | 0.0 | 0.0 | 0.0 | 5.0 | 0.0 | 0.0 |
| | | | | | | | |
| 170 | 0.2500 | 9.0 | 9.0 | | 9.0 | 9.0 | 9.0 |
| | 0.1250 | 9.0 | 9.0 | | 9.0 | 8.0 | 6.0 |
| | 0.0625 | 9.0 | 9.0 | | 9.0 | 9.0 | 8.0 |
| | 0.0313 | 9.0 | 9.0 | | 9.0 | 5.0 | 4.0 |
| | | | | | | | |
| 171 | 0.2500 | 9.0 | 9.0 | 9.0 | 9.0 | 9.0 | 7.0 |
| | 0.1250 | 9.0 | 9.0 | 9.0 | 9.0 | 8.0 | 6.0 |
| | 0.0625 | 9.0 | 9.0 | 9.0 | 9.0 | 7.0 | 6.0 |
| | 0.0313 | 8.0 | 3.0 | 9.0 | 9.0 | 6.0 | 5.0 |
| | | | | | | | |
| 172 | 0.2500 | 9.0 | 9.0 | 9.0 | 9.0 | 9.0 | 9.0 |
| | 0.1250 | 9.0 | 8.0 | 9.0 | 9.0 | 9.0 | 9.0 |
| | 0.0625 | 9.0 | 7.0 | 6.0 | 9.0 | 6.0 | 7.0 |
| | 0.0313 | 6.0 | 5.0 | | 9.0 | 6.0 | 6.0 |

### EXAMPLE 40

### Rice tolerance to post-transplant applications and postemergence weed control under flooded paddy conditions

Plastic containers containing weeds which are 3 to 5 cm tall and rice seedlings at the 1.5 to 2.5 leaf stage are flooded with water. The water is maintained at 1.5 to 3 cm above the soil surface for the duration of the experiment. Test compounds are applied as aqueous/ acetone mixtures 50/50 v/v directly into the flood water to provide the equivalent of about 0.0313 to 0.500 kg/ha of active ingredient. The treated containers are placed on greenhouse benches and cared for in accordance with conventional greenhouse procedures. Three to four weeks after treatment, the test is terminated and each container is examined and herbicidal effect rated according to the rating system provided in Example 37. The results are summarized in Table IV. The compounds evaluated are reported by compound number given in Example 37.

**TABLE IV**

| **Paddy Conditions - Post Transplant Rice** **Postemergence Weeds** | | | | | | | |
|---|---|---|---|---|---|---|---|
| **Compound** **Number** | **Rate** **(kg/ha)** | **E** **C** **H** **O** **R** **C** | **C** **Y** **P** **I** **R** | **C** **Y** **P** **S** **E** | **M** **O** **O** **V** **A** | **S** **A** **G** **P** **Y** | **O** **R** **Y** **S** **A** **T** |
| 1 | 0.5000 | 5.5 | 8.5 | 4.0 | 5.5 | 0.0 | 4.0 |
| | 0.2500 | 3.5 | 6.5 | 1.0 | 5.0 | 0.0 | 3.0 |
| | 0.1250 | 3.0 | 5.5 | 0.0 | 4.5 | 0.0 | 2.5 |
| | 0.0625 | 2.0 | 3.5 | 0.0 | 4.5 | 0.0 | 2.5 |
| | 0.0313 | 1.0 | 1.0 | 0.0 | 4.0 | 0.0 | 1.5 |
| | | | | | | | |
| 2 | 0.5000 | 4.5 | 4.0 | 0.0 | 9.0 | 1.0 | 2.5 |
| | 0.2500 | 4.0 | 1.0 | 0.0 | 9.0 | 0.0 | 1.5 |
| | 0.1250 | 3.5 | 0.0 | 0.0 | 5.5 | 0.0 | 0.5 |
| | 0.0625 | 1.0 | 0.0 | 0.0 | 4.5 | 0.0 | 0.0 |
| | 0.0313 | 1.0 | 0.0 | 0.0 | 2.0 | 0.0 | 0.0 |
| | | | | | | | |
| 3 | 0.5000 | 2.0 | 0.5 | 0.0 | 5.5 | 0.0 | 2.0 |
| | 0.2500 | 1.0 | 0.0 | 0.0 | 4.5 | 0.0 | 1.0 |
| | 0.1250 | 0.0 | 0.0 | 0.0 | 4.5 | 0.0 | 1.0 |
| | 0.0625 | 0.0 | 0.0 | 0.0 | 1.0 | 0.0 | 0.0 |
| | | | | | | | |
| 4 | 0.5000 | 5.0 | 9.0 | 0.0 | 8.0 | 0.0 | 3.0 |
| | 0.2500 | 4.0 | 6.5 | 0.0 | 5.5 | 0.0 | 2.5 |
| | 0.1250 | 3.5 | 4.5 | 0.0 | 4.0 | 0.0 | 2.0 |
| | 0.0625 | 2.0 | 2.0 | 0.0 | 3.5 | 0.0 | 1.5 |
| | 0.0313 | 1.0 | 0.0 | 0.0 | 1.0 | 0.0 | 0.0 |
| | | | | | | | |
| 5 | 0.5000 | 2.0 | 0.0 | 0.5 | 4.0 | 0.0 | 1.0 |
| | 0.2500 | 1.0 | 0.0 | 0.0 | 2.0 | 0.0 | 0.5 |
| | 0.1250 | 0.5 | 0.0 | 0.0 | 1.0 | 0.0 | 0.5 |
| | | | | | | | |
| 6 | 0.5000 | 3.5 | 3.5 | 0.0 | 4.5 | 0.0 | 0.0 |
| | 0.2500 | 3.5 | 3.0 | 0.0 | 4.5 | 0.0 | 0.0 |
| | 0.1250 | 1.0 | 2.0 | 0.0 | 4.0 | 0.0 | 0.0 |
| | 0.0625 | 0.0 | 1.0 | 0.0 | 4.0 | 0.0 | 0.0 |
| | 0.0313 | 0.0 | 0.0 | 0.0 | 1.0 | 0.0 | 0.0 |
| | | | | | | | |
| 7 | 0.2500 | 9.0 | 9.0 | 8.0 | 9.0 | 8.5 | 9.0 |
| | 0.1250 | 9.0 | 9.0 | 7.5 | 9.0 | 7.5 | 7.0 |
| | 0.0625 | 8.5 | 9.0 | 7.0 | 9.0 | 5.5 | 6.5 |
| | 0.0313 | 7.0 | 8.5 | 3.0 | 9.0 | 3.0 | 5.0 |
| | | | | | | | |
| 8 | 0.2500 | 8.5 | 4.0 | 0.0 | 9.0 | 0.0 | 6.0 |
| | 0.1250 | 7.5 | 0.0 | 0.0 | 9.0 | 0.0 | 5.0 |
| | 0.0625 | 6.5 | 0.0 | 0.0 | 9.0 | 0.0 | 3.5 |
| | 0.0313 | 6.5 | 0.0 | 0.0 | 9.0 | 0.0 | 2.5 |
| | | | | | | | |
| 9 | 0.2500 | 7.0 | 6.0 | 7.0 | 9.0 | 2.0 | 9.0 |
| | 0.1250 | 4.0 | 4.0 | 2.0 | 9.0 | 1.0 | 9.0 |
| | 0.0625 | 2.0 | 2.0 | 0.0 | 9.0 | 0.0 | 8.0 |
| | 0.0313 | 0.0 | 0.0 | 0.0 | 9.0 | 0.0 | 7.0 |
| | | | | | | | |
| 10 | 0.2500 | 2.0 | 4.0 | 0.0 | 9.0 | 0.0 | 7.0 |
| | 0.1250 | 2.0 | 2.0 | 0.0 | 9.0 | 0.0 | 6.0 |
| | 0.0625 | 1.0 | 0.0 | 0.0 | 9.0 | 0.0 | 4.0 |
| | 0.0313 | 0.0 | 0.0 | 0.0 | 9.0 | 0.0 | 3.0 |
| | | | | | | | |
| 11 | 0.2500 | 3.0 | 0.0 | 0.0 | 7.0 | 0.0 | 3.0 |
| | 0.1250 | 2.0 | 0.0 | 0.0 | 4.0 | 0.0 | 3.0 |
| | 0.0625 | 2.0 | 0.0 | 0.0 | 1.0 | 0.0 | 2.0 |
| | 0.0313 | 1.0 | 0.0 | 0.0 | 0.0 | 0.0 | 2.0 |
| | | | | | | | |
| 12 | 0.2500 | 9.0 | 0.0 | 6.0 | 9.0 | 0.0 | 7.0 |
| | 0.1250 | 8.5 | 1.0 | 1.0 | 9.0 | 4.0 | 5.5 |
| | 0.0625 | 9.0 | 0.0 | 3.0 | 9.0 | 1.0 | 3.5 |
| | 0.0313 | 5.0 | 0.0 | 1.0 | 9.0 | 0.0 | 3.0 |
| | | | | | | | |
| 13 | 0.2500 | 9.0 | 1.0 | 0.0 | 9.0 | 0.0 | 5.0 |
| | 0.1250 | 8.0 | 0.5 | 0.0 | 9.0 | 0.0 | 3.0 |
| | 0.0625 | 7.0 | 0.0 | 0.0 | 9.0 | 0.0 | 2.5 |
| | 0.0313 | 5.5 | 0.0 | 0.0 | 9.0 | 0.0 | 1.5 |
| | | | | | | | |
| 14 | 0.2500 | 7.0 | 4.5 | 0.0 | 4.5 | 0.0 | 0.0 |
| | 0.1250 | 3.8 | 6.8 | 1.5 | 6.8 | 0.0 | 0.5 |
| | 0.0625 | 2.3 | 5.8 | 0.0 | 6.8 | 0.0 | 0.3 |
| | 0.0313 | 0.8 | 4.0 | 0.0 | 6.8 | 0.0 | 0.0 |
| | | | | | | | |
| 15 | 0.2500 | 9.0 | 7.0 | 0.0 | 9.0 | 0.0 | 3.0 |
| | 0.1250 | 9.0 | 6.0 | 0.0 | 9.0 | 0.0 | 3.0 |
| | 0.0625 | 9.0 | 2.0 | 0.0 | 9.0 | 0.0 | 2.0 |
| | 0.0313 | 6.0 | 2.0 | 0.0 | 9.0 | 0.0 | 0.0 |
| | | | | | | | |
| 16 | 0.2500 | 0.0 | 9.0 | 0.0 | 3.0 | 0.0 | 0.0 |
| | 0.1250 | 0.0 | 4.0 | 0.0 | 9.0 | 0.0 | 0.0 |
| | 0.0625 | 0.0 | 0.0 | 0.0 | 9.0 | 0.0 | 0.0 |
| | 0.0313 | 0.0 | 0.0 | 0.0 | 9.0 | 0.0 | 0.0 |
| | | | | | | | |
| 17 | 0.2500 | 9.0 | 9.0 | 9.0 | 9.0 | 4.0 | 9.0 |
| | 0.1250 | 9.0 | 6.0 | 9.0 | 9.0 | 4.0 | 9.0 |
| | 0.0625 | 9.0 | 0.0 | 9.0 | 9.0 | 0.0 | 6.0 |
| | 0.0313 | 4.0 | 0.0 | 9.0 | 9.0 | 0.0 | 4.0 |
| | | | | | | | |
| 18 | 0.4630 | 7.0 | 0.0 | 4.0 | 9.0 | 6.0 | 3.0 |
| | 0.2310 | 7.0 | 0.0 | 0.0 | 9.0 | 0.0 | 3.0 |
| | 0.1160 | 7.0 | 0.0 | 0.0 | 9.0 | 0.0 | 3.0 |
| | | | | | | | |
| 19 | 0.4630 | 9.0 | 0.0 | 4.0 | 9.0 | 3.0 | 3.0 |
| | 0.2310 | 7.0 | 0.0 | 4.0 | 9.0 | 3.0 | 3.0 |
| | 0.1160 | 7.0 | 0.0 | 4.0 | 9.0 | 0.0 | 3.0 |
| | | | | | | | |
| 20 | 0.4630 | 7.0 | 0.0 | 5.0 | 9.0 | 3.0 | 2.0 |
| | 0.2310 | 7.0 | 0.0 | 5.0 | 9.0 | 0.0 | 2.0 |
| | 0.1160 | 7.0 | 0.0 | 5.0 | 9.0 | 0.0 | 2.0 |
| | | | | | | | |
| 21 | 0.4630 | 5.0 | 0.0 | 0.0 | 0.0 | 0.0 | 0.0 |
| | 0.2310 | 4.0 | 0.0 | 0.0 | 0.0 | 0.0 | 0.0 |
| | 0.1160 | 2.0 | 0.0 | 0.0 | 0.0 | 0.0 | 0.0 |
| | | | | | | | |
| 22 | 0.4630 | 9.0 | 5.0 | 4.0 | 9.0 | 5.0 | 7.0 |
| | 0.2310 | 9.0 | 0.0 | 4.0 | 9.0 | 4.0 | 6.0 |
| | 0.1160 | 6.0 | 0.0 | 4.0 | 9.0 | 4.0 | 3.0 |
| | | | | | | | |
| 23 | 0.4630 | 4.0 | 0.0 | 0.0 | 9.0 | 3.0 | 5.0 |
| | 0.2310 | 4.0 | 0.0 | 0.0 | 9.0 | 0.0 | 4.0 |
| | 0.1160 | 4.0 | 0.0 | 0.0 | 9.0 | 0.0 | 4.0 |
| | | | | | | | |
| 24 | 0.2500 | 9.0 | 0.0 | | 9.0 | 0.0 | 0.0 |
| | 0.1250 | 9.0 | 0.0 | | 9.0 | 0.0 | 0.0 |
| | 0.0625 | 9.0 | 0.0 | | 9.0 | 0.0 | 0.0 |
| | 0.0313 | 4.0 | 0.0 | | 0.0 | 0.0 | 0.0 |
| | | | | | | | |
| 25 | 0.2500 | 9.0 | 0.0 | | 9.0 | 5.0 | 5.0 |
| | 0.1250 | 7.0 | 0.0 | | 9.0 | 5.0 | 4.0 |
| | 0.0625 | 6.0 | 0.0 | | 9.0 | 0.0 | 4.0 |
| | 0.0313 | 4.0 | 0.0 | | 9.0 | 0.0 | 3.0 |
| | | | | | | | |
| 26 | 0.2500 | 9.0 | 9.0 | | 9.0 | 7.0 | 2.0 |
| | 0.1250 | 9.0 | 7.0 | | 9.0 | 5.0 | 1.0 |
| | 0.0625 | 9.0 | 4.0 | | 9.0 | 4.0 | 0.0 |
| | 0.0313 | 7.0 | 2.0 | | 9.0 | 0.0 | 0.0 |
| | | | | | | | |
| 27 | 0.2500 | 9.0 | 9.0 | 9.0 | 9.0 | 4.0 | 4.0 |
| | 0.1250 | 9.0 | 6.0 | 0.0 | 9.0 | 4.0 | 4.0 |
| | 0.0625 | 7.0 | 4.0 | 0.0 | 9.0 | 0.0 | 2.0 |
| | 0.0313 | 6.0 | 3.0 | 0.0 | 9.0 | 0.0 | 1.0 |
| | | | | | | | |
| 28 | 0.2500 | 9.0 | 9.0 | 0.0 | 9.0 | 0.0 | 1.0 |
| | 0.1250 | 7.0 | 9.0 | 0.0 | 9.0 | 0.0 | 0.0 |
| | 0.0625 | 4.0 | 9.0 | 0.0 | 9.0 | 0.0 | 0.0 |
| | 0.0313 | 0.0 | 9.0 | 0.0 | 9.0 | 0.0 | 0.0 |
| | | | | | | | |
| 29 | 0.2500 | 7.0 | 0.0 | 0.0 | 0.0 | 0.0 | 3.0 |
| | 0.1250 | 7.0 | 0.0 | 0.0 | 0.0 | 0.0 | 2.0 |
| | 0.0625 | 7.0 | 0.0 | 0.0 | 0.0 | 0.0 | 1.0 |
| | 0.0313 | 4.0 | 0.0 | 0.0 | 0.0 | 0.0 | 0.0 |
| | | | | | | | |
| 30 | 0.2500 | 0.0 | 4.5 | 0.0 | 4.5 | 2.0 | 0.0 |
| | 0.1250 | 0.0 | 2.0 | 0.0 | 1.5 | 0.0 | 0.0 |
| | | | | | | | |
| 31 | 0.2500 | 5.0 | 2.5 | 0.0 | 2.5 | 0.0 | 0.0 |
| | 0.1250 | 2.0 | 0.0 | 0.0 | 0.0 | 0.0 | 0.0 |
| | 0.0625 | 1.0 | 0.0 | 0.0 | 0.0 | 0.0 | 0.0 |
| | | | | | | | |
| 32 | 0.2500 | 6.0 | 0.0 | 0.0 | | 0.0 | 0.0 |
| | 0.1250 | 3.0 | 0.0 | 0.0 | | 0.0 | 0.0 |
| | | | | | | | |
| 33 | 0.2500 | 0.0 | 0.0 | 0.0 | | 0.0 | 0.0 |
| | | | | | | | |
| 34 | 0.2500 | 9.0 | 0.0 | 0.0 | | 0.0 | 6.0 |
| | 0.1250 | 4.0 | 0.0 | 0.0 | | 0.0 | 4.0 |
| | 0.0625 | 4.0 | 0.0 | 0.0 | | 0.0 | 3.0 |
| | 0.0313 | 3.0 | 0.0 | 0.0 | | 0.0 | 3.0 |
| | | | | | | | |
| 35 | 0.2500 | 6.0 | 0.0 | 0.0 | | 3.0 | 3.0 |
| | 0.1250 | 3.0 | 0.0 | 0.0 | | 0.0 | 2.0 |
| | | | | | | | |
| 36 | 0.2500 | 9.0 | 9.0 | 4.0 | | 9.0 | 5.0 |
| | 0.1250 | 7.0 | 5.0 | 2.0 | | 3.0 | 3.0 |
| | 0.0625 | 6.0 | 5.0 | 0.0 | | 0.0 | 3.0 |
| | 0.0313 | 4.0 | 0.0 | 0.0 | | 0.0 | 3.0 |
| | | | | | | | |
| 37 | 0.2500 | 7.0 | 0.0 | 0.0 | | 3.0 | 3.0 |
| | 0.1250 | 7.0 | 0.0 | 0.0 | | 0.0 | 0.0 |
| | 0.0625 | 6.0 | 0.0 | 0.0 | | 0.0 | 0.0 |
| | 0.0313 | 4.0 | 0.0 | 0.0 | | 0.0 | 0.0 |
| | | | | | | | |
| 38 | 0.2500 | 7.0 | 5.0 | 3.0 | 9.0 | 9.0 | 7.0 |
| | 0.1250 | 4.0 | 5.0 | 0.0 | 9.0 | 4.0 | 5.0 |
| | 0.0625 | 2.0 | 3.0 | 0.0 | 9.0 | 3.0 | 5.0 |
| | 0.0313 | 0.0 | 3.0 | 0.0 | 9.0 | 0.0 | 2.0 |
| | | | | | | | |
| 39 | 0.2500 | 3.0 | 0.0 | 0.0 | 9.0 | 4.0 | 3.0 |
| | 0.1250 | 2.0 | 0.0 | 0.0 | 9.0 | 0.0 | 2.0 |
| | 0.0625 | 0.0 | 0.0 | 0.0 | 9.0 | 0.0 | 0.0 |
| | 0.0313 | 0.0 | 0.0 | 0.0 | 9.0 | 0.0 | 0.0 |
| | | | | | | | |
| 40 | 0.2500 | 9.0 | 0.0 | 6.0 | 9.0 | 0.0 | 3.0 |
| | 0.1250 | 5.0 | 0.0 | 0.0 | 9.0 | 0.0 | 3.0 |
| | 0.0625 | 5.0 | 0.0 | 0.0 | 9.0 | 0.0 | 3.0 |
| | 0.0313 | 5.0 | 0.0 | 0.0 | 9.0 | 0.0 | 2.0 |
| | | | | | | | |
| 41 | 0.2500 | 7.0 | 4.0 | 7.0 | 9.0 | 6.0 | 5.0 |
| | 0.1250 | 6.0 | 0.0 | 2.0 | 9.0 | 0.0 | 5.0 |
| | 0.0625 | 4.0 | 0.0 | 0.0 | 9.0 | 0.0 | 5.0 |
| | 0.0313 | 3.0 | 0.0 | 0.0 | 6.0 | 0.0 | 4.0 |
| | | | | | | | |
| 42 | 0.2500 | 6.0 | 0.0 | 0.0 | 9.0 | 3.0 | 0.0 |
| | 0.1250 | 5.0 | 0.0 | 0.0 | 9.0 | 0.0 | 0.0 |
| | 0.0625 | 5.0 | 0.0 | 0.0 | 9.0 | 0.0 | 0.0 |
| | 0.0313 | 5.0 | 0.0 | 0.0 | 7.0 | 0.0 | 0.0 |
| | | | | | | | |
| 43 | 0.2500 | 9.0 | 5.0 | 9.0 | 9.0 | 9.0 | 6.0 |
| | 0.1250 | 7.0 | 4.0 | 8.0 | 9.0 | 7.0 | 4.0 |
| | 0.0625 | 6.0 | 2.0 | 5.0 | 9.0 | 7.0 | 3.0 |
| | 0.0313 | 5.0 | 0.0 | 5.0 | 9.0 | 7.0 | 2.0 |
| | | | | | | | |
| 44 | 0.2500 | 8.0 | 9.0 | 0.0 | 9.0 | | 5.0 |
| | 0.1250 | 7.0 | 6.0 | 0.0 | 7.0 | | 3.0 |
| | 0.0625 | 6.0 | 6.0 | 0.0 | 4.0 | | 3.0 |
| | 0.0313 | 6.0 | 5.0 | 0.0 | 4.0 | | 0.0 |
| | | | | | | | |
| 45 | 0.2500 | 7.0 | 7.0 | 0.0 | 9.0 | 4.0 | 3.0 |
| | 0.1250 | 7.0 | 7.0 | | 9.0 | 0.0 | 2.0 |
| | 0.0625 | 4.0 | 3.0 | 0.0 | 7.0 | 0.0 | 2.0 |
| | 0.0313 | 3.0 | 0.0 | 0.0 | 7.0 | 0.0 | 3.0 |
| | | | | | | | |
| 46 | 0.2500 | 4.0 | 0.0 | 6.0 | 7.0 | 3.0 | 3.0 |
| | 0.1250 | 0.0 | 0.0 | 0.0 | 6.0 | 0.0 | 2.0 |
| | 0.0625 | 0.0 | 0.0 | 0.0 | 4.0 | 0.0 | 2.0 |
| | 0.0313 | 0.0 | 0.0 | 0.0 | 3.0 | 0.0 | 0.0 |
| | | | | | | | |
| 47 | 0.2500 | 7.0 | 6.0 | 0.0 | 9.0 | 7.0 | 3.0 |
| | 0.1250 | 4.0 | 4.0 | 0.0 | 9.0 | 4.0 | 0.0 |
| | 0.0625 | 2.0 | 0.0 | 0.0 | 6.0 | 0.0 | 0.0 |
| | 0.0313 | 0.0 | 0.0 | 0.0 | 5.0 | 0.0 | 0.0 |
| | | | | | | | |
| 48 | 0.2500 | 6.0 | 5.0 | 0.0 | 6.0 | 6.0 | 6.0 |
| | 0.1250 | 3.0 | 2.0 | 0.0 | 3.0 | 3.0 | 4.0 |
| | | | | | | | |
| 49 | 0.2500 | 7.0 | 7.0 | 4.0 | 9.0 | 7.0 | 7.0 |
| | 0.1250 | 5.0 | 2.0 | | 9.0 | 6.0 | 7.0 |
| | 0.0625 | 2.0 | 0.0 | 3.0 | 9.0 | 4.0 | 5.0 |
| | 0.0313 | 0.0 | 0.0 | 2.0 | 9.0 | 2.0 | 5.0 |
| | | | | | | | |
| 50 | 0.2500 | 7.0 | 0.0 | 0.0 | 7.0 | 3.0 | 1.0 |
| | 0.1250 | 7.0 | 0.0 | 0.0 | 3.0 | 3.0 | 0.0 |
| | 0.0625 | 7.0 | 0.0 | 0.0 | 0.0 | 2.0 | 0.0 |
| | 0.0313 | 4.0 | 0.0 | 0.0 | 0.0 | 2.0 | 0.0 |
| | | | | | | | |
| 51 | 0.2500 | 0.0 | 6.0 | 0.0 | 4.0 | 0.0 | 0.0 |
| | 0.1250 | 0.0 | 3.0 | 0.0 | 4.0 | 0.0 | 0.0 |
| | 0.0625 | 0.0 | 2.0 | 0.0 | 3.0 | 0.0 | 0.0 |
| | | | | | | | |
| 52 | 0.2500 | 0.0 | 2.0 | 0.0 | 4.0 | 0.0 | 0.0 |
| | | | | | | | |
| 53 | 0.2500 | 3.0 | 9.0 | 0.0 | 9.0 | 0.0 | 3.0 |
| | 0.1250 | 2.0 | 9.0 | 0.0 | 9.0 | 0.0 | 2.0 |
| | 0.0625 | 0.0 | 2.0 | 0.0 | 2.0 | 0.0 | 0.0 |
| | 0.0313 | 0.0 | 2.0 | 0.0 | 2.0 | 0.0 | 0.0 |
| | | | | | | | |
| 54 | 0.2500 | 9.0 | 4.0 | 4.0 | 6.0 | 2.0 | 4.0 |
| | 0.1250 | 7.0 | 4.0 | 0.0 | 6.0 | 0.0 | 3.0 |
| | 0.0625 | 5.0 | 4.0 | 0.0 | 0.0 | 0.0 | 2.0 |
| | 0.0313 | 5.0 | 0.0 | 0.0 | 0.0 | 0.0 | 0.0 |
| | | | | | | | |
| 55 | 0.2500 | 7.0 | 9.0 | 0.0 | 9.0 | 0.0 | 4.0 |
| | 0.1250 | 7.0 | 9.0 | 0.0 | 9.0 | 0.0 | 1.0 |
| | 0.0625 | 7.0 | 9.0 | 0.0 | 9.0 | 0.0 | 1.0 |
| | 0.0313 | 7.0 | 9.0 | 0.0 | 9.0 | 0.0 | 0.0 |
| | | | | | | | |
| 56 | 0.2500 | 5.0 | 0.0 | 0.0 | 9.0 | 0.0 | 2.0 |
| | 0.1250 | 3.0 | 0.0 | 0.0 | 9.0 | 0.0 | 0.0 |
| | 0.0625 | 0.0 | 0.0 | 0.0 | 6.0 | 0.0 | 0.0 |
| | 0.0313 | 0.0 | 0.0 | 0.0 | 6.0 | 0.0 | 0.0 |
| | | | | | | | |
| 57 | 0.2500 | 5.0 | 9.0 | 3.0 | 9.0 | 6.0 | 4.0 |
| | 0.1250 | 2.0 | 9.0 | 0.0 | 9.0 | 0.0 | 0.0 |
| | 0.0625 | 2.0 | 9.0 | 0.0 | 9.0 | 0.0 | 0.0 |
| | 0.0313 | 0.0 | 0.0 | 0.0 | 9.0 | 0.0 | 0.0 |
| | | | | | | | |
| 58 | 0.2500 | 9.0 | 3.0 | 5.0 | 9.0 | 4.0 | 5.0 |
| | 0.1250 | 7.0 | 2.0 | 5.0 | 3.0 | 0.0 | 4.0 |
| | 0.0625 | 7.0 | 0.0 | 5.0 | 0.0 | 0.0 | 4.0 |
| | 0.0313 | 7.0 | 0.0 | 5.0 | 0.0 | 0.0 | 4.0 |
| | | | | | | | |
| 59 | 0.2500 | 7.0 | 9.0 | 7.0 | 9.0 | 3.0 | 2.0 |
| | 0.1250 | 5.0 | 0.0 | 0.0 | 0.0 | 0.0 | 2.0 |
| | 0.0625 | 4.0 | 0.0 | 0.0 | 0.0 | 0.0 | 0.0 |
| | | | | | | | |
| 60 | 0.2500 | 9.0 | 6.0 | 4.0 | 9.0 | 9.0 | 5.0 |
| | 0.1250 | 9.0 | 6.0 | 4.0 | 9.0 | 9.0 | 5.0 |
| | 0.0625 | 9.0 | 3.0 | 2.0 | 9.0 | 4.0 | 4.0 |
| | 0.0313 | 7.0 | 3.0 | 2.0 | 9.0 | 3.0 | 4.0 |
| | | | | | | | |
| 61 | 0.2500 | 0.0 | 0.0 | 0.0 | 0.0 | 0.0 | 0.0 |
| | | | | | | | |
| 62 | 0.2500 | 5.0 | 9.0 | 0.0 | 9.0 | 6.0 | 4.0 |
| | 0.1250 | 2.0 | 9.0 | 0.0 | 9.0 | 3.0 | 3.0 |
| | 0.0625 | 0.0 | 2.0 | 0.0 | 9.0 | 0.0 | 3.0 |
| | 0.0313 | 0.0 | 0.0 | 0.0 | 4.0 | 0.0 | 2.0 |
| | | | | | | | |
| 63 | 0.2500 | 5.0 | 7.0 | 0.0 | 9.0 | 9.0 | 3.0 |
| | 0.1250 | 5.0 | 7.0 | 0.0 | 9.0 | 9.0 | 3.0 |
| | 0.0625 | 0.0 | 6.0 | 0.0 | 9.0 | 7.0 | 3.0 |
| | 0.0313 | 0.0 | 2.0 | 0.0 | 9.0 | 4.0 | 3.0 |
| | | | | | | | |
| 64 | 0.2500 | 9.0 | 7.0 | 0.0 | 9.0 | 8.0 | 6.0 |
| | 0.1250 | 6.0 | 2.0 | 0.0 | 9.0 | 5.0 | 5.0 |
| | 0.0625 | 6.0 | 2.0 | 0.0 | 9.0 | 3.0 | 4.0 |
| | 0.0313 | 5.0 | 0.0 | 0.0 | 7.0 | 0.0 | 3.0 |
| | | | | | | | |
| 65 | 0.2500 | 7.0 | 8.0 | 0.0 | 9.0 | 7.0 | 6.0 |
| | 0.1250 | 6.0 | 7.0 | 0.0 | 9.0 | 5.0 | 6.0 |
| | 0.0625 | 5.0 | 7.0 | 0.0 | 9.0 | 4.0 | 3.0 |
| | 0.0313 | 5.0 | 6.0 | 0.0 | 9.0 | 2.0 | 2.0 |
| | | | | | | | |
| 66 | 0.2500 | 9.0 | 7.0 | 4.0 | 9.0 | 4.0 | 3.0 |
| | 0.1250 | 9.0 | 7.0 | 4.0 | 9.0 | 4.0 | 3.0 |
| | 0.0625 | 7.0 | 6.0 | 0.0 | 9.0 | 4.0 | 2.0 |
| | 0.0313 | 7.0 | 6.0 | 0.0 | 9.0 | 3.0 | 2.0 |
| | | | | | | | |
| 67 | 0.2500 | 5.0 | 8.0 | 3.0 | 9.0 | 6.0 | 6.0 |
| | 0.1250 | 5.0 | 5.0 | 3.0 | 9.0 | 4.0 | 4.0 |
| | 0.0625 | 4.0 | 5.0 | 3.0 | 9.0 | 4.0 | 3.0 |
| | 0.0313 | 0.0 | 5.0 | 0.0 | 9.0 | 0.0 | 2.0 |
| | | | | | | | |
| 68 | 0.2500 | 7.0 | 8.0 | 0.0 | 9.0 | 3.0 | 4.0 |
| | 0.1250 | 7.0 | 5.0 | 0.0 | 9.0 | 0.0 | 2.0 |
| | 0.0625 | 5.0 | 0.0 | 0.0 | 9.0 | 0.0 | 0.0 |
| | 0.0313 | 0.0 | 0.0 | 0.0 | 3.0 | 0.0 | 0.0 |
| | | | | | | | |
| 69 | 0.2500 | 7.0 | 3.0 | 9.0 | | 3.0 | 3.0 |
| | 0.1250 | 5.0 | 3.0 | 9.0 | | 3.0 | 2.0 |
| | 0.0625 | 4.0 | 3.0 | 3.0 | | 0.0 | 2.0 |
| | 0.0313 | 0.0 | 3.0 | 3.0 | | 0.0 | 0.0 |
| | | | | | | | |
| 70 | 0.2500 | 0.0 | 2.0 | 3.0 | | 4.0 | 2.0 |
| | 0.1250 | 0.0 | 2.0 | 0.0 | | 4.0 | 2.0 |
| | 0.0625 | 0.0 | 2.0 | 0.0 | | 4.0 | 0.0 |
| | 0.0313 | 0.0 | 0.0 | 0.0 | | 4.0 | 0.0 |
| | | | | | | | |
| 71 | 0.2500 | 7.0 | 7.0 | 3.0 | | 0.0 | 4.0 |
| | 0.1250 | 0.0 | 3.0 | 3.0 | | 0.0 | 4.0 |
| | | | | | | | |
| 72 | 0.2500 | 9.0 | 9.0 | 9.0 | | 4.0 | 3.0 |
| | 0.1250 | 9.0 | 7.0 | 9.0 | | 4.0 | 3.0 |
| | 0.0625 | 9.0 | 6.0 | 0.0 | | 4.0 | 2.0 |
| | 0.0313 | 9.0 | 6.0 | 0.0 | | 4.0 | 1.0 |
| | | | | | | | |
| 73 | 0.2500 | 7.0 | 6.0 | 4.0 | | 4.0 | 6.0 |
| | 0.1250 | 3.0 | 0.0 | 2.0 | | 3.0 | 5.0 |
| | | | | | | | |
| 74 | 0.2500 | 9.0 | 0.0 | 9.0 | | 9.0 | 3.0 |
| | 0.1250 | 9.0 | 0.0 | 5.0 | | 3.0 | 2.0 |
| | 0.0625 | 9.0 | 0.0 | 5.0 | | 3.0 | 0.0 |
| | 0.0313 | 7.0 | 0.0 | 0.0 | | 3.0 | 0.0 |
| | | | | | | | |
| 75 | 0.2500 | 9.0 | 6.0 | 3.0 | | 3.0 | 2.0 |
| | 0.1250 | 9.0 | 6.0 | 3.0 | | 0.0 | 1.0 |
| | 0.0625 | 6.0 | 0.0 | 3.0 | | 0.0 | 0.0 |
| | 0.0313 | 6.0 | 0.0 | 2.0 | | 0.0 | 0.0 |
| | | | | | | | |
| 76 | 0.2500 | 9.0 | 6.0 | 3.0 | | 4.0 | 4.0 |
| | 0.1250 | 9.0 | 0.0 | 2.0 | | 4.0 | 3.0 |
| | 0.0625 | 7.0 | 0.0 | 2.0 | | 0.0 | 2.0 |
| | 0.0313 | 4.0 | 0.0 | 2.0 | | 0.0 | 2.0 |
| | | | | | | | |
| 77 | 0.2500 | 9.0 | 0.0 | 4.0 | | 0.0 | 3.0 |
| | 0.1250 | 9.0 | 0.0 | 0.0 | | 0.0 | 3.0 |
| | 0.0625 | 9.0 | 0.0 | 0.0 | | 0.0 | 2.0 |
| | 0.0313 | 9.0 | 0.0 | 0.0 | | 0.0 | 2.0 |
| | | | | | | | |
| 78 | 0.2500 | 9.0 | 9.0 | 9.0 | | 4.0 | 1.0 |
| | 0.1250 | 9.0 | 6.0 | 7.0 | | 2.0 | 0.0 |
| | 0.0625 | 7.0 | 5.0 | 6.0 | | 0.0 | 0.0 |
| | 0.0313 | 7.0 | 5.0 | 6.0 | | 0.0 | 0.0 |
| | | | | | | | |
| 79 | 0.2500 | 8.0 | 6.0 | 9.0 | | 4.0 | 3.0 |
| | 0.1250 | 7.0 | 2.0 | 0.0 | | 4.0 | 3.0 |
| | 0.0625 | 6.0 | 0.0 | 0.0 | | 0.0 | 2.0 |
| | 0.0313 | 5.0 | 0.0 | 0.0 | | 0.0 | 0.0 |
| | | | | | | | |
| 80 | 0.2500 | 9.0 | 7.0 | 4.0 | | 0.0 | 2.0 |
| | 0.1250 | 9.0 | 7.0 | 3.0 | | 0.0 | 0.0 |
| | 0.0625 | 9.0 | 7.0 | 3.0 | | 0.0 | 0.0 |
| | 0.0313 | 9.0 | 5.0 | 3.0 | | 0.0 | 0.0 |
| | | | | | | | |
| 81 | 0.2500 | 3.0 | 5.0 | 0.0 | | 0.0 | 0.0 |
| | 0.1250 | 3.0 | 5.0 | 0.0 | | 0.0 | 0.0 |
| | 0.0625 | 0.0 | 4.0 | 0.0 | | 0.0 | 0.0 |
| | | | | | | | |
| 82 | 0.2500 | 9.0 | 3.0 | 4.0 | | 7.0 | 3.0 |
| | 0.1250 | 6.0 | 3.0 | 2.0 | | 4.0 | 3.0 |
| | 0.0625 | 4.0 | 0.0 | 0.0 | | 4.0 | 0.0 |
| | 0.0313 | 4.0 | 0.0 | 0.0 | | 4.0 | 0.0 |
| | | | | | | | |
| 83 | 0.2500 | 7.0 | 7.0 | 5.0 | 9.0 | 9.0 | 6.0 |
| | 0.1250 | 7.0 | 6.0 | 3.0 | 9.0 | 7.0 | 5.0 |
| | 0.0625 | 6.0 | 6.0 | | 9.0 | 7.0 | 3.0 |
| | 0.0313 | 5.0 | 5.0 | 0.0 | 9.0 | 7.0 | 3.0 |
| | | | | | | | |
| 84 | 0.2500 | 9.0 | 9.0 | 9.0 | 9.0 | 9.0 | 7.0 |
| | 0.1250 | 7.0 | 9.0 | 9.0 | 9.0 | 7.0 | 6.0 |
| | 0.0625 | 5.0 | 7.0 | 3.0 | 9.0 | 6.0 | 4.0 |
| | 0.0313 | 4.0 | 3.0 | 0.0 | 9.0 | 2.0 | 3.0 |
| | | | | | | | |
| 85 | 0.2500 | 9.0 | 9.0 | 9.0 | 9.0 | 9.0 | 5.0 |
| | 0.1250 | 7.0 | 9.0 | 2.0 | 9.0 | 9.0 | 4.0 |
| | 0.0625 | 5.0 | 7.0 | 2.0 | 9.0 | 3.0 | 4.0 |
| | 0.0313 | 4.0 | 3.0 | 0.0 | 9.0 | 3.0 | 3.0 |
| | | | | | | | |
| 86 | 0.2500 | 9.0 | 9.0 | 9.0 | 9.0 | 9.0 | 5.0 |
| | 0.1250 | 9.0 | 8.0 | 3.0 | 9.0 | 9.0 | 4.0 |
| | 0.0625 | 6.0 | 7.0 | 0.0 | 9.0 | 9.0 | 4.0 |
| | 0.0313 | 5.0 | 3.0 | 0.0 | 9.0 | 5.0 | 3.0 |
| | | | | | | | |
| 87 | 0.5000 | 3.0 | 7.0 | 5.0 | 9.0 | 7.0 | 6.0 |
| | 0.2500 | 3.0 | 5.0 | 5.0 | 9.0 | 5.0 | 4.0 |
| | 0.1250 | 2.0 | 5.0 | 5.0 | 9.0 | 4.0 | 3.0 |
| | 0.0625 | 0.0 | 5.0 | 0.0 | 9.0 | 4.0 | 3.0 |
| | 0.0313 | 0.0 | 5.0 | 0.0 | 9.0 | 0.0 | 2.0 |
| | | | | | | | |
| 88 | 0.2500 | 7.0 | 0.0 | 7.0 | 9.0 | 9.0 | 4.0 |
| | 0.1250 | 6.0 | 0.0 | 7.0 | 9.0 | 9.0 | 4.0 |
| | 0.0625 | 6.0 | 0.0 | 5.0 | 9.0 | 9.0 | 3.0 |
| | 0.0313 | 6.0 | 0.0 | 3.0 | 9.0 | 6.0 | 2.0 |
| | | | | | | | |
| 89 | 0.2500 | 6.0 | 4.0 | 6.0 | 9.0 | 9.0 | 6.0 |
| | 0.1250 | 5.0 | 2.0 | 4.0 | 9.0 | 9.0 | 5.0 |
| | 0.0625 | 4.0 | 0.0 | 0.0 | 9.0 | 5.0 | 4.0 |
| | 0.0313 | 3.0 | 0.0 | 0.0 | 9.0 | 4.0 | 2.0 |
| | | | | | | | |
| 90 | 0.2500 | 3.0 | 3.0 | 3.0 | 9.0 | 9.0 | 3.0 |
| | 0.1250 | 0.0 | 1.0 | 0.0 | 9.0 | 9.0 | 1.0 |
| | 0.0625 | 0.0 | 0.0 | 0.0 | 9.0 | 6.0 | 0.0 |
| | 0.0313 | 0.0 | 0.0 | 0.0 | 9.0 | 4.0 | 0.0 |
| | | | | | | | |
| 91 | 0.2500 | 4.0 | 0.0 | 0.0 | 9.0 | 0.0 | 2.0 |
| | 0.1250 | 2.0 | 0.0 | 0.0 | 9.0 | 0.0 | 0.0 |
| | 0.0625 | 0.0 | 0.0 | 0.0 | 9.0 | 0.0 | 0.0 |
| | 0.0313 | 0.0 | 0.0 | 0.0 | 9.0 | 0.0 | 0.0 |
| | | | | | | | |
| 92 | 0.2500 | 3.0 | 7.0 | 9.0 | 9.0 | 9.0 | 3.0 |
| | 0.1250 | 2.0 | 5.0 | 9.0 | 9.0 | 4.0 | 3.0 |
| | 0.0625 | 0.0 | 3.0 | 9.0 | 9.0 | 0.0 | 2.0 |
| | 0.0313 | 0.0 | 3.0 | 9.0 | 9.0 | 0.0 | 1.0 |
| | | | | | | | |
| 93 | 0.2500 | 7.0 | 0.0 | 9.0 | 9.0 | 9.0 | 3.0 |
| | 0.1250 | 4.0 | 0.0 | 5.0 | 9.0 | 3.0 | 0.0 |
| | 0.0625 | 3.0 | 0.0 | 0.0 | 9.0 | 0.0 | 0.0 |
| | 0.0313 | 0.0 | 0.0 | 0.0 | 9.0 | 0.0 | 0.0 |
| | | | | | | | |
| 94 | 0.2500 | 7.0 | 4.0 | 9.0 | 9.0 | 9.0 | 3.0 |
| | 0.1250 | 2.0 | 4.0 | 6.0 | 9.0 | 5.0 | 3.0 |
| | 0.0625 | 0.0 | 3.0 | | 9.0 | 0.0 | 2.0 |
| | 0.0313 | 0.0 | 0.0 | | 9.0 | 0.0 | 0.0 |
| | | | | | | | |
| 95 | 0.2500 | 3.0 | 7.0 | 3.0 | 9.0 | | 8.0 |
| | 0.1250 | 3.0 | 7.0 | 0.0 | 9.0 | | 8.0 |
| | 0.0625 | 0.0 | 4.0 | 0.0 | 5.0 | | 4.0 |
| | 0.0313 | 0.0 | 4.0 | 0.0 | 5.0 | | 3.0 |
| | | | | | | | |
| 96 | 0.2500 | 3.0 | 6.0 | 0.0 | 3.0 | | 5.0 |
| | 0.1250 | 0.0 | 6.0 | 0.0 | 3.0 | | 4.0 |
| | 0.0625 | 0.0 | 5.0 | 0.0 | 2.0 | | 4.0 |
| | 0.0313 | 0.0 | 0.0 | 0.0 | 2.0 | | 3.0 |
| | | | | | | | |
| 97 | 0.2500 | 3.0 | 4.0 | 3.0 | 9.0 | | 5.0 |
| | 0.1250 | 2.0 | 3.0 | 0.0 | 9.0 | | 5.0 |
| | 0.0625 | 0.0 | 0.0 | 0.0 | 9.0 | | 4.0 |
| | 0.0313 | 0.0 | 0.0 | 0.0 | 9.0 | | 1.0 |
| | | | | | | | |
| 98 | 0.2500 | 3.0 | 9.0 | 4.0 | 9.0 | | 7.0 |
| | 0.1250 | 1.0 | 9.0 | 4.0 | 9.0 | | 7.0 |
| | 0.0625 | 0.0 | 9.0 | 3.0 | 9.0 | | 6.0 |
| | 0.0313 | 0.0 | 9.0 | 2.0 | 9.0 | | 2.0 |
| | | | | | | | |
| 99 | 0.5000 | 4.0 | 0.0 | 0.0 | 9.0 | 4.0 | 4.0 |
| | 0.2500 | 0.0 | 0.0 | 0.0 | 9.0 | 4.0 | 4.0 |
| | 0.1250 | 0.0 | 0.0 | 0.0 | 9.0 | 3.0 | 4.0 |
| | 0.0625 | 0.0 | 0.0 | 0.0 | 9.0 | 0.0 | 2.0 |
| | 0.0313 | 0.0 | 0.0 | 0.0 | 9.0 | 0.0 | 2.0 |
| | | | | | | | |
| 100 | 0.5000 | 4.0 | 9.0 | | 9.0 | 6.0 | 4.0 |
| | 0.2500 | 3.0 | 4.0 | 0.0 | 9.0 | 4.0 | 3.0 |
| | 0.1250 | 0.0 | 0.0 | 0.0 | 9.0 | 0.0 | 3.0 |
| | 0.0625 | 0.0 | 0.0 | 0.0 | 9.0 | 0.0 | 3.0 |
| | 0.0313 | 0.0 | 0.0 | 0.0 | 9.0 | 0.0 | 3.0 |
| | | | | | | | |
| 101 | 0.5000 | 0.0 | 4.0 | 0.0 | 9.0 | 4.0 | 3.0 |
| | 0.2500 | 0.0 | 3.0 | 0.0 | 9.0 | 2.0 | 0.0 |
| | 0.1250 | 0.0 | 0.0 | 0.0 | 7.0 | 0.0 | 0.0 |
| | 0.0625 | 0.0 | 0.0 | 0.0 | 5.0 | 0.0 | 0.0 |
| | 0.0313 | 0.0 | 0.0 | 0.0 | 5.0 | 0.0 | 0.0 |
| | | | | | | | |
| 102 | 0.5000 | 2.0 | 6.0 | 0.0 | 4.0 | 0.0 | 4.0 |
| | 0.2500 | 0.0 | 2.0 | 0.0 | 0.0 | 0.0 | 2.0 |
| | | | | | | | |
| 103 | 0.2500 | 6.0 | 9.0 | 6.0 | 9.0 | 9.0 | 6.0 |
| | 0.1250 | 5.0 | 9.0 | 6.0 | 9.0 | 9.0 | 5.0 |
| | 0.0625 | 4.0 | 6.0 | 5.0 | 7.0 | 9.0 | 5.0 |
| | 0.0313 | 4.0 | 4.0 | 5.0 | 5.0 | 7.0 | 4.0 |
| | | | | | | | |
| 104 | 0.2500 | 6.0 | 9.0 | 0.0 | 9.0 | 9.0 | 6.0 |
| | 0.1250 | 6.0 | 9.0 | 0.0 | 9.0 | 9.0 | 7.0 |
| | 0.0625 | 5.0 | 9.0 | | 9.0 | 9.0 | 5.0 |
| | 0.0313 | 4.0 | 5.0 | 0.0 | 7.0 | 6.0 | 3.0 |
| | | | | | | | |
| 105 | 0.2500 | 5.0 | 9.0 | 9.0 | 9.0 | 7.0 | 6.0 |
| | 0.1250 | 4.0 | 7.0 | 7.0 | 7.0 | 5.0 | 3.0 |
| | 0.0625 | 3.0 | 7.0 | 5.0 | 7.0 | 5.0 | 5.0 |
| | 0.0313 | 0.0 | 5.0 | 2.0 | 6.0 | 3.0 | 5.0 |
| | | | | | | | |
| 109 | 0.2500 | 9.0 | 7.0 | 7.0 | 9.0 | 0.0 | 8.0 |
| | 0.1250 | 7.0 | 4.0 | | 9.0 | 0.0 | 4.0 |
| | 0.0625 | 2.0 | 2.0 | 0.0 | 4.0 | 0.0 | 2.0 |
| | 0.0313 | 2.0 | 0.0 | 0.0 | 2.0 | 0.0 | 2.0 |
| | | | | | | | |
| 114 | 0.2500 | 0.0 | 7.0 | 0.0 | 9.0 | 0.0 | 4.0 |
| | 0.1250 | 0.0 | 4.0 | 0.0 | 9.0 | 0.0 | 4.0 |
| | 0.0625 | 0.0 | 0.0 | 0.0 | 8.0 | 0.0 | 1.0 |
| | 0.0313 | 0.0 | 0.0 | 0.0 | 6.0 | 0.0 | 1.0 |
| | | | | | | | |
| 119 | 0.2500 | 4.0 | 9.0 | | 9.0 | 0.0 | 3.0 |
| | 0.1250 | 4.0 | 6.0 | 0.0 | 8.0 | 0.0 | 2.0 |
| | 0.0625 | 2.0 | | 0.0 | 7.0 | 0.0 | 2.0 |
| | 0.0313 | 0.0 | 2.0 | 0.0 | 4.0 | 0.0 | 2.0 |

## Claims

1. A compound having the structural formula I 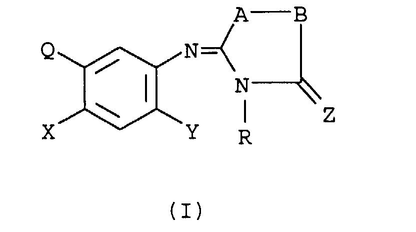 wherein
X and Y are each independently hydrogen, halogen, nitro, cyano, C₁-C₄alkyl, C₁-C₄haloalkyl or S(O)ₘR₁;
R is hydrogen,
a C₁-C₈alkyl, C₃-C₇cycloalkyl, C₃-C₈alkenyl, C₅-C₇cycloalkenyl or C₃-C₈alkynyl group, wherein each group is optionally substituted with any combination of one to six halogen atoms, one or two cyano groups, one or two nitro groups, one or two C(W)R₂ groups, one or two C(W)OR₃ groups,
one or two C(W)NR₄R₅ groups, one or two P(V) (OR₆)₂ groups, one or two OR₇ groups, one or two SR₈ groups, one or two NR₉R₁₀ groups, one or two OC(W)R₁₁ groups, one or two NR₁₂S(O)ₙR₁₃ groups, one or two C(W)NR₁₂S(O)ₙR₁₃ groups,
one 4- to 10-membered heterocyclic ring optionally substituted with any combination of one or two oxo groups, one or two thioxo groups, one to three halogen atoms, one to three nitro groups, one to three cyano groups, one to three C₁-C₄alkyl groups, one to three C₁-C₄haloalkyl groups, one to three C₁-C₄alkoxy groups, one to three C₁-C₄haloalkoxy groups, one or two C₂-C₆alkoxyalkyl groups, one or two C₂-C₆haloalkoxyalkyl groups, one or two C(O)OR₁₄ groups, one or two C(O)R₁₄ groups, one to three C₃-C₆alkoxycarbonylalkyl groups, one to three C₃-C₆haloalkoxycarbonylalkyl groups, one to three C₃-C₆alkylcarbonyalkyl groups, one to three C₃-C₆haloalkylcarbonylalkyl groups, one or two NR₁₅S(O)ₚR₁₆ groups or one or two C(W)NR₁₅S(O)ₚR₁₆ groups, or
one phenyl group optionally substituted with any combination of one to three halogen atoms, one to three nitro groups, one to three cyano groups, one to three C₁-C₄alkyl groups, one to three C₁-C₄haloalkyl groups, one to three C₁-C₄alkoxy groups, one to three C₁-C₄haloalkoxy groups, one or two C₂-C₆alkoxyalkyl groups, one or two C₂-C₆₋ haloalkoxyalkyl groups, one or two C(O)OR₁₄ groups, one or two C(O)R₁₄ groups, one to three C₃-C₆alkoxycarbonylalkyl groups, one to three C₃-C₆haloalkoxycarbonylalkyl groups, one to three C₃-C₆alkylcarbonylalkyl groups, one to three C₃-C₆haloalkylcarbonylalkyl groups one or two NR₁₅S(O)ₚR₁₆ groups or one or two C(W)NR₁₅S(O)ₚR₁₆ groups,
a 4- to 10-membered heterocyclic ring optionally substituted with any combination of one or two oxo groups, one or two thioxo groups, one to three halogen atoms, one to three nitro groups, one to three cyano groups, one to three C₁-C₄-alkyl groups, one to three C₁-C₄haloalkyl groups, one to three C₁-C₄alkoxy groups, one to three C₁-C₄haloalkoxy groups, one or two C₂-C₆alkoxyalkyl groups, one or two C₂-C₆haloalkoxyalkyl groups, one or two C(O)OR₁₄ groups, one or two C(O)R₁₄ groups, one to three C₃-C₆alkoxycarbonylalkyl groups, one to three C₃-C₆haloalkoxycarbonylalkyl groups, one to three C₃-C₆alkylcarbonylalkyl groups, one to three C₃-C₆haloalkylcarbonylalkyl groups, one or two NR₁₅S(O)ₚR₁₆ groups or one or two C(W)NR₁₅S(O)ₚR₁₆ groups,
phenyl optionally substituted with any combination of one to three halogen atoms, one to three nitro groups, one to three cyano groups, one to three C₁-C₄alkyl groups, one to three C₁-C₄haloalkyl groups, one to three C₁-C₄alkoxy groups, one to three C₁-C₄haloalkoxy groups, one or two C₂-C₆alkoxyalkyl groups, one or two C₂-C₆haloalkoxyalkyl groups, one or two C(O)OR₁₄ groups, one or two C(O)R₁₄ groups, one to three C₃-C₆alkoxycarbonylalkyl groups, one to three C₃-C₆haloalkoxycarbonylalkyl groups, one to three C₃-C₆alkylcarbonylalkyl groups, one to three C₃-C₆haloalkylcarbonylalkyl groups, one or two NR₁₅S(O)ₚR₁₆ groups or one or two C (W)NR₁₅S(O)ₚR₁₆ groups,
OR₁₇,
NR₁₈R₁₉,
NR₂₀S(O)_{q}R₂₁,
NR₂₂C(V)R₂₃,
NR₂₄P(V)(OR₂₅)₂,
C(V)NR₂₀S(O)_{q}R₂₁,
cyano,
S(O)_{q}R₂₆,
P(V) (OR₂₇)₂,
C(V)R₂₈ or
C(V)OR₂₉;
W is O, S, NR₃₀, NOR₃₁ or NNR₃₂R₃₃;
V and Z are each independently O or S;
R₁ and R₁₄ are each independently C₁-C₄alkyl or C₁-C₄haloalkyl;
R₂ is hydrogen, C₁-C₆alkyl, C₃-C₇cycloalkyl, C₁-C₆haloalkyl, C₃-C₆alkenyl, C₃-C₆alkynyl, C₂-C₆alkoxyalkyl, C₂-C₆haloalkoxyalkyl, C₃-C₆alkoxycarbonylalkyl,
phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one or two C₂-C₆₋ alkoxyalkyl groups, one or two C₂-C₆haloalkoxyalkyl groups, one or two nitro groups, one or two cyano groups, one NR₁₅S(O)ₚR₁₆ group, one C₂-C₆alkoxycarbonyl group, one C₂-C₆haloalkoxycarbonyl group, one C₂-C₆alkylcarbonyl group, one C₂-C₆haloalkylcarbonyl group, one C₃-C₆₋ alkoxycarbonylalkyl group, one C₃-C₆haloalkoxycarbonylalkyl group, one C₃-C₆alkylcarbonylalkyl group or one C₃-C₆haloalkylcarbonylalkyl group,
a 4- to 10-membered heterocyclic ring optionally substituted with any combination of one or two oxo groups, one or two thioxo groups, one to three halogen atoms, one to three C₁-C₄alkyl groups, one to three C₁-C₄haloalkyl groups, one to three C₁-C₄alkoxy groups, one to three C₁-C₄haloalkoxy groups, one or two C₂-C₆alkoxyalkyl groups, one or two C₂-C₆haloalkoxyalkyl groups, one or two nitro groups, one or two cyano groups, one NR₁₅S(O)ₚR₁₆ group, one C₂-C₆alkoxycarbonyl group, one C₂-C₆haloalkoxycarbonyl group, one C₂-C₆alkylcarbonyl group, one C₂-C₆haloalkylcarbonyl group, one C₃-C₆alkoxycarbonylalkyl group, one C₃-C₆haloalkoxycarbonylalkyl group, one C₃-C₆alkylcarbonylalkyl group or one C₃-C₆haloalkylcarbonylalkyl group,
C₁-C₄alkyl substituted with one phenyl group wherein the phenyl group is optionally substituted with any combination of one to three halogen atoms, one or two nitro groups, one or two cyano groups, one to three C₁-C₄alkyl groups, one to three C₁-C₄haloalkyl groups, one to three C₁-C₄alkoxy groups or one to three C₁-C₄haloalkoxy groups, or
C₁-C₄alkyl substituted with one 4- to 10-membered heterocyclic ring wherein the heterocyclic ring is optionally substituted with any combination of one to three halogen atoms, one or two nitro groups, one or two cyano groups, one to three C₁-C₄alkyl groups, one to three C₁-C₄haloalkyl groups, one to three C₁-C₄alkoxy groups or one to three C₁-C₄haloalkoxy groups;
R₃ is hydrogen, C₁-C₆alkyl, C₁-C₆haloalkyl, C₃-C₇cycloalkyl, C₃-C₆alkenyl C₃-C₆alkynyl, C₂-C₆alkoxyalkyl, C₂-C₆haloalkoxyalkyl, C₃-C₆alkoxycarbonylalkyl,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups,
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups,
furfural optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, or
an alkali metal, alkaline earth metal, manganese, copper, zinc, cobalt, silver, nickel, ammonium or organic ammonium cation;
R₄ and R₅ are each independently hydrogen, C₁-C₆alkyl, C₁-C₆haloalkyl, C₃-C₇cycloalkyl, C₁-C₆alkoxy, hydroxy, C₃-C₆alkenyl, C₃-C₆haloalkenyl, C₃-C₆alkynyl, C₃-C₆haloalkynyl, C₃-C₆alkylcarbonylalkyl, C₃-C₆haloalkylcarbonylalkyl, C₃-C₆alkoxycarbonylalkyl, C₃-C₆haloalkoxycarbonylalkyl, C₂-C₆hydroxycarbonylalkyl, C₂-C₆alkylsulfonylalkyl, C₂-C₆haloalkylsulfonylalkyl, S(O)ₚR₁₆,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups,
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, or
furfural optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups,
provided that only one of R₄ and R₅ can be hydroxy or C₁-C₆alkoxy, and
when R₄ and R₅ are taken together with the atom to which they are attached, they represent a three- to seven-membered saturated or unsaturated ring optionally interrupted by oxygen, sulfur or nitrogen, and optionally substituted with one to three methyl groups or one or more halogen atoms;
R₆, R₂₅ and R₂₇ are each independently hydrogen, C₁-C₆alkyl, C₁-C₆haloalkyl,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, or
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups;
R₇, R₈ and R₁₇ are each independently hydrogen, C₁-C₆-alkyl, C₁-C₆haloalkyl, C₃-C₇cycloalkyl, C₃-C₆alkenyl, C₃-C₆haloalkenyl, C₃-C₆alkynyl, C₃-C₆haloalkynyl, C₂-C₆alkoxyalkyl, C₂-C₆haloalkoxyalkyl, C₂-C₆alkylcarbonylalkyl, C₂-C₆haloalkylcarbonylalkyl, C₂-C₆₋ alkoxycarbonylalkyl, C₂-C₆haloalkoxycarbonylalkyl, C₂-C₆alkylsulfonylalkyl, C₂-C₆haloalkylsulfonylalkyl, C₂-C₆hydroxycarbonylalkyl, C(V)NR₁₂S(O)ₙR₁₃,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups,
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, or
furfural optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups;
R₉, R₁₀, R₁₈, R₁₉, R₃₂ and R₃₃ are each independently hydrogen, C₁-C₆alkyl, C₁-C₆haloalkyl, C₃-C₇cycloalkyl, C₃-C₆alkenyl, C₃-C₆haloalkenyl, C₃-C₆alkynyl, C₃-C₆haloalkynyl, C₃-C₆alkylcarbonylalkyl, C₃-C₆haloalkylcarbonylalkyl, C₃-C₆alkoxycarbonylalkyl, C₃-C₆haloalkoxycarbonylalkyl, C₂-C₆hydroxycarbonylalkyl, C₂-C₆alkylsulfonylalkyl, C₂-C₆haloalkylsulfonylalkyl,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups,
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄₋haloalkoxy groups, or
furfural optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, and
when R₉ and R₁₀ or R₁₈ and R₁₉ or R₃₂ and R₃₃ are taken together with the atom to which they are attached, they represent a three- to seven-membered saturated or unsaturated ring optionally interrupted by oxygen, sulfur or nitrogen, and optionally substituted with one to three methyl groups or one or more halogen atoms;
R₁₁ is hydrogen, C₁-C₆alkyl, C₁-C₆haloalkyl, C₃-C₇cycloalkyl, C₃-C₆alkenyl, C₃-C₆haloalkenyl, C₃-C₆alkynyl, C₃-C₆haloalkynyl, C₂-C₆alkoxyalkyl, C₂-C₆haloalkoxyalkyl, C₃-C₆alkylcarbonylalkyl, C₃-C₆haloalkylcarbonylalkyl, C₃-C₆alkoxycarbonylalkyl, C₃-C₆haloalkoxycarbonylalkyl, C₂-C₆hydroxycarbonylalkyl,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups,
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, or
furfural optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups;
R₁₂, R₁₅, R₂₀ and R₂₄ are each independently hydrogen, C₁-C₆alkyl, C₁-C₆haloalkyl, C₃-C₆alkenyl or C₃-C₆alkynyl;
R₁₃, R₂₁, R₂₂, R₂₃, R₂₆, R₂₈ and R₂₉ are each independently hydrogen, C₁-C₆alkyl, C₁-C₆haloalkyl, C₃-C₇cycloalkyl, C₃-C₆alkenyl, C₃-C₆haloalkenyl, C₃-C₆alkynyl, C₃-C₆haloalkynyl, C₃-C₆alkylcarbonylalkyl, C₃-C₆haloalkylcarbonylalkyl, C₃-C₆alkoxycarbonylalkyl, C₃-C₆haloalkoxycarbonylalkyl,
phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one or two C₂-C₆alkoxyalkyl groups, one or two C₂-C₆haloalkoxyalkyl groups, one or two nitro groups, one or two cyano groups, one C₃-C₆alkoxycarbonylalkyl group, one C₃-C₆haloalkoxycarbonylalkyl group, one C₃-C₆alkylcarbonylalkyl group, one C₃-C₆haloalkylcarbonylalkyl group or one C₂-C₆hydroxycarbonylalkyl group,
a 4- to 10-membered heterocyclic ring optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one or two C₂-C₆₋ alkoxyalkyl groups, one or two C₂-C₆haloalkoxyalkyl groups, one or two nitro groups, one or two cyano groups, one C₃-C₆alkoxycarbonylalkyl group, one C₃-C₆haloalkoxycarbonylalkyl group, one C₃-C₆alkylcarbonylalkyl group, one C₃-C₆haloalkylcarbonylalkyl group or one C₂-C₆hydroxycarbonylalkyl group,
benzyl optionally substituted on the phenyl ring with any combination of one to three halogen atoms, one to three C₁-C₄alkyl groups, one to three C₁-C₄haloalkyl groups, one to three C₁-C₄alkoxy groups or one to three C₁-C₄haloalkoxy groups, or
furfural optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups;
R₁₆ is hydrogen, C₁-C₆alkyl, C₁-C₆haloalkyl, C₃-C₇cycloalkyl, C₃-C₆alkenyl, C₃-C₆haloalkenyl, C₃-C₆alkynyl, C₃-C₆haloalkynyl, C₃-C₆alkylcarbonylalkyl, C₃-C₆-haloalkylcarbonylalkyl, C₃-C₆alkoxycarbonylalkyl, C₃-C₆haloalkoxycarbonylalkyl,
phenyl optionally substituted on the phenyl ring with any combination of one to three halogen atoms, one to three C₁-C₄alkyl groups, one to three C₁-C₄haloalkyl groups, one to three C₁-C₄alkoxy groups or one to three C₁-C₄haloalkoxy groups,
benzyl optionally substituted on the phenyl ring with any combination of one to three halogen atoms, one to three C₁-C₄alkyl groups, one to three C₁-C₄haloalkyl groups, one to three C₁-C₄alkoxy groups or one to three C₁-C₄haloalkoxy groups, or
furfural optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups;
R₃₀ is hydrogen, C₁-C₆alkyl, C₁-C₆haloalkyl, C₃-C₇cycloalkyl, C₃-C₆alkenyl, C₃-C₆alkynyl, C₂-C₆alkoxyalkyl, C₃-C₆alkoxycarbonylalkyl,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups, one to three C₁-C₄haloalkoxy groups, one C₂-C₆alkoxyalkyl group, one C₂-C₆haloalkoxyalkyl group, one NR₁₅S(O)ₙR₁₆ group, one C₂-C₆alkoxycarbonyl group, one C₂-C₆alkylcarbonyl group, one C₃-C₆-alkoxycarbonylalkyl group, one C₃-C₆alkylcarbonylalkyl group, one C₃-C₆haloalkoxycarbonylalkyl group or one C₃-C₆haloalkylcarbonylalkyl group,
a 4- to 10-membered heterocyclic ring optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups, one to three C₁-C₄haloalkoxy groups, one C₂-C₆alkoxyalkyl group, one C₂-C₆haloalkoxyalkyl group, one NR₁₅S(O)ₙR₁₆ group, one C₂-C₆alkoxycarbonyl group, one C₂-C₆alkylcarbonyl group, one C₃-C₆-alkoxycarbonylalkyl group, one C₃-C₆alkylcarbonylalkyl group, one C₃-C₆haloalkoxycarbonylalkyl group or one C₃-C₆haloalkylcarbonylalkyl group,
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄₋ haloalkoxy groups, or
furfural optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups;
R₃₁ is hydrogen, C₁-C₆alkyl, C₁-C₆haloalkyl, C₃-C₇cycloalkyl, C₃-C₆alkenyl, C₃-C₆haloalkenyl, C₃-C₆alkynyl, C₃-C₆haloalkynyl, C₂-C₆alkoxyalkyl, C₂-C₆haloalkoxyalkyl, C₃-C₆alkylcarbonylalkyl, C₃-C₆-haloalkylcarbonylalkyl, C₃-C₆alkoxycarbonylalkyl, C₃-C₆haloalkoxycarbonylalkyl, C₂-C₆alkylsulfonylalkyl, C₂-C₆haloalkylsulfonylalkyl, C₂-C₆hydroxycarbonylalkyl, C(V)NR₁₂S(O)ₙR₁₃,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups,
a 4- to 10-membered heterocyclic ring optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups,
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, or
furfural optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups;
A is -O-, -S(O)ᵣ-, t -NR₃₄- or -CR₃₅R₃₆-;
B is -CR₃₇R₃₈(CR₃₉R₄₀)ₛ-, -C(=T)- or -C(=CR₄₁R₄₂)-;
R₃₄, R₃₅, R₃₆, R₃₇, R₃₈, R₃₉ and R₄₀ are each independently hydrogen, halogen,
C₁-C₆alkyl optionally substituted with one to three halogen atoms, one or two cyano groups, one or two nitro groups, one or two C(W)R₂ groups, one or two C(W)OR₃ groups, one or two C(W)NR₄R₅ groups, one or two P(V) (OR₆)₂ groups, one or two OR₇ groups, one or two SR₈ groups, one or two NR₉R₁₀ groups or one or two OC(W)R₁₁ groups,
C₃-C₇cycloalkyl,
C₃-C₆alkenyl,
C₃-C₆haloalkenyl
C₃-C₆alkynyl,
C₃-C₆haloalkynyl,
C(V)NR₁₂S(O)ₙR₁₃,
phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one or two C₂-C₆alkoxyalkyl groups, one or two C₂-C₆haloalkoxyalkyl groups, one or two nitro groups, one or two cyano groups, one NR₁₅S(O)ₚR₁₆ group, one C₂-C₆alkoxycarbonyl group, one C₂-C₆haloalkoxycarbonyl group, one C₂-C₆alkylcarbonyl group, one C₂-C₆haloalkylcarbonyl group, one C₃-C₆alkoxycarbonylalkyl group, one C₃-C₆haloalkoxycarbonylalkyl group, one C₃-C₆alkylcarbonylalkyl group or one C₃-C₆haloalkylcarbonylalkyl group,
a 4- to 10-membered heterocyclic ring optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one or two C₂-C₆alkoxyalkyl groups, one or two C₂-C₆haloalkoxyalkyl groups, one or two nitro groups, one or two cyano groups, one NR₁₅S(O)ₚR₁₆ group, one C₂-C₆alkoxycarbonyl group, one C₂-C₆haloalkoxycarbonyl group, one C₂-C₆alkylcarbonyl group, one C₂-C₆haloalkylcarbonyl group, one C₃-C₆alkoxycarbonylalkyl group, one C₃-C₆haloalkoxycarbonylalkyl group, one C₃-C₆alkylcarbonylalkyl group or one C₃-C₆haloalkylcarbonylalkyl group,
C₁-C₄alkyl substituted with one phenyl group wherein the phenyl group is optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one or two C₂-C₆alkoxyalkyl groups, one or two C₂-C₆haloalkoxyalkyl groups, one C₂-C₆alkoxycarbonyl group, one C₂-C₆haloalkoxycarbonyl group, one C₂-C₆alkylcarbonyl group, one C₂-C₆-haloalkylcarbonyl group, one C₃-C₆alkoxycarbonylalkyl group, one C₃-C₆haloalkoxycarbonylalkyl group, one C₃-C₆alkylcarbonylalkyl group, one C₃-C₆haloalkylcarbonylalkyl group, one C₃-C₈alkoxycarbonylalkoxy group or one C₂-C₈hydroxycarbonylalkoxy group, or
C₁-C₄alkyl substituted with one 4- to 10-membered heterocyclic ring wherein the heterocyclic ring is optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one or two C₂-C₆alkoxyalkyl groups, one or two C₂-C₆haloalkoxyalkyl groups, one C₂-C₆alkoxycarbonyl group, one C₂-C₆haloalkoxycarbonyl group, one C₂-C₆alkylcarbonyl group, one C₂-C₆haloalkylcarbonyl group, one C₃-C₆alkoxycarbonylalkyl group, one C₃-C₆haloalkoxycarbonylalkyl group, one C₃-C₆alkylcarbonylalkyl group or one C₃-C₆haloalkylcarbonylalkyl group, and
when R₃₅ and R₃₆ or R₃₇ and R₃₈ or R₃₉ and R₄₀ are taken together with the atom to which they are attached, they represent a ring in which R₃₅R₃₆ or R₃₇R₃₈ or R₃₉R₄₀ is a C₂-C₆alkylene group;
T is O, S, NR₃₀, NOR₃₁ or NNR₃₂R₃₃;
R₄₁ and R₄₂ are each independently hydrogen, C₁-C₆alkyl, C₁-C₆haloalkyl, C₃-C₇cycloalkyl, C₃-C₆alkenyl, C₃-C₆haloalkenyl, C₃-C₆alkynyl, C₃-C₆haloalkynyl, C₂-C₆alkylcarbonyl, C₂-C₆haloalkylcarbonyl, C₂-C₆-alkoxycarbonyl, C₂-C₆haloalkoxycarbonyl, hydroxycarbonyl, C₃-C₆alkylcarbonylalkyl, C₃-C₆haloalkylcarbonylalkyl, C₃-C₆alkoxycarbonylalkyl, C₃-C₆haloalkoxycarbonylalkyl, C₂-C₆hydroxycarbonylalkyl, C₂-C₆alkylsulfonylalkyl, C₂-C₆haloalkylsulfonylalkyl,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups, one to three C₁-C₄haloalkoxy groups or one C₃-C₈-alkoxycarbonylalkoxy group,
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, or
furfural optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, and
when R₄₁ and R₄₂ are taken together with the atom to which they are attached, they represent a three- to seven-membered saturated or unsaturated ring optionally interrupted with one group selected from O, S(O)ᵣ or NR₃₁, and optionally substituted with one to three methyl groups or one or more halogen atoms;
m and r are each independently an integer of O, 1 or 2;
n, p and q are each independently an integer of 1 or 2;
s is an integer of 0 or 1;
Q is selected from 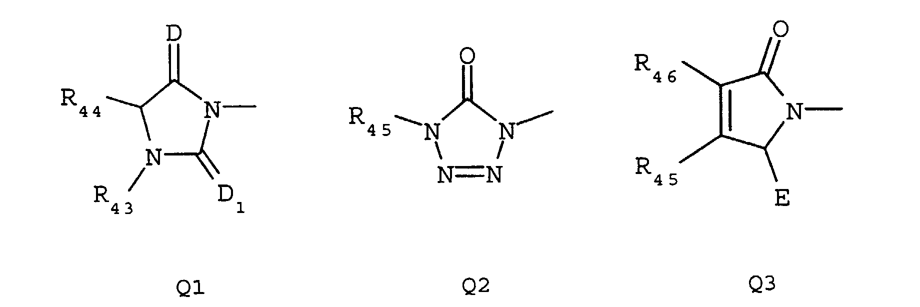 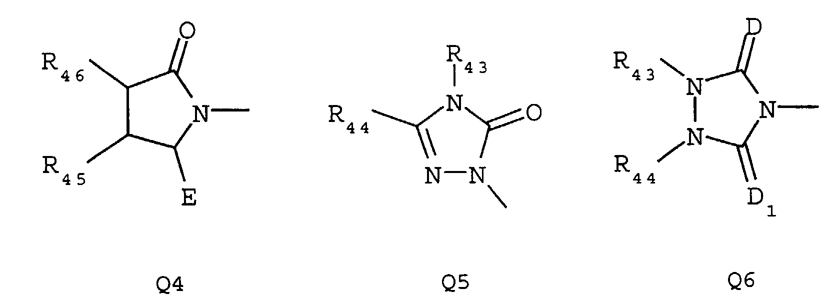 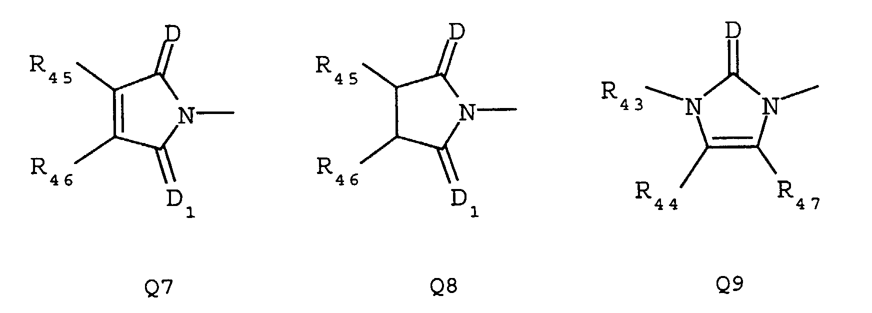 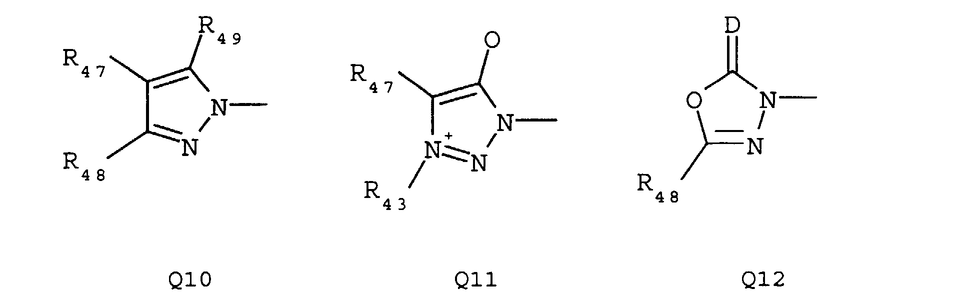 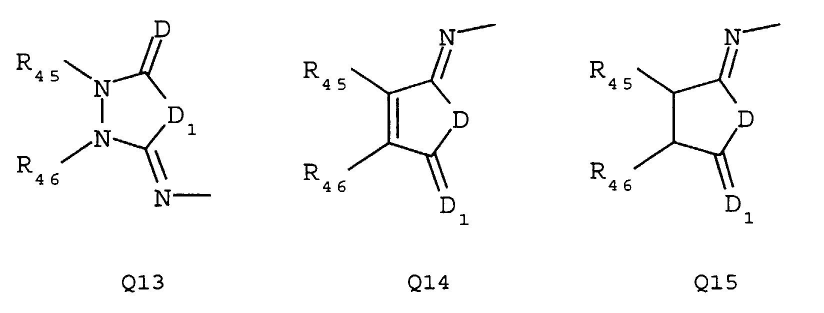 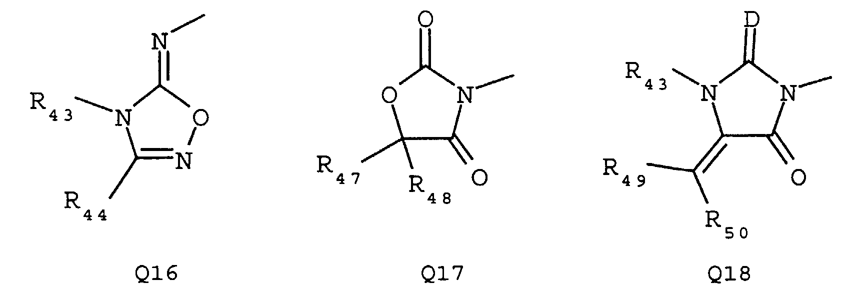 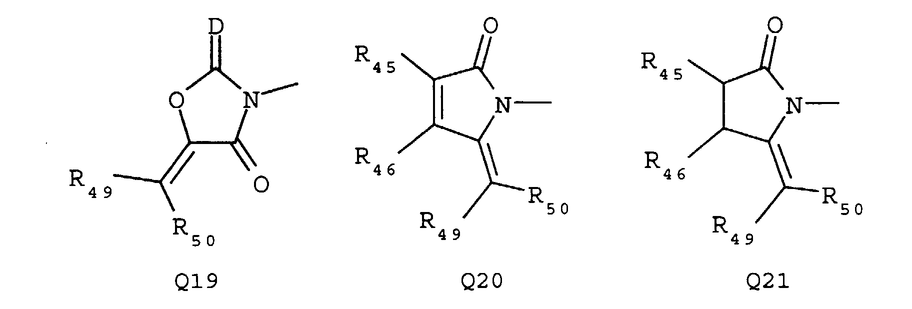 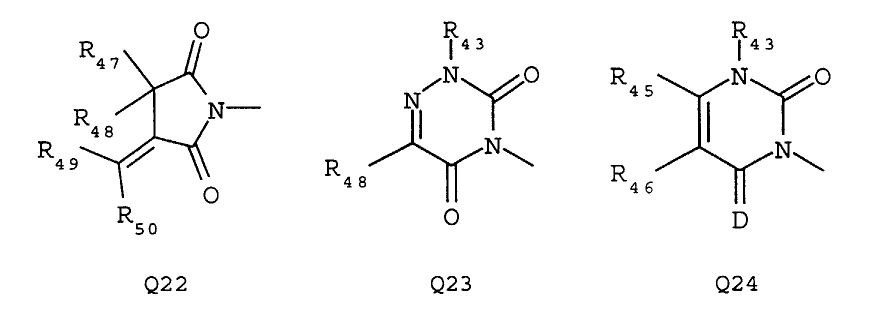 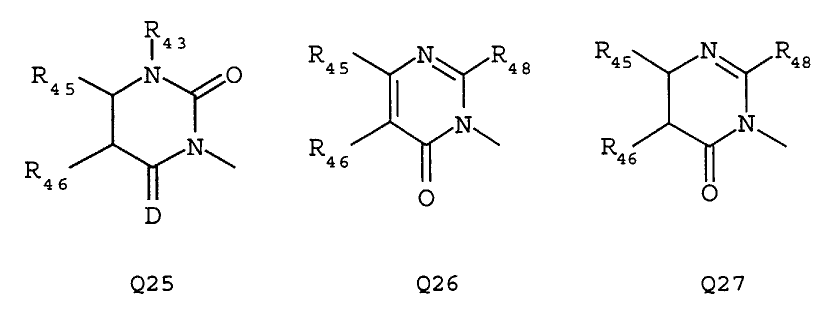 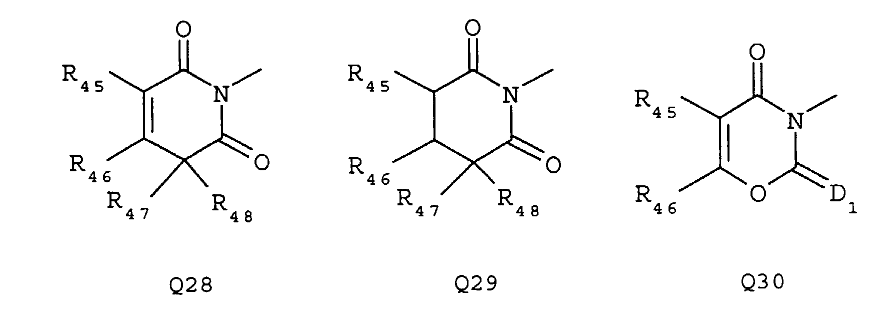 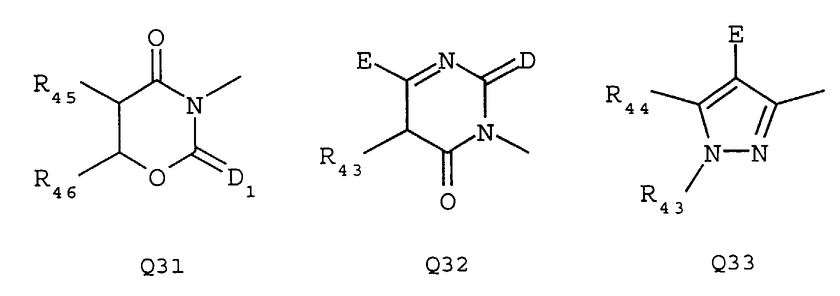 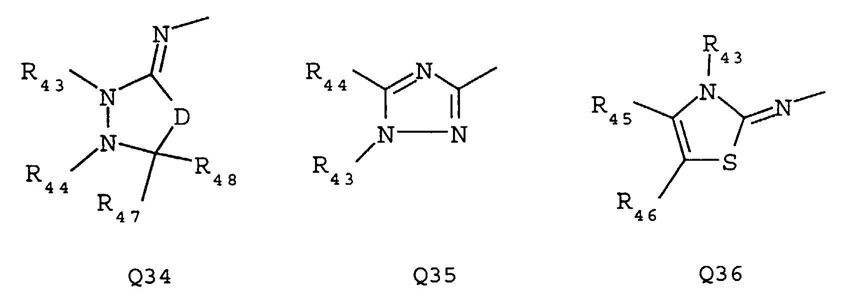 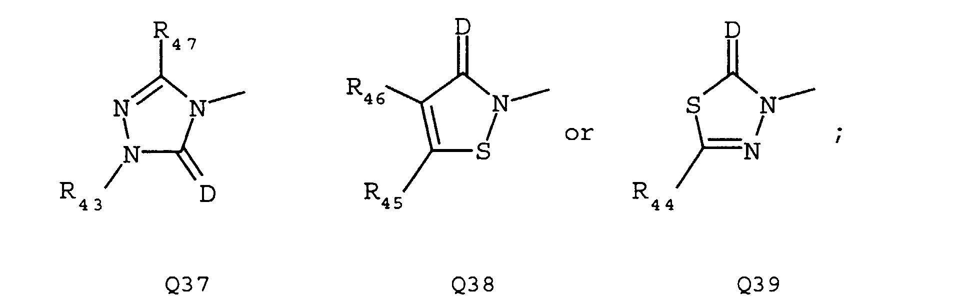
D and D₁ are each independently O or S;
E is hydroxy, halogen, C₁-C₄alkoxy or C₁-C₄alkylthio;
R₄₃ and R₄₄ are each independently hydrogen, halogen, C₁-C₆alkyl, C₁-C₆haloalkyl, C₃-C₇cycloalkyl, C₃-C₆alkenyl, C₃-C₆haloalkenyl, C₃-C₆alkynyl, cyano, amino, hydroxy or benzyl, and when R₄₃ and R₄₄ are taken together with the atom to which they are attached, they represent a three- to seven-membered saturated or unsaturated ring optionally interrupted by oxygen, sulfur or nitrogen, and optionally substituted with one to three methyl groups or one or more halogen atoms;
R₄₅ and R₄₆ are each independently hydrogen, halogen, C₁-C₆alkyl, C₁-C₆haloalkyl, C₃-C₇cycloalkyl, C₃-C₆alkenyl, C₃-C₆haloalkenyl, C₃-C₆alkynyl, OR₅₁, S(O)ₜR₅₂ or NR₅₃R₅₄, and when R₄₅ and R₄₆ are taken together with the atom to which they are attached, they represent a three- to seven-membered saturated or unsaturated ring optionally interrupted by oxygen, sulfur or nitrogen, and optionally substituted with one to three methyl groups or one or more halogen atoms;
R₄₇, R₄₉ and R₅₀ are each independently hydrogen, halogen or C₁-C₆alkyl;
R₄₈ is hydrogen, halogen, C₁-C₆alkyl, C₁-C₆haloalkyl, C₃-C₇cycloalkyl, C₃-C₆alkenyl, C₃-C₆haloalkenyl, C₃-C₆alkynyl, OR₅₁ or SR₅₂;
R₅₁ and R₅₂ are each independently hydrogen, C₁-C₄alkyl, C₁-C₄haloalkyl, C₃-C₇cycloalkyl, C₁-C₄cyanoalkyl, C₃-C₆alkenyl, C₃-C₆haloalkenyl, C₃-C₆alkynyl, benzyl or
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups;
R₅₃ is hydrogen, C₁-C₄alkyl, benzyl or
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups;
R₅₄ in hydrogen, C₁-C₄alkyl, C₃-C₆alkenyl, C₃-C₇cycloalkyl, benzyl, S(O)ₜR₅₂ or
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups; and
t is an integer of O, 1 or 2; and
the agriculturally acceptable salts thereof.

2. The compound according to claim 1 wherein
X is hydrogen or halogen;
Y is hydrogen, halogen, nitro or cyano;
R is hydrogen,
a C₁-C₈alkyl, C₃-C₇cycloalkyl, C₃-C₈alkenyl,
C₅-C₇cycloalkenyl or C₃-C₈alkynyl group, wherein each group is optionally substituted with any combination of one to six halogen atoms, one or two cyano groups, one or two nitro groups, one or two C(O)R₂ groups, one or two C(O)OR₃ groups, one or two C(O)NR₄R₅ groups, one or two P(O) (OR₆)₂ groups, one or two OR₇ groups, one or two SR₈ groups, one or two NR₉R₁₀ groups, one or two OC(O)R₁₁ groups, one or two NR₁₂S(O)ₙR₁₃ groups, one or two C(O)NR₁₂S(O)ₙR₁₃ groups,
one 4- to 10-membered heterocyclic ring optionally substituted with any combination of one or two oxo groups, one or two thioxo groups, one to three halogen atoms, one to three nitro groups, one to three cyano groups, one to three C₁-C₄alkyl groups, one to three C₁-C₄haloalkyl groups, one to three C₁-C₄alkoxy groups, one to three C₁-C₄haloalkoxy groups, one or two C₂-C₆alkoxyalkyl groups, one or two C₂-C₆-haloalkoxyalkyl groups, one or two C(O)OR₁₄ groups, one or two C(O)R₁₄ groups, one to three C₃-C₆alkoxycarbonylalkyl groups, one to three C₃-C₆haloalkoxycarbonylalkyl groups, one to three C₃-C₆alkylcarbonyalkyl groups, one to three C₃-C₆haloalkylcarbonylalkyl groups, one or two NR₁₅S(O)ₚR₁₆ groups or one or two C(O)NR₁₅S(O)ₚR₁₆groups, or
one phenyl group optionally substituted with any combination of one to three halogen atoms, one to three nitro groups, one to three cyano groups, one to three C₁-C₄alkyl groups, one to three C₁-C₄haloalkyl groups, one to three C₁-C₄alkoxy groups, one to three C₁-C₄haloalkoxy groups, one or two C₂-C₆alkoxyalkyl groups, one or two C₂-C₆haloalkoxyalkyl groups, one or two C(O)OR₁₄groups, one or two C(O)R₁₄ groups, one to three C₃-C₆alkoxycarbonylalkyl groups, one to three C₃-C₆haloalkoxycarbonylalkyl groups, one to three C₃-C₆₋ alkylcarbonylalkyl groups, one to three C₃-C₆haloalkylcarbonylalkyl groups, one or two NR₁₅S(O)ₚR₁₆ groups or one or two C(O)NR₁₅S(O)ₚR₁₆ groups,
phenyl optionally substituted with any combination of one to three halogen atoms, one to three nitro groups, one to three cyano groups, one to three C₁-C₄alkyl groups, one to three C₁-C₄-haloalkyl groups, one to three C₁-C₄alkoxy groups, one to three C₁-C₄haloalkoxy groups, one or two C₂-C₆alkoxyalkyl groups, one or two C₂-C₆haloalkoxyalkyl groups, one or two C(O)OR₁₄ groups, one or two C(O)R₁₄ groups, one to three C₃-C₆alkoxycarbonylalkyl groups, one to three C₃-C₆haloalkoxycarbonylalkyl groups, one to three C₃-C₆alkylcarbonylalkyl groups, one to three C₃-C₆haloalkylcarbonylalkyl groups or one or two NR₁₅S(O)ₚR₁₆ groups,
OR₁₇,
NR₁₈R₁₉,
NR₂₀S(O)_{q}R₂₁,
NR₂₂C(O)R₂₃,
C(O)NR₂₀S(O)_{q}R₂₁,
S(O)_{q}R₂₆,
C(O)R₂₈ or
C(O)OR₂₉;
Z is O;
R₁₄ is C₁-C₄alkyl or C₁-C₄haloalkyl;
R₂ is hydrogen, C₁-C₆alkyl, C₃-C₇cycloalkyl, C₁-C₆-haloalkyl, C₂-C₆alkoxyalkyl, C₃-C₆alkoxycarbonylalkyl, or
phenyl optionally substituted with any combination of one to three halogen atoms, one to three C₁-C₄alkyl groups, one to three C₁-C₄alkoxy groups, one or two C₂-C₆alkoxyalkyl groups, one C₂-C₆alkoxycarbonyl group, one C₃-C₆alkoxycarbonylalkyl group or one C₃-C₆haloalkoxycarbonylalkyl group;
R₃ is hydrogen, C₁-C₆alkyl, C₁-C₆haloalkyl, C₃-C₇cycloalkyl, C₃-C₆alkenyl, C₃-C₆alkynyl, C₂-C₆alkoxyalkyl, C₂-C₆haloalkoxyalkyl, C₃-C₆alkoxycarbonylalkyl,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups,
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups,
furfural optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, or
an alkali metal, alkaline earth metal, manganese, copper, zinc, cobalt, silver, nickel, ammonium or organic ammonium cation;
R₄ and R₅ are each independently hydrogen, C₁-C₆alkyl, C₃-C₇cycloalkyl, C₁-C₆alkoxy, hydroxy, C₃-C₆alkenyl, C₃-C₆alkylcarbonylalkyl, C₃-C₆alkoxycarbonylalkyl, C₂-C₆alkylsulfonylalkyl, C₂-C₆haloalkylsulfonylalkyl, S(O)ₚR₁₆,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, or
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups,
provided that only one of R₄ and R₅ can be hydroxy or C₁-C₆alkoxy, and
when R₄ and R₅ are taken together with the atom to which they are attached, they represent a three- to seven-membered saturated or unsaturated ring optionally interrupted by oxygen, sulfur or nitrogen, and optionally substituted with one to three methyl groups or one or more halogen atoms;
R₆ is hydrogen, C₁-C₆alkyl, C₁-C₆haloalkyl,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, or
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups;
R₇ and R₈ are each independently hydrogen, C₁-C₆-alkyl, C₂-C₆alkoxycarbonylalkyl or C₂-C₆hydroxycarbonylalkyl;
R₁₇ is hydrogen, C₁-C₆alkyl, C₃-C₇cycloalkyl, C₃-C₆alkenyl, C₃-C₆alkynyl, C₂-C₆alkoxyalkyl, C₂-C₆haloalkoxyalkyl, C₃-C₆alkylcarbonylalkyl, C₂-C₆haloalkylcarbonylalkyl, C₃-C₆alkoxycarbonylalkyl, C₂-C₆hydroxycarbonylalkyl,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, or
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups;
R₉ and R₁₀ are each independently hydrogen, C₁-C₆alkyl, C₃-C₆alkylcarbonylalkyl, C₃-C₆alkoxycarbonylalkyl, C₂-C₆hydroxycarbonylalkyl, or
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, and
when R₉ and R₁₀ are taken together with the atom to which they are attached, they represent a three- to seven-membered saturated or unsaturated ring optionally interrupted by oxygen, sulfur or nitrogen, and optionally substituted with one to three methyl groups or one or more halogen atoms;
R₁₈ and R₁₉ are each independently hydrogen, C₁-C₆alkyl, C₃-C₆alkoxycarbonylalkyl, C₃-C₆haloalkoxycarbonylalkyl, C₂-C₆hydroxycarbonylalkyl, C₂-C₆alkylsulfonylalkyl, C₂-C₆haloalkylsulfonylalkyl,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups,
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, or
furfural optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, and
when R₁₈ and R₁₉ are taken together with the atom to which they are attached, they represent a three- to seven-membered saturated or unsaturated ring optionally interrupted by oxygen, sulfur or nitrogen, and optionally substituted with one to three methyl groups or one or more halogen atoms;
R₁₁ is hydrogen, C₁-C₆alkyl or C₁-C₆haloalkyl;
R₁₂, R₁₅ and R₂₀ are each independently hydrogen or C₁-C₆alkyl;
R₁₃, R_{16,} R₂₁ and R₂₆ are each independently C₁-C₆alkyl, C₁-C₆haloalkyl, C₃-C₇cycloalkyl, C₃-C₆alkenyl, C₃-C₆haloalkenyl, C₃-C₆alkynyl, C₃-C₆haloalkynyl, or
benzyl optionally substituted on the phenyl ring with any combination of one to three halogen atoms, one to three C₁-C₄alkyl groups, one to three C₁-C₄haloalkyl groups, one to three C₁-C₄alkoxy groups or one to three C₁-C₄haloalkoxy groups;
R₂₂ is hydrogen or C₁-C₆alkyl;
R₂₃ is hydrogen, C₁-C₆alkyl, C₁-C₆haloalkyl, C₃-C₇cycloalkyl, C₃-C₆alkylcarbonylalkyl, C₃-C₆haloalkylcarbonylalkyl, C₃-C₆alkoxycarbonylalkyl, C₃-C₆haloalkoxycarbonylalkyl,
phenyl optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups, or
benzyl optionally substituted on the phenyl ring with any combination of one to three halogen atoms, one to three C₁-C₄alkyl groups, one to three C₁-C₄haloalkyl groups, one to three C₁-C₄alkoxy groups or one to three Cₗ-C₄₋ haloalkoxy groups;
R₂₈ is hydrogen, C₁-C₆alkyl, C₁-C₆haloalkyl, C₃-C₇-cycloalkyl, C₃-C₆alkenyl, C₃-C₆alkynyl, C₃-C₆alkoxycarbonylalkyl, or
phenyl optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups;
R₂₉ is hydrogen, C₁-C₆alkyl or benzyl;
A is -O-, -S(O)ᵣ-, or -NR₃₄-;
B is -CR₃₇R₃₈-, -C(O)- or -C(=CR₄₁R₄₂)-;
R₃₄ is hydrogen,
C₁-C₆alkyl optionally substituted with one to three halogen atoms, one or two cyano groups, one or two nitro groups, one or two C(O)R₂ groups, one or two C(O)OR₃ groups, one or two C(O)NR₄R₅ groups, one or two P(O) (OR₆)₂ groups, one or two OR₇ groups, one or two SR₈ groups, one or two NR₉R₁₀ groups or one or two OC(O)R₁₁ groups,
C₃-C₇cycloalkyl,
C₃-C₆alkenyl,
C₃-C₆alkynyl,
C(O)NR₁₂S(O)ₙR₁₃.
phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one or two C₂-C₆₋ alkoxyalkyl groups, one or two nitro groups, one or two cyano groups, one C₂-C₆alkoxycarbonyl group, one C₂-C₆alkylcarbonyl group, one C₃-C₆₋ alkoxycarbonylalkyl group or one C₃-C₆alkylcarbonylalkyl group, or
C₁-C₄alkyl substituted with one phenyl group wherein the phenyl group is optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one or two C₂-C₆₋ alkoxyalkyl groups, groups, one C₂-C₆alkoxycarbonyl group, one C₂-C₆alkylcarbonyl group, one C₃-C₆alkoxycarbonylalkyl group or one C₃-C₆alkylcarbonylalkyl group;
R₃₇ and R₃₈ are each independently hydrogen, halogen,
C₁-C₆alkyl optionally substituted with one to three halogen atoms, one or two cyano groups, one or two nitro groups, one or two C(O)R₂ groups, one or two C(O)OR₃ groups, one or two C(O)NR₄R₅ groups, one or two P(O) (OR₆)₂ groups, one or two OR₇ groups, one or two SR₈ groups, one or two NR₉R₁₀ groups or one or two OC(O)R₁₁ groups,
C₃-C₇cycloalkyl,
C₃-C₆alkenyl,
C₃-C₆haloalkenyl
C₃-C₆alkynyl,
C₃-C₆haloalkynyl,
C(O)NR₁₂S(O)ₙR_{13,}
phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one or two C₂-C₆alkoxyalkyl groups, one or two nitro groups, one or two cyano groups, one C₂-C₆₋ alkoxycarbonyl group, one C₂-C₆alkylcarbonyl group, one C₃-C₆alkoxycarbonylalkyl group or one C₃-C₆alkylcarbonylalkyl group, or
C₁-C₄alkyl substituted with one phenyl group wherein the phenyl group is optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one or two C₂-C₆alkoxyalkyl groups, one C₂-C₆alkoxycarbonyl group, one C₂-C₆alkylcarbonyl group, one C₃-C₆alkoxycarbonylalkyl group or one C₃-C₆-alkylcarbonylalkyl group, and
when R₃₇ and R₃₈ are taken together with the atom to which they are attached, they represent a ring in which R₃₇R₃₈ is a C₂-C₆alkylene group;
R₄₁ and R₄₂ are each independently hydrogen, C₁-C₆alkyl, C₂-C₆alkylcarbonyl, C₂-C₆alkoxycarbonyl, hydroxycarbonyl, C₃-C₆alkylcarbonylalkyl, C₃-C₆alkoxycarbonylalkyl, C₂-C₆hydroxycarbonylalkyl,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups,
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, or
furfural optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, and
when R₄₁ and R₄₂ are taken together with the atom to which they are attached, they represent a three- to seven-membered saturated or unsaturated ring optionally interrupted with one group selected from O, S(O)ᵣ or NR₃₁, and optionally substituted with one to three methyl groups or one or more halogen atoms;
r is an integer of O, 1 or 2;
n, p and q are each independently an integer of 1 or 2;
Q is selected from 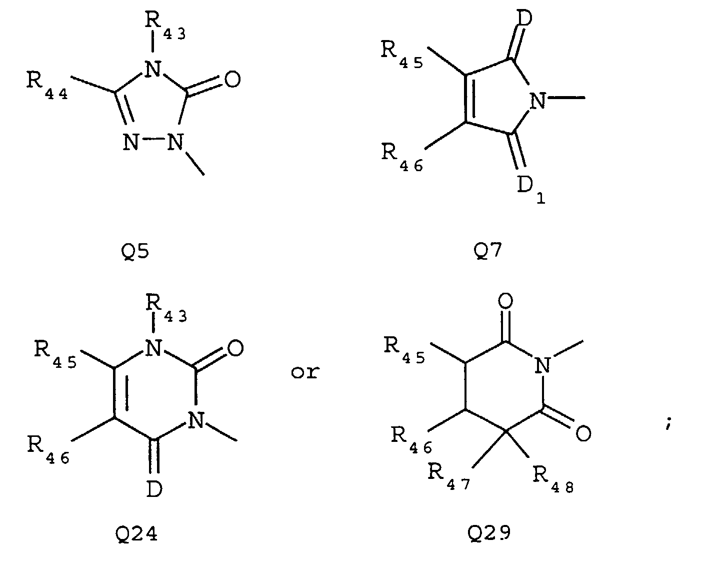
D and D₁ are each independently O or S;
R₄₃ and R₄₄ are each independently hydrogen, C₁-C₆alkyl, C₁-C₆haloalkyl, C₃-C₇cycloalkyl, C₃-C₆alkenyl, C₃-C₆haloalkenyl, C₃-C₆alkynyl, amino, or benzyl;
R₄₅ and R₄₆ are each independently hydrogen, halogen, C₁-C₆alkyl, C₁-C₆haloalkyl, C₃-C₇cycloalkyl, C₃-C₆alkenyl, C₃-C₆haloalkenyl or C₃-C₆alkynyl, and when R₄₅ and R₄₆ are taken together with the atom to which they are attached, they represent a three- to seven-membered saturated or unsaturated ring optionally interrupted by oxygen, sulfur or nitrogen, and optionally substituted with one to three methyl groups or one or more halogen atoms; and
R₄₇ and R₄₈ are each independently hydrogen, halogen or C₁-C₆alkyl.

3. The compound according to claim 2 wherein
X is hydrogen, fluorine or chlorine;
Y is fluorine, chlorine, nitro or cyano;
R is hydrogen,
C₁-C₈alkyl optionally substituted with any combination of one to six halogen atoms, one or two cyano groups, one or two nitro groups, one or two C(O)R₂ groups, one or two C(O)OR₃ groups, one or two C(O)NR₄R₅ groups, one or two P(O) (OR₆)₂ groups, one or two OR₇ groups, one or two SR₈ groups, one or two NR₉R₁₀ groups, one or two C(O)NR₁₂S(O)ₙR₁₃ groups,
one furyl group optionally substituted with any combination of one to three C₁-C₄alkyl groups, one to three C₁-C₄alkoxy groups, one or two C(O)OR₁₄groups or one or two C(O)R₁₄ groups,
one pyridyl group optionally substituted with any combination of one to three C₁-C₄alkyl groups, one to three C₁-C₄alkoxy groups, one or two C(O)OR₁₄ groups or one or two C(O)R₁₄ groups, or
one phenyl group optionally substituted with any combination of one to three C₁-C₄alkyl groups, one to three C₁-C₄alkoxy groups, one or two C(O)OR₁₄ groups or one or two C(O)R₁₄ groups,
phenyl optionally substituted with any combination of one to three C₁-C₄alkyl groups, one to three C₁-C₄alkoxy groups, one or two C₂-C₆alkoxyalkyl groups, one or two C(O)OR₁₄ groups, one or two C(O)R₁₄ groups, one to three C₃-C₆alkoxycarbonylalkyl groups or one or two NR₁₅S(O)ₚR₁₆ groups,
C₃-C₇cycloalkyl,
C₃-C₈alkenyl,
C₃-C₈alkynyl,
OR₁₇,
NR₁₈R₁₉,
C(O)R₂₈ or
C(O)OR₂₉;
Z is O;
R₁₄ is C₁-C₄alkyl;
R₂ is hydrogen, C₁-C₆alkyl, C₂-C₆alkoxyalkyl or C₃-C₆-alkoxycarbonylalkyl;
R₃ is hydrogen, C₁-C₆alkyl, C₁-C₆haloalkyl, C₃-C₆alkenyl, C₃-C₆alkynyl, C₂-C₆alkoxyalkyl, C₂-C₆haloalkoxyalkyl, C₃-C₆alkoxycarbonylalkyl,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, or
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄-haloalkoxy groups;
R₄ and R₅ are each independently hydrogen, C₁-C₆alkyl, C₃-C₆alkoxycarbonylalkyl,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, or
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, and
when R₄ and R₅ are taken together with the atom to which they are attached, they represent a ring in which R₄R₅ is a C₂-C₆alkylene group;
R₆ is hydrogen, C₁-C₆alkyl or benzyl;
R₇ and R₈ are each independently hydrogen, C₁-C₆-alkyl, C₂-C₆alkoxycarbonylalkyl or C₂-C₆hydroxycarbonylalkyl;
R₁₇ is hydrogen, C₁-C₆alkyl, C₃-C₆alkenyl, C₃-C₆alkynyl, C₂-C₆alkoxyalkyl, C₃-C₆alkoxycarbonylalkyl, C₂-C₆-hydroxycarbonylalkyl,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, or
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups;
R₉ and R₁₀ are each independently hydrogen, C₁-C₆alkyl or C₃-C₆alkoxycarbonylalkyl, and
when R₉ and R₁₀ are taken together with the atom to which they are attached, they represent a ring in which R₉R₁₀ is a C₂-C₆alkylene group;
R₁₈ and R₁₉ are each independently hydrogen, C₁-C₆alkyl, C₃-C₆alkoxycarbonylalkyl, C₂-C₆hydroxycarbonylalkyl, C₂-C₆alkylsulfonylalkyl, C₂-C₆haloalkylsulfonylalkyl, or
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, and
when R₁₈ and R₁₉ are taken together with the atom to which they are attached, they represent a ring in which R₁₈R₁₉ is a C₂-C₆alkylene group;
R₁₂ and R₁₅ are each independently hydrogen or C₁-C₆alkyl;
R₁₃ and R₁₆ are each independently C₁-C₆alkyl, C₁-C₆haloalkyl, C₃-C₇cycloalkyl, C₃-C₆alkenyl, C₃-C₆haloalkenyl, C₃-C₆alkynyl, C₃-C₆haloalkynyl, or
benzyl optionally substituted on the phenyl ring with any combination of one to three halogen atoms, one to three C₁-C₄alkyl groups, one to three C₁-C₄haloalkyl groups, one to three C₁-C₄-alkoxy groups or one to three C₁-C₄haloalkoxy groups;
R₂₈ is hydrogen, C₁-C₆alkyl, C₁-C₆haloalkyl, C₃-C₆alkenyl C₃-C₆alkynyl, C₃-C₆alkoxycarbonylalkyl, or
phenyl optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups;
R₂₉ is hydrogen, C₁-C₆alkyl or benzyl;
A is -O-, -S(O)ᵣ-, or -NR₃₄-;
B is -CR₃₇R₃₈- or -C(=CR₄₁R₄₂)-;
R₃₄ is hydrogen,
C₁-C₆alkyl optionally substituted with one or two C(O)OR₃ groups or one or two C(O)NR₄R₅ groups,
C₃-C₆alkenyl,
C₃-C₆alkynyl,
phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one or two C₂-C₆-alkoxyalkyl groups, one or two nitro groups, one or two cyano groups, one C₂-C₆alkoxycarbonyl group, one C₂-C₆alkylcarbonyl group, one C₃-C₆-alkoxycarbonylalkyl group or one C₃-C₆alkylcarbonylalkyl group, or
benzyl optionally substituted on the phenyl ring with any combination of one to three halogen atoms, one to three C₁-C₄alkyl groups, one to three C₁-C₄haloalkyl groups, one to three C₁-C₄alkoxy groups or one to three C₁-C₄haloalkoxy groups;
R₃₇ and R₃₈ are each independently hydrogen, halogen,
C₁-C₆alkyl optionally substituted with one or two cyano groups, one or two C(O)R₂ groups, one or two C(O)OR₃ groups, one or two C(O)NR₄R₅ groups, one or two P(O) (OR₆)₂ groups, one or two OR₇ groups or one or two NR₉R₁₀ groups,
C₃-C₇cycloalkyl,
C₃-C₆alkenyl,
C₃-C₆alkynyl,
C(O)NR₁₂S(O)ₙR₁₃, or
benzyl optionally substituted on the phenyl ring with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one or two C₂-C₆alkoxyalkyl groups, one C₂-C₆alkoxycarbonyl group, one C₂-C₆alkylcarbonyl group, one C₃-C₆alkoxycarbonylalkyl group or one C₃-C₆alkylcarbonylalkyl group, and
when R₃₇ and R₃₈ are taken together with the atom to which they are attached, they represent a ring in which R₃₇R₃₈ is a C₂-C₆alkylene group;
R₄₁ and R₄₂ are each independently hydrogen, C₁-C₆alkyl, C₂-C₆alkylcarbonyl, C₂-C₆alkoxycarbonyl, hydroxycarbonyl, or
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups;
r is an integer of O, 1 or 2;
n and p are each independently an integer of 2; and
Q is selected from

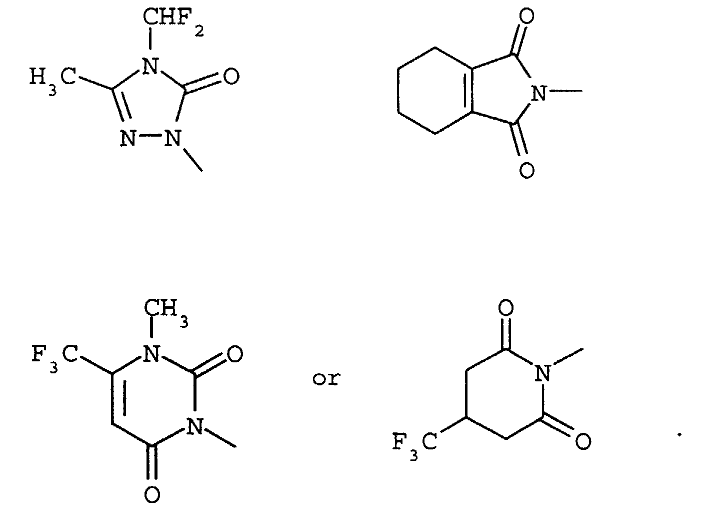

4. The compound according to claim 3 wherein
X is fluorine;
Y is chlorine;
R is C₁-C₈alkyl optionally substituted with any combination of one or two C(O)OR₃ groups, one or two
C(O)NR₄R₅ groups, one or two P(O) (OR₆)₂ groups,
one or two OR₇ groups, one or two SR₈ groups,
one or two NR₉R₁₀ groups, one or two
C(O)NR₁₂S(O)ₙR₁₃ groups, one furyl group or
one phenyl group optionally substituted with any combination of one to three halogen atoms, one to three C₁-C₄alkyl groups, one to three C₁-C₄alkoxy groups or one nitro group,
phenyl optionally substituted with any combination of one to three C₁-C₄alkyl groups, one to three C₁-C₄alkoxy groups, one or two C₂-C₆alkoxyalkyl groups, one or two C₃-C₆alkoxycarbonylalkyl groups or one or two NR₁₅S(O)ₚR₁₆ groups,
C₃-C₇cycloalkyl,
C₃-C₈alkenyl or
C₃-C₈alkynyl;
Z is O;
R₃ is hydrogen, C₁-C₆alkyl, C₁-C₆haloalkyl, C₃-C₆alkenyl, C₃-C₆alkynyl, C₃-C₆alkoxycarbonylalkyl,
phenyl optionally substituted with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄haloalkoxy groups, or
benzyl optionally substituted on the phenyl ring with any combination of one to three halogen atoms, one or two cyano groups, one or two nitro groups, one to three C₁-C₄alkyl groups, one to three C₁-C₄haloalkyl groups, one to three C₁-C₄alkoxy groups or one to three C₁-C₄-haloalkoxy groups;
R₄ and R₅ are each independently hydrogen or C₁-C₆alkyl, and when R₄ and R₅ are taken together with the atom to which they are attached, they represent a ring in which R₄R₅ is a C₂-C₆alkylene group;
R₆ is hydrogen or C₁-C₆alkyl;
R₇ and R₈ are each independently hydrogen, C₁-C₆alkyl C₂-C₆alkoxycarbonylalkyl or C₂-C₆hydroxycarbonylalkyl;
R₉ and R₁₀ are each independently hydrogen, C₁-C₆alkyl or C₃-C₆alkoxycarbonylalkyl, and when R₉ and R₁₀ are taken together with the atom to which they are attached, they represent a ring in which R₉R₁₀ is a C₂-C₆alkylene group;
R₁₂ and R₁₅ are hydrogen;
R₁₃ and R₁₆ are each independently C₁-C₆alkyl, C₁-C₆-haloalkyl, C₃-C₆alkenyl, C₃-C₆alkynyl, or
benzyl optionally substituted on the phenyl ring with any combination of one to three halogen atoms, one to three C₁-C₄alkyl groups, one to three C₁-C₄haloalkyl groups, one to three C₁-C₄-alkoxy groups or one to three C₁-C₄haloalkoxy groups;
A is -O- or -S(O)ᵣ-;
B is -CR₃₇R₃₈- or -C(=CR₄₁R₄₂)-;
R₃₇ and R₃₈ are each independently hydrogen, halogen,
C₁-C₆alkyl optionally substituted with one or two C(O)OR₃ groups, one or two C(O)NR₄R₅ groups, one or two OR₇ groups or one or two NR₉R₁₀ groups, or
benzyl optionally substituted on the phenyl ring with any combination of one to three C₁-C₄alkyl groups, one to three C₁-C₄alkoxy groups or one C₃-C₆alkoxycarbonylalkyl group, and
when R₃₇ and R₃₈ are taken together with the atom to which they are attached, they represent a ring in which R₃₇R₃₈ is a C₂-C₆alkylene group;
R₄₁ and R₄₂ are each independently hydrogen, C₁-C₆alkyl, C₂-C₆alkylcarbonyl, C₂-C₆alkoxycarbonyl or hydroxycarbonyl;
r is an integer of O, 1 or 2;
n and p are each independently an integer of 2; and
Q is selected from 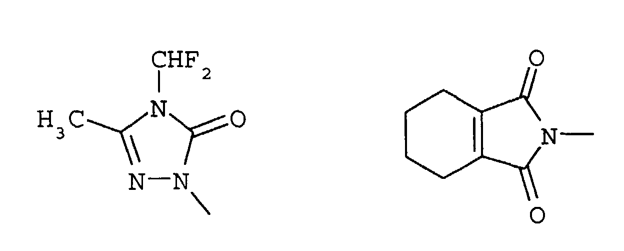 or

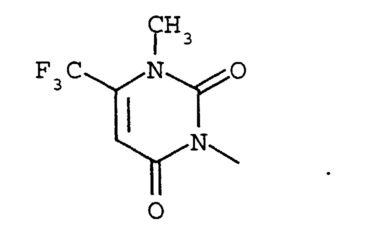

5. The compound according to claim 4 selected from the group consisting of
N-{4-chloro-2-fluoro-5-[(3-methyl-4-oxo-2-thiazolidinylidene)amino]phenyl}-1-cyclohexene-1,2-dicarboximide;
3-{4-chloro-2-fluoro-5-[3-methyl-4-oxo-2-thiazolidinylidene)amino]phenyl}-1-methyl-6-(trifluoromethyl)-2,4(1H,3H)-pyrimidinedione;
ethyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
3-(carboxymethyl)-2-{{2-chloro-5-[3,6-dihydro-3-methyl-2,6-dioxo-4-(trifluoromethyl)-1(2H)-pyrimidinyl]-4-fluorophenyl}imino}-4-oxo-delta5, alpha-thiazolidineacetic acid, diethyl ester;
ethyl 5-{{2-chloro-5-[3,6-dihydro-3-methyl-2,6-dioxo-4-(trifluoromethyl)-1(2H)-pyrimidinyl]-4-fluorophenyl}imino}-7-oxo-4-thia-6-azaspiro[2.4]heptane-6-acetate;
2-{{2-chloro-5-[3,6-dihydro-3-methyl-2,6-dioxo-4-(trifluoromethyl)-1(2H)-pyrimidinyl]-4-fluorophenyl}-4-oxo-3,5-thiazolidinediacetic acid, 3-ethyl ester;
ethyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-oxazolidineacetate;
α-benzyl-2-{{2-chloro-5-[3,6-dihydro-3-methyl-2,6-dioxo-4-(trifluoromethyl)-1(2H)-pyrimidinyl]-4-fluorophenyl}imino)-4-oxo-3-thiazolidineacetic acid, L-;
2-{{2-chloro-5-[3,6-dihydro-3-methyl-2,6-dioxo-4-(trifluoromethyl)-1(2H)-pyrimidinyl]-4-fluorophenyl}imino)-4-oxo-3,5-thiazolidinediacetic acid, 3-ethyl methyl ester;
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid, 3-ethyl isopropyl ester;
ethyl 5-(carbamoylmethyl)-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
ethyl 2-{{2-chloro-5-[3,6-dihydro-3-methyl-2,6-dioxo-4-(trifluoromethyl)-1(2H)-pyrimidinyl]-4-fluorophenyl}imino}-5-[(methylcarbamoyl)methyl]-4-oxo-3-thiazolidineacetate;
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid, 3-ethyl (2-propynyl) ester;
methyl 3-allyl-2-{{2-chloro-5-[3,6-dihydro-3-methyl-2,6-dioxo-4-(trifluoromethyl)-1(2H)-pyrimidinyl]-4-fluorophenyl}imino}-4-oxo-5-thiazolidineacetate;
isopropyl 3-allyl-2-{{2-chloro-5-[3,6-dihydro-3-methyl-2,6-dioxo-4-(trifluoromethyl)-1(2H)-pyrimidinyl]-4-fluorophenyl}imino}-4-oxo-5-thiazolidineacetate;
methyl α-benzyl-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate, L-;
2-{{2-chloro-5-[3,6-dihydro-3-methyl-2,6-dioxo-4-(trifluoromethyl)-1(2H)-pyrimidinyl]-4-fluorophenyl}imino)-4-oxo-3,5-thiazolidinediacetic acid, 3-ethyl 2-fluoroethyl ester;
2-fluoroethyl 3-allyl-2-{{2-chloro-5-[3,6-dihydro-3-methyl-2,6-dioxo-4-(trifluoromethyl)-1(2H)-pyrimidinyl]-4-fluorophenyl}imino}-4-oxo-5-thiazolidineacetate;
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-oxazolidinediacetic acid, 3-ethyl ester;
ethyl 2-{{2-chloro-5-[3,6-dihydro-3-methyl-2,6-dioxo-4-(trifluoromethyl)-1(2H)-pyrimidinyl]-4-fluorophenyl}imino}-5-(hydroxyethyl)-4-oxo-3-thiazolidineacetate;
allyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
isopropyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
3-{4-chloro-2-fluoro-5-[(3-furfuryl-4-oxo-2-thiazolidinylidene)amino]phenyl}-1-methyl-6-(trifluoromethyl)-2,4(1H,3H)-pyrimidinedione;
cyclopropylmethyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
ethyl 5-chloro-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
3-{4-chloro-5-[(3-cyclopropyl-4-oxo-2-thiazolidinylidene)amino]-2-fluorophenyl}-1-methyl-6-(trifluoromethyl)-2,4(1H,3H)-pyrimidinedione;
3-{4-chloro-2-fluoro-5-{[3-(2-methoxyethyl)-4-oxo-2-thiazolidinylidene]amino}phenyl}-1-methyl-6-(trifluoromethyl)-2,4(1H,3H)-pyrimidinedione;
3-{4-chloro-2-fluoro-5-{[3-(1-methyl-2-propynyl)-4-oxo-2-thiazolidinylidene]amino}phenyl}-1-methyl-6-(trifluoromethyl)-2,4(1H,3H)-pyrimidinedione;
ethyl 5-benzyl-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
3-{5-[(3-benzyl-4-oxo-2-thiazolidinylidene)amino]-4-chloro-2-fluorophenyl}-1-methyl-6-(trifluoromethyl)-2,4(1H,3H)-pyrimidinedione;
methyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidinepropionate;
methyl α-benzyl-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate, D-;
methyl 2-{{2-chloro-5-[3,6-dihydro-3-methyl-2,6-dioxo-4-(trifluoromethyl)-1(2H)-pyrimidinyl]-4-fluorophenyl}imino}-4-oxo-α-(*p*-nitrobenzyl)-3-thiazolidineacetate, L-;
ethyl α-benzyl-2-{{2-chloro-5-[3,6-dihydro-3-methyl-2,6-dioxo-4-(trifluoromethyl)-1(2H)-pyrimidinyl]-4-fluorophenyl}imino)-4-oxo-3-thiazolidineacetate, L-; and
methyl α-(*p*-chlorobenzyl)-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate, DL-.

6. A method for the control of undesirable plant species which comprises applying to the foliage of said plants or to the soil or water containing seeds or other propagating organs thereof, a herbicidally effective amount of a compound having the structural formula I 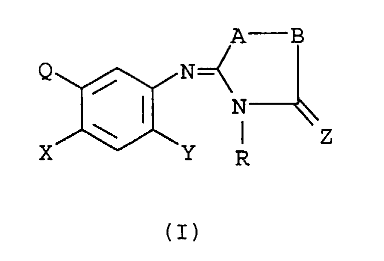 wherein A, B, Q, R, X, Y and Z are as described in claim 1.

7. The method according to claim 6 wherein A, B, Q, R, X, Y and Z are as described in claim 2.

8. The method according to claim 7 wherein A, B, Q, R, X, Y and Z are as described in claim 3.

9. The method according to claim 8 wherein A, B, Q, R, X, Y and Z are as described in claim 4.

10. The method according to claim 9 wherein the compound is selected from the group consisting of
N-{4-chloro-2-fluoro-5-[(3-methyl-4-oxo-2-thiazolidinylidene)amino]phenyl}-1-cyclohexene-1,2-dicarboximide;
3-{4-chloro-2-fluoro-5-[3-methyl-4-oxo-2-thiazolidinylidene)amino]phenyl}-1-methyl-6-(trifluoromethyl)-2,4(1H,3H)-pyrimidinedione;
ethyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
3-(carboxymethyl)-2-{{2-chloro-5-[3,6-dihydro-3-methyl-2,6-dioxo-4-(trifluoromethyl)-1(2H)-pyrimidinyl]-4-fluorophenyl}imino}-4-oxo-delta5, alpha-thiazolidineacetic acid, diethyl ester;
ethyl 5-{{2-chloro-5-[3,6-dihydro-3-methyl-2,6-dioxo-4-(trifluoromethyl)-1(2H)-pyrimidinyl]-4-fluorophenyl}imino}-7-oxo-4-thia-6-azaspiro[2.4]heptane-6-acetate;
2-{{2-chloro-5-[3,6-dihydro-3-methyl-2,6-dioxo-4-(trifluoromethyl)-1(2H)-pyrimidinyl]-4-fluorophenyl}-4-oxo-3,5-thiazolidinediacetic acid, 3-ethyl ester;
ethyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-oxazolidineacetate;
α-benzyl-2-{{2-chloro-5-[3,6-dihydro-3-methyl-2,6-dioxo-4-(trifluoromethyl)-1(2H)-pyrimidinyl]-4-fluorophenyl)imino)-4-oxo-3-thiazolidineacetic acid, L-;
2-{{2-chloro-5-[3,6-dihydro-3-methyl-2,6-dioxo-4-(trifluoromethyl)-1(2H)-pyrimidinyl]-4-fluorophenyl}imino)-4-oxo-3,5-thiazolidinediacetic acid, 3-ethyl methyl ester;
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid, 3-ethyl isopropyl ester;
ethyl 5-(carbamoylmethyl)-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
ethyl 2-{{2-chloro-5-[3,6-dihydro-3-methyl-2,6-dioxo-4-(trifluoromethyl)-1(2H)-pyrimidinyl]-4-fluorophenyl}imino}-5-[(methylcarbamoyl)methyl]-4-oxo-3-thiazolidineacetate;
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid, 3-ethyl (2-propynyl) ester;
methyl 3-allyl-2-{{2-chloro-5-[3,6-dihydro-3-methyl-2,6-dioxo-4-(trifluoromethyl)-1(2H)-pyrimidinyl]-4-fluorophenyl}imino}-4-oxo-5-thiazolidineacetate;
isopropyl 3-allyl-2-{{2-chloro-5-[3,6-dihydro-3-methyl-2,6-dioxo-4-(trifluoromethyl)-1(2H)-pyrimidinyl]-4-fluorophenyl}imino}-4-oxo-5-thiazolidineacetate;
methyl α-benzyl-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate, L-;
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid, 3-ethyl 2-fluoroethyl ester;
2-fluoroethyl 3-allyl-2-{{2-chloro-5-[3,6-dihydro-3-methyl-2,6-dioxo-4-(trifluoromethyl)-1(2H)-pyrimidinyl]-4-fluorophenyl}imino}-4-oxo-5-thiazolidineacetate;
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-oxazolidinediacetic acid, 3-ethyl ester;
ethyl 2-{{2-chloro-5-[3,6-dihydro-3-methyl-2,6-dioxo-4-(trifluoromethyl)-1(2H)-pyrimidinyl]-4-fluorophenyl}imino}-5-(hydroxyethyl)-4-oxo-3-thiazolidineacetate;
allyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
isopropyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
3-{4-chloro-2-fluoro-5-[(3-furfuryl-4-oxo-2-thiazolidinylidene)amino]phenyl}-1-methyl-6-(trifluoromethyl)-2,4(1H,3H)-pyrimidinedione;
cyclopropylmethyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
ethyl 5-chloro-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
3-{4-chloro-5-[(3-cyclopropyl-4-oxo-2-thiazolidinylidene)amino]-2-fluorophenyl}-1-methyl-6-(trifluoromethyl)-2,4(1H,3H)-pyrimidinedione;
3-{4-chloro-2-fluoro-5-{[3-(2-methoxyethyl)-4-oxo-2-thiazolidinylidene]amino}phenyl}-1-methyl-6-(trifluoromethyl)-2,4(1H,3H)-pyrimidinedione;
3-{4-chloro-2-fluoro-5-{[3-(1-methyl-2-propynyl)-4-oxo-2-thiazolidinylidene]amino}phenyl}-1-methyl-6-(trifluoromethyl)-2,4(1H,3H)-pyrimidinedione;
ethyl 5-benzyl-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
3-{5-[(3-benzyl-4-oxo-2-thiazolidinylidene)amino]-4-chloro-2-fluorophenyl}-1-methyl-6-(trifluoromethyl)-2,4(1H,3H)-pyrimidinedione;
methyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidinepropionate;
methyl α-benzyl-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate, D-;
methyl 2-{{2-chloro-5-[3,6-dihydro-3-methyl-2,6-dioxo-4-(trifluoromethyl)-1(2H)-pyrimidinyl]-4-fluorophenyl}imino}-4-oxo-α-(*p*-nitrobenzyl)-3-thiazolidineacetate, L-;
ethyl α-benzyl-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate, L-; and
methyl α-(*p*-chlorobenzyl)-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate, DL-.

11. The method according to claim 6 wherein the compound is applied to the foliage of the undesirable plant species or to the soil or water containing seeds or other propagating organs thereof in the presence of crop plants, crop seeds or other crop propagating organs.

12. The method according to claim 11 wherein the crop is a cereal crop.

13. The method according to claim 12 wherein the cereal crop is selected from the group consisting of corn, wheat and rice.

14. The method according to claim 13 wherein the cereal crop is corn and the compound is selected from the group consisting of
N-{4-chloro-2-fluoro-5-[(3-methyl-4-oxo-2-thiazolidinylidene)amino]phenyl}-1-cyclohexene-1,2-dicarboximide;
ethyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
2-{{2-chloro-5-[3,6-dihydro-3-methyl-2,6-dioxo-4-(trifluoromethyl)-1(2H)-pyrimidinyl]-4-fluorophenyl}-4-oxo-3,5-thiazolidinediacetic acid, 3-ethyl ester;
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid, 3-ethyl methyl ester;
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid, 3-ethyl isopropyl ester;
ethyl 5-(carbamoylmethyl)-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid, 3-ethyl (2-propynyl) ester;
isopropyl 3-allyl-2-{{2-chloro-5-[3,6-dihydro-3-methyl-2,6-dioxo-4-(trifluoromethyl)-1(2H)-pyrimidinyl]-4-fluorophenyl}imino}-4-oxo-5-thiazolidineacetate;
methyl α-benzyl-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate, L-;
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid, 3-ethyl 2-fluoroethyl ester;
2-fluoroethyl 3-allyl-2-{{2-chloro-5-[3,6-dihydro-3-methyl-2,6-dioxo-4-(trifluoromethyl)-1(2H)-pyrimidinyl]-4-fluorophenyl}imino}-4-oxo-5-thiazolidineacetate; and
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-oxazolidinediacetic acid, 3-ethyl ester.

15. The method according to claim 13 wherein the cereal crop is wheat and the compound is selected from the group consisting of
3-(carboxymethyl)-2-{{2-chloro-5-[3,6-dihydro-3-methyl-2,6-dioxo-4-(trifluoromethyl)-1(2H)-pyrimidinyl]-4-fluorophenyl}imino}-4-oxo-delta5, alpha-thiazolidineacetic acid, diethyl ester;
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid, 3-ethyl methyl ester;
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid, 3-ethyl isopropyl ester;
ethyl 5-(carbamoylmethyl)-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
ethyl 2-{{2-chloro-5-[3,6-dihydro-3-methyl-2,6-dioxo-4-(trifluoromethyl)-1(2H)-pyrimidinyl]-4-fluorophenyl}imino)-5-[(methylcarbamoyl)methyl]-4-oxo-3-thiazolidineacetate;
2-{{2-chloro-5-[3,6-dihydro-3-methyl-2,6-dioxo-4-(trifluoromethyl)-1(2H)-pyrimidinyl]-4-fluorophenyl}imino)-4-oxo-3,5-thiazolidinediacetic acid, 3-ethyl (2-propynyl) ester;
isopropyl 3-allyl-2-{{2-chloro-5-[3,6-dihydro-3-methyl-2,6-dioxo-4-(trifluoromethyl)-1(2H)-pyrimidinyl]-4-fluorophenyl}imino}-4-oxo-5-thiazolidineacetate;
methyl α-benzyl-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate, L-;
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid, 3-ethyl 2-fluoroethyl ester;
2-fluoroethyl 3-allyl-2-{{2-chloro-5-[3,6-dihydro-3-methyl-2,6-dioxo-4-(trifluoromethyl)-1(2H)-pyrimidinyl]-4-fluorophenyl}imino}-4-oxo-5-thiazolidineacetate;
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-oxazolidinediacetic acid, 3-ethyl ester;
ethyl 2-{{2-chloro-5-[3,6-dihydro-3-methyl-2,6-dioxo-4-(trifluoromethyl)-1(2H)-pyrimidinyl]-4-fluorophenyl}imino}-5-(hydroxyethyl)-4-oxo-3-thiazolidineacetate; and
cyclopropylmethyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate.

16. The method according to claim 13 wherein the cereal crop is rice and the compound is selected from the group consisting of
ethyl 5-benzyl-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
3-{5-[(3-benzyl-4-oxo-2-thiazolidinylidene)amino]-4-chloro-2-fluorophenyl}-1-methyl-6-(trifluoromethyl)-2,4(1H,3H)-pyrimidinedione;
methyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidinepropionate;
methyl α-benzyl-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate, L-;
methyl α-benzyl-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate, D-;
methyl 2-{{2-chloro-5-[3,6-dihydro-3-methyl-2,6-dioxo-4-(trifluoromethyl)-1(2H)-pyrimidinyl]-4-fluorophenyl}imino}-4-oxo-α-(*p*-nitrobenzyl)-3-thiazolidineacetate, L-;
ethyl α-benzyl-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate, L-; and
methyl α-(*p*-chlorobenzyl)-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate, DL-.

17. The method according to claim 11 wherein the crop is soybean.

18. The method according to claim 17 wherein the compound is selected from the group consisting of
ethyl 5-(carbamoylmethyl)-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid, 3-ethyl 2-fluoroethyl ester;
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-oxazolidinediacetic acid, 3-ethyl ester;
allyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate; and
3-{4-chloro-2-fluoro-5-[(3-furfuryl-4-oxo-2-thiazolidinylidene)amino]phenyl}-1-methyl-6-(trifluoromethyl)-2,4(1H,3H)-pyrimidinedione.

19. The method according to claim 6 wherein the compound is applied to the foliage of the undesirable plant species or to the soil or water containing seeds or other propagating organs thereof at a rate of about 0.01 kg/ha to 1 kg/ha.

20. The method according to claim 6 wherein the compound is applied in combination with or in conjunction with one or more other biological compounds.

21. A herbicidal composition which comprises an inert solid or liquid carrier and a herbicidally effective amount of a compound having the structural formula I 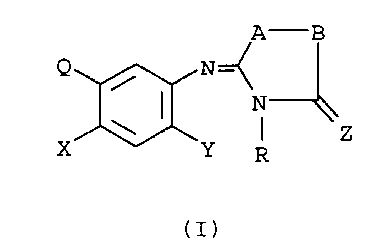 wherein A, B, Q, R, X, Y and Z are as described in claim 1.

22. The composition according to claim 21 wherein A, B, Q, R, X, Y and Z are as described in claim 2.

23. The composition according to claim 22 wherein A, B, Q, R, X, Y and Z are as described in claim 3.

24. The composition according to claim 23 wherein A, B, Q, R, X, Y and Z are as described in claim 4.

25. The composition according to claim 24 wherein the compound is selected from the group consisting of
N-{4-chloro-2-fluoro-5-[(3-methyl-4-oxo-2-thiazolidinylidene)amino]phenyl}-1-cyclohexene-1,2-dicarboximide;
3-{4-chloro-2-fluoro-5-[3-methyl-4-oxo-2-thiazolidinylidene)amino]phenyl}-1-methyl-6-(trifluoromethyl)-2,4(1H,3H)-pyrimidinedione;
ethyl 2-{{2-chloro-5-[3,6-dihydro-3-methyl-2,6-dioxo-4-(trifluoromethyl)-1(2H)pyrimidinyl]-4-fluorophenyl}imino}-4-oxo-3-thiazolidineacetate;
3-(carboxymethyl)-2-{{2-chloro-5-[3,6-dihydro-3-methyl-2,6-dioxo-4-(trifluoromethyl)-1(2H)-pyrimidinyl]-4-fluorophenyl}imino}-4-oxo-delta5, alpha-thiazolidineacetic acid, diethyl ester;
ethyl 5-{{2-chloro-5-[3,6-dihydro-3-methyl-2,6-dioxo-4-(trifluoromethyl)-1(2H)-pyrimidinyl]-4-fluorophenyl}imino}-7-oxo-4-thia-6-azaspiro[2.4]heptane-6-acetate;
2-{{2-chloro-5-[3,6-dihydro-3-methyl-2,6-dioxo-4-(trifluoromethyl)-1 (2H)-pyrimidinyl]-4-fluorophenyl}-4-oxo-3,5-thiazolidinediacetic acid, 3-ethyl ester;
ethyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-oxazolidineacetate;
α-benzyl-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetic acid, L-;
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid, 3-ethyl methyl ester;
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid, 3-ethyl isopropyl ester;
ethyl 5-(carbamoylmethyl)-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
ethyl 2-{{2-chloro-5-[3,6-dihydro-3-methyl-2,6-dioxo-4-(trifluoromethyl)-1(2H)-pyrimidinyl]-4-fluorophenyl}imino}-5-[(methylcarbamoyl)methyl]-4-oxo-3-thiazolidineacetate;
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid, 3-ethyl (2-propynyl) ester;
methyl 3-allyl-2-{{2-chloro-5-[3,6-dihydro-3-methyl-2,6-dioxo-4-(trifluoromethyl)-1(2H)-pyrimidinyl]-4-fluorophenyl}imino}-4-oxo-5-thiazolidineacetate;
isopropyl 3-allyl-2-{{2-chloro-5-[3,6-dihydro-3-methyl-2,6-dioxo-4-(trifluoromethyl)-1(2H)-pyrimidinyl]-4-fluorophenyl}imino}-4-oxo-5-thiazolidineacetate;
methyl α-benzyl-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate, L-;
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-thiazolidinediacetic acid, 3-ethyl 2-fluoroethyl ester;
2-fluoroethyl 3-allyl-2-{{2-chloro-5-[3,6-dihydro-3-methyl-2,6-dioxo-4-(trifluoromethyl)-1(2H)pyrimidinyl]-4-fluorophenyl}imino}-4-oxo-5-thiazolidineacetate;
2-{{2-chloro-5-[3,6-dihydro-3-methyl-2,6-dioxo-4-(trifluoromethyl)-1(2H)-pyrimidinyl]-4-fluorophenyl}imino}-4-oxo-3,5-oxazolidinediacetic acid, 3-ethyl ester;
ethyl 2-{{2-chloro-5-[3,6-dihydro-3-methyl-2,6-dioxo-4-(trifluoromethyl)-1(2H)-pyrimidinyl]-4-fluorophenyl}imino}-5-(hydroxyethyl)-4-oxo-3-thiazolidineacetate;
allyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
isopropyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
3-{4-chloro-2-fluoro-5-[(3-furfuryl-4-oxo-2-thiazolidinylidene)amino]phenyl}-1-methyl-6-(trifluoromethyl)-2,4(1H,3H)-pyrimidinedione;
cyclopropylmethyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
ethyl 5-chloro-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
3-{4-chloro-5-[(3-cyclopropyl-4-oxo-2-thiazolidinylidene)amino]-2-fluorophenyl}-1-methyl-6-(trifluoromethyl)-2,4(1H,3H)-pyrimidinedione;
3-{4-chloro-2-fluoro-5-{[3-(2-methoxyethyl)-4-oxo-2-thiazolidinylidene]amino}phenyl}-1-methyl-6-(trifluoromethyl)-2,4(1H,3H)-pyrimidinedione;
3-{4-chloro-2-fluoro-5-{[3-(1-methyl-2-propynyl)-4-oxo-2-thiazolidinylidene]amino}phenyl}-1-methyl-6-(trifluoromethyl)-2,4(1H,3H)-pyrimidinedione;
ethyl 5-benzyl-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate;
3-{5-[(3-benzyl-4-oxo-2-thiazolidinylidene)amino]-4-chloro-2-fluorophenyl}-1-methyl-6-(trifluoromethyl)-2,4(1H,3H)-pyrimidinedione;
methyl 2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidinepropionate;
methyl α-benzyl-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate, D-;
methyl 2-{{2-chloro-5-[3,6-dihydro-3-methyl-2,6-dioxo-4-(trifluoromethyl)-1(2H)-pyrimidinyl]-4-fluorophenyl}imino}-4-oxo-α-(*p*-nitrobenzyl)-3-thiazolidineacetate, L-;
ethyl α-benzyl-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate, L-; and
methyl α-(*p*-chlorobenzyl)-2-{{2-chloro-5-[3,6-dihydro-3-methyl-2,6-dioxo-4-(trifluoromethyl)-1(2H)-pyrimidinyl]-4-fluorophenyl}imino}-4-oxo-3-thiazolidineacetate, DL-.

26. The composition according to claim 21 which further comprises one or more other biological compounds.

27. A compound having the structural formula 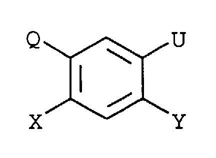 wherein
U is -N=C=NR, -N=C=S or 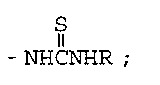 Q is 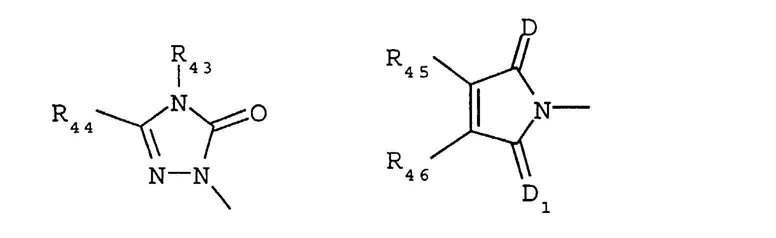 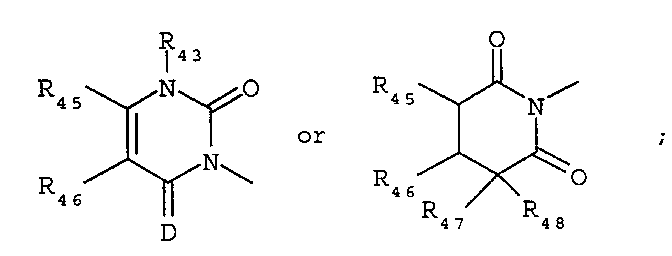 and D, D₁, R, R₄₃, R₄₄, R₄₅, R₄₆, R₄₇, R₄₈, X and Y are as described in claim 1.

28. The compound according to claim 27 wherein
U is -N=C=NR, -N=C=S or 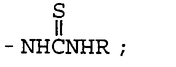 O is 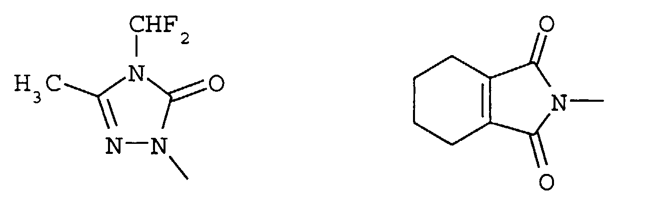 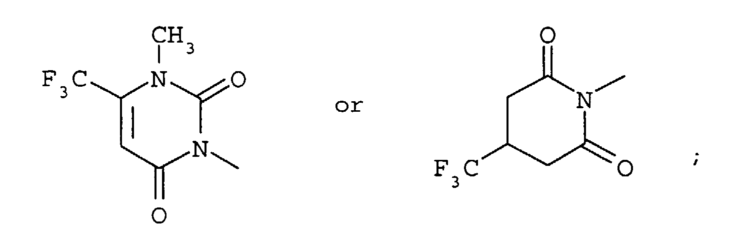
X is hydrogen, fluorine or chlorine;
Y is fluorine, chlorine, nitro or cyano;
R is hydrogen,
C₁-C₆alkyl optionally substituted with one to three halogen atoms, one or two C(O)OR₃ groups, one cyano group, one OR₇ group, one SR₈ group, one P(O) (OR₆)₂ group, or
a C₃-C₆alkenyl, C₃-C₆alkynyl, phenyl, benzyl or furfural group, wherein each group is optionally substituted with one to three halogen atoms, one C₁-C₃alkoxy group or one C(O)OR₃ group;
R₃ is hydrogen, C₁-C₆alkyl or C₁-C₆haloalkyl;
R₆ is hydrogen of C₁-C₃alkyl;
R₇ is hydrogen, C₁-C₃alkyl or C₃-C₆alkoxycarbonylalkyl; and
R₈ is hydrogen, C₁-C₃alkyl or C₃-C₆alkoxycarbonyl.

29. A method for the preparation of a compound having the structural formula 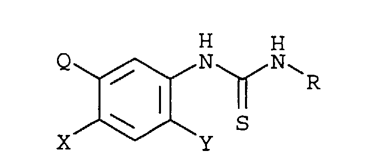 wherein
Q is 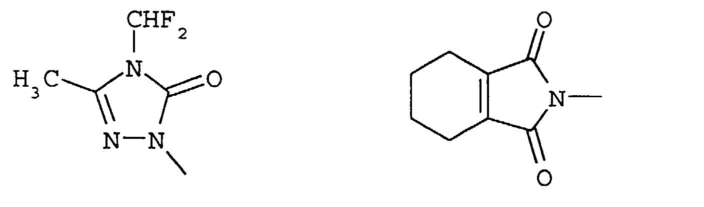 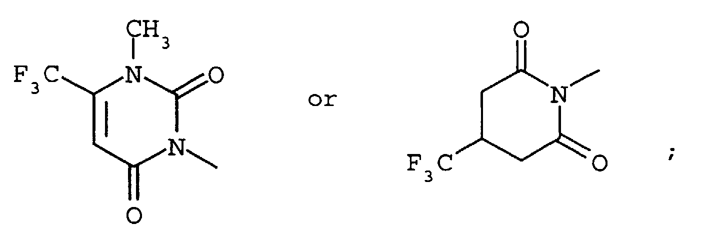
X is hydrogen, fluorine or chlorine;
Y is fluorine, chlorine, nitro or cyano;
R is hydrogen,
C₁-C₆alkyl optionally substituted with one to three halogen atoms, one or two C(O)OR₃ groups, one cyano group, one OR₇ group, one SR₈ group, one P(O) (OR₆)₂ group, or
a C₃-C₆alkenyl, C₃-C₆alkynyl, phenyl, benzyl or furfural group, wherein each group is optionally substituted with one to three halogen atoms, one C₁-C₃alkoxy group or one C(O)OR₃ group;
R₃ is hydrogen, C₁-C₆alkyl or C₁-C₆haloalkyl;
R₆ is hydrogen of C₁-C₃alkyl;
R₇ is hydrogen, C₁-C₃alkyl or C₃-C₆alkoxycarbonylalkyl; and
R₈ is hydrogen, C₁-C₃alkyl or C₃-C₆alkoxycarbonyl
which method comprises reacting a substituted aniline compound having the structural formula 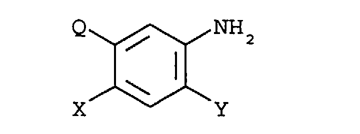 wherein Q, X and Y are as described above with an isothiocyanate compound having the structural formula
S=C=N-R
wherein R is as described above.

30. The method according to claim 29 wherein the reaction is conducted in the presence of a solvent.

31. A method for the preparation of a compound having the structural formula 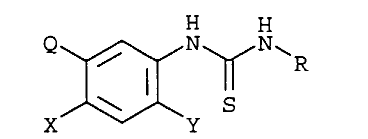 wherein
Q is 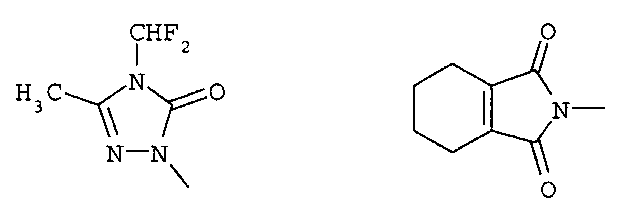 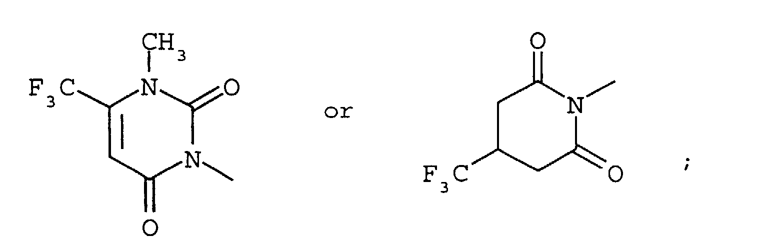
X is hydrogen, fluorine or chlorine;
Y is fluorine, chlorine, nitro or cyano;
R is hydrogen,
C₁-C₆alkyl optionally substituted with one to three halogen atoms, one or two C(O)OR₃ groups, one cyano group, one OR₇ group, one SR₈ group, one P(O) (OR₆)₂ group, or
a C₃-C₆alkenyl, C₃-C₆alkynyl, phenyl, benzyl or furfural group, wherein each group is optionally substituted with one to three halogen atoms, one C₁-C₃alkoxy group or one C(O)OR₃ group;
R₃ is hydrogen, C₁-C₆alkyl or C₁-C₆haloalkyl;
R₆ is hydrogen of C₁-C₃alkyl;
R₇ is hydrogen, C₁-C₃alkyl or C₃-C₆alkoxycarbonylalkyl; and
R₈ is hydrogen, C₁-C₃alkyl or C₃-C₆alkoxycarbonyl
which method comprises reacting an isothiocyanate compound having the structural formula 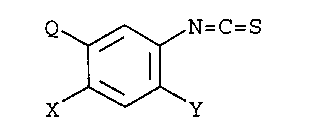 wherein Q, X and Y are as described above with a primary amine compound having the structural formula
H₂NR
wherein R is as described above.

32. The method according to claim 31 wherein the raction is conducted in the presence of a solvent.

33. A method for the preparation of a compound having the structural formula 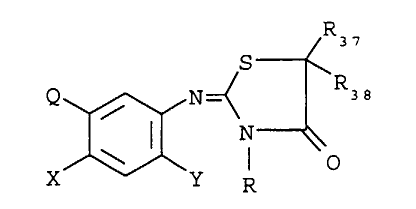 wherein
Q is 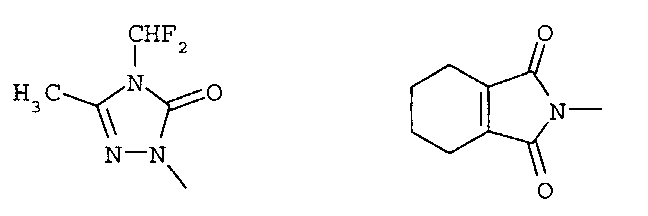 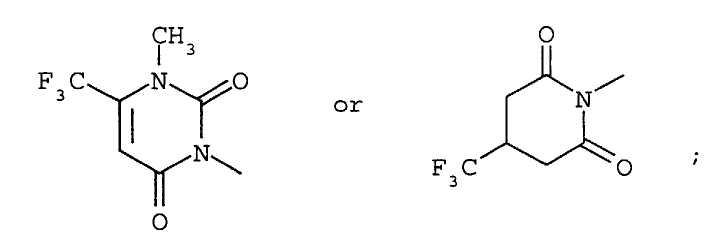
X is hydrogen, fluorine or chlorine;
Y is fluorine, chlorine, nitro or cyano;
R is hydrogen,
C₁-C₆alkyl optionally substituted with one to three halogen atoms, one or two C(O)OR₃ groups, one cyano group, one OR₇ group, one SR₈ group, one P(O) (OR₆)₂ group, or
a C₃-C₆alkenyl, C₃-C₆alkynyl, phenyl, benzyl or furfural group, wherein each group is optionally substituted with one to three halogen atoms, one C₁-C₃alkoxy group or one C(O)OR₃ group;
R₃ is hydrogen, C₁-C₆alkyl or C₁-C₆haloalkyl;
R₆ is hydrogen of C₁-C₃alkyl;
R₇ is hydrogen, C₁-C₃alkyl or C₃-C₆alkoxycarbonylalkyl;
R₈ is hydrogen, C₁-C₃alkyl or C₃-C₆alkoxycarbonyl;
R₃₇ and R₃₈ are each independently hydrogen, halogen,
C₁-C₆alkyl optionally substituted with one or two C(O)OR₃ groups, one or two C(O)NR₄R₅ groups, one or two OR₇ groups or one or two NR₉R₁₀ groups, or
benzyl optionally substituted on the phenyl ring with any combination of one to three C₁-C₄alkyl groups, one to three C₁-C₄alkoxy groups or one C₃-C₆alkoxycarbonylalkyl group, and
when R₃₇ and R₃₈ are taken together with the atom to which they are attached, they represent a ring in which R₃₇R₃₈ is a C₂-C₆alkylene group;
R₄ and R₅ are each independently hydrogen or C₁-C₆alkyl, and when R₄ and R₅ are taken together with the atom to which they are attached, they represent a ring in which R₄ and R₅ is a C₂-C₆alkylene group; and
R₉ and R₁₀ are each independently hydrogen, C₁-C₆alkyl or C₃-C₆alkoxycarbonylalkyl, and when R₉ and R₁₀ are taken together with the atom to which they are attached, they represent a ring in which R₉R₁₀ is a C₂-C₆alkylene group
which method comprises reacting a substituted thiourea compound having the structural formula 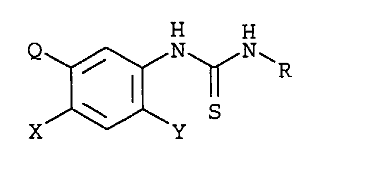 wherein Q, R, X and Y are as described above with a ketone compound having the structural formula 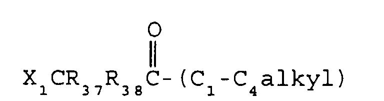 wherein R₃₇ and R₃₈ are as described above, and X₁ is halogen.

34. The method according to claim 33 wherein the reaction is conducted in the presence of a solvent.

35. The method according to claim 33 wherein the reaction is conducted in the presence of a base.

36. A method for the preparation of a compound having the structural formula 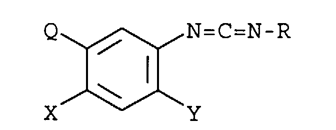 wherein
Q is 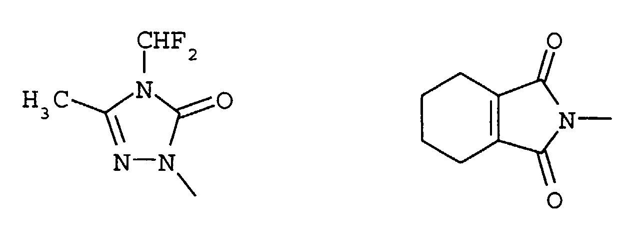 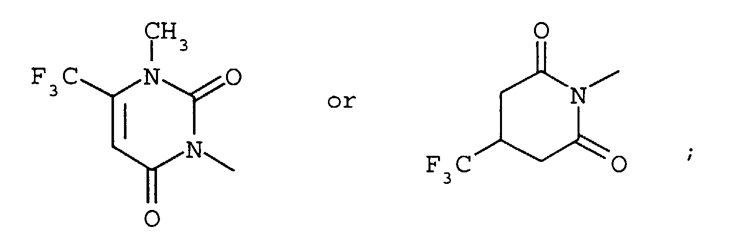
X is hydrogen, fluorine or chlorine;
Y is fluorine, chlorine, nitro or cyano;
R is hydrogen,
C₁-C₆alkyl optionally substituted with one to three halogen atoms, one or two C(O)OR₃ groups, one cyano group, one OR₇ group, one SR₈ group, one P(O) (OR₆)₂ group, or
a C₃-C₆alkenyl, C₃-C₆alkynyl, phenyl, benzyl or furfural group, wherein each group is optionally substituted with one to three halogen atoms, one C₁-C₃alkoxy group or one C(O)OR₃ group;
R₃ is hydrogen, C₁-C₆alkyl or C₁-C₆haloalkyl;
R₆ is hydrogen of C₁-C₃alkyl;
R₇ is hydrogen, C₁-C₃alkyl or C₃-C₆alkoxycarbonylalkyl; and
R₈ is hydrogen, C₁-C₃alkyl or C₃-C₆alkoxycarbonyl
which method comprises reacting a substituted thiourea compound having the structural formula 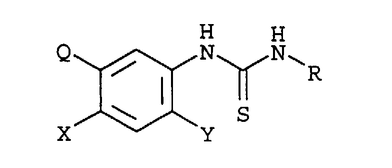 wherein Q, R, X and Y are as described above with a compound having the structural formula
R₅₅SO₂Cl
wherein R₅₅ is C₁-C₄alkyl or phenyl optionally substituted with one or two C₁-C₄alkyl groups in the presence of a base.

37. A method for the preparation of a compound having the structural formula 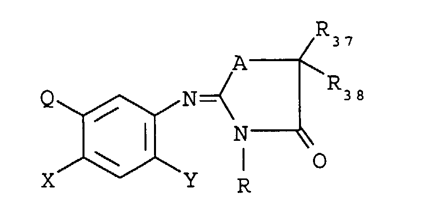 wherein
Q is 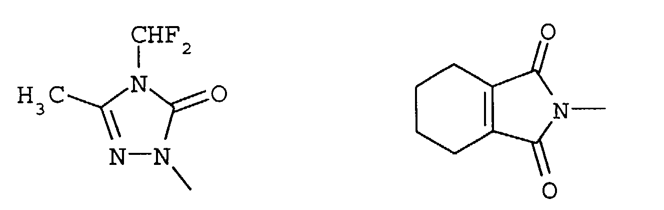 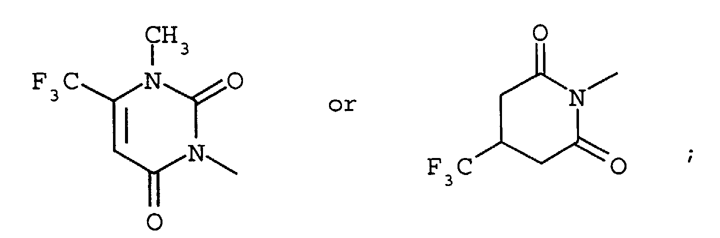
X is hydrogen, fluorine or chlorine;
Y is fluorine, chlorine, nitro or cyano;
R is hydrogen,
C₁-C₆alkyl optionally substituted with one to three halogen atoms, one or two C(O)OR₃ groups, one cyano group, one OR₇ group, one SR₈ group, one P(O) (OR₆)₂ group, or
a C₃-C₆alkenyl, C₃-C₆alkynyl, phenyl, benzyl or furfural group, wherein each group is optionally substituted with one to three halogen atoms, one C₁-C₃alkoxy group or one C(O)OR₃ group;
R₃ is hydrogen, C₁-C₆alkyl or C₁-C₆haloalkyl;
R₆ is hydrogen of C₁-C₃alkyl;
R₇ is hydrogen, C₁-C₃alkyl or C₃-C₆alkoxycarbonylalkyl;
R₈ is hydrogen, C₁-C₃alkyl or C₃-C₆alkoxycarbonyl;
R₃₇ and R₃₈ are each independently hydrogen, halogen,
C₁-C₆alkyl optionally substituted with one or two C(O)OR₃ groups, one or two C(O)NR₄R₅ groups, one or two OR₇ groups or one or two NR₉R₁₀ groups, or
benzyl optionally substituted on the phenyl ring with any combination of one to three C₁-C₄alkyl groups, one to three C₁-C₄alkoxy groups or one C₃-C₆alkoxycarbonylalkyl group, and
when R₃₇ and R₃₈ are taken together with the atom to which they are attached, they represent a ring in which R₃₇R₃₈ is a C₂-C₆alkylene group;
R₄ and R₅ are each independently hydrogen or C₁-C₆alkyl, and when R₄ and R₅ are taken together with the atom to which they are attached, they represent a ring in which R₄ and R₅ is a C₂-C₆alkylene group;
R₉ and R₁₀ are each independently hydrogen, C₁-C₆alkyl or C₃-C₆alkoxycarbonylalkyl, and
when R₉ and R₁₀ are taken together with the atom to which they are attached, they represent a ring in which R₉R₁₀ is a C₂-C₆alkylene group; and
A is O, N or S
which method comprises reacting a substituted carbodiimide having the structural formula 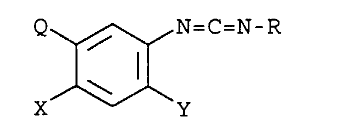 wherein Q, R, X and Y are as described above with a compound having the structural formula 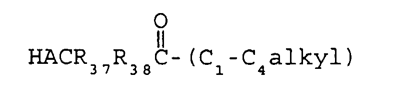 wherein A, R₃₇ and R₃₈ are as described above.

38. The method according to claim 37 wherein the reaction is conducted in the presence of a catalyst.
